# EUROPÄISCHE PATENTSCHRIFT

(11) **EP 1 888 596 B1**
(45) Veröffentlichungstag und Bekanntmachung des Hinweises auf die Patenterteilung: **22.10.2014**
(21) Anmeldenummer: 06753673.0
(22) Anmeldetag: 17.05.2006
(51) Int. Cl.: C07D 498/10, A61K 31/438, A61P 29/00

(54) **SUBSTITUIERTE SPIRO-VERBINDUNGEN UND DEREN VERWENDUNG ZUR HERSTELLUNG VON ARZNEIMITTELN GEGEN SCHMERZ**
SUBSTITUTED SPIRO COMPOUNDS AND THEIR USE FOR PRODUCING PAIN-RELIEF MEDICAMENTS
COMPOSES SPIRO SUBSTITUES, ET LEUR UTILISATION POUR PRODUIRE DES MEDICAMENTS CONTRE LA DOULEUR

(30) Priorität: 19.05.2005 DE 102005023783; 20.09.2005 DE 102005044813
(43) Veröffentlichungstag der Anmeldung: 20.02.2008
(73) Patentinhaber: Grünenthal GmbH, 52078 Aachen (DE)
(72) Erfinder: FRANK, Robert, 52070 Aachen (DE); REICH, Melanie, 52072 Aachen (DE); JOSTOCK, Ruth, 52223 Stolberg (DE); BAHRENBERG, Gregor, 52156 Monschau-Konzen (DE); SCHICK, Hans, 13086 Berlin (DE); HENKEL, Birgitta, 12555 Berlin (DE); SONNENSCHEIN, Helmut, 10245 Berlin (DE)
(74) Vertreter: Bülle, Jan
(86) Internationale Anmeldenummer: PCT/EP2006/004652
(87) Internationale Veröffentlichungsnummer: WO 2006/122770

(56) Entgegenhaltungen:
- WO-A-97/11940
- WO-A-03/000699
- WO-A-2005/110992

## Beschreibung

Die vorliegende Erfindung betrifft substituierte Spiro-Verbindungen, Verfahren zu ihrer Herstellung, Arzneimittel enthaltend diese Verbindungen und die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufrieden stellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Einen geeigneten Ansatzpunkt zur Behandlung von Schmerz, insbesondere von neuropathischem Schmerz, stellt der Vanilloid-Rezeptor vom Subtyp 1 (VR1/TRPV1) dar, der häufig auch als Capsaicin-Rezeptor bezeichnet wird. Dieser Rezeptor wird u.a. durch Vanilloide wie z.B. Capsaicin, Hitze und Protonen stimuliert und spielt eine zentrale Rolle bei der Schmerzentstehung. Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus; Störungen des kardiovaskulären Systems; Störungen der Nahrungsaufnahme; Medikamentenabhängigkeit; Medikamentenmißbrauch und insbesondere Harninkontinenz.

Die WO 03/000699 betrifft substituierte 1-Oxa-2,8-diaza-spiro[4.5]dec-2-en-Derivate, sowie Verfahren zu deren Herstellung, deren Verwendung zur Herstellung von Arzneimitteln und Arzneimittel enthaltend diese Verbindungen.

Die WO 97/11940 offenbart spirocyclische Verbindungen und deren Verwendung bei der Inhibierung von Blutplättchenaggregationen.

Aus der WO 2005/110992 sind Inhibitoren von 11-beta-Hydroxysteroid-Dehydrogenasen vom Typ 1, Antagonisten des Mineralokortikoid-Rezeptors (MR), und ihre pharmazeutischen Zusammensetzungen bekannt.

Die Verbindungen gemäß der WO 2005/110992 können bei der Behandlung von verschiedenen Krankheiten, die mit der Expression oder Aktivität von 11-beta-Hydroxysteroid-Dehydrogenasen vom Typ 1 im Zusammenhang stehen, sowie bei Krankheiten, die mit einem Aldosteron-Überschuß in Verbindung gebracht werden, eingesetzt werden.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, dass sich substituierte Spiro-Verbindungen der nachstehend angegebenen allgemeinen Formel I zur Bekämpfung von Schmerzen eignen und eine ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vanilloid-Rezeptoren 1 (VR1/TRPV1) vermittelt werden.

Ein Gegenstand der vorliegenden Erfindung sind daher substituierte Spiro-Verbindungen der allgemeinen Formel I, 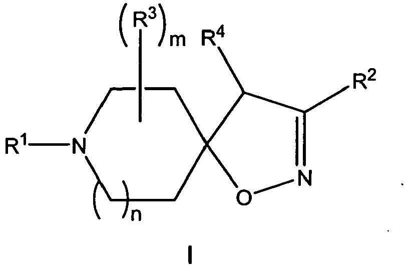 worin
- m: gleich 0, 1, 2, 3 oder 4 ist,
- n: gleich 0, 1 oder 2 ist,
- R¹: für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
für einen unsubstituierten oder wenigstens einfach substituierten Aryl- oder Heteroaryl-Rest, der über eine lineare oder verzweigte, unsubstituierte oder wenigstens einfach substituierte Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
für eine -C(=O)-NR⁵R⁶-Gruppe,
für eine -C(=S)-NR⁷R⁸-Gruppe,
für eine -C(=O)-R⁹-Gruppe
oder für eine -S(=O)₂-R¹⁰-Gruppe steht;
- R²: für einen linearen oder verzweigten, gesättigten oder ungesättigten, unsubstituierten oder wenigstens einfach substituierten, ggf. wenigstens ein Heteroatom als Kettenglied aufweisenden aliphatischen Rest;
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
für einen unsubstituierten oder wenigstens einfach substituierten Phenyl-Rest;
für einen unsubstituierten oder wenigstens einfach substituierten Phenyl-Rest, der mit einem unsubstituierten oder wenigstens einfach substituierten mono-oder polyzyklischen Ringsystem kondensiert ist,
für einen unsubstituierten oder wenigstens einfach substituierten Naphthyl-oder Heteroaryl-Rest, der mit einem unsubstituierten oder wenigstens einfach substituierten mono- oder polyzyklischen Ringsystem kondensiert sein kann,
oder für einen unsubstituierten oder wenigstens einfach substituierten Aryl-oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden ist und ggf. mit einem unsubstituierten oder wenigstens einfach substituierten mono- oder polyzyklischen Ringsystem kondensiert sein kann, steht;
- R³: für einen Halogen-Rest, für eine Nitro-Gruppe, für eine Hydroxy-Gruppe, für eine Thiol-Gruppe; für eine -O-R¹¹-Gruppe, für eine -S-R¹²-Gruppe, oder für einen linearen oder verzweigten, gesättigten oder ungesättigten, unsubstituierten oder wenigstens einfach substituierten aliphatischen Rest steht;
- R⁴: für einen Wasserstoff-Rest, für einen Halogen-Rest, für eine Nitro-Gruppe, für eine Hydroxy-Gruppe, für eine Thiol-Gruppe; für eine Oxo-Gruppe (=O), für eine -O-R¹¹-Gruppe, für eine -S-R¹²-Gruppe, oder für einen linearen oder verzweigten, gesättigten oder ungesättigten, unsubstituierten oder wenigstens einfach substituierten aliphatischen Rest steht;
- R⁵ und R⁷,: unabhängig voneinander, jeweils
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert und/oder mit wenigstens einer linearen oder verzweigten, unsubstituierten oder wenigstens einfach substituierten Alkylen-Gruppe überbrückt sein kann,
für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
- R⁶ und R⁸,: unabhängig voneinander, jeweils
für einen Wasserstoff-Rest,
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert und/oder mit wenigstens einer linearen oder verzweigten, unsubstituierten oder wenigstens einfach substituierten Alkylen-Gruppe überbrückt sein kann,
für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
- R⁹: für einen linearen oder verzweigten, gesättigten oder ungesättigten, unsubstituierten oder wenigstens einfach substituierten aliphatischen Rest;
oder für einen unsubstituierten oder wenigstens einfach substituierten Aryl-oder Heteroaryl-Rest, der über eine lineare oder verzweigte, unsubstituierte oder wenigstens einfach substituierte Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht;
- R¹⁰: für einen linearen oder verzweigten, gesättigten oder ungesättigten, unsubstituierten oder wenigstens einfach substituierten aliphatischen Rest;
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
oder für einen unsubstituierten oder wenigstens einfach substituierten Aryl-oder Heteroaryl-Rest, der über eine lineare oder verzweigte, unsubstituierte oder wenigstens einfach substituierte Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht;
und
- R¹¹ und R¹²,: unabhängig voneinander, jeweils
für einen linearen oder verzweigten, gesättigten oder ungesättigten, unsubstituierten aliphatischen Rest;
für einen unsubstituierten, ungesättigten oder gesättigten, ggf. wenigstens ein Heteroatom als Ringglied aufweisenden cycloaliphatischen Rest, der über eine lineare oder verzweigte, unsubstituierte Alkylen-, Alkenylen- oder Alkinylen-Gruppe gebunden und/oder mit einem unsubstituierten mono- oder polyzyklischen Ringsystem kondensiert sein kann,
oder für einen unsubstituierten Aryl- oder Heteroaryl-Rest, der über eine lineare oder verzweigte, unsubstituierte Alkylen-, Alkenylen- oder Alkinylen-Gruppe gebunden und/oder mit einem unsubstituierten mono- oder polyzyklischen Ringsystem kondensiert sein kann, steht;
wobei die Substituenten der vorstehend genannten aliphatischen Reste unabhängig voneinander aus der Gruppe bestehend aus F, Cl, Br, I, -CN, -NO₂, -OH, -SH und - NH₂ ausgewählt werden können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt weisen die Reste R¹, R⁴ und R² Alkylen-, Alkenylen- oder Alkinylen-Gruppen auf, die jeweils mit Substituenten unabhängig voneinander ausgewählt aus der Gruppe bestehend aus F, Cl, Br, -OH, -SH, -NH₂, -CN, -NO₂ und Phenyl substituiert sein können; wobei der Phenyl-Rest mit 1, 2, 3, 4 oder 5 Substituenten unabhängig voneinander ausgewählt aus der Gruppe bestehend aus F, Cl, Br, I, - CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann.

Bevorzugt kann der Rest R² in jeder der hierin angegebenen Definitionen für einen unsubstituierten oder wenigstens einfach substituierten Phenyl-Rest stehen, mit der Maßgabe, dass nicht eine der meta-Positionen und die para-Position dieses Phenyl-Restes mit Substituenten substituiert sind, die jeweils über ein gleiches Atom ausgewählt aus der Gruppe bestehend aus Sauerstoff, Schwefel und Stickstoff gebunden sind. Durch diese Maßgabe werden die in der Druckschrift WO 2005/21515 A1 in entsprechender Position genannten Substituenten ausgeschlossen.

Bevorzugt kann der Rest R² einen wie vorstehend definierten Phenyl-Rest umfassen, für einen unsubstituierten oder wenigstens einfach substituierten Phenyl-Rest, der mit einem unsubstituierten oder wenigstens einfach substituierten mono- oder polyzyklischen Ringsystem kondensiert ist, oder für einen unsubstituierten oder wenigstens einfach substituierten Naphthyl- oder Heteroaryl-Rest, der mit einem unsubstituierten oder wenigstens einfach substituierten mono- oder polyzyklischen Ringsystem kondensiert sein kann, steht, der ausgewählt wird aus der Gruppe bestehend aus Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl; 2H-Benzo[1.4]oxazin-3(4H)-onyl, (3,4)-Dihydrochinolin-2(1H)-onyl, [3,4]-Dihydro-2H-1,4-benzoxazinyl und Benzothiazolyl, wobei die Reste jeweils unsubstituiert oder wenigstens einfach substituiert sein können.

Aliphatische Reste umfassen im Sinne dieser Erfindung azyklische gesättigte oder ungesättigte Kohlenwasserstoffreste, die verzweigt oder geradkettig sowie unsubstituiert oder einfach substituiert oder mehrfach gleich oder verschieden substituiert sein können, mit vorzugsweise 1 bis 20 (d.h. 1, 2, 3, 4, 5, 6, 7, 8, 9, 10, 11, 12, 13, 14, 15, 16, 17, 18, 19 oder 20), besonders bevorzugt 1 bis 12 (d.h. 1, 2, 3, 4, 5, 6, 7, 8, 9, 10, 11 oder 12), ganz besonders bevorzugt 1 bis 6 (d.h. 1, 2, 3, 4, 5 oder 6) Kohlenstoffatomen, d.h. C₁₋₂₀-, C₁₋₁₂-, C₁₋₆-Alkyle, C₂₋₂₀-, C₂₋₁₂-, C₂₋₆-Alkenyle und C₂₋₂₀-, C₂₋₁₂-, C₂₋₆-Alkinyle. Dabei weisen Alkenyle mindestens eine C-C-Doppelbindung und Alkinyle mindestens eine C-C-Dreifachbindung auf. Vorteilhafterweise können aliphatische Reste ausgewählt werden aus der Gruppe, die Methyl, Ethyl, n-Propyl, Isopropyl, n-Butyl, Isobutyl, sec-Butyl, tert-Butyl, n-Pentyl, Isopentyl, neo-Pentyl, n-Hexyl, 2-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, -(CH₂)-(CH₂)-(C(CH₃)₃), -(CH₂)-(CH)(C₂H₅)-(CH₂)-(CH₂)-(CH₂)-(CH₃), Ethenyl (Vinyl), Ethinyl, Propenyl (-CH₂CH=CH₂, -CH=CH-CH₃, -C(=CH₂)-CH₃), 2-Methyl-propenyl, Propinyl (-CH₂-C=CH, -C=C-CH₃), Butenyl, Butinyl, Pentenyl, Pentinyl, Hexenyl, Hexinyl, Octenyl und Octinyl umfasst.

Die vorstehend genannten aliphatischen Reste können bevorzugt 1, 2 oder 3 Heteroatome ausgewählt aus der Gruppe umfassend Sauerstoff, Schwefel und Stickstoff, d. h. -N(H)- und -N(C₁₋₆-Alkyl), als Kettenglieder aufweisen.

Beispielhaft für aliphatische Reste, die 1, 2 oder 3 Heteroatome aufweisen, seien -(CH₂)-(CH₂)-O-CH₃, -(CH₂)-(CH₂)-(CH₂)-O-CH₃, -(CH₂)-(CH₂)-(CH₂)-N(C₂H₅)-(C₂H₅), -(CH₂)-(CH₂)-S-CH₃, -(CH₂)-(CH₂)-(CH₂)-S-CH₃, -(CH₂)-(CH₂)-(CH₂)-N(CH₃)-(CH₃) und -(CH₂)-O-CH₃ genannt.

Im Zusammenhang mit aliphatischen Resten versteht man unter dem Begriff "substituiert" - soweit nicht anders definiert - im Sinne dieser Erfindung die einfache oder mehrfache Substitution, bevorzugt die ein-, zwei-, drei-, vier-, fünf-, sechs-, sieben-, acht- oder neunfache Substitution, von einem oder mehreren Wasserstoffatomen durch beispielsweise F, Cl, Br, I, -CN, -NO₂, -OH, -SH und -NH₂, wobei die mehrfache Substitution entweder an verschiedenen oder an gleichen Atomen mehrfach, z.B. zwei- oder dreifach, erfolgt, beispielsweise dreifach am gleichen C-Atom wie im Falle von -CF₃ oder -CH₂CF₃ oder an verschiedenen Stellen wie im Falle von -CH(OH)-CH=CCl-CH₂Cl. Die Mehrfachsubstitution kann mit dem gleichen oder mit verschiedenen Substituenten erfolgen. Bevorzugte substituierte aliphatische Reste sind -CH₂-Cl, -CH₂-Br, -CH₂-CH₂-Cl, -CH₂-CH₂-Br, -CH₂-CH₂-CH₂-Br und -CH₂-CH₂-CH₂-Cl.

Cycloaliphatische Reste im Sinne dieser Erfindung sind zyklische gesättigte oder ungesättigte Kohlenwasserstoffreste mit vorzugsweise 3, 4, 5, 6, 7, 8, 9, 10, 11, 12, 13, 14, 15 oder 16, besonders bevorzugt 3, 4, 5, 6, 7 oder 8 Kohlenstoffatomen, wobei jeder Rest unsubstituiert oder einfach substituiert oder mehrfach gleich oder verschieden substituiert sein kann. Cycloaliphatische Reste können bevorzugt 1, 2, 3, 4 oder 5 Heteroatome unabhängig voneinander ausgewählt aus der Gruppe bestehend aus Sauerstoff, Stickstoff (NH) und Schwefel als Ringglieder aufweisen.

Beispielhaft für cycloaliphatische Reste, die ggf. mit 1 oder 2 linearen oder verzweigten C₁₋₅-Alkylen-Gruppen überbrückt und mit einen mono- oder polyzyklischen Ringsystem kondensiert sein können, seien Cyclopropyl, Cyclobutyl, Cyclopentyl, Cyclohexyl, Cyclooctyl, Cyclononyl, Cyclodecyl, Cycloundecyl, Cyclododecyl, Cycloheptyl, Cyclopentenyl, Cyclohexenyl, Cycloheptenyl, [6,6]-Dimethyl-[3.1.1]-bicycloheptyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl, (1,2,3,4)-Tetrahydrochinazolinyl, (1,3,4,5)-Tetrahydropyrido[4,3-b]indolyl, (3,4)-Dihydro-1H-isochinolinyl, (1,3,4,9)-Tetrahydro-[b]-carbolinyl, Imidazolidinyl, (1,3)-Thiazolidinyl, 9H-Flourenyl und 9H-Xanthenyl genannt.

Unter einem mono- oder polyzyklischen Ringsystem werden im Sinne der vorliegenden Erfindung mono- oder polyzyklische Kohlenwasserstoffreste verstanden, die gesättigt oder ungesättigt sein und ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind.
Ein solches mono- bzw. polyzyklisches Ringsystem kann beispielsweise mit einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt oder ungesättigt sein. Bevorzugt ist ein polyzyklisches Ringsystem ein bizyklisches Ringsystem.

Beispielhaft für Aryl-Reste, die mit einem mono- bzw. polyzyklischen Ringsystem kondensiert sind, seien [1,3]-Benzodioxolyl, [1,4]-Benzodioxanyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, [3,4]-Dihydro-2H-1,4-benzoxazinyl, 2H-Benzo[1.4]oxazin-3(4H)-onyl und (3,4)-Dihydrochinolin-2(1 H)-onyl genannt.

Im Zusammenhang mit cycloaliphatischen Resten und mono- oder polyzyklischen Ringsystemen versteht man unter dem Begriff "substituiert" - soweit nicht anders definiert - im Sinne dieser Erfindung die einfache oder mehrfache Substitution, bevorzugt, die ein-, zwei-, drei-, vier-, fünf-, sechs-, sieben-, acht- oder neunfache Substitution, von einem oder mehreren Wasserstoffatomen durch beispielsweise Oxo (=0), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -0-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, - C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Morpholinyl, Piperidinyl, Pyrrolidinyl, Piperazinyl, Pyridinyl, Pyridazinyl, - (CH₂)-Benzo[b]furanyl, -O-Phenyl, -O-Benzyl, Phenyl und Benzyl, wobei jeweils der zyklische Teil der Reste Pyridinyl, Cyclopentyl, Cyclohexyl, Morpholinyl, Piperidinyl, Pyrrolidinyl, Piperazinyl, Pyridazinyl, -S(=O)₂-Phenyl, -O-Phenyl, -O-Benzyl, Phenyl, - (CH₂)-Benzo[b]furanyl und Benzyl mit 1,2,3,4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann. Die mehrfache Substitution kann entweder an verschiedenen oder an gleichen Atomen mehrfach, z.B. zwei- oder dreifach, erfolgen. Die Mehrfachsubstitution kann mit dem gleichen oder mit verschiedenen Substituenten erfolgen.

Unter dem Ausdruck Aryl-Rest ist für die Zwecke der vorliegenden Erfindung bevorzugt ein Rest zu verstehen, der aus der Gruppe, die Phenyl, Naphthyl, Phenanthrenyl und Anthracenyl umfasst, ausgewählt ist und unsubstituiert oder einfach oder mehrfach gleich oder verschieden substituiert ist. Bevorzugt ist Aryl ein unsubstituiertes oder einfach substituiertes oder mehrfach, beispielsweise zwei-, drei- vier oder fünffach, gleich oder verschieden substituiertes Phenyl, 1-Naphthyl oder 2-Naphthyl.

Heteroaryl-Reste im Sinne der vorliegenden Erfindung sind solche Heterozyklen, die heteroaromatisch sind. Heteroaryl-Reste sind bevorzugt 5- bis 14-gliedrig, d. h. 5-, 6-, 7-, 8-, 9-, 10-, 11-, 12-, 13- oder 14-gliedrig und weisen bevorzugt 1, 2, 3, 4 oder 5 Heteroatome unabhängig voneinander ausgewählt aus der Gruppe umfassend Sauerstoff, Stickstoff und Schwefel auf. Jeder Heteroaryl-Rest kann unsubstituiert oder einfach substituiert oder mehrfach, beispielsweise zwei-, drei-, vier- oder fünffach, gleich oder verschieden substituiert vorliegen.

Beispielhaft für Heteroaryl-Rest im Sinne der vorliegenden Erfindung seien Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, Benzothiazolyl, Benzo[2,1,3]thiadiazolyl, [1,2,3]-Benzothiadiazolyl, [2,1,3]-Benzoxadiazolyl und [1,2,3]-Benzoxadiazolyl genannt.

In Bezug auf Aryl- und Heteroaryl-Reste versteht man im Sinne dieser Erfindung unter "substituiert" die ein- oder mehrfache, z.B. die ein-, zwei-, drei-, vier- oder fünffache, Substitution eines oder mehrerer Wasserstoffatome des Ringsystems durch geeignete Substituenten. Soweit die Bedeutung dieser geeigneten Substituenten im Zusammenhang mit Aryl- oder Heteroaryl-Resten nicht an anderer Stelle der Beschreibung oder in den Ansprüchen definiert ist, sind geeignete Substituenten F, Cl, Br, I, -CN, -CF₃, -SF₅, -OH, -O-C₁₋₁₀-Alkyl, -NH₂, -NO₂, -O-CF₃, - S-CF₃, -SH, -S-C₁₋₅-Alkyl, -C₁₋₁₀-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, - S(=O)₂-Phenyl, -NH-S(=O)₂-C₁₋₅-Alkyl, -NH-S(=O)₂-C₁₋₅-Alkylen-Phenyl, -NH-S(=O)₂-C₁₋₅-Alkylen-Naphthyl, -NH-S(=O)₂-Phenyl -NH-S(=O)₂-Naphthyl, -S(=O)₂-NH-C₁₋₅-Alkyl, Cyclohexyl, Cyclopentyl, Morpholinyl, Piperidinyl, Pyrrolidinyl, Piperazinyl, Pyridinyl, Pyridazinyl, -(CH₂)-Benzo[b]furanyl, -O-Phenyl, -O-Benzyl, Phenyl und Benzyl, wobei jeweils der zyklische Teil der Reste Pyridinyl, Cyclopentyl, Cyclohexyl, Morpholinyl, Piperidinyl, Pyrrolidinyl, Piperazinyl, Pyridazinyl, -S(=O)₂-Phenyl, -O-Phenyl, -O-Benzyl, Phenyl, -(CH₂)-Benzo[b]furanyl, -NH-S(=O)₂-C₁₋₅-Alkylen-Phenyl, -NH-S(=O)₂-C₁₋₅-Alkylen-Naphthyl, -NH-S(=O)₂-Phenyl, -NH-S(=O)₂-Naphthyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann. Die Mehrfachsubstitution erfolgt dabei mit dem gleichen oder mit unterschiedlichen Substituenten.

Die Reste ausgewählt aus der Gruppe bestehend aus Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, 2H-Benzo[1.4]oxazin-3(4H)-onyl, (3,4)-Dihydrochinolin-2(1H)-onyl, [3,4]-Dihydro-2H-1,4-benzoxazinyl und Benzothiazolyl können wie die vorstehend genannten Aryl- und Heteroaryl-Reste substituiert sein.

Sofern R² für einen substituierten Phenyl-Rest steht, kann dieser besonders bevorzugt ausgewählt werden aus der Gruppe bestehend aus Biphenyl, 2-Pentafluor-sulfanyl-phenyl, 2-Methansulfonamid-phenyl, 2-Ethansulfonamid-phenyl, 2-Trifluoromethyl-phenyl, 2-Butoxy-phenyl, 2-(1,1)-Dimethyl-propyl-phenyl, 2-Nitro-phenyl, 2-Ethyl-benzoat, 2-Acetamid-phenyl, 2-Dimethylamino-phenyl, 2-Diethylamino-phenyl, 2-Amino-phenyl, 2-Benzol-sulfonamid, 2-Trifluoromethyl-sulfanyl-phenyl, 2-Ethyl-phenyl, 2-tert-Butyl-phenyl, 2-Methyl-benzoat, 2-Methansulfonyl-phenyl, 2-Ethylamino-sulfonyl-phenyl, 2-Methylamino-sulfonyl-phenyl, 2-Bromo-phenyl, 2-Chloro-phenyl, 2-Fluoro-phenyl, 2-Methyl-phenyl, 2-Trifluoromethoxy-phenyl, 2-Methoxy-phenyl, 2-Ethoxy-phenyl, 2-Propyl-phenyl, 2-Cyano-phenyl, 2-Acetylphenyl, 2-Isopropyl-phenyl, 2-lodo-phenyl, 3-Pentafluorsulfanyl-phenyl, 3-Chloro-phenyl, 3-Methyl-phenyl, 3-Butoxy-phenyl, 3-Nitro-phenyl, 3-tert-Butyl-phenyl, 3-Trifluoromethylsulfanyl-phenyl, 3-Trifluoromethylphenyl, 3-Methansulfonyl-phenyl, 3-Methansulfonamid-phenyl, 3-Ethansulfonamid-phenyl, 3-Benzol-sulfonamid, 3-Ethyl-benzoat, 3-Fluoro-phenyl, 3-Propyl-phenyl, 3-Isopropyl-phenyl, 3-Bromo-phenyl, 3-Dimethylamino-phenyl, 3-(1,1)-Dimethyl-propyl-phenyl, 3-Acetamid-phenyl, 3-Diethylamino-phenyl, 3-Amino-phenyl, 3-Methoxy-phenyl, 3-Ethyl-phenyl, 3-Ethylamino-sulfonyl-phenyl, 3-Methylamino-sulfonyl-phenyl, 3-Ethoxy-phenyl, 3-Cyano-phenyl, 3-Iodo-phenyl, 3-Trifluoromethoxy-phenyl, 3-Acetylphenyl, 4-Methansulfonyl-phenyl, 4-Methylamino-sulfonyl-phenyl, 4-Ethylamino-sulfonyl-phenyl, 4-Methansulfonamid-phenyl, 4-Ethansulfonamid-phenyl, 4-Pentafluorsulfanyl-phenyl, 4-Bromo-phenyl, 4-Methoxy-phenyl, 4-Chloro-phenyl, 4-Benzol-sulfonamid, 4-Fluoro-phenyl, 4-tert-Butyl-phenyl, 4-Cyano-phenyl, 4-Butoxy-phenyl, 4-Nitro-phenyl, 4-Trifluoromethylsulfanyl-phenyl, 4-Methyl-phenyl, 4-Isopropyl-phenyl, 4-Trifluoromethyl-phenyl, 4-Dimethylamino-phenyl, 4-Propyl-phenyl, 4-Diethylamino-phenyl, 4-Ethyl-benzoat, 4-Amino-phenyl, 4-Iodo-phenyl, 4-Trifluoromethoxy-phenyl, 4-(1,1)-Dimethyl-propyl-phenyl, 4-(3,5-Dichloro-phenylsulfamoyl)-phenyl, 4-Acetamid-phenyl, 4-Ethyl-phenyl, 4-Ethoxy-phenyl, 4-Methyl-benzoat, 4-Acetyl-phenyl, 2-Fluoro-3-trifluoromethylphenyl, (2,3)-Difluoro-phenyl, (2,3)-Dimethyl-phenyl, (2,3)-Dichlorophenyl, 3-Fluoro-2-trifluoromethylphenyl, (2,4)-Dichloro-phenyl, (2,4)-Difluorophenyl, 4-Fluoro-2-trifluoromethyl-phenyl, (2,4)-Dimethoxyphenyl, 2-Chloro-4-fluoro-phenyl, 2-Chloro-4-nitro-phenyl, (2,4)-Dibromo-phenyl, 2-Fluoro-4-trifluoromethyl-phenyl, (2,5)-Difluoro-phenyl, 2-Fluoro-5-trifluoromethyl-phenyl, 5-Fluoro-2-trifluoromethyl-phenyl, 5-Chloro-2-trifluoromethyl-phenyl, 5-Bromo-2-trifluoromethyl-phenyl, (2,5)-Dimethoxy-phenyl, (2,5)-Bistrifluoromethyl-phenyl, (2,5)-Dichloro-phenyl, (2,5)-Dibromo-phenyl, 2-Methoxy-5-nitro-phenyl, 2-Fluor-6-trifluoromethyl-phenyl, (2,6)-Dimethoxy-phenyl, (2,6)-Dimethyl-phenyl, (2,6)-Dichloro-phenyl, 2-Chloro-6-fluoro-phenyl, 2-Bromo-6-chlorophenyl, 2-Bromo-6-fluoro-phenyl, (2,6)-Difluoro-phenyl, (2,6)-Difluoro-3-methylphenyl, (2,6)-Dibromo-phenyl, (2,6)-Dichlorophenyl, 3-Chloro-2-fluoro-phenyl, (3,4)-Dichlorophenyl, 4-Chloro-3-nitro-phenyl, 4-Fluoro-3-trifluoromethylphenyl, 3-Fluoro-4-trifluoromethyl-phenyl, (3,4)-Difluoro-phenyl, 4-Chloro-3-trifluoromethyl, 4-Bromo-3-methyl-phenyl, 4-Bromo-5-methyl-phenyl, 3-Chloro-4-fluoro-phenyl, 4-Fluoro-3-nitrophenyl, 4-Bromo-3-nitro-phenyl, (3,4)-Dibromo-phenyl, 4-Chlor-3-methyl-phenyl, 4-Bromo-3-methyl-phenyl, 4-Fluoro-3-methyl-phenyl, 4-Methyl-3-nitro-phenyl, (3,5)-Dimethoxy-phenyl, (3,5)-Bis-trifluoromethyl-phenyl, (3,5)-Difluoro-phenyl, (3,5)-Dinitro-phenyl, (3,5)-Dichloro-phenyl, 3-Fluoro-5-trifluoromethyl-phenyl, 5-Fluoro-3-trifluoromethyl-phenyl, (3,5)-Dibromo-phenyl, 5-Chloro-4-fluoro-phenyl, 5-Bromo-4-methyl-phenyl, (2,3,4)-Trifluorophenyl, (2,3,4)-Trichlorophenyl, (2,3,6)-Trifluorophenyl, 5-Chloro-2-methoxy-phenyl, (2,3)-Difluoro-4-methyl-phenyl, (2,4,5)-Trifluorophenyl, (2,4,5)-Trichloro-phenyl, (2,4)-Dichloro-5-fluoro-phenyl, (2,4,6)-Trichlorophenyl, (2,4,6)-Trimethylphenyl, (2,4,6)-Trifluoro-phenyl, (2,4,6)-Trimethoxy-phenyl, (2,3,4,5)-Tetrafluoro-phenyl, 4-Methoxy-2,3,6-trimethyl-phenyl, 4-Methoxy-2,3,6-trimethyl-phenyl, 4-Chloro-2,5-dimethyl-phenyl, 2-Chloro-6-fluoro-3-methyl-phenyl, 6-Chloro-2-fluoro-3-methyl, (2,3,4,5,6)-Pentafluoro-phenyl, 3-Fluoro-4-methylsulfonamido-phenyl, 3-Chlor-4-methylsulfonamido-phenyl, 3-Brom-4-methylsulfonamido-phenyl, 3-Methoxy-4-methylsulfonamido-phenyl, 3-Hydroxy-4-methylsulfonamido-phenyl, 3-Trifluoromethyl-4-methylsulfonamido-phenyl, 3-Trifluoromethoxy-4-methylsulfonamido-phenyl, 3-Methyl-4-methylsulfonamido-phenyl, 3-Ethyl-4-methylsulfonamido-phenyl, 3-Isopropyl-4-methylsulfonamido-phenyl, 3-Propyl-4-methylsulfonamido-phenyl, 3-tert-Butyl-4-methylsulfonamido-phenyl, 3-Fluoro-4-phenylsulfonamido-phenyl, 3-Chlor-4-phenylsulfonamido-phenyl, 3-Brom-4-phenylsulfonamido-phenyl, 3-Methoxy-4-phenylsulfonamido-phenyl, 3-Hydroxy-4-phenylsulfonamido-phenyl, 3-Trifluoromethyl-4-phenylsulfonamido-phenyl, 3-Trifluoromethoxy-4-phenylsulfonamido-phenyl, 3-Methyl-4-phenylsulfonamido-phenyl, 3-Ethyl-4-phenylsulfonamido-phenyl, 3-Isopropyl-4-phenylsulfonamido-phenyl, 3-Propyl-4-phenylsulfonamido-phenyl, 3-tert-Butyl-4-phenylsulfonamido-phenyl, 4-Fluoro-3-methylsulfonamido-phenyl, 4-Chlor-3-methylsulfonamido-phenyl, 4-Brom-3-methylsulfonamido-phenyl, 4-Methoxy-3-methylsulfonamido-phenyl, 4-Hydroxy-3-methylsulfonamido-phenyl, 4-Trifluoromethyl-3-methylsulfonamido-phenyl, 4-Trifluoromethoxy-3-methylsulfonamido-phenyl, 4-Methyl-3-methylsulfonamido-phenyl, 4-Ethyl-3-methylsulfonamido-phenyl, 4-Isopropyl-3-methylsulfonamido-phenyl, 4-Propyl-3-methylsulfonamido-phenyl, 4-tert-Butyl-3-methylsulfonamido-phenyl, 4-Fluoro-3-phenylsulfonamido-phenyl, 4-Chlor-3-phenylsulfonamido-phenyl, 4-Brom-3-phenylsulfonamido-phenyl, 4-Methoxy-3-phenylsulfonamido-phenyl, 4-Hydroxy-3-phenylsulfonamido-phenyl, 4-Trifluoromethyl-3-phenylsulfonamido-phenyl, 4-Trifluoromethoxy-3-phenylsulfonamido-phenyl, 4-Methyl-3-phenylsulfonamido-phenyl, 4-Ethyl-3-phenylsulfonamido-phenyl, 4-Isopropyl-3-phenylsulfonamido-phenyl, 4-Propyl-3-phenylsulfonamido-phenyl, 4-tert-Butyl-3-phenylsulfonamido-phenyl, 2-Cyclohexyl-phenyl, 3-Cyclohexyl-phenyl und 4-Cyclohexyl-phenyl.

Die vorstehend genannten linearen oder verzweigten Alkylen-, Alkenylen- oder Alkinylen-Gruppen weisen bevorzugt 1 bis 5 Kohlenstoffatome auf, d.h. es handelt sich um C₁₋₅-Alkylen, C₂₋₅-Alkenylen oder C₂₋₅-Alkinylen-Gruppen, die jeweils unsubstituiert oder mit 1, 2, 3, 4 oder 5 Substituenten unabhängig voneinander ausgewählt aus der Gruppe bestehend aus F, Cl, Br, -OH, -SH, -NH₂, -CN, -NO₂ und Phenyl substituiert sein können, wobei der Phenyl-Rest mit 1, 2, 3, 4 oder 5 Substituenten unabhängig voneinander ausgewählt aus der Gruppe bestehend aus Methyl, Ethyl, n-Propyl, Isopropyl, n-Butyl, Isobutyl, sec-Butyl, tert-Butyl, n-Pentyl, Isopentyl und neo-Pentyl substituiert sein kann.

Die vorstehend genannten Alkylen-, Alkenylen- oder Alkinylen-Gruppen weisen ggf. jeweils 1 oder 2 Heteroatom(e) ausgewählt aus der Gruppe bestehend aus Sauerstoff, Stickstoff, d. h. -N(H)- und -N(C₁₋₆-Alkyl)-, und Schwefel als Kettenglied(er) auf.

Bevorzugt können Alkylen-Gruppen ausgewählt werden aus der Gruppe bestehend aus -(CH₂)-, -(CH₂)₂-, -C(H)(CH₃)-, -C(CH₃)₂-, -(CH₂)₃-, -(CH₂)₄-, -(CH₂)₅-, -C(H)(CH₃)-(CH₂)-, -C(H)(C₂H₅)-(CH₂)-, -C(Phenyl)₂-, -C(H)(Phenyl)-, -(CH₂)-O-, -(CH₂)-N(CH₃)-, -(CH₂)-S-, -(CH₂)-(CH₂)-N(CH₃)- und -(CH₂)-(CH₂)-N(C₂H₅)-.

Bevorzugt können Alkenylen-Gruppen ausgewählt werden aus der Gruppe bestehend aus -CH=CH-, -C(CH₃)=CH-, -C(C₂H₅)=CH-, -CH=C(CH₃)-, -CH=C(C₂H₅)-, -CH=C(Phenyl)-, -CH=C(p-Tolyl), -C(Phenyl)=CH- und -C(p-Tolyl)=CH-.

Bevorzugt als Alkinylen-Gruppe ist eine -C≡C-Gruppe.

Bevorzugt sind substituierte Spiro-Verbindungen der vorstehend angegebenen allgemeinen Formel I, worin
- m: gleich 0, 1, 2, 3 oder 4 ist,
- n: gleich 0, 1 oder 2 ist,
- R¹: für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
für eine -C(=O)-NR⁵R⁶-Gruppe,
für eine -C(=S)-NR⁷R⁸-Gruppe,
für eine -C(=O)-R⁹-Gruppe,
für eine -S(=O)₂-R¹⁰-Gruppe,
oder für -(CHR¹³)-(CHR¹⁴)_{f}-(CHR¹⁵)ₕ-R¹⁶ mit f = 0 oder 1 und h= 0 oder 1 steht;
- R²: für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen Phenyl-Rest, der mit 1, 2, 3, 4 oder 5 Substituenten unabhängig voneinander ausgewählt aus der Gruppe bestehend aus F, Cl, Br, I, -CN, - CF₃, -SF₅, -OH, -O-C₁₋₅-Alkyl, -NH₂, -NO₂, -O-CF₃, -S-CF₃, -SH, -S-C₁₋₅-Alkyl, - C₁₋₁₀-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, - C(=O)-NH₂, -C(=O)-NH-C₁₋₅-Alkyl, C(=O)-N-(C₁₋₅-Alkyl)₂, -S(=O)₂-C₁₋₅-Alkyl, - S(=O)₂-Phenyl, -NH-S(=O)₂-C₁₋₅-Alkyl, -NH-S(=O)₂-C₁₋₅-Alkylen-Phenyl, -NH-S(=O)₂-C₁₋₅-Alkylen-Naphthyl, -NH-S(=O)₂-Phenyl, -NH-S(=O)₂-Naphthyl, Cyclohexyl, Cyclopentyl, Morpholinyl, Piperidinyl, Piperazinyl, Pyrrolidinyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Morpholinyl, Piperidinyl, Piperazinyl, Pyrrolidinyl, Pyridazinyl, -S(=O)₂-Phenyl, -O-Phenyl, -O-Benzyl, Phenyl, - (CH₂)-Benzo[b]furanyl, -NH-S(=O)₂-C₁₋₅-Alkylen-Phenyl, -NH-S(=O)₂-C₁₋₅-Alkylen-Naphthyl, -NH-S(=O)₂-Phenyl, -NH-S(=O)₂-Naphthyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
mit der Maßgabe, dass nicht eine der meta-Positionen und die para-Position dieses Phenyl-Restes mit Substituenten substituiert sind, die jeweils über ein gleiches Atom ausgewählt aus der Gruppe bestehend aus Sauerstoff, Schwefel und Stickstoff an den Phenyl-Rest gebunden sind;
für einen ggf. substituierten Rest ausgewählt aus der Gruppe bestehend aus Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, 2H-Benzo[1.4]oxazin-3(4H)-onyl, (3,4)-Dihydrochinolin-2(1H)-onyl, [3,4]-Dihydro-2H-1,4-benzoxazinyl und Benzothiazolyl steht,
oder für -(CHR¹⁷)-X_{q}-(CHR¹⁸)ᵣ-Yₛ-(CHR¹⁹)ₜ-Zᵤ-R²⁰ mit q = 0 oder 1, r = 0 oder 1, s = 0 oder 1, t = 0 oder 1, u = 0 oder 1, worin X, Y und Z, unabhängig voneinander, jeweils für O, S, NH, N(CH₃), N(C₂H₅) oder N[CH(CH₃)₂] stehen, steht;
- R³: für einen Halogen-Rest, für eine Nitro-Gruppe, für eine Hydroxy-Gruppe, für eine Thiol-Gruppe; für eine -O-R¹¹-Gruppe, für eine -S-R¹²-Gruppe, oder für einen linearen oder verzweigten, gesättigten oder ungesättigten, ggf. substituierten C₁₋₁₀ aliphatischen Rest steht;
- R⁴: für einen Wasserstoff-Rest, für einen Halogen-Rest, für eine Nitro-Gruppe, für eine Hydroxy-Gruppe, für eine Thiol-Gruppe; für eine Oxo-Gruppe (=0), für eine -O-R¹¹-Gruppe, für eine -S-R¹²-Gruppe, oder für einen linearen oder verzweigten, gesättigten oder ungesättigten, ggf. substituierten C₁₋₁₀ aliphatischen Rest steht;
- R⁵ und R⁷,: unabhängig voneinander, jeweils
für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11- oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen-Gruppen überbrückt sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann;
oder für -(CR²¹R²²)-Xᵥ-(CHR²³)_{w}-Yₓ-(CHR²⁴)_{y}-Z_{z}-R²⁵ mit v = 0 oder 1, w = 0 oder 1, x = 0 oder 1, y = 0 oder 1, z = 0 oder 1, worin X, Y und Z, unabhängig voneinander, jeweils für O, S, NH, N(CH₃), N(C₂H₅) oder N[CH(CH₃)₂] stehen, stehen;
- R⁶ und R⁸,: unabhängig voneinander, jeweils
für einen Wasserstoff-Rest;
für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11- oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen-Gruppen überbrückt sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann;
oder für -(CR²¹R²²)-Xᵥ-(CHR²³)_{w}-Yₓ-(CHR²⁴)_{y}-Z_{z}-R²⁵ mit v = 0 oder 1, w = 0 oder 1, x = 0 oder 1, y = 0 oder 1, z = 0 oder 1, worin X, Y und Z, unabhängig voneinander, jeweils für O, S, NH, N(CH₃), N(C₂H₅) oder N[CH(CH₃)₂] stehen, stehen;
- R⁹: für einen linearen oder verzweigten, gesättigten oder ungesättigten, ggf, substituierten C₁₋₁₀ aliphatischen Rest;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
für -(CR²⁶R²⁷)-(CHR²⁸)ₐₐ-(CHR²⁹)_{bb}-R³⁰ mit aa = 0 oder 1 und bb = 0 oder 1;
für -CR³¹=CR³²-R³³
oder für -C≡C-R³⁴ steht;
- R¹⁰: für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf, substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
für -(CR²⁶R²⁷)-(CHR²⁸)ₐₐ-(CHR²⁹)_{bb}-R³⁰ mit aa = 0 oder 1 und bb = 0 oder 1;
für -CR³¹=CR³²-R³³
oder für -C≡C-R³⁴ steht;
- R¹¹ und R¹²,: unabhängig voneinander, jeweils
für einen linearen oder verzweigten, gesättigten oder ungesättigten C₁₋₁₀ aliphatischen Rest;
für einen ungesättigten oder gesättigten 3-, 4-, 5-, 6-, 7-, 8- oder 9-gliedrigen cycloaliphatischen Rest, der mit einem gesättigten, ungesättigten oder aromatischen mono- oder polyzyklischen Ringsystem kondensiert sein kann;
oder für einen 5- bis 14-gliedrigen Aryl- oder Heteroaryl-Rest, der mit einem gesättigten oder ungesättigten mono- oder polyzyklischen Ringsystem kondensiert sein kann, stehen;
- R¹³, R¹⁴, R¹⁵, R¹⁷, R¹⁸, R¹⁹, R²¹, R²², R²³, R²⁴, R²⁸, R²⁹ und R³¹,: unabhängig voneinander, jeweils
für einen Wasserstoff-Rest;
für einen linearen oder verzweigten, gesättigten oder ungesättigten, ggf. substituierten C₁₋₁₀ aliphatischen Rest,
oder für einen ggf. substituierten 5- bis 14-gliedrigen Aryl- oder Heteroaryl-Rest, der mit einem gesättigten oder ungesättigten, ggf. substituierten mono-oder polyzyklischen Ringsystem kondensiert sein kann, stehen;
- R²⁶ und R²⁷,: unabhängig voneinander, jeweils
für einen Wasserstoff-Rest;
für einen linearen oder verzweigten, gesättigten oder ungesättigten, ggf. substituierten C₁₋₁₀ aliphatischen Rest,
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann; oder für -OH stehen;
- R³²: für einen Wasserstoff-Rest;
für einen linearen oder verzweigten, gesättigten oder ungesättigten, ggf. substituierten C₁₋₁₀ aliphatischen Rest,
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht;
oder für -(CH₂)_{cc}-R³⁵ mit cc = 1, 2, 3 oder 4 steht oder für -CH=CH-R³⁶ steht;
- R¹⁶, R²⁰, R²⁵, R³⁰, R³³ und R³⁴,: unabhängig voneinander, jeweils
für einen linearen oder verzweigten, gesättigten oder ungesättigten, ggf. substituierten C₁₋₁₀ aliphatischen Rest,
für einen ungesättigten oder gesättigten, ggf. substituierten 3-, 4-, 5-, 6-, 7-, 8-oder 9-gliedrigen cycloaliphatischen Rest, der mit 1, 2, 3, 4 oder 5 linearen oder verzweigten, ggf. substituierten C₁₋₅-Alkylen-Gruppen überbrückt und/oder mit einem gesättigten, ungesättigten oder aromatischen, ggf. substituierten mono- oder polyzyklischen Ringsystem kondensiert sein kann;
oder für einen ggf. substituierten 5- bis 14-gliedrigen Aryl- oder Heteroaryl-Rest, der mit einem gesättigten oder ungesättigten, ggf. substituierten mono-oder polyzyklischen Ringsystem kondensiert sein kann, stehen;
und
- R³⁵ und R³⁶,: unabhängig voneinander, jeweils
für einen ggf. substituierten 6- oder 10-gliedrigen Aryl-Rest, der mit einem gesättigten oder ungesättigten, ggf. substituierten mono- oder polyzyklischen Ringsystem kondensiert sein kann, stehen;
wobei
die vorstehend genannten C₁₋₁₀ aliphatischen Reste und C₁₋₂₀ aliphatischen Reste jeweils ggf. mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -SH und - NH₂ substituiert sein können;
die vorstehend genannten cycloaliphatischen Reste jeweils ggf. mit 1, 2, 3, 4 oder 5 Substituenten unabhängig voneinander ausgewählt aus der Gruppe bestehend aus Oxo (=0), Thioxo (=S), F, Cl, Br, I, -CN, -CF₃, -SF₅, -OH, -O-C₁₋₅-Alkyl, -NH₂, -NO₂, - O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, - C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, - CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-_{C1-5}-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Morpholinyl, Piperidinyl, Pyrrolidinyl, Piperazinyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Morpholinyl, Piperidinyl, Pyrrolidinyl, Piperazinyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und, sofern nicht anders angegeben, die vorstehend genannten Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, 2H-Benzo[1.4]oxazin-3(4H)-onyl, (3,4)-Dihydrochinolin-2(1 H)-onyl, [3,4]-Dihydro-2H-1,4-benzoxazinyl und Benzothiazolyl sowie Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₁₀-Alkyl, -C(=O)-OH, -C(=O)-O-C₁₋₅-Alkyl, -O-C(=O)-C₁₋₅-Alkyl, -NH-C₁-₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -NH-S(=O)₂-C₁₋₅-Alkylen-Phenyl, -NH-S(=O)₂-C₁₋₅-Alkylen-Naphthyl, -NH-S(=O)₂-Phenyl, -NH-S(=O)₂-Naphthyl, Cyclohexyl, Cyclopentyl, Morpholinyl, Piperidinyl, Piperazinyl, Pyrrolidinyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Morpholinyl, Piperidinyl, Piperazinyl, Pyrrolidinyl, Pyridazinyl, -S(=O)₂-Phenyl, -O-Phenyl, -O-Benzyl, Phenyl, -(CH₂)-Benzo[b]furanyl, -NH-S(=O)₂-C₁₋₅-Alkylen-Phenyl, -NH-S(=O)₂-C₁₋₅-Alkylen-Naphthyl, -NH-S(=O)₂-Phenyl, -NH-S(=O)₂-Naphthyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
und
die vorstehend genannten C₁₋₅-Alkylen-Gruppen jeweils ggf. mit 1, 2, 3, 4 oder 5 Substituenten unabhängig voneinander ausgewählt aus der Gruppe bestehend aus F, Cl, Br, -OH, -SH, -NH₂, -CN und NO₂ substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Der Fachmann versteht, dass sich für m gleich 0 die folgende allgemeine Formel la ergibt: 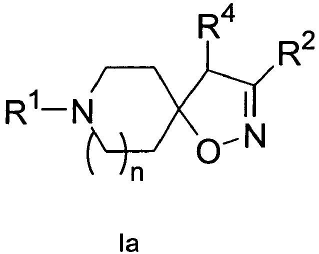 Ebenfalls bevorzugt sind substituierte Spiro-Verbindungen der vorstehend angegebenen allgemeinen Formel I, worin
n gleich 0, 1 oder 2 ist;
und jeweils m und R¹ bis R³⁶ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Der Fachmann versteht, dass sich für n gleich 1 die folgende allgemeine Formel Ib ergibt:

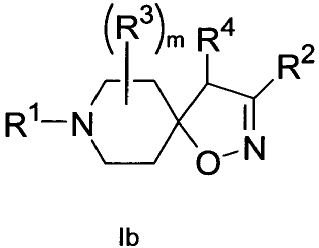

Besonders bevorzugt sind substituierte Spiro-Verbindungen der vorstehend angegebenen allgemeinen Formel I, worin
- m: gleich 0, 1, 2, 3 oder 4 ist;
- n: gleich 0, 1 oder 2 ist;
- R¹: für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl steht;
für einen Rest ausgewählt aus der Gruppe bestehend aus Indolyl, Isoindolyl, Benzo[b]furanyl, Benzo[b]thiophenyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, - C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, - C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -NH-S(=O)₂-CH₃, -NH-S(=O)₂-C₂H₅, -S(=O)₂-NH-CH₃, -S(=O)₂-NH-C₂H₅ substituiert sein kann;
für eine -C(=O)-NR⁵R⁶-Gruppe steht;
für eine -C(=S)-NR⁷R⁸-Gruppe steht;
für eine -C(=O)-R⁹-Gruppe,
für eine -S(=O)₂-R¹⁰-Gruppe,
oder für -(CHR¹³)-R¹⁶; -(CHR¹³)-(CHR¹⁴)-R¹⁶ oder -(CHR¹³)-(CHR¹⁴)-(CHR¹⁵)-R¹⁶ steht;
- R²: für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl steht;
für einen Phenyl-Rest, der mit 1, 2, 3, 4 oder 5 Substituenten unabhängig voneinander ausgewählt aus der Gruppe bestehend aus F, Cl, Br, I, -CN, - CF₃, -SF₅, -OH, -O-CH₃, -O-C₂H₅, -NH₂, -NO₂, -O-CF₃, -S-CF₃, -SH, -S-CH₃, - S-C₂H₅, Methyl, Ethyl, n-Propyl, Isopropyl, n-Butyl, sec-Butyl, Isobutyl, tert-Butyl, Cyclopentyl, Cyclohexyl, -S(=O)₂-CH₃, -S(=O)₂-C₂H₅, -NH-S(=O)₂-CH₃, - NH-S(=O)₂-C₂H₅, -NH-S(=O)₂-Phenyl, -S(=O)₂-NH-CH₃ und -S(=O)₂-NH-C₂H₅ substituiert sein kann;
mit der Maßgabe, dass nicht eine der meta-Positionen und die para-Position dieses Phenyl-Restes mit Substituenten substituiert sind, die jeweils über ein gleiches Atom ausgewählt aus der Gruppe bestehend aus Sauerstoff, Schwefel und Stickstoff an den Phenyl-Rest gebunden sind;
für einen Rest ausgewählt aus der Gruppe bestehend aus Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, [1,2,3,4]-Tetrahydronaphthyl, [1,2,3,4]-Tetrahydrochinolinyl, [1,2,3,4]-Tetrahydroisochinolinyl, 2H-Benzo[1.4]oxazin-3(4H)-onyl, (3,4)-Dihydrochinolin-2(1H)-onyl, [1,2,3,4]-Tetrahydrochinazolinyl, [3,4]-Dihydro-2H-1,4-benzoxazinyl und Benzothiazolyl steht, , wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -S(=O)₂-CH₃, -S(=O)₂-C₂H₅, - NH-S(=O)₂-CH₃, -NH-S(=O)₂-C₂H₅, -S(=O)₂-NH-CH₃ und -S(=O)₂-NH-C₂H₅ substituiert sein kann;
oder für -(CHR¹⁷)-R²⁰, -(CHR¹⁷)-(CHR¹⁸)-R²⁰ oder -(CHR¹⁷)-(CHR¹⁸)-(CHR¹⁹)-R²⁰ steht;
- R³: für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht;
- R⁴: für einen Wasserstoff-Rest steht
oder für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht;
- R⁵ und R⁷,: unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl, 2-Methyl-1-propenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen; wobei der Rest ggf. jeweils mit 1, 2 oder 3 Substituenten unabhängig voneinander ausgewählt aus der Gruppe bestehend aus Methyl, Ethyl, n-Propyl, Isopropyl, n-Butyl, sec-Butyl, Isobutyl und tert-Butyl substituiert sein kann;
für einen Rest ausgewählt aus der Gruppe bestehend aus Phenyl, (1,3)-Benzodioxolyl, (1,4)-Benzodioxanyl, Naphthyl, Thiophenyl, Furanyl, Pyrrolyl, Pyrazolyl, Pyranyl, Pyridinyl, Imidazolyl, Thiazolyl, Oxazolyl, Isoxazolyl, Pyridazinyl, Pyrazinyl und Pyrimidinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, - C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, - C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -0-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, - S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, Methyl, Ethyl, n-Propyl, Isopropyl, n-Butyl, sec-Butyl, Isobutyl, tert-Butyl, - O-CH₃, -O-C₂H₅, -O-CF₃, -S-CF₃, Phenyl und -O-Benzyl substituiert sein kann;
oder für -(CR²¹R²²)-R²⁵, -(CR²¹R²²)-(CHR²³)-R²⁵, -(CR²¹R²²)-(CHR²³)-O-R²⁵, - (CR²¹R²²)-(CHR²³)-(CHR²⁴)-R²⁵, -(CR²¹R²²)-(CHR²³)-(CHR²⁴)-O-R²⁵, - (CR²¹R²²)-(CHR²³)-(CHR²⁴)-N(CH₃)-R²⁵ oder -(CR²¹R²²)-(CHR²³)-(CHR²⁴)-N(C₂H₅)-R²⁵ stehen;
- R⁶ und R⁸: jeweils für einen Wasserstoff-Rest stehen;
oder für einen Rest ausgewählt aus der Gruppe bestehend aus Methyl, Ethyl, n-Propyl, Isopropyl, n-Butyl, sec-Butyl, Isobutyl und tert-Butyl;
- R⁹: für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -O-CF₃, -S-CF₃, -SF₅, -OH, -O-CH₃, -O-C₂H₅, -NH-S(=O)₂-CH₃, Morpholinyl, Piperidinyl, Pyrrolidinyl, Piperazinyl, Phenyl, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht; und
- R¹⁰: für einen Rest ausgewählt aus der Gruppe bestehend aus 9H-Fluorenyl, 9H-Xanthenyl, Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -O-CF₃, -S-CF₃, -SF₅, -OH, -O-CH₃, -O-C₂H₅, -NH-S(=O)₂-CH₃, Morpholinyl, Piperidinyl, Pyrrolidinyl, Piperazinyl, Phenyl, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht;
- R¹³, R¹⁴, R¹⁵, R¹⁷, R¹⁸, R¹⁹, R²¹, R²², R²³, R²⁴, R²⁸, R²⁹ und R³¹,: unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, Methyl, Ethyl, n-Propyl, Isopropyl, n-Butyl, sec-Butyl, Isobutyl und tert-Butyl substituiert sein kann;
- R¹⁶: für einen Rest ausgewählt aus der Gruppe bestehend aus Phenyl, Naphthyl, Thiophenyl, Furanyl, Pyrrolyl, Pyrazolyl, Pyrazinyl und Pyridinyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH,-S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -0-C(=O)-CH₃, -O-C(=O)-C₂H₅, -O-C(=O)-C(CH₃)₃, -S(=O)₂-CH₃, -S(=O)₂-C₂H₅,-NH-S(=O)₂-CH₃, -NH-S(=O)₂-C₂H₅, -S(=O)₂-NH-CH₃ und -S(=O)₂-NH-C₂H₅ substituiert sein kann;
- R²⁰: für einen Rest ausgewählt aus der Gruppe bestehend aus Phenyl und Naphthyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH,-S-CH₃, -S-C₂H₅, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
- R²⁵: für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH,-S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -0-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅ und -C(=O)-C(CH₃)₃ substituiert sein kann;
- R²⁶ und R²⁷,: unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, Methyl, Ethyl, n-Propyl, Isopropyl, n-Butyl, sec-Butyl, Isobutyl und tert-Butyl substituiert sein kann;
oder für -OH stehen;
- R³²: für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Phenyl, Naphthyl, Furanyl und Thiophenyl steht, der ggf. mit 1, 2, 3, 4 oder 5 Substituenten unabhängig voneinander ausgewählt aus der Gruppe bestehend aus -SF₅, F, Cl, Br, I, -CF₃, -O-CF₃, -S-CF₃, -O-CH₃, -O-C₂H₅, Phenyl, -S-CH₃, -S-C₂H₅, Cyclopentyl, Cyclohexyl, Methyl, Ethyl, n-Propyl, Isopropyl, n-Butyl, sec-Butyl, Isobutyl und tert-Butyl substituiert sein kann;
für -(CH₂)_{cc}-R³⁵ mit cc = 1, 2 oder 3 steht oder für -CH=CH-R³⁶ steht;
- R³⁰, R³³ und R³⁴,: unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Phenyl, Pyridinyl und Naphthyl stehen, wobei der Rest ggf. jeweils mit 1, 2, 3, 4 oder 5 Substituenten unabhängig voneinander ausgewählt aus der Gruppe bestehend aus -SF₅, F, Cl, Br, -CF₃, -O-CF₃, -S-CF₃, Phenyl, Morpholinyl, Piperidinyl, Pyrrolidinyl, Piperazinyl, Cyclopentyl, Cyclohexyl, -OH, -O-CH₃,-O-C₂H₅, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
und
- R³⁵ und R³⁶,: unabhängig voneinander, jeweils
für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, Methyl, Ethyl, n-Propyl, Isopropyl, n-Butyl, sec-Butyl, Isobutyl und tert-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind substituierte Spiro-Verbindungen der vorstehend angegebenen allgemeinen Formel I, worin
- m: gleich 0, 1 oder 2 ist;
- n: gleich 0, 1 oder 2 ist;
- R¹: für einen Rest ausgewählt aus der Gruppe bestehend aus Benzimidazolyl, Benzoxazolyl, Benzotriazolyl, Benzisoxazolyl und Benzothiazolyl steht, wobei der Rest jeweils mit 1, 2, 3, 4 oder 5 Substituenten unabhängig voneinander ausgewählt aus der Gruppe bestehend F, Cl, Br, -CF₃, -O-CF₃, -S-CF₃, Methyl, Ethyl, n-Propyl, Isopropyl, n-Butyl, sec-Butyl, Isobutyl und tert-Butyl substituiert sein kann;
für eine -C(=O)-NR⁵R⁶-Gruppe steht;
für eine -C(=S)-NR⁷R⁸-Gruppe steht;
für eine -C(=O)-R⁹-Gruppe steht
oder für eine -S(=O)₂-R¹⁰-Gruppe steht;
- R²: für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht;
für einen Phenyl-Rest der allgemeinen Formel XX 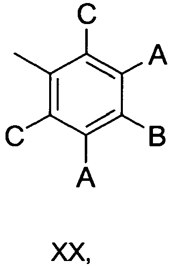 steht,
worin die frei Linie die Bindung dieses Phenyl-Restes zur Spiro-Verbindung der allgemeinen Formel I darstellt;
A und B jeweils für einen Substituenten unabhängig voneinander ausgewählt aus der Gruppe bestehend aus H, F, Cl, Br, I, -CF₃, -SF₅, -OH, -O-CH₃, -O-C₂H₅, -O-CF₃, -S-CF₃, Methyl, Ethyl, n-Propyl, Isopropyl, n-Butyl, sec-Butyl, Isobutyl, tert-Butyl, Cyclopentyl, Cyclohexyl, -NH-S(=O)₂-CH₃ und -NH-S(=O)₂-Phenyl stehen;
mit der Maßgabe, dass nicht eine der Positionen A und die Position B dieses Phenyl-Restes mit Substituenten substituiert sind, die jeweils über ein gleiches Atom ausgewählt aus der Gruppe bestehend aus Sauerstoff, Schwefel und Stickstoff an den Phenyl-Rest gebunden sind;
C jeweils für H steht;
für einen Rest ausgewählt aus der Gruppe bestehend aus Naphthyl, Chinolinyl, (1,4)-Benzodioxanyl, (1,3)-Benzodioxolyl, Pyridinyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, I, -O-CH₃, -O-C₂H₅, Methyl, Ethyl, n-Propyl, Isopropyl, n-Butyl, sec-Butyl, Isobutyl, tert-Butyl, -S(=O)₂-CH₃, -S(=O)₂-C₂H₅, -NH-S(=O)₂-CH₃, -NH-S(=O)₂-C₂H₅, -S(=O)₂-NH-CH₃ und -S(=O)₂-NH-C₂H₅ substituiert sein kann;
oder für -(CHR¹⁷)-R²⁰, -(CHR¹⁷)-(CHR¹⁸)-R²⁰ oder -(CHR¹⁷)-(CHR¹⁸)-(CHR¹⁹)-R²⁰ steht;
- R³: für einen Rest ausgewählt aus der Gruppe bestehend aus Methyl, Ethyl, n-Propyl, Isopropyl, n-Butyl, sec-Butyl, Isobutyl, tert-Butyl und n-Pentyl steht;
- R⁴: für einen Wasserstoff-Rest steht;
- R⁵ und R⁷,: unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl und 2-Methyl-1-propenyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl und Adamantyl stehen; wobei der Rest ggf. jeweils mit 1, 2 oder 3 Substituenten unabhängig voneinander ausgewählt aus der Gruppe bestehend aus Methyl, Ethyl, n-Propyl, Isopropyl, n-Butyl, sec-Butyl, Isobutyl und tert-Butyl substituiert sein kann;
für einen Rest ausgewählt aus der Gruppe bestehend aus Phenyl, Naphthyl, (1,4)-Benzodioxanyl, und Pyridinyl stehen, wobei der Rest ggf. jeweils mit 1, 2, 3, 4 oder 5 Substituenten unabhängig voneinander ausgewählt aus der Gruppe bestehend aus -SF₅, F, Cl, Br, I, -CN, -CF₃, -O-CH₃, -O-C₂H₅, -NO₂,-O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann, wobei jeweils der zyklische Teil der Reste Cyclopentyl, Cyclohexyl, -0-Phenyl, -O-Benzyl und Phenyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl und tert-Butyl substituiert sein kann;
oder für -(CR²¹R²²)-R²⁵, -(CR²¹R²²)-(CHR²³)-R²⁵, -(CR²¹R²²)-(CHR²³)-O-R²⁵,-(CR²¹R²²)-(CHR²³)-(CHR²⁴)-R²⁵, -(CR²¹R²²)-(CHR²³)-(CHR²⁴)-O-R²⁵,-(CR²¹R²²)-(CHR²³)-(CHR²⁴)-N(CH₃)-R²⁵ oder -(CR²¹R²²)-(CHR²³)-(CHR²⁴)-N(C₂H₅)-R²⁵ stehen;
- R⁶ und R⁸: jeweils für einen Wasserstoff-Rest stehen;
oder für einen Methyl- oder Ethyl-Rest stehen;
- R⁹: für einen Rest ausgewählt aus der Gruppe bestehend aus Phenyl, Pyridinyl und Naphthyl steht, wobei der Rest ggf. jeweils mit 1, 2, 3, 4 oder 5 Substituenten unabhängig voneinander ausgewählt aus der Gruppe bestehend aus F, Cl, Br, I, -CF₃, -O-CF₃, -S-CF₃, -OH, -O-CH₃, -0-C₂H₅, -NH-S(=O)₂-CH₃, Morpholinyl, Piperidinyl, Pyrrolidinyl, Piperazinyl, Phenyl, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht;
- R¹⁰: für einen Rest ausgewählt aus der Gruppe bestehend aus Phenyl und Naphthyl steht, wobei der Rest ggf. jeweils mit 1, 2, 3, 4 oder 5 Substituenten unabhängig voneinander ausgewählt aus der Gruppe bestehend aus F, Cl, Br, I, -CF₃, -OH, -O-CH₃, -O-C₂H₅, Methyl, Ethyl, n-Propyl, Isopropyl, n-Butyl, sec-Butyl, Isobutyl und tert-Butyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht;
- R¹⁷, R¹⁸, R¹⁹, R²³, R²⁴, R²⁸ und R²⁹,: unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen;
- R²⁰: für einen Phenyl-Rest steht;
- R²¹ und R²²,: unabhängig voneinander, jeweils für
einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen;
- R²⁵: für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopentyl, Cyclohexyl, Tetrahydrofuranyl, Tetrahydrothiophenyl, Pyrrolidinyl, Morpholinyl, Piperidinyl und Piperazinyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, -OH, -O-CH₃, -O-C₂H₅, Methyl, Ethyl, n-Propyl, Isopropyl, n-Butyl, sec-Butyl, Isobutyl und tert-Butyl substituiert sein kann;
- R²⁶ und R²⁷,: unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
für einen Phenyl-Rest stehen oder für -OH stehen;
- R³⁰: für einen Phenyl-Rest steht, der mit 1, 2, 3, 4 oder 5 Substituenten unabhängig voneinander ausgewählt aus der Gruppe bestehend aus F, Cl, Br, -NH-S(=O)₂-CH₃, Methyl, Ethyl, n-Propyl, Isopropyl, n-Butyl, sec-Butyl, Isobutyl und tert-Butyl substituiert sein kann;
- R³¹: für einen Wasserstoff-Rest steht;
oder für einen Rest ausgewählt aus der Gruppe bestehend aus Methyl, Ethyl, n-Propyl, Isopropyl, n-Butyl, sec-Butyl, Isobutyl und tert-Butyl steht;
- R³²: für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Phenyl, Naphthyl, Furanyl und Thiophenyl steht, der ggf. mit 1, 2, 3, 4 oder 5 Substituenten unabhängig voneinander ausgewählt aus der Gruppe bestehend aus -SF₅, F, Cl, Br, I, -CF₃, -O-CF₃, -S-CF₃, -O-CH₃, -O-C₂H₅, Phenyl, -S-CH₃, -S-C₂H₅, Cyclopentyl, Cyclohexyl, Methyl, Ethyl, n-Propyl, Isopropyl, n-Butyl, sec-Butyl, Isobutyl und tert-Butyl substituiert sein kann;
für -CH₂-R³⁵ oder für -CH=CH-R³⁶ steht;
- R³³ und R³⁴,: unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Phenyl, Pyridinyl und Naphthyl stehen, wobei der Rest ggf. jeweils mit 1, 2, 3, 4 oder 5 Substituenten unabhängig voneinander ausgewählt aus der Gruppe bestehend aus -SF₅, F, Cl, Br, -CF₃, -O-CF₃, -S-CF₃, Phenyl, Morpholinyl, Piperidinyl, Pyrrolidinyl, Piperazinyl, Cyclopentyl, Cyclohexyl, -OH, -O-CH₃,-O-C₂H₅, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
und
- R³⁵ und R³⁶: jeweils für einen Phenyl-Rest stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls ganz besonders bevorzugt sind substituierte Spiro-Verbindungen der vorstehend angegebenen allgemeinen Formel I, worin
- m: gleich 0, 1 oder 2 ist;
- n: gleich 0, 1 oder 2 ist;
- R¹: für einen Rest ausgewählt aus der Gruppe bestehend aus Benzimidazolyl, Benzoxazolyl, Benzotriazolyl, Benzisoxazolyl und Benzothiazolyl steht, wobei der Rest jeweils mit 1, 2, 3, 4 oder 5 Substituenten unabhängig voneinander ausgewählt aus der Gruppe bestehend F, Cl, Br, -CF₃, -O-CF₃, -S-CF₃, Methyl, Ethyl, n-Propyl, Isopropyl, n-Butyl, sec-Butyl, Isobutyl und tert-Butyl substituiert sein kann;
für eine -C(=O)-NR⁵R⁶-Gruppe steht;
für eine -C(=S)-NR⁷R⁸-Gruppe steht;
für eine -C(=O)-R⁹-Gruppe steht
oder für eine -S(=O)₂-R¹⁰-Gruppe steht;
- R²: für einen tert-Butyl-Rest steht;
für einen Rest ausgewählt aus der Gruppe bestehend aus Phenyl, 2-Methansulfonamid-phenyl, 2-Ethansulfonamid-phenyl, 2-Trifluoromethylphenyl, 2-Trifluoromethylsulfanyl-phenyl, 2-Ethyl-phenyl, 2-tert-Butyl-phenyl, 2-Ethylamino-sulfonyl-phenyl, 2-Methylamino-sulfonyl-phenyl, 2-Bromo-phenyl, 2-Chloro-phenyl, 2-Fluoro-phenyl, 2-Methyl-phenyl, 2-Trifluoromethoxy-phenyl, 2-Methoxy-phenyl, 2-Ethoxy-phenyl, 2-Propyl-phenyl, 2-lodo-phenyl, 3-Chloro-phenyl, 3-Methyl-phenyl, 3-tert-Butyl-phenyl, 3-Trifluoromethylsulfanyl-phenyl, 3-Trifluoromethyl-phenyl, 3-Methansulfonamid-phenyl, 3-Ethansulfonamid-phenyl, 3-Fluoro-phenyl, 3-Propyl-phenyl, 3-Isopropyl-phenyl, 3-Bromo-phenyl, 3-Methoxy-phenyl, 3-Ethyl-phenyl, 3-Ethylamino-sulfonyl-phenyl, 3-Methylamino-sulfonyl-phenyl, 3-Ethoxy-phenyl, 3-Trifluoromethoxy-phenyl, 3-lodophenyl, 4-Methylamino-sulfonyl-phenyl, 4-Ethylamino-sulfonyl-phenyl, 4-Methansulfonamid-phenyl, 4-Ethansulfonamid-phenyl, 4-Bromo-phenyl, 4-Methoxy-phenyl, 4-Chloro-phenyl, 4-Fluoro-phenyl, 4-tert-Butyl-phenyl, 4-Trifluoromethylsulfanyl-phenyl, 4-Methyl-phenyl, 4-Isopropyl-phenyl, 4-Trifluoromethyl-phenyl, 4-Propyl-phenyl, 4-lodo-phenyl, 4-Trifluoromethoxyphenyl, 4-Ethyl-phenyl, 4-Ethoxy-phenyl, 2-Fluoro-3-trifluoromethylphenyl, (2,3)-Difluorophenyl, (2,3)-Dimethyl-phenyl, (2,3)-Dichlorophenyl, 3-Fluoro-2-trifluoromethylphenyl, (2,4)-Dichloro-phenyl, (2,4)-Difluorophenyl, 4-Fluoro-2-trifluoromethyl-phenyl, (2,4)-Dimethoxyphenyl, 2-Chloro-4-fluoro-phenyl, (2,4)-Dibromo-phenyl, 2-Fluoro-4-trifluoromethyl-phenyl, (2,5)-Difluoro-phenyl, 2-Fluoro-5-trifluoromethyl-phenyl, 5-Fluoro-2-trifluoromethyl-phenyl, 5-Chloro-2-trifluoromethyl-phenyl, 5-Bromo-2-trifluoromethyl-phenyl, (2,5)-Dimethoxyphenyl, (2,5)-Bis-trifluoromethyl-phenyl, (2,5)-Dichloro-phenyl, (2,5)-Dibromo-phenyl, 2-Fluor-6-trifluoromethyl-phenyl, (2,6)-Dimethoxy-phenyl, (2,6)-Dimethyl-phenyl, (2,6)-Dichloro-phenyl, 2-Chloro-6-fluoro-phenyl, 2-Bromo-6-chloro-phenyl, 2-Bromo-6-fluoro-phenyl, (2,6)-Difluoro-phenyl, (2,6)-Difluoro-3-methyl-phenyl, (2,6)-Dibromo-phenyl, (2,6)-Dichlorophenyl, 3-Chloro-2-fluoro-phenyl, (3,4)-Dichlorophenyl, 4-Fluoro-3-trifluoromethylphenyl, 3-Fluoro-4-trifluoromethyl-phenyl, (3,4)-Difluoro-phenyl, 4-Chloro-3-trifluoromethyl, 4-Bromo-3-methyl-phenyl, 4-Bromo-5-methyl-phenyl, 3-Chloro-4-fluoro-phenyl, (3,4)-Dibromo-phenyl, 4-Chlor-3-methyl-phenyl, 4-Bromo-3-methyl-phenyl, 4-Fluoro-3-methyl-phenyl, (3,5)-Dimethoxy-phenyl, (3,5)-Bis-trifluoromethyl-phenyl, (3,5)-Difluoro-phenyl, (3,5)-Dichloro-phenyl, 3-Fluoro-5-trifluoromethyl-phenyl, 5-Fluoro-3-trifluoromethyl-phenyl, (3,5)-Dibromo-phenyl, 5-Chloro-4-fluoro-phenyl, 5-Bromo-4-methyl-phenyl, (2,3,4)-Trifluorophenyl, (2,3,4)-Trichlorophenyl, (2,3,6)-Trifluoro-phenyl, 5-Chloro-2-methoxy-phenyl, (2,3)-Difluoro-4-methyl-phenyl, (2,4,5)-Trifluoro-phenyl, (2,4,5)-Trichloro-phenyl, (2,4)-Dichloro-5-fluoro-phenyl, (2,4,6)-Trichloro-phenyl, (2,4,6)-Trimethylphenyl, (2,4,6)-Trifluoro-phenyl, (2,4,6)-Trimethoxy-phenyl, (2,3,4,5)-Tetrafluoro-phenyl, 4-Methoxy-2,3,6-trimethyl-phenyl, 4-Methoxy-2,3,6-trimethyl-phenyl, 4-Chloro-2,5-dimethyl-phenyl, 2-Chloro-6-fluoro-3-methyl-phenyl, 6-Chloro-2-fluoro-3-methyl, (2,3,4,5,6)-Pentafluoro-phenyl, 3-Fluoro-4-methylsulfonamido-phenyl, 3-Chlor-4-methylsulfonamido-phenyl, 3-Brom-4-methylsulfonamido-phenyl, 3-Methoxy-4-methylsulfonamido-phenyl, 3-Hydroxy-4-methylsulfonamido-phenyl, 3-Trifluoromethyl-4-methylsulfonamido-phenyl, 3-Trifluoromethoxy-4-methylsulfonamido-phenyl, 3-Methyl-4-methylsulfonamido-phenyl, 3-Ethyl-4-methylsulfonamido-phenyl, 3-Isopropyl-4-methylsulfonamido-phenyl, 3-Propyl-4-methylsulfonamido-phenyl, 3-tert-Butyl-4-methylsulfonamido-phenyl, 3-Fluoro-4-phenylsulfonamido-phenyl, 3-Chlor-4-phenylsulfonamido-phenyl, 3-Brom-4-phenylsulfonamido-phenyl, 3-Methoxy-4-phenylsulfonamido-phenyl, 3-Hydroxy-4-phenylsulfonamido-phenyl, 3-Trifluoromethyl-4-phenylsulfonamido-phenyl, 3-Trifluoromethoxy-4-phenylsulfonamido-phenyl, 3-Methyl-4-phenylsulfonamido-phenyl, 3-Ethyl-4-phenylsulfonamido-phenyl, 3-Isopropyl-4-phenylsulfonamido-phenyl, 3-Propyl-4-phenylsulfonamido-phenyl, 3-tert-Butyl-4-phenylsulfonamido-phenyl, 4-Fluoro-3-methylsulfonamido-phenyl, 4-Chlor-3-methylsulfonamido-phenyl, 4-Brom-3-methylsulfonamido-phenyl, 4-Methoxy-3-methylsulfonamido-phenyl, 4-Hydroxy-3-methylsulfonamido-phenyl, 4-Trifluoromethyl-3-methylsulfonamido-phenyl, 4-Trifluoromethoxy-3-methylsulfonamido-phenyl, 4-Methyl-3-methylsulfonamido-phenyl, 4-Ethyl-3-methylsulfonamido-phenyl, 4-Isopropyl-3-methylsulfonamido-phenyl, 4-Propyl-3-methylsulfonamido-phenyl, 4-tert-Butyl-3-methylsulfonamido-phenyl, 4-Fluoro-3-phenylsulfonamido-phenyl, 4-Chlor-3-phenylsulfonamido-phenyl, 4-Brom-3-phenylsulfonamido-phenyl, 4-Methoxy-3-phenylsulfonamido-phenyl, 4-Hydroxy-3-phenylsulfonamido-phenyl, 4-Trifluoromethyl-3-phenylsulfonamido-phenyl, 4-Trifluoromethoxy-3-phenylsulfonamido-phenyl, 4-Methyl-3-phenylsulfonamido-phenyl, 4-Ethyl-3-phenylsulfonamido-phenyl, 4-Isopropyl-3-phenylsulfonamido-phenyl, 4-Propyl-3-phenylsulfonamido-phenyl, 4-tert-Butyl-3-phenylsulfonamido-phenyl, 2-Cyclohexyl-phenyl, 3-Cyclohexyl-phenyl und 4-Cyclohexyl-phenyl steht;
für einen Rest ausgewählt aus der Gruppe bestehend aus Chinolinyl, (1,4)-Benzodioxanyl, (1,3)-Benzodioxolyl, Naphthyl und Thiazolyl steht;
für einen Pyridinyl-Rest steht, wobei der Rest jeweils mit 1 oder 2 Substituenten unabhängig voneinander ausgewählt aus der Gruppe bestehend aus F, Cl und Br substituiert sein kann;
für -(CHR¹⁷)-R²⁰ oder -(CHR¹⁷)-(CHR¹⁸)-R²⁰ steht;
- R³: für einen Methyl- oder Ethyl-Rest steht;
- R⁴: für einen Wasserstoff-Rest steht;
- R⁵ und R⁷,: unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl und 2-Methyl-1-propenyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl und Adamantyl stehen; wobei der Rest ggf. jeweils mit 1, 2 oder 3 Substituenten unabhängig voneinander ausgewählt aus der Gruppe bestehend aus Methyl, Ethyl, n-Propyl, Isopropyl, n-Butyl, sec-Butyl, Isobutyl und tert-Butyl substituiert sein kann;
für einen Rest ausgewählt aus der Gruppe bestehend aus Phenyl, Naphthyl, (1,4)-Benzodioxanyl und Pyridinyl stehen, wobei der Rest ggf. jeweils mit 1, 2, 3, 4 oder 5 Substituenten unabhängig voneinander ausgewählt aus der Gruppe bestehend aus -SF₅, F, Cl, Br, I, -CN, -CF₃, -O-CH₃, -O-C₂H₅, -NO₂,-O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann, wobei jeweils der zyklische Teil der Reste Cyclopentyl, Cyclohexyl, -0-Phenyl, -O-Benzyl und Phenyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl und tert-Butyl substituiert sein kann;
oder für -(CR²¹R²²)-R²⁵, -(CR²¹R²²)-(CHR²³)-R²⁵, -(CR²¹R²²)-(CHR²³)-O-R²⁵,-(CR²¹R²²)-(CHR²³)-(CHR²⁴)-R²⁵, -(CR²¹R²²)-(CHR²³)-(CHR²⁴)-O-R²⁵,-(CR²¹R²²)-(CHR²³)-(CHR²⁴)-N(CH₃)-R²⁵ oder -(CR²¹R²²)-(CHR²³)-(CHR²⁴)-N(C₂H₅)-R²⁵ stehen;
- R⁶ und R⁸: jeweils für einen Wasserstoff-Rest stehen;
oder für einen Methyl- oder Ethyl-Rest stehen;
- R⁹: für einen Rest ausgewählt aus der Gruppe bestehend aus Phenyl, Pyridinyl und Naphthyl steht, wobei der Rest ggf. jeweils mit 1, 2, 3, 4 oder 5 Substituenten unabhängig voneinander ausgewählt aus der Gruppe bestehend aus F, Cl, Br, I, -CF₃, -O-CF₃, -S-CF₃, -OH, -O-CH₃, -O-C₂H₅, -NH-S(=O)₂-CH₃, Morpholinyl, Piperidinyl, Pyrrolidinyl, Piperazinyl, Phenyl, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht;
- R¹⁰: für einen Rest ausgewählt aus der Gruppe bestehend aus Phenyl und Naphthyl steht, wobei der Rest ggf. jeweils mit 1, 2, 3, 4 oder 5 Substituenten unabhängig voneinander ausgewählt aus der Gruppe bestehend aus F, Cl, Br, I, -CF₃, -OH, -O-CH₃, -O-C₂H₅, Methyl, Ethyl, n-Propyl, Isopropyl, n-Butyl, sec-Butyl, Isobutyl und tert-Butyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht;
- R¹⁷, R¹⁸, R¹⁹, R²³, R²⁴, R²⁸ und R²⁹,: unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen;
- R²⁰: für einen Phenyl-Rest steht;
- R²¹ und R²²,: unabhängig voneinander, jeweils für
einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen;
- R²⁵: für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopentyl, Cyclohexyl, Tetrahydrofuranyl, Tetrahydrothiophenyl, Pyrrolidinyl, Morpholinyl, Piperidinyl und Piperazinyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, -OH, -O-CH₃, -O-C₂H₅, Methyl, Ethyl, n-Propyl, Isopropyl, n-Butyl, sec-Butyl, Isobutyl und tert-Butyl substituiert sein kann;
- R²⁶ und R²⁷,: unabhängig voneinander, jeweils
für einen Wasserstoff-Rest
für einen Rest ausgewählt aus der Gruppe bestehend aus Methyl, Ethyl, n-Propyl, Isopropyl, n-Butyl, sec-Butyl, Isobutyl und tert-Butyl stehen;
für einen Phenyl-Rest stehen
oder für -OH stehen;
- R³⁰: für einen Phenyl-Rest steht, der mit 1, 2, 3, 4 oder 5 Substituenten unabhängig voneinander ausgewählt aus der Gruppe bestehend aus F, Cl, Br, -NH-S(=O)₂-CH₃, Methyl, Ethyl, n-Propyl, Isopropyl, n-Butyl, sec-Butyl, Isobutyl und tert-Butyl substituiert sein kann;
- R³¹: für einen Wasserstoff-Rest steht;
oder für einen Rest ausgewählt aus der Gruppe bestehend aus Methyl, Ethyl, n-Propyl, Isopropyl, n-Butyl, sec-Butyl, Isobutyl und tert-Butyl steht;
- R³²: für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Phenyl, Naphthyl, Furanyl und Thiophenyl steht, der ggf. mit 1, 2, 3, 4 oder 5 Substituenten unabhängig voneinander ausgewählt aus der Gruppe bestehend aus -SF₅, F, Cl, Br, I, -CF₃, -O-CF₃, -S-CF₃, -O-CH₃, -O-C₂H₅, Phenyl, -S-CH₃, -S-C₂H₅, Cyclopentyl, Cyclohexyl, Methyl, Ethyl, n-Propyl, Isopropyl, n-Butyl, sec-Butyl, Isobutyl und tert-Butyl substituiert sein kann;
für -CH₂-R³⁵ oder für -CH=CH-R³⁶ steht;
- R³³ und R³⁴,: unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Phenyl, Pyridinyl und Naphthyl stehen, wobei der Rest ggf. jeweils mit 1, 2, 3, 4 oder 5 Substituenten unabhängig voneinander ausgewählt aus der Gruppe bestehend aus -SF₅, F, Cl, Br, -CF₃, -O-CF₃, -S-CF₃, Phenyl, Morpholinyl, Piperidinyl, Pyrrolidinyl, Piperazinyl, Cyclopentyl, Cyclohexyl, -OH, -O-CH₃,-O-C₂H₅, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
und
- R³⁵ und R³⁶: jeweils für einen Phenyl-Rest stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ferner können erfindungsgemäße substituierte Spiro-Verbindungen der allgemeinen Formel I bevorzugt sein, die im FLIPR-Assay in einer Konzentration von 10 µM eine Hemmung des Ca²⁺-Ionen-Einstroms in Dorsalwurzelganglien von Ratten von wenigstens 30 %, bevorzugt von wenigstens 40 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µM aufweisen.

Dabei wird Im FLIPR-Assay der Ca²⁺-Einstrom mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Ein weiterer Gegenstand der vorliegenden Erfindung ist ein Verfahren zur Herstellung von erfindungsgemäßen Verbindungen der vorstehend angegebenen allgemeinen Formel I gemäß dem wenigstens eine Verbindung der allgemeinen Formel II, 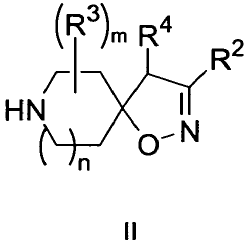 worin R², R³, R⁴, m und n die vorstehend angegebene Bedeutung haben, in einem Reaktionsmedium mit wenigstens einem Isocyanat der allgemeinen Formel R⁵-N=C=0, worin R⁵ die vorstehend angegebene Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin, Diisopropylethylamin, Pyridin und N-Methylmorpholin, zu wenigstens einer Verbindung der allgemeinen Formel I umgesetzt wird, worin R², R³, R⁴, m und n die vorstehend genannte Bedeutung haben und R¹ für -C(=O)-NR⁵R⁶ steht, wobei R⁵ die vorstehend genannte Bedeutung hat und R⁶ für einen Wasserstoff-Rest steht, und diese ggf. gereinigt und/oder isoliert wird
oder
wenigstens eine Verbindung der allgemeinen Formel II in einem Reaktionsmedium mit wenigstens einem Isothiocyanat der allgemeinen Formel S=C=N-R⁷, worin R⁷ die vorstehend genannte Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin, Diisopropylethylamin, Pyridin und N-Methylmorpholin, zu wenigstens einer Verbindung der allgemeinen Formel I umgesetzt wird, worin R², R³, R⁴, m und n die vorstehend genannte Bedeutung haben und R¹ für -C(=S)-N-R⁷R⁸ steht, wobei R⁷ die vorstehend genannte Bedeutung hat und R⁸ für einen Wasserstoff-Rest steht, und diese ggf. gereinigt und/oder isoliert wird
und ggf. wenigstens eine Verbindung der allgemeinen Formel I, worin R², R³, R⁴, m und n die vorstehend genannte Bedeutung haben und R¹ für -C(=O)-NR⁵R⁶ oder-C(=S)-N-R⁷R⁸ steht, worin R⁶ und R⁸ jeweils für einen Wasserstoff-Rest stehen, in einem Reaktionsmedium, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R⁶ oder der allgemeinen Formel LG-R⁸, worin LG für eine Abgangsgruppe, bevorzugt für ein Halogen-Atom, besonders bevorzugt für ein Chloratom steht, und R⁶ und R⁸ die vorstehend genannte Bedeutung mit Ausnahme von Wasserstoff haben, zu wenigstens einer Verbindung der allgemeinen Formel I umgesetzt wird, worin R² bis R⁴, m und n die vorstehend genannte Bedeutung haben und R¹ für -C(=O)-NR⁵R⁶ oder -C(=S)-N-R⁷R⁸ steht, und diese ggf. gereinigt und/oder isoliert wird,
oder
wenigstens eine Verbindung der allgemeinen Formel II in einem Reaktionsmedium, in Gegenwart wenigstens einer Base, bevorzugt in Gegenwart wenigstens eines Metallhydridsalzes, besonders bevorzugt in Gegenwart von Natrium- und/oder Kaliumhydrid, mit wenigstens einer Verbindung der allgemeinen Formel LG-R¹, worin R¹ die vorstehend genannte Bedeutung hat, mit Ausnahme von -C(=O)-NR⁵R⁶,-C(=S)-NR⁷R⁸, -C(=O)-R⁹ und -S(=O)-R¹⁰, und LG für eine Abgangsgruppe, bevorzugt für ein Halogen-Atom, besonders bevorzugt für ein Chloratom steht, zu wenigstens einer Verbindung der allgemeinen Formel I umgesetzt wird, worin R¹ bis R⁴, m und n die vorstehend genannte Bedeutung haben, und diese ggf. gereinigt und/oder isoliert wird
oder
wenigstens eine Verbindung der allgemeinen Formel II in einem Reaktionsmedium in Gegenwart wenigstens eines Reduktionsmittels, mit wenigstens einer Verbindung der allgemeinen Formel R¹-C(=O)-H, worin R¹ die vorstehend genannte Bedeutung hat, mit Ausnahme von -C(=O)-NR⁵R⁶, -C(=S)-NR⁷R⁸, -C(=O)-R⁹ und -S(=O)-R¹⁰, zu wenigstens einer Verbindung der allgemeinen Formel I umgesetzt wird, worin R¹ bis R⁴, m und n die vorstehend genannte Bedeutung haben, und diese ggf. gereinigt und/oder isoliert wird
oder
wenigstens eine Verbindung der allgemeinen Formel II in einem Reaktionsmedium ggf. in Gegenwart wenigstens einer Base mit wenigstens einer Verbindung der allgemeinen Formel R⁹-C(=O)-LG, worin R⁹ die vorstehend genannte Bedeutung hat und LG für eine Abgangsgruppe, vorzugsweise für einen Halogen-Rest steht, oder in einem Reaktionsmedium in Gegenwart wenigstens eines Kupplungsreagenzes ggf. in Gegenwart wenigstens einer Base mit einer Verbindung der allgemeinen Formel R⁹-C(=O)-OH, worin R⁹ die vorstehend genannte Bedeutung hat, zu wenigstens einer Verbindung der allgemeinen Formel I umgesetzt wird, worin R² bis R⁴, m und n die vorstehend genannte Bedeutung haben und R¹ für -C(=O)-R⁹ steht, und diese ggf. gereinigt und/oder isoliert wird
oder
wenigstens eine Verbindung der allgemeinen Formel II in einem Reaktionsmedium ggf. in Gegenwart wenigstens einer Base mit wenigstens einer Verbindung der allgemeinen Formel R¹⁰-S(=O)₂-LG, worin R¹⁰ die vorstehend genannte Bedeutung hat und LG für eine Abgangsgruppe, vorzugsweise für einen Halogen-Rest steht, zu wenigstens einer Verbindung der allgemeinen Formel I umgesetzt wird, worin R² bis R⁴, m und n die vorstehend genannte Bedeutung haben und R¹ für -S(=O)₂-R¹⁰ steht, und diese ggf. gereinigt und/oder isoliert wird.

Bevorzugt werden Verbindungen der allgemeinen Formel II mit einem Isocyanat der allgemeinen Formel R⁵-N=C=O, worin R⁵ die vorstehend angegebene Bedeutung hat, oder mit einem Isothiocyanat der allgemeinen Formel R⁷-N=C=S, worin R⁷ die vorstehend angegebene Bedeutung hat, in einem Reaktionsmedium, vorzugsweise ausgewählt aus der Gruppe bestehend aus Acetonitril, Toluol, Dimethylformamid, Benzol, Ethanol, Methanol, Wasser und entsprechenden Mischungen, ggf. in Gegenwart wenigstens einer Base, bevorzugt in Gegenwart wenigstens einer Base ausgewählt aus der Gruppe bestehend aus Triethylamin, N-Methylmorpholin, Pyridin, 4,4-Dimethylaminopyridin und Diisopropylethylamin, zu Verbindungen der allgemeinen Formel I umgesetzt, worin R¹ für -C(=O)-NR⁵R⁶ oder -C(=S)-NR⁷R⁸ steht, und R⁶ und R⁸ jeweils für einen Wasserstoff-Rest stehen.

Ebenfalls bevorzugt werden Verbindungen der allgemeinen Formel II mit Verbindungen der allgemeinen Formel LG-R¹, worin R¹ die vorstehend angegebene Bedeutung hat mit Ausnahme von -C(=O)-NR⁵R⁶, -C(=S)-NR⁷R⁸, -C(=O)-R⁹ und-S(=O)-R¹⁰, und LG für eine Abgangsgruppe, bevorzugt für ein Halogen-Atom, besonders bevorzugt für ein Chloratom steht, in einem Reaktionsmedium, vorzugsweise ausgewählt aus der Gruppe bestehend aus Dichlormethan, Toluol, Tetrahydrofuran, Acetonitril, Diethylether, Dioxan und entsprechenden Mischungen ggf. in Gegenwart wenigstens einer Base, bevorzugt in Gegenwart wenigstens eines Metallhydridsalzes, besonders bevorzugt in Gegenwart von Natrium- und/oder Kaliumhydrid, zu Verbindungen der allgemeinen Formel I, worin R¹ die vorstehend angegebene Bedeutung mit Ausnahme von -C(=O)-NR⁵R⁶, -C(=S)-NR⁷R⁸, -C(=O)-R⁹ und -S(=O)-R¹⁰ hat, umgesetzt.

Weiterhin bevorzugt werden Verbindungen der allgemeinen Formel II mit Verbindungen der allgemeinen Formel R¹-C(=O)-H, worin R¹ die vorstehend genannte Bedeutung mit Ausnahme von -C(=O)-NR⁵R⁶, -C(=S)-NR⁷R⁸, -C(=O)-R⁹ und -S(=O)-R¹⁰ hat, in einem Reaktionsmedium, vorzugsweise ausgewählt aus der Gruppe bestehend aus Diethylether, Tetrahydrofuran, Methanol, Ethanol, Dichlormethan, Toluol und entsprechenden Mischungen, unter Zusatz wenigstens eines Reduktionsmittels, vorzugsweise unter Zusatz wenigstens eines Reduktionsmittels ausgewählt aus der Gruppe bestehend aus Natriumborhydrid, Natriacetoxyborhydrid, Natriumcyanoborhydrid und Boran-Pyridin-Komplex (Pyridin-Boran, BH₃●C₅H₅N), besonders bevorzugt in Gegenwart von Boran-Pyridin-Komplex, zu Verbindungen der allgemeinen Formel I, worin R¹ die vorstehend angegebene Bedeutung mit Ausnahme von -C(=O)-NR⁵R⁶, -C(=S)-NR⁷R⁸, -C(=O)-R⁹ und -S(=O)-R¹⁰ hat, umgesetzt.

Ebenfalls bevorzugt werden Verbindungen der vorstehend angegebenen allgemeinen Formel II mit Carbonsäuren der allgemeinen Formel R⁹-C(=O)-OH, worin R⁹ die vorstehend genannte Bedeutung hat, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart wenigstens eines Kupplungsreagenzes, vorzugsweise ausgewählt aus der Gruppe bestehend aus N,N'-Carbonyldiimidazol, 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU), O-(Benzotriazol-1-yl)-N,N,N',N'-tetramethyluroniom tetrafluoroborat (TBTU), 1-Hydroxybenzotriazol (HOBt) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder wenigstens einer organischen Base, vorzugsweise ausgewählt aus der Gruppe bestehend aus Triethylamin, Pyridin, Dimethylaminopyridin, N-Methylmorpholin und Diisopropylethylamin vorzugsweise bei Temperaturen von -70°C bis 100°C zu Verbindungen der allgemeinen Formel I, worin R¹ für -C(=O)-R⁹ steht, umgesetzt.

Ebenfalls bevorzugt werden Verbindungen der allgemeinen Formel II mit Carbonsäurederivaten bzw. Kohlensäurederivaten der allgemeinen Formel R⁹-C(=O)-LG, wobei R⁹ die vorstehend genannte Bedeutung hat und LG für einen Halogen-Rest, vorzugsweise Chlor oder Brom, steht, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart wenigstens einer organischen Base, vorzugsweise ausgewählt aus der Gruppe bestehend aus Triethylamin, Dimethylaminopyridin, N-Methylmorpholin, Pyridin und Diisopropylamin, oder wenigstens einer anorganischen Base bei Temperaturen von vorzugsweise -70°C bis 100°C zu Verbindungen der allgemeinen Formel I umgesetzt.

Ebenfalls bevorzugt werden Verbindungen der allgemeinen Formel II mit Sulfonsäure-Derivaten der allgemeinen Formel LG-S(=O)₂-R¹⁰, worin R¹⁰ die vorstehend angegebene Bedeutung haben und LG für eine Abgangsgruppe, bevorzugt für ein Halogen-Atom, besonders bevorzugt für ein Chloratom steht, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart einer organischen Base, vorzugsweise ausgewählt aus der Gruppe bestehend aus Triethylamin, 4,4-Dimethylaminopyridin, N-Methylmorpholin, Pyridin und Diisopropylethylamin, oder einer anorganischen Base, bei Temperaturen von vorzugsweise -70°C bis 100°C zu Verbindungen der allgemeinen Formel I, worin R¹ für -S(=O)₂-R¹⁰ steht, umgesetzt.

Die Verbindungen der allgemeinen Formel II lassen sich wie in Schema 1. beschrieben erhalten.

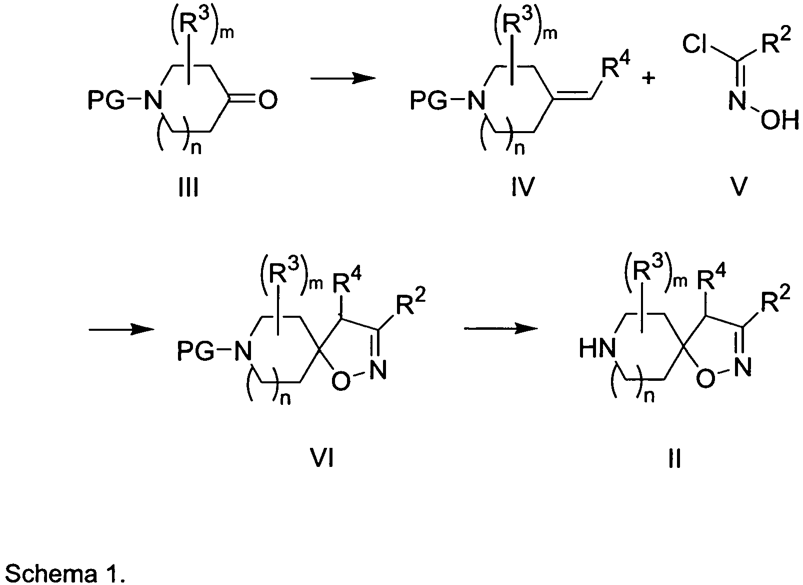

In Stufe 1 werden Verbindungen der allgemeinen Formel III, worin m, n und R³ die vorstehend genannte Bedeutung haben und PG für eine Schutzgruppe, bevorzugt für eine tert-Butyloxycarbonyl-Gruppe, steht, in einem Reaktionsmedium, vorzugsweise in einem Reaktionsmedium ausgewählt aus der Gruppe bestehend aus Tetrahydrofuran, Toluol, Diethylether und entsprechenden Mischungen, mit einem Reagenz zur Umwandlung von Carbonylgruppen in Doppelbindungen, bevorzugt mit einem Wittig-Reagenz der allgemeinen Formel R₃P(CH₂)R⁴X; worin R für einen Aryl-Rest steht, X für ein Halogenatom steht und R⁴ die vorstehend genannte Bedeutung hat; oder einem Wittig-Horner-Reagenz der allgemeinen Formel (RO)₂-P(=O)-(CH₂)-R⁴, worin R für einen Aryl-Rest steht und R⁴ die vorstehend genannte Bedeutung hat, besonders bevorzugt mit Methyltriphenylphosphoniumbromid, bei Temperaturen zwischen 0°C und 30 °C in Gegenwart einer Base, bevorzugt in Gegenwart eines Alkalimetallalkoholatsalzes, besonders bevorzugt in Gegenwart von Kalium-tert-butylat zu Verbindungen der allgemeinen Formel IV, worin m, n, R³, R⁴ und PG die vorstehend genannte Bedeutung haben, umgesetzt.

In Stufe 2 werden Verbindungen der allgemeinen Formel IV in einem Reaktionsmedium, bevorzugt in einem Reaktionsmedium ausgewählt aus der Gruppe bestehend aus Methanol, Tetrahydrofuran, Dichlormethan und entsprechenden Mischungen, in Gegenwart wenigstens einer Base, bevorzugt in Gegenwart von Natriumhydrogencarbonat, Lithiumhydroxid, Triethylamin oder N-Diisopropylethylamin, mit Verbindungen der allgemeinen Formel V, worin R² die vorstehend genannte Bedeutung hat, bei Temperaturen zwischen 0 °C und 100 °C zu Verbindungen der allgemeinen Formel VI, worin m, n, PG, R², R³ und R⁴ die vorstehend genannte Bedeutung haben, umgesetzt.

In Stufe 3 werden Verbindungen der allgemeinen Formel VI, worin PG für eine tert-Butyloxycarbonyl-Gruppe steht, in einem Reaktionsmedium vorzugsweise ausgewählt aus der Gruppe bestehend aus Methanol, Ethanol, Isopropanol, Wasser, Diethylether, Tetrahydrofuran sowie entsprechenden Mischungen in Gegenwart wenigstens einer Säure vorzugsweise ausgewählt aus der Gruppe bestehend aus Salzsäure, Schwefelsäure, Trifluoressigsäure und Essigsäure bei Temperaturen von vorzugsweise 20 bis 30 °C zu Verbindungen der allgemeinen Formel II umgesetzt. Besonders bevorzugt erfolgt die Umsetzung der Verbindung der allgemeinen Formel VI in einer 5 M Salzsäure-Lsg. in Isopropanol bei einer Temperatur von vorzugsweise 20 bis 30 °C zu einer Verbindung der allgemeinen Formel II in Form eines entsprechenden Hydrochlorids.

Die Verbindungen der vorstehend angegebenen Formeln R₃P(CH₂)R⁴X, (RO)₂-P(=O)-(CH₂)-R⁴, R¹-C(=O)-H, LG-R¹, LG-R⁶, LG-R⁸, R⁵-N=C=O, R⁷-N=C=S, III, V, R¹⁰-S(=O)₂-LG, R⁹-C(=O)-LG und R⁹-C(=O)-OH sind jeweils am Markt käuflich erhältlich und können auch nach üblichen, dem Fachmann bekannten Verfahren hergestellt werden.

Die vorstehend beschriebenen Umsetzungen können jeweils unter den üblichen dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden. Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie. Sämtliche der vorstehend beschriebenen Verfahrensschritte sowie jeweils auch die Reinigung und/oder Isolierung von Zwischen- oder Endprodukten können teilweise oder vollständig unter einer Inertgasatmosphäre, vorzugsweise unter Stickstoffatmosphäre, durchgeführt werden.

Die erfindungsgemäßen substituierten Spiro-Verbindungen der vorstehend genannten allgemeinen Formel I, la und Ib, im Folgenden nur als Spiro-Verbindungen der allgemeinen Formel I bezeichnet, sowie entsprechende Stereoisomere können sowohl in Form ihrer freien Basen, ihrer freien Säuren wie auch in Form entsprechender Salze, insbesondere physiologisch verträglicher Salze, isoliert werden. Die freien Basen der jeweiligen erfindungsgemäßen substituierten Spiro-Verbindungen der vorstehend genannten allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden. Die freien Basen der jeweiligen substituierten Spiro-Verbindungen der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden. Entsprechend können die freien Säuren der substituierten Spiro-Verbindungen der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-Alkyl-Rest steht, genannt.

Die erfindungsgemäßen substituierten Spiro-Verbindungen der vorstehend genannten allgemeinen Formel I und entsprechender Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten Spiro-Verbindungen der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Spiro-Verbindungen der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße Spiro-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Diese erfindungsgemäßen Arzneimittel eignen sich insbesondere zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1(VR1/TRPV1)-Stimulation.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1 vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Besonders bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz, und/oder Harninkontinenz.

Ein weiterer Gegenstand der vorliegenden Erfindung ist die Verwendung wenigstens einer erfindungsgemäßen Spiro-Verbindung sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1(VR1/TRPV1)-Stimulation.

Bevorzugt ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1 vermittelt werden.

Besonders bevorzugt ist die Verwendung wenigstens einer erfindungsgemäßen Spiro-Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz und Gelenkschmerz.

Besonders bevorzugt ist die Verwendung wenigstens einer erfindungsgemäßen Spiro-Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Harninkontinenz; einer überaktiven Blase (overactive bladder, OAB); Magengeschwüren; Reizdarmsyndrom; Schlaganfällen; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Besonders bevorzugt ist die Verwendung wenigstens einer erfindungsgemäßen Spiro-Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Atemwegserkrankungen, vorzugsweise ausgewählt aus der Gruppe bestehend aus Asthma und Lungenentzündung; Husten; Augenreizungen; Hautreizungen; neurotischen Hauterkrankungen und Entzündungskrankheiten, vorzugsweise Entzündungen des Darmes.

Ganz besonders bevorzugt ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Noch weiter bevorzugt ist die Verwendung wenigstens einer erfindungsgemäßen substituierten Spiro-Verbindung sowie ggf. eines oder mehrerer pharmazeutisch verträglicher Hilfsstoffe zur Herstellung eines Arzneimittels zur Behandlung und/oder Prophylaxe von Schmerz, vorzugsweise ausgewählt aus der Gruppe bestehend aus akutem Schmerz, chronischem Schmerz, neuropathischem Schmerz und visceralem Schmerz, und/oder Harninkontinenz.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einer substituierten Spiro-Verbindung der vorstehend angegebenen allgemeinen Formel I, ggf. in Form eines ihrer reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die beispielsweise ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Die in dem erfindungsgemäßen Arzneimittel zum Einsatz kommenden erfindungsgemäßen substituierten Spiro-Verbindungen in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die jeweilige erfindungsgemäße substituierte Spiro-Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung. Die an den Patienten zu verabreichende Menge der jeweiligen erfindungsgemäßen substituierten Spiro-Verbindungen der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,001 bis 100 mg/kg, vorzugsweise 0,05 bis 75 mg/kg, besonders bevorzugt 0,05 bis 50 mg/kg, Körpergewicht des Patienten wenigstens einer solchen erfindungsgemäßen Verbindung appliziert.

### Pharmakologische Methoden:

### I. Funktionelle Untersuchung am Vanilloid-Rezeptor 1 (VRI/TRPV1-Rezeptor)

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der Ca²⁺-Einstrom durch den Rezeptorkanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Komplett-Medium: 50 mL HAMS F12 Nutrient Mixture (Gibco Invitrogen GmbH, Karlsruhe, Deutschland) mit
10 Vol-% FCS (fetal calf serum, Gibco Invitrogen GmbH, Karlsruhe, Deutschland, hitzeinaktiviert);
2mM L-Glutamin (Sigma, München, Deutschland);
1 Gew-% AA-Lösung (Antibiotika/Antimykotika-Lösung, PAA, Pasching, Österreich) und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 well black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100 µg/mL mit PBS (Ca-Mg-free PBS, Gibco Invitrogen GmbH, Karlsruhe, Deutschland) verdünnt wird. Es werden Aliquots mit einer Konzentration von 100 µg/mL an Laminin entnommen und bei -20 °C gelagert. Die Aliquots werden mit PBS im Verhältnis 1:10 auf 10 µg/mL Laminin verdünnt und jeweils 50 µL der Lösung in eine Vertiefung der Zellkultur-Platte pipettiert. Die Zellkultur-Platten werden mindestens zwei Stunden bei 37 °C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

### Präparation der Zellen:

Enthaupteten Ratten wird die Wirbelsäule entnommen und diese direkt in kalten, d. h. in einem Eisbad befindlichen, HBSS-Puffer (Hank's buffered saline solution, Gibco Invitrogen GmbH, Karlsruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzelganglien (DRGs; dorsal root ganglia) entnommen und wiederum in kaltem HBSS-Puffer versetzt mit 1 Vol-% einer AA-Lösung aufbewahrt. Die vollständig von Blutresten und Spinalnerven befreiten DRGs werden jeweils in 500 µL kalte Collagenase Typ 2 (PAA, Pasching, Österreich) überführt und 35 Minuten bei 37 °C inkubiert. Nach Zugabe von 2.5 Vol-% Trypsin (PAA, Pasching, Österreich) wird weitere 10 Minuten bei 37 °C inkubiert. Nach der vollständigen Inkubation wird die Enzymlösung vorsichtig ab pipettiert und die zurückgebliebenen DRGs werden jeweils mit 500 µL Komplett-Medium versetzt. Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 µm Falcon-Filtereinsatz filtriert und 10 Minuten bei 1200 Umdrehungen und Raumtemperatur zentrifugiert. Das resultierende Pellet wird jeweils in 250 µL Komplett-Medium aufgenommen und die Zellzahl ermittelt.

Die Anzahl der Zellen in der Suspension wird auf 3 mal 10⁵ pro mL eingestellt und jeweils 150 µL dieser Suspension in eine Vertiefung der wie vorstehend beschrieben beschichteten Zellkultur-Platten gegeben. Im Brutschrank werden die Platten bei 37 °C, 5 Vol-% CO₂ und 95 % relativer Luftfeuchtigkeit zwei bis drei Tage stehen gelassen.

Anschließend werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur Ca²⁺-Messung im FLIPR-Assay eingesetzt. Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Verbindungen (10 µM) auf die Zellen pipettiert und der Ca²⁺-Einstrom mit der Kontrolle (Capsaicin 10 µM) verglichen. Daraus ergibt sich die Angabe in % Aktivierung bezogen auf das Ca²⁺-Signal nach Zugabe von 10 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca²⁺-Einstroms. Es werden % Inhibierung im Vergleich zu der maximal erreichbaren Inhibierung mit 10 µM Capsaicin berechnet.

Es werden Dreifach-Bestimmungen (n=3) durchgeführt und diese in mindestens 3 unabhängigen Experimenten wiederholt (N=4).

### II. Funktionelle Untersuchungen am Vanilloid Rezeptor (VR1)

Die agonistische bzw. antagonistische Wirkung der zu untersuchenden Substanzen auf Vanilloid Rezeptor (VR1) kann auch mit dem folgenden Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes, Europe BV, Leiden, Niederlande) im Fluorescent Imaging Plate Reader (FLIPR, Molecular Devices, Sunnyvale, USA) quantifiziert.

### Methode:

Chinese Hamster Ovary Zellen (CHO K1-Zellen, European Collection of Cell Cultures (ECACC) Großbritannien) werden stabil mit dem VR1-Gen transfiziert. Für funktionelle Untersuchungen werden diese Zellen auf Poly-D-Lysin-beschichtete, schwarze 96-Loch-Platten mit klarem Boden (BD Biosciences, Heidelberg, Deutschland) in einer Dichte von 25.000 Zellen/Loch ausplattiert. Über Nacht werden die Zellen bei 37 °C und 5 % CO₂ in einem Kulturmedium (Nutrient Mixture Ham's F12, 10 Vol-% FCS (Fetal calf serum), 18 µg/ml L-Prolin) inkubiert. Am folgenden Tag werden die Zellen mit Fluo-4 (Fluo-4 2 µM, Pluronic F127 0,01 Vol-%, Molecular Probes in HBSS (Hank's buffered saline solution), Gibco Invitrogen GmbH, Karlsruhe, Deutschland) für 30 Minuten bei 37 °C inkubiert. Anschließend werden die Platten 3 mal mit HBSS-Puffer gewaschen und nach einer weiteren Inkubation von 15 Minuten bei Raumtemperatur zur Ca²⁺⁻-Messung im FLIPR eingesetzt. Die Ca²⁺-abhängige Fluoreszenz wird dabei vor und nach Zugabe der zu untersuchenden Substanzen gemessen (Wellenlänge λₑₓ=488 nm, λₑₘ= 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Substanzen (10µM) auf die Zellen pipettiert und der Ca²⁺-Einstrom mit der Kontrolle (Capsaicin 10 µM) verglichen (% Aktivierung bezogen auf das Ca²⁺⁻-Signal nach Zugabe von 10 µM Capsaicin). Nach 5 Minuten Inkubation werden 100 nM Capsaicin appliziert und ebenfalls der Einstrom von Ca²⁺ ermittelt.

Desensitisierende Agonisten und Antagonisten führten zu einer Unterdrückung des Ca²⁺-Einstroms. Es werden % Inhibierung im Vergleich zu der maximal erreichbaren Inhibierung mit 10 µM Capsaicin berechnet.

### III. Formalin-Test an der Maus

Die Untersuchung zur Bestimmung der antinociceptiven Wirkung der erfindungsgemäßen Verbindungen wird im Formalin-Test an männlichen Mäusen (NMRI, 20 bis 30 g Körpergewicht, Iffa, Credo, Belgien) durchgeführt.

Im Formalin-Test werden gemäß D. Dubuisson et al., Pain 1977, 4, 161-174 die erste (frühe) Phase (0 bis 15 Minuten nach der Formalin-Injektion) und die zweite (späte) Phase (15 bis 60 Minuten nach der Formalin-Injektion) unterschieden. Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al., Pain 1993, 52, 259-285). Die entsprechenden Literaturbeschreibungen werden hiermit als Referenz eingeführt und gelten als Teil der Offenbarung.

Die erfindungsgemäßen Verbindungen werden in der zweiten Phase des Formalin-Tests untersucht, um Aussagen über Substanzwirkungen auf chronisch/entzündlichen Schmerz zu erhalten.

In Abhängigkeit von der Applikationsart der erfindungsgemäßen Verbindungen wird der Applikationszeitpunkt der erfindungsgemäßen Vebindungen vor der Formalin-Injektion gewählt. Die intravenöse Applikation von 10 mg/kg Körpergewicht der Testsubstanzen erfolgt 5 Minuten vor der Formalin-Injektion. Diese erfolgt durch eine einmalige subkutane Formalin-Injektion (20 µL, 1 %-ige wäßrige Lösung) in die dorsale Seite der rechten Hinterpfote, so dass bei freibeweglichen Versuchstieren eine nociceptive Reaktion induziert wird, die sich in deutlichem Lecken und Beißen der betreffenden Pfote äußert.

Anschließend wird für einen Untersuchungszeitraum von drei Minuten in der zweiten (späten) Phase des Formalin-Tests (21 bis 24 Minuten nach der Formalin-Injektion) das nociceptive Verhalten durch Beobachtung der Tiere kontinuierlich erfasst. Die Quantifizierung des Schmerzverhaltens erfolgt durch Summation der Sekunden, in denen die Tiere in dem Untersuchungszeitraum ein Lecken und Beißen der betroffenen Pfote zeigen.

Der Vergleich erfolgt jeweils mit Kontrolltieren, die anstelle der erfindungsgemäßen Verbindungen Vehikel (0.9%-ige wäßrige Natriumchlorid-Lsg.) vor Formalinapplikation erhalten. Basierend auf der Quantifizierung des Schmerzverhaltens wird die Substanzwirkung im Formalin-Test als Änderung gegen die entsprechende Kontrolle in Prozent ermittelt.

Nach Injektion von Substanzen, die im Formalin-Test antinociceptiv wirksam sind, werden die beschriebenen Verhaltensweisen der Tiere, d. h. Lecken und Beißen, reduziert bzw. aufgehoben.

### IV. Untersuchung auf analgetische Wirksamkeit im Writhing-Test

Die Untersuchung der erfindungsgemäßen Verbindungen der allgemeinen Formel I auf analgetische Wirksamkeit wurde im Phenylchinon-induzierten Writhing an der Maus, modifiziert nach I.C. Hendershot und J. Forsaith (1959) J. Pharmacol. Exp. Ther. 125, 237-240 durchgeführt. Die entsprechende Literaturbeschreibung wird hiermit als Referenz eingeführt und gilt als Teil der Offenbarung.

Dazu wurden männliche NMRI-Mäuse mit einem Gewicht von 25 bis 30 g verwendet. Gruppen von 10 Tieren pro Verbindungsdosis erhielten 10 Minuten nach intravenöser Gabe der zu untersuchenden Verbindungen 0,3 ml/Maus einer 0,02%igen wäßrigen Lösung von Phenylchinon (Phenylbenzochinon, Fa. Sigma, Deisenhofen, Deutschland; Herstellung der Lösung unter Zusatz von 5 Gew.-% Ethanol und Aufbewahrung im Wasserbad bei 45°C) intraperitoneal appliziert. Die Tiere wurden einzeln in Beobachtungskäfige gesetzt. Mit Hilfe eines Drucktastenzählers wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mit geführt, die nur physiologische Kochsalzlösung erhalten hatten. Alle Verbindungen wurden in der Standarddosierung von 10 mg/kg getestet.

Im Folgenden wird die Erfindung mit Hilfe einiger Beispiele erläutert. Diese Erläuterungen sind lediglich beispielhaft und schränken den allgemeinen Erfindungsgedanken nicht ein.

### Beispiele:

Die Ausbeuten der hergestellten Verbindungen sind nicht optimiert.

Alle Temperaturen sind unkorrigiert.

### Abkürzungen:

- abs.: absolutiert
- aq.: wäßrig
- Äq.: Stoffmengenäquivalent
- Boc: tert-Butoxycarbonyl
- D: Tage
- DCM: Dichlormethan
- DMF: Dimethylformamid
- EtOAc: Ethylacetat
- EtOH: Ethanol
- ges.: gesättigt
- MeOH: Methanol
- NMR: Kernresonanzspektroskopie
- RT: Raumtemperatur
- THF: Tetrahydrofuran

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.
Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.
Die Analytik erfolgte durch Massenspektroskopie und NMR.

### Herstellung erfindungsgemäßer substituierter 1-Oxa-2,8-diaza-spiro[4.5]dec-2-en-Derivate

### 1. Synthese von 3-Phenyl-1-oxa-2,8-diaza-spiro[4.5]dec-2-en

Die Synthese von 3-Phenyl-1-oxa-2,8-diaza-spiro[4.5]dec-2-en (E) ist in Schema 2. verdeutlicht.

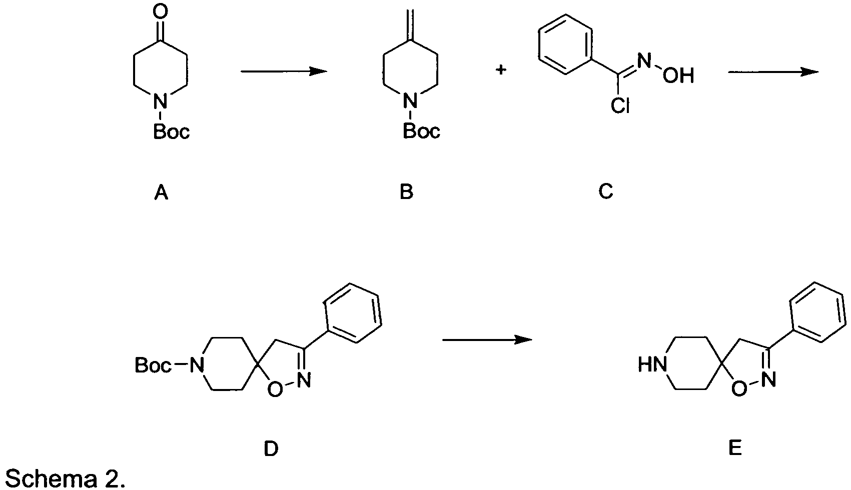

### Synthese von 4-Methylen-piperidin-1-carbonsäure-tert-butylester (B)

Zu einer Suspension von 5,34 g (15 mmol) Methyltriphenylphosphoniumbromid in 50 ml Diethylether wurden unter Rühren bei 0°C (Eisbad) 1,6 g (14 mmol) Kalium-tert-butylat zugegeben. Nach 15 min Rühren wurde eine Lösung von 2,00 g (10 mmol) 1-Boc-4-Piperidon (A) in 15 ml Diethylether langsam zugegeben. Man ließ die Suspension weitere 30 min bei 0°C rühren. Nach Zugabe von 60 ml 10%-iger aq. NH₄Cl-Lsg. wurde die organische Phase abgetrennt, über Magnesiumsulfat getrocknet und im Vakuum vom Lösungsmittel befreit. Nach Chromatographie an Kieselgel (Hexan:EtOAc = 5:1) erhielt man 1,71 g (89%) 4-Methylen-piperidin-1-carbonsäure-tert-butylester (B) als farblose Flüssigkeit.

¹H-NMR-Spektrum (d₆-DMSO): δ = 1,47 ppm (s, 9H, C(CH₃)₃); 2.16-2.19 ppm (m, 4H, CH₂); 3.40-3.44 ppm (m, 4H, CH₂); 4,74 (s, 2H, C=CH₂).

### Synthese von 1-Chlor-1-hydroxyimino-methyl-benzol (C)

Zu einer Lösung von Benzaldehydoxim (3.5 g, 28.9 mmol) in DMF (30 ml) wurde bei RT N-Chlorsuccinimid (4.63 g, 34. 7 mmol) gegeben, dabei stieg die Temperatur kurzfristig auf 50 °C an. Die Reaktionsmischung wurde im Eisbad gekühlt, 3 h bei RT gerührt, mit Wasser (100 ml) unter Eiskühlung versetzt und mit Ether (3 x 100 ml) extrahiert. Die vereinigten organischen Phasen wurden mit Wasser (150 ml) und ges. aq. NaCl-Lsg. (150 ml) gewaschen, getrocknet und das Lösungsmittel im Vakuum entfernt. Das gewünschte Produkt 1-Chlor-1-hydroxyimino-methyl-benzol (C) wurde als leicht gelber Feststoff (4.28 g) erhalten.

### Synthese von 3-Phenyl-1-oxa-2,8-diaza-spiro[4.5]dec-2-en-8-carbonsäure-tert-butylester (D)

Zu einer Lösung von 4-Methylen-piperidin-1-carbonsäure-tert-butylester (B) (39.5 g, 200 mmol) in DCM (400 ml) wurde bei 0 °C langsam eine Lösung von 1-Chlor-1-hydroxyimino-methyl-benzol (C) (93.6 g, 601 mmol) in DCM (400 ml) getropft. Anschließend wurde langsam eine Lösung von Triethylamin (6.7 ml) in DCM (400 ml) zugetropft und die resultierende Mischung wurde 48 Stunden bei RT gerührt.
Die Reaktionsmischung wurde mit DCM verdünnt und mit Wasser, 10%-iger aq. Zitronensäure-Lsg. und ges. aq. NaCl-Lsg. gewaschen. Die organische Phase wurde getrocknet und das Lösungsmittel im Vakuum entfernt. Der Rückstand wurde in Ether (40 ml) aufgenommen, wobei das gewünschte Produkt als weißer Niederschlag ausfiel, der abgesaugt und anschließend getrocknet wurde. Es wurden 36.4 g (57 % der Theorie) des gewünschten Produkts erhalten.

### Synthese von 3-Phenyl-1-oxa-2,8-diaza-spiro[4.5]dec-2-en (E) als Hydrochloridsalz

Eine Lösung von 3-Phenyl-1-oxa-2,8-diaza-spiro[4.5]dec-2-en-8-carbonsäure-tert-butylester (D) (3,08 g, 9.73 mmol) in MeOH (90 ml) wurde bei 0 °C mit konz. HCl (16.5 ml) versetzt. Die Reaktionsmischung wurde über Nacht gerührt, das Lösungsmittel im Vakuum entfernt und der Rückstand wird langsam zu gekühltem Ether (500 ml) gegeben. Nach 48 Stunden war ein feiner Niederschlag entstanden, der abgesaugt und getrocknet wurde. Es wurde das gewünschte Produkt (1.87 g, 76 % der Theorie) als weißer Feststoff erhalten.

### 2. Synthese von 3-(3-Chlorpyridin-2-yl)-1-oxa-2,8-diazaspiro[4.5]dec-2-en (K)

Die Synthese von 3-(3-Chlorpyridin-2-yl)-1-oxa-2,8-diazaspiro[4.5]dec-2-en (K) ist in Schema 3. verdeutlicht.

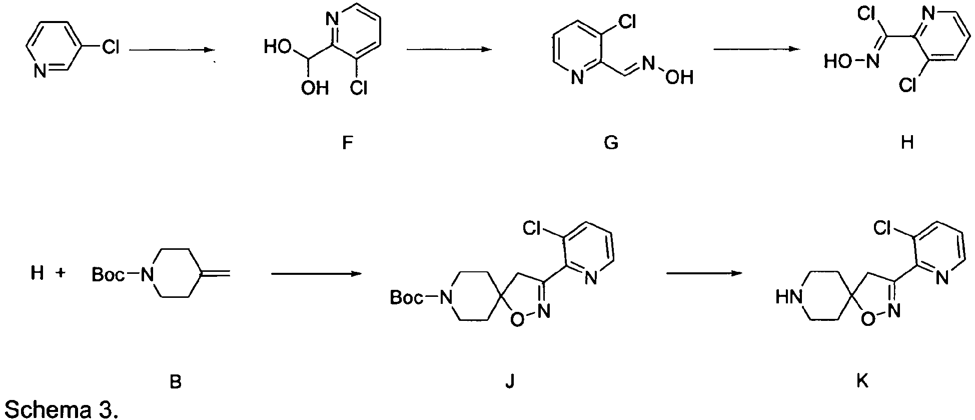

### Synthese von 3-Chlorpyridin-2-carbaldehyd (F)

Zu einer Lösung von *N,N* Dimethylaminoethanol (10.65 g, 120 mmol) in abs. THF (70 ml) wurde bei - 5 °C unter Argon innerhalb von 25 min eine 2,5 M Lösung von *n-*Butyllithium (96 ml, 240 ml) in *n*-Hexan hinzugefügt. Die Reaktionsmischung wurde 30 min bei 0 °C gerührt und auf -70 °C abgekühlt. Bei einer Temperatur von -70 bis-65 °C wurde eine Lösung von 3-Chlorpyridin (3.8 ml, 4.5 g, 40 mmol) in abs. THF (60 ml) innerhalb von 10 min zugegeben und eine weitere Stunde bei dieser Temperatur gerührt. Anschließend erfolgte die Zugabe von abs. DMF (12.3 ml, 11.7 g, 160 mmol) in THF (100 ml) innerhalb von 15 min bei -65 bis -60 °C. Die dunkelbraune Lösung wurde innerhalb von 1 h auf 10 °C erwärmt. Bei einer Temperatur von -20 bis -10 °C wurde die Reaktionsmischung mit ges. Ammoniumchlorid-Lsg. hydrolysiert (120 ml). Anschließend wurde der Ansatz 30 min bei 0 °C gerührt. Die Phasen wurden nicht getrennt. Die organischen Lösungsmittel wurden unter Vakuum entfernt. Der Rückstand wurde mit DCM (120 ml) extrahiert. Die Phasen wurden getrennt. Die wäßrige Phase wurde mit DCM (3 × 120 ml) extrahiert. Die organischen Phasen wurden vereinigt und ohne Trocknung eingeengt. Der Rückstand war ein braunes Öl, das durch Zugabe von Wasser (4 × 50 ml) und Dekantieren weiter gereinigt wurde. Nach Zugabe von DCM (250 ml) und Natriumsulfat wurde die Mischung über Nacht getrocknet und anschließend eingeengt. Dabei wurde das Rohprodukt des 3-Chlorpyridin-2-carbaldehyds (F) als Hydrat erhalten (braunes Öl 8.73 g, theoretische Ausbeute 6.3 g). Eine chromatographische Reinigung des Rohproduktes war nicht möglich. Es wurde ohne weitere Reinigung eingesetzt.

### Synthese von Chlorpyridin-2-carbaldehydoxim (G)

Das Aldehydhydrat F (6.3 g, 40 mmol) wurde in EtOH (170 ml) gelöst und nacheinander mit Hydroxylamin-hydrochlorid (4,16 g, 60 mmol) und Amberlyst A 21 (25,5 g, basischer Ionenaustauscher) versetzt. Die Reaktionsmischung wurde 20 h bei RT gerührt und der Ionenaustauscher anschließend durch Filtration abgetrennt. Das Filtrat wurde eingeengt, der feste braune Rückstand mit Wasser (3 × 50 ml) gewaschen und durch Filtration abgetrennt. Das Rohprodukt wurde in DCM (30 ml) aufgenommen und 30 min gerührt. Ein Feststoff wurde anschließend durch Filtration abgetrennt und mit DCM (2 × 8 ml) gewaschen. Es wurde als beigefarbener Feststoff in einer Ausbeute von 45 % (2,79 g) erhalten.

### Synthese von 4-(3-Chlorpyridin-2-yl)hydroxymoylchlorid (H)

Zu einer Lösung von Verbindung G (8.61 g, 55.0 mmol) in abs. DMF (70 ml) wurde eine Lösung von *N*-Chlorsuccinimid (8,94 g, 66,0 mmol) in abs. DMF (50 ml) bei einer Temperatur von 30-40 °C innerhalb von 20 min gegeben. Bei Bedarf wurde im Eisbad gekühlt. Die klare Reaktionsmischung wurde 3 h bei RT gerührt und anschließend mit Diethylether (600 ml) und Wasser (400 ml) versetzt. Die Phasen wurden getrennt. Die wäßrige Phase wurde mit Diethylether (200 ml) extrahiert. Die vereinigten organischen Phasen wurden nacheinander mit Wasser (300 ml) und ges. NaCl-Lsg. (200 ml) gewaschen. Die organische Phase wurde getrocknet und eingeengt. Das Rohprodukt der Verbindung H wurde als brauner Feststoff (8,1 g, 77 %) erhalten und sofort weiter umgesetzt.

### Synthese von 3-(3-Chlorpyridin-2-yl)-1-oxa-2,8-diazaspiro[4.5]dec-2-en-8-carbonsäure-tert-butylester (J)

Verbindung H (8,1 g, 42,4 mmol), gelöst in abs. THF (80 ml), wurde bei 0 °C innerhalb von 10 min zu einer Lösung von Verbindung B (5.97 g, 30.3 mmol) in abs. THF (80 ml) getropft. Zu dieser Mischung wurde bei 0 °C eine Lösung von Triethylamin (23.7 ml, 17.1 g, 170 mmol) in abs. THF (30 ml) innerhalb von 15 min gegeben. Da die Reaktion stark exotherm war, wurde in einer Eis/Kochsalz-Mischung gekühlt. Während der Zugabe fiel ein Feststoff aus. Die Reaktionsmischung wurde 16 h bei RT gerührt. Zur Aufarbeitung des Ansatzes wurde Wasser (120 ml) hinzugefügt und THF im Vakuum entfernt. Die zurückbleibende wäßrige Phase wurde mit DCM (130 ml) extrahiert. Die organische Phase wurde nacheinander mit 10%-iger Zitronensäure-Lsg. (2 × 150 ml) und ges. NaCl-Lsg. (150 ml) gewaschen. Die organische Phase wurde getrocknet und eingeengt (11.4 g, braunes Öl). Der Rückstand wurde chromatographisch aufgetrennt [Kieselgel 60 (400 g); EtOAc/Cyclohexan 1 : 3 (1,7 l), EtOAc/Cyclohexan 1 : 2 (2,4 l), EtOAc/Cyclohexan 1 : 1 (0,6 l)]. Die Verbindung J wurde als farbloser Feststoff in einer Ausbeute von 20 % (2.1 g) mit einem Schmelzpunkt von 97 - 102 °C gewonnen.

### Synthese von 3-(3-Chlorpyridin-2-yl)-1-oxa-2,8-diazaspiro[4.5]dec-2-en (K) als Hydrochlorid

Die Verbindung J (2,1 g, 5,97 mmol) wurde in DCM (35 ml) gelöst und mit einer 5N Salzsäure-Lsg. in Propan-2-ol (23,8 ml, 119 mmol) versetzt. Nach einer Reaktionszeit von 2 h fiel das Hydrochlorid der Verbindung K als farbloser Feststoff aus und wurde in einer Ausbeute von 99 % (1.7 g) mit einem Schmelzpunkt von 241-245 °C isoliert.

### 3. Synthese von 3-(Thiazol-2-yl)-1-oxa-2,8-diazaspiro[4.5]dec-2-en (O)

Die Synthese von 3-(Thiazol-2-yl)-1-oxa-2,8-diazaspiro[4.5]dec-2-en ist in Schema 4. verdeutlicht.

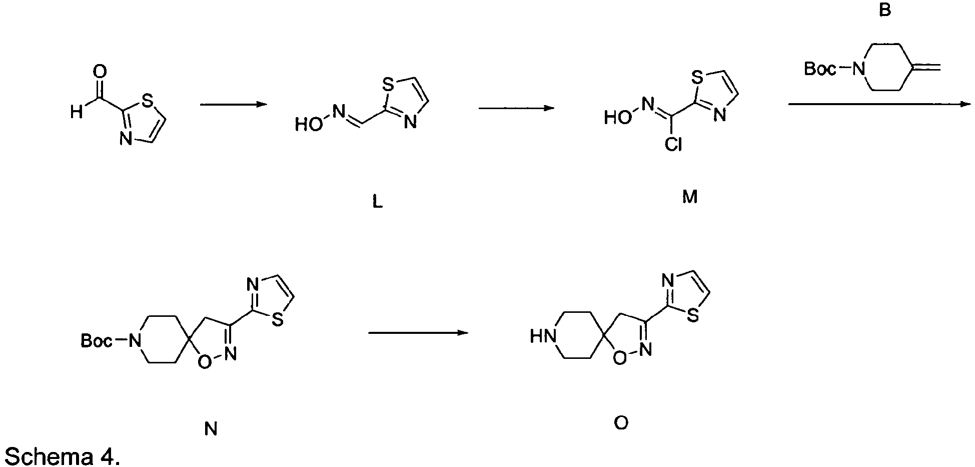

### Synthese von Thiazol-2-carbaldehydoxim (L)

Thiazol-2-carbaldehyd (14.0 g, 124 mmol) wurde in EtOH (450 ml) gelöst und nacheinander mit Hydroxylamin-hydrochlorid (12.9 g, 186 mmol) und Amberlyst A 21 (79 g, basischer Ionenaustauscher) versetzt. Die Reaktionsmischung wurde 24 h bei RT gerührt und der Ionenaustauscher anschließend durch Filtration abgetrennt. Das Filtrat wurde eingeengt, der feste Rückstand in EtOAc (350 ml) aufgenommen und mit Wasser (3 × 90 ml) gewaschen. Die organische Phase wurde getrocknet und eingeengt. Verbindung L wurde dabei als gelber Feststoff in einer Ausbeute von 94 % (14.92 g) erhalten.

### Synthese von Thiazol-2-ylhydroxymoylchlorid (M)

Zu einer Lösung von Verbindung L (14.9 g, 116 mmol) in abs. DMF (40 ml) wurde eine Lösung von *N*-Chlorsuccinimid (18.9 g, 139 mmol) in abs. DMF (65 ml) bei einer Temperatur von 30-40 °C innerhalb von 20 min gegeben. Bei Bedarf wurde im Eisbad gekühlt. Die Reaktionsmischung wurde 3 h bei RT gerührt und anschließend mit Diethylether (650 ml) und Wasser (440 ml) versetzt. Da sich zwischen den Phasen ein Feststoff absetzte, wurde die Mischung vor der Phasentrennung filtriert. Die wäßrige Phase wurde mit Diethylether (2 × 200 ml) extrahiert. Die vereinigten organischen Phasen wurden nacheinander mit Wasser (300 ml) und ges. NaCl-Lsg. (200 ml) gewaschen. Die organische Phase wurde getrocknet und eingeengt. Das Rohprodukt der Verbindung M wurde als gelber Feststoff (13.6 g) erhalten und sofort weiter umgesetzt.

### Synthese von 3-(Thiazol-2-yl)-1-oxa-2,8-diazaspiro[4.5]dec-2-en-8-carbonsäure-tert-butylester (N)

Verbindung M (13.6 g, 116 mmol), gelöst in abs. THF (180 ml) und abs. Diethylether (80 ml), wurde bei 0 °C innerhalb von 10 min zu einer Lösung von Verbindung B (13.2 g, 66.7 mmol) in abs. THF (100 ml) getropft. Zu dieser Mischung wurde bei 0 °C eine Lösung von Triethylamin (65.1 ml, 23.3 g, 464 mmol) in abs. THF (60 ml) innerhalb von 30 min gegeben. Da die Reaktion stark exotherm war, wurde in einer Eis/Kochsalz-Mischung gekühlt. Während der Zugabe fiel ein Feststoff aus. Die Reaktionsmischung wurde 16 h bei RT gerührt. Der Feststoff wurde abgesaugt, das Filtrat eingeengt und der dunkelbraune ölige Rückstand wurde mit Diethylether (300 ml) und Wasser (120 ml) 10 min gerührt. Nach Phasentrennung wurde die wäßrige Phase mit Diethylether (2 × 100 ml) extrahiert. Die vereinigten organischen Extrakte wurden nacheinander mit 10%-iger Zitronensäure-Lsg. (2 × 150 ml) und ges. NaCl-Lsg. (100 ml) gewaschen. Die organische Phase wurde getrocknet und bis auf ca. 50 ml eingeengt. Diese Lösung wurde bis zur Trübung mit n-Hexan versetzt und dann 24 h bei -18 °C aufbewahrt. Das gewünschte Produkt N fiel mit einem Nebenprodukt verunreinigt als hellbrauner Feststoff (3.81 g) aus und wurde abgesaugt. Das Filtrat wurde eingeengt und der dunkelbraune ölige Rückstand wurde durch Chromatographie [Kieselgel 60 (200g); Cyclohexan/EtOAc 5:1 (1900 ml); 3:1 (1100 ml)] aufgetrennt. Es konnte 1.0 g des gewünschten Produkts N gewonnen werden, das noch Verunreinigungen enthielt.

### Synthese von 3-(Thiazol-2-yl)-1-oxa-2,8-diazaspiro[4.5]dec-2-en (O)

Verbindung N (3.8 g, 11.75 mmol) wurde in DCM (55 ml) gelöst und mit einer 5,5 N Salzsäure-Lsg. in Propan-2-ol (43 ml, 235 mmol) versetzt. Es fiel sofort ein Feststoff aus. Nach einer Reaktionszeit von 1 h wurde der Feststoff abgesaugt und der Ansatz eingeengt. Das erhaltene hellbraune Öl wurde mit Diethylether (20 ml) und EtOH (2 ml) versetzt und 7 h bei RT gerührt. Der erhaltene Feststoff (1.0 g) wurde abgesaugt und das Filtrat 16 h gerührt. Es konnte weiterer Feststoff (618 mg) gewonnen werden. Beide Fraktionen wurden vereinigt. Das gewünschte Produkt O wurde dabei als gelbe Verbindung in einer Ausbeute von 53 % mit einem Schmelzpunkt von 244-251 °C isoliert.

### 4. Synthese von 3-Phenyl-1-oxa-2,7-diazaspiro[4.4]non-2-en (R)

Die Synthese von 3-Phenyl-1-oxa-2,7-diazaspiro[4.4]non-2-en ist in Schema 5. verdeutlicht.

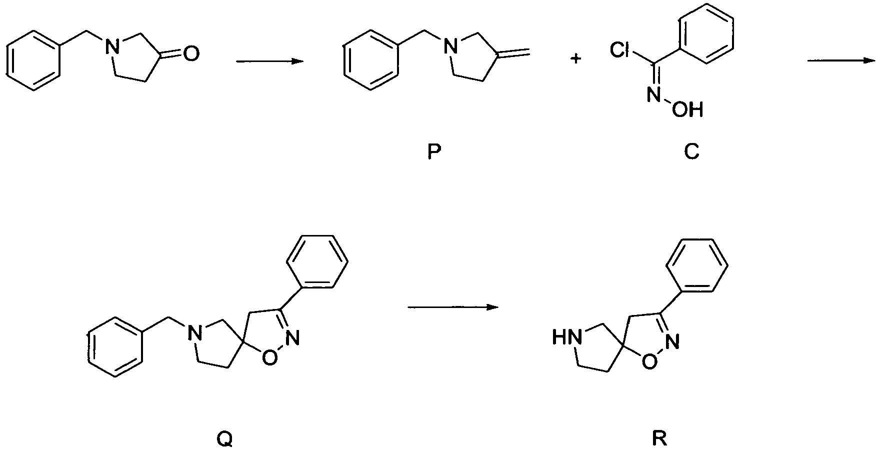

### Synthese von 1-Benzyl-3-methylenpyrrolidin (P)

Zu einer Suspension von Methyltriphenylphosphoniumbromid (7.5 g, 21 mmol) in trockenem THF (20 ml) wurde unter Argon bei 0 °C Kalium-*tert*-butylat (2.1 g, 18.8 mmol, gelöst in 20 ml abs. THF) innerhalb von 30 min gegeben. Das gelbe Reaktionsgemisch wurde 30 min bei 0 °C gerührt, bevor 1-Benzyl-pyrrolidin-3-on (2.46 g, 15 mmol, gelöst in abs. THF (20 ml) innerhalb von 30 min bei 0 °C zu getropft wurde. Das erhaltene Gemisch wurde 30 min bei 0 °C belassen, innerhalb von 1 h auf RT gebracht und weitere 15 h gerührt. Zur Aufarbeitung wurde der Ansatz auf ges. NH₄Cl-Lsg. (50 ml) gegeben und anschließend mit EtOAc (3 x 20 ml) extrahiert. Das gewünschte Produkt P wurde durch Extraktion mit 2 N HCl (20 ml) in das entsprechende Hydrochlorid überführt. Die organische Phase wurde mit Wasser (3 x 20 ml) gewaschen und die vereinigten wäßrigen Phasen wurden mit K₂CO₃ basisch gemacht. Das gewünschte Produkt P fiel in Form einer Ölschicht an und wurde durch erneute Extraktion mit EtOAc (3 x 20 ml) abgetrennt. Nach Trocknen der vereinigten organischen Phasen über Na₂SO₄ und Entfernen des Lösungsmittels lag das gewünschte Produkt P in ausreichend reiner Form vor (2.17 g, 83 %).

### Synthese von 7-Benzyl-3-phenyl-1-oxa-2,7-diazaspiro[4.4]non-2-en (Q)

Zu einer Lösung von 1-Benzyl-3-methylenpyrrolidin (P) (2.18 g, 12.6 mmol) in abs. DCM (25 ml) wurde bei 0°C innerhalb von 10 min Verbindung C (3.9 g, 25.2 mmol, gelöst in abs. DCM (25 ml)) getropft. Zu dieser Mischung wurde bei 0 °C eine Lösung von Triethylamin (14 ml, 10.2 g, 100.1 mmol) in abs. DCM (25 ml) innerhalb von 10 min gegeben. Da die Reaktion stark exotherm war, wurde in einer Eis/Kochsalz-Mischung gekühlt. Die Reaktionsmischung wurde 16 h bei RT gerührt. Zur Aufarbeitung des Ansatzes wurde DCM (50 ml) und Wasser (70 ml) hinzugefügt. Die Phasen wurden getrennt. Die wäßrige Lösung wurde mit DCM (2 x 20 ml) extrahiert. Die vereinigten organischen Phasen wurden nacheinander mit Wasser (50 ml) und ges. NaCl-Lsg. (50 ml) gewaschen und anschließend getrocknet und eingeengt (5.3 g, gelbes Öl). Der Rückstand wurde durch Säulenchromatographie gereinigt [Kieselgel 60 (500 g); EtOAc/Cyclohexan 1:2 (3 l)]. Die gewünschte Verbindung Q wurde als Feststoff in einer Ausbeute von (2.4 g, 65 %) mit einem Schmelzpunkt von 31 - 40 °C gewonnen.

### Synthese von 3-Phenyl-1-oxa-2,7-diazaspiro[4.4]non-2-en (R)

Verbindung Q (700 mg, 2.1 mmol) wurde in einem Gemisch aus THF (80 ml) und MeOH (10 ml) gelöst und mit Palladiumkatalysator (Pd/C, 5 %, 555 mg) versetzt und insgesamt 2.5 h bei RT einem Wasserstoffdruck von 3 bar ausgesetzt. Der Katalysator wurde mit Hilfe einer Fritte entfernt. Nach Entfernen des Lösungsmittels wurden 420 mg eines öligen Rückstandes (theoretische Ausbeute 424 mg) erhalten, der weitgehend aus dem gewünschten Produkt R bestand und ohne weitere Reinigung verwendet wurde.

### 5. Synthese von 6-Methyl-3-phenyl-1-oxa-2,8-diazaspiro[4.5]dec-2-en (U)

Die Synthese von 6-Methyl-3-phenyl-1-oxa-2,8-diazaspiro[4.5]dec-2-en ist in Schema 6. verdeutlicht.

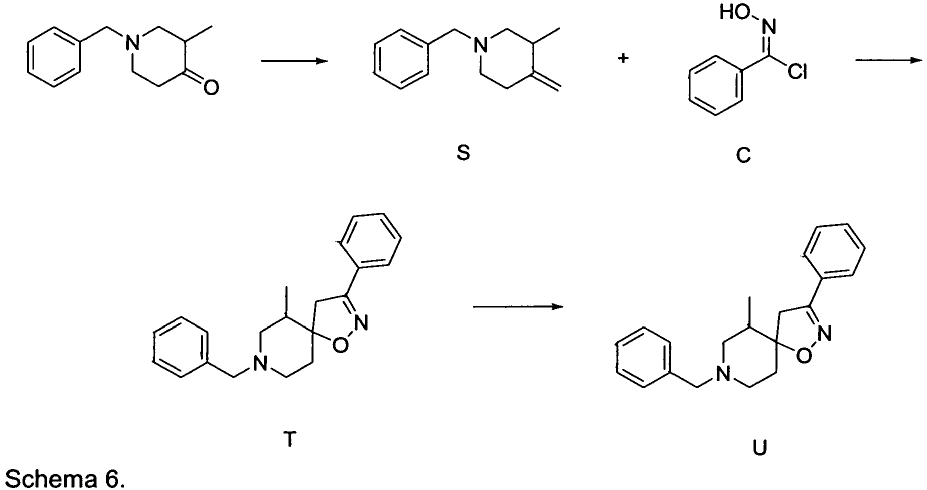

### Synthese von 1-Benzyl-3-methyl-4-methylenpiperidin (S)

Zu einer Suspension von Methyltriphenylphosphoniumbromid (7.5 g, 21 mmol) in trockenem THF (20 ml) wurde unter Argon bei 0 °C Kalium-*tert*-butylat (2.1 g, 18.8 mmol, gelöst in 20 ml abs. THF) innerhalb von 30 min gegeben. Das Reaktionsgemisch wurde 30 min bei 0 °C gerührt, bevor 1-Benzyl-3-methyl-piperidin-4-on (3.05 g, 15 mmol, gelöst in abs. THF (20 ml)) innerhalb von 30 min bei 0 °C zu getropft wurde. Das Reaktionsgemisch wurde 30 min bei 0 °C gerührt und innerhalb von 1 h auf RT gebracht und weitere 15 h gerührt. Zur Aufarbeitung wurde der Ansatz auf ges. NH₄Cl-Lsg. (50 ml) gegeben und anschließend mit EtOAc (3 x 20 ml) extrahiert. Das gewünschte Produkt S wurde durch Extraktion mit 2 N HCl-Lsg. (20 ml) in das entsprechende Hydrochlorid überführt. Die organische Phase wurde mit Wasser (3 x 20 ml) gewaschen und die vereinigten wäßrigen Phasen wurden mit K₂CO₃ basisch gemacht. Das Produkt S fiel in Form einer Ölschicht an und wurde durch erneute Extraktion mit EtOAc (3 x 20 ml) abgetrennt. Nach Trocknen der vereinigten organischen Phasen über Na₂SO₄ und Entfernung des Lösungsmittels lag das gewünschte Produkt S in ausreichend reiner Form (2.50 g, 83 %).

### Synthese von 8-Benzyl-6-methyl-3-phenyl-1-oxa-2,8-diazaspiro[4.5]dec-2-en (T)

Zu einer Lösung von Verbindung S (2.5 g, 14.45 mmol) in abs. DCM (25 ml) wurde Verbindung C (4.49 g, 28.9 mmol, gelöst in abs. DCM (25 ml)) bei 0 °C innerhalb von 10 min getropft. Zu dieser Mischung wurde bei 0 °C eine Lösung von Triethylamin (16 ml, 11.7 g, 115.6 mmol) in abs. DCM (25 ml) innerhalb von 10 min gegeben. Da die Reaktion stark exotherm war, wurde in einem Gemisch aus Eis/Kochsalz gekühlt. Die Reaktionsmischung wurde 16 h bei RT gerührt. Zur Aufarbeitung des Ansatzes wurde DCM (50 ml) und Wasser (70 ml) hinzugefügt. Die Phasen wurden getrennt. Die wäßrige Lösung wurde mit DCM (2 x 20 ml) extrahiert. Die vereinigten organischen Phasen wurden nacheinander mit Wasser (50 ml) und ges. NaCl-Lsg. (50 ml) gewaschen und anschließend getrocknet und eingeengt (4.9 g, gelbes Öl). Der Rückstand wurde durch Säulenchromatographie gereinigt [Kieselgel 60 (500 g); EtOAc/Cyclohexan 1:2 (3 l)]. Das gewünschte Produkt wurde als Diastereomerengemisch in einer Ausbeute von (2.28 g, 50 %) mit einem Schmelzpunkt von 101-108 °C erhalten.

### Synthese von 6-Methyl-3-phenyl-1-oxa-2,8-diazaspiro[4.5]dec-2-en (U)

Verbindung T (600 mg, 1.65 mmol) wurde in MeOH (50 ml) mit Palladiumkatalysator (Pd/C, 5 %, 500 mg) versetzt und 30 min bei RT einem Wasserstoffdruck von 3 bar ausgesetzt. Der Katalysator wurde mit Hilfe einer Fritte entfernt. Nach Entfernen des Lösungsmittels wurden 340 mg (∼ 90 % Ausbeute) eines öligen Rückstandes erhalten, der weitgehend aus dem gewünschten Produkt U bestand und ohne weitere Reinigung verwendet wurde.

### 6. Synthese von 3-Benzyl-1-oxa-2,8-diazaspiro[4.5]dec-2-en (Y)

Die Synthese von 3-Benzyl-1-oxa-2,8-diazaspiro[4.5]dec-2-en ist im nachfolgenden Schema 7 wieder gegeben.

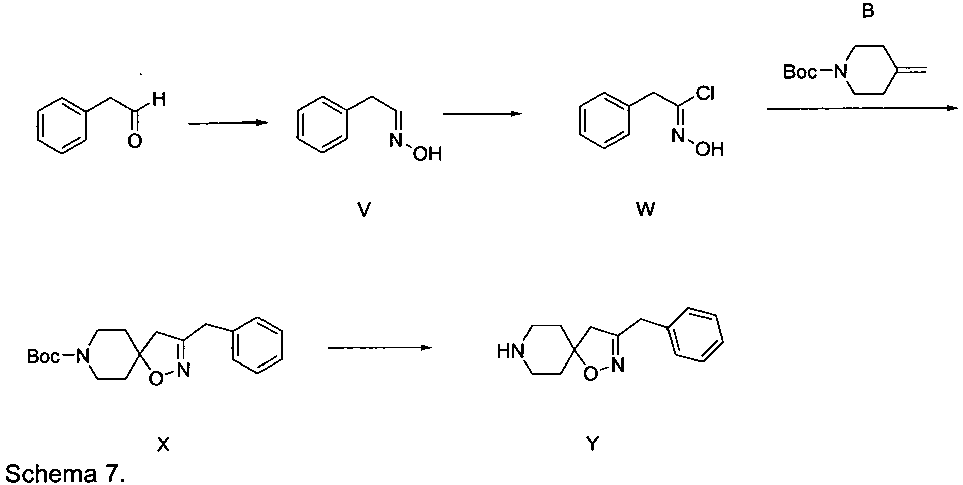

### Synthese von Phenylacetaldehyd-oxim (V)

Phenylacetaldehyd (12.0 g, 7.8 ml, 0.1 mol) wurde in EtOH (360 ml) gelöst und unter Rühren nacheinander mit Hydroxylamin-hydrochlorid (10.4 g, 0.15 mol) und abs. Pyridin (9.7 ml, 0.12 mol) versetzt. Nach 1.5 h Rühren bei RT wurde die Reaktionsmischung eingeengt, der Rückstand in Toluol (1 × 100 ml) aufgenommen und das Lösungsmittel erneut im Vakuum entfernt. Der ölige Rückstand wurde mit EtOAc (350 ml) und Wasser (90 ml) 15 min gerührt. Anschließend wurden die Phasen getrennt. Die organische Phase wurde mit Wasser (2 × 80 ml) gewaschen, getrocknet und eingeengt. Das erhaltene Öl kristallisierte nach kurzer Zeit. Es wurde aus *n*-Hexan (20 ml) umkristallisiert. Verbindung V wurde in einer Ausbeute von 31 % (4.13 g) als weißer Feststoff mit einem Schmelzpunkt von 90 - 94 °C erhalten.

### Synthese von Phenylacetohydroximoylchlorid (W)

Zu einer Lösung von Verbindung V (2.7 g, 20 mmol) in abs. DMF (12 ml) wurde eine Lösung von *N*-Chlorsuccinimid (3.25 g, 24 mmol) in abs. DMF (18 ml) bei einer Temperatur von 30 - 40 °C innerhalb von 20 min gegeben. Bei Bedarf wurde im Eisbad gekühlt. Die Reaktionsmischung wurde 3 h bei RT gerührt und anschließend mit Diethylether (175 ml) und Wasser (115 ml) versetzt. Die Phasen wurden getrennt und die wäßrige Phase wurde mit Diethylether (2 × 75 ml) extrahiert. Die vereinigten organischen Extrakte wurden nacheinander mit Wasser (100 ml) und ges. NaCl-Lsg. (100 ml) gewaschen. Die organische Phase wurde getrocknet und eingeengt. Verbindung W wurde als gelb-grünes Öl (3.33 g) erhalten und sofort weiter umgesetzt.

### Synthese von 3-Benzyl-1-oxa-2,8-diazaspiro[4.5]dec-2-en-8-carbonsäure-tert-butyl ester (X)

Verbindung W (3.33 g, 20 mmol), gelöst in abs. DCM (15 ml), wurde bei 0 °C innerhalb von 10 min zu einer Lösung von Verbindung B (2.81 g, 14.3 mmol in abs. DCM (22 ml)) getropft. Zu dieser Mischung wurde bei 0 °C eine Lösung von Triethylamin (11.2 ml, 80 mmol) in abs. DCM (10 ml) innerhalb von 10 min gegeben. Da die Reaktion stark exotherm war, wurde mit einem Gemisch aus Eis und Kochsalz gekühlt. Die Reaktionsmischung wurde 16 h bei RT gerührt. Zur Aufarbeitung des Ansatzes wurde DCM (15 ml) und Wasser (45 ml) hinzugefügt und 15 min gerührt. Nach Phasentrennung wurde die wäßrige Phase mit DCM (2 × 45 ml) extrahiert. Die Phasen wurden getrennt. Die organische Phase wurde nacheinander mit 10%-iger Zitronensäure-Lsg. (2 × 30 ml) und ges. NaCl-Lsg. (30 ml) gewaschen. Die organische Phase wurde getrocknet und eingeengt (5.3 g, braunes Öl). Der Rückstand wurde durch Chromatographie [Kieselgel 60 (180 g); Cyclohexan/EtOAc 7 : 1 (1200 ml); 5 : 1 (900 ml)] aufgetrennt. Das gewünschte Produkt X wurde in einer Ausbeute von 681 mg (11 %) erhalten.

### Synthese von 3-Benzyl-1-oxa-2,8-diazaspiro[4.5]dec-2-en (Y)

Verbindung X (670 mg, 2.0 mmol) wurde in DCM (20 ml) gelöst und mit einer 5,5 N Salzsäure-Lsg. in Propan-2-ol (7.3 ml, 40 mmol) versetzt. Nach einer Reaktionszeit von 4 h wurde der Ansatz auf ca. 10 ml eingeengt und anschließend im Eisbad gerührt. Der ausgefallene Feststoff wurde abgesaugt und mit Diethylether gewaschen. Es wurden 395 mg (74 %, Fp. 198-204 °C) des Hydrochlorids von 3-Benzyl-1-oxa-2,8-diazaspiro[4.5]dec-2-en erhalten.

Das Hydrochlorid (340 mg, 1.27 mmol) wurde mit DCM (20 ml) und ges. NaHCO₃-Lsg. (10 ml) 1 h gerührt. Nach Phasentrennung wurde die wäßrige Phase mit DCM (2 x 10 ml) extrahiert. Die organischen Extrakte wurden getrocknet und eingeengt. 3-Benzyl-1-oxa-2,8-diazaspiro[4.5]dec-2-en wurde in einer Ausbeute von 98 % (286.5 mg) mit einem Schmelzpunkt von 51-54 °C erhalten.

### 7. Synthese von 3-Phenethyl-1-oxa-2,8-diazaspiro[4.5]dec-2-en (CC)

Die Synthese von 3-Phenethyl-1-oxa-2,8-diazaspiro[4.5]dec-2-en ist im nachfolgenden Schema 8 wieder gegeben.

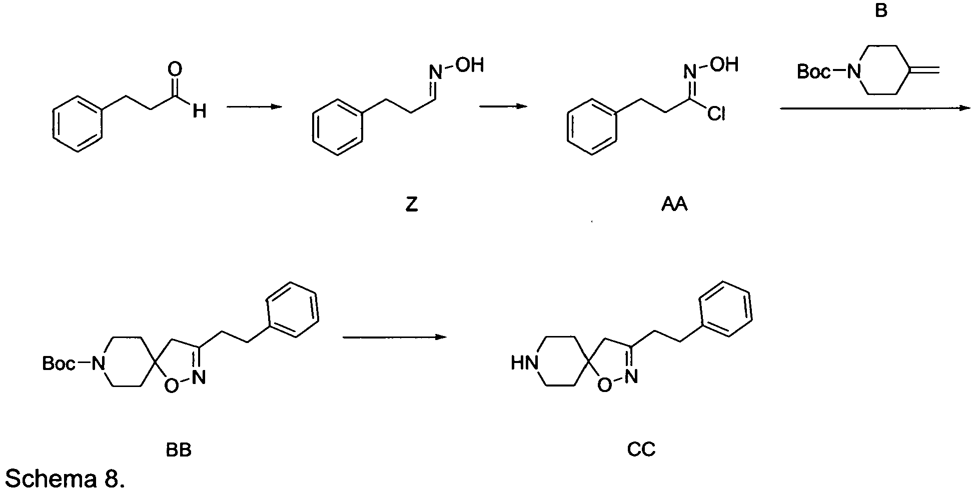

### Synthese von 3-Phenylpropionaldehyd-oxim (Z)

Phenylpropionaldehyd (3.35 g, 3.3 ml, 25 mmol) wurde in EtOH (90 ml) gelöst und unter Rühren nacheinander mit Hydroxylamin-hydrochlorid (2.6 g, 37.5 mmol) und abs. Pyridin (2.42 ml, 30 mmol) versetzt. Nach 4 h Rühren wurde das Lösungsmittel im Vakuum entfernt, der Rückstand in Toluol (1 × 50 ml) aufgenommen und das Lösungsmittel erneut im Vakuum entfernt. Der ölige Rückstand wurde mit EtOAc (50 ml) und Wasser (30 ml) 15 min gerührt. Anschließend wurden die Phasen getrennt. Die organische Phase wurde mit Wasser (2 × 20 ml) gewaschen, getrocknet und eingeengt. Das erhaltene Öl (4.71 g) war stark verunreinigt und kristallisierte nur teilweise. Es wurde in *n*-Hexan (20 ml) gelöst und 18 h bei 5 °C aufbewahrt. Die ausgefallenen Plättchen wurden abgesaugt. Verbindung Z wurde in einer Ausbeute von 24 % (892 mg) als weißer Feststoff mit einem Schmelzpunkt von 85-93 °C erhalten.

### Synthese von 3-Phenylpropanhydroximoylchlorid (AA)

Zu einer Lösung von Verbindung Z (2.06 g, 13.8 mmol) in abs. DMF (10 ml) wurde eine Lösung von *N*-Chlorsuccinimid (2.24 g, 16.6 mmol) in abs. DMF (11 ml) bei einer Temperatur von 30-40 °C innerhalb von 20 min gegeben. Bei Bedarf wurde im Eisbad gekühlt. Die Reaktionsmischung wurde 3 h bei RT gerührt und anschließend mit Diethylether (120 ml) und Wasser (80 ml) versetzt. Die Phasen wurden getrennt. Die wäßrige Phase wurde mit Diethylether (2 × 50 ml) extrahiert. Die vereinigten organischen Extrakte wurden nacheinander mit Wasser (80 ml) und ges. NaCl-Lsg. (100 ml) gewaschen. Die organische Phase wurde getrocknet und eingeengt. 3-Phenylpropanhydroximoylchlorid wurde als hellgrünes Öl (2.47 g) erhalten und sofort weiter umgesetzt.

### Synthese von 3-Phenethyl-1-oxa-2,8-diazaspiro[4.5]dec-2-en-8-carbonsäure-tert-butyl ester (BB)

Verbindung BB (2.47 g, 13.8 mmol), gelöst in abs. DCM (10 ml), wurde bei 0 °C innerhalb von 10 min zu einer Lösung von Verbindung B (1.94 g, 9.85 mmol) in abs. DCM (15 ml) getropft. Zu dieser Mischung wurde bei 0 °C eine Lösung von Triethylamin (7.75 ml, 55.2 mmol) in abs. DCM (10 ml) innerhalb von 10 min gegeben. Da die Reaktion stark exotherm war, wurde in einer Eis/Kochsalz-Mischung gekühlt. Die Reaktionsmischung wurde 16 h bei RT gerührt. Zur Aufarbeitung des Ansatzes wurde DCM (10 ml) und Wasser (30 ml) hinzugefügt und 15 min gerührt. Nach Phasentrennung wurde die wäßrige Phase mit DCM (2 × 45 ml) extrahiert. Die Phasen wurden getrennt. Die organische Phase wurde nacheinander mit 10%-iger Zitronensäure-Lsg. (2 × 20 ml) und ges. NaCl-Lsg. (20 ml) gewaschen. Die organische Phase wurde getrocknet und eingeengt. Es wurden 3.88 g eines hellbraunen Öls erhalten, das langsam kristallisierte. Das Rohprodukt wurde mit Cyclohexan (20 ml) und EtOAc (3 ml) bis zur vollständigen Lösung erwärmt und anschließend im Eisbad abgekühlt. Der ausgefallene Feststoff wurde abgesaugt und mit Cyclohexan (10 ml) gewaschen. Das gewünschte Produkt BB wurde in einer Ausbeute von 26 % (1.22 g) mit einem Schmelzpunkt von 121-124 °C als weißer Feststoff erhalten.

### Synthese von 3-Phenethyl-1-oxa-2,8-diazaspiro[4.5]dec-2-en (CC)

Verbindung BB (1.1 g, 3.2 mmol) wurde in DCM (20 ml) gelöst und mit einer 5,5 N Lösung von Chlorwasserstoff in Propan-2-ol (11.6 ml, 64 mmol) versetzt. Nach einer Reaktionszeit von 2 h wurde der Ansatz auf ca. 10 ml eingeengt und anschließend im Eisbad gerührt. Der ausgefallene Feststoff wurde abgesaugt und mit Diethylether gewaschen. Es wurden 799 mg (89 %, Fp. 252-254 °C) des Hydrochlorids von 3-Phenethyl-1-oxa-2,8-diazaspiro[4.5]dec-2-en erhalten.

Das Hydrochlorid (733 mg, 2.6 mmol) wurde mit DCM (30 ml) und ges. NaHCO₃-Lsg. (10 ml) 1.5 h gerührt. Nach Phasentrennung wurde die wäßrige Phase mit DCM (2 × 10 ml) extrahiert. Die vereinigten organischen Extrakte wurden getrocknet und eingeengt. 3-Phenethyl-1-oxa-2,8-diazaspiro[4.5]dec-2-en wurde in einer Ausbeute von 97 % (617 mg) mit einem Schmelzpunkt von 106-109 °C erhalten.

### Synthese von 3-(4-Chlorphenyl)-1-oxa-2,8-diazaspiro[4.5]dec-2-en-8-carbonsäure-tert-butylester

Das Chlorid 4-Chlorphenylhydroxymoylchlorid (14,7 g, 77,3 mmol), gelöst in abs. DCM (120 ml), wurde bei 0 °C innerhalb von 10 min zu einer Lösung des Olefins tert-Butyl 4-methylenpiperidin-1-carboxylat (11,5 g, 58,5 mmol) in abs. DCM (80 ml) getropft. Zu dieser Mischung wurde bei 0 °C eine Lösung von Triethylamin (47 ml, 33,8 g, 334 mmol) in abs. DCM (50 ml) innerhalb von 15 min gegeben. Da die Reaktion stark exotherm war, wurde mit Eis/Kochsalz gekühlt. Während der Zugabe fiel Triethylaminhydrochlorid aus. Die Reaktionsmischung wurde 16 h bei RT gerührt. Zur Aufarbeitung des Ansatzes wurde DCM (30 ml) und Wasser (130 ml) hinzugefügt. Die Phasen wurden getrennt. Die organische Phase wurde nacheinander mit 10%-iger aq. Zitronensäure-Lsg. (2 (150 ml) und ges. aq. NaCl-Lsg. (100 ml) extrahiert. Die organische Phase wurde getrocknet und eingeengt (22,8 g, gelber Feststoff). Der Rückstand wurde mit Diethylether (100 ml) versetzt und 1 h bei RT gerührt. Es blieb ein Feststoff zurück, der durch Filtration abgetrennt und mit Diethylether (2 (40 ml) gewaschen wurde (10,49 g). Das Filtrat wurde auf 25 ml eingeengt und der dabei ausgefallene Feststoff abgetrennt (1,96 g). Beide Fraktionen wurden vereinigt. Die Spiroverbindung 3-(4-Chlorphenyl)-1-oxa-2,8-diazaspiro[4.5]dec-2-en-8-carbonsäure-tert-butylester wurde als farbloser Feststoff in einer Ausbeute von 61 % mit einem Schmelzpunkt von 138 - 141 °C gewonnen.

### Synthese von 3-(4-Chlorphenyl)-1-oxa-2,8-diazaspiro[4.5]dec-2-en-hydrochlorid

Die Spiroverbindung 3-(4-Chlorphenyl)-1-oxa-2,8-diazaspiro[4.5]dec-2-en-8-carbonsäure-tert-butylester (9,48 g, 27,0 mmol) wurde in DCM (150 ml) gelöst und mit einer 5 N aq. Salzsäure-Lsg. in Propan-2-ol (130 ml, 651 mmol) versetzt. Nach einer Reaktionszeit von 1.5 h wurde der Ansatz eingeengt. Der feste Rückstand wurde mit DCM (50 ml) versetzt, 15 min bei RT gerührt, abgesaugt und mit DCM (2 × 50 ml) gewaschen. Das Hydrochlorid 3-(4-Chlorphenyl)-1-oxa-2,8-diazaspiro[4.5]dec-2-en-hydrochlorid wurde als farblose Verbindung in einer Ausbeute von 95 % (7,33 g) mit einem Schmelzpunkt von 284-287 °C gewonnen.

### Synthese von 3-(4-tert-Butylphenyl)-1-oxa-2,8-diazaspiro[4.5]dec-2-en-8-carbonsäure-tert-butylester

Das Chlorid (4-tert-Butylphenyl)hydroxymoylchlorid (16,9 g, 83,1 mmol), gelöst in abs. DCM (60 ml), wurde bei 0 °C innerhalb von 10 min zu einer Lösung des Olefins tert-Butyl 4-methylenpiperidin-1-carboxylat (11,5 g, 58,5 mmol) in abs. DCM (80 ml) getropft. Zu dieser Mischung wurde bei 0 °C eine Lösung von Triethylamin (47 ml, 33,8 g, 334 mmol) in abs. DCM (50 ml) innerhalb von 15 min gegeben. Da die Reaktion stark exotherm war, wurde mit Eis/Kochsalz gekühlt. Während der Zugabe fiel Triethylamin-hydrochlorid aus. Die Reaktionsmischung wurde 16 h bei RT gerührt. Zur Aufarbeitung des Ansatzes wurde DCM (30 ml) und Wasser (130 ml) hinzugefügt. Die Phasen wurden getrennt. Die organische Phase wurde nacheinander mit 10%-iger aq. Zitronensäure-Lsg. (2 x 150 ml) und ges. aq. NaCl-Lsg. (150 ml) extrahiert. Die organische Phase wurde getrocknet und eingeengt (24,0 g, hellbrauner Feststoff). Der Rückstand wurde mit n-Hexan (50 ml) versetzt und 20 min bei RT gerührt. Es blieb ein Feststoff zurück, der durch Filtration abgetrennt und mit n-Hexan (3 x 30 ml) gewaschen wurde. Die Spiroverbindung 3-(4-tert-Butylphenyl)-1-oxa-2,8-diazaspiro[4.5]dec-2-en-8-carbonsäure-tert-butylester wurde als farbloser Feststoff in einer Ausbeute von 61 % mit einem Schmelzpunkt von 172-173 °C gewonnen.

### Synthese von 3-(3-Fluor-4-methansulfonylaminomethylphenyl)-1-oxa-2,8-diaza-spiro-[4.5]dec-2-en-8-carbonsäure-tert-butylester

Das Chlorid 3-Fluor-4-methansulfonylaminophenyl-hydroxymoylchlorid (6,16 g, 23,09 mmol), gelöst in abs. THF (50 ml), wurde bei 0 °C innerhalb von 15 min zu einer Lösung des Olefins tert-Butyl 4-methylenpiperidin-1-carboxylat (3,24 g, 16,5 mmol) in abs. THF (30 ml) getropft. Zu dieser Mischung wurde bei 0 °C eine Lösung von Triethylamin (13 ml, 9,4 g, 92,3 mmol) in abs. THF (15 ml) innerhalb von 10 min gegeben. Da die Reaktion stark exotherm war, wurde mit Eis/Kochsalz gekühlt. Während der Zugabe fiel Triethylamin-hydrochlorid aus. Die Reaktionsmischung wurde 3 d bei RT gerührt. Zur Aufarbeitung des Ansatzes wurde Wasser (60 ml) hinzugefügt und THF im Vakuum entfernt. Nach der Zugabe von DCM (100 ml) und Wasser (20 ml) entstand ein brauner Feststoff, der vor der Phasentrennung abgetrennt wurde. Die organische Phase wurde mit 10%-iger Zitronensäure-Lsg. (100 ml) versetzt, wobei nochmals ein brauner Feststoff ausfiel der sich erst nach 4-stündigem Rühren durch Filtration abtrennen ließ. Die Phasen des Filtrats wurden getrennt. Die organische Phase wurde nacheinander mit 10%-iger Zitronensäure-Lsg. (100 ml) und ges. aq. NaCl-Lsg. (100 ml) extrahiert. Die organische Phase wurde getrocknet und eingeengt (6,1 g, hellbraunes Öl). Der Rückstand wurde chromatographisch aufgetrennt. [Kieselgel 60 (300 g); EtOAc/Cyclohexan 1 : 2 (3,2 l), 1 : 1 (2 l)]. Die Spiroverbindung 3-(3-Fluor-4-methansulfonylaminomethylphenyl)-1-oxa-2,8-diazaspiro-[4.5]dec-2-en-8-carbonsäure-tert-butylester wurde als beigefarbener Feststoff in einer Ausbeute von 1,4 g, (20 %) mit einem Schmelzpunkt von 178-182 °C gewonnen.

### Synthese von N-[2-Fluor-4-(1-oxa-2,8-diazaspiro[4.5]dec-2-en-3-yl)phenyl]methan-sulfonamid Hydrochlorid

Die Spiroverbindung 3-(3-Fluor-4-methansulfonylaminomethylphenyl)-1-oxa-2,8-diazaspiro-[4.5]dec-2-en-8-carbonsäure-tert-butylester (1,38 g, 3,23 mmol) wurde in DCM (30 ml) gelöst und mit einer 5 N aq. Salzsäure-Lsg. in Propan-2-ol (12,9 ml, 64,7 mmol) versetzt. Nach einer Reaktionszeit von 2 h wurde das Hydrochlorid N-[2-Fluor-4-(1-oxa-2,8-diazaspiro[4.5]dec-2-en-3-yl)phenyl]methan-sulfonamid Hydrochlorid durch Filtration abgetrennt und mit DCM (2 (10 ml) gewaschen (0,878 g). Das Filtrat wurde mit Diethylether (50 ml) versetzt und 30 min gerührt. Dabei fiel noch weiteres Hydrochlorid aus. Das Hydrochlorid N-[2-Fluor-4-(1-oxa-2,8-diazaspiro[4.5]dec-2-en-3-yl)phenyl]methan-sulfonamid Hydrochlorid wurde als farblose Verbindung in einer Gesamtausbeute von 87 % (1,02 g) mit einem Schmelzpunkt von 269-274 °C gewonnen (AS 4492).

### Synthese von 8-Benzyl-3-phenyl-1-oxa-2,8-diazaspiro[4.6]undec-2-en

Zu einer Lösung des 1-Benzyl-4-methylenazepan (0,94 g, 4,7 mmol) in abs. DCM (25 ml) wurde bei 0°C innerhalb von 10 min das Chlorid 1-Chlor-1-hydroxyimino-methylbenzol (1,4 g, 9,4 mmol), gelöst in abs. DCM (25 ml), getropft. Zu dieser Mischung wurde bei 0 °C eine Lösung von Triethylamin (5,2 ml, 3,8 g, 54,4 mmol) in abs. DCM (25 ml) innerhalb von 10 min gegeben. Da die Reaktion stark exotherm war, wurde mit Eis/Kochsalz gekühlt. Während der Zugabe fiel Triethylamin-hydrochlorid aus. Die Reaktionsmischung wurde 24 h bei RT gerührt. Zur Aufarbeitung des Ansatzes wurde DCM (30 ml) und Wasser (40 ml) hinzugefügt. Die Phasen wurden getrennt. Die wäßrige Lösung wurde mit DCM (2 x 20 ml) extrahiert. Die vereinigten organischen Phasen wurden nacheinander mit Wasser (50 ml) und ges. aq. NaCl-Lsg. (50 ml) gewaschen, anschließend getrocknet und eingeengt (2,1 g, gelbes Öl). Der Rückstand wurde durch Säulenchromatographie gereinigt. [Kieselgel 60 (120 g); EtOAc/Cyclohexan 1 : 1 (1,5 l)] Die Spiroverbindung 8-Benzyl-3-phenyl-1-oxa-2,8-diazaspiro[4.6]undec-2-en wurde als beiger Feststoff in einer Ausbeute von 0,9 g (60 %) mit einem Schmelzpunkt von 42-57 °C gewonnen.

### Synthese von 3-Phenyl-1-oxa-2,8-diazaspiro[4.6]undec-2-en-8-carbonsäure-benzylester

Unter Feuchtigkeitsausschluß wurde zu einer Lösung der Benzylverbindung 8-Benzyl-3-phenyl-1-oxa-2,8-diazaspiro[4.6]undec-2-en (830 mg, 2,6 mmol) in abs. Toluol (30 ml) unter Rühren bei RT Chlorameisensäure-benzylester (0,7 ml, 5 mmol) innerhalb von 10 min gegeben. Obwohl im DC bereits nach 2 h kaum noch Ausgangsmaterial nachweisbar war, wurde der Ansatz weitere 15 h bei RT belassen. Zur Aufarbeitung wurde das Reaktionsgemisch zunächst mit Et₃N (2 ml, 14,5 mmol) versetzt und 1 h bei RT gerührt. Anschließend wurde zu dem Reaktionsgemisch 1 N HCl (30 ml) gegeben. Die organische Phase wurde abgetrennt und die wäßrige Lösung mit EtOAc (3 x 20 ml) extrahiert. Die vereinigten organischen Phasen wurden mit H₂O (10 ml) sowie ges. aq. NaHCO₃-Lösung (10 ml) gewaschen, über Na₂SO₄ getrocknet und anschließend eingeengt. Durch chromatographische Reinigung (Laufmittel: Cyclohexan/EtOAc 4 : 1) des Rohproduktes wurde 3-Phenyl-1-oxa-2,8-diazaspiro[4.6]undec-2-en-8-carbonsäure-benzylester in einer Ausbeute von 823 mg (97 %) als farbloses Öl erhalten.

### Synthese von 3-Phenyl-1-oxa-2,8-diazaspiro[4.6]undec-2-en

Das Amin 3-Phenyl-1-oxa-2,8-diazaspiro[4.6]undec-2-en-8-carbonsäure-benzylester (770 mg, 2,11 mmol) wurde in MeOH (50 ml) mit dem Palladiumkatalysator (Pd/C, 5 %, 500 mg) versetzt und 45 min bei RT hydriert (Wasserstoffdruck: 2 bar). Der Katalysator wurde mit Hilfe einer Fritte, versehen mit einer 1 cm hohen Schicht Celite, entfernt. Das Lösungsmittel wurde im Vakuum abdestilliert. Das Amin 3-Phenyl-1-oxa-2,8-diazaspiro[4.6]undec-2-en wurde so in einer Ausbeute von 434 mg (89 %) als hellgelbes Öl erhalten.

### 8. Allgemeine Vorschrift zur Umsetzung von Aminen der allgemeinen Formel II mit Isocyanaten bzw. Thioisocyanaten der allgemeinen Formel R⁵-N=C=O bzw. R⁷-N=C=S

### a. Manuelle Synthese

### Allgemeine Vorschrift:

Zu einer Lösung des Amins (2 mmol) in abs. THF (20 mL) wurde unter Argon bei RT das Isocyanat (2 mmol) innerhalb von 10 min gegeben. Der Ansatz wurde 3 h gerührt und anschließend mit 2 N aq. HCl (10 ml) versetzt. Die saure Reaktionsmischung wurde 10 min gerührt und anschließend mit EtOAc (3 x 20 mL) extrahiert. Die vereinigten organischen Phasen wurden mit ges. aq. NaHCO₃-Lsg. (10 mL) gewaschen, über Na₂SO₄ getrocknet und im Vakuum bis zur Trockne eingeengt. Der Rückstand wurde aus einem geeigneten Lösungsmittel (15 mL, Hexan, Toluol oder DCM) umkristallisiert oder säulenchromatographisch (SiO₂, Hexan/EtOAc in verschiedenen Mischungen) gereinigt.

### Synthese von Verbindung 390: 3-(3-Chlor-pyridin-2-yl)-1-oxa-2,8-diaza-spiro[4.5]dec-2-en-8-carbonsäure-(4-tert-butyl-phenyl)-amid

Verbindung K (0.130 g, 0.451 mmol) wurde in Toluol (15 ml) gelöst und nacheinander wurden Triethylamin (0.12 ml, 0.867 mmol) und 4-tert-Butylphenylisocyanat (0.08 ml, 0.451 mmol) zugesetzt. Die Reaktionsmischung wurde auf Siedetemperatur erhitzt und 3 h am Rückfluß gekocht. Nach Abkühlen der Reaktionsmischung auf RT wurde 36 h gerührt. Die Reaktionsmischung wurde mit EtOAc (3 x 20 ml) extrahiert. Die vereinigten organischen Phasen wurden mit ges. Na₂HCO₃-Lsg. (10 ml) gewaschen, über Na₂SO₄ getrocknet und im Vakuum bis zur Trockne eingeengt. Nach säulenchromatographischer Reinigung (Hexan:EtOAc1:1) des Rückstandes wurde das Produkt in 83% Ausbeute (0.160 g) erhalten.

### Synthese von Beispielverbindung 438: 3-Thiazol-2-yl-1-oxa-2,8-diaza-spiro[4.5]dec-2-en-8-carbonsäure-(4-tert-butyl-phenyl)-amid

Verbindung O (0.150 g, 0.578 mmol) wurde in Toluol (15 ml) gelöst und nacheinander mit Triethylamin (0.12 ml, 0.867 mmol) und 4-tert-Butylphenylisocyanat (0.102 ml, 0.578 mmol) versetzt. Die Reaktionsmischung wurde auf Siedetemperatur erhitzt und 3 h am Rückfluß gekocht. Nach Abkühlen auf RT wurde 36 h gerührt. Die Reaktionsmischung wurde mit EtOAc (3 x 20 ml) extrahiert. Die vereinigten organischen Phasen wurden mit ges. Na₂HCO₃-Lsg. (10 ml) gewaschen, über Na₂SO₄ getrocknet und im Vakuum bis zur Trockne eingeengt. Nach säulenchromatographischer Reinigung (Hexan:EtOAc 1:1) des Rückstandes wurde das gewünschte Produkt in 52% Ausbeute (0.120 g) erhalten

### Synthese von Beispielverbindung 370: 2-(4-tert-Butylphenyl)-1-(3-phenyl-1-oxa-2,8-diazaspiro[4.5]dec-2-en-8-yl)propan-1-on

Das Hydrochlorid des Amins 3-Phenyl-1-oxa-2,8-diaza-spiro[4.5]dec-2-en (1,5 g, 5,9 mmol) wurde in Wasser (20 ml) gelöst, mit ges. aq. NaHCO₃-Lsg. (20 ml) versetzt und 1 h bei RT gerührt. Dabei fiel das Amin 3-Phenyl-1-oxa-2,8-diaza-spiro[4.5]dec-2-en aus. Es wurde durch Filtration abgetrennt und als farbloser Feststoff in einer Ausbeute von 92 % (1,17g) mit einem Schmelzpunkt von 115 °C gewonnen.
Eine Lösung der Säure 2-(4-tert-Butylphenyl)propionsäure (300 mg, 1,45 mmol) in abs. Dimethylformamid (25 ml) wurde mit N,N'-Carbonyldiimidazol (235 mg, 1,45 mmol) versetzt und 1,5 h bei Raumtemperatur gerührt. Anschließend erfolgte die Zugabe des Amins 3-Phenyl-1-oxa-2,8-diaza-spiro[4.5]dec-2-en (346 mg, 1,6 mmol). Nach einer Reaktionszeit von 4 d bei Raumtemperatur wurde die klare Reaktionsmischung eingeengt. Der Rückstand wurde in DCM (30 ml) und 0,5 N aq. Salzsäure (20 ml) aufgenommen Die Phasen wurden getrennt. Die organische Phase wurde nacheinander mit 0,5 N aq. Salzsäure (20 ml), ges. aq. NaHCO₃-Lsg. (2 x 15 ml) und ges. aq. NaCl-Lösung (15 ml) gewaschen. Die organische Phase wurde getrocknet und eingeengt. Der feste farblose Rückstand wurde in einem Gemisch aus Diethylether (10 ml) und n-Hexan (10 ml) aufgenommen und 15 min bei RT gerührt. Das Amid 2-(4-tert-Butylphenyl)-1-(3-phenyl-1-oxa-2,8-diazaspiro[4.5]dec-2-en-8-yl)propan-1-on wurde als farbloser Feststoff in einer Ausbeute von 62 % (364 mg) mit einem Schmelzpunkt von 143-145 °C gewonnen.

### Synthese von Beispielverbindung 371: N-(4-tert-Butylphenyl)-3-phenyl-1-oxa-2,7-diazaspiro[4.4jnon-2-en-7-carboxamid

Zu einer Lösung von Verbindung R (420 mg Rohprodukt, ca. 2 mmol) in abs. THF (20 ml) wurde unter Argon bei RT 4-tert-Butylphenylisocyanat (350 mg, 2 mmol) innerhalb von 10 min gegeben. Nach 1 h wurde die trübe Lösung mit Triethylamin (0.3 ml, 2,22 mmol) versetzt. Die Reaktionsmischung wurde weitere 2 h gerührt und anschließend mit 2 N HCl-Lsg. (10 ml) versetzt. Die saure Reaktionsmischung wurde 10 min gerührt und anschließend mit EtOAc (3 x 20 ml) extrahiert. Die vereinigten organischen Phasen wurden mit ges. Na₂HCO₃-Lsg. (10 ml) gewaschen, über Na₂SO₄ getrocknet und im Vakuum bis zur Trockne eingeengt. Der Rückstand wurde aus Toluol (15 ml) umkristallisiert. Das gewünschte Produkt N-(4-tert-Butylphenyl)-3-phenyl-1-oxa-2,7-diazaspiro[4.4]non-2-en-7-carboxamid wurde so in einer Ausbeute von 415 mg (55 %) mit einem Schmelzpunkt von 207 - 208 °C (aus EtOH, Kristallumwandlung zwischen 183 und 188 °C) erhalten.

### Synthese von Beispielverbindung 373: 3-Phenyl-1-oxa-2,8-diazaspiro[4.6]undec-2-en-8-carbonsäure-(4-tert-butylphenyl)amid

Zu einer Lösung des Amins 3-Phenyl-1-oxa-2,8-diazaspiro[4.6]undec-2-en (430 mg, 1,87 mmol) in abs. THF (20 ml) wurde unter Argon bei RT das Isocyanat 4-tert-Butyl-phenyl-isocyanat [350 mg, 2 mmol, gelöst in abs. THF (1 ml)] innerhalb von 10 min gegeben. Der Ansatz wurde 30 min bei RT gerührt, mit Triethylamin (0.4 ml, 2,8 mmol) versetzt und anschließend weitere 2 h gerührt. Zur Aufarbeitung wurde zu dem Reaktionsgemisch 1 N aq. HCl (30 ml) gegeben. Die saure Reaktionsmischung wurde 10 min gerührt und anschließend mit EtOAc (3 x 20 ml) extrahiert. Die vereinigten organischen Phasen wurden mit gesättigten NaCl- und NaHCO₃-Lösungen (je 10 ml) gewaschen, über Na₂SO₄ getrocknet und im Vakuum bis zur Trockne eingeengt. Der erhaltene Rückstand wurde mittels Flash-Chromatographie (Laufmittel: a: Cyclohexan/EtOAc = 3 : 1; b: Cyclohexan/EtOAc = 1 : 1) gereinigt. Die Verbindung 3-Phenyl-1-oxa-2,8-diazaspiro[4.6]undec-2-en-8-carbonsäure-(4-tert-butylphenyl)amid (270 mg, Schmelzpunkt: 137-142 °C) wurde mit 34 % Ausbeute erhalten.

### Synthese von Beispielverbindung 379: 8-(5-tert-Butyl-1H-benzimidazol-2-yl)-3-phenyl-1-oxa-2,8-diaza-spiro[4.5]dec-2-en

Die Chlorverbindung 5-tert-Butyl-2-chlor-1H-benzimidazol (104,3 mg, 0,5 mmol) und das Amin 3-Phenyl-1-oxa-2,8-diaza-spiro[4.5]dec-2-en (108,2 mg, 0,5 mmol) wurden in Dimethylformamid (5 ml) gelöst. Da die Stabilität der Reaktanten nicht bekannt war, wurde erst 2 d bei RT gerührt, dann die Temperatur 6 h auf 80 °C erhöht und danach bei 140 °C 15 h unter Rühren erhitzt. Erst bei höherer Temperatur wurde im DC eine Umsetzung beobachtet. Zur Aufarbeitung wurde das klare dunkelbraune Reaktionsgemisch mit Wasser (30 ml) und Diethylether (20 ml) versetzt. Die Phasen wurden getrennt. Die wäßrige Phase wurde mit Dieethylether (3 × 15 ml) extrahiert. Die Extrakte wurden vereinigt, mit ges. aq. NaCl-Lösung (5 ml) gewaschen und dann mit wasserfreiem Na₂SO₄ getrocknet. Das Lösungsmittel wurde abdestilliert. Der Rückstand wurde durch Flash-Chromatographie [Kieselgel 60 (20 g); Eluent: Cyclohexan/EtOAc 1 : 1 (400 ml), 1 : 3 (400 ml)] gereinigt. Dabei wurden zwei neue Produkte isoliert, ein unpolareres Produkt 8-(5-tert-Butyl-1H-benzimidazol-2-yl)-3-phenyl-1-oxa-2,8-diaza-spiro[4.5]dec-2-en (60,0 mg, Fp. 230-236 °C, 31 %) und ein polareres Produkt 8-(5-tert-Butyl-1H-benzimidazol-2-yl)-3-phenyl-1-oxa-2,8-diaza-spiro[4.5]dec-2-en (57,8 mg, Fp. 112 °C, 30 %). Die gewünschte Struktur wurde analytisch nur für den unpolareren Anteil 8-(5-tert-Butyl-1H-benzimidazol-2-yl)-3-phenyl-1-oxa-2,8-diaza-spiro[4.5]dec-2-en bestätigt.

### Synthese von Beispielverbindung 369: 6-Methyl-3-phenyl-1-oxa-2,8-diaza-spiro[4.5]dec-2-en-8-carbonsäure-(4-tert-butyl-phenyl)-amid

Zu einer Lösung von Verbindung U (320 mg Rohprodukt, ca. 1,39 mmol) in abs. THF (20 ml) wurde unter Argon bei RT 4-tert-Butylphenylisocyanat [260 mg, 1,48 mmol, gelöst in abs. THF (1 ml)] innerhalb von 10 min gegeben. Der Ansatz wurde zwei Stunden gerührt und anschließend mit 2N HCl-Lsg. (10 ml) versetzt. Die saure Reaktionsmischung wurde 10 min gerührt und anschließend mit EtOAc (3 x 20 ml) extrahiert. Die vereinigten organischen Phasen wurden mit ges. NaHCO₃-Lsg. (10 ml) gewaschen, über Na₂SO₄ getrocknet und im Vakuum bis zur Trockne eingeengt. Der erhaltene Rückstand wurde mittels Flash-Chromatographie (Laufmittel: Cyclohexan/EtOAc = 7:3) gereinigt. 6-Methyl-3-phenyl-1-oxa-2,8-diaza-spiro[4.5]dec-2-en-8-carbonsäure-(4-tert-butyl-phenyl)-amid wurde so in einer Ausbeute von 170 mg (30 %) als Diastereoisomerengemisch (Schmelzpunkt ab 65 °C) erhalten.

### Synthese von Beispielverbindung 386: 3-Benzyl-1-oxa-2,8-diaza-spiro[4.5]dec-2-en-8-carbonsäure-(4-tert-butyl-phenyl)-amid

Zu einer Lösung von Verbindung Y (286 mg, 1.24 mmol) in abs. DCM (10 ml) wurde 4-tert-Butylphenylisocyanat (0.218 ml, 1.24 mmol), gelöst in abs. DCM (5 ml), bei RT innerhalb von 20 min getropft. Nach 3.5 h Rühren bei RT wurde die Lösung mit 10%-iger Zitronensäure-Lsg. (1 × 10 ml) und Wasser (1 × 10 ml) gewaschen, getrocknet und eingeengt. Das erhaltene farblose Öl (742 mg) enthielt geringe Verunreinigungen. Es wurde mit Diethylether (10 ml) versetzt und bei 5 °C aufbewahrt. Der ausgefallene Feststoff wurde abgesaugt und mit Diethylether (5 ml) gewaschen. Das gewünschte Produkt 3-Benzyl-1-oxa-2,8-diaza-spiro[4.5]dec-2-en-8-carbonsäure-(4-tert-butyl-phenyl)-amid wurde in einer Ausbeute von 73 % (369 mg) mit einem Schmelzpunkt von 137-139 °C erhalten.

### Synthese von Beispielverbindung 387: N-(4-tert-Butylphenyl)-3-phenethyl-1-oxa-2,8-diazaspiro[4.5]dec-2-en-8-carboxamid

Zu einer Lösung von Verbindung CC (610 mg, 2,5 mmol) in abs. DCM (15 ml) wurde 4-tert-Butylphenylisocyanat (0.44 ml, 2.5 mmol), gelöst in abs. DCM (5 ml), bei RT innerhalb von 20 min getropft. Nach 3 h Rühren bei RT wurde die Lösung mit 10%-iger Zitronensäure-Lsg. (1 × 10 ml) und Wasser (1 × 10 ml) gewaschen, getrocknet und eingeengt. Der Rückstand (683 mg) wurde mit Diethylether (10 ml) 18 h gerührt. Der ausgefallene Feststoff wurde abgesaugt und mit Diethylether (5 ml) gewaschen. Das Zielprodukt N-(4-tert-Butylphenyl)-3-phenethyl-1-oxa-2,8-diazaspiro[4.5]dec-2-en-8-carboxamid wurde in einer Ausbeute von 55 % (575 mg) mit einem Schmelzpunkt von 153-154 °C erhalten.

### b. Automatisierte Synthese

Es wurden zunächst folgende Stammlösungen erzeugt:
- Lösung I:: 0.05 M Lösung des Amins der allgemeinen Formel II in Toluol
- Lösung II:: 0.1 M Lösung des Isocyanats der allgemeinen Formel R⁵-N=C=O bzw. des Isothiocyanats der allgemeinen Formel R⁷-N=C=S in Toluol

Lösung I (2 mL) wurde in einem trockenen Gewindeglas mit Septumkappe bei RT vorgelegt und mit Lösung II (1 mL) versetzt. Das Reaktionsgemisch wurde 6 h im Trefferreaktor unter Rückfluß gerührt. Die Reaktionsmischung wurde in Vials überführt und das Lösungsmittel in der GeneVac entfernt.

Die folgenden Isocyanate der allgemeinen Formel R⁵-N=C=O wurden zur Synthese verwendet: Phenylisocyanat, 2-Methylphenylisocyanat, m-Tolylisocyanat, p-Tolylisocyanat, 2-Ethylphenylisocyanat, 3-Ethylphenylisocyanat, 4-Ethylphenylisocyanat, 2-Propylphenylisocyanat, 2-Fluorphenylisocyanat, 3-Fluorphenylisocyanat, 4-Fluorphenylisocyanat, 2-Chlorphenylisocyanat, 3-Chlorphenylisocyanat, 4-Chlorphenylisocyanat, 2-Bromphenylisocyanat, 3-Bromphenylisocyanat,4-Bromphenylisocyanat, 3-lodphenylisocyanat, 4-lodphenylisocyanat, 2-Methoxy-äphenylisocyanat, 3-Methoxyphenylisocyanat, 4-Methoxyphenylisocyanat, 2-Ethoxyphenylisocyanat, 4-Ethoxyphenylisocyanat, 2-(Methylthio)-phenylisocyanat, 3-(Methylthio)-phenylisocyanat, 4-(Methylthio)-phenylisocyanat, 2-Isopropyl-phenylisocyanat, 4-Isopropylphenylisocyanat, 4-Butylphenylisocyanat, 3-Cyanphenylisocyanat, 2-Methoxycarbonylphenylisocyanat, 3-Methoxycarbonylphenylisocyanat, Ethyl-2-isocyanatbenzoat, 3-Ethoxycarbonylphenylisocyanat, 4-Ethoxycarbonylphenylisocyanat, 2-(Trifluormethyl)-phenylisocyanat, 3-(Trifluormethyl)-phenylisocyanat, 4-(Trifluormethyl)-phenylisocyanat.1-Naphthylisocyanat, 2-Biphenylisocyanat, 4-Biphenylisocyanat, 4-Pentafluorsulfanyl-isocyanat, 2-Phenoxyphenylisocyanat, 4-Phenoxyphenylisocyanat, 4-Benzyloxyphenylisocyanat, 4-(Dimethylamino)-phenylisocyanat, 2,6-Difluorphenylisocyanat, 2,5-Difluorphenylisocyanat, 2,4-Difluorphenylisocyanat, 3,4-Difluorphenylisocyanat, 2,6-Dichlorphenylisocyanat, 2,3-Dichlorphenylisocyanat, 2,5-Dichlorphenylisocyanat, 3,5-Dichlorphenylisocyanat, 2,4-Dichlorphenylisocyanat, 3,4-Dichlorphenylisocyanat, 2,4-Dibromphenylisocyanat, 2-Chlor-5-(trifluormethyl)-phenylisocyanat, 4-Chlor-2-(trifluormethyl)-phenylisocyanat, 4-Chlor-3-(trifluormethyl)-phenylisocyanat, 4-Brom-2-(trifluormethyl)-phenylisocyanat, 3,5-Bis-(trifluormethyl)-phenylisocyanat, 2-(Trifluormethoxy)-phenylisocyanat, 2,4-Dimethoxyphenylisocyanat, 2,5-Dimethoxyphenylisocyanat, 3,5-Dimethoxyphenylisocyanat, 2-Fluor-5-methylphenylisocyanat, 3-Fluor-4-methylphenylisocyanat, 3-Chlor-2-methylphenylisocyanat, 4-Chlor-2-methylphenylisocyanat, 5-Chlor-2-methylphenylisocyanat, 3-Chlor-4-methylphenylisocyanat, 4-Brom-2-methylphenylisocyanat, 3-Chlor-4-fluorphenylisocyanat, 4-Brom-2-fluorphenylisocyanat, 3,5-Dimethylphenylisocyanat, 2,6-Dimethylphenylisocyanat, 3,4-Dimethylphenylisocyanat, 2,5-Dimethylphenylisocyanat, 2,4-Dimethylphenylisocyanat, 2-Ethyl-6-methylphenylisocyanat, 2-Isopropyl-6-methylphenylisocyanat, 2-tert.-Butyl-6-methylphenylisocyanat, 2,6-Diethylphenylisocyanat, 2-Ethyl-6-isopropylphenylisocyanat, 2,6-Diisopropylphenylisocyanat, 4-Methoxy-2-methylphenylisocyanat, 2-Methoxy-5-methylphenylisocyanat, 5-Chlor-2-methoxyphenylisocyanat, 4-Brom-2,6-dimethylphenylisocyanat, 2,4,5-Trichlorphenylisocyanat, 2,4-Dibrom-6-fluorphenylisocyanat, 2-Brom-4,6-difluorphenylisocyanat, 5-Chlor-2,4-dimethoxyphenylisocyanat, 3,4,5-Trimethoxyphenylisocyanat, 2,4,5-Trimethylphenylisocyanat, 2,4,6-Trimethylphenylisocyanat, 4-Trifluormethoxyphenylisocyanat, n-Propylisocyanat, n-Butylisocyanat, Cyclohexylisocyanat, 2-Chlorethylisocyanat, 3-Chlorpropylisocyanat, 2-Bromethylisocyanat, Benzylisocyanat, Phenylethylisocyanat, 3-Methylbenzylisocyanat, 4-Methylbenzylisocyanat, 2-Ethylbenzylisocyanat, 3-Ethylbenzylisocyanat, 4-Ethylbenzylisocyanat, 4-Fluorbenzylisocyanat, 2-Chlorbenzylisocyanat, 1-(4-Bromphenyl)ethylisocyanat, 2,4-Dichlorbenzylisocyanat, 3,4-Dichlorbenzylisocyanat, 4-Methoxybenzylisocyanat, 1-(1-Naphthyl)ethylisocyanat, 2-Methylbenzylisocyanat, n-Pentylisocyanat, 4-tert-Butylphenylisocyanat.
Die folgenden Isothiocyanate der allgemeinen Formel R⁷-N=C=S wurden zur Synthese verwendet: Methylisothiocyanat, Ethylisothiocyanat, n-Propylisothiocyanat, n-Butylisothiocyanat, n-Pentylisothiocyanat, n-Hexylisothiocyanat, n-Heptylisothiocyanat, n-Octylisothiocyanat, n-Nonylisothiocyanat, n-Decylisothiocyanat, n-Dodecylisothiocyanat, n-Tetradecylisothiocyanat, n-Octadecylisothiocyanat, Isopropylisothiocyanat, Isobutylisothiocyanat, tert-Butylisothiocyanat, tert.-Amylisothiocyanat, Allylisothiocyanat, Methallylisothiocyanat, Chlormethylthiocyanat, 2-Chlorethylisothiocyanat, 2-Methoxyethylisothiocyanat, 3-Ethoxypropylisothiocyanat, 3-(Diethylamino)propyl-isothiocyanat, Cyclopropylisothiocyanat, Cyclopentylisothiocyanat, Cyclohexylisothiocyanat, Cyclohexylmethyllisothiocyanat, Cyclooctylisothiocyanat, Cyclododecylisothiocyanat, 1-Adamtylisothiocyanat, 2-(Isothiocyanatomethyl)-tetrahydrofuran, 2-(4-Morpholino)ethylisothiocyanat, 3-(4-Morpholino)-propylisothiocyanat, 2-Furylmethylisothiocyanat, Phenylisothiocyanat, 2-Methylphenylisothiocyanat, 3-Methylphenylisothiocyanat, p-Tolylisothiocyanat, 2-Ethylphenylisothiocyanat, Benzylisothiocyanat, 2-Phenylethylisothiocyanat, Alpha-methylbenzylisothiocyanat, 3-Phenylpropylisothiocyanat, 2-Isopropylphenylisothiocyanat, 4-lsopropylphenylisothiocyanat, 4-Butylphenylisothiocyanat, 2-Fluorphenylisothiocyanat, 3-Fluorphenylisothiocyanat, 4-Fluorphenylisothiocyanat, 2-Chlorphenylisothiocyanat, 3-Chlorphenylisothiocyanat, 4-Chlorphenylisothiocyanat, 2-Bromphenylisothiocyanat, 3-Bromphenylisothiocyanat, 4-Bromphenylisothiocyanat, 2-lodphenylisothiocyanat, 4-lodphenylisothiocyanat, 4-Fluorbenzylisothiocyanat, 1-(4-Fluorphenyl)ethylisothiocyanat, 2-Chlorbenzylisothiocyanat, 4-Chlorbenzylisothiocyanat, 2-(4-Chlorphenyl)ethylisothiocyanat, 2-(Trifluormethyl)-phenylisothiocyanat, 3-(Trifluormethyl)-phenylisothiocyanat, 4-(Trifluormethyl)-phenylisothiocyanat, 2-(Methylthio)phenylisothiocyanat, 3-(Methylthio)phenylisothiocyanat, 4-(Methylthio)phenylisothiocyanat, 2-Methoxyphenylisothiocyanat, 3-Methoxyphenylisothiocyanat, 4-Methoxyphenylisothiocyanat, 4-Methoxybenzylisothiocyanat, 2-Nitrophenylisothiocyanat, 3-Nitrophenylisothiocyanat, 4-Nitrophenylisothiocyanat, 3-Pyridylisothiocyanat, 4-Cyanophenylisothiocyanat, 3-Cyanophenylisothiocyanat, (4-Isothiocyanato-phenyl)-dimethylamin, 4-Diethylaminophenylisothiocyanat, 4-Acetylphenylisothiocyanat, 3-Carbonylphenylisothiocyanat, 4-Ethoxyphenylisothiocyanat, 4-Isothiocyanatophenylacetat, 4-Benzyloxyphenylisothiocyanat, 2-Methoxycarbonylphenylisothiocyanat, 3-Methoxycarbonylphenylisothiocyanat, 4-Methoxycarbonylphenylisothiocyanat, Ethyl-2-isothiocyanatobenzoat, 4-Ethoxycarbonylphenylisothiocyanat, 2,4-Dimethylphenylisothiocyanat, 2,6-Dimethylphenylisothiocyanat, 3,5-Dimethylphenylisothiocyanat, 2-Ethyl-6-methylphenylisothiocyanat, 4-Pentafluorsulfanyl-isothiocyanat, 2-Ethyl-6-isopropylphenylisothiocyanat, 2,6-Diethylphenylisothiocyanat, 2,6-Diisopropylphenylisothiocyanat, 2-Chlor-6-methylphenylisothiocyanat, 5-Chlor-2-methylphenylisothiocyanat, 3-Chlor-4-methylphenylisothiocyanat, 4-Chlor-2-methylphenylisothiocyanat, 4-Brom-2-methylphenylisothiocyanat, 2-Brom-4-methylphenylisothiocyanat, 2,4-Difluorphenyl-isothiocyanat, 2,6-Difluorphenylisothiocyanat, 2,5-Difluorphenylisothiocyanat, 2,3-Dichlorphenylisothiocyanat, 2,6-Dichlorphenylisothiocyanat, 2,5-Dichlorphenylisothiocyanat, 3,4-Dichlorphenylisothiocyanat, 2,4-Dichlorphenylisothiocyanat, 3,5-Dichlorphenylisothiocyanat, 3,4-Dichlorbenzylisothiocyanat, 4-Brom-2-chlorphenylisothiocyanat, 4-Chlor-3-nitrophenylisothiocyanat, 2-Chlor-4-nitrophenylisothiocyanat, 5-Chlor-2-methoxyphenylisothiocyanat, 2-Chloro-5-(trifluormethyl)phenylisothiocyanat, 4-Chloro-3-(trifluormethyl)phenylisothiocyanat, 4-Bromo-2-(trifluormethyl)isothiocyanat, 3,5-Bis-(trifluormethyl)-phenylisothiocyanat, 2-Methoxy-5-methylphenylisothiocyanat, 2,5-Dimethoxyphenylisothiocyanat, 2,4-Dimethoxyphenylisothiocyanat, 3,5-Dimethoxyphenylisothiocyanat, 3,4-Dimethoxyphenylisothiocyanat, 4-Methoxy-2-nitrophenylisothiocyanat, 2-Methoxy-4-nitrophenylisothiocyanat, 4-Methyl-2-nitrophenylisothiocyanat, (2-Methoxy-5-phenyl)phenylisothiocyanat, 2,4,6-Trifluorphenylisothiocyanat, 2,4,5-Trichlorphenylisothiocyanat, 2,4,6-Trichlorphenylisothiocyanat, 2,3,4-Trichlorphenylisothiocyanat, 2,4,6-Tribromphenylisothiocyanat, 2,4,6-Trimethylphenylisothocyanat, 4-Brom-2,6-dimethylphenylisothiocyanat, 3,4,5-Trimethoxyphenylisothiocyanat, 2,3,5,6-Tetrafluorphenylisothiocyanat, 2,3,4,5-Tetrachlorphenylisothiocyanat, Pentaflourphenylisothiocyanat, 1-Naphthylisothiocyanat, 3,4-Methylendioxobenzylisothiocyanat, Triphenylmethylisothiocyanat, 4-(trans-4-propylcyclohexyl)phenylisothiocyanat, 4-tert.-Butylphenylisothiocyanat.

Die folgenden erfindungsgemäßen substituierten Spiro-Verbindungen wurden wie unter 1 a. beschrieben hergestellt.

| | Name | [M+H]⁺ |
|---|---|---|
| [1] | 3-(3-Methoxy-phenyl)-1-oxa-2,8-diaza-spiro[4.5]dec-2-en-8-carbonsäurephenylamid | 366,4 |
| [2] | 3-Phenyl-1-oxa-2,8-diaza-spiro[4.5]dec-2-en-8-carbonsäurephenylamid | 336,4 |
| [3] | 3-(4-Chlor-phenyl)-1-oxa-2,8-diaza-spiro[4.5]dec-2-en-8-carbonsäurephenylamid | 370,9 |
| [4] | 3-(4-tert-Butyl-phenyl)-1-oxa-2,8-diaza-spiro[4.5]dec-2-en-8-carbonsäurephenylamid | 392,5 |
| [5] | 3-p-Tolyl-1-oxa-2,8-diaza-spiro[4.5]dec-2-en-8-carbonsäurephenylamid | 350,4 |
| [6] | 3-(4-Trifluormethyl-phenyl)-1-oxa-2,8-diaza-spiro[4.5]dec-2-en-8-carbonsäurephenylamid | 404,4 |
| [7] | 3-(3-Methoxy-phenyl)-1-oxa-2,8-diaza-spiro[4.5]dec-2-en-8-carbonsäure-m-tolylamid | 380,5 |
| [8] | 3-(3-Methoxy-phenyl)-1-oxa-2,8-diaza-spiro[4.5]dec-2-en-8-carbonsäure-m-tolylamid | 380,5 |
| [9] | 3-Phenyl-1-oxa-2,8-diaza-spiro[4.5]dec-2-en-8-carbonsäure-m-tolylamid | 350,4 |
| [10] | 3-(4-Chlor-phenyl)-1-oxa-2,8-diaza-spiro[4.5]dec-2-en-8-carbonsäure-m-tolylamid | 384,9 |
| [11] | 3-(4-tert-Butyl-phenyl)-1-oxa-2,8-diaza-spiro[4.5]dec-2-en-8-carbonsäure-m-tolylamid | 406,5 |
| [12] | 3-p-Tolyl-1-oxa-2,8-diaza-spiro[4.5]dec-2-en-8-carbonsäure-m-tolylamid | 364,5 |
| [13] | 3-(4-Trifluormethyl-phenyl)-1-oxa-2,8-diaza-spiro[4.5]dec-2-en-8-carbonsäure-m-tolylamid | 418,4 |
| [14] | 3-(3-Methoxy-phenyl)-1-oxa-2,8-diaza-spiro[4.5]dec-2-en-8-carbonsäure-(3-ethyl-phenyl)-amid | 394,5 |
| [15] | 3-(3-Methoxy-phenyl)-1-oxa-2,8-diaza-spiro[4.5]dec-2-en-8-carbonsäure-(3-ethyl-phenyl)-amid | 394,5 |
| [16] | 3-Phenyl-1-oxa-2,8-diaza-spiro[4.5]dec-2-en-8-carbonsäure-(3-ethyl-phenyl)-amid | 364,5 |
| [17] | 3-(4-Chlor-phenyl)-1-oxa-2,8-diaza-spiro[4.5]dec-2-en-8-carbonsäure-(3-ethyl-phenyl)-amid | 398,9 |
| [18] | 3-(4-tert-Butyl-phenyl)-1-oxa-2,8-diaza-spiro[4.5]dec-2-en-8-carbonsäure-(3-ethyl-phenyl)-amid | 420,6 |
| [19] | 3-p-Tolyl-1-oxa-2,8-diaza-spiro[4.5]dec-2-en-8-carbonsäure-(3-ethyl-phenyl)-amid | 378,5 |
| [20] | 3-(4-Trifluormethyl-phenyl)-1-oxa-2,8-diaza-spiro[4.5]dec-2-en-8-carbonsäure-(3-ethyl-phenyl)-amid | 432,5 |
| [21] | 3-(3-Methoxy-phenyl)-1-oxa-2,8-diaza-spiro[4.5]dec-2-en-8-carbonsäure-(2-propyl-phenyl)-amid | 408,5 |
| [22] | 3-Phenyl-1-oxa-2,8-diaza-spiro[4.5]dec-2-en-8-carbonsäure-(2-propyl-phenyl)-amid | 378,5 |
| [23] | 3-(4-Chlor-phenyl)-1-oxa-2,8-diaza-spiro[4.5]dec-2-en-8-carbonsäure-(2-propyl-phenyl)-amid | 412,9 |
| [24] | 3-(4-tert-Butyl-phenyl)-1-oxa-2,8-diaza-spiro[4.5]dec-2-en-8-carbonsäure-(2-propyl-phenyl)-amid | 434,6 |
| [25] | 3-p-Tolyl-1-oxa-2,8-diaza-spiro[4.5]dec-2-en-8-carbonsäure-(2-propyl-phenyl)-amid | 392,5 |
| [26] | 3-(4-Trifluormethyl-phenyl)-1-oxa-2,8-diaza-spiro[4.5]dec-2-en-8-carbonsäure-(2-propyl-phenyl)-amid | 446,5 |
| [27] | 3-(3-Methoxy-phenyl)-1-oxa-2,8-diaza-spiro[4.5]dec-2-en-8-carbonsäure-(4-fluor-phenyl)-amid | 384,4 |
| [28] | 3-Phenyl-1-oxa-2,8-diaza-spiro[4.5]dec-2-en-8-carbonsäure-(4-fluor-phenyl)-amid | 354,4 |
| [29] | 3-(4-Chlor-phenyl)-1-oxa-2,8-diaza-spiro[4.5]dec-2-en-8-carbonsäure-(4-fluor-phenyl)-amid | 388,8 |
| [30] | 3-(4-tert-Butyl-phenyl)-1-oxa-2,8-diaza-spiro[4.5]dec-2-en-8-carbonsäure-(4-fluor-phenyl)-amid | 410,5 |
| [31] | 3-p-Tolyl-1-oxa-2,8-diaza-spiro[4.5]dec-2-en-8-carbonsäure-(4-fluor-phenyl)-amid | 368,4 |
| [32] | 3-(4-Trifluormethyl-phenyl)-1-oxa-2,8-diaza-spiro[4.5]dec-2-en-8-carbonsäure-(4-fluor-phenyl)-amid | 422,4 |
| [33] | 3-(3-Methoxy-phenyl)-1-oxa-2,8-diaza-spiro[4.5]dec-2-en-8-carbonsäure-(3-chlor-phenyl)-amid | 400,9 |
| [34] | 3-Phenyl-1-oxa-2,8-diaza-spiro[4.5]dec-2-en-8-carbonsäure-(3-chlor-phenyl)-amid | 370,9 |
| [35] | 3-(4-Chlor-phenyl)-1-oxa-2,8-diaza-spiro[4.5]dec-2-en-8-carbonsäure-(3-chlor-phenyl)-amid | 405,3 |
| [36] | 3-(4-tert-Butyl-phenyl)-1-oxa-2,8-diaza-spiro[4.5]dec-2-en-8-carbonsäure-(3-chlor-phenyl)-amid | 427,0 |
| [37] | 3-p-Tolyl-1-oxa-2,8-diaza-spiro[4.5]dec-2-en-8-carbonsäure-(3-chlor-phenyl)-amid | 384,9 |
| [38] | 3-(4-Trifluormethyl-phenyl)-1-oxa-2,8-diaza-spiro[4.5]dec-2-en-8-carbonsäure-(3-chlor-phenyl)-amid | 438,8 |
| [39] | 3-(3-Methoxy-phenyl)-1-oxa-2,8-diaza-spiro[4.5]dec-2-en-8-carbonsäure-(4-chlor-phenyl)-amid | 400,9 |
| [40] | 3-Phenyl-1-oxa-2,8-diaza-spiro[4.5]dec-2-en-8-carbonsäure-(4-chlor-phenyl)-amid | 370,9 |
| [41] | 3-(4-Chlor-phenyl)-1-oxa-2,8-diaza-spiro[4.5]dec-2-en-8-carbonsäure-(4-chlor-phenyl)-amid | 405,3 |
| [42] | 3-(4-tert-Butyl-phenyl)-1-oxa-2,8-diaza-spiro[4.5]dec-2-en-8-carbonsäure-(4-chlor-phenyl)-amid | 427,0 |
| [43] | 3-p-Tolyl-1-oxa-2,8-diaza-spiro[4.5]dec-2-en-8-carbonsäure-(4-chlor-phenyl)-amid | 384,9 |
| [44] | 3-Phenyl-1-oxa-2,8-diaza-spiro[4.5]dec-2-en-8-carbonsäure-(4-iodo-phenyl)-amid | 462,3 |
| [45] | 3-(4-tert-Butyl-phenyl)-1-oxa-2,8-diaza-spiro[4.5]dec-2-en-8-carbonsäure-(4-iodo-phenyl)-amid | 518,4 |
| [46] | 3-(4-Trifluormethyl-phenyl)-1-oxa-2,8-diaza-spiro[4.5]dec-2-en-8-carbonsäure-(4-iodo-phenyl)-amid | 530,3 |
| [47] | 3-(3-Methoxy-phenyl)-1-oxa-2,8-diaza-spiro[4.5]dec-2-en-8-carbonsäure-(4-methoxy-phenyl)-amid | 396,5 |
| [48] | 3-Phenyl-1-oxa-2,8-diaza-spiro[4.5]dec-2-en-8-carbonsäure-(4-methoxy-phenyl)-amid | 366,4 |
| [49] | 3-(4-Chlor-phenyl)-1-oxa-2,8-diaza-spiro[4.5]dec-2-en-8-carbonsäure-(4-methoxy-phenyl)-amid | 400,9 |
| [50] | 3-(4-tert-Butyl-phenyl)-1-oxa-2,8-diaza-spiro[4.5]dec-2-en-8-carbonsäure-(4-methoxy-phenyl)-amid | 422,5 |
| [51] | 3-p-Tolyl-1-oxa-2,8-diaza-spiro[4.5]dec-2-en-8-carbonsäure-(4-methoxy-phenyl)-amid | 380,5 |
| [52] | 3-(4-Trifluormethyl-phenyl)-1-oxa-2,8-diaza-spiro[4.5]dec-2-en-8-carbonsäure-(4-methoxy-phenyl)-amid | 434,4 |
| [53] | 3-(3-Methoxy-phenyl)-1-oxa-2,8-diaza-spiro[4.5]dec-2-en-8-carbonsäure-(2-methylsulfanyl-phenyl)-amid | 412,5 |
| [54] | 3-Phenyl-1-oxa-2,8-diaza-spiro[4.5]dec-2-en-8-carbonsäure-(2-methylsulfanyl-phenyl)-amid | 382,5 |
| [55] | 3-(4-Chlor-phenyl)-1-oxa-2,8-diaza-spiro[4.5]dec-2-en-8-carbonsäure-(2-methylsulfanyl-phenyl)-amid | 416,9 |
| [56] | 3-(4-tert-Butyl-phenyl)-1-oxa-2,8-diaza-spiro[4.5]dec-2-en-8-carbonsäure-(2-methylsulfanyl-phenyl)-amid | 438,6 |
| [57] | 3-p-Tolyl-1-oxa-2,8-diaza-spiro[4.5]dec-2-en-8-carbonsäure-(2-methylsulfanyl-phenyl)-amid | 396,5 |
| [58] | 3-(4-Trifluormethyl-phenyl)-1-oxa-2,8-diaza-spiro[4.5]dec-2-en-8-carbonsäure-(2-methylsulfanyl-phenyl)-amid | 450,5 |
| [59] | 3-(3-Methoxy-phenyl)-1-oxa-2,8-diaza-spiro[4.5]dec-2-en-8-carbonsäure-(3-methylsulfanyl-phenyl)-amid | 412,5 |
| [60] | 3-Phenyl-1-oxa-2,8-diaza-spiro[4.5]dec-2-en-8-carbonsäure-(3-methylsulfanyl-phenyl)-amid | 382,5 |
| [61] | 3-(4-Chlor-phenyl)-1-oxa-2,8-diaza-spiro[4.5]dec-2-en-8-carbonsäure-(3-methylsulfanyl-phenyl)-amid | 416,9 |
| [62] | 3-(4-tert-Butyl-phenyl)-1-oxa-2,8-diaza-spiro[4.5]dec-2-en-8-carbonsäure-(3-methylsulfanyl-phenyl)-amid | 438,6 |
| [63] | 3-p-Tolyl-1-oxa-2,8-diaza-spiro[4.5]dec-2-en-8-carbonsäure-(3-methylsulfanyl-phenyl)-amid | 396,5 |
| [64] | 3-(4-Trifluormethyl-phenyl)-1-oxa-2,8-diaza-spiro[4.5]dec-2-en-8-carbonsäure-(3-methylsulfanyl-phenyl)-amid | 450,5 |
| [65] | 3-(3-Methoxy-phenyl)-1-oxa-2,8-diaza-spiro[4.5]dec-2-en-8-carbonsäure-(4-methylsulfanyl-phenyl)-amid | 412,5 |
| [66] | 3-Phenyl-1-oxa-2,8-diaza-spirof4.5ldec-2-en-8-carbonsäure-(4-methylsulfanyl-phenyl)-amid | 382,5 |
| [67] | 3-(4-Chlor-phenyl)-1-oxa-2,8-diaza-spiro[4.5]dec-2-en-8-carbonsäure-(4-methylsulfanyl-phenyl)-amid | 416,9 |
| [68] | 3-(4-tert-Butyl-phenyl)-1-oxa-2,8-diaza-spiro[4.5]dec-2-en-8-carbonsäure-(4-methylsulfanyl-phenyl)-amid | 438,6 |
| [69] | 3-p-Tolyl-1-oxa-2,8-diaza-spiro[4.5]dec-2-en-8-carbonsäure-(4-methylsulfanyl-phenyl)-amid | 396,5 |
| [70] | 3-(4-Trifluormethyl-phenyl)-1-oxa-2,8-diaza-spiro[4.5]dec-2-en-8-carbonsäure-(4-methylsulfanyl-phenyl)-amid | 450,5 |
| [71] | 3-(3-Methoxy-phenyl)-1-oxa-2,8-diaza-spiro[4.5]dec-2-en-8-carbonsäure-(2-isopropyl-phenyl)-amid | 408,5 |
| [72] | 3-Phenyl-1-oxa-2,8-diaza-spiro[4.5]dec-2-en-8-carbonsäure-(2-isopropyl-phenyl)-amid | 378,5 |
| [73] | 3-(4-Chlor-phenyl)-1-oxa-2,8-diaza-spiro[4.5]dec-2-en-8-carbonsäure-(2-isopropyl-phenyl)-amid | 412,9 |
| [74] | 3-(4-tert-Butyl-phenyl)-1-oxa-2,8-diaza-spiro[4.5]dec-2-en-8-carbonsäure-(2-isopropyl-phenyl)-amid | 434,6 |
| [75] | 3-p-Tolyl-1-oxa-2,8-diaza-spiro[4.5]dec-2-en-8-carbonsäure-(2-isopropyl-phenyl)-amid | 392,5 |
| [76] | 3-(4-Trifluormethyl-phenyl)-1-oxa-2,8-diaza-spiro[4.5]dec-2-en-8-carbonsäure-(2-isopropyl-phenyl)-amid | 446,5 |
| [77] | 3-(3-Methoxy-phenyl)-1-oxa-2,8-diaza-spiro[4.5]dec-2-en-8-carbonsäure-(4-isopropyl-phenyl)-amid | 408,5 |
| [78] | 3-Phenyl-1-oxa-2,8-diaza-spiro[4.5]dec-2-en-8-carbonsäure-(4-isopropyl-phenyl)-amid | 378,5 |
| [79] | 3-(4-Chlor-phenyl)-1-oxa-2,8-diaza-spiro[4.5]dec-2-en-8-carbonsäure-(4-isopropyl-phenyl)-amid | 412,9 |
| [80] | 3-(4-tert-Butyl-phenyl)-1-oxa-2,8-diaza-spiro[4.5]dec-2-en-8-carbonsäure-(4-isopropyl-phenyl)-amid | 434,6 |
| [81] | 3-p-Tolyl-1-oxa-2,8-diaza-spiro[4.5]dec-2-en-8-carbonsäure-(4-isopropyl-phenyl)-amid | 392,5 |
| [82] | 3-(4-Trifluormethyl-phenyl)-1-oxa-2,8-diaza-spiro[4.5]dec-2-en-8-carbonsäure-(4-isopropyl-phenyl)-amid | 446,5 |
| [83] | 3-(3-Methoxy-phenyl)-1-oxa-2,8-diaza-spiro[4.5]dec-2-en-8-carbonsäure-(2-trifluormethyl-phenyl)-amid | 434,4 |
| [84] | 3-Phenyl-1-oxa-2,8-diaza-spiro[4.5]dec-2-en-8-carbonsäure-(2-trifluormethyl-phenyl)-amid | 404,4 |
| [85] | 3-(4-Chlor-phenyl)-1-oxa-2,8-diaza-spiro[4.5]dec-2-en-8-carbonsäure-(2-trifluormethyl-phenyl)-amid | 438,8 |
| [86] | 3-(4-tert-Butyl-phenyl)-1-oxa-2,8-diaza-spiro[4.5]dec-2-en-8-carbonsäure-(2-trifluormethyl-phenyl)-amid | 460,5 |
| [87] | 3-p-Tolyl-1-oxa-2,8-diaza-spiro[4.5]dec-2-en-8-carbonsäure-(2-trifluormethyl-phenyl)-amid | 418,4 |
| [88] | 3-(4-Trifluormethyl-phenyl)-1-oxa-2,8-diaza-spiro[4.5]dec-2-en-8-carbonsäure-(2-trifluormethyl-phenyl)-amid | 472,4 |
| [89] | 3-(3-Methoxy-phenyl)-1-oxa-2,8-diaza-spiro[4.5]dec-2-en-8-carbonsäure-(3-trifluormethyl-phenyl)-amid | 434,4 |
| [90] | 3-Phenyl-1-oxa-2,8-diaza-spiro[4.5]dec-2-en-8-carbonsäure-(3-trifluormethyl-phenyl)-amid | 404,4 |
| [91] | 3-(4-Chlor-phenyl)-1-oxa-2,8-diaza-spiro[4.5]dec-2-en-8-carbonsäure-(3-trifluormethyl-phenyl)-amid | 438,8 |
| [92] | 3-(4-tert-Butyl-phenyl)-1-oxa-2,8-diaza-spiro[4.5]dec-2-en-8-carbonsäure-(3-trifluormethyl-phenyl)-amid | 460,5 |
| [93] | 3-p-Tolyl-1-oxa-2,8-diaza-spiro[4.5]dec-2-en-8-carbonsäure-(3-trifluormethyl-phenyl)-amid | 418,4 |
| [94] | 3-(4-Trifluormethyl-phenyl)-1-oxa-2,8-diaza-spiro[4.5]dec-2-en-8-carbonsäure-(3-trifluormethyl-phenyl)-amid | 472,4 |
| [95] | 3-Phenyl-1-oxa-2,8-diaza-spiro[4.5]dec-2-en-8-carbonsäurebiphenyl-4-ylamid | 412,5 |
| [96] | 3-(4-tert-Butyl-phenyl)-1-oxa-2,8-diaza-spiro[4.5]dec-2-en-8-carbonsäurebiphenyl-4-ylamid | 468,6 |
| [97] | 3-p-Tolyl-1-oxa-2,8-diaza-spiro[4.5]dec-2-en-8-carbonsäurebiphenyl-4-ylamid | 426,5 |
| [98] | 3-(3-Methoxy-phenyl)-1-oxa-2,8-diaza-spiro[4.5]dec-2-en-8-carbonsäure-(2-phenoxy-phenyl)-amid | 458,5 |
| [99] | 3-Phenyl-1-oxa-2,8-diaza-spiro[4.5]dec-2-en-8-carbonsäure-(2-phenoxy-phenyl)-amid | 428,5 |
| [100] | 3-(4-Chlor-phenyl)-1-oxa-2,8-diaza-spiro[4.5]dec-2-en-8-carbonsäure-(2-phenoxy-phenyl)-amid | 462,9 |
| [101] | 3-(4-tert-Butyl-phenyl)-1-oxa-2,8-diaza-spiro[4.5]dec-2-en-8-carbonsäure-(2-phenoxy-phenyl)-amid | 484,6 |
| [102] | 3-p-Tolyl-1-oxa-2,8-diaza-spiro[4.5]dec-2-en-8-carbonsäure-(2-phenoxy-phenyl)-amid | 442,5 |
| [103] | 3-(4-Trifluormethyl-phenyl)-1-oxa-2,8-diaza-spiro[4.5]dec-2-en-8-carbonsäure-(2-phenoxy-phenyl)-amid | 496,5 |
| [104] | 3-(3-Methoxy-phenyl)-1-oxa-2,8-diaza-spiro[4.5]dec-2-en-8-carbonsäure-(4-benzyloxy-phenyl)-amid | 472,6 |
| [105] | 3-Phenyl-1-oxa-2,8-diaza-spiro[4.5]dec-2-en-8-carbonsäure-(4-benzyloxy-phenyl)-amid | 442,5 |
| [106] | 3-(4-Chlor-phenyl)-1-oxa-2,8-diaza-spiro[4.5]dec-2-en-8-carbonsäure-(4-benzyloxy-phenyl)-amid | 477,0 |
| [107] | 3-(4-tert-Butyl-phenyl)-1-oxa-2,8-diaza-spiro[4.5]dec-2-en-8-carbonsäure-(4-benzyloxy-phenyl)-amid | 498,6 |
| [108] | 3-p-Tolyl-1-oxa-2,8-diaza-spiro[4.5]dec-2-en-8-carbonsäure-(4-benzyloxy-phenyl)-amid | 456,6 |
| [109] | 3-(4-Trifluormethyl-phenyl)-1-oxa-2,8-diaza-spiro[4.5]dec-2-en-8-carbonsäure-(4-benzyloxy-phenyl)-amid | 510,5 |
| [110] | 3-(3-Methoxy-phenyl)-1-oxa-2,8-diaza-spiro[4.5]dec-2-en-8-carbonsäurecyclohexylamid | 372,5 |
| [111] | 3-Phenyl-1-oxa-2,8-diaza-spiro[4.5]dec-2-en-8-carbonsäurecyclohexylamid | 342,5 |
| [112] | 3-(4-Chlor-phenyl)-1-oxa-2,8-diaza-spiro[4.5]dec-2-en-8-carbonsäurecyclohexylamid | 376,9 |
| [113] | 3-(4-tert-Butyl-phenyl)-1-oxa-2,8-diaza-spiro[4.5]dec-2-en-8-carbonsäurecyclohexylamid | 398,6 |
| [114] | 3-p-Tolyl-1-oxa-2,8-diaza-spiro[4.5]dec-2-en-8-carbonsäurecyclohexylamid | 356,5 |
| [115] | 3-(4-Trifluormethyl-phenyl)-1-oxa-2,8-diaza-spiro[4.5]dec-2-en-8-carbonsäurecyclohexylamid | 410,5 |
| [116] | 3-(3-Methoxy-phenyl)-1-oxa-2,8-diaza-spiroj4.5jdec-2-en-8-carbonsäurebenzylamid | 380,5 |
| [117] | 3-Phenyl-1-oxa-2,8-diaza-spiro[4.5]dec-2-en-8-carbonsäurebenzylamid | 350,4 |
| [118] | 3-(4-Chlor-phenyl)-1-oxa-2,8-diaza-spiro[4.5]dec-2-en-8-carbonsäurebenzylamid | 384,9 |
| [119] | 3-(4-tert-Butyl-phenyl)-1-oxa-2,8-diaza-spiro[4.5]dec-2-en-8-carbonsäurebenzylamid | 406,5 |
| [120] | 3-p-Tolyl-1-oxa-2,8-diaza-spiro[4.5]dec-2-en-8-carbonsäurebenzylamid | 364,5 |
| [121] | 3-(4-Trifluormethyl-phenyl)-1-oxa-2,8-diaza-spiro[4.5]dec-2-en-8-carbonsäurebenzylamid | 418,4 |
| [122] | 3-(3-Methoxy-phenyl)-1-oxa-2,8-diaza-spiro[4.5]dec-2-en-8-carbonsäurephenethyl-amid | 394,51 |
| [123] | 3-Phenyl-1-oxa-2,8-diaza-spiro[4.5]dec-2-en-8-carbonsäurephenethyl-amid | 364,5 |
| [124] | 3-(4-Chlor-phenyl)-1-oxa-2,8-diaza-spiro[4.5]dec-2-en-8-carbonsäurephenethyl-amid | 398,9 |
| [125] | 3-(4-tert-Butyl-phenyl)-1-oxa-2,8-diaza-spiro[4.5]dec-2-en-8-carbonsäurephenethyl-amid | 420,6 |
| [126] | 3-p-Tolyl-1-oxa-2,8-diaza-spiro[4.5]dec-2-en-8-carbonsäurephenethyl-amid | 378,5 |
| [127] | 3-(4-Trifluormethyl-phenyl)-1-oxa-2,8-diaza-spiro[4.5]dec-2-en-8-carbonsäurephenethyl-amid | 432,5 |
| [128] | 3-(3-Methoxy-phenyl)-1-oxa-2,8-diaza-spiro[4.5]dec-2-en-8-carbonsäure-4-methyl-benzylamid | 394,5 |
| [129] | 3-Phenyl-1-oxa-2,8-diaza-spiro[4.5]dec-2-en-8-carbonsäure-4-methyl-benzylamid | 364,5 |
| [130] | 3-(4-Chlor-phenyl)-1-oxa-2,8-diaza-spiro[4.5]dec-2-en-8-carbonsäure-4-methyl-benzylamid | 398,9 |
| [131] | 3-(4-tert-Butyl-phenyl)-1-oxa-2,8-diaza-spiro[4.5]dec-2-en-8-carbonsäure-4-methyl-benzylamid | 420,6 |
| [132] | 3-p-Tolyl-1-oxa-2,8-diaza-spiro[4.5]dec-2-en-8-carbonsäure-4-methyl-benzylamid | 378,5 |
| [133] | 3-(4-Trifluormethyl-phenyl)-1-oxa-2,8-diaza-spiro[4.5]dec-2-en-8-carbonsäure-4-methyl-benzylamid | 432,5 |
| [134] | 3-(3-Methoxy-phenyl)-1-oxa-2,8-diaza-spiro[4.5]dec-2-en-8-carbonsäure-4-methoxy-benzylamid | 410,5 |
| [135] | 3-Phenyl-1-oxa-2,8-diaza-spiro[4.5]dec-2-en-8-carbonsäure-4-methoxy-benzylamid | 380,5 |
| [136] | 3-(4-Chlor-phenyl)-1-oxa-2,8-diaza-spiro[4.5]dec-2-en-8-carbonsäure-4-methoxy-benzylamid | 414,9 |
| [137] | 3-(4-tert-Butyl-phenyl)-1-oxa-2,8-diaza-spiro[4.5]dec-2-en-8-carbonsäure-4-methoxy-benzylamid | 436,6 |
| [138] | 3-p-Tolyl-1-oxa-2,8-diaza-spiro[4.5]dec-2-en-8-carbonsäure-4-methoxy-benzylamid | 394,5 |
| [139] | 3-(4-Trifluormethyl-phenyl)-1-oxa-2,8-diaza-spiro[4.5]dec-2-en-8-carbonsäure-4-methoxy-benzylamid | 448,5 |
| [140] | 3-(3-Methoxy-phenyl)-1-oxa-2,8-diaza-spiro[4.5]dec-2-en-8-carbonsäure-(4-tert-butyl-phenyl)-amid | 422,5 |
| [141] | 3-Phenyl-1-oxa-2,8-diaza-spiro[4.5]dec-2-en-8-carbonsäure-(4-tert-butyl-phenyl)-amid | 392,5 |
| [142] | 3-(4-Chlor-phenyl)-1-oxa-2,8-diaza-spiro[4.5]dec-2-en-8-carbonsäure-(4-tert-butyl-phenyl)-amid | 427,0 |
| [143] | 3-(4-tert-Butyl-phenyl)-1-oxa-2,8-diaza-spiro[4.5]dec-2-en-8-carbonsäure-(4-tert-butyl-phenyl)-amid | 448,6 |
| [144] | 3-p-Tolyl-1-oxa-2,8-diaza-spiro[4.5]dec-2-en-8-carbonsäure-(4-tert-butyl-phenyl)-amid | 406,5 |
| [145] | 3-(4-Trifluormethyl-phenyl)-1-oxa-2,8-diaza-spiro[4.5]dec-2-en-8-carbonsäure-(4-tert-butyl-phenyl)-amid | 460,5 |
| [146] | 3-(3-Methoxy-phenyl)-1-oxa-2,8-diaza-spiro[4.5]dec-2-en-8-carbonsäure-p-tolylamid | 380,5 |
| [147] | 3-Phenyl-1-oxa-2,8-diaza-spiro[4.5]dec-2-en-8-carbonsäure-p-tolylamid | 350,4 |
| [148] | 3-(4-Chlor-phenyl)-1-oxa-2,8-diaza-spiro[4.5]dec-2-en-8-carbonsäure-p-tolylamid | 384,9 |
| [149] | 3-(4-tert-Butyl-phenyl)-1-oxa-2,8-diaza-spiro[4.5]dec-2-en-8-carbonsäure-p-tolyiamid | 406,5 |
| [150] | 3-(3-Methoxy-phenyl)-1-oxa-2,8-diaza-spiro[4.5]dec-2-en-8-carbonsäure-o-tolylamid | 380,5 |
| [151] | 3-Phenyl-1-oxa-2,8-diaza-spiro[4.5]dec-2-en-8-carbonsäure-o-tolylamid | 350,4 |
| [152] | 3-(4-Chlor-phenyl)-1-oxa-2,8-diaza-spiro[4.5]dec-2-en-8-carbonsäure-o-tolylamid | 384,9 |
| [153] | 3-(4-tert-Butyl-phenyl)-1-oxa-2,8-diaza-spiro[4.5]dec-2-en-8-carbonsäure-o-tolylamid | 406,5 |
| [154] | 3-p-Tolyl-1-oxa-2,8-diaza-spiro[4.5]dec-2-en-8-carbonsäure-o-tolylamid | 364,5 |
| [155] | 3-(4-Trifluormethyl-phenyl)-1-oxa-2,8-diaza-spiro[4.5]dec-2-en-8-carbonsäure-o-tolylamid | 418,4 |
| [156] | 3-(3-Methoxy-phenyl)-1-oxa-2,8-diaza-spiro[4.5]dec-2-en-8-carbonsäure-(2-ethyl-phenyl)-amid | 394,5 |
| [157] | 3-Phenyl-1-oxa-2,8-diaza-spiro[4.5]dec-2-en-8-carbonsäure-(2-ethyl-phenyl)-amid | 364,5 |
| [158] | 3-(4-Chlor-phenyl)-1-oxa-2,8-diaza-spiro[4.5]dec-2-en-8-carbonsäure-(2-ethyl-phenyl)-amid | 398,9 |
| [159] | 3-(4-tert-Butyl-phenyl)-1-oxa-2,8-diaza-spiro[4.5]dec-2-en-8-carbonsäure-(2-ethyl-phenyl)-amid | 420,6 |
| [160] | 3-p-Tolyl-1-oxa-2,8-diaza-spiro[4.5]dec-2-en-8-carbonsäure-(2-ethyl-phenyl)-amid | 378,5 |
| [161] | 3-(4-Trifluormethyl-phenyl)-1-oxa-2,8-diaza-spiro[4.5]dec-2-en-8-carbonsäure-(2-ethyl-phenyl)-amid | 432,5 |
| [162] | 3-(3-Methoxy-phenyl)-1-oxa-2,8-diaza-spiro[4.5]dec-2-en-8-carbonsäure-(4-ethyl-phenyl)-amid | 394,5 |
| [163] | 3-Phenyl-1-oxa-2,8-diaza-spiro[4.5]dec-2-en-8-carbonsäure-(4-ethyl-phenyl)-amid | 364,5 |
| [164] | 3-(4-Chlor-phenyl)-1-oxa-2,8-diaza-spiro[4.5]dec-2-en-8-carbonsäure-(4-ethyl-phenyl)-amid | 398,9 |
| [165] | 3-(4-tert-Butyl-phenyl)-1-oxa-2,8-diaza-spiro[4.5]dec-2-en-8-carbonsäure-(4-ethyl-phenyl)-amid | 420,6 |
| [166] | 3-p-Tolyl-1-oxa-2,8-diaza-spiro[4.5]dec-2-en-8-carbonsäure-(4-ethyl-phenyl)-amid | 378,5 |
| [167] | 3-(4-Trifluormethyl-phenyl)-1-oxa-2,8-diaza-spiro[4.5]dec-2-en-8-carbonsäure-(4-ethyl-phenyl)-amid | 432,5 |
| [168] | 3-(3-Methoxy-phenyl)-1-oxa-2,8-diaza-spiro[4.5]dec-2-en-8-carbonsäure-(2-fluor-phenyl)-amid | 384,4 |
| [169] | 3-Phenyl-1-oxa-2,8-diaza-spiro[4.5]dec-2-en-8-carbonsäure-(2-fluor-phenyl)-amid | 354,4 |
| [170] | 3-(4-Chlor-phenyl)-1-oxa-2,8-diaza-spiro[4.5]dec-2-en-8-carbonsäure-(2-fluor-phenyl)-amid | 388,8 |
| [171] | 3-(4-tert-Butyl-phenyl)-1-oxa-2,8-diaza-spiro[4.5]dec-2-en-8-carbonsäure-(2-fluor-phenyl)-amid | 410,5 |
| [172] | 3-p-Tolyl-1-oxa-2,8-diaza-spiro[4.5]dec-2-en-8-carbonsäure-(2-fluor-phenyl)-amid | 368,4 |
| [173] | 3-(4-Trifluormethyl-phenyl)-1-oxa-2,8-diaza-spiro[4.5]dec-2-en-8-carbonsäure-(2-fluor-phenyl)-amid | 422,4 |
| [174] | 3-(3-Methoxy-phenyl)-1-oxa-2,8-diaza-spiro[4.5]dec-2-en-8-carbonsäure-(3-fluor-phenyl)-amid | 384,4 |
| [175] | 3-Phenyl-1-oxa-2,8-diaza-spiro[4.5]dec-2-en-8-carbonsäure-(3-fluor-phenyl)-amid | 354,4 |
| [176] | 3-(4-Chlor-phenyl)-1-oxa-2,8-diaza-spiro[4.5]dec-2-en-8-carbonsäure-(3-fluor-phenyl)-amid | 388,8 |
| [177] | 3-(4-tert-Butyl-phenyl)-1-oxa-2,8-diaza-spiro[4.5]dec-2-en-8-carbonsäure-(3-fluor-phenyl)-amid | 410,5 |
| [178] | 3-p-Tolyl-1-oxa-2,8-diaza-spiro[4.5]dec-2-en-8-carbonsäure-(3-fluor-phenyl)-amid | 368,4 |
| [179] | 3-(4-Trifluormethyl-phenyl)-1-oxa-2,8-diaza-spiro[4.5]dec-2-en-8-carbonsäure-(3-fluor-phenyl)-amid | 422,4 |
| [180] | 3-(3-Methoxy-phenyl)-1-oxa-2,8-diaza-spiro[4.5]dec-2-en-8-carbonsäure-(2-chlor-phenyl)-amid | 400,9 |
| [181] | 3-Phenyl-1-oxa-2,8-diaza-spiro[4.5]dec-2-en-8-carbonsäure-(2-chlor-phenyl)-amid | 370,9 |
| [182] | 3-(4-Chlor-phenyl)-1-oxa-2,8-diaza-spiro[4.5]dec-2-en-8-carbonsäure-(2-chlor-phenyl)-amid | 405,3 |
| [183] | 3-(4-tert-Butyl-phenyl)-1-oxa-2,8-diaza-spiro[4.5]dec-2-en-8-carbonsäure-(2-chlor-phenyl)-amid | 427,0 |
| [184] | 3-p-Tolyl-1-oxa-2,8-diaza-spiro[4.5]dec-2-en-8-carbonsäure-(2-chlor-phenyl)-amid | 384,9 |
| [185] | 3-(4-Trifluormethyl-phenyl)-1-oxa-2,8-diaza-spiro[4.5]dec-2-en-8-carbonsäure-(2-chlor-phenyl)-amid | 438,8 |
| [186] | 3-(3-Methoxy-phenyl)-1-oxa-2,8-diaza-spiro[4.5]dec-2-en-8-carbonsäure-(2-bromo-phenyl)-amid | 445,3 |
| [187] | 3-Phenyl-1-oxa-2,8-diaza-spiro[4.5]dec-2-en-8-carbonsäure-(2-bromo-phenyl)-amid | 415,3 |
| [188] | 3-(4-Chlor-phenyl)-1-oxa-2,8-diaza-spiro[4.5]dec-2-en-8-carbonsäure-(2-bromo-phenyl)-amid | 449,8 |
| [189] | 3-(4-tert-Butyl-phenyl)-1-oxa-2,8-diaza-spiro[4.5]dec-2-en-8-carbonsäure-(2-bromo-phenyl)-amid | 471,4 |
| [190] | 3-p-Tolyl-1-oxa-2,8-diaza-spiro[4.5]dec-2-en-8-carbonsäure-(2-bromo-phenyl)-amid | 429,3 |
| [191] | 3-(4-Trifluormethyl-phenyl)-1-oxa-2,8-diaza-spiro[4.5]dec-2-en-8-carbonsäure-(2-bromo-phenyl)-amid | 483,3 |
| [192] | 3-(3-Methoxy-phenyl)-1-oxa-2,8-diaza-spiro[4.5]dec-2-en-8-carbonsäure-(3-bromo-phenyl)-amid | 445,3 |
| [193] | 3-Phenyl-1-oxa-2,8-diaza-spiro[4.5]dec-2-en-8-carbonsäure-(3-bromo-phenyl)-amid | 415,3 |
| [194] | 3-(4-tert-Butyl-phenyl)-1-oxa-2,8-diaza-spiro[4.5]dec-2-en-8-carbonsäure-(3-bromo-phenyl)-amid | 471,4 |
| [195] | 3-(4-Trifluormethyl-phenyl)-1-oxa-2,8-diaza-spiro[4.5]dec-2-en-8-carbonsäure-(3-bromo-phenyl)-amid | 483,3 |
| [196] | 3-(3-Methoxy-phenyl)-1-oxa-2,8-diaza-spiro[4.5]dec-2-en-8-carbonsäure-(4-bromo-phenyl)-amid | 445,3 |
| [197] | 3-Phenyl-1-oxa-2,8-diaza-spiro[4.5]dec-2-en-8-carbonsäure-(4-bromo-phenyl)-amid | 415,3 |
| [198] | 3-(3-Methoxy-phenyl)-1-oxa-2,8-diaza-spiro[4.5]dec-2-en-8-carbonsäure-(2-methoxy-phenyl)-amid | 396,5 |
| [199] | 3-Phenyl-1-oxa-2,8-diaza-spiro[4.5]dec-2-en-8-carbonsäure-(2-methoxy-phenyl)-amid | 366,4 |
| [200] | 3-(4-Chlor-phenyl)-1-oxa-2,8-diaza-spiro[4.5]dec-2-en-8-carbonsäure-(2-methoxy-phenyl)-amid | 400,9 |
| [201] | 3-(4-tert-Butyl-phenyl)-1-oxa-2,8-diaza-spiro[4.5]dec-2-en-8-carbonsäure-(2-methoxy-phenyl)-amid | 422,5 |
| [202] | 3-p-Tolyl-1-oxa-2,8-diaza-spiro[4.5]dec-2-en-8-carbonsäure-(2-methoxy-phenyl)-amid | 380,5 |
| [203] | 3-(4-Trifluormethyl-phenyl)-1-oxa-2,8-diaza-spiro[4.5]dec-2-en-8-carbonsäure-(2-methoxy-phenyl)-amid | 434,4 |
| [204] | 3-(3-Methoxy-phenyl)-1-oxa-2,8-diaza-spiro[4.5]dec-2-en-8-carbonsäure-(3-methoxy-phenyl)-amid | 396,5 |
| [205] | 3-Phenyl-1-oxa-2,8-diaza-spiro[4.5]dec-2-en-8-carbonsäure-(3-methoxy-phenyl)-amid | 366,4 |
| [206] | 3-(4-Chlor-phenyl)-1-oxa-2,8-diaza-spiro[4.5]dec-2-en-8-carbonsäure-(3-methoxy-phenyl)-amid | 400,9 |
| [207] | 3-(4-tert-Butyl-phenyl)-1-oxa-2,8-diaza-spiro[4.5]dec-2-en-8-carbonsäure-(3-methoxy-phenyl)-amid | 422,5 |
| [208] | 3-p-Tolyl-1-oxa-2,8-diaza-spiro[4.5]dec-2-en-8-carbonsäure-(3-methoxy-phenyl)-amid | 380,5 |
| [209] | 3-(4-Trifluormethyl-phenyl)-1-oxa-2,8-diaza-spiro[4.5]dec-2-en-8-carbonsäure-(3-methoxy-phenyl)-amid | 434,4 |
| [210] | 3-(3-Methoxy-phenyl)-1-oxa-2,8-diaza-spiro[4.5]dec-2-en-8-carbonsäure-(4-trifluormethyl-phenyl)-amid | 434,4 |
| [211] | 3-Phenyl-1-oxa-2,8-diaza-spiro[4.5]dec-2-en-8-carbonsäure-(4-trifluormethyl-phenyl)-amid | 404,4 |
| [212] | 3-(4-Chlor-phenyl)-1-oxa-2,8-diaza-spiro[4.5]dec-2-en-8-carbonsäure-(4-trifluormethyl-phenyl)-amid | 438,8 |
| [213] | 3-(4-tert-Butyl-phenyl)-1-oxa-2,8-diaza-spiro[4.5]dec-2-en-8-carbonsäure-(4-trifluormethyl-phenyl)-amid | 460,5 |
| [214] | 3-p-Tolyl-1-oxa-2,8-diaza-spiro[4.5]dec-2-en-8-carbonsäure-(4-trifluormethyl-phenyl)-amid | 418,4 |
| [215] | 3-(4-Trifluormethyl-phenyl)-1-oxa-2,8-diaza-spiro[4.5]dec-2-en-8-carbonsäure-(4-trifluormethyl-phenyl)-amid | 472,4 |
| [216] | 3-(3-Methoxy-phenyl)-1-oxa-2,8-diaza-spiro[4.5]dec-2-en-8-carbonsäure-(4-phenoxy-phenyl)-amid | 458,5 |
| [217] | 3-(4-Chlor-phenyl)-1-oxa-2,8-diaza-spiro[4.5]dec-2-en-8-carbonsäure-(4-phenoxy-phenyl)-amid | 462,9 |
| [218] | 3-(4-tert-Butyl-phenyl)-1-oxa-2,8-diaza-spiro[4.5]dec-2-en-8-carbonsäure-(4-phenoxy-phenyl)-amid | 484,6 |
| [219] | 3-p-Tolyl-1-oxa-2,8-diaza-spiro[4.5]dec-2-en-8-carbonsäure-(4-phenoxy-phenyl)-amid | 442,5 |
| [220] | 3-(4-Trifluormethyl-phenyl)-1-oxa-2,8-diaza-spiro[4.5]dec-2-en-8-carbonsäure-(4-phenoxy-phenyl)-amid | 496,5 |
| [221] | 3-(3-Methoxy-phenyl)-1-oxa-2,8-diaza-spiro[4.5]dec-2-en-8-carbonsäure-(2-chlor-5-trifluormethyl-phenyl)-amid | 468,9 |
| [222] | 3-Phenyl-1-oxa-2,8-diaza-spiro[4.5]dec-2-en-8-carbonsäure-(2-chlor-5-trifluormethyl-phenyl)-amid | 438,8 |
| [223] | 3-(4-Chlor-phenyl)-1-oxa-2,8-diaza-spiro[4.5]dec-2-en-8-carbonsäure-(2-chlor-5-trifluormethyl-phenyl)-amid | 473,3 |
| [224] | 3-(4-tert-Butyl-phenyl)-1-oxa-2,8-diaza-spiro[4.5]dec-2-en-8-carbonsäure-(2-chlor-5-trifluormethyl-phenyl)-amid | 495,0 |
| [225] | 3-p-Tolyl-1-oxa-2,8-diaza-spiro[4.5]dec-2-en-8-carbonsäure-(2-chlor-5-trifluormethyl-phenyl)-amid | 452,9 |
| [226] | 3-(4-Trifluormethyl-phenyl)-1-oxa-2,8-diaza-spiro[4.5]dec-2-en-8-carbonsäure-(2-chlor-5-trifluormethyl-phenyl)-amid | 506,8 |
| [227] | 3-(3-Methoxy-phenyl)-1-oxa-2,8-diaza-spiro[4.5]dec-2-en-8-carbonsäure-(4-chlor-2-trifluormethyl-phenyl)-amid | 468,9 |
| [228] | 3-Phenyl-1-oxa-2,8-diaza-spiro[4.5]dec-2-en-8-carbonsäure-(4-chlor-2-trifluormethyl-phenyl)-amid | 438,8 |
| [229] | 3-(4-Chlor-phenyl)-1-oxa-2,8-diaza-spiro[4.5]dec-2-en-8-carbonsäure-(4-chlor-2-trifluormethyl-phenyl)-amid | 473,3 |
| [230] | 3-(4-tert-Butyl-phenyl)-1-oxa-2,8-diaza-spiro[4.5]dec-2-en-8-carbonsäure-(4-chlor-2-trifluormethyl-phenyl)-amid | 495,0 |
| [231] | 3-p-Tolyl-1-oxa-2,8-diaza-spiro[4.5]dec-2-en-8-carbonsäure-(4-chlor-2-trifluormethyl-phenyl)-amid | 452,9 |
| [232] | 3-(3-Methoxy-phenyl)-1-oxa-2,8-diaza-spiro[4.5]dec-2-en-8-carbonsäure-(4-chlor-3-trifluormethyl-phenyl)-amid | 468,9 |
| [233] | 3-Phenyl-1-oxa-2,8-diaza-spiro[4.5]dec-2-en-8-carbonsäure-(4-chlor-3-trifluormethyl-phenyl)-amid | 438,8 |
| [234] | 3-(4-Chlor-phenyl)-1-oxa-2,8-diaza-spiro[4.5]dec-2-en-8-carbonsäure-(4-chlor-3-trifluormethyl-phenyl)-amid | 473,3 |
| [235] | 3-(4-tert-Butyl-phenyl)-1-oxa-2,8-diaza-spiro[4.5]dec-2-en-8-carbonsäure-(4-chlor-3-trifluormethyl-phenyl)-amid | 495,0 |
| [236] | 3-p-Tolyl-1-oxa-2,8-diaza-spiro[4.5]dec-2-en-8-carbonsäure-(4-chlor-3-trifluormethyl-phenyl)-amid | 452,9 |
| [237] | 3-(4-Trifluormethyl-phenyl)-1-oxa-2,8-diaza-spiro[4.5]dec-2-en-8-carbonsäure-(4-chlor-3-trifluormethyl-phenyl)-amid | 506,8 |
| [238] | 3-(3-Methoxy-phenyl)-1-oxa-2,8-diaza-spiro[4.5]dec-2-en-8-carbonsäure-(2-tert-butyl-6-methyl-phenyl)-amid | 436,6 |
| [239] | 3-Phenyl-1-oxa-2,8-diaza-spiro[4.5]dec-2-en-8-carbonsäure-(2-tert-butyl-6-methyl-phenyl)-amid | 406,5 |
| [240] | 3-(4-Chlor-phenyl)-1-oxa-2,8-diaza-spiro[4.5]dec-2-en-8-carbonsäure-(2-tert-butyl-6-methyl-phenyl)-amid | 441,0 |
| [241] | 3-(4-tert-Butyl-phenyl)-1-oxa-2,8-diaza-spiro[4.5]dec-2-en-8-carbonsäure-(2-tert-butyl-6-methyl-phenyl)-amid | 462,6 |
| [242] | 3-p-Tolyl-1-oxa-2,8-diaza-spiro[4.5]dec-2-en-8-carbonsäure-(2-tert-butyl-6-methyl-phenyl)-amid | 420,6 |
| [243] | 3-(4-Trifluormethyl-phenyl)-1-oxa-2,8-diaza-spiro[4.5]dec-2-en-8-carbonsäure-(2-tert-butyl-6-methyl-phenyl)-amid | 474,5 |
| [244] | 3-(3-Methoxy-phenyl)-1-oxa-2,8-diaza-spiro[4.5]dec-2-en-8-carbonsäure-(4-trifluormethoxy-phenyl)-amid | 450,4 |
| [245] | 3-Phenyl-1-oxa-2,8-diaza-spiro[4.5]dec-2-en-8-carbonsäure-(4-trifluormethoxy-phenyl)-amid | 420,4 |
| [246] | 3-(4-Chlor-phenyl)-1-oxa-2,8-diaza-spiro[4.5]dec-2-en-8-carbonsäure-(4-trifluormethoxy-phenyl)-amid | 454,8 |
| [247] | 3-(4-tert-Butyl-phenyl)-1-oxa-2,8-diaza-spiro[4.5]dec-2-en-8-carbonsäure-(4-trifluormethoxy-phenyl)-amid | 476,5 |
| [248] | 3-p-Tolyl-1-oxa-2,8-diaza-spiro[4.5]dec-2-en-8-carbonsäure-(4-trifluormethoxy-phenyl)-amid | 434,4 |
| [249] | 3-(3-Methoxy-phenyl)-1-oxa-2,8-diaza-spiro[4.5]dec-2-en-8-carbonthiosäurecyclohexylamid | 388,5 |
| [250] | 3-Phenyl-1-oxa-2,8-diaza-spiro[4.5]dec-2-en-8-carbonthiosäurecyclohexylamid | 358,5 |
| [251] | 3-(4-Chlor-phenyl)-1-oxa-2,8-diaza-spiro[4.5]dec-2-en-8-carbonthiosäurecyclohexylamid | 393,0 |
| [252] | 3-(4-tert-Butyl-phenyl)-1-oxa-2,8-diaza-spiro[4.5]dec-2-en-8-carbonthiosäurecyclohexylamid | 414,6 |
| [253] | 3-p-Tolyl-1-oxa-2,8-diaza-spiro[4.5]dec-2-en-8-carbonthiosäurecyclohexylamid | 372,5 |
| [254] | 3-(4-Trifluormethyl-phenyl)-1-oxa-2,8-diaza-spiro[4.5]dec-2-en-8-carbonthiosäurecyclohexylamid | 426,5 |
| [255] | 3-(3-Methoxy-phenyl)-1-oxa-2,8-diaza-spiro[4.5]dec-2-en-8-carbonthiosäurephenylamid | 382,5 |
| [256] | 3-Phenyl-1-oxa-2,8-diaza-spiro[4.5]dec-2-en-8-carbonthiosäurephenylamid | 352,5 |
| [257] | 3-(4-Chlor-phenyl)-1-oxa-2,8-diaza-spiro[4.5]dec-2-en-8-carbonthiosäurephenylamid | 386,9 |
| [258] | 3-(4-tert-Butyl-phenyl)-1-oxa-2,8-diaza-spiro[4.5]dec-2-en-8-carbonthiosäurephenylamid | 408,6 |
| [259] | 3-p-Tolyl-1-oxa-2,8-diaza-spiro[4.5]dec-2-en-8-carbonthiosäurephenylamid | 366,5 |
| [260] | 3-(4-Trifluormethyl-phenyl)-1-oxa-2,8-diaza-spiro[4.5]dec-2-en-8-carbonthiosäurephenylamid | 420,5 |
| [261] | 3-(3-Methoxy-phenyl)-1-oxa-2,8-diaza-spiro[4.5]dec-2-en-8-carbonthiosäure-(3-trifluormethyl-phenyl)-amid | 450,5 |
| [262] | 3-Phenyl-1-oxa-2,8-diaza-spiro[4.5]dec-2-en-8-carbonthiosäure-(3-trifluormethyl-phenyl)-amid | 420,5 |
| [263] | 3-(4-Chlor-phenyl)-1-oxa-2,8-diaza-spiro[4.5]dec-2-en-8-carbonthiosäure-(3-trifluormethyl-phenyl)-amid | 454,9 |
| [264] | 3-(4-tert-Butyl-phenyl)-1-oxa-2,8-diaza-spiro[4.5]dec-2-en-8-carbonthiosäure-(3-trifluormethyl-phenyl)-amid | 476,6 |
| [265] | 3-p-Tolyl-1-oxa-2,8-diaza-spiro[4.5]dec-2-en-8-carbonthiosäure-(3-trifluormethyl-phenyl)-amid | 434,5 |
| [266] | 3-(4-Trifluormethyl-phenyl)-1-oxa-2,8-diaza-spiro[4.5]dec-2-en-8-carbonthiosäure-(3-trifluormethyl-phenyl)-amid | 488,5 |
| [267] | 3-(3-Methoxy-phenyl)-1-oxa-2,8-diaza-spiro[4.5]dec-2-en-8-carbonthiosäure-(2-methoxy-phenyl)-amid | 412,5 |
| [268] | 3-Phenyl-1-oxa-2,8-diaza-spiro[4.5]dec-2-en-8-carbonthiosäure-(2-methoxy-phenyl)-amid | 382,5 |
| [269] | 3-(4-Chlor-phenyl)-1-oxa-2,8-diaza-spiro[4.5]dec-2-en-8-carbonthiosäure-(2-methoxy-phenyl)-amid | 416,9 |
| [270] | 3-(4-tert-Butyl-phenyl)-1-oxa-2,8-diaza-spiro[4.5]dec-2-en-8-carbonthiosäure-(2-methoxy-phenyl)-amid | 438,6 |
| [271] | 3-p-Tolyl-1-oxa-2,8-diaza-spiro[4.5]dec-2-en-8-carbonthiosäure-(2-methoxy-phenyl)-amid | 396,5 |
| [272] | 3-(4-Trifluormethyl-phenyl)-1-oxa-2,8-diaza-spiro[4.5]dec-2-en-8-carbonthiosäure-(2-methoxy-phenyl)-amid | 450,5 |
| [273] | 3-(3-Methoxy-phenyl)-1-oxa-2,8-diaza-spiro[4.5]dec-2-en-8-carbonthiosäure-(3-methoxy-phenyl)-amid | 412,5 |
| [274] | 3-Phenyl-1-oxa-2,8-diaza-spiro[4.5]dec-2-en-8-carbonthiosäure-(3-methoxy-phenyl)-amid | 382,5 |
| [275] | 3-(4-Chlor-phenyl)-1-oxa-2,8-diaza-spiro[4.5]dec-2-en-8-carbonthiosäure-(3-methoxy-phenyl)-amid | 416,9 |
| [276] | 3-p-Tolyl-1-oxa-2,8-diaza-spiro[4.5]dec-2-en-8-carbonthiosäure-(3-methoxy-phenyl)-amid | 396,5 |
| [277] | 3-(4-Trifluormethyl-phenyl)-1-oxa-2,8-diaza-spiro[4.5]dec-2-en-8-carbonthiosäure-(3-methoxy-phenyl)-amid | 450,5 |
| [278] | 3-(3-Methoxy-phenyl)-1-oxa-2,8-diaza-spiro[4.5]dec-2-en-8-carbonthiosäure-(4-tert-butyl-phenyl)-amid | 438,6 |
| [279] | 3-Phenyl-1-oxa-2,8-diaza-spiro[4.5]dec-2-en-8-carbonthiosäure-(4-tert-butyl-phenyl)-amid | 408,6 |
| [280] | 3-(4-Chlor-phenyl)-1-oxa-2,8-diaza-spiro[4.5]dec-2-en-8-carbonthiosäure-(4-tert-butyl-phenyl)-amid | 443,0 |
| [281] | 3-(4-tert-Butyl-phenyl)-1-oxa-2,8-diaza-spiro[4.5]dec-2-en-8-carbonthiosäure-(4-tert-butyl-phenyl)-amid | 464,7 |
| [282] | 3-p-Tolyl-1-oxa-2,8-diaza-spiro[4.5]dec-2-en-8-carbonthiosäure-(4-tert-butyl-phenyl)-amid | 422,6 |
| [283] | 3-(4-Trifluormethyl-phenyl)-1-oxa-2,8-diaza-spiro[4.5]dec-2-en-8-carbonthiosäure-(4-tert-butyl-phenyl)-amid | 476,6 |
| [284] | 3-(3-Methoxy-phenyl)-1-oxa-2,8-diaza-spiro[4.5]dec-2-en-8-carbonsäure-2-methyl-benzylamid | 394,5 |
| [285] | 3-Phenyl-1-oxa-2,8-diaza-spiro[4.5]dec-2-en-8-carbonsäure-2-methyl-benzylamid | 364,5 |
| [286] | 3-(4-Chlor-phenyl)-1-oxa-2,8-diaza-spiro[4.5]dec-2-en-8-carbonsäure-2-methyl-benzylamid | 398,9 |
| [287] | 3-(4-tert-Butyl-phenyl)-1-oxa-2,8-diaza-spiro[4.5]dec-2-en-8-carbonsäure-2-methyl-benzylamid | 420,6 |
| [288] | 3-(4-Chlor-phenyl)-1-oxa-2,8-diaza-spiro[4.5]dec-2-en-8-carbonthiosäurecyclopentylamid | 378,9 |
| [289] | 3-(4-tert-Butyl-phenyl)-1-oxa-2,8-diaza-spiro[4.5]dec-2-en-8-carbonthiosäurecyclopentylamid | 400,6 |
| [290] | 3-p-Tolyl-1-oxa-2,8-diaza-spiro[4.5]dec-2-en-8-carbonthiosäurecyclopentylamid | 358,5 |
| [291] | 3-(4-Trifluormethyl-phenyl)-1-oxa-2,8-diaza-spiro[4.5]dec-2-en-8-carbonthiosäurecyclopentylamid | 412,5 |
| [292] | 3-Phenyl-1-oxa-2,8-diaza-spiro[4.5]dec-2-en-8-carbonthiosäurecyclohexylmethyl-amid | 372,5 |
| [293] | 3-(4-Chlor-phenyl)-1-oxa-2,8-diaza-spiro[4.5]dec-2-en-8-carbonthiosäurecyclohexylmethyl-amid | 407,0 |
| [294] | 3-(4-tert-Butyl-phenyl)-1-oxa-2,8-diaza-spiro[4.5]dec-2-en-8-carbonthiosäurecyclohexylmethyl-amid | 428,7 |
| [295] | 3-p-Tolyl-1-oxa-2,8-diaza-spiro[4.5]dec-2-en-8-carbonthiosäurecyclohexylmethyl-amid | 386,6 |
| [296] | 3-(4-Trifluormethyl-phenyl)-1-oxa-2,8-diaza-spiro[4.5]dec-2-en-8-carbonthiosäurecyclohexylmethyl-amid | 440,5 |
| [297] | 3-(3-Methoxy-phenyl)-1-oxa-2,8-diaza-spiro[4.5]dec-2-en-8-carbonthiosäurecyclooctylamid | 416,6 |
| [298] | 3-Phenyl-1-oxa-2,8-diaza-spiro[4.5]dec-2-en-8-carbonthiosäurecyclooctylamid | 386,6 |
| [299] | 3-(4-Chlor-phenyl)-1-oxa-2,8-diaza-spiro[4.5]dec-2-en-8-carbonthiosäurecyclooctylamid | 421,0 |
| [300] | 3-p-Tolyl-1-oxa-2,8-diaza-spiro[4.5]dec-2-en-8-carbonthiosäurecyclooctylamid | 400,6 |
| [301] | 3-(4-Trifluormethyl-phenyl)-1-oxa-2,8-diaza-spiro[4.5]dec-2-en-8-carbonthiosäurecyclooctylamid | 454,6 |
| [302] | 3-(3-Methoxy-phenyl)-1-oxa-2,8-diaza-spiro[4.5]dec-2-en-8-carbonthiosäure-(2-morpholin-4-yl-ethyl)-amid | 419,6 |
| [303] | 3-tert-Butyl-1-oxa-2,8-diaza-spiro[4.5]dec-2-en-8-carbonsäure-(4-tert-butyl-phenyl)-amid | 372,4 |
| [306] | 3-Phenyl-1-oxa-2,8-diaza-spiro[4.5]dec-2-en-8-carbonsäure-(4-tert-butyl-phenyl)-methyl-amid | 406,5 |
| [310] | 3-tert-Butyl-1-oxa-2,8-diaza-spiro[4.5]dec-2-en-8-carbonsäure-p-tolylamid | 330,4 |
| [311] | 3-(4-Trifluoromethoxy-phenyl)-1-oxa-2,8-diaza-spiro[4.5]dec-2-en-8-carbonsäure-p-tolylamid | 434,4 |
| [312] | 3-(3-Methoxy-phenyl)-1-oxa-2,8-diaza-spiro[4.5]dec-2-en-8-carbonsäure-(4-tert-butyl-phenyl)-amid | 422,6 |
| [319] | 3-Naphthalen-2-yl-1-oxa-2,8-diaza-spiro[4.5]dec-2-en-8-carbonsäure-(4-ethyl-phenyl)-amid | 442,4 |
| [322] | 3-Phenyl-1-oxa-2,8-diaza-spiro[4.5]dec-2-en-8-carbonsäurebenzhydryl-amid | 426,4 |
| [329] | 3-(3-Chloro-pyridin-2-yl)-1-oxa-2,8-diaza-spiro[4.5]dec-2-en-8-carbonsäure-(4-tert-butyl-phenyl)-amid | 427,9 |
| [330] | 3-Phenyl-1-oxa-2,8-diaza-spiro[4.5]dec-2-en-8-carbonthiosäure-(4-pentafluorsulfanyl-phenyl)-amid. | |
| 402 | 3-(3-Methoxy-phenyl)-1-oxa-2,8-diaza-spiro[4.5]dec-2-en-8-carbonsäure-(2,3)-dihydrobenzo[1.4]dioxin-6-yl)-amid | |
| 407 | 3-(4-Isopropyl-phenyl)-1-oxa-2,8-diaza-spiro[4.5]dec-2-en-8-carbonsäure-(4-tert-butyl-phenyl)-amid | |
| 408 | 3-(2,3-Dihydro-benzo[1,4]dioxin-6-yl)-1-oxa-2,8-diaza-spiro[4.5]dec-2-en-8-carbonsäure-(4-tert-butyl-phenyl)amid | |
| 424 | 3-(2,3-Dihydro-benzo[1.4]dioxin-6-yl)-1-oxa-2,8-diaza-spiro[4.5]dec-2-en-8-carbonsäure-(4-trilfluormethyl-phenyl)-amid | |
| 425 | 3-(3-Chlor-phenyl)-1-oxa-2,8-diaza-spiro[4.5]dec-2-en-8-carbonsäure-(4-trilfuormethyl-phenyl)-amid | |
| 427 | 3-(4-Cyclohexyl-phenyl)-1-oxa-2,8-diaza-sprio[4.5]dec-2-en-8-carbonsäure-(4-trifluormethyl-phenyl)amid | |
| [364] | N-(4-tert-Butylphenyl)-3-(2-fluorphenyl)-6-methyl-1-oxa-2,8-diazaspiro[4.5]dec-2-en-8-carboxamid | 424,53 |
| [369] | N-(4-tert-Butylphenyl)-6-methyl-3-phenyl-1-oxa-2,8-diazaspiro[4.5]dec-2-en-8-carboxamid | 406,54 |
| [371] | N-(4-tert-Butylphenyl)-3-phenyl-1-oxa-2,7-diazaspiro[4.4]non-2-en-7-carboxamid | 378,49 |
| [372] | N-(4-tert-Butylbenzyl)-3-phenyl-1-oxa-2,8-diazaspiro[4.5]dec-2-en-8-carboxamid | 406,54 |
| [373] | N-(4-tert-Butylphenyl)-3-phenyl-1-oxa-2,8-diazaspiro[4.6]undec-2-en-8-carboxamid | 406,54 |
| [374] | N-(4-tert-Butylbenzyl)-3-(3-methoxyphenyl)-1-oxa-2,8-diazaspiro[4.5]dec-2-en-8-carboxamid | 436,57 |
| [375] | N-(1-(4-tert-Butylphenyl)ethyl)-3-(3-methoxyphenyl)-1-oxa-2,8-diazaspiro[4.5]dec-2-en-8-carboxamid | 450,59 |
| [376] | N-(4-tert-Butylcyclohexyl)-3-phenyl-1-oxa-2,8-diazaspiro[4.5]dec-2-en-8-carboxamid | 398,56 |
| [377] | N-(4-tert-Butylphenethyl)-3-phenyl-1-oxa-2,8-diazaspiro[4.5]dec-2-en-8-carboxamid | 420,57 |
| [378] | N-(4-tert-Butylphenyl)-3-(4-chlor-3-methoxyphenyl)-1-oxa-2,8-diazaspiro[4.5]dec-2-en-8-carboxamid | 456,99 |
| [385] | 6-Methyl-3-phenyl-N-(4-(trifluormethyl)phenyl)-1-oxa-2,8-diazaspiro[4.5]dec-2-en-8-carboxamid | 418,43 |
| [386] | 3-Benzyl-N-(4-tert-butylphenyl)-1-oxa-2,8-diazaspiro[4.5]dec-2-en-8-carboxamid | 406,54 |
| [387] | N-(4-tert-Butylphenyl)-3-phenethyl-1-oxa-2,8-diazaspiro[4.5]dec-2-en-8-carboxamid | 420,57 |

### 9. Umsetzung von Aminen der allgemeinen Formel II mit Carbonsäuren der allgemeinen Formel R⁹-C(=O)-OH

### a. Manuelle Synthese

### Synthese von Beispielverbindung 307: (4-tert-Butyl-phenyl)-(3-phenyl-1-oxa-2,8-diaza-spiro[4-5]dec-2-en-8-yl)-methanon

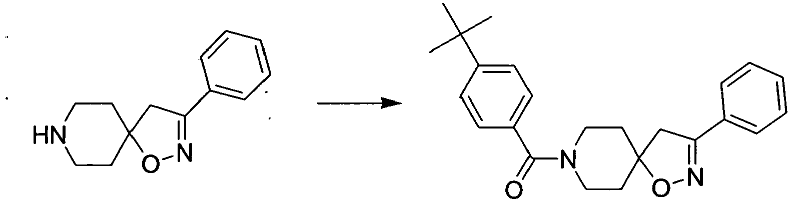

Zu einer Lösung von N-Ethyldiisopropylamin (560 µl, 3 mmol), 1-Hydroxybenzotriazolhydrat (133 mg, 1 mmol), O-(Benzotriazol-1-yl)-N,N,N',N'-tetramethyluroniomtetrafluoroborat (318 mg, 1 mmol) und 4-tert-Butylbenzoesäure (176 mg, 1mmol) in abs. THF (8 ml) wurde langsam 3-Phenyl-1-oxa-2,8-diaza-spiro[4.5]dec-2-en (E) als Hydrochloridsalz (250 mg) gegeben. Die Reaktionsmischung wurde über Nacht gerührt und mit EtOAc verdünnt. Die organische Phase wurde nacheinander mit ges. aq. NaCl-Lsg., ges. aq. NaHCO₃-Lsg., ges. aq. NaCl-Lsg. und ges. aq. NH₄Cl-Lsg. gewaschen und das Lösungsmittel im Vakuum entfernt. Das gewünschte Produkt (4-tert-Butyl-phenyl)-(3-phenyl-1-oxa-2,8-diaza-spiro[4.5]dec-2-en-8-yl)-methanon wurde in einer Ausbeute von 0.42 g erhalten.

### Synthese von Beispielverbindung 325: 3-(4-Isopropyl-phenyl)-1-(3-phenyl-1-oxa-2,8-diaza-spiro[4.5]dec-2-en-8-yl)-propenon

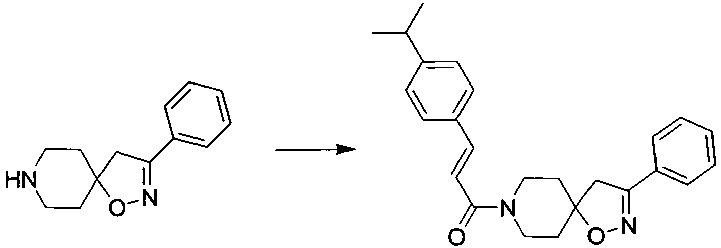

Zu einer Lösung von N-Ethyldiisopropylamin (560 µl, 3 mmol), 1-Hydroxybenzotriazolhydrat (133 mg, 1 mmol), O-(Benzotriazol-1-yl)-N,N,N',N'-tetramethyluroniomtetrafluoroborat (318 mg, 1 mmol) und 4-Isopropylzimtsäure (188 mg, 1 mmol) in abs. THF (8 ml) wurde langsam 3-Phenyl-1-oxa-2,8-diaza-spiro[4.5]dec-2-en (E) als Hydrochloridsalz (250 mg) gegeben. Die Reaktionsmischung wurde über Nacht gerührt und mit EtOAc verdünnt. Die organische Phase wurde nacheinander mit ges. aq. NaCl-Lsg., ges. aq. NaHCO₃-Lsg., ges. aq. NaCl-Lsg. und ges. aq. NH₄Cl-Lsg. gewaschen und das Lösungsmittel im Vakuum entfernt. Das gewünschte Produkt 3-(4-Isopropyl-phenyl)-1-(3-phenyl-1-oxa-2,8-diaza-spiro[4.5]dec-2-en-8-yl)-propenon wurde in einer Ausbeute von 0.45 g erhalten.

### Synthese von Beispielverbindung 370: 2-(4-tert-Butylphenyl)-1-(3-phenyl-1-oxa-2,8-diazaspiro[4.5]dec-2-en-8-yl)propan-1-on

### Synthese von 2-(4-tert-Butylphenyl)propionsäure-methylester

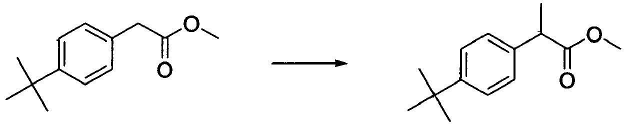

Eine Lösung von Methyl-2-(4-tert-butylphenyl)acetat (1 g, 4.85 mmol) in abs. THF (30 ml) wurde bei einer Temperatur von - 70 °C innerhalb von 15 min mit einer 1 M Lösung von Bis-(trimethylsilyl)lithiumamid in THF (5.33 ml, 5.33 mmol) versetzt. Die Reaktion war stark exotherm. Die Mischung wurde 20 min bei -70 °C gehalten und bei dieser Temperatur Methyljodid (0.452 ml, 1.03 g, 7.26 mmol) innerhalb von 5 min hinzugefügt. Innerhalb von 16 h wurde der Ansatz auf RT erwärmt. Zur Aufarbeitung wurde die Reaktionsmischung mit ges. NH₄Cl-Lsg. (30 ml) versetzt. Es entstanden zwei Phasen sowie als Feststoff Ammoniumchlorid, der durch Filtration und Waschen mit THF (2 x 8 ml) abgetrennt wurde. Das Filtrat wurde mit DCM (70 ml) versetzt und die Phasen getrennt. Die wäßrige Phase wurde mit DCM (2 x 20 ml) extrahiert. Die organischen Phasen wurden vereinigt, getrocknet und eingeengt. Es blieb ein farbloses Öl (1,08 g) zurück, das chromatographisch gereinigt wurde [Kieselgel 60 (50 g); Cyclohexan (800 ml), EtOAc/Cyclohexan 1:30 (600 ml)]. 2-(4-*tert-*Butylphenyl)-propionsäure-methylester wurde als farbloses Öl in einer Ausbeute von 89 % (946 mg) erhalten.

### Synthese von 2-(4-tert-Butylphenyl)propionsäure

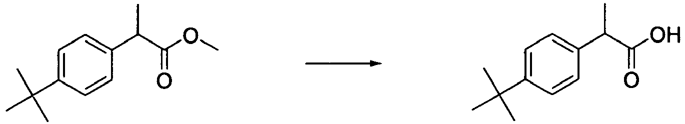

2-(4-*tert*-Butylphenyl)-propionsäure-methylester (946 mg, 4.29 mmol) wurde in MeOH (10 ml) gelöst und mit einer Lösung von Lithiumhydroxid (154 mg, 6.44 mmol) in Wasser (5 ml) versetzt. Die Reaktionsmischung wurde 18 h bei RT gerührt. Das Lösungsmittel wurde im Vakuum entfernt und der wäßrige Rückstand mit Diethylether (30 ml) versetzt. Die Phasen wurden getrennt. Die wäßrige Phase wurde nochmals mit Diethylether (20 ml) extrahiert. Die wäßrige Phase wurde mit 1 N Salzsäure-Lsg. auf einen pH-Wert von 3 eingestellt. Dabei entstand eine Trübung bzw. Fällung, die durch Zugabe von EtOAc (2 x 20 ml) aus der wäßrigen Phase extrahiert wurde. Die organische Phase wurde mit ges. NaCl-Lsg. (20 ml) gewaschen, getrocknet und eingeengt. 2-(4-*tert*-Butylphenyl)propionsäure wurde als farbloser Feststoff in einer Ausbeute von 83 % (735 mg) erhalten.

### Synthese von 2-(4-tert-Butylphenyl)-1-(3-phenyl-1-oxa-2,8-diazaspiro[4.5]dec-2-en-8-yl)propan-1-on

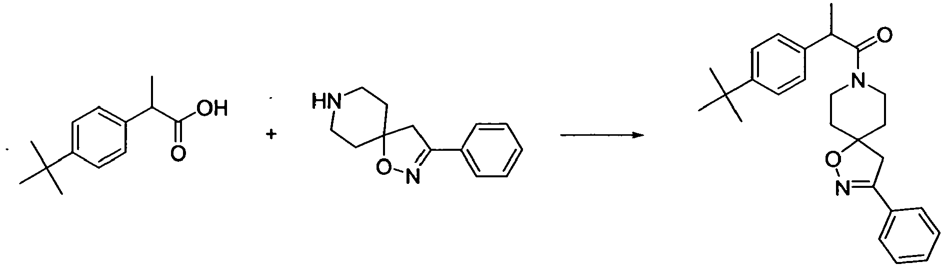

Das Hydrochlorid der Verbindung E (1.5 g, 5.9 mmol) wurde in Wasser (20 ml) gelöst, mit ges. NaHCO₃-Lsg. (20 ml) versetzt und 1 h bei RT gerührt. Dabei fiel Verbindung E aus, die durch Filtration abgetrennt wurde. Verbindung E wurde als farbloser Feststoff in einer Ausbeute von 92 % (1,17g) mit einem Schmelzpunkt von 115 °C gewonnen.

Eine Lösung von 2-(4-*tert*-Butylphenyl)propionsäure(300 mg, 1,45 mmol) in abs. DMF (25 ml) wurde mit *N*,*N*'-Carbonyldiimidazol (235 mg, 1,45 mmol) versetzt und 1.5 h bei RT gerührt. Anschließend erfolgte die Zugabe von Verbindung E (346 mg, 1.6 mmol). Nach einer Reaktionszeit von 4 d bei RT wurde die klare Reaktionsmischung eingeengt. Der Rückstand wurde in DCM (30 ml) und 0,5 N Salzsäure-Lsg. (20 ml) aufgenommen Die Phasen wurden getrennt. Die organische Phase wurde nacheinander mit 0,5 N Salzsäure-Lsg. (20 ml), ges. NaHCO₃-Lsg. (2 x 15 ml) und ges. NaCl-Lsg. (15 ml) gewaschen. Die organische Phase wurde getrocknet und eingeengt. Der feste farblose Rückstand wurde in einem Gemisch aus Diethylether (10 ml) und *n*-Hexan (10 ml) aufgenommen und 15 min bei RT gerührt. 22-(4-tert-Butylphenyl)-1-(3-phenyl-1-oxa-2,8-diazaspiro[4.5]dec-2-en-8-yl)propan-1-on wurde als farbloser Feststoff in einer Ausbeute von 62 % (364 mg) mit einem Schmelzpunkt von 143-145 °C gewonnen.

Die folgenden Carbonsäuren der allgemeinen Formeln HO-C(=O)-R⁹ können bevorzugt verwendet werden:

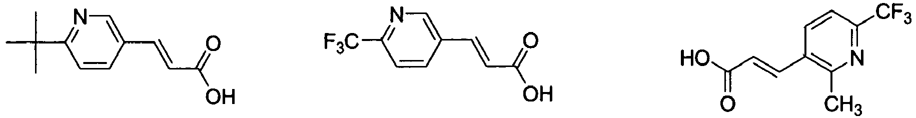

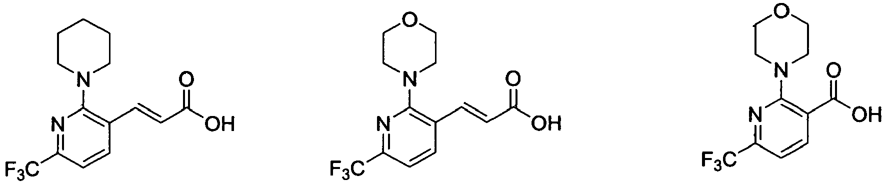

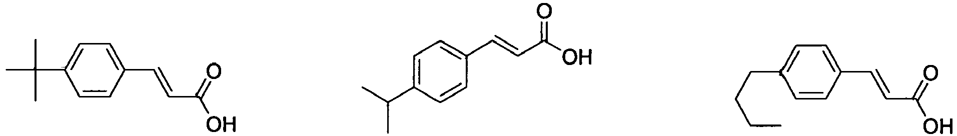

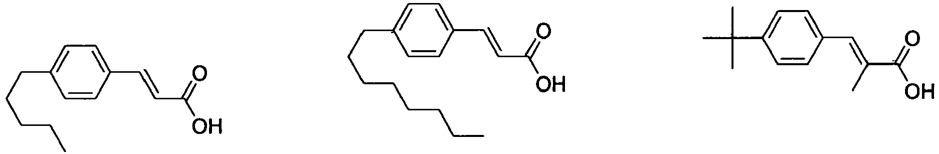

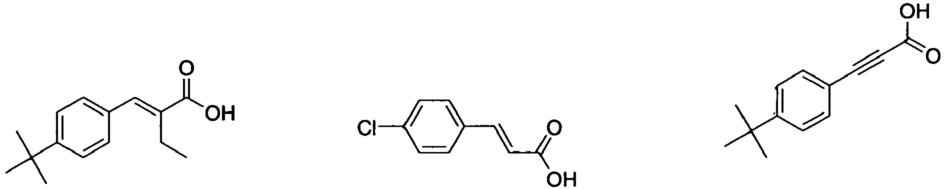

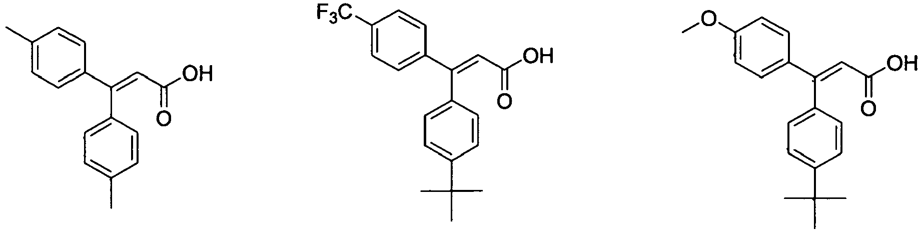

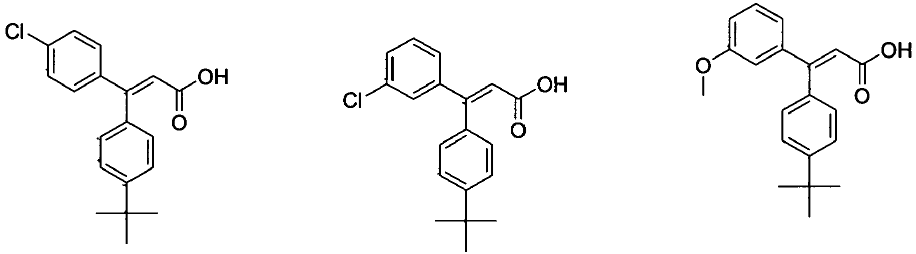

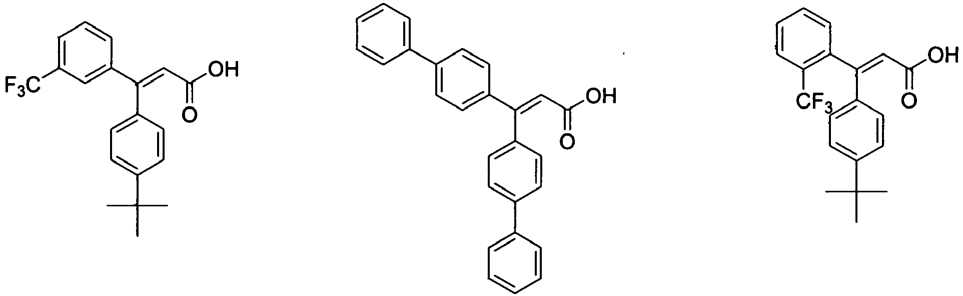

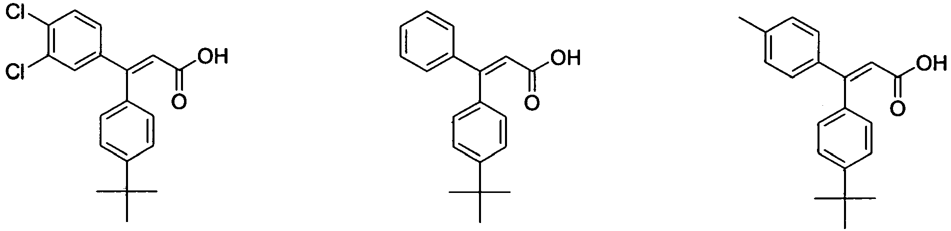

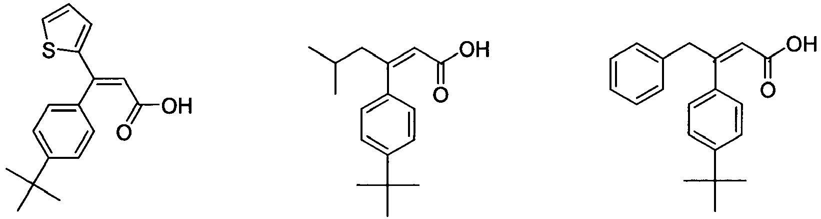

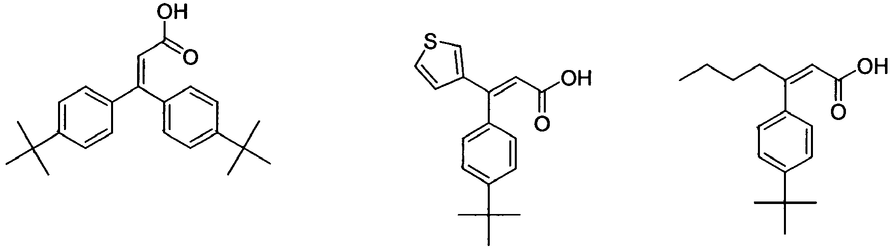

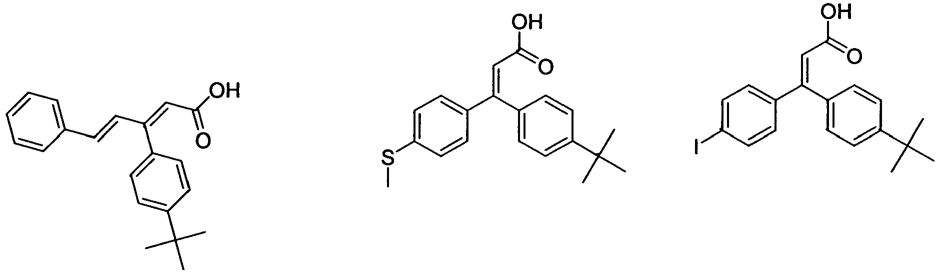

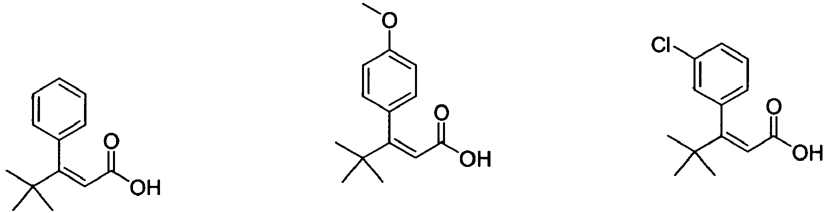

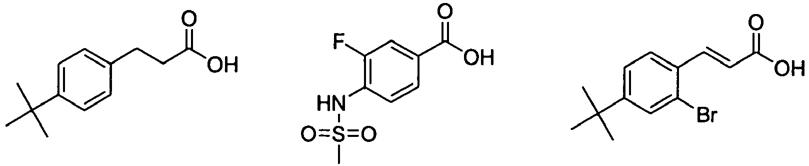

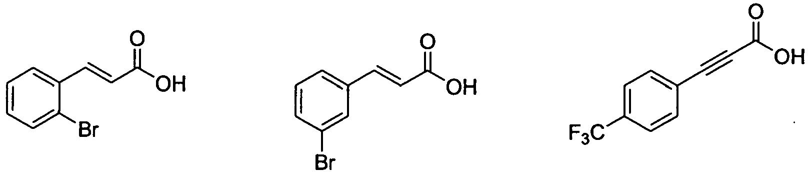

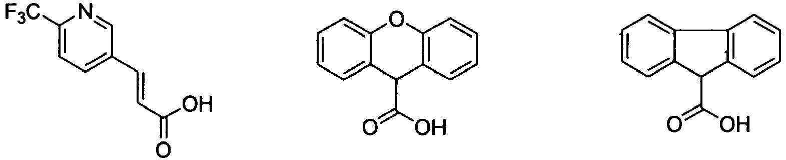

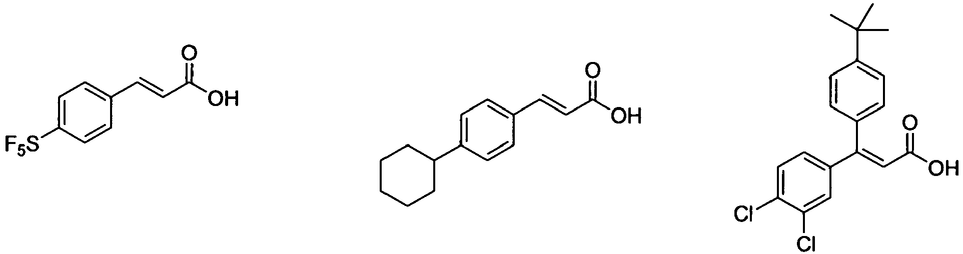

Die folgenden erfindungsgemäßen substituierten Spiro-Verbindungen wurden wie unter 2a. beschrieben hergestellt.

| | Name | [M+H] |
|---|---|---|
| 304 | 3-Phenyl-1-(3-phenyl-1-oxa-2,8-diaza-spiro[4.5]dec-2-en-8-yl)-propinon | 345,5 |
| 305 | 1-(3-tert-Butyl-1-oxa-2,8-diaza-spiro[4.5]dec-2-en-8-yl)-3-phenyl-propinon | 325,5 |
| 308 | (4-Hydroxy-3-methoxy-phenyl)-(3-phenyl-1-oxa-2,8-diaza-spiro[4.5]dec-2-en-8-yl)-methanon | 367,4 |
| 309 | (4-Iodo-phenyl)-(3-phenyl-1-oxa-2,8-diaza-spiro[4.5]dec-2-en-8-yl)-methanon | 447,2 |
| 313 | 2-(4-tert-Butyl-phenyl)-1-(3-phenyl-1-oxa-2,8-diaza-spiro[4.5]dec-2-en-8-yl)-ethanon | 391,4 |
| 314 | 1-(3-Phenyl-1-oxa-2,8-diaza-spiro[4.5]dec-2-en-8-yl)-3-(4-trifluoromethyl-phenyl)-propenon | 415,5 |
| 315 | 3-(4-Hydroxy-3-methoxy-phenyl)-1-(3-phenyl-1-oxa-2,8-diaza-spiro[4.5]dec-2-en-8-yl)-propenon | 393,6 |
| 316 | (4-tert-Butyl-phenyl)-[3-(4-tert-butyl-phenyl)-1-oxa-2,8-diaza-spiro[4.5]dec-2-en-8-yl]-methanon | 384,2 |
| 317 | 3-(4-tert-Butyl-phenyl)-1-[3-(4-tert-butyl-phenyl)-1-oxa-2,8-diaza-spiro[4.5]dec-2-en-8-yl]-propenon | 459,7 |
| 318 | 3-(4-tert-Butyl-phenyl)-1-[3-(4-trifluoromethoxy-phenyl)-1-oxa-2,8-diaza-spiro[4.5]dec-2-en-8-yl]-propenon | 487,5 |
| 320 | 1-(3-Phenyl-1-oxa-2,8-diaza-spiro[4.5]dec-2-en-8-yl)-3,3-di-p-tolyl-propenon | 451,3 |
| 321 | 3-(4-tert-Butyl-phenyl)-2-methyl-1-(3-phenyl-1-oxa-2,8-diaza-spiro[4.5]dec-2-en-8-yl)-propenon | 417,8 |
| 323 | 3-(4-tert-Butyl-phenyl)-1-(3-phenyl-1-oxa-2,8-diaza-spiro[4.5]dec-2-en-8-yl)-propenon | 403,5 |
| 324 | 2-(4-tert-Butyl-benzylidene)-1-(3-phenyl-1-oxa-2,8-diaza-spiro[4.5]dec-2-en-8-yl)-butan-1-on | 431,5 |
| 326 | 3-(4-Octyl-phenyl)-1-(3-phenyl-1-oxa-2,8-diaza-spiro[4.5]dec-2-en-8-yl)-propenon | 459,6 |
| 327 | 3-(4-Butyl-phenyl)-1-(3-phenyl-1-oxa-2,8-diaza-spiro[4.5]dec-2-en-8-yl)-propenon | 403,4 |
| 328 | 3-(4-Pentyl-phenyl)-1-(3-phenyl-1-oxa-2,8-diaza-spiro[4.5]dec-2-en-8-yl)-propenon | 417,7 |
| 388 | 3-(4-Hydroxy-3-methoxy-phenyl)-1-(3-phenyl-1-oxa-2,8-diaza-spiro[4.5]decen-8-yl)-propenon | |
| 389 | 3-(4-tert-Butyl-phenyl)-1-(3-phenyl-1-oxa-2,8-diaza-spiro[4.5]decen-8-yl)-propenon | |
| 391 | 3,3-Di-p-tolyl-1-(3-p-tolyl-1-oxa-2,8-diaza-spiro[4.5]dec-2-en-8-yl)-propenon | |
| 392 | [3-(4-tert-Butyl-phenyl)-1-[3-(4-chlor-phenyl)1-oxa-2,8-diaza-spiro[4.5]dec-2-en-8-yl]-propenon | |
| 393 | 3-(4-tert-Butyl-phenyl)-2-ethyl-1-(3-p-tolyl-1-oxa-2,8-diaza-spiro[4.5]dec-2-en-8-yl)-propenon | |
| 394 | (4-tert-Butyl-phenyl)-(3-p-tolyl-1-oxa-2,8-diaza-spiro[4.5]dec-2-en-8-yl)-methanon | |
| 395 | 3-(4-tert-Butyl-phenyl)-1-(3-p-tolyl-1-oxa-2,8-diaza-spiro[4.5]dec-2-en-8-yl)-propenon | |
| 396 | 3-(4-tert-Butyl-phenyl)-2-methyl-1-(3-p-tolyl-1-oxa-2,8-diaza-spiro[4.5]dec-2-en-8-yl)-propenon | |
| 397 | 2-(4-tert-Butyl-phenyl)-1-[3-(3-methoxy-phenyl)-1-oxa-2,8-diaza-spiro[4.5]dec-2-en-8-yl]-but-2-en-1-on | |
| 398 | 3-(4-tert-Butyl-phenyl)-1-[3-(3-methoxy-phenyl)-1-oxa-2,8-diaza-spiro[4.5]dec-2-en-8-yl]-propinon | |
| 399 | 3-(4-tert-Butyl-phenyl)-1-[3-(3-methoxy-phenyl)-1-oxa-2,8-diaza-spiro[4.5]dec-2-en-8-yl]-propenon | |
| 400 | 1-[3-(3-Methoxy-phenyl)-1-oxa-2,8-diaza-spiro[4.5]dec-2-en-8-yl]-2,2-diphenyl-propan-1-on | |
| 401 | 1-[3-(3-Methoxy-phenyl)-1-oxa-2,8-diaza-spiro[4.5]dec-2-en-8-yl]-2,2-diphenyl-ethanon | |
| 403 | 3-(6-tert-Butyl-pyridin-3-yl)-1-[3-(3-methoxy-phenyl)-1-oxa-2,8-diaza-spiro[4.5]dec-2-en-8-yl]-propenon | |
| 404 | 3-(6-tert-Butyl-pyridin-3-yl)-1-[3-(4-chlor-3-methoxy-phenyl)-1-oxa-2,8-diaza-spiro[4.5]dec-2-en-8-yl]-propenon | |
| 405 | 2,2-Diphenyl-1-(3-phenyl-1-oxa-2,8-diaza-spiro[4.5]dec-2-en-8-yl)-propan-1-on | |
| 406 | 3-(6-tert-Butyl-pyridin-3-yl)-1-(3-phenyl-1-oxa-2,8-diaza-spiro[4.5]dec-2-en-8-yl)-propenon | |
| 409 | N-[2-Fluor-4-[3-(3-methoxy-phenyl)-1-oxa-2,8-diaza-spiro[4.5][dec-2-en-8-carbonyl]-phenyl]-methansulfonamid | |
| 410 | N-[2-Fluor-4-(3-phenyl-1-oxa-2,8-diaza-spiro[4.5][dec-2-en-8-carbonyl]-phenyl]-methansulfonamid | |
| 411 | 3-(4-Cyclohexyl-phenyl)-1-(3-phenyl-1-oxa-2,8-diaza-spiro[4.5]dec-2-en-8-yl)-propenon | |
| 412 | 1-[3-(3-Methoxy-phenyl)-1-oxa-2,8-diaza-spiro[4.5]dec-2-en-8-yl]-2,2,2-triphenyl-ethanon | |
| 413 | [3-(3-Methoxy-phenyl)-1-oxa-2,8-diaza-spiro[4.5]dec-2-en-8-yl]-9H-xanthen-9-yl)-methanon | |
| 414 | [3-(4-Chlor-3-methoxy-phenyl)-1-oxa-2,8-diaza-spiro[4.5]dec-2-en-8-yl]-(9H-fluoren-9-yl)-methanon | |
| 415 | [3-(4-Chlor-3-methoxy-phenyl)-1-oxa-2,8-diaza-spiro[4.5]dec-2-en-8-yl]-(2-chlor-6-trifluormethyl-pyridin-3-yl)-methanon | |
| 416 | [3-(3-Methoxy-phenyl)-1-oxa-2,8-diaza-spiro[4.5]dec-2-en-8-yl]-(2-morpholin-4-yl-6-trifluormethyl-pyridin-3-yl)-methanon | |
| 417 | 3-(4-tert-Butyl-phenyl)-1-[3-(3-chlor-phenyl)-1-oxa-2,8-diaza-spiro[4.5]dec-2-en-8-yl]-propenon | |
| 418 | 3-(4-tert-Butyl-phenyl)-1-[3-(2,3-dihydro-benzo[1.4]dioxin-6-yl)-1-oxa-2,8-diaza-spiro[4.5]dec-2-en-8-yl-propenon | |
| 419 | 3-(4-tert-Butyl-phenyl)-1-[3-(4-cyclohexyl-phenyl)-1-oxa-2,8-diaza-spiro[4.5]dec-2-en-8-yl]-propenon | |
| 420 | N-[4-[3-(4-Cyclohexyl-phenyl)-1-oxa-2,8-diaza-spiro[4.5]dec-2-en-8-carbonyl]-2-fluor-phenyl]-methansulfonamid | |
| 421 | 2-Hydroxy-2,2-diphenyl-1-(3-phenyl-1-oxa-2,8-diaza-spiro[4.5]dec-2-8-yl]-ethanon | |
| 422 | (3-Phenyl-1-oxa-2,8-diaza-spiro[4.5]dec-2-en-8-yl)-(9H-xanthen-9-yl)-methanon | |
| 423 | 3-(4-tert-Butyl-phenyl)-1-(3-chinolin-3-yl-1-oxa-2,8-diaza-spiro[4.5]dec-2-en-8-yl)-propenon | |
| | | |
| 426 | (4-tert-Butyl-phenyl)-[3-(3-methoxy-phenyl)-1-oxa-2,8-diaza-spiro[4.5]dec-2-en-8-yl]-methanon | |
| 428 | 3-(4-tert-Butyl-phenyl)-3-(4-chlor-phenyl)-1-[3-(3-chloro-phenyl)-1-oxa-2,8-diaza-spiro[4.5]dec-2-en-8-yl]-propenon | |
| 429 | 3-(4-tert-Butyl-phenyl)-1-[3-(3-chlor-phenyl)-1-oxa-2,8-diaza-spiro[4.5]dec-2-en-8-yl]-3-(3-methoxy-phenyl)-propenon | |
| 430 | 3-(4-tert-Butyl-phenyl)-1-[3-(4-chlor-3-methoxy-phenyl)-1-oxa-2,8-diaza-spiro[4.5]dec-2-en-8-yl]-3-(4-trifluormethyl-phenyl)-propenon | |
| 431 | 3-(4-Pentafluorsulfanyl-phenyl)-1-[3-(3-methoxy-phenyl)-1-oxa-2,8-diaza-spiro[4.5]dec-2-en-8-yl]-propenon | |
| 432 | 3-(4-tert-Butyl-phenyl)-1-[3-(4-chlor-3-methoxy-phenyl)-1-oxa-2,8-diaza-spiro[4.5]dec-2-en-8-yl]-propenon | |
| 433 | [3-(4-tert-Butyl-phenyl)-1-[3-(4-chlor-3-methoxy-phenyl)-1-oxa-2,8-diaza-spiro[4.5]dec-2-en-8-yl]-propinon | |
| 434 | 3-(4-tert-Butyl-phenyl)-1-[3-(3-methoxy-phenyl)-1-oxa-2,8-diaza-spiro[4.5]dec-2-en-8-yl]-3-(2-trifluormethyl-phenyl)-propenon | |
| 437 | 3,3-Bis-(4-tert-butyl-phenyl)-1-(3-phenyl-1-oxa-2,8-diaza-spiro[4.5]dec-2-en-8-yl)-propenon | |
| [331] | (3-(4-Chlor-3-methoxyphenyl)-1-oxa-2,8-diazaspiro[4.5]dec-2-en-8-yl)(9H-xanthen-9-yl)methanon | 489,97 |
| [332] | (9H-Fluoren-9-yl)(3-(3-methoxyphenyl)-1-oxa-2,8-diazaspiro[4.5]dec-2-en-8-yl)methanon | 439,53 |
| [333] | N-(4-tert-Butylphenyl)-3-(3-chlorphenyl)-1-oxa-2,8-diazaspiro[4.5]dec-2-en-8-carboxamid | 426,96 |
| [334] | (E)-3-(4-Cyclohexylphenyl)-1-(3-(3-methoxyphenyl)-1-oxa-2,8-diazaspiro[4.5]dec-2-en-8-yl)prop-2-en-1-on | 459,60 |
| [335] | N-(4-tert-Butylphenyl)-3-(4-cyclohexylphenyl)-1-oxa-2,8-diazaspiro[4.5]dec-2-en-8-carboxamid | 474.66 |
| [336] | (E)-3-(4-tert-Butylphenyl)-3-phenyl-1-(3-phenyl-1-oxa-2,8-diazaspiro[4.5]dec-2-en-8-yl)prop-2-en-1-on | 479,64 |
| [337] | (2E,4E)-3-(4-tert-Butylphenyl)-5-phenyl-1-(3-phenyl-1-oxa-2,8-diazaspiro[4.5]dec-2-en-8-yl)penta-2,4-dien-1-on | 505,67 |
| [338] | (Z)-3-(4-tert-Butylphenyl)-1-(3-phenyl-1-oxa-2,8-diazaspiro[4.5]dec-2-en-8-yl)-3-(thiophen-3-yl)prop-2-en-1-on | 485,66 |
| [339] | (E)-3-(4-tert-Butylphenyl)-1-(3-(3-methoxyphenyl)-1-oxa-2,8-diazaspiro[4.5]dec-2-en-8-yl)-3-(4-(trifluormethyl)phenyl)prop-2-en-1-on | 577,66 |
| [340] | (E)-N-(5-(8-(3-(4-tert-Butylphenyl)acryloyl)-1-oxa-2,8-diazaspiro[4.5]dec-2-en-3-yl)-2-fluorphenyl)methanesulfonamid | 514,63 |
| [341] | (E)-3-(4-Pentafluorsulfanyl)-1-(3-(3-methoxyphenyl)-1-oxa-2,8-diazaspiro[4.5]dec-2-en-8-yl)prop-2-en-1-on | 503,50 |
| [342] | (E)-1-(3-(3-Methoxyphenyl)-1-oxa-2,8-diazaspiro[4.5]dec-2-en-8-yl)-3-(2-morpholino-6-(trifluormethyl)pyridin-3-yl)prop-2-en-1-on | 531,55 |
| [343] | N-(2-Fluor-4-(1-oxo-1-(3-phenyl-1-oxa-2,8-diazaspiro[4.5]dec-2-en-8-yl)propan-2-yl)phenyl)methanesulfonamid | 460,54 |
| [344] | N-(2-Fluor-4-(1-(3-(3-methoxyphenyl)-1-oxa-2,8-diazaspiro[4.5]dec-2-en-8-yl)-1-oxopropan-2-yl)phenyl)methanesulfonamid | 490,57 |
| [345] | (Z)-3-(4-tert-Butylphenyl)-1-(3-(3-methoxyphenyl)-1-oxa-2,8-diazaspiro[4.5]dec-2-en-8-yl)-3-(thiophen-3-yl)prop-2-en-1-on | 515,69 |
| [346] | (E)-3-(3-Bromphenyl)-1-(3-phenyl-1-oxa-2,8-diazaspiro[4.5]dec-2-en-8-yl)prop-2-en-1-on | 426,33 |
| [347] | (E)-3-(2-Bromphenyl)-1-(3-phenyl-1-oxa-2,8-diazaspiro[4.5]dec-2-en-8-yl)prop-2-en-1-on | 426,33 |
| [348] | 3-(3-Fluorphenyl)-N-(4-(trifluormethyl)phenyl)-1-oxa-2,8-diazaspiro[4.5]dec-2-en-8-carboxamid | 422,39 |
| [349] | N-(4-tert-Butylphenyl)-3-(4-fluorphenyl)-1-oxa-2,8-diazaspiro[4.5]dec-2-en-8-carboxamid | 410,50 |
| [350] | 3-(4-tert-Butylphenyl)-1-(3-phenyl-1-oxa-2,8-diazaspiro[4.5]dec-2-en-8-yl)propan-1-on | 405,55 |
| [351] | N-(4-tert-Butylphenyl)-3-(3-fluorphenyl)-1-oxa-2,8-diazaspiro[4.5]dec-2-en-8-carboxamid | 410,50 |
| [352] | N-(4-tert-Butylphenyl)-3-(2-fluorphenyl)-1-oxa-2,8-diazaspiro[4.5]dec-2-en-8-carboxamid | 410,50 |
| [353] | (E)-3-(4-tert-Butylphenyl)-1-(3-(3-fluorphenyl)-1-oxa-2,8-diazaspiro[4.5]dec-2-en-8-yl)prop-2-en-1-on | 421,53 |
| [354] | (Z)-1-(3-(3-Methoxyphenyl)-1-oxa-2,8-diazaspiro[4.5]dec-2-en-8-yl)-4,4-dimethyl-3-phenylpent-2-en-1-on | 433,56 |
| [355] | (E)-1-(3-Phenyl-1-oxa-2,8-diazaspiro[4.5]dec-2-en-8-yl)-3-(2-(piperidin-1-yl)-6-(trifluormethyl)pyridin-3-yl)prop-2-en-1-on | 499,55 |
| [356] | (E)-1-(3-(3-Chlorphenyl)-1-oxa-2,8-diazaspiro[4.5]dec-2-en-8-yl)-3-(2-(piperidin-1-yl)-6-(trifluormethyl)pyridin-3-yl)prop-2-en-1-on | 533,99 |
| [357] | (2E,4E)-3-tert-Butyl-1-(3-(3-methoxyphenyl)-1-oxa-2,8-diazaspiro[4.5]dec-2-en-8-yl)-5-phenylpenta-2,4-dien-1-on | 459,60 |
| [358] | (2E,4E)-3-(4-tert-Butylphenyl)-1-(3-(3-fluorphenyl)-1-oxa-2,8-diazaspiro[4.5]dec-2-en-8-yl)-5-phenylpenta-2,4-dien-1-on | 523,66 |
| [359] | (Z)-3-(4-tert-Butylphenyl)-3-(3,4-dichlorphenyl)-1-(3-(2-fluorphenyl)-1-oxa-2,8-diazaspiro[4.5]dec-2-en-8-yl)prop-2-en-1-on | 566,52 |
| [360] | (Z)-3-(4-tert-Butylphenyl)-3-(3,4-dichlorphenyl)-1-(3-(3-fluorphenyl)-1-oxa-2,8-diazaspiro[4.5]dec-2-en-8-yl)prop-2-en-1-on | 566,52 |
| [361] | 1-(3-(3-Methoxyphenyl)-1-oxa-2,8-diazaspiro[4.5]dec-2-en-8-yl)-3-(4-(trifluormethyl)phenyl)prop-2-yn-1-on | 443,44 |
| [362] | (E)-1-(3-(3-Methoxyphenyl)-1-oxa-2,8-diazaspiro[4.5]dec-2-en-8-yl)-3-(6-(trifluormethyl)pyridin-3-yl)prop-2-en-1-on | 446,44 |
| [363] | 1-(3-Phenyl-1-oxa-2,8-diazaspiro[4.5]dec-2-en-8-yl)-3-(4-(trifluormethyl)phenyl)prop-2-yn-1-on | 413,41 |
| [365] | (E)-1-(3-(3-Fluorphenyl)-1-oxa-2,8-diazaspiro[4.5]dec-2-en-8-yl)-3-(2-(piperidin-1-yl)-6-(trifluormethyl)pyridin-3-yl)prop-2-en-1-on | 517,54 |
| [366] | N-(2-Fluor-4-(1-(3-(3-fluorphenyl)-1-oxa-2,8-diazaspiro[4.5]dec-2-en-8-yl)-1-oxopropan-2-yl)phenyl)methanesulfonamid | 478,53 |
| [367] | (E)-1-(6-Methyl-3-phenyl-1-oxa-2,8-diazaspiro[4.5]dec-2-en-8-yl)-3-(2-(piperidin-1-yl)-6-(trifluormethyl)pyridin-3-yl)prop-2-en-1-on | 513,58 |
| [368] | (E)-3-(2-Brom-4-tert-butylphenyl)-1-(3-phenyl-1-oxa-2,8-diazaspiro[4.5]dec-2-en-8-yl)prop-2-en-1-on | 482,44 |
| [370] | 2-(4-tert-Butylphenyl)-1-(3-phenyl-1-oxa-2,8-diazaspiro[4.5]dec-2-en-8-yl)propan-1-on | 405,55 |
| [381] | (E)-N-(2-Fluor-4-(3-oxo-3-(3-phenyl-1-oxa-2,8-diazaspiro[4.5]dec-2-en-8-yl)prop-1-enyl)phenyl)methanesulfonamid | 458,52 |
| [382] | (E)-N-(2-Fluor-4-(3-(3-(3-methoxyphenyl)-1-oxa-2,8-diazaspiro[4.5]dec-2-en-8-yl)-3-oxoprop-1-enyl)phenyl)methanesulfonamid | 488,55 |

| | | |
|---|---|---|
| * Referenzbeispiel | | |

### 10. Allgemeine Vorschrift zur Umsetzung von Aminen der allgemeinen Formel II mit Aldehyden der allgemeinen Formel R¹-C(=O)-H

Zu einer Lösung der Verbindung der allgemeinen Formel II (120 µmol in 0.5 mL MeOH) wurde unter Rühren bei RT der jeweilige Aldehyd der allgemeinen Formel R¹-C(=O)-H(120 µmol in 0.5 mL MeOH) und anschließend Boran-Pyridin-Komplex (BH₃•C₅H₅N, 100 µmol in 0.5 mL MeOH) gegeben. Die Reaktionsmischung wurde wenigstens 16 Stunden bei 64 °C gerührt und anschließend unter Rühren mit 5%-iger (Gewichtsprozent) Salzsäure-Lsg. in Wasser (0.5 mL) versetzt. Zu der Reaktionsmischung wurde 10%-ige (Gewichtsprozent) Natriumhydroxid-Lsg. in Wasser (1 mL) gegeben und die Mischung wurde dreimal mit DCM (jeweils 2 mL) extrahiert. Die vereinigten organischen Phasen wurden über MgSO₄-Kartuschen getrocknet, das Lösungsmittel im Vakuum entfernt und der Rückstand mittels präparativer HPLC gereinigt, um das gewünschte Produkt der allgemeinen Formel I zu erhalten.

### 11. Allgemeine Vorschrift zur Umsetzung von Aminen der allgemeinen Formel II mit Carbonsäurehalogeniden der allgemeinen Formel R⁹-C(=O)-LG oder mit Sulfonsäurehalogeniden der allgemeinen Formel R¹⁰-S(=O)₂-LG

Zu einer Lösung aus dem jeweiligen Säurehalogenid (1.5 Äquivalente), Triethylamin (2.0 Äquivalente) und einer katalytischen Menge DMAP in DCM wurde bei 0 °C die Verbindung der allgemeinen Formel II (1.0 Äquivalente) gegeben. Die Reaktionslösung wurde auf RT erwärmt und über Nacht gerührt. Nach Zugabe von 10%-iger (Gewichtsprozent) aq. NH₄Cl-Lsg. wurde die organische Phase abgetrennt und über MgSO₄ getrocknet. Das Lösungsmittel wurde im Vakuum entfernt und der Rückstand säulenchromatographisch an Kieselgel mit EtOAc/Hexan-Gemischen als Eluent gereinigt, um das gewünschte Produkt der allgemeinen Formel I zu erhalten.

### Pharmakologische Daten

Die Affinität der erfindungsgemäßen Spiro-Verbindungen für den Vanilloid-Rezeptor 1 (VR1/TRPV1-Rezeptor) wurde wie vorstehend beschrieben bestimmt.

| Verbindung gemäß Beispiel | VR1 (Mensch) (% Stimulation im Vgl. zu 10 µM CP) | VR1 (Mensch) (% Hemmung im Vgl. zu 10 µM CP) | VR1 (Ratte) (% Stimulation im Vgl. zu 10 µM CP) | VR1 (Ratte) (% Hemmung im Vgl. zu 10 µM CP) |
|---|---|---|---|---|
| 3 | 0,01 | 13,41 | -0,73 | 43,84 |
| 22 | -0,14 | 10,14 | -0,73 | 40,30 |
| 39 | -0,14 | 24,96 | 0,11 | 73,96 |
| 42 | -0,22 | 2,57 | -0,73 | 39,91 |
| 44 | -0,15 | -4,40 | -0,73 | 85,64 |
| 45 | -0,04 | 0,17 | -0,73 | 55,49 |
| 47 | 0,01 | 11,44 | 0,92 | 48,33 |
| 56 | -0,22 | 16,36 | -0,04 | 47,31 |
| 59 | 0,01 | 2,09 | 0,51 | 48,58 |
| 65 | -0,07 | -10,79 | 0,08 | 80,47 |
| 69 | -0,32 | 7,01 | -0,47 | 87,20 |
| 77 | 0,10 | 5,49 | 3,81 | 95,44 |
| 78 | 0,11 | 11,82 | 0,02 | 95,02 |
| 79 | -0,35 | -17,55 | 0,38 | 46,46 |
| 80 | 0,89 | 3,60 | -0,47 | 62,09 |
| 81 | -0,35 | -2,79 | -0,47 | 58,80 |
| 90 | -0,15 | -7,62 | -0,47 | 55,87 |
| 103 | -0,35 | 8,28 | -0,47 | 50,85 |
| 122 | 0,07 | -13,10 | -0,47 | 49,19 |
| 140 | 9,37 | 9,67 | 1,40 | 99,22 |
| 141 | -0,30 | 20,36 | 0,42 | 98,48 |
| 142 | -0,07 | -18,80 | -0,20 | 63,02 |
| 143 | 4,12 | -7,17 | -0,33 | 45,73 |
| 144 | -0,18 | -16,58 | -0,43 | 94,47 |
| 145 | 2,49 | 4,26 | -0,20 | 59,87 |
| 146 | 0,29 | 9,95 | -0,13 | 58,05 |
| 156 | 0,69 | -7,89 | 0,20 | 48,20 |
| 163 | 0,12 | -3,15 | -0,43 | 84,27 |
| 164 | -0,04 | -2,13 | -0,43 | 72,93 |
| 165 | 1,92 | 1,69 | -0,43 | 56,21 |
| 167 | 0,06 | 17,47 | -0,43 | 47,40 |
| 174 | 5,90 | -25,03 | -0,28 | 47,79 |
| 192 | -0,04 | -6,74 | -0,07 | 52,07 |
| 193 | 0,11 | -2,64 | -0,17 | 43,59 |
| 197 | 0,06 | -17,17 | -0,35 | 59,91 |
| 201 | 8,56 | -0,70 | -0,43 | 54,17 |
| 205 | 0,33 | -23,85 | 0,15 | 39,94 |
| 210 | -0,21 | 7,12 | 0,22 | 98,00 |
| 211 | -0,18 | 9,23 | 0,33 | 97,52 |
| 212 | -0,21 | -16,07 | -0,43 | 61,93 |
| 221 | -0,04 | -11,55 | 0,95 | 72,82 |
| 225 | 0,31 | -4,50 | -0,47 | 47,78 |
| 236 | -0,28 | -10,27 | -0,47 | 45,15 |
| 244 | -0,28 | 3,09 | 9,79 | 89,14 |
| 245 | 0,28 | 10,98 | 2,55 | 99,05 |
| 262 | -0,28 | 7,81 | -0,54 | 52,53 |
| 278 | 0,66 | 28,06 | 0,73 | 88,07 |
| 290 | -0,28 | -7,84 | -0,47 | 48,38 |
| 314 | 0 | 6 | -6 | 82 |
| 323 | 0 | 36 | 0 | 98 |
| 325 | 0 | 6 | -6 | 63 |
| 329 | 38 | 37 | 71 | 96 |
| 330 | | | 1 | 103 |
| 333 | 8 | 29 | 9 | 101 |
| 337 | -1 | 16 | 1 | 95 |
| 342 | 0 | 53 | 0 | 60 |
| 346 | 0 | 3 | 0 | 62 |
| 348 | 0 | 60 | 3 | 101 |
| 349 | 1 | -12 | 5 | 100 |
| 350 | 0 | -4 | 1 | 81 |
| 351 | 3 | 83 | 6 | 101 |
| 352 | 2 | -3 | 7 | 95 |
| 353 | 0 | 23 | 0 | 84 |
| 355 | 0 | 63 | 0 | 98 |
| 356 | 0 | 52 | 0 | 88 |
| 358 | 0 | 41 | 0 | 95 |
| 359 | 0 | 26 | 3 | 57 |
| 360 | 0 | 12 | 3 | 80 |
| 361 | 0 | 12 | 0 | 58 |
| 362 | 0 | 19 | -1 | 44 |
| 363 | 0 | 30 | 0 | 81 |
| 364 | -1 | 50 | 4 | 106 |
| 365 | -1 | 74 | 0 | 94 |
| 366 | -1 | -20 | 0 | 29 |
| 367 | 0 | 93 | -1 | 96 |
| 368 | 0 | 66 | 0 | 83 |
| 369 | -1 | 19 | 0 | 110 |
| 371 | 1 | 12 | 35 | 99 |
| 378 | 42 | 81 | 33 | 99 |
| 385 | -2 | 18 | 1 | 77 |

| **Verbindung gemäß Beispiel** | **IC₅₀ CAP Ratte Mittel [µM]** | **IC₅₀ CAP Mensch Mittel [µM]** |
|---|---|---|
| 141 | 0,0780 | |
| 314 | 0,1500 | 0,8250 |
| 323 | 0,1810 | 1,4427 |
| 325 | 0,2700 | 0,8750 |
| 342 | 0,4785 | 1,5488 |
| 346 | 1,3580 | 4,3760 |
| 358 | 0,1485 | 1,1110 |
| 369 | 0,1752 | 1,0674 |
| 385 | 0,0598 | |

## Patentansprüche

1. Substituierte Spiro-Verbindungen der allgemeinen Formel I, 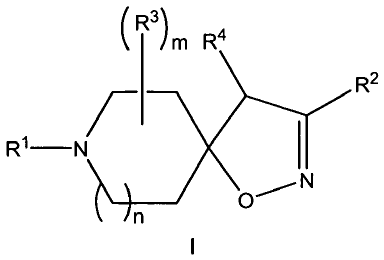 worin
m gleich 0, 1, 2, 3 oder 4 ist,
n gleich 0, 1 oder 2 ist,
R¹ für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
für einen unsubstituierten oder wenigstens einfach substituierten Aryl- oder Heteroaryl-Rest, der über eine lineare oder verzweigte, unsubstituierte oder wenigstens einfach substituierte Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
für eine -C(=O)-NR⁵R⁶-Gruppe,
für eine -C(=S)-NR⁷R⁸-Gruppe,
für eine -C(=O)-R⁹-Gruppe
oder für eine -S(=O)₂-R¹⁰-Gruppe steht;
R² für einen linearen oder verzweigten, gesättigten oder ungesättigten, unsubstituierten oder wenigstens einfach substituierten, ggf. wenigstens ein Heteroatom als Kettenglied aufweisenden aliphatischen Rest;
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
für einen unsubstituierten oder wenigstens einfach substituierten Phenyl-Rest,
mit der Maßgabe, dass nicht eine der meta-Positionen und die para-Position dieses Phenyl-Restes mit Substituenten substituiert sind, die jeweils über ein gleiches Atom ausgewählt aus der Gruppe bestehend aus Sauerstoff, Schwefel und Stickstoff an den Phenyl-Rest gebunden sind;
für einen unsubstituierten oder wenigstens einfach substituierten Rest ausgewählt aus der Gruppe bestehend aus Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl; 2H-Benzo[1.4]oxazin-3(4H)-onyl, (3,4)-Dihydrochinolin-2(1H)-onyl, [3,4]-Dihydro-2H-1,4-benzoxazinyl und Benzothiazolyl steht,
oder für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden ist und ggf. mit einem unsubstituierten oder wenigstens einfach substituierten mono- oder polyzyklischen Ringsystem kondensiert sein kann, steht;
R³ für einen Halogen-Rest, für eine Nitro-Gruppe, für eine Hydroxy-Gruppe, für eine Thiol-Gruppe; für eine -O-R¹¹-Gruppe, für eine -S-R¹²-Gruppe, oder für einen linearen oder verzweigten, gesättigten oder ungesättigten, unsubstituierten oder wenigstens einfach substituierten aliphatischen Rest steht;
R⁴ für einen Wasserstoff-Rest, für einen Halogen-Rest, für eine Nitro-Gruppe, für eine Hydroxy-Gruppe, für eine Thiol-Gruppe; für eine Oxo-Gruppe (=0), für eine -O-R¹¹-Gruppe, für eine -S-R¹²-Gruppe, oder für einen linearen oder verzweigten, gesättigten oder ungesättigten, unsubstituierten oder wenigstens einfach substituierten aliphatischen Rest steht;
R⁵ und R⁷, unabhängig voneinander, jeweils
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert und/oder mit wenigstens einer linearen oder verzweigten, unsubstituierten oder wenigstens einfach substituierten Alkylen-Gruppe überbrückt sein kann,
für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
R⁶ und R⁸, unabhängig voneinander, jeweils
für einen Wasserstoff-Rest,
für einen linearen oder verzweigten, gesättigten oder ungesättigten, unsubstituierten oder wenigstens einfach substituierten, ggf. wenigstens ein Heteroatom als Kettenglied aufweisenden aliphatischen Rest,
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert und/oder mit wenigstens einer linearen oder verzweigten, unsubstituierten oder wenigstens einfach substituierten Alkylen-Gruppe überbrückt sein kann,
für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
R⁹ für einen linearen oder verzweigten, gesättigten oder ungesättigten, unsubstituierten oder wenigstens einfach substituierten aliphatischen Rest;
oder für einen unsubstituierten oder wenigstens einfach substituierten Aryl-oder Heteroaryl-Rest, der über eine lineare oder verzweigte, unsubstituierte oder wenigstens einfach substituierte Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht;
R¹⁰ für einen linearen oder verzweigten, gesättigten oder ungesättigten, unsubstituierten oder wenigstens einfach substituierten aliphatischen Rest;
für einen unsubstituierten oder wenigstens einfach substituierten, ungesättigten oder gesättigten, ggf. wenigstens ein Heteroatom als Ringglied aufweisenden cycloaliphatischen Rest, der über eine lineare oder verzweigte, unsubstituierte oder wenigstens einfach substituierte Alkylen-, Alkenylen- oder Alkinylen-Gruppe gebunden und/oder mit einem unsubstituierten oder wenigstens einfach substituierten mono- oder polyzyklischen Ringsystem kondensiert sein kann,
oder für einen unsubstituierten oder wenigstens einfach substituierten Aryl-oder Heteroaryl-Rest, der über eine lineare oder verzweigte, unsubstituierte oder wenigstens einfach substituierte Alkylen-, Alkenylen- oder Alkinylen-Gruppe gebunden und/oder mit einem unsubstituierten oder wenigstens einfach substituierten mono- oder polyzyklischen Ringsystem kondensiert sein kann, steht;
und
R¹¹ und R¹², unabhängig voneinander, jeweils
für einen linearen oder verzweigten, gesättigten oder ungesättigten, unsubstituierten aliphatischen Rest;
für einen unsubstituierten, ungesättigten oder gesättigten, ggf. wenigstens ein Heteroatom als Ringglied aufweisenden cycloaliphatischen Rest, der über eine lineare oder verzweigte, unsubstituierte Alkylen-, Alkenylen- oder Alkinylen-Gruppe gebunden und/oder mit einem unsubstituierten mono- oder polyzyklischen Ringsystem kondensiert sein kann,
oder für einen unsubstituierten Aryl- oder Heteroaryl-Rest, der über eine lineare oder verzweigte, unsubstituierte Alkylen-, Alkenylen- oder Alkinylen-Gruppe gebunden und/oder mit einem unsubstituierten mono- oder polyzyklischen Ringsystem kondensiert sein kann, steht;
wobei die Substituenten der vorstehend genannten aliphatischen Reste unabhängig voneinander aus der Gruppe bestehend aus F, Cl, Br, I, -CN, -NO₂, -OH, -SH und-NH₂ ausgewählt werden können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, dass**
m gleich 0, 1, 2, 3 oder 4 ist,
n gleich 0, 1 oder 2 ist,
R¹ für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
für eine -C(=O)-NR⁵R⁶-Gruppe,
für eine -C(=S)-NR⁷R⁸-Gruppe,
für eine -C(=O)-R⁹-Gruppe,
für eine -S(=O)₂-R¹⁰-Gruppe,
oder für -(CHR¹³)-(CHR¹⁴)_{f}-(CHR¹⁵)ₕ-R¹⁶ mit f = 0 oder 1 und h= 0 oder 1 steht;
R² für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen Phenyl-Rest, der mit 1, 2, 3, 4 oder 5 Substituenten unabhängig voneinander ausgewählt aus der Gruppe bestehend aus F, Cl, Br, I, -CN,-CF₃, -SF₅, -OH, -O-C₁₋₅-Alkyl, -NH₂, -NO₂, -O-CF₃, -S-CF₃, -SH, -S-C₁₋₅-Alkyl, - C₁₋₁₀-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl,-C(=O)-NH₂, -C(=O)-NH-C₁₋₅-Alkyl, C(=O)-N-(C₁₋₅-Alkyl)₂, -S(=O)₂-C₁₋₅-Alkyl,-S(=O)₂-Phenyl, -NH-S(=O)₂-C₁₋₅-Alkyl, -NH-S(=O)₂-C₁₋₅-Alkylen-Phenyl, -NH-S(=O)₂-C₁₋₅-Alkylen-Naphthyl, -NH-S(=O)₂-Phenyl, -NH-S(=O)₂-Naphthyl, Cyclohexyl, Cyclopentyl, Morpholinyl, Piperidinyl, Piperazinyl, Pyrrolidinyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Morpholinyl, Piperidinyl, Piperazinyl, Pyrrolidinyl, Pyridazinyl, -S(=O)₂-Phenyl, -O-Phenyl, -O-Benzyl, Phenyl,-(CH₂)-Benzo[b]furanyl, -NH-S(=O)₂-C₁₋₅-Alkylen-Phenyl, -NH-S(=O)₂-C₁₋₅-Alkylen-Naphthyl, -NH-S(=O)₂-Phenyl, -NH-S(=O)₂-Naphthyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -0-C₁₋₅-Alkyl, -O-CF₃, -S-CF₃, Phenyl und -O-Benzyl substituiert sein kann,
mit der Maßgabe, dass nicht eine der meta-Positionen und die para-Position dieses Phenyl-Restes mit Substituenten substituiert sind, die jeweils über ein gleiches Atom ausgewählt aus der Gruppe bestehend aus Sauerstoff, Schwefel und Stickstoff an den Phenyl-Rest gebunden sind;
für einen ggf. substituierten Rest ausgewählt aus der Gruppe bestehend aus Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, 2H-Benzo[1.4]oxazin-3(4H)-onyl, (3,4)-Dihydrochinolin-2(1H)-onyl, [3,4]-Dihydro-2H-1,4-benzoxazinyl und Benzothiazolyl steht,
oder für -(CHR¹⁷)-X_{q}-(CHR¹⁸)ᵣ-Yₛ-(CHR¹⁹)ₜZᵤ-R²⁰ mit q = 0 oder 1, r = 0 oder 1, s = 0 oder 1, t = 0 oder 1, u = 0 oder 1, worin X, Y und Z, unabhängig voneinander, jeweils für O, S, NH, N(CH₃), N(C₂H₅) oder N[CH(CH₃)₂] stehen, steht;
R³ für einen Halogen-Rest, für eine Nitro-Gruppe, für eine Hydroxy-Gruppe, für eine Thiol-Gruppe; für eine -O-R¹¹-Gruppe, für eine -S-R¹²-Gruppe, oder für einen linearen oder verzweigten, gesättigten oder ungesättigten, ggf. substituierten C₁₋₁₀ aliphatischen Rest steht;
R⁴ für einen Wasserstoff-Rest, für einen Halogen-Rest, für eine Nitro-Gruppe, für eine Hydroxy-Gruppe, für eine Thiol-Gruppe; für eine Oxo-Gruppe (=0), für eine -O-R¹¹-Gruppe, für eine -S-R¹²-Gruppe, oder für einen linearen oder verzweigten, gesättigten oder ungesättigten, ggf. substituierten C₁₋₁₀ aliphatischen Rest steht;
R⁵ und R⁷, unabhängig voneinander, jeweils
für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11- oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen-Gruppen überbrückt sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann;
oder für -(CR²¹R²²)-Xᵥ-(CHR²³)_{w}-Yₓ-(CHR²⁴)_{y}-Z₂-R²⁵ mit v = 0 oder 1, w = 0 oder 1, x = 0 oder 1, y = 0 oder 1, z = 0 oder 1, worin X, Y und Z, unabhängig voneinander, jeweils für O, S, NH, N(CH₃), N(C₂H₅) oder N[CH(CH₃)₂] stehen, stehen;
R⁶ und R⁸, unabhängig voneinander, jeweils
für einen Wasserstoff-Rest;
für einen linearen oder verzweigten, gesättigten oder ungesättigten, ggf. substituierten C₁₋₂₀ aliphatischen Rest;
für einen ungesättigten oder gesättigten, ggf. substituierten 3-, 4-, 5-, 6-, 7-, 8-, 9-, 10-, 11- oder 12-gliedrigen cycloaliphatischen Rest, der mit einem gesättigten, ungesättigten oder aromatischen, ggf. substituierten mono- oder polyzyklischen Ringsystem kondensiert und/oder mit einer oder zwei linearen oder verzweigten, ggf. substituierten C₁₋₅-Alkylen-Gruppen überbrückt sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann;
oder für -(CR²¹R²²)-Xᵥ-(CHR²³)_{w}-Yₓ-(CHR²⁴)_{y}-Z_{z}-R²⁵ mit v = 0 oder 1, w = 0 oder 1, x = 0 oder 1, y = 0 oder 1, z = 0 oder 1, worin X, Y und Z, unabhängig voneinander, jeweils für O, S, NH, N(CH₃), N(C₂H₅) oder N[CH(CH₃)₂] stehen, stehen;
R⁹ für einen linearen oder verzweigten, gesättigten oder ungesättigten, ggf. substituierten C₁₋₁₀ aliphatischen Rest;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
für -(CR²⁶R²⁷)-(CHR²⁸)ₐₐ-(CHR²⁹)_{bb}-R³⁰ mit aa = 0 oder 1 und bb = 0 oder 1;
für -CR³¹=CR³²-R³³
oder für -C≡C-R³⁴ steht;
R¹⁰ für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert sein kann;
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann,
für -(CR²⁶R²⁷)-(CHR²⁸)ₐₐ-(CHR²⁹)_{bb}-R³⁰ mit aa = 0 oder 1 und bb = 0 oder 1;
für -CR³¹=CR³²-R³³
oder für -C≡C-R³⁴ steht;
R¹¹ und R¹², unabhängig voneinander, jeweils
für einen linearen oder verzweigten, gesättigten oder ungesättigten C₁₋₁₀ aliphatischen Rest;
für einen ungesättigten oder gesättigten 3-, 4-, 5-, 6-, 7-, 8- oder 9-gliedrigen cycloaliphatischen Rest, der mit einem gesättigten, ungesättigten oder aromatischen mono- oder polyzyklischen Ringsystem kondensiert sein kann;
oder für einen 5- bis 14-gliedrigen Aryl- oder Heteroaryl-Rest, der mit einem gesättigten oder ungesättigten mono- oder polyzyklischen Ringsystem kondensiert sein kann, stehen;
R¹³, R¹⁴, R¹⁵, R¹⁷, R¹⁸, R¹⁹, R²¹, R²², R²³, R²⁴, R²⁸, R²⁹ und R³¹, unabhängig voneinander, jeweils
für einen Wasserstoff-Rest;
für einen linearen oder verzweigten, gesättigten oder ungesättigten, ggf. substituierten C₁₋₁₀ aliphatischen Rest,
oder für einen ggf. substituierten 5- bis 14-gliedrigen Aryl- oder Heteroaryl-Rest, der mit einem gesättigten oder ungesättigten, ggf. substituierten mono-oder polyzyklischen Ringsystem kondensiert sein kann, stehen;
R²⁶ und R²⁷, unabhängig voneinander, jeweils
für einen Wasserstoff-Rest;
für einen linearen oder verzweigten, gesättigten oder ungesättigten, ggf. substituierten C₁₋₁₀ aliphatischen Rest,
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann; oder für -OH stehen;
R³² für einen Wasserstoff-Rest;
für einen linearen oder verzweigten, gesättigten oder ungesättigten, ggf. substituierten C₁₋₁₀ aliphatischen Rest,
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht; oder für -(CH₂)_{cc}-R³⁵ mit cc = 1, 2, 3 oder 4 steht oder für -CH=CH-R³⁶ steht;
R¹⁶, R²⁰, R²⁵, R³⁰, R³³ und R³⁴, unabhängig voneinander, jeweils
für einen linearen oder verzweigten, gesättigten oder ungesättigten, ggf. substituierten C₁₋₁₀ aliphatischen Rest,
für einen ungesättigten oder gesättigten, ggf. substituierten 3-, 4-, 5-, 6-, 7-, 8-oder 9-gliedrigen cycloaliphatischen Rest, der mit 1, 2, 3, 4 oder 5 linearen oder verzweigten, ggf. substituierten C₁₋₅-Alkylen-Gruppen überbrückt und/oder mit einem gesättigten, ungesättigten oder aromatischen, ggf. substituierten mono- oder polyzyklischen Ringsystem kondensiert sein kann;
oder für einen ggf. substituierten 5- bis 14-gliedrigen Aryl- oder Heteroaryl-Rest, der mit einem gesättigten oder ungesättigten, ggf. substituierten mono-oder polyzyklischen Ringsystem kondensiert sein kann, stehen;
und
R³⁵ und R³⁶, unabhängig voneinander, jeweils für einen ggf. substituierten 6- oder 10-gliedrigen Aryl-Rest, der mit einem gesättigten oder ungesättigten, ggf. substituierten mono- oder polyzyklischen Ringsystem kondensiert sein kann, stehen;
wobei
die vorstehend genannten C₁₋₁₀ aliphatischen Reste und C₁₋₂₀ aliphatischen Reste jeweils ggf. mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -SH und -NH₂ substituiert sein können;
die vorstehend genannten cycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂,-O-CF₃, -S-CF₃, -SH, -S-C₁₋₅-Alkyl, -C₁₋₅-Alkyl, -C(=O)-OH, -C(=O)-O-C₁₋₅-Alkyl, -0-C(=O)-C₁₋₅-Alkyl, -NH-C₁₋₅-Alkyl, -N(C₁₋₅-Alkyl)₂, -NH-C(=O)-O-C₁₋₅-Alkyl, -C(=0)-H, - C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -0-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH,-CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=0), Thioxo (=S), F, Cl, Br, I, -CN, -CF₃, -SF₅, -OH, - O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, - C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=0)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Morpholinyl, Piperidinyl, Pyrrolidinyl, Piperazinyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Morpholinyl, Piperidinyl, Pyrrolidinyl, Piperazinyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und, sofern nicht anders angegeben, die vorstehend genannten Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, 2H-Benzo[1.4]oxazin-3(4H)-onyl, (3,4)-Dihydrochinolin-2(1H)-onyl, [3,4]-Dihydro-2H-1,4-benzoxazinyl und Benzothiazolyl sowie Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₁₀-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl,-NH-S(=O)₂-C₁₋₅-Alkyl, -NH-S(=O)₂-C₁₋₅-Alkylen-Phenyl, -NH-S(=O)₂-C₁₋₅-Alkylen-Naphthyl, -NH-S(=O)₂-Phenyl, -NH-S(=O)₂-Naphthyl, Cyclohexyl, Cyclopentyl, Morpholinyl, Piperidinyl, Piperazinyl, Pyrrolidinyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Morpholinyl, Piperidinyl, Piperazinyl, Pyrrolidinyl, Pyridazinyl, -S(=O)₂-Phenyl, -0-Phenyl, -O-Benzyl, Phenyl, -(CH₂)-Benzo[b]furanyl, -NH-S(=O)₂-C₁₄₋₅-Alkylen-Phenyl,-NH-S(=O)₂-C₁₋₅-Alkylen-Naphthyl, -NH-S(=O)₂-Phenyl, -NH-S(=O)₂-Naphthyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
und
die vorstehend genannten C₁₋₅-Alkylen-Gruppen jeweils ggf. mit 1, 2, 3, 4 oder 5 Substituenten unabhängig voneinander ausgewählt aus der Gruppe bestehend aus F, Cl, Br, -OH, -SH, -NH₂, -CN und NO₂ substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

3. Verbindungen gemäß Anspruch 1 oder 2, **dadurch gekennzeichnet, dass**
R¹ für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl steht, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂,-NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅,-C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅ -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂,-NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅,-C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für eine -C(=O)-NR⁵R⁶-Gruppe steht;
für eine -C(=S)-NR⁷R⁸-Gruppe steht;
für eine -C(=O)-R⁹-Gruppe,
für eine -S(=O)₂-R¹⁰-Gruppe,
oder für -(CHR¹³)-R¹⁶; -(CHR¹³)-(CHR¹⁴)-R¹⁶ oder (CHR¹³)-(CHR¹⁴)-(CHR¹⁵)-R¹⁶ steht.

4. Verbindungen gemäß einem oder mehreren der Ansprüche 1 bis 3, **dadurch gekennzeichnet, dass**
R² für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl steht, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅ -C(=O)-N-(CH₃)₂, -C(=O)-N-(C₂H₅)₂, -S(=O)₂-CH₃,-S(=O)₂-C₂H₅, -S(=O)₂-Phenyl, -NH-S(=O)₂-CH₃, -NH-S(=O)₂-C₂H₅, -S(=O)₂-NH-CH₃,-S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃,-S-CF₃, Phenyl und -O-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, [3,4]-Dihydro-2H-1,4-benzoxazinyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅ -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -NH-S(=O)₂-Phenyl, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -NH-S(=O)₂-Phenyl,-O-Phenyl, -O-Benzyl, Phenyl, -(CH₂)-Benzo[b]furanyl und Benzyl mit 1,2,3,4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
mit der Maßgabe, dass nicht eine der meta-Positionen und die para-Position dieses Phenyl-Restes mit Substituenten substituiert sind, die jeweils über ein gleiches Atom ausgewählt aus der Gruppe bestehend aus Sauerstoff, Schwefel und Stickstoff an den Phenyl-Rest gebunden sind;
oder für -(CHR¹⁷)-R²⁰, -(CHR¹⁷)-(CHR¹⁸)-R²⁰ oder -(CHR¹⁷)-(CHR¹⁸)-(CHR¹⁹)-R²⁰ steht.

5. Verbindungen gemäß einem oder mehreren der Ansprüche 1 bis 3, **dadurch gekennzeichnet, dass**
R² für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl, (steht, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃,-N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅ -C(=O)-N-(CH₃)₂, -C(=O)-N-(C₂H₅)₂, -S(=O)₂-CH₃ und -S(=O)₂-C₂H₅ substituiert sein kann;
für einen Phenyl-Rest der allgemeinen Formel XX 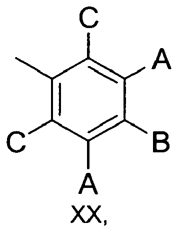 steht,
worin die Linie die Bindung dieses Phenyl-Restes zur Spiro-Verbindung der allgemeinen Formel I darstellt;
und A, B und C jeweils für einen Substituenten unabhängig voneinander ausgewählt aus der Gruppe bestehend aus H, F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, Cyclohexyl, Cyclopentyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃,-C(=O)-NH₂, -C(=O)-NH-CH₃, -C(=O)-NH-C₂H₅ -C(=O)-N-(CH₃)₂, -C(=O)-N-(C₂H₅)₂,-S(=O)₂-CH₃, -S(=O)₂-C₂H₅, -S(=O)₂-Phenyl, -NH-S(=O)₂-CH₃, -NH-S(=O)₂-C₂H₅,-S(=O)₂-NH-CH₃, -S(=O)₂-NH-C₂H₅ und -NH-S(=O)₂-Phenyl stehen;
mit der Maßgabe, dass nicht eine der Positionen A und die Position B dieses Phenyl-Restes mit Substituenten substituiert sind, die jeweils über ein gleiches Atom ausgewählt aus der Gruppe bestehend aus Sauerstoff, Schwefel und Stickstoff an den Phenyl-Rest gebunden sind;
für einen Rest ausgewählt aus der Gruppe bestehend aus Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl, 2H-Benzo[1.4]oxazin-3(4H)-onyl, (3,4)-Dihydrochinolin-2(1H)-onyl, [3,4]-Dihydro-2H-1,4-benzoxazinyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅,-C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅ -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
oder für -(CHR¹⁷)-R²⁰, -(CHR¹⁷)-(CHR¹⁸)-R²⁰ oder -(CHR¹⁷)-(CHR¹⁸)-(CHR¹⁹)-R²⁰ steht.

6. Verbindungen gemäß einem oder mehreren der Ansprüche 1 bis 5, **dadurch gekennzeichnet, dass**
R³ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann.

7. Verbindungen gemäß einem oder mehreren der Ansprüche 1 bis 6, **dadurch gekennzeichnet, dass**
R⁴ für einen Wasserstoff-Rest steht,
oder für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann.

8. Verbindungen gemäß einem oder mehreren der Ansprüche 1 bis 7, **dadurch gekennzeichnet, dass**
R⁵ und R⁷, unabhängig voneinander, jeweils
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl, 2-Methyl-1-propenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen, wobei der Rest ggf. jeweils mit 1, 2, 3, 4 oder 5 Substituenten unabhängig voneinander ausgewählt aus der Gruppe bestehend aus Oxo (=0), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃,-N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃,-S(=O)₂-C₂H₅, -S(=O)₂-Phenyl, -NH-S(=O)₂-CH₃, -NH-S(=O)₂-C₂H₅, -S(=O)₂-NH-CH₃,-S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂,-NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅,-C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅ -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -0-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
oder für -(CR²¹R²²)-R²⁵, -(CR²¹R²²)-(CHR²³)-R²⁵, -(CR²¹R²²)-(CHR²³)-O-R²⁵,-(CR²¹R²²)-(CHR²³)-(CHR²⁴)-R²⁵, -(CR²¹R²²)-(CHR²³)-(CHR²⁴)-O-R²⁵, -(CR²¹R²²)-(CHR²³)-(CHR²⁴)-N(CH₃)-R²⁵ oder -(CR²¹R²²)-(CHR²³)-(CHR²⁴)-N(C₂H₅)-R²⁵ stehen.

9. Verbindungen gemäß einem oder mehreren der Ansprüche 1 bis 8, **dadurch gekennzeichnet, dass**
R⁶ und R⁸ jeweils für einen Wasserstoff-Rest stehen
oder für einen Rest ausgewählt aus der Gruppe bestehend aus Methyl, Ethyl, n-Propyl, Isopropyl, n-Butyl, sec-Butyl, Isobutyl und tert-Butyl.

10. Verbindungen gemäß einem oder mehreren der Ansprüche 1 bis 9, **dadurch gekennzeichnet, dass**
R⁹ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, Vinyl, 1-Propenyl, 2-Propenyl, 1-Butenyl, 2-Butenyl, 3-Butenyl, 2-Methyl-1-propenyl, Ethinyl, 1-Propinyl, 2-Propinyl, 1-Butinyl, 2-Butinyl und 3-Butinyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann; oder
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂,-NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, n-Pentyl, n-Hexyl, n-Heptyl, n-Octyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅,-O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Morpholinyl, Piperidinyl, Piperazinyl, Pyrrolidinyl, Pyridinyl, Pyridazinyl, -(CH₂)-Benzo[b]furanyl, -0-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht und
R¹⁰ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, Vinyl, 1-Propenyl, 2-Propenyl, 1-Butenyl, 2-Butenyl, 3-Butenyl, 2-Methyl-1-propenyl, Ethinyl, 1-Propinyl, 2-Propinyl, 1-Butinyl, 2-Butinyl und 3-Butinyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus 9H-Flourenyl, 9H-Xanthenyl, Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl steht, wobei der Rest ggf. jeweils mit 1, 2, 3, 4 oder 5 Substituenten unabhängig voneinander ausgewählt aus der Gruppe bestehend aus Oxo (=0), Thioxo (=S), F, Cl, Br, I, -CN, -CF₃, -SF₅, -OH,-O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, - C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃,-C(=O)-NH₂, -C(=O)-NH-CH₃, -C(=O)-NH-C₂H₅, -C(=O)-N-(CH₃)₂, -C(=O)-N-(C₂H₅)₂,-S(=O)₂-CH₃, -S(=O)₂-C₂H₅, -S(=O)₂-Phenyl, -NH-S(=O)₂-CH₃, -NH-S(=O)₂-C₂H₅,-S(=O)₂-NH-CH₃, -S(=O)₂-NH-C₂H₅, Cyclohexyl, Cyclopentyl, Pyridinyl, Pyridazinyl,-(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃,-S-CF₃, Phenyl und -O-Benzyl substituiert sein kann;
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂,-NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, n-Pentyl, n-Hexyl, n-Heptyl, n-Octyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅,-O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅ -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Morpholinyl, Piperidinyl, Piperazinyl, Pyrrolidinyl, Pyridinyl, Pyridazinyl, -(CH₂)-Benzo[b]furanyl, -0-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ stehen.

11. Verbindungen gemäß einem oder mehreren der Ansprüche 1 bis 10, **dadurch gekennzeichnet, dass**
R¹¹ und R¹², unabhängig voneinander, jeweils
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl stehen;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl stehen;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl stehen.

12. Verbindungen gemäß einem oder mehreren der Ansprüche 1 bis 11, **dadurch gekennzeichnet, dass**
R¹³, R¹⁴, R¹⁵, R¹⁷, R¹⁸, R¹⁹, R²¹, R²², R²³, R²⁴, R²⁸, R²⁹ und R³¹, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl stehen;
oder für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅,-C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann.

13. Verbindungen gemäß einem oder mehreren der Ansprüche 1 bis 12, **dadurch gekennzeichnet, dass**
R²⁶ und R²⁷, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl,-(CH₂)-(CH)(C₂H₅)-(CH₂)-(CH₂)-(CH₂)-(CH₃), Vinyl, 1-Propenyl, 2-Propenyl, 1-Butenyl, 2-Butenyl, 3-Butenyl, Ethinyl, 1-Propinyl, 2-Propinyl, 1-Butinyl, 2-Butinyl und 3-Butinyl stehen;
für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅,-C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann;
oder für -OH stehen.

14. Verbindungen gemäß einem oder mehreren der Ansprüche 1 bis 13, **dadurch gekennzeichnet, dass**
R¹⁶, R²⁰, R²⁵, R³⁰, R³³ und R³⁴, unabhängig voneinander, jeweils
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) stehen,
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl stehen, wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂,-NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅,-C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -0-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂,-NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, n-Pentyl, n-Hexyl, n-Heptyl, n-Octyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅,-O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Morpholinyl, Piperidinyl, Piperazinyl, Pyrrolidinyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Morpholinyl, Piperidinyl, Piperazinyl, Pyrrolidinyl, Pyridazinyl, -S(=O)₂-Phenyl, -O-Phenyl, -O-Benzyl, Phenyl,-(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅,-CN, -NO₂, Methyl, Ethyl, n-Propyl, Isopropyl, n-Butyl, sec-Butyl, Isobutyl, tert-Butyl,-O-CH₃, -O-C₂H₅, -O-CF₃, -S-CF₃, Phenyl und -O-Benzyl substituiert sein kann.

15. Verbindungen gemäß einem oder mehreren der Ansprüche 1 bis 14, **dadurch gekennzeichnet, dass**
m gleich 0, 1 oder 2 ist.

16. Verbindungen gemäß einem oder mehreren der Ansprüche 1 bis 15, **dadurch gekennzeichnet, dass**
n gleich 0, 1 oder 2 ist.

17. Verbindungen gemäß einem oder mehreren der Ansprüche 1 bis 16, **dadurch gekennzeichnet, dass**
R³² für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht,
für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂,-NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, n-Pentyl, n-Hexyl, n-Heptyl, n-Octyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅,-O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein kann, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl,-(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅,-CN, -NO₂, Methyl, Ethyl, n-Propyl, Isopropyl, n-Butyl, sec-Butyl, Isobutyl, tert-Butyl,-O-CH₃, -O-C₂H₅, -O-CF₃, -S-CF₃, Phenyl und -O-Benzyl substituiert sein kann;
oder für -(CH₂)_{cc}-R³⁵ mit cc = 1, 2, 3 oder 4 steht oder für -CH=CH-R³⁶ steht.

18. Verbindungen gemäß einem oder mehreren der Ansprüche 1 bis 17, **dadurch gekennzeichnet, dass**
R³⁵ und R³⁶, unabhängig voneinander, jeweils für einen Rest ausgewählt aus der Gruppe bestehend aus Phenyl und Naphthyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, n-Pentyl, n-Hexyl, n-Heptyl, n-Octyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃,-C(=O)-NH₂, -C(=O)-NH-CH₃, -C(=O)-NH-C₂H₅, -C(=O)-N-(CH₃)₂, -C(=O)-N-(C₂H₅)₂,-S(=O)₂-CH₃, -S(=O)₂-C₂H₅, -S(=O)₂-Phenyl, -NH-S(=O)₂-CH₃, -NH-S(=O)₂-C₂H₅,-S(=O)₂-NH-CH₃ und -S(=O)₂-NH-C₂H₅ substituiert sein kann.

19. Verbindungen gemäß einem oder mehreren der Ansprüche 1 bis 18, **dadurch gekennzeichnet, dass**
m gleich 0, 1, 2, 3 oder 4 ist;
n gleich 0, 1 oder 2 ist;
R¹ für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl steht;
für einen Rest ausgewählt aus der Gruppe bestehend aus Indolyl, Isoindolyl, Benzo[b]furanyl, Benzo[b]thiophenyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅,-NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅,-C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -NH-S(=O)₂-CH₃, -NH-S(=O)₂-C₂H₅, -S(=O)₂-NH-CH₃, -S(=O)₂-NH-C₂H₅ substituiert sein kann;
für eine -C(=O)-NR⁵R⁶-Gruppe steht;
für eine -C(=S)-NR⁷R⁸-Gruppe steht;
für eine -C(=O)-R⁹-Gruppe,
für eine -S(=O)₂-R¹⁰-Gruppe,
oder für -(CHR¹³)-R¹⁶; -(CHR¹³)-(CHR¹⁴)-R¹⁶ oder -(CHR¹³)-(CHR¹⁴)-(CHR¹⁵)-R¹⁶ steht;
R² für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl steht;
für einen Phenyl-Rest, der mit 1, 2, 3, 4 oder 5 Substituenten unabhängig voneinander ausgewählt aus der Gruppe bestehend aus F, Cl, Br, I, -CN,-CF₃, -SF₅, -OH, -O-CH₃, -O-C₂H₅, -NH₂, -NO₂, -O-CF₃, -S-CF₃, -SH, -S-CH₃,-S-C₂H₅, Methyl, Ethyl, n-Propyl, Isopropyl, n-Butyl, sec-Butyl, Isobutyl, tert-Butyl, Cyclopentyl, Cyclohexyl, -S(=O)₂-CH₃, -S(=O)₂-C₂H₅, -NH-S(=O)₂-CH₃,-NH-S(=O)₂-C₂H₅, -NH-S(=O)₂-Phenyl, -S(=O)₂-NH-CH₃ und -S(=O)₂-NH-C₂H₅ substituiert sein kann;
mit der Maßgabe, dass nicht eine der meta-Positionen und die para-Position dieses Phenyl-Restes mit Substituenten substituiert sind, die jeweils über ein gleiches Atom ausgewählt aus der Gruppe bestehend aus Sauerstoff, Schwefel und Stickstoff an den Phenyl-Rest gebunden sind;
für einen Rest ausgewählt aus der Gruppe bestehend aus Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl, [1,2,3,4]-Tetrahydronaphthyl, [1,2,3,4]-Tetrahydrochinolinyl, [1,2,3,4]-Tetrahydroisochinolinyl, 2H-Benzo[1.4]oxazin-3(4H)-onyl, (3,4)-Dihydrochinolin-2(1H)-onyl, [1,2,3,4]-Tetrahydrochinazolinyl, [3,4]-Dihydro-2H-1,4-benzoxazinyl und Benzothiazolyl steht, , wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅,-NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -S(=O)₂-CH₃, -S(=O)₂-C₂H₅,-NH-S(=O)₂-CH₃, -NH-S(=O)₂-C₂H₅, -S(=O)₂-NH-CH₃ und -S(=O)₂-NH-C₂H₅ substituiert sein kann;
oder für -(CHR¹⁷)-R²⁰, -(CHR¹⁷)-(CHR¹⁸)-R²⁰ oder -(CHR¹⁷)-(CHR¹⁸)-(CHR¹⁹)-R²⁰ steht;
R³ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht;
R⁴ für einen Wasserstoff-Rest steht oder für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht;
R⁵ und R⁷, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl, 2-Methyl-1-propenyl, Ethinyl, 1-Propinyl, 2-Propinyl, 1-Butinyl, 2-Butinyl und 3-Butinyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen, wobei der Rest ggf. jeweils mit 1, 2 oder 3 Substituenten unabhängig voneinander ausgewählt aus der Gruppe bestehend aus Methyl, Ethyl, n-Propyl, Isopropyl, n-Butyl, sec-Butyl, Isobutyl und tert-Butyl substituiert sein kann;
für einen Rest ausgewählt aus der Gruppe bestehend aus Phenyl, (1,3)-Benzodioxolyl, (1,4)-Benzodioxanyl, Naphthyl, Thiophenyl, Furanyl, Pyrrolyl, Pyrazolyl, Pyranyl, Pyridinyl, Imidazolyl, Thiazolyl, Oxazolyl, Isoxazolyl, Pyridazinyl, Pyrazinyl und Pyrimidinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅,-C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -0-Phenyl, -O-Benzyl, Phenyl und Benzyl substituiert sein kann, wobei jeweils der zyklische Teil der Reste Pyridinyl, Cyclopentyl, Cyclohexyl, Pyridazinyl,-S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN,-NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
oder für -(CR²¹R²²)-R²⁵, -(CR²¹R²²)-(CHR²³)-R²⁵, -(CR²¹R²²)-(CHR²³)-O-R²⁵,-(CR²¹R²²)-(CHR²³)-(CHR²⁴)-R²⁵, -(CR²¹R²²)-(CHR²³)-(CHR²⁴)-O-R²⁵,-(CR²¹R²²)-(CHR²³)-(CHR²⁴)-N(CH₃)-R²⁵ oder -(CR²¹R²²)-(CHR²³)-(CHR²⁴)-N(C₂H₅)-R²⁵ stehen;
R⁶ und R⁸ jeweils für einen Wasserstoff-Rest stehen;
oder für einen Rest ausgewählt aus der Gruppe bestehend aus Methyl, Ethyl, n-Propyl, Isopropyl, n-Butyl, sec-Butyl, Isobutyl und tert-Butyl;
R⁹ für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -O-CF₃, -S-CF₃, -SF₅, -OH, -O-CH₃, -O-C₂H₅, -NH-S(=O)₂-CH₃, Morpholinyl, Piperidinyl, Pyrrolidinyl, Piperazinyl, Phenyl, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht; und
R¹⁰ für einen Rest ausgewählt aus der Gruppe bestehend aus 9H-Flourenyl, 9H-Xanthenyl, Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinoxalinyl, Chinolinyl, Isochinolinyl, Benzimidazolyl, Benzoxazolyl, Benzotriazolyl, Benzisoxazolyl und Benzothiazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -O-CF₃, -S-CF₃, -SF₅, -OH, -O-CH₃, -O-C₂H₅, -NH-S(=O)₂-CH₃, Morpholinyl, Piperidinyl, Pyrrolidinyl, Piperazinyl, Phenyl, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht;
R¹³, R¹⁴, R¹⁵, R¹⁷, R¹⁸, R¹⁹, R²¹, R²², R²³, R²⁴, R²⁸, R²⁹ und R³¹, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, Methyl, Ethyl, n-Propyl, Isopropyl, n-Butyl, sec-Butyl, Isobutyl und tert-Butyl substituiert sein kann;
R¹⁶ für einen Rest ausgewählt aus der Gruppe bestehend aus Phenyl, Naphthyl, Thiophenyl, Furanyl, Pyrrolyl, Pyrazolyl, Pyrazinyl und Pyridinyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH,-S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -0-C(=O)-CH₃, -O-C(=O)-C₂H₅, -O-C(=O)-C(CH₃)₃, -S(=O)₂-CH₃, -S(=O)₂-C₂H₅,-NH-S(=O)₂-CH₃, -NH-S(=O)₂-C₂H₅, -S(=O)₂-NH-CH₃ und -S(=O)₂-NH-C₂H₅ substituiert sein kann;
R²⁰ für einen Rest ausgewählt aus der Gruppe bestehend aus Phenyl und Naphthyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH,-S-CH₃, -S-C₂H₅, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
R²⁵ für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH,-S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -0-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃,-C(=O)-H, -C(=O)-CH₃, -C(=O)-C₂H₅ und -C(=O)-C(CH₃)₃ substituiert sein kann;
R²⁶ und R²⁷, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, Methyl, Ethyl, n-Propyl, Isopropyl, n-Butyl, sec-Butyl, Isobutyl und tert-Butyl substituiert sein kann;
oder für -OH stehen;
R³² für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Phenyl, Naphthyl, Furanyl und Thiophenyl steht, der ggf. mit 1, 2, 3, 4 oder 5 Substituenten unabhängig voneinander ausgewählt aus der Gruppe bestehend aus -SF₅, F, Cl, Br, I, -CF₃, -O-CF₃, -S-CF₃, -O-CH₃, -O-C₂H₅, Phenyl, -S-CH₃, -S-C₂H₅, Cyclopentyl, Cyclohexyl, Methyl, Ethyl, n-Propyl, Isopropyl, n-Butyl, sec-Butyl, Isobutyl und tert-Butyl substituiert sein kann;
für -(CH₂)_{cc}-R³⁵ mit cc = 1, 2 oder 3 steht oder für -CH=CH-R³⁶ steht;
R³⁰, R³³ und R³⁴, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Phenyl, Pyridinyl und Naphthyl stehen, wobei der Rest ggf. jeweils mit 1, 2, 3, 4 oder 5 Substituenten unabhängig voneinander ausgewählt aus der Gruppe bestehend aus -SF₅, F, Cl, Br, -CF₃, -O-CF₃, -S-CF₃, Phenyl, Morpholinyl, Piperidinyl, Pyrrolidinyl, Piperazinyl, Cyclopentyl, Cyclohexyl, -OH, -O-CH₃, -O-C₂H₅, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
und
R³⁵ und R³⁶, unabhängig voneinander, jeweils
für einen Phenyl-Rest stehen, der mit 1, 2, 3, 4 oder 5 Substituenten unabhängig voneinander ausgewählt aus der Gruppe bestehend aus F, Cl, Br, I, -CN, -CF₃, -O-CH₃, -O-C₂H₅, -NO₂, -O-CF₃, Methyl, Ethyl, n-Propyl, Isopropyl, n-Butyl, sec-Butyl, Isobutyl und tert-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

20. Verbindungen gemäß einem oder mehreren der Ansprüche 1 bis 19, **dadurch gekennzeichnet, dass**
m gleich 0, 1 oder 2 ist;
n gleich 0, 1 oder 2 ist;
R¹ für einen Rest ausgewählt aus der Gruppe bestehend aus Benzimidazolyl, Benzoxazolyl, Benzotriazolyl, Benzisoxazolyl und Benzothiazolyl steht, wobei der Rest jeweils mit 1, 2, 3, 4 oder 5 Substituenten unabhängig voneinander ausgewählt aus der Gruppe bestehend F, Cl, Br, -CF₃, -O-CF₃, -S-CF₃, Methyl, Ethyl, n-Propyl, Isopropyl, n-Butyl, sec-Butyl, Isobutyl und tert-Butyl substituiert sein kann;
für eine -C(=O)-NR⁵R⁶-Gruppe steht;
für eine -C(=S)-NR⁷R⁸-Gruppe steht;
für eine -C(=O)-R⁹-Gruppe steht
oder für eine -S(=O)₂-R¹⁰-Gruppe steht;
R² für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl und n-Heptyl steht;
für einen Phenyl-Rest der allgemeinen Formel XX 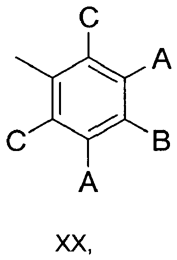 steht,
worin die Linie die Bindung dieses Phenyl-Restes zur Spiro-Verbindung der allgemeinen Formel I darstellt;
A und B jeweils für einen Substituenten unabhängig voneinander ausgewählt aus der Gruppe bestehend aus H, F, Cl, Br, I, -CF₃, -SF₅, -OH, -O-CH₃, -O-C₂H₅, -O-CF₃, -S-CF₃, Methyl, Ethyl, n-Propyl, Isopropyl, n-Butyl, sec-Butyl, Isobutyl, tert-Butyl, Cyclopentyl, Cyclohexyl, -NH-S(=O)₂-CH₃ und -NH-S(=O)₂-Phenyl stehen;
mit der Maßgabe, dass nicht eine der Positionen A und die Position B dieses Phenyl-Restes mit Substituenten substituiert sind, die jeweils über ein gleiches Atom ausgewählt aus der Gruppe bestehend aus Sauerstoff, Schwefel und Stickstoff an den Phenyl-Rest gebunden sind;
C jeweils für H steht;
für einen Rest ausgewählt aus der Gruppe bestehend aus Naphthyl, Chinolinyl, (1,4)-Benzodioxanyl, (1,3)-Benzodioxolyl, Pyridinyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, I, -O-CH₃, -O-C₂H₅, Methyl, Ethyl, n-Propyl, Isopropyl, n-Butyl, sec-Butyl, Isobutyl, tert-Butyl, -S(=O)₂-CH₃, -S(=O)₂-C₂H₅, -NH-S(=O)₂-CH₃, -NH-S(=O)₂-C₂H₅, -S(=O)₂-NH-CH₃ und -S(=O)₂-NH-C₂H₅ substituiert sein kann;
oder für -(CHR¹⁷)-R²⁰, -(CHR¹⁷)-(CHR¹⁸)-R²⁰ oder -(CHR¹⁷)-(CHR¹⁸)-(CHR¹⁹)-R²⁰ steht;
R³ für einen Rest ausgewählt aus der Gruppe bestehend aus Methyl, Ethyl, n-Propyl, Isopropyl, n-Butyl, sec-Butyl, Isobutyl, tert-Butyl und n-Pentyl steht;
R⁴ für einen Wasserstoff-Rest steht;
R⁵ und R⁷, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl und 2-Methyl-1-propenyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl und Adamantyl stehen; wobei der Rest ggf. jeweils mit 1, 2 oder 3 Substituenten unabhängig voneinander ausgewählt aus der Gruppe bestehend aus Methyl, Ethyl, n-Propyl, Isopropyl, n-Butyl, sec-Butyl, Isobutyl und tert-Butyl substituiert sein kann;
für einen Rest ausgewählt aus der Gruppe bestehend aus Phenyl, Naphthyl, (1,4)-Benzodioxanyl, und Pyridinyl stehen, wobei der Rest ggf. jeweils mit 1, 2, 3, 4 oder 5 Substituenten unabhängig voneinander ausgewählt aus der Gruppe bestehend aus -SF₅, F, Cl, Br, I, -CN, -CF₃, -O-CH₃, -O-C₂H₅, -NO₂,-O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann, wobei jeweils der zyklische Teil der Reste Cyclopentyl, Cyclohexyl, -0-Phenyl, -O-Benzyl und Phenyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl und tert-Butyl substituiert sein kann;
oder für -(CR²¹R²²)-R²⁵, -(CR²¹R²²)-(CHR²³)-R²⁵, -(CR²¹R²²)-(CHR²³)-O-R²⁵,-(CR²¹R²²)-(CHR²³)-(CHR²⁴)-R²⁵, -(CR²¹R²²)-(CHR²³)-(CHR²⁴)-O-R²⁵,-(CR²¹R²²)-(CHR²³)-(CHR²⁴)-N(CH₃)-R²⁵ oder -(CR²¹R²²)-(CHR²³)-(CHR²⁴)-N(C₂H₅)-R²⁵ stehen;
R⁶ und R⁸ jeweils für einen Wasserstoff-Rest stehen;
oder für einen Methyl- oder Ethyl-Rest stehen;
R⁹ für einen Rest ausgewählt aus der Gruppe bestehend aus Phenyl, Pyridinyl und Naphthyl steht, wobei der Rest ggf. jeweils mit 1, 2, 3, 4 oder 5 Substituenten unabhängig voneinander ausgewählt aus der Gruppe bestehend aus F, Cl, Br, I, -CF₃, -O-CF₃, -S-CF₃, -OH, -O-CH₃, -O-C₂H₅, -NH-S(=O)₂-CH₃, Morpholinyl, Piperidinyl, Pyrrolidinyl, Piperazinyl, Phenyl, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht;
R¹⁰ für einen Rest ausgewählt aus der Gruppe bestehend aus Phenyl und Naphthyl steht, wobei der Rest ggf. jeweils mit 1, 2, 3, 4 oder 5 Substituenten unabhängig voneinander ausgewählt aus der Gruppe bestehend aus F, Cl, Br, I, -CF₃, -OH, -O-CH₃, -O-C₂H₅, Methyl, Ethyl, n-Propyl, Isopropyl, n-Butyl, sec-Butyl, Isobutyl und tert-Butyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht;
R¹⁷, R¹⁸, R¹⁹, R²³, R²⁴, R²⁸ und R²⁹, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen;
R²⁰ für einen Phenyl-Rest steht;
R²¹ und R²², unabhängig voneinander, jeweils für
einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen;
R²⁵ für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopentyl, Cyclohexyl, Tetrahydrofuranyl, Tetrahydrothiophenyl, Pyrrolidinyl, Morpholinyl, Piperidinyl und Piperazinyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, -OH, -O-CH₃, -O-C₂H₅, Methyl, Ethyl, n-Propyl, Isopropyl, n-Butyl, sec-Butyl, Isobutyl und tert-Butyl substituiert sein kann;
R²⁶ und R²⁷, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
für einen Phenyl-Rest stehen oder für -OH stehen;
R³⁰ für einen Phenyl-Rest steht, der mit 1, 2, 3, 4 oder 5 Substituenten unabhängig voneinander ausgewählt aus der Gruppe bestehend aus F, Cl, Br, -NH-S(=O)₂-CH₃, Methyl, Ethyl, n-Propyl, Isopropyl, n-Butyl, sec-Butyl, Isobutyl und tert-Butyl substituiert sein kann;
R³¹ für einen Wasserstoff-Rest steht;
oder für einen Rest ausgewählt aus der Gruppe bestehend aus Methyl, Ethyl, n-Propyl, Isopropyl, n-Butyl, sec-Butyl, Isobutyl und tert-Butyl steht;
R³² für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Phenyl, Naphthyl, Furanyl und Thiophenyl steht, der ggf. mit 1, 2, 3, 4 oder 5 Substituenten unabhängig voneinander ausgewählt aus der Gruppe bestehend aus -SF₅, F, Cl, Br, I, -CF₃, -O-CF₃, -S-CF₃, -O-CH₃, -O-C₂H₅, Phenyl, -S-CH₃, -S-C₂H₅, Cyclopentyl, Cyclohexyl, Methyl, Ethyl, n-Propyl, Isopropyl, n-Butyl, sec-Butyl, Isobutyl und tert-Butyl substituiert sein kann;
für -CH₂-R³⁵ oder für -CH=CH-R³⁶ steht;
R³³ und R³⁴, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Phenyl, Pyridinyl und Naphthyl stehen, wobei der Rest ggf. jeweils mit 1, 2, 3, 4 oder 5 Substituenten unabhängig voneinander ausgewählt aus der Gruppe bestehend aus -SF₅, F, Cl, Br, -CF₃, -O-CF₃, -S-CF₃, Phenyl, Morpholinyl, Piperidinyl, Pyrrolidinyl, Piperazinyl, Cyclopentyl, Cyclohexyl, -OH, -O-CH₃, -O-C₂H₅, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
und
R³⁵ und R³⁶ jeweils für einen Phenyl-Rest stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

21. Verbindungen gemäß einem oder mehreren der Ansprüche 1 bis 20, **dadurch gekennzeichnet, dass**
m gleich 0, 1 oder 2 ist;
n gleich 0, 1 oder 2 ist;
R¹ für einen Rest ausgewählt aus der Gruppe bestehend aus Benzimidazolyl, Benzoxazolyl, Benzotriazolyl, Benzisoxazolyl und Benzothiazolyl steht, wobei der Rest jeweils mit 1, 2, 3, 4 oder 5 Substituenten unabhängig voneinander ausgewählt aus der Gruppe bestehend F, Cl, Br, -CF₃, -O-CF₃, -S-CF₃, Methyl, Ethyl, n-Propyl, Isopropyl, n-Butyl, sec-Butyl, Isobutyl und tert-Butyl substituiert sein kann;
für eine -C(=O)-NR⁵R⁶-Gruppe steht;
für eine -C(=S)-NR⁷R⁸-Gruppe steht;
für eine -C(=O)-R⁹-Gruppe steht
oder für eine -S(=O)₂-R¹⁰-Gruppe steht;
R² für einen tert-Butyl-Rest steht;
für einen Rest ausgewählt aus der Gruppe bestehend aus Phenyl, 2-Methansulfonamid-phenyl, 2-Ethansulfonamid-phenyl, 2-Trifluoromethylphenyl, 2-Trifluoromethylsulfanyl-phenyl, 2-Ethyl-phenyl, 2-tert-Butyl-phenyl, 2-Ethylamino-sulfonyl-phenyl, 2-Methylamino-sulfonyl-phenyl, 2-Bromo-phenyl, 2-Chloro-phenyl, 2-Fluoro-phenyl, 2-Methyl-phenyl, 2-Trifluoromethoxy-phenyl, 2-Methoxy-phenyl, 2-Ethoxy-phenyl, 2-Propyl-phenyl, 2-Iodo-phenyl, 3-Chlorophenyl, 3-Methyl-phenyl, 3-tert-Butyl-phenyl, 3-Trifluoromethylsulfanyl-phenyl, 3-Trifluoromethyl-phenyl, 3-Methansulfonamid-phenyl, 3-Ethansulfonamidphenyl, 3-Fluoro-phenyl, 3-Propyl-phenyl, 3-Isopropyl-phenyl, 3-Bromo-phenyl, 3-Methoxy-phenyl, 3-Ethyl-phenyl, 3-Ethylamino-sulfonyl-phenyl, 3-Methylamino-sulfonyl-phenyl, 3-Ethoxy-phenyl, 3-Trifluoromethoxy-phenyl, 3-lodophenyl, 4-Methylamino-sulfonyl-phenyl, 4-Ethylamino-sulfonyl-phenyl, 4-Methansulfonamid-phenyl, 4-Ethansulfonamid-phenyl, 4-Bromo-phenyl, 4-Methoxy-phenyl, 4-Chloro-phenyl, 4-Fluoro-phenyl, 4-tert-Butyl-phenyl, 4-Trifluoromethylsulfanyl-phenyl, 4-Methyl-phenyl, 4-Isopropyl-phenyl, 4-Trifluoromethyl-phenyl, 4-Propyl-phenyl, 4-Iodo-phenyl, 4-Trifluoromethoxyphenyl, 4-Ethyl-phenyl, 4-Ethoxy-phenyl, 2-Fluoro-3-trifluoromethylphenyl, (2,3)-Difluorophenyl, (2,3)-Dimethyl-phenyl, (2,3)-Dichlorophenyl, 3-Fluoro-2-trifluoromethylphenyl, (2,4)-Dichloro-phenyl, (2,4)-Difluorophenyl, 4-Fluoro-2-trifluoromethyl-phenyl, (2,4)-Dimethoxyphenyl, 2-Chloro-4-fluoro-phenyl, (2,4)-Dibromo-phenyl, 2-Fluoro-4-trifluoromethylphenyl, (2,5)-Difluoro-phenyl, 2-Fluoro-5-trifluoromethyl-phenyl, 5-Fluoro-2-trifluoromethyl-phenyl, 5-Chloro-2-trifluoromethyl-phenyl, 5-Bromo-2-trifluoromethyl-phenyl, (2,5)-Dimethoxy-phenyl, (2,5)-Bis-trifluoromethylphenyl, (2,5)-Dichloro-phenyl, (2,5)-Dibromo-phenyl, 2-Fluor-6-trifluoromethylphenyl, (2,6)-Dimethoxy-phenyl, (2,6)-Dimethyl-phenyl, (2,6)-Dichloro-phenyl, 2-Chloro-6-fluoro-phenyl, 2-Bromo-6-chloro-phenyl, 2-Bromo-6-fluoro-phenyl, (2,6)-Difluoro-phenyl, (2,6)-Difluoro-3-methyl-phenyl, (2,6)-Dibromo-phenyl, (2,6)-Dichlorophenyl, 3-Chloro-2-fluoro-phenyl, (3,4)-Dichlorophenyl, 4-Fluoro-3-trifluoromethylphenyl, 3-Fluoro-4-trifluoromethyl-phenyl, (3,4)-Difluorophenyl, 4-Chloro-3-trifluoromethyl, 4-Bromo-3-methyl-phenyl, 4-Bromo-5-methyl-phenyl, 3-Chloro-4-fluoro-phenyl, (3,4)-Dibromo-phenyl, 4-Chlor-3-methyl-phenyl, 4-Bromo-3-methyl-phenyl, 4-Fluoro-3-methyl-phenyl, (3,5)-Dimethoxy-phenyl, (3,5)-Bis-trifluoromethyl-phenyl, (3,5)-Difluoro-phenyl, (3,5)-Dichloro-phenyl, 3-Fluoro-5-trifluoromethyl-phenyl, 5-Fluoro-3-trifluoromethyl-phenyl, (3,5)-Dibromo-phenyl, 5-Chloro-4-fluoro-phenyl, 5-Bromo-4-methyl-phenyl, (2,3,4)-Trifluorophenyl, (2,3,4)-Trichlorophenyl, (2,3,6)-Trifluorophenyl, 5-Chloro-2-methoxy-phenyl, (2,3)-Difluoro-4-methyl-phenyl, (2,4,5)-Trifluoro-phenyl, (2,4,5)-Trichloro-phenyl, (2,4)-Dichloro-5-fluoro-phenyl, (2,4,6)-Trichloro-phenyl, (2,4,6)-Trimethylphenyl, (2,4,6)-Trifluoro-phenyl, (2,4,6)-Trimethoxy-phenyl, (2,3,4,5)-Tetrafluoro-phenyl, 4-Methoxy-2,3,6-trimethyl-phenyl, 4-Methoxy-2,3,6-trimethyl-phenyl, 4-Chloro-2,5-dimethyl-phenyl, 2-Chloro-6-fluoro-3-methyl-phenyl, 6-Chloro-2-fluoro-3-methyl, (2,3,4,5,6)-Pentafluoro-phenyl, 3-Fluoro-4-methylsulfonamido-phenyl, 3-Chlor-4-methylsulfonamido-phenyl, 3-Brom-4-methylsulfonamido-phenyl, 3-Methoxy-4-methylsulfonamido-phenyl, 3-Hydroxy-4-methylsulfonamido-phenyl, 3-Trifluoromethyl-4-methylsulfonamido-phenyl, 3-Trifluoromethoxy-4-methylsulfonamido-phenyl, 3-Methyl-4-methylsulfonamido-phenyl, 3-Ethyl-4-methylsulfonamido-phenyl, 3-Isopropyl-4-methylsulfonamido-phenyl, 3-Propyl-4-methylsulfonamido-phenyl, 3-tert-Butyl-4-methylsulfonamido-phenyl, 3-Fluoro-4-phenylsulfonamido-phenyl, 3-Chlor-4-phenylsulfonamido-phenyl, 3-Brom-4-phenylsulfonamido-phenyl, 3-Methoxy-4-phenylsulfonamido-phenyl, 3-Hydroxy-4-phenylsulfonamido-phenyl, 3-Trifluoromethyl-4-phenylsulfonamido-phenyl, 3-Trifluoromethoxy-4-phenylsulfonamido-phenyl, 3-Methyl-4-phenylsulfonamido-phenyl, 3-Ethyl-4-phenylsulfonamido-phenyl, 3-Isopropyl-4-phenylsulfonamido-phenyl, 3-Propyl-4-phenylsulfonamido-phenyl, 3-tert-Butyl-4-phenylsulfonamido-phenyl, 4-Fluoro-3-methylsulfonamido-phenyl, 4-Chlor-3-methylsulfonamido-phenyl, 4-Brom-3-methylsulfonamido-phenyl, 4-Methoxy-3-methylsulfonamido-phenyl, 4-Hydroxy-3-methylsulfonamido-phenyl, 4-Trifluoromethyl-3-methylsulfonamido-phenyl, 4-Trifluoromethoxy-3-methylsulfonamido-phenyl, 4-Methyl-3-methylsulfonamido-phenyl, 4-Ethyl-3-methylsulfonamido-phenyl, 4-Isopropyl-3-methylsulfonamido-phenyl, 4-Propyl-3-methylsulfonamido-phenyl, 4-tert-Butyl-3-methylsulfonamido-phenyl, 4-Fluoro-3-phenylsulfonamido-phenyl, 4-Chlor-3-phenylsulfonamido-phenyl, 4-Brom-3-phenylsulfonamido-phenyl, 4-Methoxy-3-phenylsulfonamido-phenyl, 4-Hydroxy-3-phenylsulfonamido-phenyl, 4-Trifluoromethyl-3-phenylsulfonamido-phenyl, 4-Trifluoromethoxy-3-phenylsulfonamido-phenyl, 4-Methyl-3-phenylsulfonamido-phenyl, 4-Ethyl-3-phenylsulfonamido-phenyl, 4-Isopropyl-3-phenylsulfonamido-phenyl, 4-Propyl-3-phenylsulfonamido-phenyl, 4-tert-Butyl-3-phenylsulfonamido-phenyl, 2-Cyclohexyl-phenyl, 3-Cyclohexyl-phenyl und 4-Cyclohexyl-phenyl steht;
für einen Rest ausgewählt aus der Gruppe bestehend aus Chinolinyl, (1,4)-Benzodioxanyl, (1,3)-Benzodioxolyl, Naphthyl und Thiazolyl steht;
für einen Pyridinyl-Rest steht, wobei der Rest jeweils mit 1 oder 2 Substituenten unabhängig voneinander ausgewählt aus der Gruppe bestehend aus F, Cl und Br substituiert sein kann;
für -(CHR¹⁷)-R²⁰ oder -(CHR¹⁷)-(CHR¹⁸)-R²⁰ steht;
R³ für einen Methyl- oder Ethyl-Rest steht;
R⁴ für einen Wasserstoff-Rest steht;
R⁵ und R⁷, unabhängig voneinander, jeweils für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Vinyl, 1-Propenyl, 2-Propenyl, 1-Butenyl, 2-Butenyl, 3-Butenyl und 2-Methyl-1-propenyl stehen, wobei der Rest ggf. jeweils mit 1, 2, 3, 4 oder 5 Substituenten unabhängig voneinander ausgewählt aus der Gruppe bestehend aus F, Cl und Br substituiert sein kann;
für einen Rest ausgewählt aus der Gruppe bestehend aus Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentenyl, Cyclohexenyl, Cycloheptenyl und Adamantyl stehen; wobei der Rest ggf. jeweils mit 1, 2 oder 3 Substituenten unabhängig voneinander ausgewählt aus der Gruppe bestehend aus Methyl, Ethyl, n-Propyl, Isopropyl, n-Butyl, sec-Butyl, Isobutyl und tert-Butyl substituiert sein kann;
für einen Rest ausgewählt aus der Gruppe bestehend aus Phenyl, Naphthyl, (1,4)-Benzodioxanyl und Pyridinyl stehen, wobei der Rest ggf. jeweils mit 1, 2, 3, 4 oder 5 Substituenten unabhängig voneinander ausgewählt aus der Gruppe bestehend aus -SF₅, F, Cl, Br, I, -CN, -CF₃, -O-CH₃, -O-C₂H₅, -NO₂,-O-CF₃, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -C(=O)-CH₃, -C(=O)-C₂H₅, -C(=O)-C(CH₃)₃, Cyclohexyl, Cyclopentyl, -O-Phenyl, -O-Benzyl und Phenyl substituiert sein kann, wobei jeweils der zyklische Teil der Reste Cyclopentyl, Cyclohexyl, -0-Phenyl, -O-Benzyl und Phenyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl und tert-Butyl substituiert sein kann;
oder für -(CR²¹R²²)-R²⁵, -(CR²¹R²²)-(CHR²³)-R²⁵, -(CR²¹R²²)-(CHR²³)-O-R²⁵,-(CR²¹R²²)-(CHR²³)-(CHR²⁴)-R²⁵, -(CR²¹R²²)-(CHR²³)-(CHR²⁴)-O-R²⁵,-(CR²¹R²²)-(CHR²³)-(CHR²⁴)-N(CH₃)-R²⁵ oder -(CR²¹R²²)-(CHR²³)-(CHR²⁴)-N(C₂H₅)-R²⁵ stehen;
R⁶ und R⁸ jeweils für einen Wasserstoff-Rest stehen;
oder für einen Methyl- oder Ethyl-Rest stehen;
R⁹ für einen Rest ausgewählt aus der Gruppe bestehend aus Phenyl, Pyridinyl und Naphthyl steht, wobei der Rest ggf. jeweils mit 1, 2, 3, 4 oder 5 Substituenten unabhängig voneinander ausgewählt aus der Gruppe bestehend aus F, Cl, Br, I, -CF₃, -O-CF₃, -S-CF₃, -OH, -O-CH₃, -O-C₂H₅, -NH-S(=O)₂-CH₃, Morpholinyl, Piperidinyl, Pyrrolidinyl, Piperazinyl, Phenyl, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht;
R¹⁰ für einen Rest ausgewählt aus der Gruppe bestehend aus Phenyl und Naphthyl steht, wobei der Rest ggf. jeweils mit 1, 2, 3, 4 oder 5 Substituenten unabhängig voneinander ausgewählt aus der Gruppe bestehend aus F, Cl, Br, I, -CF₃, -OH, -O-CH₃, -O-C₂H₅, Methyl, Ethyl, n-Propyl, Isopropyl, n-Butyl, sec-Butyl, Isobutyl und tert-Butyl substituiert sein kann;
für -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ oder für -C≡C-R³⁴ steht;
R¹⁷, R¹⁸, R¹⁹, R²³, R²⁴, R²⁸ und R²⁹, unabhängig voneinander, jeweils
für einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen;
R²⁰ für einen Phenyl-Rest steht;
R²¹ und R²², unabhängig voneinander, jeweils für
einen Wasserstoff-Rest stehen;
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Phenyl-Rest stehen;
R²⁵ für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Cyclopentyl, Cyclohexyl, Tetrahydrofuranyl, Tetrahydrothiophenyl, Pyrrolidinyl, Morpholinyl, Piperidinyl und Piperazinyl steht;
oder für einen Rest ausgewählt aus der Gruppe bestehend aus Phenyl, Naphthyl, (1,3)-Benzodioxolyl, (1,4)-Benzodioxanyl, Thiophenyl und Furanyl steht, wobei der Rest ggf. jeweils mit 1, 2, 3, 4 oder 5 Substituenten unabhängig voneinander ausgewählt aus der Gruppe bestehend aus F, Cl, Br, -OH, -O-CH₃, -O-C₂H₅, Methyl, Ethyl, n-Propyl, Isopropyl, n-Butyl, sec-Butyl, Isobutyl und tert-Butyl substituiert sein kann;
R²⁶ und R²⁷, unabhängig voneinander, jeweils
für einen Wasserstoff-Rest
für einen Rest ausgewählt aus der Gruppe bestehend aus Methyl, Ethyl, n-Propyl, Isopropyl, n-Butyl, sec-Butyl, Isobutyl und tert-Butyl stehen;
für einen Phenyl-Rest stehen
oder für -OH stehen;
R³⁰ für einen Phenyl-Rest steht, der mit 1, 2, 3, 4 oder 5 Substituenten unabhängig voneinander ausgewählt aus der Gruppe bestehend aus F, Cl, Br, -NH-S(=O)₂-CH₃, Methyl, Ethyl, n-Propyl, Isopropyl, n-Butyl, sec-Butyl, Isobutyl und tert-Butyl substituiert sein kann;
R³¹ für einen Wasserstoff-Rest steht;
oder für einen Rest ausgewählt aus der Gruppe bestehend aus Methyl, Ethyl, n-Propyl, Isopropyl, n-Butyl, sec-Butyl, Isobutyl und tert-Butyl steht;
R³² für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl und tert-Butyl steht;
für einen Rest ausgewählt aus der Gruppe bestehend aus Phenyl, Naphthyl, Furanyl und Thiophenyl steht, der ggf. mit 1, 2, 3, 4 oder 5 Substituenten unabhängig voneinander ausgewählt aus der Gruppe bestehend aus -SF₅, F, Cl, Br, I, -CF₃, -O-CF₃, -S-CF₃, -O-CH₃, -O-C₂H₅, Phenyl, -S-CH₃, -S-C₂H₅, Cyclopentyl, Cyclohexyl, Methyl, Ethyl, n-Propyl, Isopropyl, n-Butyl, sec-Butyl, Isobutyl und tert-Butyl substituiert sein kann;
für -CH₂-R³⁵ oder für -CH=CH-R³⁶ steht;
R³³ und R³⁴, unabhängig voneinander, jeweils
für einen Rest ausgewählt aus der Gruppe bestehend aus Methyl, Ethyl, n-Propyl, Isopropyl, n-Butyl, sec-Butyl, Isobutyl und tert-Butyl stehen;
oder für einen Rest ausgewählt aus der Gruppe bestehend aus Phenyl, Pyridinyl und Naphthyl stehen, wobei der Rest ggf. jeweils mit 1, 2, 3, 4 oder 5 Substituenten unabhängig voneinander ausgewählt aus der Gruppe bestehend aus -SF₅, F, Cl, Br, -CF₃, -O-CF₃, -S-CF₃, Phenyl, Morpholinyl, Piperidinyl, Pyrrolidinyl, Piperazinyl, Cyclopentyl, Cyclohexyl, -OH, -O-CH₃, -O-C₂H₅, Methyl, Ethyl, n-Propyl, Isopropyl, n-Butyl, sec-Butyl, Isobutyl, tert-Butyl, n-Pentyl, n-Hexyl, n-Heptyl und n-Octyl substituiert sein kann;
und
R³⁵ und R³⁶ jeweils für einen Phenyl-Rest stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

22. Verbindungen gemäß einem oder mehreren der Ansprüche 1 bis 21 ausgewählt aus der Gruppe bestehend aus
[1] 3-(3-Methoxy-phenyl)-1-oxa-2,8-diaza-spiro[4.5]dec-2-en-8-carbonsäurephenylamid
[2] 3-Phenyl-1-oxa-2,8-diaza-spiro[4.5]dec-2-en-8-carbonsäurephenylamid
[3] 3-(4-Chlor-phenyl)-1-oxa-2,8-diaza-spiro[4.5]dec-2-en-8-carbonsäurephenylamid
[4] 3-(4-tert-Butyl-phenyl)-1-oxa-2,8-diaza-spiro[4.5]dec-2-en-8-carbonsäurephenylamid
[5] 3-p-Tolyl-1-oxa-2,8-diaza-spiro[4.5]dec-2-en-8-carbonsäurephenylamid
[6] 3-(4-Trifluormethyl-phenyl)-1-oxa-2,8-diaza-spiro[4.5]dec-2-en-8-carbonsäurephenylamid
[7] 3-(3-Methoxy-phenyl)-1-oxa-2,8-diaza-spiro[4.5]dec-2-en-8-carbonsäure-m-tolylamid
[8] 3-(3-Methoxy-phenyl)-1-oxa-2,8-diaza-spiro[4.5]dec-2-en-8-carbonsäure-m-tolylamid
[9] 3-Phenyl-1-oxa-2,8-diaza-spiro[4.5]dec-2-en-8-carbonsäure-m-tolylamid
[10] 3-(4-Chlor-phenyl)-1-oxa-2,8-diaza-spiro[4.5]dec-2-en-8-carbonsäure-m-tolylamid
[11] 3-(4-tert-Butyl-phenyl)-1-oxa-2,8-diaza-spiro[4.5]dec-2-en-8-carbonsäure-m-tolylamid
[12] 3-p-Tolyl-1-oxa-2,8-diaza-spiro[4.5]dec-2-en-8-carbonsäure-m-tolylamid
[13] 3-(4-Trifluormethyl-phenyl)-1-oxa-2,8-diaza-spiro[4.5]dec-2-en-8-carbonsäure-m-tolylamid
[14] 3-(3-Methoxy-phenyl)-1-oxa-2,8-diaza-spiro[4.5]dec-2-en-8-carbonsäure-(3-ethyl-phenyl)-amid
[15] 3-(3-Methoxy-phenyl)-1-oxa-2,8-diaza-spiro[4.5]dec-2-en-8-carbonsäure-(3-ethyl-phenyl)-amid
[16] 3-Phenyl-1-oxa-2,8-diaza-spiro[4.5]dec-2-en-8-carbonsäure-(3-ethyl-phenyl)-amid
[17] 3-(4-Chlor-phenyl)-1-oxa-2,8-diaza-spiro[4.5]dec-2-en-8-carbonsäure-(3-ethylphenyl)-amid
[18] 3-(4-tert-Butyl-phenyl)-1-oxa-2,8-diaza-spiro[4.5]dec-2-en-8-carbonsäure-(3-ethyl-phenyl)-amid
[19] 3-p-Tolyl-1-oxa-2,8-diaza-spiro[4.5]dec-2-en-8-carbonsäure-(3-ethyl-phenyl)-amid
[20] 3-(4-Trifluormethyl-phenyl)-1-oxa-2,8-diaza-spiro[4.5]dec-2-en-8-carbonsäure-(3-ethyl-phenyl)-amid
[21] 3-(3-Methoxy-phenyl)-1-oxa-2,8-diaza-spiro[4.5]dec-2-en-8-carbonsäure-(2-propyl-phenyl)-amid
[22] 3-Phenyl-1-oxa-2,8-diaza-spiro[4.5]dec-2-en-8-carbonsäure-(2-propyl-phenyl)-amid
[23] 3-(4-Chlor-phenyl)-1-oxa-2,8-diaza-spiro[4.5]dec-2-en-8-carbonsäure-(2-propyl-phenyl)-amid
[24] 3-(4-tert-Butyl-phenyl)-1-oxa-2,8-diaza-spiro[4.5]dec-2-en-8-carbonsäure-(2-propyl-phenyl)-amid
[25] 3-p-Tolyl-1-oxa-2,8-diaza-spiro[4.5]dec-2-en-8-carbonsäure-(2-propyl-phenyl)-amid
[26] 3-(4-Trifluormethyl-phenyl)-1-oxa-2,8-diaza-spiro[4.5]dec-2-en-8-carbonsäure-(2-propyl-phenyl)-amid
[27] 3-(3-Methoxy-phenyl)-1-oxa-2,8-diaza-spiro[4.5]dec-2-en-8-carbonsäure-(4-fluor-phenyl)-amid
[28] 3-Phenyl-1-oxa-2,8-diaza-spiro[4.5]dec-2-en-8-carbonsäure-(4-fluor-phenyl)-amid
[29] 3-(4-Chlor-phenyl)-1-oxa-2,8-diaza-spiro[4.5]dec-2-en-8-carbonsäure-(4-fluorphenyl)-amid
[30] 3-(4-tert-Butyl-phenyl)-1-oxa-2,8-diaza-spiro[4.5]dec-2-en-8-carbonsäure-(4-fluor-phenyl)-amid
[31] 3-p-Tolyl-1-oxa-2,8-diaza-spiro[4.5]dec-2-en-8-carbonsäure-(4-fluor-phenyl)-amid
[32] 3-(4-Trifluormethyl-phenyl)-1-oxa-2,8-diaza-spiro[4.5]dec-2-en-8-carbonsäure-(4-fluor-phenyl)-amid
[33] 3-(3-Methoxy-phenyl)-1-oxa-2,8-diaza-spiro[4.5]dec-2-en-8-carbonsäure-(3-chlor-phenyl)-amid
[34] 3-Phenyl-1-oxa-2,8-diaza-spiro[4.5]dec-2-en-8-carbonsäure-(3-chlor-phenyl)-amid
[35] 3-(4-Chlor-phenyl)-1-oxa-2,8-diaza-spiro[4.5]dec-2-en-8-carbonsäure-(3-chlorphenyl)-amid
[36] 3-(4-tert-Butyl-phenyl)-1-oxa-2,8-diaza-spiro[4.5]dec-2-en-8-carbonsäure-(3-chlor-phenyl)-amid
[37] 3-p-Tolyl-1-oxa-2,8-diaza-spiro[4.5]dec-2-en-8-carbonsäure-(3-chlor-phenyl)-amid
[38] 3-(4-Trifluormethyl-phenyl)-1-oxa-2,8-diaza-spiro[4.5]dec-2-en-8-carbonsäure-(3-chlor-phenyl)-amid
[39] 3-(3-Methoxy-phenyl)-1-oxa-2,8-diaza-spiro[4.5]dec-2-en-8-carbonsäure-(4-chlor-phenyl)-amid
[40] 3-Phenyl-1-oxa-2,8-diaza-spiro[4.5]dec-2-en-8-carbonsäure-(4-chlor-phenyl)-amid
[41] 3-(4-Chlor-phenyl)-1-oxa-2,8-diaza-spiro[4.5]dec-2-en-8-carbonsäure-(4-chlorphenyl)-amid
[42] 3-(4-tert-Butyl-phenyl)-1-oxa-2,8-diaza-spiro[4.5]dec-2-en-8-carbonsäure-(4-chlor-phenyl)-amid
[43] 3-p-Tolyl-1-oxa-2,8-diaza-spiro[4.5]dec-2-en-8-carbonsäure-(4-chlor-phenyl)-amid
[44] 3-Phenyl-1-oxa-2,8-diaza-spiro[4.5]dec-2-en-8-carbonsäure-(4-iodo-phenyl)-amid
[45] 3-(4-tert-Butyl-phenyl)-1-oxa-2,8-diaza-spiro[4.5]dec-2-en-8-carbonsäure-(4-iodo-phenyl)-amid
[46] 3-(4-Trifluormethyl-phenyl)-1-oxa-2,8-diaza-spiro[4.5]dec-2-en-8-carbonsäure-(4-iodo-phenyl)-amid
[47] 3-(3-Methoxy-phenyl)-1-oxa-2,8-diaza-spiro[4.5]dec-2-en-8-carbonsäure-(4-methoxy-phenyl)-amid
[48] 3-Phenyl-1-oxa-2,8-diaza-spiro[4.5]dec-2-en-8-carbonsäure-(4-methoxy-phenyl)-amid
[49] 3-(4-Chlor-phenyl)-1-oxa-2,8-diaza-spiro[4.5]dec-2-en-8-carbonsäure-(4-methoxy-phenyl)-amid
[50] 3-(4-tert-Butyl-phenyl)-1-oxa-2,8-diaza-spiro[4.5]dec-2-en-8-carbonsäure-(4-methoxy-phenyl)-amid
[51] 3-p-Tolyl-1-oxa-2,8-diaza-spiro[4.5]dec-2-en-8-carbonsäure-(4-methoxy-phenyl)-amid
[52] 3-(4-Trifluormethyl-phenyl)-1-oxa-2,8-diaza-spiro[4.5]dec-2-en-8-carbonsäure-(4-methoxy-phenyl)-amid
[53] 3-(3-Methoxy-phenyl)-1-oxa-2,8-diaza-spiro[4.5]dec-2-en-8-carbonsäure-(2-methylsulfanyl-phenyl)-amid
[54] 3-Phenyl-1-oxa-2,8-diaza-spiro[4.5]dec-2-en-8-carbonsäure-(2-methylsulfanyl-phenyl)-amid
[55] 3-(4-Chlor-phenyl)-1-oxa-2,8-diaza-spiro[4.5]dec-2-en-8-carbonsäure-(2-methylsulfanyl-phenyl)-amid
[56] 3-(4-tert-Butyl-phenyl)-1-oxa-2,8-diaza-spiro[4.5]dec-2-en-8-carbonsäure-(2-methylsulfanyl-phenyl)-amid
[57] 3-p-Tolyl-1-oxa-2,8-diaza-spiro[4.5]dec-2-en-8-carbonsäure-(2-methylsulfanyl-phenyl)-amid
[58] 3-(4-Trifluormethyl-phenyl)-1-oxa-2,8-diaza-spiro[4.5]dec-2-en-8-carbonsäure-(2-methylsulfanyl-phenyl)-amid
[59] 3-(3-Methoxy-phenyl)-1-oxa-2,8-diaza-spiro[4.5]dec-2-en-8-carbonsäure-(3-methylsulfanyl-phenyl)-amid
[60] 3-Phenyl-1-oxa-2,8-diaza-spiro[4.5]dec-2-en-8-carbonsäure-(3-methylsulfanyl-phenyl)-amid
[61] 3-(4-Chlor-phenyl)-1-oxa-2,8-diaza-spiro[4.5]dec-2-en-8-carbonsäure-(3-methylsulfanyl-phenyl)-amid
[62] 3-(4-tert-Butyl-phenyl)-1-oxa-2,8-diaza-spiro[4.5]dec-2-en-8-carbonsäure-(3-methylsulfanyl-phenyl)-amid
[63] 3-p-Tolyl-1-oxa-2,8-diaza-spiro[4.5]dec-2-en-8-carbonsäure-(3-methylsulfanyl-phenyl)-amid
[64] 3-(4-Trifluormethyl-phenyl)-1-oxa-2,8-diaza-spiro[4.5]dec-2-en-8-carbonsäure-(3-methylsulfanyl-phenyl)-amid
[65] 3-(3-Methoxy-phenyl)-1-oxa-2,8-diaza-spiro[4.5]dec-2-en-8-carbonsäure-(4-methylsulfanyl-phenyl)-amid
[66] 3-Phenyl-1-oxa-2,8-diaza-spiro[4.5]dec-2-en-8-carbonsäure-(4-methylsulfanyl-phenyl)-amid
[67] 3-(4-Chlor-phenyl)-1-oxa-2,8-diaza-spiro[4.5]dec-2-en-8-carbonsäure-(4-methylsulfanyl-phenyl)-amid
[68] 3-(4-tert-Butyl-phenyl)-1-oxa-2,8-diaza-spiro[4.5]dec-2-en-8-carbonsäure-(4-methylsulfanyl-phenyl)-amid
[69] 3-p-Tolyl-1-oxa-2,8-diaza-spiro[4.5]dec-2-en-8-carbonsäure-(4-methylsulfanyl-phenyl)-amid
[70] 3-(4-Trifluormethyl-phenyl)-1-oxa-2,8-diaza-spiro[4.5]dec-2-en-8-carbonsäure-(4-methylsulfanyl-phenyl)-amid
[71] 3-(3-Methoxy-phenyl)-1-oxa-2,8-diaza-spiro[4.5]dec-2-en-8-carbonsäure-(2-isopropyl-phenyl)-amid
[72] 3-Phenyl-1-oxa-2,8-diaza-spiro[4.5]dec-2-en-8-carbonsäure-(2-isopropyl-phenyl)-amid
[73] 3-(4-Chlor-phenyl)-1-oxa-2,8-diaza-spiro[4.5]dec-2-en-8-carbonsäure-(2-isopropyl-phenyl)-amid
[74] 3-(4-tert-Butyl-phenyl)-1-oxa-2,8-diaza-spiro[4.5]dec-2-en-8-carbonsäure-(2-isopropyl-phenyl)-amid
[75] 3-p-Tolyl-1-oxa-2,8-diaza-spiro[4.5]dec-2-en-8-carbonsäure-(2-isopropylphenyl)-amid
[76] 3-(4-Trifluormethyl-phenyl)-1-oxa-2,8-diaza-spiro[4.5]dec-2-en-8-carbonsäure-(2-isopropyl-phenyl)-amid
[77] 3-(3-Methoxy-phenyl)-1-oxa-2,8-diaza-spiro[4.5]dec-2-en-8-carbonsäure-(4-isopropyl-phenyl)-amid
[78] 3-Phenyl-1-oxa-2,8-diaza-spiro[4.5]dec-2-en-8-carbonsäure-(4-isopropylphenyl)-amid
[79] 3-(4-Chlor-phenyl)-1-oxa-2,8-diaza-spiro[4.5]dec-2-en-8-carbonsäure-(4-isopropyl-phenyl)-amid
[80] 3-(4-tert-Butyl-phenyl)-1-oxa-2,8-diaza-spiro[4.5]dec-2-en-8-carbonsäure-(4-isopropyl-phenyl)-amid
[81] 3-p-Tolyl-1-oxa-2,8-diaza-spiro[4.5]dec-2-en-8-carbonsäure-(4-isopropylphenyl)-amid
[82] 3-(4-Trifluormethyl-phenyl)-1-oxa-2,8-diaza-spiro[4.5]dec-2-en-8-carbonsäure-(4-isopropyl-phenyl)-amid
[83] 3-(3-Methoxy-phenyl)-1-oxa-2,8-diaza-spiro[4.5]dec-2-en-8-carbonsäure-(2-trifluormethyl-phenyl)-amid
[84] 3-Phenyl-1-oxa-2,8-diaza-spiro[4.5]dec-2-en-8-carbonsäure-(2-trifluormethyl-phenyl)-amid
[85] 3-(4-Chlor-phenyl)-1-oxa-2,8-diaza-spiro[4.5]dec-2-en-8-carbonsäure-(2-trifluormethyl-phenyl)-amid
[86] 3-(4-tert-Butyl-phenyl)-1-oxa-2,8-diaza-spiro[4.5]dec-2-en-8-carbonsäure-(2-trifluormethyl-phenyl)-amid
[87] 3-p-Tolyl-1-oxa-2,8-diaza-spiro[4.5]dec-2-en-8-carbonsäure-(2-trifluormethylphenyl)-amid
[88] 3-(4-Trifluormethyl-phenyl)-1-oxa-2,8-diaza-spiro[4.5]dec-2-en-8-carbonsäure-(2-trifluormethyl-phenyl)-amid
[89] 3-(3-Methoxy-phenyl)-1-oxa-2,8-diaza-spiro[4.5]dec-2-en-8-carbonsäure-(3-trifluormethyl-phenyl)-amid
[90] 3-Phenyl-1-oxa-2,8-diaza-spiro[4.5]dec-2-en-8-carbonsäure-(3-trifluormethylphenyl)-amid
[91] 3-(4-Chlor-phenyl)-1-oxa-2,8-diaza-spiro[4.5]dec-2-en-8-carbonsäure-(3-trifluormethyl-phenyl)-amid
[92] 3-(4-tert-Butyl-phenyl)-1-oxa-2,8-diaza-spiro[4.5]dec-2-en-8-carbonsäure-(3-trifluormethyl-phenyl)-amid
[93] 3-p-Tolyl-1-oxa-2,8-diaza-spiro[4.5]dec-2-en-8-carbonsäure-(3-trifluormethylphenyl)-amid
[94] 3-(4-Trifluormethyl-phenyl)-1-oxa-2,8-diaza-spiro[4.5]dec-2-en-8-carbonsäure-(3-trifluormethyl-phenyl)-amid
[95] 3-Phenyl-1-oxa-2,8-diaza-spiro[4.5]dec-2-en-8-carbonsäurebiphenyl-4-ylamid
[96] 3-(4-tert-Butyl-phenyl)-1-oxa-2,8-diaza-spiro[4.5]dec-2-en-8-carbonsäurebiphenyl-4-ylamid
[97] 3-p-Tolyl-1-oxa-2,8-diaza-spiro[4.5]dec-2-en-8-carbonsäurebiphenyl-4-ylamid
[98] 3-(3-Methoxy-phenyl)-1-oxa-2,8-diaza-spiro[4.5]dec-2-en-8-carbonsäure-(2-phenoxy-phenyl)-amid
[99] 3-Phenyl-1-oxa-2,8-diaza-spiro[4.5]dec-2-en-8-carbonsäure-(2-phenoxy-phenyl)-amid
[100] 3-(4-Chlor-phenyl)-1-oxa-2,8-diaza-spiro[4.5]dec-2-en-8-carbonsäure-(2-phenoxy-phenyl)-amid
[101] 3-(4-tert-Butyl-phenyl)-1-oxa-2,8-diaza-spiro[4.5]dec-2-en-8-carbonsäure-(2-phenoxy-phenyl)-amid
[102] 3-p-Tolyl-1-oxa-2,8-diaza-spiro[4.5]dec-2-en-8-carbonsäure-(2-phenoxy-phenyl)-amid
[103] 3-(4-Trifluormethyl-phenyl)-1-oxa-2,8-diaza-spiro[4.5]dec-2-en-8-carbonsäure-(2-phenoxy-phenyl)-amid
[104] 3-(3-Methoxy-phenyl)-1-oxa-2,8-diaza-spiro[4.5]dec-2-en-8-carbonsäure-(4-benzyloxy-phenyl)-amid
[105] 3-Phenyl-1-oxa-2,8-diaza-spiro[4.5]dec-2-en-8-carbonsäure-(4-benzyloxy-phenyl)-amid
[106] 3-(4-Chlor-phenyl)-1-oxa-2,8-diaza-spiro[4.5]dec-2-en-8-carbonsäure-(4-benzyloxy-phenyl)-amid
[107] 3-(4-tert-Butyl-phenyl)-1-oxa-2,8-diaza-spiro[4.5]dec-2-en-8-carbonsäure-(4-benzyloxy-phenyl)-amid
[108] 3-p-Tolyl-1-oxa-2,8-diaza-spiro[4.5]dec-2-en-8-carbonsäure-(4-benzyloxy-phenyl)-amid
[109] 3-(4-Trifluormethyl-phenyl)-1-oxa-2,8-diaza-spiro[4.5]dec-2-en-8-carbonsäure-(4-benzyloxy-phenyl)-amid
[110] 3-(3-Methoxy-phenyl)-1-oxa-2,8-diaza-spiro[4.5]dec-2-en-8-carbonsäurecyclohexylamid
[111] 3-Phenyl-1-oxa-2,8-diaza-spiro[4.5]dec-2-en-8-carbonsäurecyclohexylamid
[112] 3-(4-Chlor-phenyl)-1-oxa-2,8-diaza-spiro[4.5]dec-2-en-8-carbonsäurecyclohexylamid
[113] 3-(4-tert-Butyl-phenyl)-1-oxa-2,8-diaza-spiro[4.5]dec-2-en-8-carbonsäurecyclohexylamid
[114] 3-p-Tolyl-1-oxa-2,8-diaza-spiro[4.5]dec-2-en-8-carbonsäurecyclohexylamid
[115] 3-(4-Trifluormethyl-phenyl)-1-oxa-2,8-diaza-spiro[4.5]dec-2-en-8-carbonsäurecyclohexylamid
[116] 3-(3-Methoxy-phenyl)-1-oxa-2,8-diaza-spiro[4.5]dec-2-en-8-carbonsäurebenzylamid
[117] 3-Phenyl-1-oxa-2,8-diaza-spiro[4.5]dec-2-en-8-carbonsäurebenzylamid
[118] 3-(4-Chlor-phenyl)-1-oxa-2,8-diaza-spiro[4.5]dec-2-en-8-carbonsäurebenzylamid
[119] 3-(4-tert-Butyl-phenyl)-1-oxa-2,8-diaza-spiro[4.5]dec-2-en-8-carbonsäurebenzylamid
[120] 3-p-Tolyl-1-oxa-2,8-diaza-spiro[4.5]dec-2-en-8-carbonsäurebenzylamid
[121] 3-(4-Trifluormethyl-phenyl)-1-oxa-2,8-diaza-spiro[4.5]dec-2-en-8-carbonsäurebenzylamid
[122] 3-(3-Methoxy-phenyl)-1-oxa-2,8-diaza-spiro[4.5]dec-2-en-8-carbonsäurephenethyl-amid
[123] 3-Phenyl-1-oxa-2,8-diaza-spiro[4.5]dec-2-en-8-carbonsäurephenethyl-amid
[124] 3-(4-Chlor-phenyl)-1-oxa-2,8-diaza-spiro[4.5]dec-2-en-8-carbonsäurephenethyl-amid
[125] 3-(4-tert-Butyl-phenyl)-1-oxa-2,8-diaza-spiro[4.5]dec-2-en-8-carbonsäurephenethyl-amid
[126] 3-p-Tolyl-1-oxa-2,8-diaza-spiro[4.5]dec-2-en-8-carbonsäurephenethyl-amid
[127] 3-(4-Trifluormethyl-phenyl)-1-oxa-2,8-diaza-spiro[4.5]dec-2-en-8-carbonsäurephenethyl-amid
[128] 3-(3-Methoxy-phenyl)-1-oxa-2,8-diaza-spiro[4.5]dec-2-en-8-carbonsäure-4-methyl-benzylamid
[129] 3-Phenyl-1-oxa-2,8-diaza-spiro[4.5]dec-2-en-8-carbonsäure-4-methyl-benzylamid
[130] 3-(4-Chlor-phenyl)-1-oxa-2,8-diaza-spiro[4.5]dec-2-en-8-carbonsäure-4-methyl-benzylamid
[131] 3-(4-tert-Butyl-phenyl)-1-oxa-2,8-diaza-spiro[4.5]dec-2-en-8-carbonsäure-4-methyl-benzylamid
[132] 3-p-Tolyl-1-oxa-2,8-diaza-spiro[4.5]dec-2-en-8-carbonsäure-4-methyl-benzylamid
[133] 3-(4-Trifluormethyl-phenyl)-1-oxa-2,8-diaza-spiro[4.5]dec-2-en-8-carbonsäure-4-methyl-benzylamid
[134] 3-(3-Methoxy-phenyl)-1-oxa-2,8-diaza-spiro[4.5]dec-2-en-8-carbonsäure-4-methoxy-benzylamid
[135] 3-Phenyl-1-oxa-2,8-diaza-spiro[4.5]dec-2-en-8-carbonsäure-4-methoxy-benzylamid.
[136] 3-(4-Chlor-phenyl)-1-oxa-2,8-diaza-spiro[4.5]dec-2-en-8-carbonsäure-4-methoxy-benzylamid
[137] 3-(4-tert-Butyl-phenyl)-1-oxa-2,8-diaza-spiro[4.5]dec-2-en-8-carbonsäure-4-methoxy-benzylamid
[138] 3-p-Tolyl-1-oxa-2,8-diaza-spiro[4.5]dec-2-en-8-carbonsäure-4-methoxy-benzylamid
[139] 3-(4-Trifluormethyl-phenyl)-1-oxa-2,8-diaza-spiro[4.5]dec-2-en-8-carbonsäure-4-methoxy-benzylamid
[140] 3-(3-Methoxy-phenyl)-1-oxa-2,8-diaza-spiro[4.5]dec-2-en-8-carbonsäure-(4-tert-butyl-phenyl)-amid
[141] 3-Phenyl-1-oxa-2,8-diaza-spiro[4.5]dec-2-en-8-carbonsäure-(4-tert-butyl-phenyl)-amid
[142] 3-(4-Chlor-phenyl)-1-oxa-2,8-diaza-spiro[4.5]dec-2-en-8-carbonsäure-(4-tert-butyl-phenyl)-amid
[143] 3-(4-tert-Butyl-phenyl)-1-oxa-2,8-diaza-spiro[4.5]dec-2-en-8-carbonsäure-(4-tert-butyl-phenyl)-amid
[144] 3-p-Tolyl-1-oxa-2,8-diaza-spiro[4.5]dec-2-en-8-carbonsäure-(4-tert-butylphenyl)-amid
[145] 3-(4-Trifluormethyl-phenyl)-1-oxa-2,8-diaza-spiro[4.5]dec-2-en-8-carbonsäure-(4-tert-butyl-phenyl)-amid
[146] 3-(3-Methoxy-phenyl)-1-oxa-2,8-diaza-spiro[4.5]dec-2-en-8-carbonsäure-p-tolylamid
[147] 3-Phenyl-1-oxa-2,8-diaza-spiro[4.5]dec-2-en-8-carbonsäure-p-tolylamid
[148] 3-(4-Chlor-phenyl)-1-oxa-2,8-diaza-spiro[4.5]dec-2-en-8-carbonsäure-p-tolylamid
[149] 3-(4-tert-Butyl-phenyl)-1-oxa-2,8-diaza-spiro[4.5]dec-2-en-8-carbonsäure-p-tolylamid
[150] 3-(3-Methoxy-phenyl)-1-oxa-2,8-diaza-spiro[4.5]dec-2-en-8-carbonsäure-o-tolylamid
[151] 3-Phenyl-1-oxa-2,8-diaza-spiro[4.5]dec-2-en-8-carbonsäure-o-tolylamid
[152] 3-(4-Chlor-phenyl)-1-oxa-2,8-diaza-spiro[4.5]dec-2-en-8-carbonsäure-o-tolylamid
[153] 3-(4-tert-Butyl-phenyl)-1-oxa-2,8-diaza-spiro[4.5]dec-2-en-8-carbonsäure-o-tolylamid
[154] 3-p-Tolyl-1-oxa-2,8-diaza-spiro[4.5]dec-2-en-8-carbonsäure-o-tolylamid
[155] 3-(4-Trifluormethyl-phenyl)-1-oxa-2,8-diaza-spiro[4.5]dec-2-en-8-carbonsäure-o-tolylamid
[156] 3-(3-Methoxy-phenyl)-1-oxa-2,8-diaza-spiro[4.5]dec-2-en-8-carbonsäure-(2-ethyl-phenyl)-amid
[157] 3-Phenyl-1-oxa-2,8-diaza-spiro[4.5]dec-2-en-8-carbonsäure-(2-ethyl-phenyl)-amid
[158] 3-(4-Chlor-phenyl)-1-oxa-2,8-diaza-spiro[4.5]dec-2-en-8-carbonsäure-(2-ethylphenyl)-amid
[159] 3-(4-tert-Butyl-phenyl)-1-oxa-2,8-diaza-spiro[4.5]dec-2-en-8-carbonsäure-(2-ethyl-phenyl)-amid
[160] 3-p-Tolyl-1-oxa-2,8-diaza-spiro[4.5]dec-2-en-8-carbonsäure-(2-ethyl-phenyl)-amid
[161] 3-(4-Trifluormethyl-phenyl)-1-oxa-2,8-diaza-spiro[4.5]dec-2-en-8-carbonsäure-(2-ethyl-phenyl)-amid
[162] 3-(3-Methoxy-phenyl)-1-oxa-2,8-diaza-spiro[4.5]dec-2-en-8-carbonsäure-(4-ethyl-phenyl)-amid
[163] 3-Phenyl-1-oxa-2,8-diaza-spiro[4.5]dec-2-en-8-carbonsäure-(4-ethyl-phenyl)-amid
[164] 3-(4-Chlor-phenyl)-1-oxa-2,8-diaza-spiro[4.5]dec-2-en-8-carbonsäure-(4-ethylphenyl)-amid
[165] 3-(4-tert-Butyl-phenyl)-1-oxa-2,8-diaza-spiro[4.5]dec-2-en-8-carbonsäure-(4-ethyl-phenyl)-amid
[166] 3-p-Tolyl-1-oxa-2,8-diaza-spiro[4.5]dec-2-en-8-carbonsäure-(4-ethyl-phenyl)-amid
[167] 3-(4-Trifluormethyl-phenyl)-1-oxa-2,8-diaza-spiro[4.5]dec-2-en-8-carbonsäure-(4-ethyl-phenyl)-amid
[168] 3-(3-Methoxy-phenyl)-1-oxa-2,8-diaza-spiro[4.5]dec-2-en-8-carbonsäure-(2-fluor-phenyl)-amid
[169] 3-Phenyl-1-oxa-2,8-diaza-spiro[4.5]dec-2-en-8-carbonsäure-(2-fluor-phenyl)-amid
[170] 3-(4-Chlor-phenyl)-1-oxa-2,8-diaza-spiro[4.5]dec-2-en-8-carbonsäure-(2-fluorphenyl)-amid
[171] 3-(4-tert-Butyl-phenyl)-1-oxa-2,8-diaza-spiro[4.5]dec-2-en-8-carbonsäure-(2-fluor-phenyl)-amid
[172] 3-p-Tolyl-1-oxa-2,8-diaza-spiro[4.5]dec-2-en-8-carbonsäure-(2-fluor-phenyl)-amid
[173] 3-(4-Trifluormethyl-phenyl)-1-oxa-2,8-diaza-spiro[4.5]dec-2-en-8-carbonsäure-(2-fluor-phenyl)-amid
[174] 3-(3-Methoxy-phenyl)-1-oxa-2,8-diaza-spiro[4.5]dec-2-en-8-carbonsäure-(3-fluor-phenyl)-amid
[175] 3-Phenyl-1-oxa-2,8-diaza-spiro[4.5]dec-2-en-8-carbonsäure-(3-fluor-phenyl)-amid
[176] 3-(4-Chlor-phenyl)-1-oxa-2,8-diaza-spiro[4.5]dec-2-en-8-carbonsäure-(3-fluorphenyl)-amid
[177] 3-(4-tert-Butyl-phenyl)-1-oxa-2,8-diaza-spiro[4.5]dec-2-en-8-carbonsäure-(3-fluor-phenyl)-amid
[178] 3-p-Tolyl-1-oxa-2,8-diaza-spiro[4.5]dec-2-en-8-carbonsäure-(3-fluor-phenyl)-amid
[179] 3-(4-Trifluormethyl-phenyl)-1-oxa-2,8-diaza-spiro[4.5]dec-2-en-8-carbonsäure-(3-fluor-phenyl)-amid
[180] 3-(3-Methoxy-phenyl)-1-oxa-2,8-diaza-spiro[4.5]dec-2-en-8-carbonsäure-(2-chlor-phenyl)-amid
[181] 3-Phenyl-1-oxa-2,8-diaza-spiro[4.5]dec-2-en-8-carbonsäure-(2-chlor-phenyl)-amid
[182] 3-(4-Chlor-phenyl)-1-oxa-2,8-diaza-spiro[4.5]dec-2-en-8-carbonsäure-(2-chlorphenyl)-amid
[183] 3-(4-tert-Butyl-phenyl)-1-oxa-2,8-diaza-spiro[4.5]dec-2-en-8-carbonsäure-(2-chlor-phenyl)-amid
[184] 3-p-Tolyl-1-oxa-2,8-diaza-spiro[4.5]dec-2-en-8-carbonsäure-(2-chlor-phenyl)-amid
[185] 3-(4-Trifluormethyl-phenyl)-1-oxa-2,8-diaza-spiro[4.5]dec-2-en-8-carbonsäure-(2-chlor-phenyl)-amid
[186] 3-(3-Methoxy-phenyl)-1-oxa-2,8-diaza-spiro[4.5]dec-2-en-8-carbonsäure-(2-bromo-phenyl)-amid
[187] 3-Phenyl-1-oxa-2,8-diaza-spiro[4.5]dec-2-en-8-carbonsäure-(2-bromo-phenyl)-amid
[188] 3-(4-Chlor-phenyl)-1-oxa-2,8-diaza-spiro[4.5]dec-2-en-8-carbonsäure-(2-bromo-phenyl)-amid
[189] 3-(4-tert-Butyl-phenyl)-1-oxa-2,8-diaza-spiro[4.5]dec-2-en-8-carbonsäure-(2-bromo-phenyl)-amid
[190] 3-p-Tolyl-1-oxa-2,8-diaza-spiro[4.5]dec-2-en-8-carbonsäure-(2-bromo-phenyl)-amid
[191] 3-(4-Trifluormethyl-phenyl)-1-oxa-2,8-diaza-spiro[4.5]dec-2-en-8-carbonsäure-(2-bromo-phenyl)-amid
[192] 3-(3-Methoxy-phenyl)-1-oxa-2,8-diaza-spiro[4.5]dec-2-en-8-carbonsäure-(3-bromo-phenyl)-amid
[193] 3-Phenyl-1-oxa-2,8-diaza-spiro(4.5]dec-2-en-8-carbonsäure-(3-bromo-phenyl)-amid
[194] 3-(4-tert-Butyl-phenyl)-1-oxa-2,8-diaza-spiro[4.5]dec-2-en-8-carbonsäure-(3-bromo-phenyl)-amid
[195] 3-(4-Trifluormethyl-phenyl)-1-oxa-2,8-diaza-spiro[4.5]dec-2-en-8-carbonsäure-(3-bromo-phenyl)-amid
[196] 3-(3-Methoxy-phenyl)-1-oxa-2,8-diaza-spiro[4.5]dec-2-en-8-carbonsäure-(4-bromo-phenyl)-amid
[197] 3-Phenyl-1-oxa-2,8-diaza-spiro[4.5]dec-2-en-8-carbonsäure-(4-bromo-phenyl)-amid
[198] 3-(3-Methoxy-phenyl)-1-oxa-2,8-diaza-spiro[4.5]dec-2-en-8-carbonsäure-(2-methoxy-phenyl)-amid
[199] 3-Phenyl-1-oxa-2,8-diaza-spiro[4.5]dec-2-en-8-carbonsäure-(2-methoxyphenyl)-amid
[200] 3-(4-Chlor-phenyl)-1-oxa-2,8-diaza-spiro[4.5]dec-2-en-8-carbonsäure-(2-methoxy-phenyl)-amid
[201] 3-(4-tert-Butyl-phenyl)-1-oxa-2,8-diaza-spiro[4.5]dec-2-en-8-carbonsäure-(2-methoxy-phenyl)-amid
[202] 3-p-Tolyl-1-oxa-2,8-diaza-spiro[4.5]dec-2-en-8-carbonsäure-(2-methoxyphenyl)-amid
[203] 3-(4-Trifluormethyl-phenyl)-1-oxa-2,8-diaza-spiro[4.5]dec-2-en-8-carbonsäure-(2-methoxy-phenyl)-amid
[204] 3-(3-Methoxy-phenyl)-1-oxa-2,8-diaza-spiro[4.5]dec-2-en-8-carbonsäure-(3-methoxy-phenyl)-amid
[205] 3-Phenyl-1-oxa-2,8-diaza-spiro[4.5]dec-2-en-8-carbonsäure-(3-methoxy-phenyl)-amid
[206] 3-(4-Chlor-phenyl)-1-oxa-2,8-diaza-spiro[4.5]dec-2-en-8-carbonsäure-(3-methoxy-phenyl)-amid
[207] 3-(4-tert-Butyl-phenyl)-1-oxa-2,8-diaza-spiro[4.5]dec-2-en-8-carbonsäure-(3-methoxy-phenyl)-amid
[208] 3-p-Tolyl-1-oxa-2,8-diaza-spiro[4.5]dec-2-en-8-carbonsäure-(3-methoxy-phenyl)-amid
[209] 3-(4-Trifluormethyl-phenyl)-1-oxa-2,8-diaza-spiro[4.5]dec-2-en-8-carbonsäure-(3-methoxy-phenyl)-amid
[210] 3-(3-Methoxy-phenyl)-1-oxa-2,8-diaza-spiro[4.5]dec-2-en-8-carbonsäure-(4-trifluormethyl-phenyl)-amid
[211] 3-Phenyl-1-oxa-2,8-diaza-spiro[4.5]dec-2-en-8-carbonsäure-(4-trifluormethyl-phenyl)-amid
[212] 3-(4-Chlor-phenyl)-1-oxa-2,8-diaza-spiro[4.5]dec-2-en-8-carbonsäure-(4-trifluormethyl-phenyl)-amid
[213] 3-(4-tert-Butyl-phenyl)-1-oxa-2,8-diaza-spiro[4.5]dec-2-en-8-carbonsäure-(4-trifluormethyl-phenyl)-amid
[214] 3-p-Tolyl-1-oxa-2,8-diaza-spiro[4.5]dec-2-en-8-carbonsäure-(4-trifluormethylphenyl)-amid
[215] 3-(4-Trifluormethyl-phenyl)-1-oxa-2,8-diaza-spiro[4.5]dec-2-en-8-carbonsäure-(4-trifluormethyl-phenyl)-amid
[216] 3-(3-Methoxy-phenyl)-1-oxa-2,8-diaza-spiro[4.5]dec-2-en-8-carbonsäure-(4-phenoxy-phenyl)-amid
[217] 3-(4-Chlor-phenyl)-1-oxa-2,8-diaza-spiro[4.5]dec-2-en-8-carbonsäure-(4-phenoxy-phenyl)-amid
[218] 3-(4-tert-Butyl-phenyl)-1-oxa-2,8-diaza-spiro[4.5]dec-2-en-8-carbonsäure-(4-phenoxy-phenyl)-amid
[219] 3-p-Tolyl-1-oxa-2,8-diaza-spiro[4.5]dec-2-en-8-carbonsäure-(4-phenoxy-phenyl)-amid
[220] 3-(4-Trifluormethyl-phenyl)-1-oxa-2,8-diaza-spiro[4.5]dec-2-en-8-carbonsäure-(4-phenoxy-phenyl)-amid
[221] 3-(3-Methoxy-phenyl)-1-oxa-2,8-diaza-spiro[4.5]dec-2-en-8-carbonsäure-(2-chlor-5-trifluormethyl-phenyl)-amid
[222] 3-Phenyl-1-oxa-2,8-diaza-spiro[4.5]dec-2-en-8-carbonsäure-(2-chlor-5-trifluormethyl-phenyl)-amid
[223] 3-(4-Chlor-phenyl)-1-oxa-2,8-diaza-spiro[4.5]dec-2-en-8-carbonsäure-(2-chlor-5-trifluormethyl-phenyl)-amid
[224] 3-(4-tert-Butyl-phenyl)-1-oxa-2,8-diaza-spiro[4.5]dec-2-en-8-carbonsäure-(2-chlor-5-trifluormethyl-phenyl)-amid
[225] 3-p-Tolyl-1-oxa-2,8-diaza-spiro[4.5]dec-2-en-8-carbonsäure-(2-chlor-5-trifluormethyl-phenyl)-amid
[226] 3-(4-Trifluormethyl-phenyl)-1-oxa-2,8-diaza-spiro[4.5]dec-2-en-8-carbonsäure-(2-chlor-5-trifluormethyl-phenyl)-amid
[227] 3-(3-Methoxy-phenyl)-1-oxa-2,8-diaza-spiro[4.5]dec-2-en-8-carbonsäure-(4-chlor-2-trifluormethyl-phenyl)-amid
[228] 3-Phenyl-1-oxa-2,8-diaza-spiro[4.5]dec-2-en-8-carbonsäure-(4-chlor-2-trifluormethyl-phenyl)-amid
[229] 3-(4-Chlor-phenyl)-1-oxa-2,8-diaza-spiro[4.5]dec-2-en-8-carbonsäure-(4-chlor-2-trifluormethyl-phenyl)-amid
[230] 3-(4-tert-Butyl-phenyl)-1-oxa-2,8-diaza-spiro[4.5]dec-2-en-8-carbonsäure-(4-chlor-2-trifluormethyl-phenyl)-amid
[231] 3-p-Tolyl-1-oxa-2,8-diaza-spiro[4.5]dec-2-en-8-carbonsäure-(4-chlor-2-trifluormethyl-phenyl)-amid
[232] 3-(3-Methoxy-phenyl)-1-oxa-2,8-diaza-spiro[4.5]dec-2-en-8-carbonsäure-(4-chlor-3-trifluormethyl-phenyl)-amid
[233] 3-Phenyl-1-oxa-2,8-diaza-spiro[4.5]dec-2-en-8-carbonsäure-(4-chlor-3-trifluormethyl-phenyl)-amid
[234] 3-(4-Chlor-phenyl)-1-oxa-2,8-diaza-spiro[4.5]dec-2-en-8-carbonsäure-(4-chlor-3-trifluormethyl-phenyl)-amid
[235] 3-(4-tert-Butyl-phenyl)-1-oxa-2,8-diaza-spiro[4.5]dec-2-en-8-carbonsäure-(4-chlor-3-trifluormethyl-phenyl)-amid
[236] 3-p-Tolyl-1-oxa-2,8-diaza-spiro[4.5]dec-2-en-8-carbonsäure-(4-chlor-3-trifluormethyl-phenyl)-amid
[237] 3-(4-Trifluormethyl-phenyl)-1-oxa-2,8-diaza-spiro[4.5]dec-2-en-8-carbonsäure-(4-chlor-3-trifluormethyl-phenyl)-amid
[238] 3-(3-Methoxy-phenyl)-1-oxa-2,8-diaza-spiro[4.5]dec-2-en-8-carbonsäure-(2-tert-butyl-6-methyl-phenyl)-amid
[239] 3-Phenyl-1-oxa-2,8-diaza-spiro[4.5]dec-2-en-8-carbonsäure-(2-tert-butyl-6-methyl-phenyl)-amid
[240] 3-(4-Chlor-phenyl)-1-oxa-2,8-diaza-spiro[4.5]dec-2-en-8-carbonsäure-(2-tert-butyl-6-methyl-phenyl)-amid
[241] 3-(4-tert-Butyl-phenyl)-1-oxa-2,8-diaza-spiro[4.5]dec-2-en-8-carbonsäure-(2-tert-butyl-6-methyl-phenyl)-amid
[242] 3-p-Tolyl-1-oxa-2,8-diaza-spiro[4.5]dec-2-en-8-carbonsäure-(2-tert-butyl-6-methyl-phenyl)-amid
[243] 3-(4-Trifluormethyl-phenyl)-1-oxa-2,8-diaza-spiro[4.5]dec-2-en-8-carbonsäure-(2-tert-butyl-6-methyl-phenyl)-amid
[244] 3-(3-Methoxy-phenyl)-1-oxa-2,8-diaza-spiro[4.5]dec-2-en-8-carbonsäure-(4-trifluormethoxy-phenyl)-amid
[245] 3-Phenyl-1-oxa-2,8-diaza-spiro[4.5]dec-2-en-8-carbonsäure-(4-trifluormethoxy-phenyl)-amid
[246] 3-(4-Chlor-phenyl)-1-oxa-2,8-diaza-spiro[4.5]dec-2-en-8-carbonsäure-(4-trifluormethoxy-phenyl)-amid
[247] 3-(4-tert-Butyl-phenyl)-1-oxa-2,8-diaza-spiro[4.5]dec-2-en-8-carbonsäure-(4-trifluormethoxy-phenyl)-amid
[248] 3-p-Tolyl-1-oxa-2,8-diaza-spiro[4.5]dec-2-en-8-carbonsäure-(4-trifluormethoxy-phenyl)-amid
[249] 3-(3-Methoxy-phenyl)-1-oxa-2,8-diaza-spiro[4.5]dec-2-en-8-carbonthiosäurecyclohexylamid
[250] 3-Phenyl-1-oxa-2,8-diaza-spiro[4.5]dec-2-en-8-carbonthiosäurecyclohexylamid
[251] 3-(4-Chlor-phenyl)-1-oxa-2,8-diaza-spiro[4.5]dec-2-en-8-carbonthiosäurecyclohexylamid
[252] 3-(4-tert-Butyl-phenyl)-1-oxa-2,8-diaza-spiro[4.5]dec-2-en-8-carbonthiosäurecyclohexylamid
[253] 3-p-Tolyl-1-oxa-2,8-diaza-spiro[4.5]dec-2-en-8-carbonthiosäurecyclohexylamid
[254] 3-(4-Trifluormethyl-phenyl)-1-oxa-2,8-diaza-spiro[4.5]dec-2-en-8-carbonthiosäurecyclohexylamid
[255] 3-(3-Methoxy-phenyl)-1-oxa-2,8-diaza-spiro[4.5]dec-2-en-8-carbonthiosäurephenylamid
[256] 3-Phenyl-1-oxa-2,8-diaza-spiro[4.5]dec-2-en-8-carbonthiosäurephenylamid
[257] 3-(4-Chlor-phenyl)-1-oxa-2,8-diaza-spiro[4.5]dec-2-en-8-carbonthiosäurephenylamid
[258] 3-(4-tert-Butyl-phenyl)-1-oxa-2,8-diaza-spiro[4.5]dec-2-en-8-carbonthiosäurephenylamid
[259] 3-p-Tolyl-1-oxa-2,8-diaza-spiro[4.5]dec-2-en-8-carbonthiosäurephenylamid
[260] 3-(4-Trifluormethyl-phenyl)-1-oxa-2,8-diaza-spiro[4.5]dec-2-en-8-carbonthiosäurephenylamid
[261] 3-(3-Methoxy-phenyl)-1-oxa-2,8-diaza-spiro[4.5]dec-2-en-8-carbonthiosäure-(3-trifluormethyl-phenyl)-amid
[262] 3-Phenyl-1-oxa-2,8-diaza-spiro[4.5]dec-2-en-8-carbonthiosäure-(3-trifluormethyl-phenyl)-amid
[263] 3-(4-Chlor-phenyl)-1-oxa-2,8-diaza-spiro[4.5]dec-2-en-8-carbonthiosäure-(3-trifluormethyl-phenyl)-amid
[264] 3-(4-tert-Butyl-phenyl)-1-oxa-2,8-diaza-spiro[4.5]dec-2-en-8-carbonthiosäure-(3-trifluormethyl-phenyl)-amid
[265] 3-p-Tolyl-1-oxa-2,8-diaza-spiro[4.5]dec-2-en-8-carbonthiosäure-(3-trifluormethyl-phenyl)-amid
[266] 3-(4-Trifluormethyl-phenyl)-1-oxa-2,8-diaza-spiro[4.5]dec-2-en-8-carbonthiosäure-(3-trifluormethyl-phenyl)-amid
[267] 3-(3-Methoxy-phenyl)-1-oxa-2,8-diaza-spiro[4.5]dec-2-en-8-carbonthiosäure-(2-methoxy-phenyl)-amid
[268] 3-Phenyl-1-oxa-2,8-diaza-spiro[4.5]dec-2-en-8-carbonthiosäure-(2-methoxy-phenyl)-amid
[269] 3-(4-Chlor-phenyl)-1-oxa-2,8-diaza-spiro[4.5]dec-2-en-8-carbonthiosäure-(2-methoxy-phenyl)-amid
[270] 3-(4-tert-Butyl-phenyl)-1-oxa-2,8-diaza-spiro[4.5]dec-2-en-8-carbonthiosäure-(2-methoxy-phenyl)-amid
[271] 3-p-Tolyl-1-oxa-2,8-diaza-spiro[4.5]dec-2-en-8-carbonthiosäure-(2-methoxy-phenyl)-amid
[272] 3-(4-Trifluormethyl-phenyl)-1-oxa-2,8-diaza-spiro[4.5]dec-2-en-8-carbonthiosäure-(2-methoxy-phenyl)-amid
[273] 3-(3-Methoxy-phenyl)-1-oxa-2,8-diaza-spiro[4.5]dec-2-en-8-carbonthiosäure-(3-methoxy-phenyl)-amid
[274] 3-Phenyl-1-oxa-2,8-diaza-spiro[4.5]dec-2-en-8-carbonthiosäure-(3-methoxy-phenyl)-amid
[275] 3-(4-Chlor-phenyl)-1-oxa-2,8-diaza-spiro[4.5]dec-2-en-8-carbonthiosäure-(3-methoxy-phenyl)-amid
[276] 3-p-Tolyl-1-oxa-2,8-diaza-spiro[4.5]dec-2-en-8-carbonthiosäure-(3-methoxy-phenyl)-amid
[277] 3-(4-Trifluormethyl-phenyl)-1-oxa-2,8-diaza-spiro[4.5]dec-2-en-8-carbonthiosäure-(3-methoxy-phenyl)-amid
[278] 3-(3-Methoxy-phenyl)-1-oxa-2,8-diaza-spiro[4.5]dec-2-en-8-carbonthiosäure-(4-tert-butyl-phenyl)-amid
[279] 3-Phenyl-1-oxa-2,8-diaza-spiro[4.5]dec-2-en-8-carbonthiosäure-(4-tert-butyl-phenyl)-amid
[280] 3-(4-Chlor-phenyl)-1-oxa-2,8-diaza-spiro[4.5]dec-2-en-8-carbonthiosäure-(4-tert-butyl-phenyl)-amid
[281] 3-(4-tert-Butyl-phenyl)-1-oxa-2,8-diaza-spiro[4.5]dec-2-en-8-carbonthiosäure-(4-tert-butyl-phenyl)-amid
[282] 3-p-Tolyl-1-oxa-2,8-diaza-spiro[4.5]dec-2-en-8-carbonthiosäure-(4-tert-butylphenyl)-amid
[283] 3-(4-Trifluormethyl-phenyl)-1-oxa-2,8-diaza-spiro[4.5]dec-2-en-8-carbonthiosäure-(4-tert-butyl-phenyl)-amid
[284] 3-(3-Methoxy-phenyl)-1-oxa-2,8-diaza-spiro[4.5]dec-2-en-8-carbonsäure-2-methyl-benzylamid
[285] 3-Phenyl-1-oxa-2,8-diaza-spiro[4.5]dec-2-en-8-carbonsäure-2-methyl-benzylamid
[286] 3-(4-Chlor-phenyl)-1-oxa-2,8-diaza-spiro[4.5]dec-2-en-8-carbonsäure-2-methyl-benzylamid
[287] 3-(4-tert-Butyl-phenyl)-1-oxa-2,8-diaza-spiro[4.5]dec-2-en-8-carbonsäure-2-methyl-benzylamid
[288] 3-(4-Chlor-phenyl)-1-oxa-2,8-diaza-spiro[4.5]dec-2-en-8-carbonthiosäurecyclopentylamid
[289] 3-(4-tert-Butyl-phenyl)-1-oxa-2,8-diaza-spiro[4.5]dec-2-en-8-carbonthiosäurecyclopentylamid
[290] 3-p-Tolyl-1-oxa-2,8-diaza-spiro[4.5]dec-2-en-8-carbonthiosäurecyclopentylamid
[291] 3-(4-Trifluormethyl-phenyl)-1-oxa-2,8-diaza-spiro[4.5]dec-2-en-8-carbonthiosäurecyclopentylamid
[292] 3-Phenyl-1-oxa-2,8-diaza-spiro[4.5]dec-2-en-8-carbonthiosäurecyclohexylmethyl-amid
[293] 3-(4-Chlor-phenyl)-1-oxa-2,8-diaza-spiro[4.5]dec-2-en-8-carbonthiosäurecyclohexylmethyl-amid
[294] 3-(4-tert-Butyl-phenyl)-1-oxa-2,8-diaza-spiro[4.5]dec-2-en-8-carbonthiosäurecyclohexylmethyl-amid
[295] 3-p-Tolyl-1-oxa-2,8-diaza-spiro[4.5]dec-2-en-8-carbonthiosäurecyclohexylmethyl-amid
[296] 3-(4-Trifluormethyl-phenyl)-1-oxa-2,8-diaza-spiro[4.5]dec-2-en-8-carbonthiosäurecyclohexylmethyl-amid
[297] 3-(3-Methoxy-phenyl)-1-oxa-2,8-diaza-spiro[4.5]dec-2-en-8-carbonthiosäurecyclooctylamid
[298] 3-Phenyl-1-oxa-2,8-diaza-spiro[4.5]dec-2-en-8-carbonthiosäurecyclooctylamid
[299] 3-(4-Chlor-phenyl)-1-oxa-2,8-diaza-spiro[4.5]dec-2-en-8-carbonthiosäurecyclooctylamid
[300] 3-p-Tolyl-1-oxa-2,8-diaza-spiro[4.5]dec-2-en-8-carbonthiosäurecyclooctylamid
[301] 3-(4-Trifluormethyl-phenyl)-1-oxa-2,8-diaza-spiro[4.5]dec-2-en-8-carbonthiosäurecyclooctylamid
[302] 3-(3-Methoxy-phenyl)-1-oxa-2,8-diaza-spiro[4.5]dec-2-en-8-carbonthiosäure-(2-morpholin-4-yl-ethyl)-amid
[303] 3-tert-Butyl-1-oxa-2,8-diaza-spiro[4.5]dec-2-en-8-carbonsäure-(4-tert-butyl-phenyl)-amid
[304] 3-Phenyl-1-(3-phenyl-1-oxa-2,8-diaza-spiro[4.5]dec-2-en-8-yl)-propinon
[305] 1-(3-tert-Butyl-1-oxa-2,8-diaza-spiro[4.5]dec-2-en-8-yl)-3-phenyl-propinon
[306] 3-Phenyl-1-oxa-2,8-diaza-spiro[4.5]dec-2-en-8-carbonsäure-(4-tert-butyl-phenyl)-methyl-amid
[307] ((4-tert-Butyl-phenyl)-(3-phenyl-1-oxa-2,8-diaza-spiro[4.5]dec-2-en-8-yl)-methanon
[308] ((4-Hydroxy-3-methoxy-phenyl)-(3-phenyl-1-oxa-2,8-diaza-spiro[4.5]dec-2-en-8-yl)-methanon
[309] ((4-lodo-phenyl)-(3-phenyl-1-oxa-2,8-diaza-spiro[4.5]dec-2-en-8-yl)-methanon
[310] 3-tert-Butyl-1-oxa-2,8-diaza-spiro[4.5]dec-2-en-8-carbonsäure-p-tolylamid
[311] 3-(4-Trifluoromethoxy-phenyl)-1-oxa-2,8-diaza-spiro[4.5]dec-2-en-8-carbonsäure-p-tolylamid
[312] 3-(3-Methoxy-phenyl)-1-oxa-2,8-diaza-spiro[4.5]dec-2-en-8-carbonsäure-(4-tert-butyl-phenyl)-amid
[313] 2-(4-tert-Butyl-phenyl)-1-(3-phenyl-1-oxa-2,8-diaza-spiro[4.5]dec-2-en-8-yl)-ethanon
[314] 1-(3-Phenyl-1-oxa-2,8-diaza-spiro[4.5]dec-2-en-8-yl)-3-(4-trifluoromethyl-phenyl)-propenon
[315] 3-(4-Hydroxy-3-methoxy-phenyl)-1-(3-phenyl-1-oxa-2,8-diaza-spiro[4.5]dec-2-en-8-yl)-propenon
[316] ((4-tert-Butyl-phenyl)-[3-(4-tert-butyl-phenyl)-1-oxa-2,8-diaza-spiro[4.5]dec-2-en-8-yl]-methanon
[317] 3-(4-tert-Butyl-phenyl)-1-[3-(4-tert-butyl-phenyl)-1-oxa-2,8-diaza-spiro[4.5]dec-2-en-8-yl]-propenon
[318] 3-(4-tert-Butyl-phenyl)-1-[3-(4-trifluoromethoxy-phenyl)-1-oxa-2,8-diaza-spiro[4.5]dec-2-en-8-yl]-propenon
[319] 3-Naphthalen-2-yl-1-oxa-2,8-diaza-spiro[4.5]dec-2-en-8-carbonsäure-(4-ethyl-phenyl)-amid
[320] 1-(3-Phenyl-1-oxa-2,8-diaza-spiro[4.5]dec-2-en-8-yl)-3,3-di-p-tolyl-propenon
[321] 3-(4-tert-Butyl-phenyl)-2-methyl-1-(3-phenyl-1-oxa-2,8-diaza-spiro[4.5]dec-2-en-8-yl)-propenon
[322] 3-Phenyl-1-oxa-2,8-diaza-spiro[4.5]dec-2-en-8-carbonsäurebenzhydryl-amid
[323] 3-(4-tert-Butyl-phenyl)-1-(3-phenyl-1-oxa-2,8-diaza-spiro[4.5]dec-2-en-8-yl)-propenon
[324] 2-(4-tert-Butyl-benzylidene)-1-(3-phenyl-1-oxa-2,8-diaza-spiro[4.5]dec-2-en-8-yl)-buten-1-on
[325] 3-(4-Isopropyl-phenyl)-1-(3-phenyl-1-oxa-2,8-diaza-spiro[4.5]dec-2-en-8-yl)-propenon
[326] 3-(4-Octyl-phenyl)-1-(3-phenyl-1-oxa-2,8-diaza-spiro[4.5]dec-2-en-8-yl)-propenon
[327] 3-(4-Butyl-phenyl)-1-(3-phenyl-1-oxa-2,8-diaza-spiro[4.5]dec-2-en-8-yl)-propenon
[328] 3-(4-Pentyl-phenyl)-1-(3-phenyl-1-oxa-2,8-diaza-spiro[4.5]dec-2-en-8-yl)-propenon
[329] 3-(3-Chloro-pyridin-2-yl)-1-oxa-2,8-diaza-spiro[4.5]dec-2-en-8-carbonsäure-(4-tert-butyl-phenyl)-amid
[330] 3-Phenyl-1-oxa-2,8-diaza-spiro[4.5]dec-2-en-8-carbonthiosäure-(4-pentafluorsulfanyl-phenyl)-amid
[333] N-(4-tert-Butylphenyl)-3-(3-chlorphenyl)-1-oxa-2,8-diazaspiro[4.5]dec-2-en-8-carboxamid
[334] ((E)-3-(4-Cyclohexylphenyl)-1-(3-(3-methoxyphenyl)-1-oxa-2,8-diazaspiro[4.5]dec-2-en-8-yl)prop-2-en-1-on
[335] N-(4-tert-Butylphenyl)-3-(4-cyclohexylphenyl)-1-oxa-2,8-diazaspiro[4.5]dec-2-en-8-carboxamid
[336] ((E)-3-(4-tert-Butylphenyl)-3-phenyl-1-(3-phenyl-1-oxa-2,8-diazaspiro[4.5]dec-2-en-8-yl)prop-2-en-1-on
[337] ((2E,4E)-3-(4-tert-Butylphenyl)-5-phenyl-1-(3-phenyl-1-oxa-2,8-diazaspiro[4.5]dec-2-en-8-yl)penta-2,4-dien-1-on
[338] ((Z)-3-(4-tert-Butylphenyl)-1-(3-phenyl-1-oxa-2,8-diazaspiro[4.5]dec-2-en-8-yl)-3-(thiophen-3-yl)prop-2-en-1-on
[339] ((E)-3-(4-tert-Butylphenyl)-1-(3-(3-methoxyphenyl)-1-oxa-2,8-diazaspiro[4.5]dec-2-en-8-yl)-3-(4-(trifluormethyl)phenyl)prop-2-en-1-on
[340] ((E)-N-(5-(8-(3-(4-tert-Butylphenyl)acryloyl)-1-oxa-2,8-diazaspiro[4.5]dec-2-en-3-yl)-2-fluorphenyl)methanesulfonamid
[341] ((E)-3-(4-Pentafluorsulfanyl)-1-(3-(3-methoxyphenyl)-1-oxa-2,8-diazaspiro[4.5]dec-2-en-8-yl)prop-2-en-1-on
[342] ((E)-1-(3-(3-Methoxyphenyl)-1-oxa-2,8-diazaspiro[4.5]dec-2-en-8-yl)-3-(2-morpholino-6-(trifluormethyl)pyridin-3-yl)prop-2-en-1-on
[343] N-(2-Fluor-4-(1-oxo-1-(3-phenyl-1-oxa-2,8-diazaspiro[4.5]dec-2-en-8-yl)propan-2-yl)phenyl)methanesulfonamid
[344] N-(2-Fluor-4-(1-(3-(3-methoxyphenyl)-1-oxa-2,8-diazaspiro[4.5]dec-2-en-8-yl)-1-oxopropan-2-yl)phenyl)methanesulfonamid
[345] ((Z)-3-(4-tert-Butylphenyl)-1-(3-(3-methoxyphenyl)-1-oxa-2,8-diazaspiro[4.5]dec-2-en-8-yl)-3-(thiophen-3-yl)prop-2-en-1-on
[346] ((E)-3-(3-Bromphenyl)-1-(3-phenyl-1-oxa-2,8-diazaspiro[4.5]dec-2-en-8-yl)prop-2-en-1-on
[347] ((E)-3-(2-Bromphenyl)-1-(3-phenyl-1-oxa-2,8-diazaspiro[4.5]dec-2-en-8-yl)prop-2-en-1-on
[348] 3-(3-Fluorphenyl)-N-(4-(trifluormethyl)phenyl)-1-oxa-2,8-diazaspiro[4.5]dec-2-en-8-carboxamid
[349] N-(4-tert-Butylphenyl)-3-(4-fluorphenyl)-1-oxa-2,8-diazaspiro[4.5]dec-2-en-8-carboxamid
[350] 3-(4-tert-Butylphenyl)-1-(3-phenyl-1-oxa-2,8-diazaspiro[4.5]dec-2-en-8-yl)propan-1-on
[351] N-(4-tert-Butylphenyl)-3-(3-fluorphenyl)-1-oxa-2,8-diazaspiro[4.5]dec-2-en-8-carboxamid
[352] N-(4-tert-Butylphenyl)-3-(2-fluorphenyl)-1-oxa-2,8-diazaspiro[4.5]dec-2-en-8-carboxamid
[353] ((E)-3-(4-tert-Butylphenyl)-1-(3-(3-fluorphenyl)-1-oxa-2,8-diazaspiro[4.5]dec-2-en-8-yl)prop-2-en-1-on
[354] ((Z)-1-(3-(3-Methoxyphenyl)-1-oxa-2,8-diazaspiro[4.5]dec-2-en-8-yl)-4,4-dimethyl-3-phenylpent-2-en-1-on
[355] ((E)-1-(3-Phenyl-1-oxa-2,8-diazaspiro[4.5]dec-2-en-8-yl)-3-(2-(piperidin-1-yl)-6-(trifluormethyl)pyridin-3-yl)prop-2-en-1-on
[356] ((E)-1-(3-(3-Chlorphenyl)-1-oxa-2,8-diazaspiro[4.5]dec-2-en-8-yl)-3-(2-(piperidin-1-yl)-6-(trifluormethyl)pyridin-3-yl)prop-2-en-1-on
[357] ((2E,4E)-3-tert-Butyl-1-(3-(3-methoxyphenyl)-1-oxa-2,8-diazaspiro[4.5]dec-2-en-8-yl)-5-phenylpenta-2,4-dien-1-on
[358] ((2E,4E)-3-(4-tert-Butylphenyl)-1-(3-(3-fluorphenyl)-1-oxa-2,8-diazaspiro[4.5]dec-2-en-8-yl)-5-phenylpenta-2,4-dien-1-on
[359] ((Z)-3-(4-tert-Butylphenyl)-3-(3,4-dichlorphenyl)-1-(3-(2-fluorphenyl)-1-oxa-2,8-diazaspiro[4.5]dec-2-en-8-yl)prop-2-en-1-on
[360] ((Z)-3-(4-tert-Butylphenyl)-3-(3,4-dichlorphenyl)-1-(3-(3-fluorphenyl)-1-oxa-2,8-diazaspiro[4.5]dec-2-en-8-yl)prop-2-en-1-on
[361] 1-(3-(3-Methoxyphenyl)-1-oxa-2,8-diazaspiro[4.5]dec-2-en-8-yl)-3-(4-(trifluormethyl)phenyl)prop-2-yn-1-on
[362] ((E)-1-(3-(3-Methoxyphenyl)-1-oxa-2,8-diazaspiro[4.5]dec-2-en-8-yl)-3-(6-(trifluormethyl)pyridin-3-yl)prop-2-en-1-on
[363] 1-(3-Phenyl-1-oxa-2,8-diazaspiro[4.5]dec-2-en-8-yl)-3-(4-(trifluormethyl)phenyl)prop-2-yn-1-on
[364] N-(4-tert-Butylphenyl)-3-(2-fluorphenyl)-6-methyl-1-oxa-2,8-diazaspiro[4.5]dec-2-en-8-carboxamid
[365] ((E)-1-(3-(3-Fluorphenyl)-1-oxa-2,8-diazaspiro[4.5]dec-2-en-8-yl)-3-(2-(piperidin-1-yl)-6-(trifluormethyl)pyridin-3-yl)prop-2-en-1-on
[366] N-(2-Fluor-4-(1-(3-(3-fluorphenyl)-1-oxa-2,8-diazaspiro[4.5]dec-2-en-8-yl)-1-oxopropan-2-yl)phenyl)methanesulfonamid
[367] ((E)-1-(6-Methyl-3-phenyl-1-oxa-2,8-diazaspiro[4.5]dec-2-en-8-yl)-3-(2-(piperidin-1-yl)-6-(trifluormethyl)pyridin-3-yl)prop-2-en-1-on
[368] ((E)-3-(2-Brom-4-tert-butylphenyl)-1-(3-phenyl-1-oxa-2,8-diazaspiro[4.5]dec-2-en-8-yl)prop-2-en-1-on
[369] N-(4-tert-Butylphenyl)-6-methyl-3-phenyl-1-oxa-2,8-diazaspiro[4.5]dec-2-en-8-carboxamid
[370] 2-(4-tert-Butylphenyl)-1-(3-phenyl-1-oxa-2,8-diazaspiro[4.5]dec-2-en-8-yl)propan-1-on
[371] N-(4-tert-Butylphenyl)-3-phenyl-1-oxa-2,7-diazaspiro[4.4]non-2-en-7-carboxamid
[372] N-(4-tert-Butylbenzyl)-3-phenyl-1-oxa-2,8-diazaspiro[4.5]dec-2-en-8-carboxamid
[373] N-(4-tert-Butylphenyl)-3-phenyl-1-oxa-2,8-diazaspiro[4.6]undec-2-en-8-carboxamid
[374] N-(4-tert-Butylbenzyl)-3-(3-methoxyphenyl)-1-oxa-2,8-diazaspiro[4.5]dec-2-en-8-carboxamid
[375] N-(1-(4-tert-Butylphenyl)ethyl)-3-(3-methoxyphenyl)-1-oxa-2,8-diazaspiro[4.5]dec-2-en-8-carboxamid
[376] N-(4-tert-Butylcyclohexyl)-3-phenyl-1-oxa-2,8-diazaspiro[4.5]dec-2-en-8-carboxamid
[377] N-(4-tert-Butylphenethyl)-3-phenyl-1-oxa-2,8-diazaspiro[4.5]dec-2-en-8-carboxamid
[378] N-(4-tert-Butylphenyl)-3-(4-chlor-3-methoxyphenyl)-1-oxa-2,8-diazaspiro[4.5]dec-2-en-8-carboxamid
[379] 8-(5-tert-Butyl-1H-benzo[d]imidazol-2-yl)-3-phenyl-1-oxa-2,8-diazaspiro[4.5]dec-2-en
[380] 3-Phenyl-8-(5-(trifluormethyl)-1H-benzo[d]imidazol-2-yl)-1-oxa-2,8-diazaspiro[4.5]dec-2-en
[381] ((E)-N-(2-Fluor-4-(3-oxo-3-(3-phenyl-1-oxa-2,8-diazaspiro[4.5]dec-2-en-8-yl)prop-1-enyl)phenyl)methanesulfonamid
[382] ((E)-N-(2-Fluor-4-(3-(3-(3-methoxyphenyl)-1-oxa-2,8-diazaspiro[4.5]dec-2-en-8-yl)-3-oxoprop-1-enyl)phenyl)methanesulfonamid
[383] 5-tert-Butyl-2-(3-phenyl-1-oxa-2,8-diazaspiro[4.5]dec-2-en-8-yl)benzo[d]oxazol
[384] 5-tert-Butyl-2-(3-(3-methoxyphenyl)-1-oxa-2,8-diazaspiro[4.5]dec-2-en-8-yl)benzo[d]oxazol
[385] 6-Methyl-3-phenyl-N-(4-(trifluormethyl)phenyl)-1-oxa-2,8-diazaspiro[4.5]dec-2-en-8-carboxamid
[386] 3-Benzyl-N-(4-tert-butylphenyl)-1-oxa-2,8-diazaspiro[4.5]dec-2-en-8-carboxamid
[387] N-(4-tert-Butylphenyl)-3-phenethyl-1-oxa-2,8-diazaspiro[4.5]dec-2-en-8-carboxamid
[388] 3-(4-Hydroxy-3-methoxy-phenyl)-1-(3-phenyl-1-oxa-2,8-diaza-spiro[4.5]decen-8-yl)-propenon
[389] 3-(4-tert-Butyl-phenyl)-1-(3-phenyl-1-oxa-2,8-diaza-spiro[4.5]decen-8-yl)-propenon
[390] 3-(3-Chlor-pyridin-2-yl)-1-oxa-2,8-diaza-spiro[4.5]dec-2-en-8-carbonsäure-(4-tert-butyl-phenyl)-amid
[391] 3,3-Di-p-tolyl-1-(3-p-tolyl-1-oxa-2,8-diaza-spiro[4.5]dec-2-en-8-yl)-propenon
[392] 3-(4-tert-Butyl-phenyl)-1-[3-(4-chlor-phenyl)1-oxa-2,8-diaza-spiro[4.5]dec-2-en-8-yl]-propenon
[393] 3-(4-tert-Butyl-phenyl)-2-ethyl-1-(3-p-tolyl-1-oxa-2,8-diaza-spiro[4.5]dec-2-en-8-yl)-propenon
[394] ((4-tert-Butyl-phenyl)-(3-p-tolyl-1-oxa-2,8-diaza-spiro[4.5]dec-2-en-8-yl)-methanon
[395] 3-(4-tert-Butyl-phenyl)-1-(3-p-tolyl-1-oxa-2,8-diaza-spiro[4.5]dec-2-en-8-yl)-propenon
[396] 3-(4-tert-Butyl-phenyl)-2-methyl-1-(3-p-tolyl-1-oxa-2,8-diaza-spiro[4.5]dec-2-en-8-yl)-propenon
[397] 2-(4-tert-Butyl-phenyl)-1-[3-(3-methoxy-phenyl)-1-oxa-2,8-diaza-spiro[4.5]dec-2-en-8-yl]-but-2-en-1-on
[398] 3-(4-tert-Butyl-phenyl)-1-[3-(3-methoxy-phenyl)-1-oxa-2,8-diaza-spiro[4.5]dec-2-en-8-yl]-propinon
[399] 3-(4-tert-Butyl-phenyl)-1-[3-(3-methoxy-phenyl)-1-oxa-2,8-diaza-spiro[4.5]dec-2-en-8-yl]-propenon
[400] 1-[3-(3-Methoxy-phenyl)-1-oxa-2,8-diaza-spiro[4.5]dec-2-en-8-yl]-2,2-diphenyl-propan-1-on
[401] 1-[3-(3-Methoxy-phenyl)-1-oxa-2,8-diaza-spiro[4.5]dec-2-en-8-yl]-2,2-diphenyl-ethanon
[402] 3-(3-Methoxy-phenyl)-1-oxa-2,8-diaza-spiro[4.5]dec-2-en-8-carbonsäure-(2,3)-dihydrobenzo[1.4]dioxin-6-yl)-amid
[403] 3-(6-tert-Butyl-pyridin-3-yl)-1-[3-(3-methoxy-phenyl)-1-oxa-2,8-diaza-spiro[4.5]dec-2-en-8-yl]-propenon
[404] 3-(6-tert-Butyl-pyridin-3-yl)-1-[3-(4-chlor-3-methoxy-phenyl)-1-oxa-2,8-diaza-spiro[4.5]dec-2-en-8-yl]-propenon
[405] 2,2-Diphenyl-1-(3-phenyl-1-oxa-2,8-diaza-spiro[4.5]dec-2-en-8-yl)-propan-1-on
[406] 3-(6-tert-Butyl-pyridin-3-yl)-1-(3-phenyl-1-oxa-2,8-diaza-spiro[4.5]dec-2-en-8-yl)-propenon
[407] 3-(4-Isopropyl-phenyl)-1-oxa-2,8-diaza-spiro[4.5]dec-2-en-8-carbonsäure-(4-tert-butyl-phenyl)-amid
[408] 3-(2,3-Dihydro-benzo[1,4]dioxin-6-yl)-1-oxa-2,8-diaza-spiro[4.5]dec-2-en-8-carbonsäure-(4-tert-butyl-phenyl)amid
[409] N-[2-Fluor-4-[3-(3-methoxy-phenyl)-1-oxa-2,8-diaza-spiro[4.5][dec-2-en-8-carbonyl]-phenyl]-methansulfonamid
[410] N-[2-Fluor-4-(3-phenyl-1-oxa-2,8-diaza-spiro[4.5][dec-2-en-8-carbonyl]-phenyl]-methansulfonamid
[411] 3-(4-Cyclohexyl-phenyl)-1-(3-phenyl-1-oxa-2,8-diaza-spiro[4.5]dec-2-en-8-yl)-propenon
[412] 1-[3-(3-Methoxy-phenyl)-1-oxa-2,8-diaza-spiro[4.5]dec-2-en-8-yl]-2,2,2-triphenyl-ethanon
[415] [3-(4-Chlor-3-methoxy-phenyl)-1-oxa-2,8-diaza-spiro[4.5]dec-2-en-8-yl]-(2-chlor-6-trifluormethyl-pyridin-3-yl)-methanon
[416] [3-(3-Methoxy-phenyl)-1-oxa-2,8-diaza-spiro[4.5]dec-2-en-8-yl]-(2-morpholin-4-yl-6-trifluormethyl-pyridin-3-yl)-methanon
[417] 3-(4-tert-Butyl-phenyl)-1-[3-(3-chlor-phenyl)-1-oxa-2,8-diaza-spiro[4.5]dec-2-en-8-yl]-propenon
[418] 3-(4-tert-Butyl-phenyl)-1-[3-(2,3-dihydro-benzo[1.4]dioxin-6-yl)-1-oxa-2,8-diaza-spiro[4.5]dec-2-en-8-yl-propenon
[419] 3-(4-tert-Butyl-phenyl)-1-[3-(4-cyclohexyl-phenyl)-1-oxa-2,8-diaza-spiro[4.5]dec-2-en-8-yl]-propenon
[420] N-[4-[3-(4-Cyclohexyl-phenyl)-1-oxa-2,8-diaza-spiro[4.5]dec-2-en-8-carbonyl]-2-fluor-phenyl]-methansulfonamid
[421] 2-Hydroxy-2,2-diphenyl-1-(3-phenyl-1-oxa-2,8-diaza-spiro[4.5]dec-2-8-yl]-ethanon
[423] 3-(4-tert-Butyl-phenyl)-1-(3-chinolin-3-yl-1-oxa-2,8-diaza-spiro[4.5]dec-2-en-8-yl)-propenon
[424] 3-(2,3-Dihydro-benzo[1.4]dioxin-6-yl)-1-oxa-2,8-diaza-spiro[4.5]dec-2-en-8-carbonsäure-(4-trilfluormethyl-phenyl)-amid
[425] 3-(3-Chlor-phenyl)-1-oxa-2,8-diaza-spiro[4.5]dec-2-en-8-carbonsäure-(4-trilfuormethyl-phenyl)-amid
[426] ((4-tert-Butyl-phenyl)-[3-(3-methoxy-phenyl)-1-oxa-2,8-diaza-spiro[4.5]dec-2-en-8-yl]-methanon
[427] 3-(4-Cyclohexyl-phenyl)-1-oxa-2,8-diaza-sprio[4.5]dec-2-en-8-carbonsäure-(4-trifluormethyl-phenyl)amid
[428] 3-(4-tert-Butyl-phenyl)-3-(4-chlor-phenyl)-1-[3-(3-chloro-phenyl)-1-oxa-2,8-diaza-spiro[4.5]dec-2-en-8-yl]-propenon
[429] 3-(4-tert-Butyl-phenyl)-1-[3-(3-chlor-phenyl)-1-oxa-2,8-diaza-spiro[4.5]dec-2-en-8-yl]-3-(3-methoxy-phenyl)-propenon
[430] 3-(4-tert-Butyl-phenyl)-1-[3-(4-chlor-3-methoxy-phenyl)-1-oxa-2,8-diaza-spiro[4.5]dec-2-en-8-yl]-3-(4-trifluormethyl-phenyl)-propenon
[431] 3-(4-Pentafluorsulfanyl-phenyl)-1-[3-(3-methoxy-phenyl)-1-oxa-2,8-diaza-spiro[4.5]dec-2-en-8-yl]-propenon
[432] 3-(4-tert-Butyl-phenyl)-1-[3-(4-chlor-3-methoxy-phenyl)-1-oxa-2,8-diaza-spiro[4.5]dec-2-en-8-yl]-propenon
[433] 3-(4-tert-Butyl-phenyl)-1-[3-(4-chlor-3-methoxy-phenyl)-1-oxa-2,8-diaza-spiro[4.5]dec-2-en-8-yl]-propinon
[434] 3-(4-tert-Butyl-phenyl)-1-[3-(3-methoxy-phenyl)-1-oxa-2,8-diaza-spiro[4.5]dec-2-en-8-yl]-3-(2-trifluormethyl-phenyl)-propenon
[435] 3-Thiazol-2-yl-1-oxa-2,8-diaza-spiro[4.5]dec-2-en-8-carbonsäure-(4-isopropyl-phenyl)-amid
[436] 3-Thiazol-2-yl-1-oxa-2,8-diaza-spiro[4.5]dec-2-en-8-carbonsäure-(4-trifluormethoxy-phenyl)-amid
[437] 3,3-Bis-(4-tert-butyl-phenyl)-1-(3-phenyl-1-oxa-2,8-diaza-spiro[4.5]dec-2-en-8-yl)-propenon
und
[438] 3-Thiazol-2-yl-1-oxa-2,8-diaza-spiro[4.5]dec-2-en-8-carbonsäure-(4-tert-butyl-phenyl)-amid ;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

23. Verbindungen gemäß einem oder mehreren der Ansprüche 1 bis 22, **dadurch gekennzeichnet, dass** sie im FLIPR-Assay in einer Konzentration von 10 µM eine Hemmung des Ca²⁺-Ionen-Einstroms in Dorsalwurzelganglien von Ratten von wenigstens 30 %, bevorzugt von wenigstens 40 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µM aufweisen.

24. Verfahren zur Herstellung von substituierten Spiro-Verbindungen der allgemeinen Formel I gemäß einem oder mehreren der Ansprüche 1 bis 23,
**dadurch gekennzeichnet, dass** wenigstens eine Verbindung der allgemeinen Formel II, 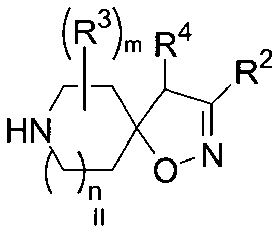 worin R², R³, R⁴, m und n die Bedeutung gemäß einem oder mehreren der Ansprüche 1 bis 23 haben, in einem Reaktionsmedium mit wenigstens einem Isocyanat der allgemeinen Formel R⁵-N=C=O, worin R⁵ die Bedeutung gemäß einem oder mehreren der Ansprüche 1 bis 23 hat, ggf. in Gegenwart wenigstens einer Base, bevorzugt in Gegenwart wenigstens einer Base ausgewählt aus der Gruppe bestehend aus Triethylamin, 4,4-Dimethylaminopyridin, Diisopropylethylamin, Pyridin und N-Methylmorpholin, zu wenigstens einer Verbindung der allgemeinen Formel I umgesetzt wird, worin R², R³, R⁴, m und n die vorstehend genannte Bedeutung haben und R¹ für -C(=O)-NR⁵R⁶ steht, wobei R⁵ die vorstehend genannte Bedeutung hat und R⁶ für einen Wasserstoff-Rest steht, und diese ggf. gereinigt und/oder isoliert wird
oder
wenigstens eine Verbindung der allgemeinen Formel II in einem Reaktionsmedium mit wenigstens einem Isothiocyanat der allgemeinen Formel S=C=N-R⁷, worin R⁷ die Bedeutung gemäß einem oder mehreren der Ansprüche 1 bis 23 hat, ggf. in Gegenwart wenigstens einer Base, bevorzugt in Gegenwart wenigstens einer Base ausgewählt aus der Gruppe bestehend aus Triethylamin, 4,4-Dimethylaminopyridin, Diisopropylethylamin, Pyridin und N-Methylmorpholin, zu wenigstens einer Verbindung der allgemeinen Formel I umgesetzt wird, worin R², R³, R⁴, m und n die vorstehend genannte Bedeutung haben und R¹ für -C(=S)-N-R⁷R⁸ steht, wobei R⁷ die vorstehend genannte Bedeutung hat und R⁸ für einen Wasserstoff-Rest steht, und diese ggf. gereinigt und/oder isoliert wird
und ggf. wenigstens eine Verbindung der allgemeinen Formel I, worin R², R³, R⁴, m und n die vorstehend genannte Bedeutung haben und R¹ für -C(=O)-NR⁵R⁶ oder-C(=S)-N-R⁷R⁸ steht, worin R⁶ und R⁸ jeweils für einen Wasserstoff-Rest stehen, in einem Reaktionsmedium, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R⁶ oder der allgemeinen Formel LG-R⁸, worin LG für eine Abgangsgruppe, bevorzugt für ein Halogen-Atom, besonders bevorzugt für ein Chloratom steht, und R⁶ und R⁸ die vorstehend genannte Bedeutung mit Ausnahme von Wasserstoff haben, zu wenigstens einer Verbindung der allgemeinen Formel I umgesetzt wird, worin R² bis R⁴, m und n die vorstehend genannte Bedeutung haben und R¹ für -C(=O)-NR⁵R⁶ oder -C(=S)-NR⁷R⁸ steht, und diese ggf. gereinigt und/oder isoliert wird,
oder
wenigstens eine Verbindung der allgemeinen Formel II in einem Reaktionsmedium, in Gegenwart wenigstens einer Base, bevorzugt in Gegenwart wenigstens eines Metallhydridsalzes, besonders bevorzugt in Gegenwart von Natrium- und/oder Kaliumhydrid, mit wenigstens einer Verbindung der allgemeinen Formel LG-R¹, worin R¹ die Bedeutung gemäß einem oder mehreren der Ansprüche 1 bis 23 hat, mit Ausnahme von -C(=O)-NR⁵R⁶, -C(=S)-NR⁷R⁸, -C(=O)-R⁹ und -S(=O)-R¹⁰, und LG für eine Abgangsgruppe, bevorzugt für ein Halogen-Atom, besonders bevorzugt für ein Chloratom steht, zu wenigstens einer Verbindung der allgemeinen Formel I umgesetzt wird, worin R¹ bis R⁴, m und n die vorstehend genannte Bedeutung haben, und diese ggf. gereinigt und/oder isoliert wird
oder
wenigstens eine Verbindung der allgemeinen Formel II in einem Reaktionsmedium in Gegenwart wenigstens eines Reduktionsmittels, mit wenigstens einer Verbindung der allgemeinen Formel R¹-C(=O)-H, worin R¹ die Bedeutung gemäß einem oder mehreren der Ansprüche 1 bis 23 hat, mit Ausnahme von -C(=O)-NR⁵R⁶, -C(=S)-NR⁷R⁸, -C(=O)-R⁹ und -S(=O)-R¹⁰, zu wenigstens einer Verbindung der allgemeinen Formel I umgesetzt wird, worin R¹ bis R⁴, m und n die vorstehend genannte Bedeutung haben, und diese ggf. gereinigt und/oder isoliert wird
oder
wenigstens eine Verbindung der allgemeinen Formel II in einem Reaktionsmedium ggf. in Gegenwart wenigstens einer Base mit wenigstens einer Verbindung der allgemeinen Formel R⁹-C(=O)-LG, worin R⁹ die Bedeutung gemäß einem oder mehreren der Ansprüche 1 bis 23 hat und LG für eine Abgangsgruppe, vorzugsweise für einen Halogen-Rest steht, oder in einem Reaktionsmedium in Gegenwart wenigstens eines Kupplungsreagenzes ggf. in Gegenwart wenigstens einer Base mit einer Verbindung der allgemeinen Formel R⁹-C(=O)-OH, worin R⁹ die Bedeutung gemäß einem oder mehreren der Ansprüche 1 bis 23 hat, zu wenigstens einer Verbindung der allgemeinen Formel I umgesetzt wird, worin R² bis R⁴, m und n die vorstehend genannte Bedeutung haben und R¹ für -C(=O)-R⁹ steht, und diese ggf. gereinigt und/oder isoliert wird
oder
wenigstens eine Verbindung der allgemeinen Formel II in einem Reaktionsmedium ggf. in Gegenwart wenigstens einer Base mit wenigstens einer Verbindung der allgemeinen Formel R¹⁰-S(=O)₂-LG, worin R¹⁰ die Bedeutung gemäß einem oder mehreren der Ansprüche 1 bis 23 hat und LG für eine Abgangsgruppe, vorzugsweise für einen Halogen-Rest steht, zu wenigstens einer Verbindung der allgemeinen Formel I umgesetzt wird, worin R² bis R⁴, m und n die vorstehend genannte Bedeutung haben und R¹ für -S(=O)₂-R¹⁰ steht, und diese ggf. gereinigt und/oder isoliert wird.

25. Arzneimittel enthaltend wenigstens eine Verbindung gemäß einem oder mehreren der Ansprüche 1 bis 23 und ggf. einen oder mehrere physiologisch verträgliche Hilfsstoffe.

26. Arzneimittel gemäß Anspruch 25 zur Prophylaxe und/oder Behandlung von Schmerz, vorzugsweise von Schmerz ausgewählt aus der Gruppe bestehend aus akutem Schmerz, chronischem Schmerz und neuropathischem Schmerz.

27. Arzneimittel gemäß Anspruch 25 zur Behandlung und/oder Prophylaxe von einer oder mehreren Erkrankungen ausgewählt aus der Gruppe bestehend aus Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

28. Verwendung wenigstens einer Verbindung gemäß einem oder mehreren der Ansprüche 1 bis 23 zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz und neuropathischem Schmerz.

29. Verwendung wenigstens einer Verbindung gemäß einem oder mehreren der Ansprüche 1 bis 23 zur Herstellung eines Arzneimittels zur Prophylaxe und/oder Behandlung von einer oder mehreren Erkrankungen ausgewählt aus der Gruppe bestehend aus Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Harninkontinenz; einer überaktiven Blase (overactive bladder, OAB); Magengeschwüren; Reizdarmsyndrom; Schlaganfällen; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

30. Verwendung wenigstens einer Verbindung gemäß einem oder mehreren der Ansprüche 1 bis 23 zur Herstellung eines Arzneimittels zur Prophylaxe und/oder Behandlung von einer oder mehreren Erkrankungen ausgewählt aus der Gruppe bestehend aus Atemwegserkrankungen, vorzugsweise ausgewählt aus der Gruppe bestehend aus Asthma und Lungenentzündung; Husten; Augenreizungen; Hautreizungen; neurotischen Hauterkrankungen und Entzündungskrankheiten, vorzugsweise Entzündungen des Darmes.

## Claims

1. Substituted spiro compounds of the general formula I 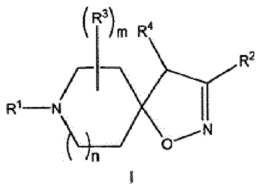 wherein
m equals 0, 1, 2, 3 or 4,
n equals 0, 1 or 2,
R¹ stands for a unsubstituted or at least monosubstituted, unsaturated or saturated cycloaliphatic radical, possibly having at least one heteroatom as ring member, which can be bonded via a linear or branched, unsubstituted or at last monosubstituted alkylene, alkenylene or alkinylene group and/or condensed with an unsubstituted or at least monosubstituted mono- or polycyclic ring system,
for an unsubstituted or at least monosubstituted aryl or heteroaryl radical, which can be bonded via a linear or branched, unsubstituted or at least monosubstituted alkylene, alkenylene or alkinylene group and/or condensed with an unsubstituted or at least monosubstituted mono- or polycyclic ring system,
for a -C(=O)-NR⁵R⁶ group,
a -C(=S)-NR⁷R⁸ group,
a -C(=O)-R⁹ group,
or for an -S(=O)₂-R¹⁰ group;
R² stands for a linear or branched, saturated or unsaturated, unsubstituted or at least monosubstituted aliphatic radical possibly having at least one heteroatom as chain member;
for an unsubstituted or at least monosubstituted, unsaturated or saturated cycloaliphatic radical, possibly having at least one heteroatom as ring member, which can be bonded via a linear or branched, unsubstituted or at least monosubstituted alkylene, alkenylene or alkinylene group, possibly having at least one heteroatom as chain member, and/or can be condensed with an unsubstituted or at least monosubstituted mono- or polycyclic ring system,
for an unsubstituted or at least monosubstituted phenyl radical,
on condition that not one of the meta positions and the para position of this phenyl radical are substituted with substituents, which are respectively bonded to the phenyl radical via the same atom selected from the group consisting of oxygen, sulphur and nitrogen;
for an unsubstituted or at least monosubstituted radical selected from the group consisting of naphthyl, (1, 3)-benzodioxolyl, (1, 4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl, [1, 2, 3, 4]-tetrahydronaphthyl, [1, 2, 3, 4]-tetrahydroquinolinyl, [1, 2, 3,4]-tetrahydroisoquinolinyl, [1, 2, 3, 4]-tetrahydroquinazolinyl; 2H-benzo[1,4]oxazin-3(4H)-onyl, (3, 4)-dihydroquinolin-2(1H)-onyl, [3,4]-dihydro-2H-1,4-benzoxazinyl and benzothiazolyl,
or for an unsubstituted or at least monosubstituted aryl or heteroaryl radical, which can be bonded via a linear or branched, unsubstituted or at least monosubstituted alkylene, alkenylene or alkinylene group, possibly having at least one heteroatom as chain member, and/or can possibly be condensed with an unsubstituted or at least monosubstituted mono- or polycyclic ring system;
R³ stands for a halogen radical, for a nitro group, a hydroxy group, a thiol group; for an -O-R¹¹ group, an -S-R¹² group, or for a linear or branched, saturated or unsaturated, unsubstituted or at least monosubstituted aliphatic radical;
R⁴ stands for a hydrogen radical, for a halogen radical, a nitro group, a hydroxy group, a thiol group; for an oxo group (=0), an -O-R¹¹ group, an -S-R¹² group or for a linear or branched, saturated or unsaturated, unsubstituted or at least monosubstituted aliphatic radical;
R⁵ and R⁷, independently of one another, respectively stand for a linear or branched, saturated or unsaturated, unsubstituted or at least monosubstituted aliphatic radical possibly having at least one heteroatom as chain member;
an unsubstituted or at least monosubstituted, unsaturated or saturated cycloaliphatic radical, possibly having at least one heteroatom as ring member, which can be bonded via a linear or branched, unsubstituted or at least monosubstituted alkylene, alkenylene or alkinylene group, possibly having at least one heteroatom as chain member, and/or can be condensed with an unsubstituted or at least monosubstituted mono- or polycyclic ring system and/or can be bridged with at least one linear or branched, unsubstituted or at least monosubstituted alkylene group,
an unsubstituted or at least monosubstituted aryl or heteroaryl radical, which can be bonded via a linear or branched, unsubstituted or at least monosubstituted alkylene, alkenylene or alkinylene group, possibly having at least one heteroatom as chain member, and/or can possibly be condensed with an unsubstituted or at least monosubstituted mono- or polycyclic ring system,
R⁶ and R⁸, independently of one another, respectively stand for a hydrogen radical,
a linear or branched, saturated or unsaturated, unsubstituted or at least monosubstituted aliphatic radical possibly having at least one heteroatom as chain member;
an unsubstituted or at least monosubstituted, unsaturated or saturated cycloaliphatic radical, possibly having at least one heteroatom as ring member, which can be bonded via a linear or branched, unsubstituted or at least monosubstituted alkylene, alkenylene or alkinylene group, possibly having at least one heteroatom as chain member, and/or can be condensed with an unsubstituted or at least monosubstituted mono- or polycyclic ring system and/or can be bridged with at least one linear or branched, unsubstituted or at least monosubstituted alkylene group,
an unsubstituted or at least monosubstituted aryl or heteroaryl radical, which can be bonded via a linear or branched, unsubstituted or at least monosubstituted alkylene, alkenylene or alkinylene group, possibly having at least one heteroatom as chain member, and/or can possibly be condensed with an unsubstituted or at least monosubstituted mono- or polycyclic ring system,
R⁹ stands for a linear or branched, saturated or unsaturated, unsubstituted or at least monosubstituted aliphatic radical;
or an unsubstituted or at least monosubstituted aryl or heteroaryl radical, which can be bonded via a linear or branched, unsubstituted or at least monosubstituted alkylene, alkenylene or alkinylene group and/or can be condensed with an unsubstituted or at least monosubstituted mono- or polycyclic ring system;
R¹⁰ stands for a linear or branched, saturated or unsaturated, unsubstituted or at least monosubstituted aliphatic radical;
for an unsubstituted or at least monosubstituted, unsaturated or saturated cycloaliphatic radical, possibly having at least one heteroatom as ring member, which can be bonded via a linear or branched, unsubstituted or at least monosubstituted alkylene, alkenylene or alkinylene group and/or can be condensed with an unsubstituted or at least monosubstituted mono- or polycyclic ring system,
or an unsubstituted or at least monosubstituted aryl or heteroaryl radical, which can be bonded via a linear or branched, unsubstituted or at least monosubstituted alkylene, alkenylene or alkinylene group and/or can be condensed with an unsubstituted or at least monosubstituted mono- or polycyclic ring system;
and
R¹¹ and R¹², independently of one another, respectively stand for a linear or branched, saturated or unsaturated, unsubstituted monosubstituted aliphatic radical;
for an unsubstituted, unsaturated or saturated cycloaliphatic radical, possibly having at least one heteroatom as ring member, which can be bonded via a linear or branched, unsubstituted alkylene, alkenylene or alkinylene group and/or can be condensed with an unsubstituted monosubstituted mono- or polycyclic ring system,
or an unsubstituted aryl or heteroaryl radical, which can be bonded via a linear or branched, unsubstituted alkylene, alkenylene or alkinylene group and/or can be condensed with an unsubstituted mono- or polycyclic ring system;
wherein the substituents of the aforementioned aliphatic radicals can be selected independently of one another from the group consisting of F, Cl, Br, I, -CN, -NO₂,-OH, -SH and -NH₂;
in each case optionally in the form of one of the pure stereoisomers thereof, particularly enantiomers or diastereoisomers or the racemates thereof, or in the form of a mixture of stereoisomers, particularly the enantiomers and/or diastereoisomers, in an arbitrary mixing ratio, or respectively in the form of corresponding salts, or respectively in the form of corresponding solvates.

2. Compounds according to claim 1, **characterised in that**
m equals 0, 1, 2, 3 or 4,
n equals 0, 1 or 2,
R¹ stands for an unsaturated or saturated, possibly substituted 3-, 4-, 5-, 6-, 7-, 8- or 9-member cycloaliphatic radical, which can be condensed with a saturated, unsaturated or aromatic, possibly substituted mono- or polycyclic ring system;
for a possibly substituted 5- to 14-member aryl or heteroaryl radical, which can be condensed with saturated or unsaturated, possibly substituted mono- or polycyclic ring system,
for a -C(=O)-NR⁵R⁶ group,
a -C(=S)-NR⁷R⁸ group,
a -C(=O)-R⁹ group,
or for an -S(=O)₂-R¹⁰ group,
or for -(CHR¹³)-(CHR¹⁴)_{f}-(CHR¹⁵)ₕ-R¹⁶ with f = 0 or 1 and h = 0 or 1;
R² stands for a linear or branched, saturated or unsaturated, possibly substituted C₁₋₁₀ aliphatic radical;
for an unsaturated or saturated, possibly substituted 3-, 4-, 5-, 6-, 7-, 8- or 9-member cycloaliphatic radical, which can be condensed with a saturated, unsaturated or aromatic, possibly substituted mono- or polycyclic ring system;
for a phenyl radical, which can be substituted with 1, 2, 3, 4 or 5 substituents selected independently of one another from the group consisting of F, Cl, Br, I, -CN, - CF₃,-SF₅, -OH, -O-C₁₋₅ alkyl, -NH₂, -NO₂, -O-CF₃, -S-CF₃, -SH, -S-C₁₋₅ alkyl, -C₁₋₁₀ alkyl, -C(=O)-OH, -C(=O)-O-C₁₋₅alkyl, -O-C(=O)-C₁₋₅ alkyl, -NH-C₁₋₅ alkyl, -N(C₁₋₅ alkyl)₂, -NH-C(=O)-O-C₁₋₅ alkyl, -C(=O)-H, -C(=O)-C₁₋₅ alkyl, -C(=O)-NH₂, -C(=O)-NH-C₁₋₅ alkyl, C(=O)-N-(C₁₋₅ alkyl)₂, -S(=O)₂-C₁₋₅ alkyl, -S(=O)₂ phenyl, -NH-S(=O)₂-C₁₋₅ alkyl, -NH-S(=O)₂-C₁₋₅ alkylene phenyl, -NH-S(=O)₂-C₁₋₅ alkylene-naphthyl, -NH-S(=O)₂-phenyl, -NH-S(=O)₂-naphthyl, cyclohexyl, cyclopentyl, morpholinyl, piperidinyl, piperazinyl, pyrrolidinyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, morpholinyl, piperidinyl, piperazinyl, pyrrolidinyl, pyridazinyl, -S(=O)₂ phenyl, -O-phenyl, -O-benzyl, phenyl, -(CH₂)-benzo[b]furanyl, -NH-S(=O)₂-C₁₋₅ alkylene phenyl, -NH-S(=O)₂-C₁₋₅ alkylene-naphthyl, -NH-S(=O)₂-phenyl, -NH-S(=O)₂-naphthyl and benzyl can be substituted with 1, 2, 3, 4 or 5 substituents selected independently of one another from the group consisting of F, Cl, Br, -OH, -CF₃, -SF₅, -CN, -NO₂, -C₁₋₅ alkyl, -O-C₁₋₅ alkyl, -O-CF₃, -S-CF₃, phenyl and -O-benzyl,
on condition that not one of the meta positions and the para position of this phenyl radical are substituted with substituents, which are respectively bonded to the phenyl radical via the same atom selected from the group consisting of oxygen, sulphur and nitrogen;
for a possibly substituted radical selected from the group consisting of naphthyl, (1, 3)-benzodioxolyl, (1, 4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl, [1, 2, 3, 4]-tetrahydronaphthyl, [1, 2, 3, 4]-tetrahydroquinolinyl, [1, 2, 3,4]-tetrahydroisoquinolinyl, [1, 2, 3, 4]-tetrahydroquinazolinyl; 2H-benzo[1,4]oxazin-3(4H)-onyl, (3, 4)-dihydroquinolin-2(1H)-onyl, [3,4]-dihydro-2H-1,4-benzoxazinyl and benzothiazolyl,
or for -(CHR¹⁷)-X_{q}-(CHR¹⁸)ᵣ-Yₛ-(CHR¹⁹)ₜ-Zᵤ-R²⁰ with q = 0 or 1, r = 0 or 1, s = 0 or 1, t = 0 or 1, u = 0 or 1, wherein X, Y and Z independently of one another respectively stand for O, S, NH, N(CH₃), N(C₂H₅) or N[CH(CH₃)₂];
R³ stands for a halogen radical, for a nitro group, a hydroxy group, a thiol group; for an -O-R¹¹ group, an -S-R¹² group, or for a linear or branched, saturated or unsaturated, possibly substituted C₁₋₁₀ aliphatic radical;
R⁴ stands for a hydrogen radical, for a halogen radical, a nitro group, a hydroxy group, a thiol group; for an oxo group (=0), an -O-R¹¹ group, an -S-R¹² group or for a linear or branched, saturated or unsaturated, possibly substituted C₁₋₁₀ aliphatic radical;
R⁵ and R⁷, independently of one another, respectively stand for a linear or branched, saturated or unsaturated, possibly substituted C₁₋₂₀ aliphatic radical;
an unsaturated or saturated, possibly substituted 3-, 4-, 5-, 6-, 7-, 8-, 9-, 10-, 11- or 12-member cycloaliphatic radical, which can be condensed with a saturated, unsaturated or aromatic, possibly substituted mono- or polycyclic ring system and/or can be bridged with one or two linear or branched, possibly substituted C₁₋₅ alkylene groups;
a possibly substituted 5- to 14-member aryl or heteroaryl radical, which can be condensed with a saturated or unsaturated, possibly substituted mono- or polycyclic ring system;
or for -(CR²¹R²²)-Xᵥ-(CHR²³)_{w}-Yₓ-(CHR²⁴)_{y}-Z_{z}-R²⁵ with v = 0 or 1, w = 0 or 1, x = 0 or 1, y = 0 or 1, z = 0 or 1, wherein X, Y and Z independently of one another respectively stand for O, S, NH, N(CH₃), N(C₂H₅) or N[CH(CH₃)₂];
R⁶ and R⁸, independently of one another, respectively stand for a hydrogen radical,
a linear or branched, saturated or unsaturated, possibly substituted C₁₋₂₀ aliphatic radical;
an unsaturated or saturated, possibly substituted 3-, 4-, 5-, 6-, 7-, 8-, 9-, 10-, 11- or 12-member cycloaliphatic radical, which can be condensed with a saturated, unsaturated or aromatic, possibly substituted mono- or polycyclic ring system and/or can be bridged with one or two linear or branched, possibly substituted C₁₋₅ alkylene groups;
a possibly substituted 5- to 14-member aryl or heteroaryl radical, which can be condensed with a saturated or unsaturated, possibly substituted mono- or polycyclic ring system;
or for -(CR²¹R²²)-Xᵥ-(CHR²³)_{w}-Yₓ-(CHR²⁴)_{y}-Z_{z}-R²⁵ with v = 0 or 1, w = 0 or 1, x = 0 or 1, y = 0 or 1, z = 0 or 1, wherein X, Y and Z independently of one another respectively stand for O, S, NH, N(CH₃), N(C₂H₅) or N[CH(CH₃)₂];
R⁹ stands for a linear or branched, saturated or unsaturated, possibly substituted C₁₋₁₀ aliphatic radical;
a possibly substituted 5- to 14-member aryl or heteroaryl radical, which can be condensed with a saturated or unsaturated, possibly substituted mono- or polycyclic ring system;
for -(CR²⁶R²⁷)-(CHR²⁸)ₐₐ-(CHR²⁹)_{bb} -R³⁰ with aa = 0 or 1 and bb = 0 or 1;
for -CR³¹=CR³²-R³³
or for -C≡C-R³⁴;
R¹⁰ stands for a linear or branched, saturated or unsaturated, possibly substituted C₁₋₁₀ aliphatic radical;
an unsaturated or saturated, possibly substituted 3-, 4-, 5-, 6-, 7-, 8- or 9-member cycloaliphatic radical, which can be condensed with a saturated, unsaturated or aromatic, possibly substituted mono- or polycyclic ring system;
a possibly substituted 5- to 14-member aryl or heteroaryl radical, which can be condensed with a saturated or unsaturated, possibly substituted mono- or polycyclic ring system;
for -(CR²⁶R²⁷)-(CHR²⁸)ₐₐ-(CHR²⁹)_{bb} -R³⁰ with aa = 0 or 1 and bb = 0 or 1;
for -CR³¹=CR³²-R³³
or for -C=C-R³⁴;
R¹¹ and R¹², independently of one another, respectively stand for a linear or branched, saturated or unsaturated C₁₋₁₀ aliphatic radical;
an unsaturated or saturated, possibly substituted 3-, 4-, 5-, 6-, 7-, 8- or 9-member cycloaliphatic radical, which can be condensed with a saturated, unsaturated or aromatic mono- or polycyclic ring system;
or for a 5- to 14-member aryl or heteroaryl radical, which can be condensed with a saturated or unsaturated mono- or polycyclic ring system;
R¹³, R¹⁴, R¹⁵, R¹⁷, R¹⁸, R¹⁹, R²¹, R²², R²³, R²⁴, R²⁸, R²⁹ and R³¹ independently of one another stand respectively for
a hydrogen radical,
a linear or branched, saturated or unsaturated, possibly substituted C₁₋₁₀ aliphatic radical;
or for a possibly substituted 5- to 14-member aryl or heteroaryl radical, which can be condensed with a saturated or unsaturated, possibly substituted mono- or polycyclic ring system;
R²⁶ and R²⁷ independently of one another stand respectively for a hydrogen radical,
a linear or branched, saturated or unsaturated, possibly substituted C₁₋₁₀ aliphatic radical;
or for a possibly substituted 5- to 14-member aryl or heteroaryl radical, which can be condensed with a saturated or unsaturated, possibly substituted mono- or polycyclic ring system;
or for -OH;
R³² stands for a hydrogen radical,
a linear or branched, saturated or unsaturated, possibly substituted C₁₋₁₀ aliphatic radical;
or for a possibly substituted 5- to 14-member aryl or heteroaryl radical, which can be condensed with a saturated or unsaturated, possibly substituted mono- or polycyclic ring system;
or for -(CH₂)_{cc}-R³⁵ with cc = 1, 2, 3 or 4, or for -CH=CH-R³⁶;
R¹⁶, R²⁰, R²⁵, R³⁰, R³³ and R³⁴ independently of one another stand respectively for
a linear or branched, saturated or unsaturated, possibly substituted C₁₋₁₀ aliphatic radical;
an unsaturated or saturated, possibly substituted 3-, 4-, 5-, 6-, 7-, 8- or 9-member cycloaliphatic radical, which can be bridged with 1, 2, 3, 4 or 5 linear or branched, possibly substituted C₁₋₅ alkylene groups and/or can be condensed with a saturated, unsaturated or aromatic, possibly substituted mono- or polycyclic ring system;
or for a possibly substituted 5- to 14-member aryl or heteroaryl radical, which can be condensed with a saturated or unsaturated, possibly substituted mono- or polycyclic ring system;
and
R³⁵ and R³⁶ independently of one another respectively stand for a possibly substituted 6- or 10-member aryl radical, which can be condensed with a saturated or unsaturated, possibly substituted mono- or polycyclic ring system;
wherein
the above-mentioned C₁₋₁₀ aliphatic radicals and C₁₋₂₀ aliphatic radicals can possibly be respectively substituted with 1, 2, 3, 4, 5, 6, 7, 8 or 9 substituents selected independently of one another from the group consisting of F, Cl, Br, I, -CN, -NO₂, -OH, -SH and -NH₂;
the above-mentioned cycloaliphatic radicals can be substituted with 1, 2, 3, 4 or 5 substituents selected independently of one another from the group consisting of oxo (=0), thioxo (=S), F, Cl, Br, I, -CN, - CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-OH, -C(=O)-O-C₁₋₅ alkyl, -O-C(=O)-C₁₋₅ alkyl, -NH-C₁₋₅ alkyl, -N(C₁₋₅ alkyl)₂, -NH-C(=O)-O-C₁₋₅ alkyl, -C(=O)-H, -C(=O)-C₁₋₅ alkyl, -C(=O)-NH₂, -C(=O)-NH-C₁₋₅ alkyl, C(=O)-N-(C₁₋₅ alkyl)₂,-S(=O)₂-C₁₋₅ alkyl, -S(=O)₂ phenyl, -NH-S(=O)₂-C₁₋₅ alkyl, -S(=O)₂-C₁₋₅ alkyl,-S(=O)₂ phenyl, -NH-S(=O)₂-C₁₋₅ alkyl, -S(=O)₂-NH-C₁₋₅ alkyl, cyclohexyl, cyclopentyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl, -O-benzyl, phenyl,-(CH₂)-benzo[b]furanyl and benzyl can be substituted with 1, 2, 3, 4 or 5 substituents selected independently of one another from the group consisting of F, Cl, Br, -OH,-CF₃, -SF₅, -CN, -NO₂, -C₁₋₅ alkyl, -O-C₁₋₅ alkyl, -O-CF₃, -S-CF₃, phenyl and -O-benzyl,
and the aforementioned cycloaliphatic radicals can possibly respectively have 1, 2, 3, 4 or 5 heteroatom(s) selected independently of one another from the group consisting of oxygen, nitrogen and sulphur as ring member(s);
the rings of the above-mentioned mono- or polycyclic ring systems can possibly be respectively substituted with 1, 2, 3, 4 or 5 substituents selected independently of one another from the group consisting of oxo (=0), thioxo (=S), F, Cl, Br, I, -CN, -CF₃,-SF₅, -OH, -O-C₁₋₅ alkyl, -NH₂, -NO₂, -O-CF₃, -S-CF₃, -SH, -S-C₁₋₅ alkyl, -C₁₋₅ alkyl,-C(=O)-OH, -C(=O)-O-C₁₋₅ alkyl, -O-C(=O)-C₁₋₅ alkyl, -NH-C₁₋₅ alkyl, -N(C₁₋₅ alkyl)₂, -NH-C(=O)-O-C₁₋₅ alkyl, -C(=O)-H, -C(=O)-C₁₋₅ alkyl, -C(=O)-NH₂, -C(=O)-NH-C₁₋₅ alkyl, C(=O)-N-(C₁₋₅ alkyl)₂, -S(=O)₂-C₁₋₅ alkyl, -S(=O)₂ phenyl, -NH-S(=O)₂-C₁₋₅ alkyl, -S(=O)₂-NH-C₁₋₅ alkyl, cyclohexyl, cyclopentyl, morpholinyl, piperidinyl, pyrrolidinyl, piperazinyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, morpholinyl, piperidinyl, pyrrolidinyl, piperazinyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, -C₁₋₅ alkyl, -O-C₁₋₅ alkyl, -O-CF₃, -S-CF₃, phenyl and -O-benzyl,
and the rings of the above-mentioned mono- or polycyclic ring systems can respectively be 5-, 6- or 7-membered and can possibly respectively have 1, 2, 3, 4 or 5 heteroatom(s) as ring member(s), which are selected independently of one another from the group consisting of oxygen, nitrogen and sulphur;
and, unless otherwise specified, the above-mentioned radical selected from the group consisting of phenyl, naphthyl, (1, 3)-benzodioxolyl, (1, 4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl,quinolinyl, isoquinolinyl, benzimidazolyl, benzoxazolyl, benzotriazolyl, benzisoxazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, 2H-benzo[1.4]oxazin-3(4H)-onyl, (3,4)-dihydroquinolin-2(1H)-onyl, [3,4]-dihydro-2H-1,4-benzoxazinyl and benzothiazolyl as well as aryl or heteroaryl radicals possibly respectively with 1, 2, 3,4 or 5 substituents selected independently of one another from the group consisting of F, Cl, Br, I, -CN, -CF₃, -SF₅, -OH, -O-C₁₋₅ alkyl, -NH₂, -NO₂, -O-CF₃,-S-CF₃, -SH, -S-C₁₋₅ alkyl, -C₁₋₁₀ alkyl, -C(=O)-OH, -C(=O)-O-C₁₋₅ alkyl, -O-C(=O)-C₁₋₅ alkyl, -NH-C₁₋₅ alkyl, -N(C₁₋₅ alkyl)₂, -NH-C(=O)-O-C₁₋₅ alkyl, -C(=O)-H,-C(=O)-C₁₋₅ alkyl, -C(=O)-NH₂, -C(=O)-NH-C₁₋₅ alkyl, C(=O)-N-(C₁₋₅ alkyl)₂,-S(=O)₂-C₁₋₅ alkyl, -S(=O)₂ phenyl, -NH-S(=O)₂-C₁₋₅ alkyl, -NH-S(=O)₂-C₁₋₅ alkylene phenyl, -NH-S(=O)₂-C₁₋₅ alkylene naphthyl, -NH-S(=O)₂ phenyl, -NH-S(=O)₂ naphthyl, cyclohexyl, cyclopentyl, morpholinyl, piperidinyl, piperazinyl, pyrrolidinyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, morpholinyl, piperidinyl, piperazinyl, pyrrolidinyl, pyridazinyl, -S(=O)₂ phenyl, -O-phenyl, -O-benzyl, phenyl, -(CH₂)-benzo[b]furanyl, NH-S(=O)₂-C₁₋₅ alkylene phenyl, -NH-S(=O)₂-C₁₋₅ alkylene naphthyl, -NH-S(=O)₂ phenyl, -NH-S(=O)₂ naphthyl and benzyl can be substituted with 1, 2, 3, 4 or 5 substituents selected independently of one another from the group consisting of F, Cl, Br, -OH,-CF₃, -SF₅, -CN, -NO₂, -C₁₋₅ alkyl, -O-C₁₋₅ alkyl, -O-CF₃, -S-CF₃, phenyl and -O-benzyl,
the aforementioned heteroaryl radicals can possibly respectively have 1, 2, 3, 4 or 5 heteroatom(s) selected independently of one another from the group consisting of oxygen, nitrogen and sulphur as ring member(s);
and
the above-mentioned C₁₋₅ alkylene groups can possibly be respectively substituted with 1, 2, 3, 4 or 5 substituents selected independently of one another from the group consisting of F, Cl, Br, -OH, -SH, -NH₂, -CN and NO₂;
in each case optionally in the form of one of the pure stereoisomers thereof, particularly enantiomers or diastereoisomers, the racemates thereof, or in the form of a mixture of stereoisomers, particularly the enantiomers and/or diastereoisomers, in an arbitrary mixing ratio, or respectively in the form of corresponding salts, or respectively in the form of corresponding solvates.

3. Compounds according to claim 1 or 2, **characterised in that**
R¹ stands for a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-benzodioxanyl, (1,2,3,4)-tetrahydromaphthyl, (1,2,3,4)-tetrahydroquinolinyl and (1,2,3,4)-tetrahydroquinazolyl, wherein the radical can possibly be respectively substituted with 1, 2, 3, 4 or 5 substituents selected independently of one another from the group consisting of oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃,-N(CH₃)₂, N(C₂H₅)₂, -NH-CH₃, -NH-C₂H₅, -NH-C(=O)-O-CH₃, -NH-C(=O)-O-C₂H₅,-NH-C(=O)-O-C(CH₃)₃, -C(=O)-H, -C(=O)-CH₃, -C(=O)-C₂H₅, -C(=O)-C(CH₃)₃,-C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
for a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl and benzothiazolyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃,-O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃,-N(CH₃)₂, N(C₂H₅)₂, -NH-CH₃, -NH-C₂H₅, -NH-C(=O)-O-CH₃, -NH-C(=O)-O-C₂H₅,-NH-C(=O)-O-C(CH₃)₃, -C(=O)-H, -C(=O)-CH₃, -C(=O)-C₂H₅), -C(=O)-C(CH₃)₃,-C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
for a -C(=O)-NR⁵R⁶ group;
for a -C(=S)-NR⁷R⁸ group;
for a -C(=O)-R⁹ group;
for a -C(=O)₂-R¹⁰ group;
or for -(CHR¹³)-R¹⁶, -(CHR¹³)-(CHR¹⁴)-R¹⁶ or (CHR¹³)-(CHR¹⁴)-(CHR¹⁵)-R¹⁶.

4. Compounds according to one of more of claims 1 to 3, **characterised in that**
R² stands for a radical selected from the group consisting of methyl, ethyl, n-propyl, isopropyl, n-butyl, sec-butyl, isobutyl, tert-butyl, n-pentyl, sec-pentyl, n-hexyl and n-heptyl, wherein the radical can possibly be respectively substituted with 1, 2, 3, 4 or 5 substituents selected independently of one another from the group consisting of F, Cl, Br, I, -CN, -NO₂, -OH, -SH and -NH₂;
for a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl and indenyl, wherein the radical can possibly be respectively substituted with 1, 2, 3, 4 or 5 substituents selected independently of one another from the group consisting of oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -0-C(=O)-C(CH₃)₃, -N(CH₃)₂, N(C₂H₅)₂, -NH-CH₃, -NH-C₂H₅, -NH-C(=O)-O-CH₃,-NH-C(=O)-O-C₂H₅, -NH-C(=O)-O-C(CH₃)₃, -C(=O)-H, -C(=O)-CH₃, -C(=O)-C₂H₅,-C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl, -O-benzyl, phenyl,-(CH₂)-benzo[b]furanyl and benzyl can be substituted with 1, 2, 3, 4 or 5 substituents selected independently of one another from the group consisting of F, Cl, Br, -OH,-CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
for a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, [3,4]-dihydro-2H-1,4-benzoxazinyl and benzothiazolyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H,-C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅,-S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -NH-S(=O)₂ phenyl, -O-phenyl, -O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃,-S-CF₃, phenyl and -O-benzyl;
on condition that not one of the meta positions and the para position of this phenyl radical are substituted with substituents, which are respectively bonded to the phenyl radical via the same atom selected from the group consisting of oxygen, sulphur and nitrogen;
or for -(CHR¹⁷)-R²⁰, -(CHR¹⁷)-(CHR¹⁸)-R²⁰ or (CHR¹⁷)-(CHR¹⁸)-(CHR¹⁹)-R²⁰.

5. Compounds according to one or more of claims 1 to 3, **characterised in that**
R² stands for a radical selected from the group consisting of methyl, ethyl, n-propyl, isopropyl, n-butyl, sec-butyl, isobutyl, tert-butyl, n-pentyl, sec-pentyl, n-hexyl and n-heptyl, wherein the radical can possibly be respectively substituted with 1, 2, 3, 4 or 5 substituents selected independently of one another from the group consisting of F, Cl, Br, I, -CN, -NO₂, -OH, -SH and -NH₂;
for a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl and indenyl, wherein the radical can possibly be respectively substituted with 1, 2, 3, 4 or 5 substituents selected independently of one another from the group consisting of oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -0-C(=O)-C(CH₃)₃, -N(CH₃)₂, N(C₂H₅)₂, -NH-CH₃, -NH-C₂H₅, -NH-C(=O)-O-CH₃,-NH-C(=O)-O-C₂H₅, -NH-C(=O)-O-C(CH₃)₃, -C(=O)-H, -C(=O)-CH₃, -C(=O)-C₂H₅,-C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃ and -S(=O)₂-C₂H₅;
for a phenyl radical of the general formula XX 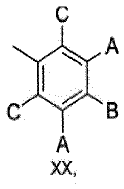 wherein the line represents the bond of this phenyl radical to the spiro compound of the general formula I;
and A, B and C respectively stand for a substituent selected independently of one another from the group consisting of H, F, Cl, Br, I, -CN, -CF₃, -SF₅, -OH, -O-CH₃,-O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, cyclohexyl, cyclopentyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅ and -NH-S(=O)₂ phenyl;
on condition that not one of the A positions and the B position of this phenyl radical are substituted with substituents, which are respectively bonded to the phenyl radical via the same atom selected from the group consisting of oxygen, sulphur and nitrogen;
for a radical selected from the group consisting of naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl, 2H-benzo[1.4]oxazin-3(4H)-onyl, (3,4)-dihydroquinolin-2(1H)-onyl, [3,4]-dihydro-2H-1,4-benzoxazinyl and benzothiazolyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, N(C₂H₅)₂,-NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂,-C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃,-S-CF₃, phenyl and -O-benzyl;
or for -(CHR¹⁷)-R²⁰, -(CHR¹⁷)-(CHR¹⁸)-R²⁰ or (CHR¹⁷)-(CHR¹⁸)-(CHR¹⁹)-R²⁰.

6. Compounds according to one or more of claims 1 to 5, **characterised in that**
R³ stands for a radical selected from the group consisting of methyl, ethyl, n-propyl, isopropyl, n-butyl, sec-butyl, isobutyl, tert-butyl, n-pentyl, sec-pentyl, n-hexyl and n-heptyl, wherein the radical can possibly be respectively substituted with 1, 2, 3, 4 or 5 substituents selected independently of one another from the group consisting of F, Cl, Br, I, -CN, -NO₂, -OH, -SH and -NH₂.

7. Compounds according to one of more of claims 1 to 6, **characterised in that**
R⁴ stands for a hydrogen radical,
or for a radical selected from the group consisting of methyl, ethyl, n-propyl, isopropyl, n-butyl, sec-butyl, isobutyl, tert-butyl, n-pentyl, sec-pentyl, n-hexyl and n-heptyl, wherein the radical can possibly be respectively substituted with 1, 2, 3, 4 or 5 substituents selected independently of one another from the group consisting of F, Cl, Br, I, -CN, -NO₂, -OH, -SH and -NH₂.

8. Compounds according to one or more of claims 1 to 7, **characterised in that**
R⁵ and R⁷, independently of one another, respectively stand
for a radical selected from the group consisting of methyl, ethyl, n-propyl, isopropyl, n-butyl, sec-butyl, isobutyl, tert-butyl, n-pentyl, sec-pentyl, n-hexyl, n-heptyl, n-octyl, n-nonyl, n-decyl, n-undecyl, n-docecyl, n-tridecyl, n-tetradecyl, n-pentadecyl, n-hexadecyl, n-heptadecyl, n-octadecyl, n-nonodecyl, n-eicosanyl, vinyl, 1-propenyl, 2-propenyl, 1-butenyl, 2-butenyl, 3-butenyl, 2-methyl-1-propenyl, ethinyl, 1-propinyl, 2-propinyl, 1- butinyl, 2-butinyl and 3-butinyl, wherein the radical can possibly be respectively substituted with 1, 2, 3, 4 or 5 substituents selected independently of one another from the group consisting of F, Cl, Br, I, -CN, -NO₂, -OH, -SH and -NH₂;
for a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl, adamantyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl and indenyl, wherein the radical can possibly be respectively substituted with 1, 2, 3, 4 or 5 substituents selected independently of one another from the group consisting of oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -0-C(=O)-C(CH₃)₃, -N(CH₃)₂, N(C₂H₅)₂, -NH-CH₃, -NH-C₂H₅, -NH-C(=O)-O-CH₃,-NH-C(=O)-O-C₂H₅, -NH-C(=O)-O-C(CH₃)₃, -C(=O)-H, -C(=O)-CH₃, -C(=O)-C₂H₅,-C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl, -O-benzyl, phenyl,-(CH₂)-benzo[b]furanyl and benzyl can be substituted with 1, 2, 3, 4 or 5 substituents selected independently of one another from the group consisting of F, Cl, Br, -OH,-CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
for a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl and benzothiazolyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃,-O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃,-N(CH₃)₂, N(C₂H₅)₂, -NH-CH₃, -NH-C₂H₅, -NH-C(=O)-O-CH₃, -NH-C(=O)-O-C₂H₅,-NH-C(=O)-O-C(CH₃)₃, -C(=O)-H, -C(=O)-CH₃, -C(=O)-C₂H₅, -C(=O)-C(CH₃)₃, - C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
or for -(CR²¹R²²)-R²⁵, -(CR²¹R²²)-(CHR²³)-R²⁵, -(CR²¹R²²)-(CHR²³)-O-R²⁵,-(CR²¹R²²)-(CHR²³)-(CHR²⁴)-R²⁵, -(CR²¹R²²)-(CHR²³)-(CHR²⁴)-O-R²⁵, -(CR²¹R²²)-(CHR²³)-(CHR²⁴)-N(CH₃)-R²⁵ or -(CR²¹R²²)-(CHR²³)-(CHR²⁴)-N(C₂H₅)-R²⁵.

9. Compounds according to one or more of claims 1 to 8, **characterised in that**
R⁶ and R⁷ respectively stand for a hydrogen radical
or for a radical selected from the group consisting of methyl, ethyl, n-propyl, isopropyl, n-butyl, sec-butyl, isobutyl and tert-butyl.

10. Compounds according to one or more of claims 1 to 9, **characterised in that**
R⁹ stands for a radical selected from the group consisting of methyl, ethyl, n-propyl, isopropyl, n-butyl, sec-butyl, isobutyl, tert-butyl, n-pentyl, sec-pentyl, n-hexyl, n-heptyl, vinyl, 1-propenyl, 2-propenyl, 1-butenyl, 2-butenyl, 3-butenyl, 2-methyl-1-propenyl, ethinyl, 1-propinyl, 2-propinyl, 1-butinyl, 2-butinyl and 3-butinyl, wherein the radical can possibly be respectively substituted with 1, 2, 3, 4 or 5 substituents selected independently of one another from the group consisting of F, Cl, Br, I, -CN, -NO₂, -OH, -SH and -NH₂; or
for a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl and benzothiazolyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃,-O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, n-pentyl, n-hexyl, n-heptyl, n-octyl, -C(=O)-OH, -C(=O)-O-CH₃,-C(=O)-O-C₂H₅, -C(=O)-O-C(CH₃)₃, -O-C(=O)-CH₃,-O-C(=O)-C₂H₅, -O-C(=O)-C(CH₃)₃, -N(CH₃)₂, N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, morpholinyl, piperidinyl, piperazinyl, pyrrolidinyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
for -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ or for -C≡C-R³⁴ and
R¹⁰ stands for a radical selected from the group consisting of methyl, ethyl, n-propyl, isopropyl, n-butyl, sec-butyl, isobutyl, tert-butyl, n-pentyl, sec-pentyl, n-hexyl, n-heptyl, vinyl, 1-propenyl, 2-propenyl, 1-butenyl, 2-butenyl, 3-butenyl, 2-methyl-1-propenyl, ethinyl, 1-propinyl, 2-propinyl, 1-butinyl, 2-butinyl and 3-butinyl, wherein the radical can possibly be respectively substituted with 1, 2, 3, 4 or 5 substituents selected independently of one another from the group consisting of F, Cl, Br, I, -CN, -NO₂, -OH, -SH and -NH₂;
for a radical selected from the group consisting of 9H-fluorenyl, 9H-zanthenyl, cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-benzodioxanyl, (1,2,3,4)-tetrahydronaphthyl, (1,2,3,4)-tetrahydroquinolinyl and (1,2,3,4)-tetrahydroquinazolinyl, wherein the radical can possibly be respectively substituted with 1, 2, 3, 4 or 5 substituents selected independently of one another from the group consisting of oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -0-C(=O)-C(CH₃)₃, -N(CH₃)₂, N(C₂H₅)₂, -NH-CH₃, -NH-C₂H₅, -NH-C(=O)-O-CH₃,-NH-C(=O)-O-C₂H₅, -NH-C(=O)-O-C(CH₃)₃, -C(=O)-H, -C(=O)-CH₃, -C(=O)-C₂H₅,-C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl, -O-benzyl, phenyl,-(CH₂)-benzo[b]furanyl and benzyl can be substituted with 1, 2, 3, 4 or 5 substituents selected independently of one another from the group consisting of F, Cl, Br, -OH,-CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
for a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl and benzothiazolyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃,-O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, n-pentyl, n-hexyl, n-heptyl, n-octyl, -C(=O)-OH, -C(=O)-O-CH₃,-C(=O)-O-C₂H₅, -C(=O)-O-C(CH₃)₃, -O-C(=O)-CH₃,-O-C(=O)-C₂H₅, -O-C(=O)-C(CH₃)₃, -N(CH₃)₂, N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, morpholinyl, piperidinyl, piperazinyl, pyrrolidinyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
for -(CR²⁶R²⁷)-R³⁰, -(CR²⁶R²⁷)-(CHR²⁸)-R³⁰, -(CR²⁶R²⁷)-(CHR²⁸)- (CHR²⁹)-R³⁰,-CR³¹=CR³²-R³³ or for -C≡C-R³⁴.

11. Compounds according to one or more of claims 1 to 10, **characterised in that**
R¹¹ and R¹² independently of one another respectively stand for a radical selected from the group consisting of methyl, ethyl, n-propyl, isopropyl, n-butyl, sec-butyl, isobutyl, tert-butyl, n-pentyl, sec-pentyl, n-hexyl and n-heptyl;
for a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl and thiomorpholinyl;
or for a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl and benzothiazolyl.

12. Compounds according to one or more of claims 1 to 11, **characterised in that**
R¹³, R¹⁴, R¹⁵, R¹⁷, R¹⁸, R¹⁹, R²¹, R²², R²³, R²⁴, R²⁸, R²⁹ and R³¹ independently of one another stand respectively for
a hydrogen radical;
for a radical selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃, n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl and 3-butinyl;
or for a phenyl radical, which can be substituted with 1, 2, 3, 4 or 5 substituents selected independently of one another from the group consisting of F, Cl, Br, I, -CN,-CF₃, -O-CH₃, -O-C₂H₅, -NO₂, -O-CF₃, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃),-N(CH₃)₂, N(C₂H₅)₂, -NH-CH₃, -NH-C₂H₅, -C(=O)-CH₃, -C(=O)-C₂H₅, -C(=O)-C(CH₃)₃, cyclohexyl, cyclopentyl, -O-phenyl, -O-benzyl and phenyl.

13. Compounds according to one or more of claims 1 to 12, **characterised in that**
R²⁶ and R²⁷ independently of one another stand respectively for
a hydrogen radical;
for a radical selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl, -(CH₂)-(CH)(C₂H₅)-(CH₂)-(CH₂)-(CH₂)-(CH₃), vinyl, 1-propenyl, 2-propenyl, 1-butenyl, 2-butenyl, 3-butenyl, ethinyl, 1-propinyl, 2-propinyl, 1-butinyl, 2-butinyl and 3-butinyl;
for a phenyl radical, which can be substituted with 1, 2, 3, 4 or 5 substituents selected independently of one another from the group consisting of F, Cl, Br, I, -CN, - CF₃,-O-CH₃, -O-C₂H₅, -NO₂, -O-CF₃, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅,-C(=O)-O-C(CH₃)₃, -O-C(=O)-CH₃, -O-C(=O)-C₂H₅, -O-C(=O)-C(CH₃)₃, -N(CH₃)₂, N(C₂H₅)₂, -NH-CH₃, -NH-C₂H₅, -C(=O)-CH₃, -C(=O)-C₂H₅, -C(=O)-C(CH₃)₃, cyclohexyl, cyclopentyl, -O-phenyl, -O-benzyl and phenyl;
or for -OH.

14. Compounds according to one or more of claims 1 to 13, **characterised in that**
R¹⁶, R²⁰, R²⁵, R³⁰, R³³ and R³⁴ independently of one another respectively stand
for a radical selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and -(CH₂)-(CH)(C₂H₅)-(CH₂)-(CH₂)-(CH₂)-(CH₃),
for a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-benzodioxanyl, (1,2,3,4)-tetrahydromaphthyl, (1,2,3,4)-tetrahydroquinolinyl and (1,2,3,4)-tetrahydroquinazolyl, wherein the radical can possibly be respectively substituted with 1, 2, 3, 4 or 5 substituents selected independently of one another from the group consisting of oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H,-C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅,-S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl,-O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
or for a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl and benzothiazolyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃,-O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, n-pentyl, n-hexyl, n-heptyl, n-octyl, -C(=O)-OH, -C(=O)-O-CH₃,-C(=O)-O-C₂H₅, -C(=O)-O-C(CH₃)₃, -O-C(=O)-CH₃,-O-C(=O)-C₂H₅, -O-C(=O)-C(CH₃)₃, -N(CH₃)₂, N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, morpholinyl, piperidinyl, piperazinyl, pyrrolidinyl, pyridinyl, pyridazinyl, -(CH₂)-benzo[b]furanyl, -O-phenyl, -O-benzyl, phenyl and benzyl, wherein respectively the cyclic part of the radicals pyridinyl, cyclopentyl, cyclohexyl, morpholinyl, piperidinyl, piperazinyl, pyrrolidinyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃,-S-CF₃, phenyl and -O-benzyl.

15. Compounds according to one or more of claims 1 to 14, **characterised in that**
m equals 0, 1 or 2.

16. Compounds according to one or more of claims 1 to 15, **characterised in that**
n equals 0, 1 or 2.

17. Compounds according to one or more of claims 1 to 16, **characterised in that**
R³² stands for a hydrogen radical;
for a radical selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and -(CH₂)-(CH)(C₂H₅)-(CH₂)-(CH₂)-(CH₂)-(CH₃),
for a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl and benzothiazolyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃,-O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, n-pentyl, n-hexyl, n-heptyl, n-octyl, -C(=O)-OH, -C(=O)-O-CH₃,-C(=O)-O-C₂H₅, -C(=O)-O-C(CH₃)₃, -O-C(=O)-CH₃,-O-C(=O)-C₂H₅, -O-C(=O)-C(CH₃)₃, -N(CH₃)₂, N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -O-phenyl,-O-benzyl, phenyl and benzyl, wherein respectively the cyclic part of the radicals pyridinyl, cyclopentyl, cyclohexyl, pyridazinyl, -S(=O)₂ phenyl, -O-phenyl, -O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
or for -(CH₂)_{cc}-R³⁵ with cc = 1, 2, 3 or 4, or for -CH=CH-R³⁶.

18. Compounds according to one or more of claims 1 to 17, **characterised in that**
R³⁵ and R³⁶, independently of one another, respectively stand for a radical selected from the group consisting of phenyl and naphthyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, n-pentyl, n-hexyl, n-heptyl, n-octyl, -C(=O)-OH, -C(=O)-O-CH₃,-C(=O)-O-C₂H₅, -C(=O)-O-C(CH₃)₃, -O-C(=O)-CH₃,-O-C(=O)-C₂H₅, -O-C(=O)-C(CH₃)₃, -N(CH₃)₂, N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃ and -S(=O)₂-NH-C₂H₅.

19. Compounds according to one or more of claims 1 to 18, **characterised in that**
m equals 0, 1, 2, 3 or 4;
n equals 0, 1 or 2;
R¹ stands for a (hetero)cycloaliphatic radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl and thiomorpholinyl;
for a radical selected from the group consisting of indolyl, isoindolyl, benzo[b]furanyl, benzo[b]thiophenyl, indazolyl, quinazolinyl, quinoxalinyl, quinolinyl, isoquinolinyl, benzimidazolyl, benzoxazolyl, benzotriazolyl, benzisoxazolyl and benzothiazolyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃, -O-C₂H₅, -NH₂, NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅,-C(=O)-O-C(CH₃)₃, -O-C(=O)-CH₃, -O-C(=O)-C₂H₅, -O-C(=O)-C(CH₃)₃, -N(CH₃)₂, N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH;, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂,-S(=O)₂-CH₃, -S(=O)₂-C₂H₅, -NH-S(=O)₂-CH₃, -NH-S(=O)₂-C₂H₅, -S(=O)₂-NH-CH₃, -S(=O)₂-NH-C₂H₅;
for a -C(=O)-NR⁵R⁶ group,
a -C(=S)-NR⁷R⁸ group,
a -C(=O)-R⁹ group,
an -S(=O)₂-R¹⁰ group,
or for -(CHR¹³)-R¹⁶, -(CHR¹³)-(CHR¹⁴)-R¹⁶ or (CHR¹³)-(CHR¹⁴)-(CHR¹⁵)-R¹⁶;
R² stands for a radical selected from the group consisting of methyl, ethyl, n-propyl, isopropyl, n-butyl, sec-butyl, isobutyl, tert-butyl, n-pentyl, sec-pentyl, n-hexyl and n-heptyl;
for a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl and thiomorpholinyl;
for a phenyl radical, which can be substituted with 1, 2, 3, 4 or 5 substituents selected independently of one another from the group consisting of F, Cl, Br, I, -CN, - CF₃,-SF5, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, cyclopentyl, cyclohexyl, -S(=O)₂-CH₃, -S(=O)₂-C₂H₅, -NH-S(=O)₂-CH₃, -NH-S(=O)₂-C₂H₅, -NH-S(=O)₂ phenyl, -S(=O)₂-NH-CH₃ and -S(=O)₂-NH-C₂H₅;
on condition that not one of the meta positions and the para position of this phenyl radical are substituted with substituents, which are respectively bonded to the phenyl radical via the same atom selected from the group consisting of oxygen, sulphur and nitrogen;
for a radical selected from the group consisting of naphthyl, (1, 3)-benzodioxolyl, (1, 4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl, [1, 2, 3, 4]-tetrahydronaphthyl, [1, 2, 3, 4]-tetrahydroquinolinyl, [1, 2, 3,4]-tetrahydroisoquinolinyl, 2H-benzo[1,4]oxazin-3(4H)-onyl, (3, 4)-dihydroquinolin-2(1H)-onyl, [1,2,3,4]-tetrahydroquinazolinyl, [3,4]-dihydro-2H-1,4-benzoxazinyl and benzothiazolyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃, -O-C₂H₅, -NH₂,-NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -S(=O)₂-CH₃, -S(=O)₂-C₂H₅, -NH-S(=O)₂-CH₃,-NH-S(=O)₂-C₂H₅, -S(=O)₂-NH-CH₃ and -S(=O)₂-NH-C₂H₅;
or for -(CHR¹⁷)-R²⁰, -(CHR¹⁷)-(CHR¹⁸)-R²⁰ or (CHR¹⁷)-(CHR¹⁸)-(CHR¹⁹)-R²⁰;
R³ stands for a radical selected from the group consisting of methyl, ethyl, n-propyl, isopropyl, n-butyl, sec-butyl, isobutyl, tert-butyl, n-pentyl, sec-pentyl, n-hexyl and n-heptyl;
R⁴ stands for a hydrogen radical
or for a radical selected from the group consisting of methyl, ethyl, n-propyl, isopropyl, n-butyl, sec-butyl, isobutyl, tert-butyl, n-pentyl, sec-pentyl, n-hexyl and n-heptyl;
R⁵ and R⁷, independently of one another, respectively stand for a radical selected from the group consisting of methyl, ethyl, n-propyl, isopropyl, n-butyl, sec-butyl, isobutyl, tert-butyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonodecyl, n-eicosanyl, vinyl, 1-propenyl, 2-propenyl, 1-butenyl, 2-butenyl, 3-butenyl, 2-methyl-1-propenyl, ethinyl, 1-propinyl, 2-propinyl, 1- butinyl, 2-butinyl and 3-butinyl, wherein the radical can possibly be respectively substituted with 1, 2, 3, 4 or 5 substituents selected independently of one another from the group consisting of F, Cl and Br;
for a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl, adamantyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl and indenyl, wherein the radical can possibly be respectively substituted with 1, 2 or 3 substituents selected independently of one another from the group consisting of methyl, ethyl, n-propyl, isopropyl, n-butyl, sec-butyl, isobutyl and tert-butyl;
for a radical selected from the group consisting of phenyl, (1,3)-benzodioxolyl, (1,4)-benzodioxanyl, naphthyl, thiophenyl, furanyl, pyrrolyl, pyrazolyl, pyranyl, pyridinyl, imidazolyl, thiazolyl, oxazolyl, isoxazolyl, pyridazinyl, pyrazinyl and pyrimidinyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H,-C(=O)-CH₃, -C(=O)-C₂H₅), -C(=O)-C(CH₃)₃, -C(=O)-NH₂, -C(=O)-NH-CH₃,-C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂ phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl,-O-phenyl, -O-benzyl, phenyl and benzyl, wherein respectively the cyclic part of the radicals pyridinyl, cyclopentyl, cyclohexyl, pyridazinyl, -S(=O)₂ phenyl, -O-phenyl,-O-benzyl, phenyl, -(CH₂)-benzo[b]furanyl and benzyl can be substituted with 1, 2, 3, 4 or 5 substituents selected independently of one another from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
or for -(CR²¹R²²)-R²⁵, -(CR²¹R²²)-(CHR²³)-R²⁵, -(CR²¹R²²)-(CHR²³)-O-R²⁵,-(CR²¹R²²)-(CHR²³)-(CHR²⁴)-R²⁵, -(CR²¹R²²)-(CHR²³)-(CHR²⁴)-O-R²⁵, -(CR²¹R²²)-(CHR²³)-(CHR²⁴)-N(CH₃)-R²⁵ or -(CR²¹R²²)-(CHR²³)-(CHR²⁴)-N(C₂H₅)-R²⁵;
R⁶ and R⁸ respectively stand for a hydrogen radical;
or for a radical selected from the group consisting of methyl, ethyl, n-propyl, isopropyl, n-butyl, sec-butyl, isobutyl and tert-butyl;
R⁹ stands for a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl,
benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl and benzothiazolyl, wherein the radical can possibly be respectively substituted with 1, 2, 3, 4 or 5 substituents selected independently of one another from the group consisting of F, Cl, Br, I, -CN, -CF₃, -O-CF₃, -S-CF₃, -SF₅, -OH, O-CH₃, -O-C₂H₅, -NH-S(=O)₂-CH₃, morpholinyl, piperidinyl, pyrrolidinyl, piperazinyl, phenyl, methyl, ethyl, n-propyl, isopropyl, n-butyl, sec-butyl, isobutyl, tert-butyl, n-pentyl, n-hexyl, n-heptyl and n-octyl;
for -(CR²⁶R²⁷)-R³⁰, -(CR²⁶R²⁷)-(CHR²⁸)-R³⁰, -(CR²⁶R²⁷)-(CHR²⁸)- (CHR²⁹)-R³⁰,-CR³¹=CR³²-R³³ or for -C≡C-R³⁴;
R¹⁰ stands for a radical selected from the group consisting of 9H-fluoroenyl, 9H-xanthenyl, phenyl, napthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinoxalinyl, quinolinyl, isoquinolinyl, benzimidazolyl, benzoxazolyl, benzotriazolyl, benzisoxazolyl and benzothiazolyl, wherein the radical can possibly be respectively substituted with 1, 2, 3, 4 or 5 substituents selected independently of one another from the group consisting of F, Cl, Br, I, -CN, -CF₃, -O-CF₃, -S-CF₃, -SF₅, -OH, -O-CH₃, -O-C₂H₅, -NH-S(=O)₂-CH₃, morpholinyl, piperidinyl, pyrrolidinyl, piperazinyl, phenyl, methyl, ethyl, n-propyl, isopropyl, n-butyl, sec-butyl, isobutyl, tert-butyl, n-pentyl, n-hexyl, n-heptyl and n-octyl;
for -(CR²⁶R²⁷)-R₃₀, -(CR²⁶R²⁷)-(CHR²⁸)-R³⁰, -(CR²⁶R²⁷)-(CHR²⁸)- (CHR²⁹)-R³⁰,-CR³¹=CR³²-R³³ or for -C≡C-R³⁴;
R¹³, R¹⁴, R¹⁵, R¹⁷, R¹⁸, R¹⁹, R²¹, R²², R²³, R²⁴, R²⁸, R²⁹ and R³¹ independently of one another stand respectively for
a hydrogen radical;
for a radical selected from the group consisting of methyl, ethyl, n-propyl, isopropyl, n-butyl, sec-butyl, isobutyl and tert-butyl;
or for a phenyl radical, which can be substituted with 1, 2, 3, 4 or 5 substituents selected independently of one another from the group consisting of F, Cl, Br, I, -CN,-CF₃, -O-CH₃, -O-C₂H₅, -NO₂, -O-CF₃, methyl, ethyl, n-propyl, isopropyl, n-butyl, sec-butyl, isobutyl and tert-butyl;
R¹⁶ stands for a radical selected from the group consisting of phenyl, naphthyl, thiophenyl, furanyl, pyrrolyl, pyrazolyl, pyrazinyl and pyridinyl, wherein the radical can possibly be respectively substituted with 1, 2, 3, 4 or 5 substituents selected independently of one another from the group consisting of F, Cl, Br, I, -CN, -CF₃,-SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, -S(=O)₂-CH₃, -S(=O)₂-C₂H₅, -NH-S(=O)₂-CH₃, -NH-S(=O)₂-C₂H₅, -S(=O)₂-NH-CH₃ and -S(=O)₂-NH-C₂H₅;
R²⁰ stands for a radical selected from the group consisting of phenyl and naphthyl, wherein the radical can possibly be respectively substituted with 1, 2, 3, 4 or 5 substituents selected independently of one another from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, n-pentyl, n-hexyl, n-heptyl and n-octyl;
R²⁵ stands for a radical selected from the group consisting of methyl, ethyl, n-propyl, isopropyl, n-butyl, sec-butyl, isobutyl and tert-butyl;
for a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl;
or for a radical selected from the group consisting of phenyl, naphthyl, (1, 3)-benzodioxolyl, (1, 4)-benzodioxanyl, thiophenyl and furanyl, wherein the radical can possibly be respectively substituted with 1, 2, 3, 4 or 5 substituents selected independently of one another from the group consisting of F, Cl, Br, I, -CN, -CF₃,-SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, N(CH₃)₂, N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅ and -C(=O)-C(CH₃)₃;
R²⁶ and R²⁷ independently of one another respectively stand
for a hydrogen radical;
for a radical selected from the group consisting of methyl, ethyl, n-propyl, isopropyl, n-butyl, sec-butyl, isobutyl and tert-butyl;
for a phenyl radical, which can be substituted with 1, 2, 3, 4 or 5 substituents selected independently of one another from the group consisting of F, Cl, Br, I, -CN, - CF₃,-O-CH₃, -O-C₂H₅, -NO₂, -O-CF₃, methyl, ethyl, n-propyl, isopropyl, n-butyl, sec-butyl, isobutyl and tert-butyl;
or for -OH;
R³² stands for a hydrogen radical;
for a radical selected from the group consisting of methyl, ethyl, n-propyl, isopropyl, n-butyl, sec-butyl, isobutyl and tert-butyl;
for a radical selected from the group consisting of phenyl, naphthyl, furanyl and thiophenyl, which can be substituted with 1, 2, 3, 4 or 5 substituents selected independently of one another from the group consisting of -SF₅, F, Cl, Br, I, -CF₃, -O-CF₃, -S-CF₃, -O-CH₃, -O-C₂H₅, phenyl,-S-CH₃, -S-C₂H₅, cyclopentyl, cyclohexyl, methyl, ethyl, n-propyl, isopropyl, n-butyl, sec-butyl, isobutyl and tert-butyl;
for -(CH₂)_{cc}-R³⁵ with cc = 1, 2 or 3, or for -CH=CH-R³⁶;
R³⁰, R³³ and R³⁴, independently of one another, respectively stand for
a radical selected from the group consisting of methyl, ethyl, n-propyl, isopropyl, n-butyl, sec-butyl, isobutyl and tert-butyl;
or for a radical selected from the group consisting of phenyl, pyridinyl and naphthyl, which can be substituted with 1, 2, 3, 4 or 5 substituents selected independently of one another from the group consisting of -SF₅, F, Cl, Br, -CF₃, -O-CF₃, -S-CF₃, phenyl, morpholinyl, piperidinyl, pyrrolidinyl, piperazinyl, cyclopentyl, cyclohexyl, -OH, -O-CH₃, -O-C₂H₅, methyl, ethyl, n-propyl, isopropyl, n-butyl, sec-butyl, isobutyl, tert-butyl, n-pentyl, n-hexyl, h-heptyl and n-octyl;
and
R³⁵ and R³⁶, independently of one another, respectively stand
for a phenyl radical, which can be substituted with 1, 2, 3, 4 or 5 substituents selected independently of one another from the group consisting of F, Cl, Br, I, -CN, - CF₃,-O-CH₃, -O-C₂H₅, -NO₂, -O-CF₃, methyl, ethyl, n-propyl, isopropyl, n-butyl, sec-butyl, isobutyl and tert-butyl;
in each case optionally in the form of one of the pure stereoisomers thereof, particularly enantiomers or diastereoisomers , the racemates thereof, or in the form of a mixture of stereoisomers, particularly the enantiomers and/or diastereoisomers, in an arbitrary mixing ratio, or respectively in the form of corresponding salts, or respectively in the form of corresponding solvates.

20. Compounds according to one or more of claims 1 to 19, **characterised in that**
m equals 0, 1 or 2;
n equals 0, 1 or 2;
R¹ stands for a radical selected from the group consisting of benzimidazolyl, benzoxazolyl, benzotriazolyl, benzisoxazolyl and benzothiazolyl, wherein the radical can be respectively substituted with 1, 2, 3, 4 or 5 substituents selected independently of one another from the group consisting of F, Cl, Br, CF₃, -O-CF₃, -S-CF₃, methyl, ethyl, n-propyl, isopropyl, n-butyl, sec-butyl, isobutyl and tert-butyl;
for a -C(=O)-NR⁵R⁶ group;
a -C(=S)-NR⁷R⁸ group,
a -C(=O)-R⁹ group,
or for an -S(=O)₂-R¹⁰ group;
R² stands for a radical selected from the group consisting of methyl, ethyl, n-propyl, isopropyl, n-butyl, sec-butyl, isobutyl, tert-butyl, n-pentyl, sec-pentyl, n-hexyl and n-heptyl;
for a phenyl radical of the general formula XX 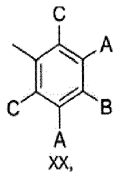 wherein the line represents the bond of this phenyl radical to the spiro compound of the general formula I;
A and B respectively stand for a substituent selected independently of one another from the group consisting of H, F, Cl, Br, I, -CF₃, -SF₅, -OH, -O-CH₃, -O-C₂H₅, -O-CF₃, -S-CF₃, methyl, ethyl, n-propyl, isopropyl, n-butyl, sec-butyl, isobutyl, tert-butyl, cyclopentyl, cyclohexyl, -NH-S(=O)₂-CH₃ and -NH-S(=O)₂ phenyl;
on condition that not one of the A positions and the B position of this phenyl radical are substituted with substituents, which are respectively bonded to the phenyl radical via the same atom selected from the group consisting of oxygen, sulphur and nitrogen;
C respectively stands for H;
for a radical selected from the group consisting of naphthyl, quinolinyl, (1,4)-benzodioxanyl, (1,3)-benzodioxolyl, pyridinyl, thiazolyl and oxazolyl, wherein the radical can possibly be respectively substituted with 1, 2, 3, 4 or 5 substituents selected independently of one another from the group consisting of F, Cl, Br, I, -O-CH₃, -O-C₂H₅, methyl, ethyl, n-propyl, isopropyl, n-butyl, sec-butyl, isobutyl, tert-butyl, -S(=O)₂-CH₃, -S(=O)₂-C₂H₅, -NH-S(=O)₂-CH₃, -NH-S(=O)₂-C₂H₅, -S(=O)₂-NH-CH₃ and -S(=O)₂-NH-C₂H₅;
or for -(CHR¹⁷)-R²⁰, -(CHR¹⁷)-(CHR¹⁸)-R²⁰ or (CHR¹⁷)-(CHR¹⁸)-(CHR¹⁹)-R²⁰;
R³ stands for a radical selected from the group consisting of methyl, ethyl, n-propyl, isopropyl, n-butyl, sec-butyl, isobutyl, tert-butyl and n-pentyl;
R⁴ stands for a hydrogen radical;
R⁵ and R⁷, independently of one another, respectively stand for a radical selected from the group consisting of methyl, ethyl, n-propyl, isopropyl, n-butyl, sec-butyl, isobutyl, tert-butyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonodecyl, n-eicosanyl, vinyl, 1-propenyl, 2-propenyl, 1-butenyl, 2-butenyl, 3-butenyl, 2-methyl-1-propenyl, ethinyl, 1-propinyl, 2-propinyl, 1- butinyl, 2-butinyl and 3-butinyl, wherein the radical can possibly be respectively substituted with 1, 2, 3, 4 or 5 substituents selected independently of one another from the group consisting of F, Cl and Br;
for a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl and adamantyl, wherein the radical can possibly be respectively substituted with 1, 2 or 3 substituents selected independently of one another from the group consisting of methyl, ethyl, n-propyl, isopropyl, n-butyl, sec-butyl, isobutyl and tert-butyl;
for a radical selected from the group consisting of phenyl, naphthyl, (1,4)-benzodioxanyl and pyridinyl, wherein the radical can possibly be respectively substituted with 1, 2, 3, 4 or 5 substituents selected independently of one another from the group consisting of -SF₅, F, Cl, Br, I, -CN, -CF₃, -SF₅, -O-CH₃, -O-C₂H₅, -NO₂,-O-CF₃, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, N(C₂H₅)₂, -NH-CH₃, -NH-C₂H₅, -C(=O)-CH₃, -C(=O)-C₂H₅, -C(=O)-C(CH₃)₃, cyclohexyl, cyclopentyl,-O-phenyl, -O-benzyl and phenyl, wherein the cyclic part of the radicals cyclopentyl, cyclohexyl, -O-phenyl, -O-benzyl and phenyl can be respectively substituted with 1, 2, 3, 4 or 5 substituents selected independently of one another from the group consisting of F, Cl, Br, methyl, ethyl, n-propyl, isopropyl, n-butyl, sec-butyl, isobutyl and tert-butyl;
or for -(CR²¹R²²)-R²⁵, -(CR²¹R²²)-(CHR²³)-R²⁵, -(CR²¹R²²)-(CHR²³)-O-R²⁵,-(CR²¹R²²)-(CHR²³)-(CHR²⁴)-R²⁵, -(CR²¹R²²)-(CHR²³)-(CH²⁴)-O-R²⁵, -(CR²¹R²²)-(CHR²³)-(CHR²⁴)-N(CH₃)-R²⁵ or -(CR²¹R²²)-(CHR²³)-(CHR²⁴)-N(C₂H₅)-R²⁵;
R⁶ and R⁸ respectively stand for a hydrogen radical;
or for a methyl or ethyl radical;
R⁹ stands for a radical selected from the group consisting of phenyl, pyridinyl and naphthyl, wherein the radical can possibly be respectively substituted with 1, 2, 3, 4 or 5 substituents selected independently of one another from the group consisting of F, Cl, Br, I, -CF₃, -O-CF₃, -S-CF₃, -OH, -O-CH₃, -O-C₂H₅, -NH-S(=O)₂-CH₃, morpholinyl, piperidinyl, pyrrolidinyl,, piperazinyl, phenyl,methyl, ethyl, n-propyl, isopropyl, n-butyl, sec-butyl, isobutyl, tert-butyl, n-pentyl, n-hexyl, n-heptyl and n-octyl;
for -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ or for -C≡C-R³⁴;
R¹⁰ stands for a radical selected from the group consisting of phenyl and naphthyl, wherein the radical can possibly be respectively substituted with 1, 2, 3, 4 or 5 substituents selected independently of one another from the group consisting of F, Cl, Br, I, -CF₃, -OH, -O-CH₃, -O-C₂H₅, methyl, ethyl, n-propyl, isopropyl, n-butyl, sec-butyl, isobutyl and tert-butyl;
for -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ or for -C≡C-R³⁴;
R¹⁷, R¹⁸, R¹⁹, R²³, R²⁴, R²⁸ and R²⁹, independently of one another, respectively stand for a hydrogen radical;
for a radical selected from the group consisting of methyl, ethyl, n-propyl, isopropyl, n-butyl, sec-butyl, isobutyl and tert-butyl;
or for a phenyl radical;
R²⁰ stands for a phenyl radical;
R²¹ and R²², independently of one another, stand for
a hydrogen radical;
for a radical selected from the group consisting of methyl, ethyl, n-propyl, isopropyl, n-butyl, sec-butyl, isobutyl and tert-butyl;
or for a phenyl radical;
R²⁵ stands for a radical selected from the group consisting of methyl, ethyl, n-propyl, isopropyl, n-butyl, sec-butyl, isobutyl and tert-butyl;
for a radical selected from the group consisting of cyclopentyl, cyclohexyl, tetrahydrofuranyl, tetrahydrothiophenyl, pyrrolidinyl, morpholinyl, piperidinyl and piperazinyl;
or for a radical selected from the group consisting of phenyl, naphthyl, (1, 3)-benzodioxolyl, (1, 4)-benzodioxanyl, thiophenyl and furanyl, wherein the radical can possibly be respectively substituted with 1, 2, 3, 4 or 5 substituents selected independently of one another from the group consisting of F, Cl, Br, -OH, -O-CH₃,-O-C₂H₅, methyl, ethyl, n-propyl, isopropyl, n-butyl, sec-butyl, isobutyl and tert-butyl;
R²⁶ and R²⁷, independently of one another, respectively
stand for a hydrogen radical;
for a radical selected from the group consisting of methyl, ethyl, n-propyl, isopropyl, n-butyl, sec-butyl, isobutyl and tert-butyl;
for a phenyl radical or for -OH;
R³⁰ stands for a phenyl radical, which can be substituted with 1, 2, 3, 4 or 5 substituents selected independently of one another from the group consisting of F, Cl, Br, -NH-S(=O)₂-CH₃, methyl, ethyl, n-propyl, isopropyl, n-butyl, sec-butyl, isobutyl, and tert-butyl;
R³¹ stands for a hydrogen radical;
or for a radical selected from the group consisting of methyl, ethyl, n-propyl, isopropyl, n-butyl, sec-butyl, isobutyl and tert-butyl;
R³² stands for a hydrogen radical;
for a radical selected from the group consisting of methyl, ethyl, n-propyl, isopropyl, n-butyl, sec-butyl, isobutyl and tert-butyl;
for a radical selected from the group consisting of phenyl, naphthyl, furanyl and thiophenyl, which can be substituted with 1, 2, 3, 4 or 5 substituents selected independently of one another from the group consisting of -SF₅, F, Cl, Br, I, -CF₃, -O-CF₃, -S-CF₃, -O-CH₃, -O-C₂H₅, phenyl,-S-CH₃, -S-C₂H₅, cyclopentyl, cyclohexyl, methyl, ethyl, n-propyl, isopropyl, n-butyl, sec-butyl, isobutyl and tert-butyl;
for -CH₂-R³⁵ or for -CH=CH-R³⁶;
R³³ and R³⁴, independently of one another, respectively
stand for a radical selected from the group consisting of methyl, ethyl, n-propyl, isopropyl, n-butyl, sec-butyl, isobutyl and tert-butyl;
or for a radical selected from the group consisting of phenyl, pyridinyl and naphthyl, which can be substituted with 1, 2, 3, 4 or 5 substituents selected independently of one another from the group consisting of -SF₅, F, Cl, Br, -CF₃, -O-CF₃, -S-CF₃, phenyl, morpholinyl, piperidinyl, pyrrolidinyl, piperazinyl, cyclopentyl, cyclohexyl, -OH, -O-CH₃, -O-C₂H₅, methyl, ethyl, n-propyl, isopropyl, n-butyl, sec-butyl, isobutyl, tert-butyl, n-pentyl, n-hexyl, h-heptyl and n-octyl;
and
R³⁵ and R³⁶, respectively stand for a phenyl radical;
in each case optionally in the form of one of the pure stereoisomers thereof, particularly enantiomers or diastereoisomers , the racemates thereof, or in the form of a mixture of stereoisomers, particularly the enantiomers and/or diastereoisomers, in an arbitrary mixing ratio, or respectively in the form of corresponding salts, or respectively in the form of corresponding solvates.

21. Compounds according to one or more of claims 1 to 20, **characterised in that**
m equals 0, 1 or 2;
n equals 0, 1 or 2;
R¹ stands for a radical selected from the group consisting of benzimidazolyl, benzoxazolyl, benzotriazolyl, benzisoxazolyl and benzothiazolyl, wherein the radical can be respectively substituted with 1, 2, 3, 4 or 5 substituents selected independently of one another from the group consisting of F, Cl, Br, CF₃, -O-CF₃, -S-CF₃, methyl, ethyl, n-propyl, isopropyl, n-butyl, sec-butyl, isobutyl and tert-butyl;
for a -C(=O)-NR⁵R⁶ group;
a -C(=S)-NR⁷R⁸ group,
a -C(=O)-R⁹ group,
or for an -S(=O)₂-R¹⁰ group;
R² stands for a tert-butyl radical;
for a radical selected from the group consisting of phenyl, 2-methanesulphonamide phenyl, 2-ethanesulphonamide phenyl, 2-trifluoromethyl phenyl, 2-trifluoromethylsulphanyl phenyl, 2-ethyl phenyl, 2-tert-butyl phenyl, 2-ethylamino-sulphonyl phenyl, 2-methylamino-sulphonyl phenyl, 2-bromo-phenyl,2-chloro-phenyl, 2-fluoro-phenyl, 2-methyl phenyl, 2-trifluoromethoxy phenyl, 2-methoxy phenyl, 2-ethoxy phenyl, 2-propyl phenyl, 2-iodo-phenyl, 2-chloro-phenyl, 2-methyl phenyl, 3-tert-butyl phenyl, 3-trifluoromethylsulphanyl phenyl, 3-trifluoromethyl phenyl, 3-methanesulphonamide phenyl, 3-ethanesulphonamide phenyl, 3-fluoro phenyl, 3-propyl-phenyl, 3-isopropyl phenyl, 3-bromo-phenyl, 3-methoxy phenyl, 3-ethyl phenyl, 3-ethylamino-sulphonyl phenyl, 3-methylamino-sulphonyl phenyl, 3-ethoxy phenyl, 3-trifluoromethoxy phenyl, 3-iodophenyl, 4-methylamino-sulphonyl phenyl, 4-ethylamino-sulphonyl phenyl, 4-methansulphonamide phenyl, 4-ethanesulphonamide phenyl, 4-bromo phenyl, 4-methoxy phenyl, 4-chloro phenyl, 4-fluoro phenyl, 4-tert-tutyl phenyl, 4-trifuloromethylsulphanyl phenyl, 4-methyl phenyl, 4-isopropyl phenyl, 4-trifluoromethyl phenyl, 4-propyl phenyl, 4-iodo-phenyl, 4-trifluoromethoxy phenyl, 4-ethyl phenyl, 4-ethoxy-phenyl, 2-fluoro-3-trifluoromethylphenyl, (2, 3)-difluorophenyl, (2, 3)-dimethyl phenyl, (2, 3)-dichlorophenyl, 3-fluoro-2-trifluoromethylphenyl, (2,4)-dichloro-phenyl, (2,4)-difluorophenyl, 4-fluoro-2-trifluoromethyl phenyl, (2,4)-dimethoxyphenyl, 2-chloro-4-fluoro-phenyl, (2,4)-dibromo-phenyl, 2-fluoro-4-trifluoromethyl phenyl, (2, 5)-difluoro-phenyl, 2-fluoro-5-trifluoromethyl phenyl, 5-fluoro-2-trifluoromethyl phenyl, 5-chloro-2-trifluoromethyl phenyl, 5-bromo-2-trifluoromethyl phenyl, (2,5)-dimethoxy phenyl, (2,5)-bis-trifluoromethyl phenyl (2,5)-dichloro-phenyl, (2,5)-dibromo-phenyl, 2-fluoro-6-trifluoromethyl phenyl, (2,6)-dimethoxy phenyl, (2,6)-dimethoxy phenyl, (2,6)-dimethyl phenyl, (2,6)-dichloro phenyl, 2-chloro-6-fluoro-phenyl, 2-bromo-6-chloro-phenyl, 2-bromo-6-fluoro-phenyl, (2,6)-difluoro-phenyl, (2,6)-difluoro-3-methyl phenyl, (2,6)-dibromo-phenyl, (2,6)-dichlorophenyl, 3-chloro-2-fluoro-phenyl, (3,4)-dichlorophenyl, 4-fluoro-3-trifluoromethylphenyl, 3-fluoro-4-trifluoromethyl phenyl, (3,4)-difluoro-phenyl, 4-chloro-3-trifluoromethyl, 4-bromo-3-methyl phenyl, 4-bromo-5-methyl phenyl, 3-chloro-4-fluoro-phenyl, (3, 4)-dibromo-phenyl, 4-chloro-3-methyl phenyl, 4-bromo-3-methyl phenyl, 4-fluoro-3-methyl phenyl, (3,5)-dimethoxy phenyl, (3,5)-bis-trifluoromethyl phenyl, (3,5)-difluoro-phenyl, (3,5)-dichloro-phenyl, 3-fluoro-5-trifluoromethyl phenyl, 5-fluoro-3-trifluoromethyl phenyl, (3,5)-dibromo-phenyl, 5-chloro-4-fluoro-phenyl, 5-bromo-4-methyl phenyl, (2,3,4)-trifluorophenyl, (2,3,4)-trichlorophenyl, (2,3,6)-trifluorophenyl, 5-chloro-2-methoxy-pheny. (2,3)-difluoro-4-methyl phenyl, (2,4,5)-trifluorophenyl, (2,4,5)-trichlorophenyl, (2,4)-dichloro-5-fluoro-phenyl, (2,4,6)-trichlorophneyl, (2,4,6)-trimethylphenyl, (2,4,6)-trifluorophenyl, (2,4,6)-trimethoxy-phenyl, (2,3,4,5)-tetrafluoro-phenyl, 4-methoxy-2,3,6-trimethyl phenyl, 4-methoxy-2,3,6-trimethyl phenyl., 4-choloro-2,5-dimethyl-phenyl, 2.-chloro-6-fluoro-3-pmethyl phenyl, 6-chloro-2-fluoro-3-methyl, (2,3,4,5,6)-pentafluoro-phenyl, 3-fluoro-4-methylsulphonamido-phenyl, 3-chloro-4-methylsulphonamido-phenyl, 3-bromo-4-methylsulphonamido-phenyl, 3-methoxy-4-methylsulphonamido-phenyl, 3-hydroxy-4-methylsulphonamido-phenyl, 3-trifluoromethyl-4-methylsulphonamido-phenyl, 3-trifluoromethoxy-4-methylsulphonamido-phenyl, 3-methyl-4-methylsulphonamido-phenyl, 3-ethyl-4-methylsulphonamido-phenyl, 3-isopropyl-4-methylsulphonamido-phenyl, 3-propyl-4-methylsulphonamido-phenyl, 3-tert-butyl-4-methylsulphonamido-phenyl, 3-fluoro-4-phenylsulphonamido-phenyl, 3-chloro-4-phenylsulphonamido-phenyl, 3-bromo-4-phenylsulphonamido-phenyl, 3-methoxy-4-phenylsulphonamido-phenyl, 3-hydroxy-4-phenylsulphonamido-phenyl, 3-trifluoromethyl-4-phenylsulphonamido-phenyl, 3-trifluoromethoxy-4-phenylsulphonamido-phenyl, 3-methyl-4-phenylsulphonamido-phenyl, 3-ethyl-4-phenylsulphonamido-phenyl, 3-isopropyl-4-phenylsulphonamido-phenyl, 3-propyl-4-phenylsulphonamido-phenyl, 3-tert-butyl-4-phenylsulphonamido-phenyl, 4-fluoro-3-methylsulphonamido-phenyl, 4-chloro-3-methylsulphonamido-phenyl, 4-bromo-3-methylsulphonamido-phenyl, 4-methoxysulphonamido-phenyl, 4-hydroxy-3-methylsulphonamido-phenyl, 4-trifluoromethyl-3-methylsulphonamido-phenyl,4-trifluoromethoxy-3-methylsulphonamido-phenyl, 4-methyl-3-methylsulphonamido-phenyl, 4-ethyl-3-methylsulphonamido-phenyl, 4-isopropyl-3-methylsulphonamido-phenyl, 4-propyl-3-methylsulphonamido-phenyl, 4-tert-butyl-3-methylsulphonamido-phenyl, 4-fluoro-3-phenylsulphonamido-phenyl, 4-chloro-3-phenylsulphonamido-phenyl, 4-bromo-3-phenylsulphonamido-phenyl, 4-methoxy-3-phenylsulphonamido-phenyl, 4hydroxy-3-phenylsulphonamido-phenyl, 4-trifluoromethyl-3-phenylsulphonamido-phenyl, 4-trifluoromethoxy-3-phenylsulphonamido-phenyl, 4-methyl-3-phenylsulphonamido-phenyl, 4-ethyl-3-phenylsulphonamido-phenyl, 4-isopropyl-3-phenylsulphonamido-phenyl, 4-propyl-3-phenylsulphonamido-phenyl, 4-tert-butyl-3-phenylsulphonamido-phenyl, 2-cyclohexyl-phenyl, 3-cyclohexyl-phenyl and 4-cyclohexyl-phenyl;
for a radical selected from the group consisting of quinolinyl, (1,4)-benzodioxanyl, (1,3)-benzodioxolyl, naphthyl and thiazolyl;
for a pyridinyl radical, wherein the radical can be respectively substituted with 1 or 2 substituents selected independently of one another from the group consisting of F, Cl and Br;
for -(CHR¹⁷)-R²⁰ or -(CHR¹⁷)-(CHR¹⁸)-R²⁰;
R³ stands for a methyl or ethyl radical;
R⁴ stands for a hydrogen radical;
R⁵ and R⁷, independently of one another, respectively stand for a radical selected from the group consisting of methyl, ethyl, n-propyl, isopropyl, n-butyl, sec-butyl, isobutyl, tert-butyl, n-pentyl, sec-pentyl, n-hexyl, n-heptyl, n-octyl, n-nonyl, n-decyl, n-undecyl, n-docecyl, n-tridecyl, n-tetradecyl, n-pentadecyl, n-hexadecyl, n-heptadecyl, n-octadecyl, n-nonodecyl, n-eicosanyl, vinyl, 1-propenyl, 2-propenyl, 1-butenyl, 2-butenyl, 3-butenyl and 2-methyl-1-propenyl, wherein the radical can possibly be respectively substituted with 1, 2, 3, 4 or 5 substituents selected independently of one another from the group consisting of F, Cl and Br;
for a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl and adamantyl, wherein the radical can possibly be respectively substituted with 1, 2 or 3 substituents selected independently of one another from the group consisting of methyl, ethyl, n-propyl, isopropyl, n-butyl, sec-butyl, isobutyl and tert-butyl;
for a radical selected from the group consisting of phenyl, naphthyl, (1,4)-benzodioxanyl and pyridinyl, wherein the radical can possibly be respectively substituted with 1, 2, 3, 4 or 5 substituents selected independently of one another from the group consisting of -SF₅, F, Cl, Br, I, -CN, -CF₃, -O-CH₃, -O-C₂H₅, -NO₂, -O-CF₃, -S-CH₃, -S-C₂H₅, methyl, ethyl, n-propyl, isopropyl, n-butyl, sec-butyl, isobutyl, tert-butyl, -C(=O)-OH, -C(=O)-O-CH₃,-C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, N(C₂H₅)₂, -NH-CH₃, -NH-C₂H₅, -C(=O)-CH₃, -C(=O)-C₂H₅, -C(=O)-C(CH₃)₃, cyclohexyl, cyclopentyl, -O-phenyl, -O-benzyl and phenyl, wherein the cyclic part of the radicals cyclopentyl, cyclohexyl, -O-phenyl, -O-benzyl and phenyl can be respectively substituted with 1, 2, 3, 4 or 5 substituents selected independently of one another from the group consisting of F, Cl, Br, methyl, ethyl, n-propyl, isopropyl, n-butyl, sec-butyl, isobutyl and tert-butyl;
or for -(CR²¹R²²)-R²⁵, -(CR²¹R²²)-(CHR²³)-R²⁵, -(CR²¹R²²)-(CHR²³)-O-R²⁵,-(CR²¹R²²)-(CHR²³)-(CHR²⁴)-R²⁵, -(CR²¹R²²)-(CHR²³)-(CH²⁴)-O-R²⁵, -(CR²¹R²²)-(CHR^{z3})-(CHR²⁴)-N(CH₃)-R²⁵ or -(CR²¹R²²)-(CHR²³)-(CHR²⁴)-N(C₂H₅)-R²⁵;
R⁶ and R⁸ respectively stand for a hydrogen radical;
or for a methyl or ethyl radical;
R⁹ stands for a radical selected from the group consisting of phenyl, pyridinyl and naphthyl, wherein the radical can possibly be respectively substituted with 1, 2, 3, 4 or 5 substituents selected independently of one another from the group consisting of F, Cl, Br, I, -CF₃, -O-CF₃, -S-CF₃, -OH, -O-CH₃, -O-C₂H₅, -NH-S(=O)₂-CH₃, morpholinyl, piperidinyl, pyrrolidinyl, piperazinyl, phenyl,methyl, ethyl, n-propyl, isopropyl, n-butyl, sec-butyl, isobutyl, tert-butyl, n-pentyl, n-hexyl, n-heptyl and n-octyl;
for -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ or for -C≡C-R³⁴;
R¹⁰ stands for a radical selected from the group consisting of phenyl and naphthyl, wherein the radical can possibly be respectively substituted with 1, 2, 3, 4 or 5 substituents selected independently of one another from the group consisting of F, Cl, Br, I, -CF₃, -OH, -O-CH₃, -O-C₂H₅, methyl, ethyl, n-propyl, isopropyl, n-butyl, sec-butyl, isobutyl and tert-butyl;
for -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰,-CR³¹=CR³²-R³³ or for -C≡C-R³⁴;
R¹⁷, R¹⁸, R¹⁹, R²³, R²⁴, R²⁸ and R²⁹, independently of one another, respectively stand for a hydrogen radical;
for a radical selected from the group consisting of methyl, ethyl, n-propyl, isopropyl, n-butyl, sec-butyl, isobutyl and tert-butyl;
or for a phenyl radical;
R²⁰ stands for a phenyl radical;
R²¹ and R²², independently of one another, stand for
a hydrogen radical;
for a radical selected from the group consisting of methyl, ethyl, n-propyl, isopropyl, n-butyl, sec-butyl, isobutyl and tert-butyl;
or for a phenyl radical;
R²⁵ stands for a radical selected from the group consisting of methyl, ethyl, n-propyl, isopropyl, n-butyl, sec-butyl, isobutyl and tert-butyl;
for a radical selected from the group consisting of cyclopentyl, cyclohexyl, tetrahydrofuranyl, tetrahydrothiophenyl, pyrrolidinyl, morpholinyl, piperidinyl and piperazinyl;
or for a radical selected from the group consisting of phenyl, naphthyl, (1, 3)-benzodioxolyl, (1, 4)-benzodioxanyl, thiophenyl and furanyl, wherein the radical can possibly be respectively substituted with 1, 2, 3, 4 or 5 substituents selected independently of one another from the group consisting of F, Cl, Br, -OH, -O-CH₃,-O-C₂H₅, methyl, ethyl, n-propyl, isopropyl, n-butyl, sec-butyl, isobutyl and tert-butyl;
R²⁶ and R²⁷, independently of one another, respectively
stand for a hydrogen radical;
for a radical selected from the group consisting of methyl, ethyl, n-propyl, isopropyl, n-butyl, sec-butyl, isobutyl and tert-butyl;
for a phenyl radical
or for -OH;
R³⁰ stands for a phenyl radical, which can be substituted with 1, 2, 3, 4 or 5 substituents selected independently of one another from the group consisting of F, Cl, Br, -NH-S(=O)₂-CH₃, methyl, ethyl, n-propyl, isopropyl, n-butyl, sec-butyl, isobutyl, and tert-butyl;
R³¹ stands for a hydrogen radical;
or for a radical selected from the group consisting of methyl, ethyl, n-propyl, isopropyl, n-butyl, sec-butyl, isobutyl and tert-butyl;
R³² stands for a hydrogen radical;
for a radical selected from the group consisting of methyl, ethyl, n-propyl, isopropyl, n-butyl, sec-butyl, isobutyl and tert-butyl;
for a radical selected from the group consisting of phenyl, naphthyl, furanyl and thiophenyl, which can be substituted with 1, 2, 3, 4 or 5 substituents selected independently of one another from the group consisting of -SF₅, F, Cl, Br, I, -CF₃, -O-CF₃, -S-CF₃, -O-CH₃, -O-C₂H₅, phenyl,-S-CH₃, -S-C₂H₅, cyclopentyl, cyclohexyl, methyl, ethyl, n-propyl, isopropyl, n-butyl, sec-butyl, isobutyl and tert-butyl;
for -CH₂-R³⁵ or for -CH=CH-R³⁶;
R³³ and R³⁴, independently of one another, respectively
stand for a radical selected from the group consisting of methyl, ethyl, n-propyl, isopropyl, n-butyl, sec-butyl, isobutyl and tert-butyl;
or for a radical selected from the group consisting of phenyl, pyridinyl and naphthyl, which can be substituted with 1, 2, 3, 4 or 5 substituents selected independently of one another from the group consisting of -SF₅, F, Cl, Br, -CF₃, -O-CF₃, -S-CF₃, phenyl, morpholinyl, piperidinyl, pyrrolidinyl, piperazinyl, cyclopentyl, cyclohexyl, -OH, -O-CH₃, -O-C₂H₅, methyl, ethyl, n-propyl, isopropyl, n-butyl, sec-butyl, isobutyl, tert-butyl, n-pentyl, n-hexyl, h-heptyl and n-octyl;
and
R³⁵ and R³⁶, respectively stand for a phenyl radical;
in each case optionally in the form of one of the pure stereoisomers thereof, particularly enantiomers or diastereoisomers , the racemates thereof, or in the form of a mixture of stereoisomers, particularly the enantiomers and/or diastereoisomers, in an arbitrary mixing ratio, or respectively in the form of corresponding salts, or respectively in the form of corresponding solvates.

22. Compounds according to one or more of claims 1 to 21 selected from the group consisting of
[1] 3-(3-methoxy-phenyl)-1-oxa-2,8-diaza-spiro[4.5]dec-2-en-8-carboxylic acid phenylamide
[2] 3-phenyl-1-oxa-2,8-diaza-spiro[4.5]dec-2-en-8-carboxylic acid phenylamide
[3] 3-(4-chloro-phenyl)-1-oxa-2,8-diaza-spiro[4.5]dec-2-en-8- carboxylic acid phenylamide
[4] 3-(4-tert-butyl-phenyl)-1-oxa-2,8-diaza-spiro[4.5]dec-2-en-8-carboxylic phenylamide
[5] 3-p-tolyl-1-oxa-2,8-diaza-spiro[4.5]dec-2-en-8-carboxylic phenylamide
[6] 3-(4-trifluoromethyl-phenyl)-1-oxa-2,8-diaza-spiro[4.5]dec-2-en-8-carboxylic phenylamide
[7] 3-(3-methoxy-phenyl)-1-oxa-2,8-diaza-spiro[4.5]dec-2-en-8-carboxylic-m-tolylamide
[8] 3-(3-methodxy-phenyl)-1-oxa-2,8-diaza-spiro[4.5]dec-2-en-8-m-tolylamide
[9] 3-phenyl-1-oxa-2,8-diaza-spiro[4.5]dec-2-en-8-carboxylic acid-m-tolylamide
[10] 3-(4-chloro-phenyl)-1-oxa-2,8-diaza-spiro[4.5]dec-2-en-8-carboxylic acid-m-tolylamide
[11] 3-(4-tert-butyl-phenyl)-1-oxa-2,8-diaza-spiro[4.5]dec-2-en-8-carboxylic acid-m-tolylamide
[12] 3-p-tolyl-1-oxa-2,8-diaza-spiro[4.5]dec-2-en-8-carboxylic acid-m-tolylamide
[13] 3-4(trifluoromethyl-phenyl)-1-oxa-2,8-diaza-spiro[4.5]dec-2-en-8-carboxylic acid-m-tolylamide
[14] 3-(3-methoxy-phenyl)-1-oxa-2,8-diaza-spiro[4.5]dec-2-en-8-carboxylic acid-(3-ethyl-phenyl)amide
[15] 3-(3-methoxy-phenyl)-1-oxa-2,8-diaza-spiro[4.5]dec-2-en-8-carboxylic acid-(3-ethyl-phenyl)amide
[16] 3-phenyl-1-oxa-2,8-diaza-spiro[4.5]dec-2-en-8-carboxylic acid-(3-ethyl-phenyl)amide
[17] 3-(4-chloro-phenyl)-1-oxa-2,8-diaza-spiro[4.5]dec-2-en-8-carboxylic acid-(3-ethyl-phenyl)amide
[18] 3-(4-tert-butyl-phenyl)-1-oxa-2,8-diaza-spiro[4.5]dec-2-en-8-carboxylic acid-(3-ethyl-phenyl)amide
[19] 3-p-tolyl-a-oxa-2,8-diaza-spiro[4.5]dec-2-en-8-carboxylic acid-(3-ethyl-phenyl)amide
[20] 3-(4-trifluoromethyl-phenyl)-1-oxa-2,8-diaza-spiro[4.5]dec-2-en-80-carboxylic acid-(3-ethyl-phenyl)amide
[21] 3-(3-methodxy-phenyl)-1-oxa-2,8-diaza-spiro[4.5]dec-2-en-8-carboxylic acid-(2-propyl-phenyl)amide
[22] 3-phenyl-1-oxa-2,8-diaza-spiro[4.5]dec-2-en-8-carboxylic acid-(2-propyl-phenyl)amide
[23] 3-(4-chloro-phenyl)-1-oxa-2,8-diaza-spiro[4.5]dec-2-en-8-carboxylic acid-(2-propyl-phenyl)amide
[24] 3-(4-tert-butyl-phenyl)-1-oxa-2,8-diaza-spiro[4.5]dec-2-en-8-carboxylic acid-(2-propyl-phenyl)amide
[25] 3-p-tolyl-1-oxa-2,8-diaza-spiro[4.5]dec-2-en-8-carboylic acid-2)2-propyl-phenyl)amide
[26] 3-(4-trifluoromethyl-phenyl)-1-oxa-2,8-diaza-spiro[4.5]dec-2-en-8-carboxylic acid-(2-propyl-phenyl)amide
[27] 3-(3-methoxy-phenyl)-1-oxa-2,8-diaza-spiro[4.5]dec-2-en-8-carboxylic acid-(4-fluoro-phenyl)amide
[28] 3-phenyl-1-oxa-2,8-diaza-spiro[4.5]dec-2-en-8-carboxylic acid-(4-fluoro-phenyl)amide
[29] 3-(4-chloro-phenyl)-1-oxa-2,8-diaza-spiro[4.5]dec-2-en-8-carboxylic acid-(4-fluoro-phenyl)amide
[30] 3-(4-tert-butyl-phenyl)-1-oxa-2,8-diaza-spiro[4.5]dec-2-en-8-carboxylic acid-(4-fluoro-phenyl)amide
[31] 3-p-tolyl-1-oxa-2,8-diaza-spiro[4.5]dec-2-en-8-carboxylic acid-(4-fluoro-phenyl)amide
[32] 3-(4-trifluoromethyl-phenyl)-1-oxa-2,8-diaza-spiro[4.5]dec-2-en-8-carboxylic acid-(4-fluoro-phenyl)amide
[33] 3-(3-methoxy-phenyl)-1-oxa-2,8-diaza-spiro[4.5]dec-2-en-8-carboxylic acid-(3-chloro-phenyl)amide
[34] 3-phenyl-1-oxa-2,8-diaza-spiro[4.5]dec-2-en-8-carboxylic acid-(3-chloro-phenyl)amide
[35] 3-(4-chloro-phenyl)-1-oxa-2,8-diaza-spiro[4.5]dec-2-en-8-carboxylic acid-(3-chloro-phenyl)amide
[36] 3-(4-tert-butyl-phenyl)-1-oxa-2,8-diaza-spiro[4.5]dec-2-en-8-carboxylic acid-(3-chloro-phenyl)amide
[37] 3-tolyl-1-oxa-2,8-diaza-spiro[4.5]dec-2-en-8-carboxylic acid-(3-chloro-phenyl)amide
[38] 3-(4-trifluoromethyl-phenyl)-1-oxa-2,8-diaza-spiro[4.5]dec-2-en-8-carboxylic acid-(3-chloro-phenyl)amide
[39] 3-(3-methoxy-phenyl)-1-oxa-2,8-diaza-spiro[4.5]dec-2-en-8-carboxylic acid-(4-chloro-phenyl)amide
[40] 3-phenyl-1-oxa-2,8-diaza-spiro[4.5]dec-2-en-8-carboxylic acid-(4-chloro-phenyl)amide
[41] 3-(4-chloro-phenyl)-1-oxa-2,8-diaza-spiro[4.5]dec-2-en-8-carboxylic acid-(4-chloro-phenyl)amide
[42] 3-(4-tert-butyl-phenyl0-1-oxa-2,8-diaza-spiro[4.5]dec-2-en-8-carboxylic acid-(4-chloro-phenyl)amide
[43] 3-p-tolyl-1-oxa-2,8-diaza-spiro[4.5]dec-2-en-8-carboxylic acid-(4-chloro-phenyl)amide
[44] 3-phenyl-1-oxa-2,8-diaza-spiro[4.5]dec-2-en-8-carboxylic acid-(4-iodo-phenyl)amide
[45] 3-(4-tert-butyl-phenyl)-1-oxa-2,8-diaza-spiro[4.5]dec-2-en-8-carboxylic acid-(4-iodo-phenyl)amide
[46] 3-(4-trifluoromethyl-phenyl)-1-oxa-2,8-diaza-spiro[4.5]dec-2-en-8-carbocylic acid (4-iodo-phenyl)amide
[47] 3-(3-methoxy-phenyl)-1-oxa-2,8-diaza-spiro[4.5]dec-2-en-8-carboxylic acid-(4-methoxy-phenyl)amide
[48] 3-phenyl-1-oxa-2,8-diaza-spiro[4.5]dec-2-en-8-carboxylic acid-(4-methoxy-phenyl)amide
[49] 3-(4-chloro-phenyl)-1-oxa-2,8-diaza-spiro[4.5]dec-2-en-8-carboxylic acid-(4-methoxy-phenyl)amide
[50] 3-(4-tert-butyl-phenyl)-1-oxa-2,8-diaza-spiro[4.5]dec-2-en-8-carboxylic acid-(4-methoxy-phenyl)amide
[51] 3-p-tolyl-1-oxa-2,8-diaza-spiro[4.5]dec-2-en-8-carboxylic acid-(4-methoxy-phenyl)amide
[52] 3-(4-trifluoromethyl-phenyl)-1-oxa-2,8-diaza-spiro[4.5]dec-2-en-8-carboxylic acid-(4-methoxy-phenyl)amide
[53] 3-(3-methoxy-phenyl)-1-oxa-2,8-diaza-spiro[4.5]dec-2-en-8-carboxylic acid-(2-methylsulphanyl-phenyl)amide
[54] 3-phenyl-1-oxa-2,8-diaza-spiro[4.5]dec-2-en-8-carboxylic acid-(2-methylsulphanyl-phenyl)amide
[55] 3-(4-chloro-phenyl)-1-oxa-2,8-diaza-spiro[4.5]dec-2-en-8-carboxylic acid-(2-methylsulphanyl-phenyl)amide
[56] 3-(4-tert-butyl-phenyl)-1-oxa-2,8-diaza-spiro[4.5]dec-2-en-8-carboxylic acid-(2-methylsulphanyl-phenyl)amide
[57] 3-p-tolyl-1-oxa-2,8-diaza-spiro[4.5]dec-2-en-8-carboxylic acid-(2-methylsulphanyl-phenyl)amide
[58] 3-(4-trifluoromethyl-phenyl)-1-oxa-2,8-diaza-spiro[4.5]dec-2-en-8-carboxylic acid-(2-methylsulphanyl-phenyl)amide
[59] 3-(3-methoxy-phenyl)-1-oxa-2,8-diaza-spiro[4.5]dec-2-en-8-carboxylic acid-(3-methylsulphanyl-phenyl)amide
[60] 3-phenyl-1-oxa-2,8-diaza-spiro[4.5]dec-2-en-8-carboxylic acid-(3-methylsulphanyl-phenyl)amide
[61] 3-(4-chloro-phenyl)-1-oxa-2,8-diaza-spiro[4.5]dec-2-en-8-carboxylic acid-(3-methylsulphanyl-phenyl)amide
[62] 3-(4-tert-butyl-phenyl)-1-oxa-2,8-diaza-spiro[4.5]dec-2-en-8-carboxylic acid-(3-methylsulphanyl-phenyl)amide
[63] 3-p-tolyl-1-oxa-2,8-diaza-spiro[4.5]dec-2-en-8-carboxylic acid-(3-methylsulphanyl-phenyl)amide
[64] 3-(4-trifluoromethyl-phenyl)-1-oxa-2,8-diaza-spiro[4.5]dec-2-en-8-carboxylic acid-(3-methylsulphanyl-phenyl)amide
[65] 3-(3-methoxy-phenyl)-1-oxa-2,8-diaza-spiro[4.5]dec-2-en-8-carboxylic acid-(4-methylsulphanyl-phenyl)amide
[66] 3-phenyl-1-oxa-2,8-diaza-spiro[4.5]dec-2-en-8-carboxylic acid-(4-methylsulphanyl-phenyl)amide
[67] 3-(4-chloro-phenyl)-1-oxa-2,8-diaza-spiro[4.5]dec-2-en-8-carboxylic acid-(4-methylsulphanyl-phenyl)amide
[68] 3-(4-tert-butyl-phenyl)-1-oxa-2,8-diaza-spiro[4.5]dec-2-en-8-carboxylic acid-(4-methylsulphanyl-phenyl)amide
[69] 3-p-tolyl-1-oxa-2,8-diaza-spiro[4.5]dec-2-en-8-carboxylic acid-(4-methylsulphanyl-phenyl)amide
[70] 3-(4-trifluoromethyl-phenyl)-1-oxa-2,8-diaza-spiro[4.5]dec-2-en-8-carboxylic acid-(4-methylsulphanyl-phenyl)amide
[71] 3-(3-methoxy-phenyl)-1-oxa-2,8-diaza-spiro[4.5]dec-2-en-8-carboxylic acid-(2-isopropyl-phenyl)amide
[72] 3-phenyl-1-oxa-2,8-diaza-spiro[4.5]dec-2-en-8-carboxylic acid-(2-isopropyl-phenyl)amide
[73] 3-(4-chloro-phenyl)-1-oxa-2,8-diaza-spiro[4.5]dec-2-en-8-carboxylic acid-(2-isopropyl-phenyl)amide
[74] 3-(4-tert-butyl-phenyl)-1-oxa-2,8-diaza-spiro[4.5]dec-2-en-8-carboxylic acid-(2-isopropyl-phenyl)amide
[75] 3-p-tolyl-1-oxa-2,8-diaza-spiro[4.5]dec-2-en-8-carboxylic acid-(2-isopropyl-phenyl)amide
[76] 3-(4-trifluoromethyl-phenyl)-1-oxa-2,8-diaza-spiro[4.5]dec-2-en-8-carboxylic acid-(2-isopropyl-phenyl)amide
[77] 3-(3-methoxy-phenyl)-1-oxa-2,8-diaza-spiro[4.5]dec-2-en-8-carboxylic acid-(4-isopropyl-phenyl)amide
[78] 3-phenyl-1-oxa-2,8-diaza-spiro[4.5]dec-2-en-8-carboxylic acid-(4-isopropyl-phenyl)amide
[79] 3-(4-chloro-phenyl)-1-oxa-2,8-diaza-spiro[4.5]dec-2-en-8-carboxylic acid-(4-isopropyl-phenyl)amide)
[80] 3-(4-tert-butyl-phenyl)-1-oxa-2.8-diaza-spiro[4.5]dec-2-en-8-carboxylic acid-(4-isopropyl-phenyl)amide
[81] 3-p-tolyl-1-oxa-2,8-diaza-spiro[4.5]dec-2-en-8-carboxylic acid-(4-isopropyl-phenyl)amide
[82] 3-(4-trifluoromethyl-phenyl)-1-oxa-2,8-diaza-spiro[4.5]dec-2-en-8-carboxylic acid-(4-isopropyl-phenyl)amide
[83] 3-(3-methoxy-phenyl)-1-oxa-2,8-diaza-spiro[4.5]dec-2-en-8-carboxylic acid-(2-trifluoromethyl-phenyl)amide
[84] 3-phenyl-1-oxa-2,8-diaza-spiro[4.5]dec-2-en-8-carboxylic acid-(2-trifluoromethyl-phenyl)amide
[85] 3-(4-chloro-phenyl)-1-oxa-2,8-diaza-spiro[4.5]dec-2-en-8-carboxylic acid-(2-trifluoromethyl-phenyl)amide
[86] 3-(4-tert-butyl-phenyl)-1-oxa-2,8-diaza-spiro[4.5]dec-2-en-8-carboxylic acid-(2-trifluoromethyl-phenyl)amide
[87] 3-p-tolyl-1-oxa-2,8-diaza-spiro[4.5]dec-2-en-8-carboxylic acid-(2-trifluoromethyl-phenyl)amide
[88] 3-(4-trifluoromethyl-phenyl)-1-oxa-2,8-diaza-spiro[4.5]dec-2-en-8-carboxylic acid-(2-trifluoromethyl-phenyl)amide
[89] 3-(3-methoxy-phenyl)-1-oxa-2,8-diaza-spiro[4.5]dec-2-en-8-carboxylic acid-(3-trifluoromethyl-phenyl)amide
[90] 3-phenyl-1-oxa-2,8-diaza-spiro[4.5]dec-2-en-8-carboxylic acid-(3-trifluoromethyl-phenyl)amide
[91] 3-(4-chloro-phenyl)-1-oxa-2,8-diaza-spiro[4.5]dec-2-en-8-carboxylic acid-(3-trifluoromethyl-phenyl)amide
[92] 3-(4-tert-butyl-phenyl)-1-oxa-2,8-diaza-spiro[4.5]dec-2-en-8-carboxylic acid-(3-trifluoromethyl-phenyl)amide
[93] 3-p-tolyl-1-oxa-2,8-diaza-spiro[4.5]dec-2-en-8-carboxylic acid-(3-trifluoromethyl-phenyl)amide
[94] 3-(4-trifluoromethyl-phenyl)-1-oxa-2,8-diaza-spiro[4.5]dec-2-en-8-carboxylic acid-(3-trifluoromethyl-phenyl)amide
[95] 3-phenyl-1-oxa-2,8-diaza-spiro[4.5]dec-2-en-8-carboxylic acid biphenyl-4-ylamide
[96] 3-(4-tert-butyl-phenyl)-1-oxa-2,8-diaza-spiro[4.5]dec-2-en-8-carboxylic acid biphenyl-4-ylamide
[97] 3-p-tolyl-1-oxa-2,8-diaza-spiro[4.5]dec-2-en-8-carboxylic acid biphenyl-4-ylamide
[98] 3-(3-methoxy-phenyl)-1-oxa-2,8-diaza-spiro[4.5]dec-2-en-8-carboxylic acid-(2-phenoxy-phenyl)amide
[99] 3-phenyl-1-oxa-2,8-diaza-spiro[4.5]dec-2-en-8-carboxylic acid-(2-phenoxy-phenyl)amide
[100] 3-(4-chloro-phenyl)-1-oxa-2,8-diaza-spiro[4.5]dec-2-en-8-carboxylic acid-(2-phenoxy-phenyl)amide
[101] 3-(4-tert-butyl-phenyl)-1-oxa-2,8-diaza-spiro[4.5]dec-2-en-8-carboxylic acid-(2-phenoxy-phenyl)amide
[102] 3-p-tolyl-1-oxa-2,8-diaza-spiro[4.5]dec-2-en-8-carboxylic acid-(2-phenoxy-phenyl)amide
[103] 3-(4-trifluoromethyl-phenyl)-1-oxa-2,8-diaza-spiro[4.5]dec-2-en-8-carboxylic acid-(2-phenoxy-phenyl)amide
[104] 3-(3-methoxy-phenyl) -1-oxa-2,8-diaza-spiro[4.5]dec-2-en-8-carboxylic acid-(4-benzyloxy-phenyl)amide
[105] 3-phenyl-1-oxa-2,8-diaza-spiro[4.5]dec-2-en-8-carboxylic acid-(4-benzyloxy-phenyl)amide
[106] 3-(4-chloro-phenyl)-1-oxa-2,8-diaza-spiro[4.5]dec-2-en-8-carboxylic acid-(4-benzyloxy-phenyl)amide
[107] 3-(4-tert-butyl-phenyl)-1-oxa-2,8-diaza-spiro[4.5]dec-2-en-8-carboxylic acid-(4-benzyloxy-phenyl)amide
[108] 3-p-tolyl-1-oxa-2,8-diaza-spiro[4.5]dec-2-en-8-carboxylic acid-(4-benzyloxy-phenyl)amide
[109] 3-(4-trifluoromethyl-phenyl)-1-oxa-2,8-diaza-spiro[4.5]dec-2-en-8-carboxylic acid-(4-benzyloxy-phenyl)amide
[110] 3-(3-methoxy-phenyl)-1-oxa-2,8-diaza-spiro[4.5]dec-2-en-8-carboxylic acid cyclohexylamide
[111] 3-phenyl-1-oxa-2,8-diaza-spiro[4.5]dec-2-en-8-carboxylic acid cyclohexylamide
[112] 3-(4-chloro-phenyl)-1-oxa-2,8-diaza-spiro[4.5]dec-2-en-8-carboxylic acid cyclohexylamide
[113] 3-(4-tert-butyl-phenyl)-1-oxa-2,8-diaza-spiro[4.5]dec-2-en-8-carboxylic acid cyclohexylamide
[114] 3-p-tolyl-1-oxa-2,8-diaza-spiro[4.5]dec-2-en-8-carboxylic acid cyclohexylamide
[115] 3-(4-trifluoromethyl-phenyl)-1-oxa-2,8-diaza-spiro[4.5]dec-2-en-8-carboxylic acid cyclohexylamide
[116] 3-(3-methoxy-phenyl)-1-oxa-2,8-diaza-spiro[4.5]dec-2-en-8-carboxylic acid benzylamide
[117] 3-phenyl-1-oxa-2,8-diaza-spiro[4.5]dec-2-en-8-carboxylic acid benzylamide
[118] 3-(4-chloro-phenyl)-1-oxa-2,8-diaza-spiro[4.5]dec-2-en-8-carboxylic acid benzylamide
[119] 3-(4-tert-butyl-phenyl)-1-oxa-2,8-diaza-spiro[4.5]dec-2-en-8-carboxylic acid benzylamide
[120] 3-p-tolyl-1-oxa-2,8-diaza-spiro[4.5]dec-2-en-8-carboxylic acid benzylamide
[121] 3-(4-trifluoromethyl-phenyl)-1-oxa-2,8-diaza-spiro[4.5]dec-2-en-8-carboxylic acid benzylamide
[122] 3-(3-methoxy-phenyl)-1-oxa-2,8-diaza-spiro[4.5]dec-2-en-8-carboxylic acid phenethylamide
[123] 3-phenyl-1-oxa-2,8-diaza-spiro[4.5]dec-2-en-8-carboxylic acid phenethylamide
[124] 3-(4-chloro-phenyl)-1-oxa-2,8-diaza-spiro[4.5]dec-2-en-8-carboxylic acid phenethylamide
[125] 3-(4-tert-butyl-phenyl)-1-oxa-2,8-diaza-spiro[4.5]dec-2-en-8-carboxylic acid phenethylamide
[126] 3-p-tolyl-1-oxa-2,8-diaza-spiro[4.5]dec-2-en-8-carboxylic acid phenethylamide
[127] 3-(4-trifluoromethyl-phenyl)-1-oxa-2,8-diaza-spiro[4.5]dec-2-en-8-carboxylic acid phenthylamide
[128] 3-(3-methoxy-phenyl)-1-oxa-2,8-diaza-spiro[4.5]dec-2-en-8-carboxylic acid-4-methyl-benzylamide
[129] 3-phenyl-1-oxa-2,8-diaza-spiro[4.5]dec-2-en-8-carboxylic acid-4-methyl-benzylamide
[130] 3-(4-chloro-phenyl)-1-oxa-2,8-diaza-spiro[4.5]dec-2-en-8-carboxylic acid-4-methyl-benzylamide
[131] 3-(4-tert-butyl-phenyl)-1-oxa-2,8-diaza-spiro[4.5]dec-2-en-8-carboxylic acid-4-methyl-benzylamide
[132] 3-p-tolyl-1-oxa-2,8-diaza-spiro[4.5]dec-2-en-8-carboxylic acid-4-methyl-benzylamide
[133] 3-(4-trifluoromethyl-phenyl)-1-oxa-2,8-diaza-spiro[4.5]dec-2-en-8-carboxylic acid-4-methyl-benzylamide
[134] 3-(3-methoxy-phenyl)-1-oxa-2,8-diaza-spiro[4.5]dec-2-en-8-carboxylic acid-4-methoxy-benzylamide
[135] 3-phenyl-1-oxa-2,8-diaza-spiro[4.5]dec-2-en-8-carboxylic acid-4-methoxy-benzylamide
[136] 3-(4-chloro-phenyl)-1-oxa-2,8-diaza-spiro[4.5]dec-2-en-8-carboxylic acid-4-methoxy-benzylamide
[137] 3-(4-tert-butyl-phenyl)-1-oxa-2,8-diaza-spiro[4.5]dec-2-en-8-carboxylic acid-4-methoxy-benzylamide
[138] 3-p-tolyl-1-oxa-2,8-diaza-spiro[4.5]dec-2-en-8-carboxylic acid-4-methoxy-benzylamide
[139] 3-(4-trifluoromethyl-phenyl)-1-oxa-2,8-diaza-spiro[4.5]dec-2-en-8-carboxylic acid-4-methoxy-benzylamide
[140] 3-(3-methoxy-phenyl)-1-oxa-2,8-diaza-spiro[4.5]dec-2-en-8-carboxylic acid-(4-tert-butyl-phenyl)amide
[141] 3-phenyl-1-oxa-2,8-diaza-spiro[4.5]dec-2-en-8-carboxylic acid-(4-tert-butyl-phenyl)amide
[142] 3-(4-chloro-phenyl)-1-oxa-2,8-diaza-spiro[4.5]dec-2-en-8-carboxylic acid-(4-tert-butyl-phenyl)amide
[143] 3-(4-tert-butyl-phenyl)-1-oxa-2,8-diaza-spiro[4.5]dec-2-en-8-carboxylic acid-4-tert-butyl-phenyl)amide
[144] 3-p-tolyl-1-oxa-2,8-diaza-spiro[4.5]dec-2-en-8-carboxylic acid-(4-tert-butyl-phenyl)amide
[145] 3-(4-trifluoromethyl-phenyl)-1-oxa-2,8-diaza-spiro[4.5]dec-2-en-8-carboxylic acid-(4-tert-butyl-phenyl)amide
[146] 3-(3-methoxy-phenyl)-1-oxa-2,8-diaza-spiro[4.5]dec-2-en-8-carboxylic acid-p-tolylamide
[147] 3-phenyl-1-oxa-2,8-diaza-spiro[4.5]dec-2-en-8-carboxylic acid-p-tolylamide
[148] 3-(4-chloro-phenyl)-1-oxa-2,8-diaza-spiro[4.5]dec-2-en-8-carboxylic acid-p-tolylamide
[149] 3-(4-tert-butyl-phenyl) -1-oxa-2,8-diaza-spiro[4.5]dec-2-en-8-carboxylic acid-p-tolylamide
[150] 3-(3-methoxy-phenyl)-1-oxa-2,8-diaza-spiro[4.5]dec-2-en-8-carboxylic acid-o-tolylamide
[151] 3-phenyl-1-oxa-2,8-diaza-spiro[4.5]dec-2-en-8-carboxylic acid-o-tolylamide
[152] 3-(4-chloro-phenyl)-1-oxa-2,8-diaza-spiro[4.5]dec-2-en-8-carboxylic acid-o-tolylamide
[153] 3-(4-tert-butyl-phenyl)-1-oxa-2,8-diaza-spiro[4.5]dec-2-en-8-carboxylic acid-o-tolylamide
[154] 3-p-tolyl-1-oxa-2,8-diaza-spiro[4.5]dec-2-en-8-carboxylic acid-o-tolylamide
[155] 3-(4-trifluoromethyl-phenyl)-1-oxa-2,8-diaza-spiro[4.5]dec-2-en-8-carboxylic acid-o-tolylamide
[156] 3-(3-methoxy-phenyl)-1-oxa-2,8-diaza-spiro[4.5]dec-2-en-8-carboxylic acid-(2-ethyl-phenyl)amide
[157] 3-phenyl-1-oxa-2,8-diaza-spiro[4.5]dec-2-en-8-carboxylic acid-(3-ethyl-phenyl)amide
[158] 3-(4-chloro-phenyl)-1-oxa-2,8-diaza-spiro[4.5]dec-2-en-8-carboxylic acid-(2-ethyl-phenyl)amide
[159] 3-(4-tert-butyl-phenyl)-1-oxa-2,8-diaza-spiro[4.5]dec-2-en-8-carboxylic acid-(2-ethyl-phenyl)amide
[160] 3-p-tolyl-1-oxa-2,8-diaza-spiro[4.5]dec-2-en-8-carboxylic acid-(2-ethyl-phenyl)amide
[161] 3-(4-trifluoromethyl-phenyl)-1-oxa-2,8-diaza-spiro[4.5]dec-2-en-8-carboxylic acid-(2-ethyl-phenyl)amide
[162] 3-(3-methoxy-phenyl)-1-oxa-2,8-diaza-spiro[4.5]dec-2-en-8-carboxylic acid-(4-ethyl-phenyl)amide
[163] 3-phenyl-1-oxa-2,8-diaza-spiro[4.5]dec-2-en-8-carboxylic acid-(4-ethyl-phenyl)amide
[164] 3-(4-chloro-phenyl)-1-oxa-2,8-diaza-spiro[4.5]dec-2-en-8-carboxylic acid-(4-ethyl-phenyl)amide
[165] 3-(4-tert-butyl-phenyl)-1-oxa-2,8-diaza-spiro[4.5]dec-2-en-8-carboxylic acid-(4-ethyl-phenyl)amide
[166] 3-p-tolyl-1-oxa-2,8-diaza-spiro[4.5]dec-2-en-8-carboxylic acid-(4-ethyl-phenyl)amide
[167] 3-(4-trifluoromethyl-phenyl)-1-oxa-2,8-diaza-spiro[4.5]dec-2-en-8-carboxylic acid-(4-ethyl-phenyl)amide
[168] 3-(3-methoxy-phenyl)-1-oxa-2,8-diaza-spiro[4.5]dec-2-en-8-carboxylic acid-(2-fluoro-phenyl)amide
[169] 3-phenyl-1-oxa-2,8-diaza-spiro[4.5]dec-2-en-8-carboxylic acid-(2-fluoro-phenyl)amide
[170] 3-(4-chloro-phenyl)-1-oxa-2,8-diaza-spiro[4.5]dec-2-en-8-carboxylic acid-(2-fluoro-phenyl)amide
[171] 3-(4-tert-butyl-phenyl)-1-oxa-2,8-diaza-spiro[4.5]dec-2-en-8-carboxylic acid-(2-fluoro-phenyl)amide
[172] 3-p-tolyl-1-oxa-2,8-diaza-spiro[4.5]dec-2-en-8-carboxylic acid-(2-fluoro-phenyl)amide
[173] 3-(4-trifluoromethyl-phenyl)-1-oxa-2,8-diaza-spiro[4.5]dec-2-en-8-carboxylic acid-2-fluoro-phenyl)amide
[174] 3-(3-methoxy-phenyl)-1-oxa-2,8-diaza-spiro[4.5]dec-2-en-8-carboxylic acid-(3-fluoro-phenyl)amide
[175] 3-phenyl-1-oxa-2,8-diaza-spiro[4.5]dec-2-en-8-carboxylic acid-(3-fluoro-phenyl)amide
[176] 3-(4-chloro-phenyl)-1-oxa-2,8-diaza-spiro[4.5]dec-2-en-8-carboxylic acid-(3-fluoro-phenyl)amide
[177] 3-(4-tert-butyl-phenyl)-1-oxa-2,8-diaza-spiro[4.5]dec-2-en-8-carboxylic acid-(3-fluoro-phenyl)amide
[178] 3-p-tolyl-1-oxa-2,8-diaza-spiro[4.5]dec-2-en-8-carboxylic acid-(3-fluoro-phenyl)amide
[179] 3-(4-trifluoromethyl-phenyl)-1-oxa-2,8-diaza-spiro[4.5]dec-2-en-8-carboxylic acid-(3-fluoro-phenyl)amide
[180] 3-(3-methoxy-phenyl)-1-oxa-2,8-diaza-spiro[4.5]dec-2-en-8-carboxylic acid-(2-chloro-phenyl)amide
[181] 3-phenyl-1-oxa-2,8-diaza-spiro[4.5]dec-2-en-8-carboxylic acid-(2-chloro-phenyl)amide
[182] 3-(4-chloro-phenyl) -1-oxa-2,8-diaza-spiro[4.5]dec-2-en-8-carboxylic acid-(2-chloro-phenyl)amide
[183] 3-(4-tert-butyl-phenyl)-1-oxa-2,8-diaza-spiro[4.5]dec-2-en-8-carboxylic acid-(2-chloro-phenyl)amide
[184] 3-p-tolyl-1-oxa-2,8-diaza-spiro[4.5]dec-2-en-8-carboxylic acid-(2-chloro-phenyl)amide
[185] 3-(4-trifluoromethyl-phenyl)-1-oxa-2,8-diaza-spiro[4.5]dec-2-en-8-carboxylic acid-(2-chloro-phenyl)amide
[186] 3-(3-methoxy-phenyl)-1-oxa-2,8-diaza-spiro[4.5]dec-2-en-8-carboxylic acid-(2-bromo-phenyl)amide
[187] 3-phenyl-1-oxa-2,8-diaza-spiro[4.5]dec-2-en-8-carboxylic acid-(2-bromo-phenyl)amide
[188] 3-(4-chloro-phenyl)-1-oxa-2,8-diaza-spiro[4.5]dec-2-en-8-carboxylic acid-(2-bromo-phenyl)amide
[189] 3-(4-tert-butyl-phenyl)-1-oxa-2,8-diaza-spiro[4.5]dec-2-en-8-carboxylic acid-(2-bromo-phenyl)amide
[190] 3-p-tolyl-1-oxa-2,8-diaza-spiro[4.5]dec-2-en-8-carboxylic acid-(2-bromo-phenyl)amide
[191] 3-(4-trifluoromethyl-phenyl)-1-oxa-2,8-diaza-spiro[4.5]dec-2-en-8-carboxylic acid-(2-bromo-phenyl)amide
[192] 3-(3-methoxy-phenyl)-1-oxa-2,8-diaza-spiro[4.5]dec-2-en-8-carboxylic acid-(3-bromo-phenyl)amide
[193] 3-phenyl-1-oxa-2,8-diaza-spiro[4.5]dec-2-en-8-carboxylic acid-(3-bromo-phenyl)amide
[194] 3-(4-tert-butyl-phenyl)-1-oxa-2,8-diaza-spiro[4.5]dec-2-en-8-carboxylic acid-(3-bromo-phenyl)amide
[195] 3-(4-trifluoromethyl-phenyl)-1-oxa-2,8-diaza-spiro[4.5]dec-2-en-8-carboxylic acid-(3-bromo-phenyl)amide
[196] 3-(3-methoxy-phenyl)-1-oxa-2,8-diaza-spiro[4.5]dec-2-en-8-carboxylic acid-(4-bromo-phenyl)amide
[197] 3-phenyl-1-oxa-2,8-diaza-spiro[4.5]dec-2-en-8-carboxylic acid-(4-bromo-phenyl)amide
[198] 3-(3-methoxy-phenyl)-1-oxa-2,8-diaza-spiro[4.5]dec-2-en-8-carboxylic acid-(2-methoxyl-phenyl)amide
[199] 3-phenyl-1-oxa-2,8-diaza-spiro[4.5]dec-2-en-8-carboxylic acid-(2-methoxy-phenyl)amide
[200] 3-(4-chloro-phenyl)-1-oxa-2,8-diaza-spiro[4.5]dec-2-en-8-carboxylic acid-(2-methoxy-phenyl)amide
[201] 3-(4-tert-butyl-phenyl)-1-oxa-2,8-diaza-spiro[4.5]dec-2-en-8-carboxylic acid-(2-methoxy-phenyl)amide
[202] 3-p-tolyl-1-oxa-2,8-diaza-spiro[4.5]dec-2-en-8-carboxylic acid-(2-methoxy-phenyl)amide
[203] 3-(4-trifluoromethyl-phenyl)-1-oxa-2,8-diaza-spiro[4.5]dec-2-en-8-carboxylic acid-(2-methoxy-phenyl)amide
[204] 3-(3-methoxy-phenyl)-1-oxa-2,8-diaza-spiro[4.5]dec-2-en-8-carboxylic acid-(3-methoxy-phenyl)amide
[205] 3-phenyl-1-oxa-2,8-diaza-spiro[4.5]dec-2-en-8-carboxylic acid-(3-methoxy-phenyl)amide
[206] 3-(4-chloro-phenyl)-1-oxa-2,8-diaza-spiro[4.5]dec-2-en-8-carboxylic acid-(3-methoxy-phenyl)amide
[207] 3-(4-tert-butyl-phenyl)-1-oxa-2,8-diaza-spiro[4.5]dec-2-en-8-carboxylic acid-(3-methoxy-phenyl)amide
[208] 3-p-tolyl-1-oxa-2,8-diaza-spiro[4.5]dec-2-en-8-carboxylic acid-(3-methoxy-phenyl)amide
[209] 3-(4-trifluoromethyl-phenyl)-1-oxa-2,8-diaza-spiro[4.5]dec-2-en-8-carboxylic acid-(3-methoxy-phenyl)amide
[210] 3-(3-methoxy-phenyl)-1-oxa-2,8-diaza-spiro[4.5]dec-2-en-8-carboxylic acid-(4-trifluoromethyl-phenyl)amide
[211] 3-phenyl-1-oxa-2,8-diaza-spiro[4.5]dec-2-en-8-carboxylic acid-(4-trifluoromethyl-phenyl)amide
[212] 3-(4-chloro-phenyl)-1-oxa-2,8-diaza-spiro[4.5]dec-2-en-8-carboxylic acid-(4-trifluoromethyl-phenyl)amide
[213] 3-(4-tert-butyl-phenyl)-1-oxa-2,8-diaza-spiro[4.5]dec-2-en-8-carboxylic acid-(4-trifluoromethyl-phenyl)amide
[214] 3-p-tolyl-1-oxa-2,8-diaza-spiro[4.5]dec-2-en-8-carboxylic acid-(4-trifluoromethyl-phenyl)amide
[215] 3-(4-trifluoromethyl-phenyl)-1-oxa-2,8-diaza-spiro[4.5]dec-2-en-8-carboxylic acid-4-trifluoromethyl-phenyl)amide
[216] 3-(3-methoxy-phenyl)-1-oxa-2,8-diaza-spiro[4.5]dec-2-en-8-carboxylic acid-(4-phenoxy-phenyl)amide
[217] 3-(4-chloro-phenyl)-1-oxa-2,8-diaza-spiro[4.5]dec-2-en-8-carboxylic acid-(4-phenoxy-phenyl)amide
[218] 3-(4-tert-butyl-phenyl)-1-oxa-2,8-diaza-spiro[4.5]dec-2-en-8-carboxylic acid-(4-phenoxy-phenyl)amide
[219] 3-p-tolyl-1-oxa-2,8-diaza-spiro[4.5]dec-2-en-8-carboxylic acid-(4-phenoxy-phenyl)amide
[220] 3-(4-trifluoromethyl-phenyl)-1-oxa-2,8-diaza-spiro[4.5]dec-2-en-8-carboxylic acid-(4-phenoxy-phenyl)amide
[221] 3-(3-methoxy-phenyl)-1-oxa-2,8-diaza-spiro[4.5]dec-2-en-8-carboxylic acid-(2-chloro-5-trifluoromethyl-phenyl)amide
[222] 3-phenyl-1-oxa-2,8-diaza-spiro[4.5]dec-2-en-8-carboxylic acid-(2-chloro-5-trifluoromethyl-phenyl)amide
[223] 3-(4-chloro-phenyl)-1-oxa-2,8-diaza-spiro[4.5]dec-2-en-8-carboxylic acid-(2-chloro-5-trifluoromethyl-phenyl)amide
[224] 3-(4-tert-butyl-phenyl)-1-oxa-2,8-diaza-spiro[4.5]dec-2-en-8-carboxylic acid-(2-chloro-5-trifluoromethyl-phenyl)amide
[225] 3-p-tolyl-1-oxa-2,8-diaza-spiro[4.5]dec-2-en-8-carboxylic acid-(2-chloro-5-trifluoromethyl-phenyl)amide
[226] 3-(4-trifluoromethyl-phenyl)-1-oxa-2,8-diaza-spiro[4.5]dec-2-en-8-carboxylic acid-(2-chloro-5-trifluoromethyl-phenyl)amide
[227] 3-(3-methoxy-phenyl)-1-oxa-2,8-diaza-spiro[4.5]dec-2-en-8-carboxylic acid-(4-chloro-2-trifluoromethyl-phenyl)amide
[228] 3-phenyl-1-oxa-2,8-diaza-spiro[4.5]dec-2-en-8-carboxylic acid-(4-chloro-2-trifluoromethyl-phenyl)amide
[229] 3-(4-chloro-phenyl)-1-oxa-2,8-diaza-spiro[4.5]dec-2-en-8-carboxylic acid-(4-chloro-2-trifluoromethyl-phenyl)amide
[230] 3-(4-tert-butyl-phenyl)-1-oxa-2,8-diaza-spiro[4.5]dec-2-en-8-carboxylic acid-(4-chloro2-trifluoromethyl-phenyl)amide
[231] 3-p-tolyl-1-oxa-2,8-diaza-spiro[4.5]dec-2-en-8-carboxylic acid-(4-chloro-2-trifluoromethyl-phenyl)amide
[232] 3-(3-methoxy-phenyl)-1-oxa-2,8-diaza-spiro[4.5]dec-2-en-8-carboxylic acid-4-chloro-3-trifluoromethyl-phenyl)amide
[233] 3-phenyl-1-oxa-2,8-diaza-spiro[4.5]dec-2-en-8-carboxylic acid-(4-chloro-3-trifluoromethyl-phenyl)amide
[234] 3-(4-chloro-phenyl)-1-oxa-2,8-diaza-spiro[4.5]dec-2-en-8-carboxylic acid-(4-chloro-3-trifluoromethyl-phenyl)amide
[235] 3-(4-tert-butyl-phenyl)-1-oxa-2,8-diaza-spiro[4.5]dec-2-en-8-carboxylic acid-(4-chloro-3-trifluoromethyl-phenyl)amide
[236] 3-p-tolyl-1-oxa-2,8-diaza-spiro[4.5]dec-2-en-8-carboxylic acid-(4-chloro-3-trifluoromethyl-phenyl)amide
[237] 3-(4-trifluoromethyl-phenyl)-1-oxa-2,8-diaza-spiro[4.5]dec-2-en-8-carboxylic acid-(4-chloro-3-trifluoromethyl-phenyl)amide
[238] 3-(3-methoxy-phenyl)-1-oxa-2,8-diaza-spiro[4.5]dec-2-en-8-carboxylic acid-(2-tert-butyl-6-methyl-phenyl)amide
[239] 3-phenyl-1-oxa-2,8-diaza-spiro[4.5]dec-2-en-8-carboxylic acid-(2-tert-butyl-6-methyl-phenyl)amide
[240] 3-(4-chloro-phenyl)-1-oxa-2,8-diaza-spiro[4.5]dec-2-en-8-carboxylic acid-(2-tert-butyl-6-methyl-phenyl)amide
[241] 3-(4-tert-butyl-phenyl)-1-oxa-2,8-diaza-spiro[4.5]dec-2-en-8-carboxylic acid-(2-tert-butyl-6-methyl-phenyl)amide
[242] 3-p-tolyl-1-oxa-2,8-diaza-spiro[4.5]dec-2-en-8-carboxylic acid-(2-tert-butyl-6-methyl-phenyl)amide
[243] 3-(4-trifluoromethyl-phenyl)-1-oxa-2,8-diaza-spiro[4.5]dec-2-en-8-carboxylic acid-(2-tert-butyl-6-methyl-phenyl)amide
[244] 3-(3-methoxy-phenyl)-1-oxa-2,8-diaza-spiro[4.5]dec-2-en-8-carboxylic acid-(4-trifluoromethoxy-phenyl)amide
[245] 3-phenyl-1-oxa-2,8-diaza-spiro[4.5]dec-2-en-8-carboxylic acid-(4-trifluoromethoxy-phenyl)amide
[246] 3-(4-chloro-phenyl)-1-oxa-2,8-diaza-spiro[4.5]dec-2-en-8-carboxylic acid-(4-trifluoromethoxy-phenyl)amide
[247] 3-(4-tert-butyl-phenyl)-1-oxa-2,8-diaza-spiro[4.5]dec-2-en-8-carboxylic acid-(4-trifluoromethoxy-phenyl)amide
[248] 3-p-tolyl-1-oxa-2,8-diaza-spiro[4.5]dec-2-en-8-carboxylic acid-(4-trifluoromethoxyl-phenyl)amide
[249] 3-(3-methoxy-phenyl)-1-oxa-2,8-diaza-spiro[4.5]dec-2-en-8-carboxylic thioacid cyclohexylamide
[250] 3-phenyl-1-oxa-2,8-diaza-spiro[4.5]dec-2-en-8-carboxylic thioacid cyclohexylamide
[251] 3-(4-chloro-phenyl)-1-oxa-2,8-diaza-spiro[4.5]dec-2-en-8-carboxylic thioacid cyclohexylamide
[252] 3-(4-tert-butyl-phenyl) -1-oxa-2,8-diaza-spiro[4.5]dec-2-en-8-carboxylic thioacid cyclohexylamide
[253] 3-p-tolyl-1-oxa-2,8-diaza-spiro[4.5]dec-2-en-8-carboxylic thioacid cyclohexylamide
[254] 3-(4-trifluoromethyl-phenyl)-1-oxa-2,8-diaza-spiro[4.5]dec-2-en-8-carboxylic thioacid cyclohexylamide
[255] 3-(3-methoxy-phenyl)-1-oxa-2,8-diaza-spiro[4.5]dec-2-en-8-carboxylic thioacid phenylamide
[256] 3-phenyl-1-oxa-2,8-diaza-spiro[4.5]dec-2-en-8-carboxylic thioacid phenylamide
[257] 3-(4-chloro-phenyl) -1-oxa-2,8-diaza-spiro[4.5]dec-2-en-8-carboxylic thioacid phenylamide
[258] 3-(4-tert-butyl-phenyl)-1-oxa-2,8-diaza-spiro[4.5]dec-2-en-8-carboxylic thioacid phenylamide
[259] 3-p-tolyl-1-oxa-2,8-diaza-spiro[4.5]dec-2-en-8-carboxylic thioacid phenylamide
[260] 3-(4-trifluoromethyl-phenyl)-1-oxa-2,8-diaza-spiro[4.5]dec-2-en-8-carboxylic thioacid phenylamide
[261] 3-(3-methoxy-phenyl)-1-oxa-2,8-diaza-spiro[4.5]dec-2-en-8-carboxylic thioacid-(3-trifluoromethyl-phenyl)amide
[262] 3-phenyl-1-oxa-2,8-diaza-spiro[4.5]dec-2-en-8-carboxylic thioacid-(3-trifluoromethyl-phenyl)amide
[263] 3-(4-chloro-phenyl)-1-oxa-2,8-diaza-spiro[4.5]dec-2-en-8-carboxylic thioacid-(3-trifluoromethyl-phenyl)amide
[264] 3-(4-tert-butyl-phenyl)-1-oxa-2,8-diaza-spiro[4.5]dec-2-en-8-carboxylic thioacid-(3-trifluoromethyl-phenyl)amide
[265] 3-p-tolyl-1-oxa-2,8-diaza-spiro[4.5]dec-2-en-8-carboxylic thioacid-(3-trifluoromethyl-phenyl)amide
[266] 3-(4-trifluoromethyl-phenyl)-1-oxa-2,8-diaza-spiro[4.5]dec-2-en-8-carboxylic thioacid-(3-trifluoromethyl-phenyl)amide
[267] 3-(3-methoxy-phenyl)-1-oxa-2,8-diaza-spiro[4.5]dec-2-en-8-carboxylic thioacid-(2-methoxy-phenyl)amide
[268] 3-phenyl-1-oxa-2,8-diaza-spiro[4.5]dec-2-en-8-carboxylic thioacid-(2-methoxy-phenyl)amide
[269] 3-(4-chloro-phenyl)-1-oxa-2,8-diaza-spiro[4.5]dec-2-en-8-carboxylic thioacid-(2-methoxy-phenyl)amide
[270] 3-(4-tert-butyl)-1-oxa-2,8-diaza-spiro[4.5]dec-2-en-8-carboxylic thioacid-(2-methoxy-phenyl)amide
[271] 3-p-tolyl-1-oxa-2,8-diaza-spiro[4.5]dec-2-en-8-carboxylic thioacid-(2-methoxy-phenyl)amide
[272] 3-(4-trifluoromethyl-phenyl)-1-oxa-2,8-diaza-spiro[4.5]dec-2-en-8-carboxylic thioacid-(2-methoxy-phenyl)amide
[273] 3-(3-methoxy-phenyl)-1-oxa-2,8-diaza-spiro[4.5]dec-2-en-8-carboxylic thioacid-(3-methoxy-phenyl)amide
[274] 3-phenyl-1-oxa-2,8-diaza-spiro[4.5]dec-2-en-8-carboxylic thioacid-(3-methoxy-phenyl)amide
[275] 3-(4-chloro-phenyl)-1-oxa-2,8-diaza-spiro[4.5]dec-2-en-8-carboxylic thioacid-(3-methoxy-phenyl)amide
[276] 3-p-tolyl-1-oxa-2,8-diaza-spiro[4.5]dec-2-en-8-carboxylic thioacid-(3-methoxy-phenyl)amide
[277] 3-(4-trifluoromethyl-phenyl)-1-oxa-2,8-diaza-spiro[4.5]dec-2-en-8-carboxylic thioacid-(3-methoxy-phenyl)amide
[278] 3-(3-methoxy-phenyl)-1-oxa-2,8-diaza-spiro[4.5]dec-2-en-8-carboxylic thioacid-(4-tert-butyl-phenyl)amide
[279] 3-phenyl-1-oxa-2,8-diaza-spiro[4.5]dec-2-en-8-carboxylic thioacid-(4-tert-butyl-phenyl)amide
[280] 3-(4-chloro-phenyl)-1-oxa-2,8-diaza-spiro[4.5]dec-2-en-8-carboxylic thioacid-(4-tert-butyl-phenyl)amide
[281] 3-(4-tert-butyl-phenyl)-1-oxa-2,8-diaza-spiro[4.5]dec-2-en-8-carboxylic thioacid-(4-tert-butyl-phenyl)amide
[282] 3-p-tolyl-1-oxa-2,8-diaza-spiro[4.5]dec-2-en-8-carboxylic thioacid-(4-tert-butyl-phenyl)amide
[283] 3-(4-trifluoromethyl-phenyl)-1-oxa-2,8-diaza-spiro[4.5]dec-2-en-8-carboxylic thioacid-(4-tert-butyl-phenyl)amide
[284] 3-(3-methoxy-phenyl)-1-oxa-2,8-diaza-spiro[4.5]dec-2-en-8-carboxylic thioacid-2-methyl-benzylamide
[285] 3-phenyl-1-oxa-2,8-diaza-spiro[4.5]dec-2-en-8-carboxylic thioacid-2-methyl-benzylamide
[286] 3-(4-chloro-phenyl)-1-oxa-2,8-diaza-spiro[4.5]dec-2-en-8-carboxylic thioacid-2-methyl-benzylamide
[287] 3-(4-tert-butyl-phenyl)-1-oxa-2,8-diaza-spiro[4.5]dec-2-en-8-carboxylic thioacid-2methyl-benzylamide
[288] 3-(4-chloro-phenyl)-1-oxa-2,8-diaza-spiro[4.5]dec-2-en-8-carboxylic thioacid cyclopentylamide
[289] 3-(4-tert-butyl-phenyl) -1-oxa-2,8-diaza-spiro[4.5]dec-2-en-8-carboxylic thioacid cyclopentylamide
[290] 3-p-tolyl-1-oxa-2,8-diaza-spiro[4.5]dec-2-en-8-carboxylic thioacid cyclopentylamide
[291] 3-(4-trifluoromethyl-phenyl)-1-oxa-2,8-diaza-spiro[4.5]dec-2-en-8-carboxylic thioacid cyclopentylamide
[292] 3-phenyl-1-oxa-2,8-diaza-spiro[4.5]dec-2-en-8-carboxylic thioacid cyclohexylmethylamide
[293] 3-(4-chloro-phenyl)-1-oxa-2,8-diaza-spiro[4.5]dec-2-en-8-carboxylic thioacid cyclohexylmethylamide
[294] 3-(4-tert-butyl-phenyl)-1-oxa-2,8-diaza-spiro[4.5]dec-2-en-8-carboxylic thioacid cyclohexylmethylamide
[295] 3-p-tolyl-1-oxa-2,8-diaza-spiro[4.5]dec-2-en-8-carboxylic thioacid cyclohexylmethylamide
[296] 3-(4-trifluoromethyl-phenyl)-1-oxa-2,8-diaza-spiro[4.5]dec-2-en-8-carboxylic thioacid cyclohexylmethylamide
[297] 3-(3-methoxy-phenyl)-1-oxa-2,8-diaza-spiro[4.5]dec-2-en-8-carboxylic thioacid cyclooctylamide
[298] 3-phenyl-1-oxa-2,8-diaza-spiro[4.5]dec-2-en-8-carboxylic thioacid cyclooctylamide
[299] 3-(4-chloro-phenyl)-1-oxa-2,8-diaza-spiro[4.5]dec-2-en-8-carboxylic thioacid cyclooctylamide
[300] 3-p-tolyl-1-oxa-2,8-diaza-spiro[4.5]dec-2-en-8-carboxylic thioacid cyclooctylamide
[301] 3-(4-trifluoromethyl-phenyl)-1-oxa-2,8-diaza-spiro[4.5]dec-2-en-8-carboxylic thioacid cyclooctylamide
[302] 3-(3-methoxy-phenyl)-1-oxa-2,8-diaza-spiro[4.5]dec-2-en-8-carboxylic thioacid-(2-morpholin-4-yl-ethyl)amide
[303] 3-tert-butyl-1-oxa-2,8-diaza-spiro[4.5]dec-2-en-8-carboxylic acid-(4-tert-butyl-phenyl)amide
[304] 3-phenyl-1-(3-phenyl-1-oxa-2,8-diaza-spiro[4.5]dec-2-en-8-yl)-propinone
[305] 1-(3-tert-butyl-1-oxa-2,8-diaza-spiro[4.5]dec-2-en-8-yl)-3-phenyl-propinone
[306] 3-phenyl-1-oxa-2,8-diaza-spiro[4.5]dec-2-en-8-carboxylic acid-(4-tert-butyl-phenyl)methylamide
[307] ((4-tert-butyl-phenyl)-(3-phenyl-1-oxa-2,8-diaza-spiro[4.5]dec-2-en-8-yl)-methanone
[308] ((4-hydroxy-3-methoxy-phenyl)-(3-phenyl-1-oxa-2,8-diaza-spiro[4.5]dec-2-en-8-yl)-methanone
[309] ((4-iodo-phenyl)-(3-phenyl-1-oxa-2,8-diaza-spiro[4.5]dec-2-en-8-yl)-methanone
[310] 3-tert-butyl-1-oxa-2,8-diaza-spiro[4.5]dec-2-en-8-carboxylic acid-p-tolylamide
[311] 3-(4-trifluoromethoxy-phenyl)-1-oxa-2,8-diaza-spiro[4.5]dec-2-en-8-carboxylic acid-p-tolylamide
[312] 3-(3-methoxy-phenyl) -1-oxa-2,8-diaza-spiro[4.5]dec-2-en-8-carboxylic acid-(4-tert-butyl-phenyl)amide
[313] 2-(4-tert-butyl-phenyl)-1-(3-phenyl-1-oxa-2,8-diaza-spiro[4.5]dec-2-en-8-yl)-ethanone
[314] 1-(3-phenyl-1-oxa-2,8-diaza-spiro[4.5]dec-2-en-8-yl)-3-(4-trifluoromethyl-phenyl)-propenone
[315] 3-(4-hydroxy-3-methoxyl-phenhyl)-1-(3-phenyl-1-oxa-2,8-diaza-spiro[4.5]dec-2-en-8-yl)-propenone
[316] ((4-tert-butyl-phenyl)-[3-(4-tert-butyl-phenyl)-1-oxa-2,8-diaza-spiro[4.5]dec-2-en-8-yl]-methanone
[317] 3-(4-tert-butyl-phenyl)-1-[3-(4-tert-butyl-phenhyl)-1-oxa-2,8-diaza-spiro[4.5]dec-2-en-8-yl]-propenone
[318] 3-(4-tert-butyl-phenyl)-1-[3-(4-trifluoromethoxy-phenyl)-1-oxa-2,8-diaza-spiro[4.5]dec-2-en-8-yl]-propenone
[319] 3-naphthalen-2-yl-1-oxa-2,8-diaza-spiro[4.5]dec-2-en-8-carboxylic acid-(4-ethyl-phenyl)amide
[320] 1-(3-phenyl-1-oxa-2,8-diaza-spiro[4.5]dec-2-en-8-yl)-3,3-di-p-tolyl-propenone
[321] 3-(4-tert-butyl-phenyl)-2-methyl-1-(3-phenyl-1-oxa-2,8-diaza-spiro[4.5]dec-2-en-8-yl)-propenone
[322] 3-phenyl-1-oxa-2,8-diaza-spiro[4.5]dec-2-en-8-carboxylic acid benzhydrylamide
[323] 3-(4-tert-butyl-phenyl)-1-(3-phenyl-1-oxa-2,8-diaza-spiro[4.5]dec-2-en-8-yl)-propenone
[324] 2-(4-tert-butyl-benzylidene)-1-(3-phenhyl-1-oxa-2,8-diaza-spiro[4.5]dec-2-en-8-yl)-buten-1-one
[325] 3-(4-isopropyl-phenyl)-1-(3-phenyl-1-oxa-2,8-diaza-spiro[4.5]dec-2-en-8-yl)-propenone
[326] 3-(4-octyl-phenyl)-1-(3-phenyl-1-oxa-2,8-diaza-spiro[4.5]dec-2-en-8-yl)-propenone
[327] 3-(4-butyl-phenyl)-1-(3-phenyl-1-oxa-2,8-diaza-spiro[4.5]dec-2-en-8-yl)-propenone
[328] 3-(4-pentyl-phenyl)-1-(3-phenyl-1-oxa-2,8-diaza-spiro[4.5]dec-2-en-8-yl)-propenone
[329] 3-(3-chloro-pyridin-2-yl) -1-oxa-2,8-diaza-spiro[4.5]dec-2-en-8-carboxylic acid-(4-tert-butyl-phenyl)amide
[330] 3-phenyl-1-oxa-2,8-diaza-spiro[4.5]dec-2-en-8-carboxylic thioacid-(4-pentafluorosulphanyl-phenyl)amide
[333] N-(4-tert-butylphenyl)-3-(3-chlorophenyl)-1-oxa-2,8-diazaspiro[4.5]dec-2-en-8-carboxamide
[334] ((E)-3-(4-cyclohexylphenyl)-1-(3-methoxyphenyl)-1-oxa-2,8-diazaspiro[4.5]dec-2-en-8-yl)prop-2-en-1-one
[335] N-(4-tert-butylphenyl)-3-(4-cyclohexylphenyl)-1-oxa-2,8-diazaspiro[4.5]dec-2-en-8-carboxamide
[336] ((E)-3-(4-tert-butylphenyl)-3-phenyl-1-(3-phenyl-1-oxa-2,8-diazaspiro[4.5]dec-2-en-8-yl)prop-2-en-1-one
[337] ((2E,4E)-3-(4-tert-butylphenyl)-5-phenyl-1-(3-phenyl-1-oxa-2,8-diazaspiro[4.5]dec-2-en-8-yl)penta-2,4-dien-1-one
[338] ((Z)-3-(4-tert-butylphenyl)-1-(3-phenyl-1-oxa-2,8-diazaspiro[4.5]dec-2-en-8-yl)-3-(thiophen-3-yl)prop-2-en-1-one
[339] ((E)-3-(4-tert-butylphenyl-1-(3-(3-methoxyphenyl)-1-oxa-2,8-diazaspiro[4.5]dec-2-en-8-yl)-3-(4-trifluoromethyl)phenyl)prop-2-en-1-one
[340] ((E)-N-(5-(8-(3-(4-tert-butylphenyl)acryloyl)-1-oxa-2,8-diazaspiro[4.5]dec-2-en-8-yl)-2-fluorophenyl)methanesulphonamide
[341] ((E)-3-(4-pentafluorosulphanyl)-1-(3-(3-methoxyphenyl-1-oxa-2,8-diazaspiro[4.5]dec-2-en-8-yl)prop-2-en-1-one
[342] ((E)-1-(3-(3-methoxyphenyl)-1-oxa-2,8-diazaspiro[4.5]dec-2-en-8-yl)-3-(2-morpholino-6-(trifluoromethyl)pyridin-3-yl)prop-2-en-1-one
[343] N-(2-fluoro-4-(1-oxo-1-(3-phenyl-1-oxa-2,8-diazaspiro[4.5]dec-2-en-8-yl)propan-2-yl)phenyl)methanesulphonamide
[344] N-(2-fluoro-4-(1-(3-(3-methoxyphenyl)-1-oxa-2,8-diazaspiro[4.5]dec-2-en-8-yl)-1-oxopropan-2-yl)phenyl)methanesulphonamide
[345] ((Z)-3-(4-tert-butylphenyl)-1-(3-(3-methoxyphenyl)-1-oxa-2,8-diazaspiro[4.5]dec-2-en-8-yl)-3-(thiophen-3-yl)prop-2-en-1-one
[346] ((E)-3-(3-bromophenyl)-1-(3-phenyl-1-oxa-2,8-diazaspiro[4.5]dec-2-en-8-yl)prop-2-en-1-one
[347] ((E)-3-(2-bromophenyl)-1-(3-phenyl-1-oxa-2,8-diazaspiro[4.5]dec-2-en-8-yl)prop-2-en-1-one
[348] 3-(3-fluorophenyl)-N-(4-(trifluoromethyl)phenyl-1-oxa-2,8-diazaspiro[4.5]dec-2-en-8-carboxamide
[349] N-(4-tert-bytulyphenyl)-3-(4-fluorophenyl) -1-oxa-2,8-diazaspiro[4.5]dec-2-en-8-carboxamide
[350] 3-(4-tert-butylphenyl)-1-(3-phenyl-1-oxa-2,8-diazaspiro[4.5]dec-2-en-8-yl)propan-1-one
[351] N-(4-tert-butylphenyl)-3-(3-fluorophenyl)-1-oxa-2,8-diazaspiro[4.5]dec-2-en-8-carboxamide
[352] N-(4-tert-butylphenyl)-3-(2-fluorophenyl)-1-oxa-2,8-diazaspiro[4.5]dec-2-en-8-carboxamide
[353] ((E)-3-(4-tert-butylphenyl)-1-(3-(3-fluorophenyl)-1-oxa-2,8-diazaspiro[4.5]dec-2-en-8-yl)prop-2-en-1-one
[354] ((Z)-1-(3-(3-methoxyphenyl)-1-oxa-2,8-diazaspiro[4.5]dec-2-en-8-yl)-4,4-dimethyl-3-phenylpent-2-en-1-one
[355] ((E)-1-(3-phenyl-1-oxa-2,8-diazaspiro[4.5]dec-2-en-8-yl)-3-(2-piperidin-1-yl)-6-(trifluoromethyl)pyridin-3-yl)prop-2-en-1-one
[356] ((E)-1-(3-(3-chlorophenyl)-1-oxa-2,8-diazaspiro[4.5]dec-2-en-8-yl)-3-(2-piperidin-1-yl)-6-(trifluoromethyl)pyridin-3-yl)prop-2-en-1-one
[357] ((2E,4E)-3-tert-butyl-1-(3-(3-methoxyphenyl) -1-oxa-2,8-diazaspiro[4.5]dec-2-en-8-yl)-5-phenylpenta-2,4-dien-1-one
[358] ((2E, 4E)-3-(4-tert-butylphenyl)-1-(3-(3-fluorophenyl) -1-oxa-2,8-diazaspiro[4.5]dec-2-en-8-yl)-5-phenylpenta-2,4-dien-1-one
[359] ((Z)-3-(4-tert-butylphenyl)-3-(3,4-dichlorophenyl)-1-(3-(2-fluorophenyl) -1-oxa-2,8-diazaspiro[4.5]dec-2-en-8-yl)prop-2-en-1-one
[360] ((Z)-3-(4-tert-butylphenyl)-3-(3,4-dichlorophenyl)-1-(3-(3-fluorophenyl)-1-oxa-2,8-diazaspiro[4.5]dec-2-en-8-yl)prop-2-en-1-one
[361] 1-(3-(3-methoxyphenyl)-1-oxa-2,8-diazaspiro[4.5]dec-2-en-8-yl)-3-(4-(trifluoromethyl)phenyl)prop-2-yn-1-one
[362] ((E)-1-(3-(3-methoxyphenyl)-1-oxa-2,8-diazaspiro[4.5]dec-2-en-8-yl)-3-(6-(trifluoromethyl)pyridin-3-yl)prop-2-en-1-one
[363] 1-(3-phenyl-1-oxa-2,8-diazaspiro[4.5]dec-2-en-8-yl)-3-(4-(trifluoromethyl)phenyl)prop-2-yn-1-one
[364] N-(4-tert-butylphenyl)-3-(2-fluorophenyl)-6-methyl-1-oxa-2,8-diazaspiro[4.5]dec-2-en-8-carboxamide
[365] ((E)-1-(3-(3-fluorophenyl)-1-oxa-2,8-diazaspiro[4.5]dec-2-en-8-yl)-3-(2-piperidin-1-yl)-6-(trifluoromethyl)pyridin-3-yl)prop-2-en-1-one
[366] N-(2-fluoro-4-(1-(3-(3-fluorophenyl)-1-oxa-2,8-diazaspiro[4.5]dec-2-en-8-yl)-1-oxopropan-2-yl)phenyl)methanesulphonamide
[367] ((E)-1-(6-methyl-3-phenyl-1-oxa-2,8-diazaspiro[4.5]dec-2-en-8-yl)-3-(2-(piperidin-1-yl)-6-(trifluoromethyl)pyridin-3-yl)prop-2-en-1-one
[368] ((E)-3-(2-bromo-4-tert-butylphenyl)-1-(3-phenyl-1-oxa-2,8-diazaspiro[4.5]dec-2-en-8-yl)prop-2-en-1-one
[369] N-(4-tert-butylphenyl)-6-methyl-3-phenyl-1-oxa-2,8-diazaspiro[4.5]dec-2-en-8-carboxamide
[370] 2-(4-tert-butylphenyl)-1-(3-phenyl-1-oxa-2,8-diazaspiro[4.5]dec-2-en-8-yl)propan-1-one
[371] N-(4-tert-butylphenyl)-3phenyl-1-oxa-2,7-diazaspiro[4.4]non-2-en-7-carboxamide
[372] N-(4-tert-butylbenzyl)-3-phenyl-1-oxa-2,8-diazaspiro[4.5]dec-2-en-8-carboxamide
[373] N-(4-tert-butylbenzyl)-3-phenyl-1-oxa-2,8-diazaspiro[4.6]undec-2-en-8-carboxamide
[374] N-(4-tert-butylbenzyl)-3-(3-methoxyphenyl)-1-oxa-2,8-diazaspiro[4.5]dec-2-en-8-carboxamide
[375] N-(1-(4-tert-butylbenzyl)ethyl-3-(3-methoxylphenyl)-1-oxa-2,8-diazaspiro[4.5]dec-2-en-8-carboxamide
[376] N-(4-tert-butylcyclohexyl)-3-phenyl-1-oxa-2,8-diazaspiro[4.5]dec-2-en-8-carboxamide
[377] N-(4-tert-butylphenethyl)-3-phenyl-1-oxa-2,8-diazaspiro[4.5]dec-2-en-8-carboxamide
[378] N-(4-tert-butylphenyl)-3-(4-chloro-3-methoxyphenyl)-1-oxa-2,8-diazaspiro[4.5]dec-2-en-8-carboxamide
[379] 8-(5-tert-butyl-1H-benzo[d]imidazol-2-yl)-3-phenyl-1-oxa-2,8-diazaspiro[4.5]dec-2-ene
[380] 3-phenyl-8-(5-(trifluoromethyl)-1H-benzo[d]imidazol-2-yl)-1-oxa-2,8-diazaspiro[4.5]dec-2-ene
[381] ((E)-N-(2-fluoro-4-(3-oxo-3-(3-phenyl-1-oxa-2,8-diazaspiro[4.5]dec-2-en-8-yl)prop-1-enyl)methanesulphonamide
[382] ((E)-N-(2-fluoro-4-(3-(3-(3-methoxyphenyl) -1-oxa-2,8-diazaspiro[4.5]dec-2-en-8-yl)-3-oxoprop-l-enyl)phenyl)methansulphonamide
[383] 5-tert-butyl-2-(3-phenyl-1-oxa-2,8-diazaspiro[4.5]dec-2-en-8-yl)benzo[d]oxazole
[384] 5-tert-butyl-2-(3-(3-methoxyphenyl)-1-oxa-2,8-diazaspiro[4.5]dec-2-en-8-yl)benzo[d]oxazole
[385] 6-methyl-3-phenyl-N-(4-(trifluoromethyl)phenyl-1-oxa-2,8-diazaspiro[4.5] dec-2-en-8-carboxamide
[386] 3-benzyl-N-(4-tert-butylphenyl)-1-oxa-2,8-diazaspiro[4.5]dec-2-en-8-carboxamide
[387] N-(4-tert-butylphenyl)-3-phenethyl-1-oxa-2,8-diazaspiro[4.5]dec-2-en-8-carboxamide
[388] 3-(4-hydroxy-3-methoxy-phenyl)-1-(3-phenyl-1-oxa-2,8-diazaspiro[4.5] decen-8-yl)-propenone
[389] 3-(4-tert-butyl-phenyl)-1-(3-phenyl-1-oxa-2,8-diazaspiro[4.5]decen-8-yl)-propenone
[390] 3-(3-chloro-pyridin-2-yl) -1-oxa-2,8-diazaspiro[4.5]dec-2-en-8-carboxylic acid-(4-tert-butyl-phenyl)amide
[391] 3,3-di-p-tolyl-1-(3-p-tolyl-1-oxa-2,8-diazaspiro[4.5]dec-2-en-8-yl)-propenone
[392] 3-(4-tert-butyl-phenyl)-1-[3-(4-chloro-phenyl)-1-oxa-2,8-diazaspiro[4.5]dec-2-en-8-yl]-propenone
[393] 3-(4-tert-butyl-phenyl)-2-ethyl-1-(3-p-tolyl-1-oxa-2,8-diazaspiro[4.5]dec-2-en-8-yl)propenone
[394] ((4-tert-butyl-phenyl)-(3-p-tolyl-1-oxa-2,8-diazaspiro[4.5]dec-2-en-8-yl)-methanone
[395] 3-(4-tert-butyl-phenyl)-1-(3-p-tolyl-1-oxa-2,8-diazaspiro[4.5]dec-2-en-8-yl)-propenone
[396] 3-(4-tert-butyl-phenyl)-2-methyl-1-(3-p-tolyl-1-oxa-2,8-diazaspiro[4.5]dec-2-en-8-yl)-propenone
[397] 2-(4-tert-butyl-phenyl)-1-[3-(3-methoxy-phenyl)-1-oxa-2,8-diazaspiro[4.5] dec-2-en-8-yl]-but-2-en-1-one
[398] 3-(4-tert-butyl-phenyl)-1-[3-(3-methoxy-phenyl)-1-oxa-2,8-diazaspiro[4.5] dec-2-en-8-yl]-propinone
[399] 3-(4-tert-butyl-phenyl)-1-[3-(3-methoxy-phenyl)-1-oxa-2,8-diazaspiro[4.5] dec-2-en-8-yl]-propenone
[400] 1-[3-(3-methoxy-phenyl)-1-oxa-2,8-diazaspiro[4.5]dec-2-en-8-tl]-2,2-diphenyl-propan-1-one
[401] 1-[3-(3-methoxy-phenyl)-1-oxa-2,8-diazaspiro[4.5]dec-2-en-8-yl]-2,2-diphenyl-ethanone
[402] 3-(3-methoy-phenyl)-1-oxa-2,8-diazaspiro[4.5]dec-2-en-8-carboxylic acid-(2,3)-dihydrobenzo[1.4]dioxin-6-yl)amide
[403] 3-(6-tert-butyl-pyridin-3-yl)-1-[3-(3-methoxy-phenyl)-1-oxa-2,8-diazaspiro[4.5]dec-2-en-8-yl]-propenone
[404] 3-(6-tert-butyl-pyridin-3-yl)-1-[3-(4-chloro-3-methoxy-phenyl)-1-oxa-2,8-diazaspiro[4.5]dec-2-en-8-yl]-propenone
[405] 2,2-diphenyl-1-(3-phenyl-1-oxa-2,8-diazaspiro[4.5]dec-2-en-8-)-propan-1-one
[406] 3-(6-tert-butyl-pyridin-3-yl)-1-(3-phenyl-1-oxa-2,8-diazaspiro[4.5]dec-2-en-8-yl)-propenone
[407] 3-(4-isopropyl-phenyl)-1-oxa-2,8-diazaspiro[4.5]dec-2-en-8-carboxylic acid-(4-tert-butyl-phenyl)amide
[408] 3-(2,3-dihydro-benzo[1,4]dioxin-6-yl) -1-oxa-2,8-diazaspiro[4.5]dec-2-en-8-carboxylic acid-(4-tert-butyl-phenyl)amide
[409] N-[2-fluoro-4-[3-(3-methoxy-phenyl)-1-oxa-2,8-diazaspiro[4.5]dec-2-en-8-carbonyl]-phenyl]-methanesulphonamide
[410] N-[2-fluoro-4-(3-phenyl-1-oxa-2,8-diazaspiro[4.5]dec-2-en-8-carbonyl]-phenyl]-methansulphonamide
[411] 3-(4-cyclohexyl-phenyl)-1-(3-phenyl-1-oxa-2,8-diazaspiro[4.5]dec-2-en-8-yl)-propenone
[412] 1-[3-(3-methoxy-phenyl)-1-oxa-2,8-diazaspiro[4.5]dec-2-en-8-yl]-2,2,2-triphenyl-ethanone
[415] [3-(4-chloro-3-methoxy-phenyl)-1-oxa-2,8-diazaspiro[4.5]dec-2-en-8-yl]-(2-chloro-6-trifluoromethyl-pyridin-3-yl)-methanone
[416] [3-(3-methoxy-phenyl) -1-oxa-2,8-diazaspiro[4.5]dec-2-en-8-yl]-(2-morpholin-4-yl-6-trifluoromethyl-pyridin-3-yl)-methanone
[417] 3-(4-tert-butyl-phenyl)-1-[3-(3-chloro-phenyl)-1-oxa-2,8-diazaspiro[4.5]dec-2-en-8-yl]-propenone
[418] 3-(4-tert-butyl-phenyl)-1-[3-(2,3-dihydro-benzo[1.4]dioxin-6-yl)-1-oxa-2,8-diazaspiro[4.5]dec-2-en-8-yl-propenone
[419] 3-(4-tert-butyl-phenyl)-1-[3-(4-cyclohexyl-phenyl)-1-oxa-2,8-diazaspiro[4.5] dec-2-en-8-yl]-propenone
[420] N-[4-[3-(4-cyclohexyl-phenyl)-1-oxa-2,8-diazaspiro[4.5]dec-2-en-8-carbonyl]-2-fluoro-phenyl]-methanesulphonamide
[421] 2-hydroxy-2,2-diphenyl-1-(3-phenyl-1-oxa-2,8-diazaspiro[4.5]dec-2-en-8-yl]-ethanone
[423] 3-(4-tert-butyl-phenyl)-1-(3-quinolin-3-yl-1-oxa-2,8-diazaspiro[4.5]dec-2-en-8-yl)-propenone
[424] 3-(2,3-dihydro-benzo[1.4]dioxin-6-yl)-1-oxa-2,8-diazaspiro[4.5]dec-2-en-8-carboxylic acid-(4-trifluoromethyl-phenyl)amide
[425] 3-(3-chloro-phenyl) -1-oxa-2,8-diazaspiro[4.5]dec-2-en-8-carboxylic acid-(4-trifluoromethyl-phenyl)amide
[426] ((4-tert-butyl-phenyl)-[3-(3-methoxy-phenyl)-1-oxa-2,8-diazaspiro[4.5]dec-2-en-8-yl]-methanone
[427] 3-(4-cyclohexyl-phenhyl)-1-oxa-2,8-diaza-spiro[4.5]dec-2-en-8-carboxylic acid-(4-trifluoromethyl-phenyl)amide
[428] 3-(4-tert-butyl-phenyl)-3-(4-chloro-phenyl)-1-oxa-2,8-diazaspiro[4.5]dec-2-en-8-yl]-propenone
[429] 3-(4-tert-butyl-phenyl)-1-[3-(3-chloro-phenyl)-1-oxa-2,8-diazaspiro[4.5]dec-2-en-8-yl]-3-(3-methoxy-phenyl)-propenone
[430] 3-(4-tert-butyl-phenyl-1-[3-(4-chloro-3-methoxy-phenyl)-1-oxa-2,8-diazaspiro[4.5]dec-2-en-8-yl]-3-(4-trifluoromethyl-phenyl)-propenone
[431] 3-(4-pentafluorosulphanyl-phenyl)-1-[3-(3-methoxy-phenyl)-1-oxa-2,8-diazaspiro[4.5]dec-2-en-8-yl]-propenone
[432] 3-(4-tert-butyl-phenyl)-1-[3-(4-chloro-3-methoxy-phenyl)-1-oxa-2,8-diazaspiro[4.5]dec-2-en-8-yl]-propenone
[433] 3-(4-tert-butyl-phenyl)-1-[3-(4-chloro-3-methoxy-phenyl)-1-oxa-2,8-diazaspiro[4.5]dec-2-en-8-yl]-propinone
[434] 3-(4-tert-butyl-phenyl)-1-[3-(3-methoxy-pphenyl)-1-oxa-2,8-diazaspiro[4.5] dec-2-en-8-yl]-3-(2-trifluoromethyl-phenyl)propenone
[435] 3-thiazol-2-yl-1-oxa-2,8-diazaspiro[4.5]dec-2-en-8-carboxylic acid-(4-isopropyl-phenyl)amide
[436] 3-thiazol-2-yl-1-oxa-2,8-diazaspiro[4.5]dec-2-en-8-carboxylic acid-(4-trifluoromethoxy-phenyl)amide
[437] 3,3-bis-(4-tert-butyl-phenyl)-1-(3-phenyl-1-oxa-2,8-diazaspiro[4.5]dec-2-en-8-yl)-propenone
and
[438] 3-thiazol-2-yl-l-oxa-2,8-diazaspiro[4.5]dec-2-en-8-carboxylic acid-(4-tert-butyl-phenyl)amide;
in each case optionally in the form of one of the pure stereoisomers thereof, particularly enantiomers or diastereoisomers, the racemates thereof, or in the form of a mixture of stereoisomers, particularly the enantiomers and/or diastereoisomers, in an arbitrary mixing ratio, or respectively in the form of corresponding salts, or respectively in the form of corresponding solvates.

23. Compounds according to one or more of claims 1 to 22, **characterised in that** in a concentration of 10 µM in the FLIPR assay they exhibit an inhibition of the Ca²⁺ ion inflow in dorsal root ganglions in rats of at least 30%, preferably at least 40%, particularly preferred at least 50%, most particularly preferred at least 70%, even further preferred at least 90%, in comparison to the maximum attainable inhibition of the Ca²⁺ ion inflow with capsaicin in a concentration of 10 µM.

24. Method for the production of substituted spiro compounds of the general formula I according to one or more of claims 1 to 23, **characterised in that** at least one compound of the general formula II 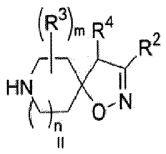 wherein R², R³, R⁴, m and n have the meaning in accordance with one or more of claims 1 to 23, is converted in a reaction medium with at least one isocyanate of the general formula R⁵-N=C=O, wherein R⁵ has the meaning in accordance with one or more of claims 1 to 23, possibly in the presence of at least one base, preferably in the presence of at least base selected from the group consisting of triethylamine, 4.4-dimethylaminopyridine, diisopropylethylamine, pyridine and N-methylmorpholine, to at least one compound of the general formula I, wherein R², R³, R⁴, m and n have the above-specified meaning and R¹ stands for -C(=O)-NR⁵R⁶, wherein R⁵ has the above-specified meaning and R⁶ stands for a hydrogen radical, and this is possibly purified and/or isolated
or
at least one compound of the general formula II is converted in a reaction medium with at least one isocyanate of the general formula S=C=N-R⁷, wherein R⁷ has the meaning in accordance with one or more of claims 1 to 23, possibly in the presence of at least one base, preferably in the presence of at least base selected from the group consisting of triethylamine, 4.4-dimethylaminopyridine, diisopropylethylamine, pyridine and N-methylmorpholine, to at least one compound of the general formula I, wherein R², R³, R⁴, m and n have the above-specified meaning and R¹ stands for-C(=S)-NR⁷R⁸, wherein R⁷ has the above-specified meaning and R⁸ stands for a hydrogen radical, and this is possibly purified and/or isolated
and possibly at least one compound of the general formula I, wherein R², R³, R⁴, m and n have the above-specified meaning and R¹ stands for -C(=O)-NR⁵R⁶, or -C(=S)-NR⁷R⁸, wherein R⁶ and R⁸ respectively stand for a hydrogen radical, is converted in a reaction medium in the presence of at least one base, preferably in the presence of at least one metal hydride salt or a metal alcoholate salt, particularly preferred in the presence of a metal hydride salt or a metal alcoholate salt selected from the group consisting of sodium hydride, potassium hydride, potassium tert-butanolate, sodium tert-butanolate, potassium methanolate, sodium methanolate, sodium ethanolate and potassium ethanolate, with at least one compound of the general formula LG-R⁶ or the general formula LG-R⁸, wherein LG stands for a leaving group, preferably for a halogen atom, particularly preferred for a chlorine atom, and R⁶ and R⁸ have the above-specified meaning with the exception of hydrogen, to at least one compound of the general formula I, wherein R² to R⁴, m and n have the above-specified meaning and R¹ stands for -C(=O)-NR⁵R⁶ or -C(=S)-NR⁷R⁸, and this is possibly purified and/or isolated,
or
at least one compound of the general formula II is converted in a reaction medium in the presence of at least one base, preferably in the presence of at least one metal hydride salt, particularly preferred in the presence of sodium and/or potassium hydride, with at least one compound of the general formula LG-R¹, wherein R¹ has the meaning in accordance with one or more of claims 1 to 23, with the exception of -C(=O)-NR⁵R⁶, -C(=S)-NR⁷R⁸, -C(=O)-R⁹ and -S(=O)-R¹⁰, and LG stands for a leaving group, preferably for a halogen atom, particularly preferred for a chlorine atom, to at least one compound of the general formula I, wherein R¹ to R⁴, m and n have the above-specified meaning, and this is possibly purified and/or isolated,
or
at least one compound of the general formula II is converted in a reaction medium in the presence of at least one reducing agent, with at least one compound of the general formula R¹-C(=O)-H, wherein R¹ has the meaning in accordance with one or more of claims 1 to 23, with the exception of -C(=O)-NR⁵R⁶, -C(=S)-NR⁷R⁸, -C(=O)-R⁹ and - S(=O)-R¹⁰, to at least one compound of the general formula I, wherein R¹ to R⁴, m and n have the above-specified meaning, and this is possibly purified and/or isolated,
or
at least one compound of the general formula II is converted in a reaction medium in the presence of at least one base with at least one compound of the general formula R⁹-C(=O)-LG, wherein R⁹ has the meaning in accordance with one or more of claims 1 to 23 and LG stands for a leaving group, preferably for a halogen radical, or in a reaction medium in the presence of at least one coupling reagent possibly in the presence of at least one base with a compound of the general formula R⁹-C(=O)-OH, wherein R⁹ has the meaning in accordance with one or more of claims 1 to 23, to at least one compound of the general formula I, wherein R² to R⁴, m and n have the above-specified meaning and R¹ stands for -C(=O)-R⁹, and this is possibly purified and/or isolated,
or
at least one compound of the general formula II is converted in a reaction medium in the presence of at least one base with at least one compound of the general formula R¹⁰-S(=O)₂-LG, wherein R¹⁰ has the meaning in accordance with one or more of claims 1 to 23 and LG stands for a leaving group, preferably for a halogen radical, to at least one compound of the general formula I, wherein R² to R⁴, m and n have the above-specified meaning and R¹ stands for -S(=O)₂-R¹⁰, and this is possibly purified and/or isolated.

25. Medication containing at least one compound according to one of more of claims 1 to 23 and possibly one of more physiologically compatible auxiliary substances.

26. Medication according to claim 25 for the prophylaxis and/or treatment of pain, preferably pain selected from the group consisting of acute pain, chronic pain and neuropathic pain.

27. Medication according to claim 25 for the treatment and/or prophylaxis of one or more diseases selected from the group consisting of joint pain; migraine; depression; neuropathy; nerve injuries; neurodegenerative illnesses, preferably selected from the group consisting of multiple sclerosis, Alzheimer's disease, Parkinson's disease and Huntington's disease; cognitive dysfunction, preferably cognitive deficiencies, particularly preferred memory disorders; epilepsy; respiratory diseases, preferably selected from the group consisting of asthma and pneumonia; coughing; urinary incontinence; overactive bladder (OAB); gastric ulcers; irritable bowel syndrome; strokes; eye irritations; skin irritations; neurotic skin diseases; inflammatory diseases, preferably inflammation of the intestine; diarrhoea; pruritus; eating disorders, preferably selected from the group consisting of bulimia, cachexia, anorexia and obesity; dependency on medicines; medication abuse; withdrawal symptoms in the case of dependency on medications; development of tolerance in relation to medications, preferably in relation to natural or synthetic opioids; drug dependency; drug abuse; withdrawal symptoms in the case of drug dependency; alcohol dependency; alcohol abuse and withdrawal symptoms in the case of alcohol dependency; for diuresis; for antinatriuresis; for influencing the cardiovascular system; for increasing vigilance; for increasing libido; for modulating locomotor activity; for anxiolysis; for local anaesthesia and/or inhibition of undesirable side-effects, preferably selected from the group consisting of hyperthermia, high blood pressure and narrowing of the bronchi, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil.

28. Use of at least one compound according to one or more of claims 1 to 23 for the production of a medication for the prophylaxis and/or treatment of pain, preferably pain selected from the group consisting of acute pain, chronic pain and neuropathic pain.

29. Use of at least one compound according to one or more of claims 1 to 23 for the production of a medication for the prophylaxis and/or treatment of one or more diseases selected from the group consisting of joint pain; migraine; depression; neuropathy; nerve injuries; neurodegenerative illnesses, preferably selected from the group consisting of multiple sclerosis, Alzheimer's disease, Parkinson's disease and Huntington's disease; cognitive dysfunction, preferably cognitive deficiencies, particularly preferred memory disorders; epilepsy; urinary incontinence; overactive bladder (OAB); gastric ulcers; irritable bowel syndrome; strokes; diarrhoea; pruritus; eating disorders, preferably selected from the group consisting of bulimia, cachexia, anorexia and obesity; dependency on medicines; medication abuse; withdrawal symptoms in the case of dependency on medications; development of tolerance in relation to medications, preferably in relation to natural or synthetic opioids; drug dependency; drug abuse; withdrawal symptoms in the case of drug dependency; alcohol dependency; alcohol abuse and withdrawal symptoms in the case of alcohol dependency; for diuresis; for antinatriuresis; for influencing the cardiovascular system; for increasing vigilance; for increasing libido; for modulating locomotor activity; for anxiolysis; for local anaesthesia and/or inhibition of undesirable side-effects, preferably selected from the group consisting of hyperthermia, high blood pressure and narrowing of the bronchi, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil.

30. Use of at least one compound according to one or more of claims 1 to 23 for the production of a medication for the prophylaxis and/or treatment of one or more disorders selected from the group consisting of respiratory diseases, preferably selected from the group consisting of asthma and pneumonia; coughing; eye irritations; skin irritations; neurotic skin diseases and inflammatory diseases, preferably inflammation of the intestine.

## Revendications

1. Composés spiro substitués de formule générale I 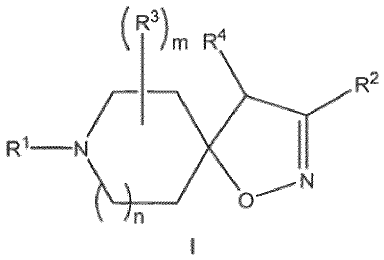 dans laquelle
m représente 0, 1, 2, 3 ou 4,
n représente 0, 1 ou 2,
R¹ représente un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, et/ou condensé avec un système cyclique mono- ou polycyclique non substitué ou substitué au moins une fois,
un radical aryle ou hétéroaryle non substitué ou substitué au moins une fois, qui peut être relié par un groupe alkylène, alcénylène ou alcynylène linéaire ou ramifié, non substitué ou substitué au moins une fois, et/ou condensé avec un système cyclique mono- ou polycyclique non substitué ou substitué au moins une fois,
un groupe -C(=O)-NR⁵R⁶,
un groupe -C(=S)-NR⁷R⁸,
un groupe -C(=O)-R⁹,
ou un groupe -S(=O)₂-R¹⁰;
R² représente un radical aliphatique linéaire ou ramifié, saturé ou insaturé, non substitué ou substitué au moins une fois, comprenant éventuellement au moins un hétéroatome en tant qu'élément de chaîne ;
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
un radical phényle non substitué ou substitué au moins une fois,
à condition qu'une des positions méta et la position para de ce radical phényle ne soient pas substituées avec des substituants qui sont reliés au radical phényle à chaque fois par un atome identique choisi dans le groupe constitué par l'oxygène, le soufre et l'azote ;
un radical non substitué ou substitué au moins une fois choisi dans le groupe constitué par naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle, [1,2,3,4]-tétrahydronaphtyle, [1,2,3,4]-tétrahydroquinolinyle, [1,2,3,4]-tétrahydroisoquinolinyle, [1,2,3,4]-tétrahydroquinazolinyle ; 2H-benzo[1.4]oxazin-3(4H)-onyle, (3,4)-dihydroquinolin-2(1H)-onyle, [3,4]-dihydro-2H-1,4-benzoxazinyle et benzothiazolyle,
ou un radical aryle ou hétéroaryle non substitué ou substitué au moins une fois, qui est relié par un groupe alkylène, alcénylène ou alcynylène linéaire ou ramifié, non substitué ou substitué au moins une fois, comprenant éventuellement au moins un hétéroatome en tant qu'élément de chaîne, et qui peut éventuellement être condensé avec un système cyclique mono- ou polycyclique non substitué ou substitué au moins une fois ;
R³ représente un radical halogène, un groupe nitro, un groupe hydroxy, un groupe thiol ; un groupe -O-R¹¹, un groupe -S-R¹² ou un radical aliphatique linéaire ou ramifié, saturé ou insaturé, non substitué ou substitué au moins une fois ;
R⁴ représente un radical hydrogène, un radical halogène, un groupe nitro, un groupe hydroxy, un groupe thiol ; un groupe oxo (=O), un groupe -O-R¹¹, un groupe -S-R¹², ou un radical aliphatique linéaire ou ramifié, saturé ou insaturé, non substitué ou substitué au moins une fois ;
R⁵ et R⁷ représentent chacun indépendamment l'un de l'autre
un radical aliphatique linéaire ou ramifié, saturé ou insaturé, non substitué ou substitué au moins une fois, comprenant éventuellement au moins un hétéroatome en tant qu'élément de chaîne,
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois et/ou ponté avec au moins un groupe alkylène linéaire ou ramifié, non substitué ou substitué au moins une fois,
un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
R⁶ et R⁸ représentent chacun indépendamment l'un de l'autre
un radical hydrogène,
un radical aliphatique linéaire ou ramifié, saturé ou insaturé, non substitué ou substitué au moins une fois, comprenant éventuellement au moins un hétéroatome en tant qu'élément de chaîne,
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois et/ou ponté avec au moins un groupe alkylène linéaire ou ramifié, non substitué ou substitué au moins une fois,
un radical aryle ou hétéroaryle non substitué ou substitué au moins une fois, qui peut être relié par un groupe alkylène, alcénylène ou alcynylène linéaire ou ramifié, non substitué ou substitué au moins une fois, comprenant éventuellement au moins un hétéroatome en tant qu'élément de chaîne, et/ou condensé avec un système cyclique mono- ou polycyclique non substitué ou substitué au moins une fois,
R⁹ représente un radical aliphatique linéaire ou ramifié, saturé ou insaturé, non substitué ou substitué au moins une fois ;
ou un radical aryle ou hétéroaryle non substitué ou substitué au moins une fois, qui peut être relié par un groupe alkylène, alcénylène ou alcynylène linéaire ou ramifié, non substitué ou substitué au moins une fois et/ou condensé avec un système cyclique mono- ou polycyclique non substitué ou substitué au moins une fois ;
R¹⁰ représente un radical aliphatique linéaire ou ramifié, saturé ou insaturé, non substitué ou substitué au moins une fois ;
un radical cycloaliphatique non substitué ou substitué au moins une fois, insaturé ou saturé, comprenant éventuellement au moins un hétéroatome en tant qu'élément de cycle, qui peut être relié par un groupe alkylène, alcénylène ou alcynylène linéaire ou ramifié, non substitué ou substitué au moins une fois, et/ou condensé avec un système cyclique mono- ou polycyclique non substitué ou substitué au moins une fois,
ou un radical aryle ou hétéroaryle non substitué ou substitué au moins une fois, qui peut être relié par un groupe alkylène, alcénylène ou alcynylène linéaire ou ramifié, non substitué ou substitué au moins une fois et/ou condensé avec un système cyclique mono- ou polycyclique non substitué ou substitué au moins une fois ;
et
R¹¹ et R¹² représentent chacun indépendamment l'un de l'autre
un radical aliphatique linéaire ou ramifié, saturé ou insaturé, non substitué ;
un radical cycloaliphatique non substitué, insaturé ou saturé, comprenant éventuellement au moins un hétéroatome en tant qu'élément de cycle, qui peut être relié par un groupe alkylène, alcénylène ou alcynylène linéaire ou ramifié, non substitué, et/ou condensé avec un système cyclique mono- ou polycyclique non substitué,
ou un radical aryle ou hétéroaryle non substitué, qui peut être relié par un groupe alkylène, alcénylène ou alcynylène linéaire ou ramifié, non substitué, et/ou condensé avec un système cyclique mono- ou polycyclique non substitué ;
les substituants des radicaux aliphatiques susmentionnés pouvant être choisis indépendamment les uns des autres dans le groupe constitué par F, Cl, Br, I, -CN, -NO₂, -OH, -SH et -NH₂ ;
à chaque fois éventuellement sous la forme d'un de leurs stéréoisomères purs, notamment des énantiomères ou des diastéréomères, de leurs racémats ou sous la forme d'un mélange de stéréoisomères, notamment des énantiomères ou/ou des diastéréomères, en un rapport de mélange quelconque, ou à chaque fois sous la forme de sels correspondants, ou à chaque fois sous la forme de solvates correspondants.

2. Composés selon la revendication 1, **caractérisés en ce que**
m représente 0, 1, 2, 3 ou 4,
n représente 0, 1 ou 2,
R¹ représente un radical cycloaliphatique de 3, 4, 5, 6, 7, 8 ou 9 éléments, insaturé ou saturé, éventuellement substitué, qui peut être condensé avec un système cyclique mono- ou polycyclique saturé, insaturé ou aromatique, éventuellement substitué ;
un radical aryle ou hétéroaryle de 5 à 14 éléments, éventuellement substitué, qui peut être condensé avec un système cyclique mono- ou polycyclique saturé ou insaturé, éventuellement substitué,
un groupe -C(=O)-NR⁵R⁶,
un groupe -C(=S)-NR⁷R⁸,
un groupe -C(=O)-R⁹,
un groupe -S (=O)₂-R¹⁰,
ou -(CHR¹³)-(CHR¹⁴)_{f}-(CHR¹⁵)ₕ-R¹⁶ avec f = 0 ou 1 et h = 0 ou 1 ;
R² représente un radical aliphatique en C₁₋₁₀ linéaire ou ramifié, saturé ou insaturé, éventuellement substitué ;
un radical cycloaliphatique de 3, 4, 5, 6, 7, 8 ou 9 éléments, insaturé ou saturé, éventuellement substitué, qui peut être condensé avec un système cyclique mono- ou polycyclique saturé, insaturé ou aromatique, éventuellement substitué ;
un radical phényle, qui peut être substitué avec 1, 2, 3, 4 ou 5 substituants choisis indépendamment les uns des autres dans le groupe constitué par F, Cl, Br, I, -CN, -CF₃, -SF₅, -OH, -0-alkyle en C₁₋₅, -NH₂, -NO₂,-O-CF₃, -S-CF₃, -SH, -S-alkyle en C₁₋₅, -alkyle en C₁₋₁₀, - C(=O)-OH, -C(=O)-O-alkyle en C₁₋₅, -O-C(=O)-alkyle en C₁₋₅, -NH-alkyle en C₁₋₅, -N (alkyle en C₁₋₅)₂, -NH-C(=O)-O-alkyle en C₁₋₅, -C(=O)-H, -C(=O)-alkyle en C₁₋₅, -C(=O)-NH₂, -C(=O)-NH-alkyle en C₁₋₅, C(=O)-N-(alkyle en C₁₋₅)₂, -S(=O)₂-alkyle en C₁₋₅, -S(=O)₂-phényle, -NH-S(=O)₂-alkyle en C₁₋₅, -NH-S(=O)₂-alkylène en C₁₋₅-phényle, -NH-S (=O)₂-alkylène en C₁₋₅-naphtyle, -NH-S (=O)₂-phényle, -NH-S(=O)₂-naphtyle, cyclohexyle, cyclopentyle, morpholinyle, pipéridinyle, pipérazinyle, pyrrolidinyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, morpholinyle, pipéridinyle, pipérazinyle, pyrrolidinyle, pyridazinyle, -S(=O)₂-phényle, -0-phényle, -0-benzyle, phényle, -(CH₂)-benzo[b]furanyle,-NH-S(=O)₂-alkylène en C₁₋₅-phényle,-NH-S(=O)₂-alkylène en C₁₋₅-naphtyle, -NH-S(=O)₂-phényle, -NH-S(=O)₂-naphtyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, - NO₂, -alkyle en C₁₋₅, -0-alkyle en C₁₋₅, -O-CF₃, -S-CF₃, phényle et -0-benzyle,
à condition qu'une des positions méta et la position para de ce radical phényle ne soient pas substituées avec des substituants qui sont reliés au radical phényle à chaque fois par un atome identique choisi dans le groupe constitué par l'oxygène, le soufre et l'azote ;
un radical éventuellement substitué choisi dans le groupe constitué par naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle, [1,2,3,4]-tétrahydronaphtyle, [1,2,3,4]-tétrahydroquinolinyle, [1,2,3,4]-tétrahydroisoquinolinyle, [1,2,3,4]-tétrahydroquinazolinyle ; 2H-benzo[1.4]oxazin-3(4H)-onyle, (3,4)-dihydroquinolin-2(1H)-onyle, [3,4]-dihydro-2H-1,4-benzoxazinyle et benzothiazolyle,
ou - (CHR¹⁷)-X_{q}-(CHR¹⁸)ᵣ-Yₛ-(CHR¹⁹)ₜ-Zᵤ-R²⁰ avec q = 0 ou 1, r = 0 ou 1, s = 0 ou 1, t = 0 ou 1, u = 0 ou 1, X, Y et Z représentant chacun indépendamment les uns des autres 0, S, NH, N(CH₃), N(C₂H₅) ou N[CH(CH₃)₂] ;
R³ représente un radical halogène, un groupe nitro, un groupe hydroxy, un groupe thiol ; un groupe -O-R¹¹, un groupe -S-R¹² ou un radical aliphatique en C₁₋₁₀ linéaire ou ramifié, saturé ou insaturé, éventuellement substitué ;
R⁴ représente un radical hydrogène, un radical halogène, un groupe nitro, un groupe hydroxy, un groupe thiol ; un groupe oxo (=O), un groupe -O-R¹¹, un groupe -S-R¹², ou un radical aliphatique en C₁₋₁₀ linéaire ou ramifié, saturé ou insaturé, éventuellement substitué ;
R⁵ et R⁷ représentent chacun indépendamment l'un de l'autre
un radical aliphatique en C₁₋₂₀ linéaire ou ramifié, saturé ou insaturé, éventuellement substitué ;
un radical cycloaliphatique de 3, 4, 5, 6, 7, 8, 9, 10, 11 ou 12 éléments, insaturé ou saturé, éventuellement substitué, qui peut être condensé avec un système cyclique mono- ou polycyclique saturé, insaturé ou aromatique, éventuellement substitué, et/ou ponté avec un ou deux groupes alkylène en C₁₋₅ linéaires ou ramifiés, éventuellement substitués ;
un radical aryle ou hétéroaryle de 5 à 14 éléments, éventuellement substitué, qui peut être condensé avec un système cyclique mono- ou polycyclique saturé ou insaturé, éventuellement substitué ;
ou -(CR²¹R²²)-Xᵥ-(CHR²³)_{w}-Yₓ-(CHR²⁴)_{y}-Z_{z}-R²⁵ avec v = 0 ou 1, w = 0 ou 1, x = 0 ou 1, y = 0 ou 1, z = 0 ou 1, X, Y et Z représentent chacun indépendamment les uns des autres 0, S, NH, N(CH₃), N(C₂H₅) ou N[CH(CH₃)₂] ;
R⁶ et R⁸ représentent chacun indépendamment l'un de l'autre
un radical hydrogène,
un radical aliphatique en C₁₋₂₀ linéaire ou ramifié, saturé ou insaturé, éventuellement substitué ;
un radical cycloaliphatique de 3, 4, 5, 6, 7, 8, 9, 10, 11 ou 12 éléments, insaturé ou saturé, éventuellement substitué, qui peut être condensé avec un système cyclique mono- ou polycyclique saturé, insaturé ou aromatique, éventuellement substitué, et/ou ponté avec un ou deux groupes alkylène en C₁₋₅ linéaires ou ramifiés, éventuellement substitués ;
un radical aryle ou hétéroaryle de 5 à 14 éléments, éventuellement substitué, qui peut être condensé avec un système cyclique mono- ou polycyclique saturé ou insaturé, éventuellement substitué ;
ou -(CR²¹R²²)-Xᵥ-(CHR²³)_{w}-Yₓ-(CHR²⁴)_{y}-Z_{z}-R²⁵ avec v = 0 ou 1, w = 0 ou 1, x = 0 ou 1, y = 0 ou 1, z = 0 ou 1, X, Y et Z représentent chacun indépendamment les uns des autres 0, S, NH, N(CH₃), N(C₂H₅) ou N[CH(CH₃)₂] ;
R⁹ représente un radical aliphatique en C₁₋₁₀ linéaire ou ramifié, saturé ou insaturé, éventuellement substitué ;
un radical aryle ou hétéroaryle de 5 à 14 éléments, éventuellement substitué, qui peut être condensé avec un système cyclique mono- ou polycyclique saturé ou insaturé, éventuellement substitué ;
-(CR²⁶R²⁷)-(CHR²⁸)ₐₐ-(CHR²⁹)_{bb}-R³⁰ avec aa = 0 ou 1 et bb = 0 ou 1 ;
-CR³¹=CR³²-R³³
ou -C≡C-R³⁴ ;
R¹⁰ représente un radical aliphatique en C₁₋₁₀ linéaire ou ramifié, saturé ou insaturé, éventuellement substitué ;
un radical cycloaliphatique de 3, 4, 5, 6, 7, 8 ou 9 éléments, insaturé ou saturé, éventuellement substitué, qui peut être condensé avec un système cyclique mono- ou polycyclique saturé, insaturé ou aromatique, éventuellement substitué ;
un radical aryle ou hétéroaryle de 5 à 14 éléments éventuellement substitué, qui peut être condensé avec un système cyclique mono- ou polycyclique saturé ou insaturé, éventuellement substitué ;
-(CR²⁶R²⁷)-(CHR²⁸)ₐₐ-(CHR²⁹)-R³⁰ avec aa = 0 ou 1 et bb = 0 ou 1 ;
-CR³¹=CR³²-R³³
ou -C≡C-R³⁴ ;
R¹¹ et R¹² représentent chacun indépendamment l'un de l'autre
un radical aliphatique en C₁₋₁₀ linéaire ou ramifié, saturé ou insaturé ;
un radical cycloaliphatique de 3, 4, 5, 6, 7, 8 ou 9 éléments, insaturé ou saturé, qui peut être condensé avec un système cyclique mono- ou polycyclique saturé, insaturé ou aromatique,
ou un radical aryle ou hétéroaryle de 5 à 14 éléments, qui peut être condensé avec un système cyclique mono- ou polycyclique saturé ou insaturé ;
R¹³, R¹⁴, R¹⁵, R¹⁷, R¹⁸, R¹⁹, R²¹, R²², R²³, R²⁴, R²⁸ et R³¹ représentent chacun indépendamment les uns des autres
un radical hydrogène,
un radical aliphatique en C₁₋₁₀ linéaire ou ramifié, saturé ou insaturé, éventuellement substitué,
ou un radical aryle ou hétéroaryle de 5 à 14 éléments, éventuellement substitué, qui peut être condensé avec un système cyclique mono- ou polycyclique saturé ou insaturé, éventuellement substitué ;
R²⁶ et R²⁷ représentent chacun indépendamment l'un de l'autre
un radical hydrogène,
un radical aliphatique en C₁₋₁₀ linéaire ou ramifié, saturé ou insaturé, éventuellement substitué,
un radical aryle ou hétéroaryle de 5 à 14 éléments, éventuellement substitué, qui peut être condensé avec un système cyclique mono- ou polycyclique saturé ou insaturé, éventuellement substitué ;
ou -OH ;
R³² représente un radical hydrogène ;
un radical aliphatique en C₁₋₁₀ linéaire ou ramifié, saturé ou insaturé, éventuellement substitué,
un radical aryle ou hétéroaryle de 5 à 14 éléments, éventuellement substitué, qui peut être condensé avec un système cyclique mono- ou polycyclique saturé ou insaturé, éventuellement substitué ;
ou -(CH₂)_{cc}-R³⁵ avec cc = 1, 2, 3 ou 4 ou -CH=CH-R³⁶ ;
R¹⁶ R²⁰, R²⁵, R³⁰, R³³ et R³⁴ représentent chacun indépendamment les uns des autres
un radical aliphatique en C₁₋₁₀ linéaire ou ramifié, saturé ou insaturé, éventuellement substitué,
un radical cycloaliphatique de 3, 4, 5, 6, 7, 8 ou 9 éléments, insaturé ou saturé, éventuellement substitué, qui peut être ponté avec 1, 2, 3, 4 ou 5 groupes alkylène en C₁₋₅ linéaires ou ramifiés, éventuellement substitués, et/ou condensé avec un système cyclique mono- ou polycyclique saturé, insaturé ou aromatique, éventuellement substitué ;
ou un radical aryle ou hétéroaryle de 5 à 14 éléments, éventuellement substitué, qui peut être condensé avec un système cyclique mono- ou polycyclique saturé ou insaturé, éventuellement substitué ;
et
R³⁵ et R³⁶ représentent chacun indépendamment l'un de l'autre
un radical aryle de 6 ou 10 éléments, éventuellement substitué, qui peut être condensé avec un système cyclique mono- ou polycyclique saturé ou insaturé, éventuellement substitué ;
les radicaux aliphatiques en C₁₋₁₀ et les radicaux aliphatiques en C₁₋₂₀ susmentionnés pouvant à chaque fois éventuellement être substitués avec 1, 2, 3, 4, 5, 6, 7, 8 ou 9 substituants choisis indépendamment les uns des autres dans le groupe constitué par F, Cl, Br, I,-CN, -NO₂, -OH, -SH et -NH₂ ;
les radicaux cycloaliphatiques susmentionnés pouvant à chaque fois éventuellement être substitués avec 1, 2, 3, 4 ou 5 substituants choisis indépendamment les uns des autres dans le groupe constitué par oxo (=0), thioxo (=S), F, Cl, Br, I, -CN, -CF₃, -SF₅, -OH, -0-alkyle en C₁₋₅, -NH₂, -NO₂, -O-CF₃, -S-CF₃, -SH, -S-alkyle en C₁₋₅, -alkyle en C₁₋₅, -C(=O)-OH, -C(=O)-O-alkyle en C₁₋₅, -O-C(=O)-alkyle en C₁₋₅, -NH-alkyle en C₁₋₅, - N (alkyle en C₁₋₅)₂, -NH-C(=O)-O-alkyle en C₁₋₅, -C(=O)-H, -C(=O)-alkyle en C₁₋₅, -C(=O)-NH₂, -C(=O)-NH-alkyle en C₁₋₅, C(=O)-N-(alkyle en C₁₋₅)₂, -S(=O)₂-alkyle en C₁₋₅,-S (=O)₂-phényle, -NH-S(=O)₂-alkyle en C₁₋₅, -S(=O)₂-NH-alkyle en C₁₋₅, cyclohexyle, cyclopentyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH,-CF₃, -SF₅, -CN, -NO₂, -alkyle en C₁₋₅, -0-alkyle en C₁₋₅, -O-CF₃, -S-CF₃, phényle et -0-benzyle,
et les radicaux cycloaliphatiques susmentionnés pouvant à chaque fois éventuellement comprendre 1, 2, 3, 4 ou 5 hétéroatomes choisis indépendamment les uns des autres dans le groupe constitué par oxygène, azote et soufre en tant qu'élément de cycle ;
les cycles des systèmes cycliques mono- ou polycycliques susmentionnés pouvant chacun éventuellement être substitués avec 1, 2, 3, 4 ou 5 substituants choisis indépendamment les uns des autres dans le groupe constitué par oxo (=0), thioxo (=S), F, Cl, Br, I, -CN, -CF₃, -SF₅, -OH, -0-alkyle en C₁₋₅, -NH₂, -NO₂, -O-CF₃, -S-CF₃, -SH, -S-alkyle en C₁₋₅,-alkyle en C₁₋₅, -C(=O)-OH, -C(=O)-O-alkyle en C₁₋₅, -0-C(=O)-alkyle en C₁₋₅, -NH-alkyle en C₁₋₅, -N (alkyle en C₁₋₅)₂, -NH-C(=O)-O-alkyle en C₁₋₅, -C(=O)-H, -C(=O)-alkyle en C₁₋₅, -C(=O)-NH₂, -C(=O)-NH-alkyle en C₁₋₅, C(=O)-N-(alkyle en C₁₋₅)₂, -S(=O)₂-alkyle en C₁₋₅, -S(=O)₂-phényle, -NH-S(=O)₂-alkyle en C₁₋₅, -S(=O)₂-NH-alkyle en C₁₋₅, cyclohexyle, cyclopentyle, morpholinyle, pipéridinyle, pyrrolidinyle, pipérazinyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, morpholinyle, pipéridinyle, pyrrolidinyle, pipérazinyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH,-CF₃, -SF₅, -CN, -NO₂, -alkyle en C₁₋₅, -0-alkyle en C₁₋₅, -O-CF₃, -S-CF₃, phényle et -0-benzyle,
et les cycles des systèmes cycliques mono- ou polycycliques susmentionnés étant chacun à 5, 6 ou 7 éléments et pouvant chacun éventuellement comprendre 1, 2, 3, 4 ou 5 hétéroatomes en tant qu'élément de cycle, qui sont choisis indépendamment les uns des autres dans le groupe constitué par oxygène, azote et soufre ;
et, sauf indication contraire, le radical susmentionné étant choisi dans le groupe constitué par phényle, naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle, [1,2,3,4]-tétrahydronaphtyle, [1,2,3,4]-tétrahydroquinolinyle, [1,2,3,4]-tétrahydroisoquinolinyle, [1,2,3,4]-tétrahydroquinazolinyle, 2H-benzo[1.4]oxazin-3(4H)-onyle, (3,4)-dihydroquinolin-2(1H)-onyle, [3,4]-dihydro-2H-1,4-benzoxazinyle et benzothiazolyle, ainsi que les radicaux aryle ou hétéroaryle, pouvant à chaque fois éventuellement être substitués avec 1, 2, 3, 4 ou 5 substituants choisis indépendamment les uns des autres dans le groupe constitué par F, Cl, Br, I, -CN, -CF₃, -SF₅, -OH, -0-alkyle en C₁₋₁₀, -NH₂, -NO₂, -O-CF₃, - S-CF₃, -SH, -S-alkyle en C₁₋₅, -alkyle en C₁₋₁₀, -C(=O)-OH, -C(=O)-O-alkyle en C₁₋₅, -O-C(=O)-alkyle en C₁₋₅, - NH-alkyle en C₁₋₅, -N (alkyle en C₁₋₅)₂, -NH-C (=O)-O-alkyle en C₁₋₅, -C(=O)-H, -C(=O)-alkyle en C₁₋₅, -C(=O)-NH₂, - C(=O)-NH-alkyle en C₁₋₅, C(=O)-N-(alkyle en C₁₋₅)₂, - S (=0) ₂-alkyle en C₁₋₅, -S (=0) ₂-phényle, -NH-S (=0) ₂-alkyle en C₁₋₅, -NH-S(=O)₂-alkylène en C₁₋₅-phényle, -NH-S(=O)₂-alkylène en C₁₋₅-naphtyle, -NH-S(=O)₂-phényle, -NH-S(=O)₂-naphtyle, cyclohexyle, cyclopentyle, morpholinyle, pipéridinyle, pipérazinyle, pyrrolidinyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, morpholinyle, pipéridinyle, pipérazinyle, pyrrolidinyle, pyridazinyle, -S(=O)₂-phényle, -0-phényle, -0-benzyle, phényle, -(CH₂)-benzo[b]furanyle, -NH-S(=O)₂-alkylène en C₁₋₅-phényle, - NH-S(=O)₂-alkylène en C₁₋₅-naphtyle, -NH-S(=O)₂-phényle, -NH-S(=O)₂-naphtyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, - NO₂, -alkyle en C₁₋₅, -0-alkyle en C₁₋₅, -O-CF₃, -S-CF₃, phényle et -0-benzyle,
les radicaux hétéroaryle susmentionnés pouvant à chaque fois éventuellement comprendre 1, 2, 3, 4 ou 5 hétéroatomes choisis indépendamment les uns des autres dans le groupe constitué par oxygène, azote et soufre en tant qu'élément de cycle ;
et
les groupes alkylène en C₁₋₅ susmentionnés pouvant à chaque fois éventuellement être substitués avec 1, 2, 3, 4 ou 5 substituants choisis indépendamment les uns des autres dans le groupe constitué par F, Cl, Br, -OH, -SH, -NH₂, -CN et NO₂ ;
à chaque fois éventuellement sous la forme d'un de leurs stéréoisomères purs, notamment des énantiomères ou des diastéréomères, de leurs racémats ou sous la forme d'un mélange de stéréoisomères, notamment des énantiomères ou/ou des diastéréomères, en un rapport de mélange quelconque, ou à chaque fois sous la forme de sels correspondants, ou à chaque fois sous la forme de solvates correspondants.

3. Composés selon la revendication 1 ou 2, **caractérisés en ce que**
R¹ représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle, indényle, (1,4)-benzodioxanyle, (1,2,3,4)-tétrahydronaphtyle, (1,2,3,4)-tétrahydroquinolinyle et (1,2,3,4)-tétrahydroquinazolinyle, le radical pouvant à chaque fois éventuellement être substitué avec 1, 2, 3, 4 ou 5 substituants choisis indépendamment les uns des autres dans le groupe constitué par oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C (=O)-O-C₂H₅, -NH-C(=O)-OC(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, - C(=O)-N-(CH₃)₂, -C(=O)-N-(C₂H₅)₂, -S(=O)₂-CH₃, -S(=O)₂-C₂H₅, -S (=0) ₂-phényle, -NH-S (=0)₂-CH₃, -NH-S (=0) ₂-C₂H₅, - S(=O)₂-NH-CH₃, -S(=O)₂-NH-C₂H₅, cyclohexyle, cyclopentyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
un radical choisi dans le groupe constitué par phényle, naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -0-C (=0) -C₂H₅, -O-C(=O)-C(CH₃)₃, -N(CH₃)₂, -N(C₂H₅)₂, -NH-CH₃, -NH-C₂H₅, -NH-C(=O)-O-CH₃, - NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C (=O)-NH-C₂H₅, -C(=O)-N-(CH₃)₂, -C(=O)-N-(C₂H₅)₂, - S(=O)₂-CH₃, -S(=O)₂-C₂H₅, -S(=0) ₂-phényle, -NH-S (=O)₂-CH₃, -NH-S (=O)₂-C₂H₅, -S(=O)₂-NH-CH₃, -S (=0) ₂-NH-C₂H₅, cyclohexyle, cyclopentyle, pyridinyle, pyridazinyle, - (CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
un groupe -C(=O)-NR⁵R⁶ ;
un groupe -C(=S)-NR⁷R⁸ ;
un groupe -C(=O)-R⁹ ;
un groupe -S(=O)₂-R¹⁰ ;
ou -(CHR¹³)-R¹⁶; -(CHR¹³)-(CHR¹⁴)-R¹⁶ ou (CHR¹³)-(CHR¹⁴)-(CHR¹⁵)-R¹⁶.

4. Composés selon une ou plusieurs des revendications 1 à 3, **caractérisés en ce que**
R² représente un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH et - NH₂ ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle, le radical pouvant à chaque fois éventuellement être substitué avec 1, 2, 3, 4 ou 5 substituants choisis indépendamment les uns des autres dans le groupe constitué par oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C (=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, - C(=O)-N-(CH₃)_{2,} -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, - S(=O)₂-NH-CH₃, -S(=O)₂-NH-C₂H₅, cyclohexyle, cyclopentyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
un radical choisi dans le groupe constitué par phényle, naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle, [1,2,3,4]-tétrahydronaphtyle, [1,2,3,4]-tétrahydroquinolinyle, [1,2,3,4]-tétrahydroisoquinolinyle, [1,2,3,4]-tétrahydroquinazolinyle, [3,4]-dihydro-2H-1,4-benzoxazinyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C (=O)-O-C₂H₅, -C(=O)-O-C(CH₃)₃, -0-C (=0) -CH₃, -0-C (=0) -C₂H₅, -O-C(=O)-C(CH₃)₃, -N(CH₃)₂, -N(C₂H₅)₂, -NH-CH₃, -NH-C₂H₅, -NH-C(=O)-O-CH₃, - NH-C (=O)-O-C₂H₅, -NH-C (=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C (=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-phényle, -NH-S(=O)₂-CH₃, -NH-S(=O)₂-C₂H₅, -NH-S(=O)₂-phényle, -S(=O)₂-NH-CH₃, - S(=O)₂-NH-C₂H₅, cyclohexyle, cyclopentyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, pyridazinyle, *-S*(=O)₂-phényle, -NH-S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
à condition qu'une des positions méta et la position para de ce radical phényle ne soient pas substituées avec des substituants qui sont reliés au radical phényle à chaque fois par un atome identique choisi dans le groupe constitué par l'oxygène, le soufre et l'azote ;
ou - (CHR¹⁷)-R²⁰, -(CHR¹⁷)-(CHR¹⁸)-R²⁰ ou -(CHR¹⁷)-(CHR¹⁸)-(CHR¹⁹)-R²⁰.

5. Composés selon une ou plusieurs des revendications 1 à 3, **caractérisés en ce que** R² représente un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH et - NH₂ ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle, le radical pouvant à chaque fois éventuellement être substitué avec 1, 2, 3, 4 ou 5 substituants choisis indépendamment les uns des autres dans le groupe constitué par oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C (=O)-O-C₂H₅, -NH-C(=O)-OC(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, - C(=O)-N-(CH₃)₂, -C(=O)-N-(C₂H₅)₂, -S(=O)₂-CH₃ et -S(=O)₂-C₂H₅ ;
un radical phényle de formule générale XX 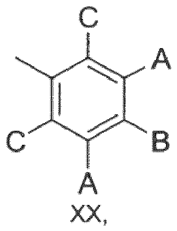 dans laquelle la ligne représente la liaison de ce radical phényle au composé spiro de formule générale I ;
et A, B et C représentent chacun un substituant choisi indépendamment dans le groupe constitué par H, F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, cyclohexyle, cyclopentyle, -C(=O)-OH, - C(=O)-O-CH₃, -C(=O)-O-C₂H₅, -C(=O)-O-C(CH₃)₃, -O-C(=O)-CH₃, -O-C (=0) -C₂H₅, -O-C(=O)-C(CH₃)₃, -N(CH₃)₂, -N(C₂H₅)₂, -NH-CH₃, -NH-C₂H₅, -NH-C (=O)-O-CH₃, -NH-C (=O)-O-C₂H₅, - NH-C(=O)-O-C(CH₃)₃, -C(=O)-H, -C(=O)-CH₃, -C(=O)-C₂H₅, - C(=O)-C(CH₃)₃, -C(=O)-NH₂, -C (=0) -NH-CH₃, -C (=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, - S(=O)₂-NH-CH₃, -S(=O)₂-NH-C₂H₅ et -NH-S(=O)₂-phényle ;
à condition qu'une des positions A et la position B de ce radical phényle ne soient pas substituées avec des substituants qui sont reliés au radical phényle à chaque fois par un atome identique choisi dans le groupe constitué par l'oxygène, le soufre et l'azote ; un radical choisi dans le groupe constitué par naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle, [1,2,3,4]-tétrahydronaphtyle, [1,2,3,4]-tétrahydroquinolinyle, [1,2,3,4]-tétrahydroisoquinolinyle, [1,2,3,4]-tétrahydroquinazolinyle, 2H-benzo[1.4]oxazin-3(4H)-onyle, (3,4)-bihydroquinolin-2(1H)-onyle, [3,4]-bihydro-2H-1,4-benzoxazinyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=0)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-OC(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, - C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, - S(=O)₂-NH-CH₃, -S(=O)₂-NH-C₂H₅, cyclohexyle, cyclopentyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
ou -(CHR¹⁷)-R²⁰, -(CHR¹⁷)-(CHR¹⁸)-R²⁰ ou -(CHR¹⁷)-(CHR¹⁸)-(CHR¹⁹)-R²⁰.

6. Composés selon une ou plusieurs des revendications 1 à 5, **caractérisés en ce que**
R³ représente un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH et -NH₂.

7. Composés selon une ou plusieurs des revendications 1 à 6, **caractérisés en ce que**
R⁴ représente un radical hydrogène,
ou un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH et -NH₂.

8. Composés selon une ou plusieurs des revendications 1 à 7, **caractérisés en ce que**
R⁵ et R⁷ représentent chacun indépendamment l'un de l'autre
un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle, n-eicosanyle, vinyle, 1-propényle, 2-propényle, 1-butényle, 2-butényle, 3-butényle, 2-méthyl-1-propényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH et - NH₂ ;
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle, adamantyle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle, le radical pouvant à chaque fois éventuellement être substitué avec 1, 2, 3, 4 ou 5 substituants choisis indépendamment les uns des autres dans le groupe constitué par oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C (=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, - C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, - S(=O)₂-NH-CH₃, -S(=O)₂-NH-C₂H₅, cyclohexyle, cyclopentyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
un radical choisi dans le groupe constitué par phényle, naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C (=O)-O-C₂H₅, -C(=O)-O-C(CH₃)₃, -0-C (=0) -CH₃, -0-C (=0) -C₂H₅, -O-C(=O)-C(CH₃)₃, -N(CH₃)₂, -N(C₂H₅)₂, -NH-CH₃, -NH-C₂H₅, -NH-C(=O)-O-CH₃, - NH-C (=O)-O-C₂H₅, -NH-C (=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C (=O)-NH-C₂H₅, -C(=O)-N-(CH₃)₂, -C(=O)-N-(C₂H₅)₂, - S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=0) ₂-NH-C₂H₅, cyclohexyle, cyclopentyle, pyridinyle, pyridazinyle, - (CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
ou -(CR²¹R²²)-R²⁵, -(CR²¹R²²)-(CHR²³)-R²⁵, -(CR²¹R²²)-(CHR²³)-O-R²⁵, -(CR²¹R²²)-(CHR²³)-(CHR²⁴)-R²⁵, -(CR²¹R²²)-(CHR²³)-(CHR²⁴)-O-R²⁵, -(CR²¹R²²)-(CHR 23) -(CHR 24) -N (CH₃)-R²⁵ ou - (CR²¹R²²)-(CHR²³)-(CHR²⁴)-N (C₂H₅)-R²⁵.

9. Composés selon une ou plusieurs des revendications 1 à 8, **caractérisés en ce que**
R⁶ et R⁸ représentent chacun un radical hydrogène,
ou un radical choisi dans le groupe constitué par méthyle, éthyle, n-propyle, isopropyle, n-butyle, sec-butyle, isobutyle et tert-butyle.

10. Composés selon une ou plusieurs des revendications 1 à 9, **caractérisés en ce que**
R⁹ représente un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, vinyle, 1-propényle, 2-propényle, 1-butényle, 2-butényle, 3-butényle, 2-méthyl-1-propényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH et - NH₂ ; ou
un radical choisi dans le groupe constitué par phényle, naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, n-pentyle, n-hexyle, n-heptyle, n-octyle, - C(=O)-OH, -C(=O)-O-CH₃, -C (=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, - S(=O)₂-C₂H₅, -S (=0) ₂-phényle, -NH-S (=0) ₂-CH₃, -NH-S(=O)₂-C₂H₅, -S (=O)₂-NH-CH₃, -S (=O)₂-NH-C₂H₅, cyclohexyle, cyclopentyle, morpholinyle, pipéridinyle, pipérazinyle, pyrrolidinyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
-(CR²⁶R²⁷)-R³⁰, -(CR²⁶R²⁷)-(CHR²⁸)-R³⁰, -(CR²⁶R²⁷)-(CHR²⁸) (CHR²⁹)-R³⁰, -CR³¹=CR³²-R³³ ou -C≡C-R³⁴ et
R¹⁰ représente un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, vinyle, 1-propényle, 2-propényle, 1-butényle, 2-butényle, 3-butényle, 2-méthyl-1-propényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH et - NH₂ ;
un radical choisi dans le groupe constitué par 9H-fluorényle, 9H-xanthényle, cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle, indényle, (1,4)-benzodioxanyle, (1,2,3,4)-tétrahydronaphtyle, (1,2,3,4)-tétrahydroquinolinyle et (1,2,3,4)-tétrahydroquinazolinyle, le radical pouvant à chaque fois éventuellement être substitué avec 1, 2, 3, 4 ou 5 substituants choisis indépendamment les uns des autres dans le groupe constitué par oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=0) -CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C (=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, - C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, - S(=O)₂-NH-CH₃, -S(=O)₂-NH-C₂H₅, cyclohexyle, cyclopentyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
un radical choisi dans le groupe constitué par phényle, naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, n-pentyle, n-hexyle, n-heptyle, n-octyle, - C(=O)-OH, -C(=O)-O-CH₃, -C (=O)-O-C₂H₅, -C (=O)-O-C(CH₃)₃, -O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, - S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, morpholinyle, pipéridinyle, pipérazinyle, pyrrolidinyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
-(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰, -CR³¹=CR³²-R³³ ou -C≡C-R³⁴.

11. Composés selon une ou plusieurs des revendications 1 à 10, **caractérisés en ce que**
R¹¹ et R¹² représentent chacun indépendamment l'un de l'autre
un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle et thiomorpholinyle ;
ou un radical choisi dans le groupe constitué par phényle, naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle et benzothiazolyle.

12. Composés selon une ou plusieurs des revendications 1 à 11, **caractérisés en ce que**
R¹³, R¹⁴, R¹⁵, R¹⁷, R¹⁸, R¹⁹, R²¹, R²², R²³, R²⁴, R²⁸, R²⁹ et R³¹ représentent chacun indépendamment les uns des autres
un radical hydrogène ;
un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C (CH₃)₃), n-hexyle, n-heptyle, n-octyle, - (CH₂)-(CH) (C₂H₅)-(CH₂)-(CH₂)-(CH₂)-(CH₃), vinyle, 1-propényle, 2-propényle, 1-butényle, 2-butényle, 3-butényle, éthynyle, 1-propynyle, 2-propynyle, 1-butynyle, 2-butynyle et 3-butynyle ;
ou un radical phényle, qui peut être substitué avec 1, 2, 3, 4 ou 5 substituants choisis indépendamment les uns des autres dans le groupe constitué par F, Cl, Br, I, -CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=0)-O-CH₃, -C (=0) -O-C₂H₅, -C(=O)-O-C(CH₃)₃, -O-C(=O)-CH₃, -0-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -0-phényle, -0-benzyle et phényle.

13. Composés selon une ou plusieurs des revendications 1 à 12, **caractérisés en ce que**
R²⁶ et R²⁷ représentent chacun indépendamment l'un de l'autre
un radical hydrogène ;
un radical choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle, -(CH₂)-(CH)(C₂H₅)-(CH₂)-(CH₂)-(CH₂)-(CH₃), vinyle, 1-propényle, 2-propényle, 1-butényle, 2-butényle, 3-butényle, éthynyle, 1-propynyle, 2-propynyle, 1-butynyle, 2-butynyle et 3-butynyle ;
un radical phényle, qui peut être substitué avec 1, 2, 3, 4 ou 5 substituants choisis indépendamment les uns des autres dans le groupe constitué par F, Cl, Br, I,-CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C (=0) -O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -0-phényle, -0-benzyle et phényle ;
ou -OH.

14. Composés selon une ou plusieurs des revendications 1 à 13, **caractérisés en ce que**
R¹⁶, R²⁰, R²⁵, R³⁰, R³³ et R³⁴ représentent chacun indépendamment les uns des autres
un radical choisi dans le groupe constitué par méthyle, éthyle, n-propyle, isopropyle, n-butyle, sec-butyle, isobutyle, tert-butyle, n-pentyle, sec-pentyle,-(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃),
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle, indényle, (1,4)-benzodioxanyle, (1,2,3,4)-tétrahydronaphtyle, (1,2,3,4)-tétrahydroquinolinyle et (1,2,3,4)-tétrahydroquinazolinyle, le radical pouvant à chaque fois éventuellement être substitué avec 1, 2, 3, 4 ou 5 substituants choisis indépendamment les uns des autres dans le groupe constitué par oxo (=0), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, - C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅, - S(=O)₂-NH-CH₃, -S(=O)₂-NH-C₂H₅, cyclohexyle, cyclopentyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
ou un radical choisi dans le groupe constitué par phényle, naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, n-pentyle, n-hexyle, n-heptyle, n-octyle, -C(=O)-OH, -C(=O)-O-CH₃, - C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C (=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅,-C(=O)-N-(CH₃)₂, -C(=O)-N-(C₂H₅)₂, -S(=O)₂-CH₃, -S(=O)₂-C₂H₅, -S(=O)₂-phényle, -NH-S(=O)₂-CH₃, -NH-S(=O)₂-C₂H₅,-S(=O)₂-NH-CH₃, -S(=O)₂-NH-C₂H₅, cyclohexyle, cyclopentyle, morpholinyle, pipéridinyle, pipérazinyle, pyrrolidinyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, morpholinyle, pipéridinyle, pipérazinyle, pyrrolidinyle, pyridazinyle, -S(=O)₂-phényle, -0-phényle, -0-benzyle, phényle, -(CH₂)-benzo[b]furanyle et benzyle pouvant à chaque fois éventuellement être substituée avec 1, 2, 3, 4 ou 5 substituants choisis indépendamment les uns des autres dans le groupe constitué par F, Cl, Br, -OH,-CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle.

15. Composés selon une ou plusieurs des revendications 1 à 14, **caractérisés en ce que**
m représente 0, 1 ou 2.

16. Composés selon une ou plusieurs des revendications 1 à 15, **caractérisés en ce que**
n représente 0, 1 ou 2.

17. Composés selon une ou plusieurs des revendications 1 à 16, **caractérisés en ce que**
R³² représente un radical hydrogène ;
un radical choisi dans le groupe constitué par méthyle, éthyle, n-propyle, isopropyle, n-butyle, sec-butyle, isobutyle, tert-butyle, n-pentyle, sec-pentyle,-(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃),
un radical choisi dans le groupe constitué par phényle, naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, n-pentyle, n-hexyle, n-heptyle, n-octyle,-C(=O)-OH, -C (=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C (=0) -NH-CH₃, -C(=O)-NH-C₂H₅, -C(=O)-N-(CH₃)₂, -C(=O)-N-(C₂H₅)₂, -S(=O)₂-CH₃,-S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
ou -(CH₂)_{cc}-R³⁵ avec cc = 1, 2, 3 ou 4, ou -CH=CH-R³⁶.

18. Composés selon une ou plusieurs des revendications 1 à 17, **caractérisés en ce que**
R³⁵ et R³⁶ représentent chacun indépendamment l'un de l'autre un radical choisi dans le groupe constitué par phényle et naphtyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, n-pentyle, n-hexyle, n-heptyle, n-octyle,-C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂,-N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃,-S(=O)₂-C₂H₅, -S(=O)₂-phényle, -NH-S (=0) ₂-CH₃, -NH-S(=O)₂-C₂H₅, -S(=O)₂-NH-CH₃ et -S(=O)₂-NH-C₂H₅.

19. Composés selon une ou plusieurs des revendications 1 à 18, **caractérisés en ce que**
m représente 0, 1, 2, 3 ou 4 ;
n représente 0, 1 ou 2 ;
R¹ représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle et thiomorpholinyle ;
un radical choisi dans le groupe constitué par indolyle, isoindolyle, benzo[b]furanyle, benzo[b]thiophényle, indazolyle, quinazolinyle, quinoxalinyle, quinolinyle, isoquinolinyle, benzimidazolyle, benzoxazolyle, benzotriazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I,-CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃,-NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, - S(=O)₂-CH₃, -S(=O)₂-C₂H₅, -NH-S(=O)₂-CH₃, -NH-S(=O)₂-C₂H₅, -S(=O)₂-NH-CH₃, -S(=O)₂-NH-C₂H₅ ;
un groupe -C(=O)-NR⁵R⁶ ;
un groupe -C(=S)-NR⁷R⁸ ;
un groupe -C(=O)-R⁹ ;
un groupe -S(=O)₂-R¹⁰ ;
ou -(CHR¹³)-R¹⁶ ; -(CHR¹³)-(CHR¹⁴)-R¹⁶ ou (CHR¹³)-(CHR¹⁴)-(CHR¹⁵)-R¹⁶ ;
R² représente un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle et thiomorpholinyle ;
un radical phényle, qui peut être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, cyclopentyle, cyclohexyle, -S(=O)₂-CH₃,-S(=O)₂-C₂H₅, -NH-S(=O)(CH₃, -NH-S (=O)₂-C₂H₅, -NH-S(=O)₂-phényle, -S(=O)₂-NH-CH₃ et -S(=O)₂-NH-C₂H₅ ;
à condition qu'une des positions méta et la position para de ce radical phényle ne soient pas substituées avec des substituants qui sont reliés au radical phényle à chaque fois par un atome identique choisi dans le groupe constitué par l'oxygène, le soufre et l'azote ;
un radical choisi dans le groupe constitué par naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle, [1,2,3,4]-tétrahydronaphtyle, [1,2,3,4]-tétrahydroquinolinyle, [1,2,3,4]-tétrahydroisoquinolinyle, 2H-benzo[1.4]oxazin-3(4H)-onyle, (3,4)-dihydroquinolin-2(1H)-onyle, [1,2,3,4]-tétrahydroquinazolinyle, [3,4]-dihydro-2H-1,4-benzoxazinyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -S(=O)₂-CH₃, -S(=O)₂-C₂H₅, -NH-S(=O)₂-CH₃, -NH-S(=O)₂-C₂H₅, -S(=O)₂-NH-CH₃ et -S(=O)₂-NH-C₂H₅ ;
ou -(CHR¹⁷)-R²⁰, -(CHR¹⁷)-(CHR¹⁸)-R²⁰ ou -(CHR¹⁷)-(CHR¹⁸)-(CHR¹⁹)-R²⁰ ;
R³ représente un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle ;
R⁴ représente un radical hydrogène,
ou un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle ;
R⁵ et R⁷ représentent chacun indépendamment l'un de l'autre un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle, n-eicosanyle, vinyle, 1-propényle, 2-propényle, 1-butényle, 2-butényle, 3-butényle, 2-méthyl-1-propényle, éthynyle, 1-propynyle, 2-propynyle, 1-butynyle, 2-butynyle et 3-butynyle, le radical pouvant à chaque fois éventuellement être substitué avec 1, 2, 3, 4 ou 5 substituants choisis indépendamment les uns des autres dans le groupe constitué par F, Cl et Br ;
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle, adamantyle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle, le radical pouvant à chaque fois éventuellement être substitué avec 1, 2 ou 3 substituants choisis indépendamment les uns des autres dans le groupe constitué par méthyle, éthyle, n-propyle, isopropyle, n-butyle, sec-butyle, isobutyle et tert-butyle ;
un radical choisi dans le groupe constitué par phényle, (1,3)-benzodioxolyle, (1,4)-benzodioxanyle, naphtyle, thiophényle, furanyle, pyrrolyle, pyrazolyle, pyranyle, pyridinyle, imidazolyle, thiazolyle, oxazolyle, isoxazolyle, pyridazinyle, pyrazinyle et pyrimidinyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C (=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃,-NH-C (=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂,-S(=O)₂-CH₃, -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle,-(CH₂)-benzo[b]furanyle, -0-phényle, -0-benzyle, phényle et benzyle, la partie cyclique des radicaux pyridinyle, cyclopentyle, cyclohexyle, pyridazinyle, -S(=O)₂-phényle, -0-phényle, -0-benzyle, phényle, -(CH₂)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
ou -(CR²¹R²²)-R²⁵, -(CR²¹R²²)-(CHR²³)-R²⁵, -(CR²¹R²²)-(CHR²³)-O-R²⁵, -(CR²¹R²²)-(CHR²³)-(CHR²⁴)-R²⁵, -(CR²¹R²²)-(CHR²³)-(CHR²⁴)-O-R²⁵, -(CR²¹R²²)-(CHR²³)-(CHR²⁴)-N(CH₃)-R²⁵ ou -(CR²¹R²²)-(CHR²³)-(CHR²⁴)-N(C₂H₅)-R²⁵ ;
R⁶ et R⁸ représentent chacun un radical hydrogène ;
ou un radical choisi dans le groupe constitué par méthyle, éthyle, n-propyle, isopropyle, n-butyle, sec-butyle, isobutyle et tert-butyle ;
R⁹ représente un radical choisi dans le groupe constitué par phényle, naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -O-CF₃, -S-CF₃,-SF₅, -OH, -O-CH₃, -O-C₂H₅, -NH-S(=O)₂-CH₃, morpholinyle, pipéridinyle, pyrrolidinyle, pipérazinyle, phényle, méthyle, éthyle, n-propyle, isopropyle, n-butyle, sec-butyle, isobutyle, tert-butyle, n-pentyle, n-hexyle, n-heptyle et n-octyle ;
-(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰, -CR³¹=CR³²-R³³ ou -C≡C-R³⁴ ; et
R¹⁰ représente un radical choisi dans le groupe constitué par 9H-fluorényle, 9H-xanthényle, phényle, naphtyle, (1,3)-benzodioxolyle, (1,4)-benzodioxanyle, thiophényle, furanyle, pyrrolyle, pyrazolyle, pyranyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinoxalinyle, quinolinyle, isoquinolinyle, benzimidazolyle, benzoxazolyle, benzotriazolyle, benzisoxazolyle et benzothiazolyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -O-CF₃, -S-CF₃, -SF₅, -OH, -O-CH₃, -O-C₂H₅, -NH-S(=O)₂-CH₃, morpholinyle, pipéridinyle, pyrrolidinyle, pipérazinyle, phényle, méthyle, éthyle, n-propyle, isopropyle, n-butyle, sec-butyle, isobutyle, tert-butyle, n-pentyle, n-hexyle, n-heptyle et n-octyle ;
-(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰, -CR³¹=CR³²-R³³-C≡C-R³⁴ ;
R¹³, R¹⁴, R¹⁵, R¹⁷, R¹⁸, R¹⁹, R²¹, R²², R²³, R²⁴, R²⁸, R²⁹ et R³¹, représentent chacun, indépendamment les uns des autres
un radical hydrogène ;
un radical choisi dans le groupe constitué par méthyle, éthyle, n-propyle, isopropyle, n-butyle, sec-butyle, isobutyle et tert-butyle ;
ou un radical phényle, qui peut être substitué avec 1, 2, 3, 4 ou 5 substituants choisis indépendamment les uns des autres dans le groupe constitué par F, Cl, Br, I, -CN, -CF₃, -O-CH₃, -O-C₂H₅,-NO₂, -O-CF₃, méthyle, éthyle, n-propyle, isopropyle, n-butyle, sec-butyle, isobutyle et tert-butyle ;
R¹⁶ représente un radical choisi dans le groupe constitué par phényle, naphtyle, thiophényle, furanyle, pyrrolyle, pyrazolyle, pyrazinyle et pyridinyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C (=0) -O-C₂H₅, -C(=O)-O-C(CH₃)₃, -O-C(=O)-CH₃, -0-C(=O)-C₂H₅, -O-C(=O)-C(CH₃)₃, -S(=O)₂-CH₃, -S(=O)₂-C₂H₅, - NH-S(=O)₂-CH₃, -NH-S(=O)₂-C₂H₅, -S(=O)₂-NH-CH₃ et -S(=O)₂-NH-C₂H₅ ;
R²⁰ représente un radical choisi dans le groupe constitué par phényle et naphtyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, n-pentyle, n-hexyle, n-heptyle et n-octyle ;
R²⁵ représente un radical choisi dans le groupe constitué par méthyle, éthyle, n-propyle, isopropyle, n-butyle, sec-butyle, isobutyle et tert-butyle ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle et dithiolanyle ;
ou un radical choisi dans le groupe constitué par phényle, naphtyle, (1,3)-benzodioxolyle, (1,4)-benzodioxanyle, thiophényle et furanyle, le radical pouvant à chaque fois éventuellement être substitué avec 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=0)-O-CH₃, -C(=O)-O-C₂H₅, -C(=O)-O-C(CH₃)₃, -O-C(=O)-CH₃, -0-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-OC(CH₃)₃, -C(=O)-H, -C(=O)-CH₃, -C(=O)-C₂H₅ et -C(=O)-C(CH₃)₃ ;
R²⁶ et R²⁷ représentent chacun indépendamment l'un de l'autre
un radical hydrogène ;
un radical choisi dans le groupe constitué par méthyle, éthyle, n-propyle, isopropyle, n-butyle, sec-butyle, isobutyle et tert-butyle ;
un radical phényle, qui peut être substitué avec 1, 2, 3, 4 ou 5 substituants choisis indépendamment les uns des autres dans le groupe constitué par F, Cl, Br, I, -CN, -CF₃, -O-CH₃, -O-C₂H₅, -NO₂, -O-CF₃, méthyle, éthyle, n-propyle, isopropyle, n-butyle, sec-butyle, isobutyle et tert-butyle ;
ou -OH ;
R³² représente un radical hydrogène ;
un radical choisi dans le groupe constitué par méthyle, éthyle, n-propyle, isopropyle, n-butyle, sec-butyle, isobutyle et tert-butyle ;
un radical choisi dans le groupe constitué par phényle, naphtyle, furanyle et thiophényle, qui peut éventuellement être substitué avec 1, 2, 3, 4 ou 5 substituants choisis indépendamment les uns des autres dans le groupe constitué par -SF₅, F, Cl, Br, I, -CF₃, -O-CF₃, -S-CF₃, -O-CH₃, -O-C₂H₅, phényle, -S-CH₃,-S-C₂H₅, cyclopentyle, cyclohexyle, méthyle, éthyle, n-propyle, isopropyle, n-butyle, sec-butyle, isobutyle et tert-butyle ;
-(CH₂)_{cc}-R³⁵ avec cc = 1, 2 ou 3, ou -CH=CH-R³⁶ ;
R³⁰, R³³ et R³⁴ représentent chacun indépendamment les uns des autres
un radical choisi dans le groupe constitué par méthyle, éthyle, n-propyle, isopropyle, n-butyle, sec-butyle, isobutyle et tert-butyle ;
ou un radical choisi dans le groupe constitué par phényle, pyridinyle et naphtyle, le radical pouvant à chaque fois éventuellement être substitué avec 1, 2, 3, 4 ou 5 substituants choisis indépendamment les uns des autres dans le groupe constitué par -SF₅, F, Cl, Br,-CF₃, -O-CF₃, -S-CF₃, phényle, morpholinyle, pipéridinyle, pyrrolidinyle, pipérazinyle, cyclopentyle, cyclohexyle, -OH, -O-CH₃, -O-C₂H₅, méthyle, éthyle, n-propyle, isopropyle, n-butyle, sec-butyle, isobutyle, tert-butyle, n-pentyle, n-hexyle, n-heptyle et n-octyle ;
et
R³⁵ et R³⁶ représentent chacun indépendamment l'un de l'autre
un radical phényle, qui peut être substitué avec 1, 2, 3, 4 ou 5 substituants choisis indépendamment les uns des autres dans le groupe constitué par F, Cl, Br, I, -CN, -CF₃, -O-CH₃, -O-C₂H₅, -NO₂, -O-CF₃, méthyle, éthyle, n-propyle, isopropyle, n-butyle, sec-butyle, isobutyle et tert-butyle ;
à chaque fois éventuellement sous la forme d'un de leurs stéréoisomères purs, notamment des énantiomères ou des diastéréomères, de leurs racémats ou sous la forme d'un mélange de stéréoisomères, notamment des énantiomères ou/ou des diastéréomères, en un rapport de mélange quelconque, ou à chaque fois sous la forme de sels correspondants, ou à chaque fois sous la forme de solvates correspondants.

20. Composés selon une ou plusieurs des revendications 1 à 19, **caractérisés en ce que**
m représente 0, 1 ou 2 ;
n représente 0, 1 ou 2 ;
R¹ représente un radical choisi dans le groupe constitué par benzimidazolyle, benzoxazolyle, benzotriazolyle, benzisoxazolyle et benzothiazolyle, le radical pouvant à chaque fois être substitué avec 1, 2, 3, 4 ou 5 substituants choisis indépendamment les uns des autres dans le groupe constitué par F, Cl, Br, -CF₃,
-O-CF₃, -S-CF₃, méthyle, éthyle, n-propyle, isopropyle, n-butyle, sec-butyle, isobutyle et tert-butyle ;
un groupe -C(=O)-NR⁵R⁶ ;
un groupe -C(=S)-NR⁷R⁸ ;
un groupe -C(=O)-R⁹
ou un groupe -S(=O)₂-R¹⁰ ;
R² représente un radical choisi dans le groupe constitué par méthyle, éthyle, n-propyle, isopropyle, n-butyle, sec-butyle, isobutyle, tert-butyle, n-pentyle, sec-pentyle, n-hexyle et n-heptyle ;
un radical phényle de formule générale XX 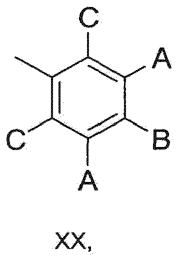 dans lequel la ligne représente la liaison de ce radical phényle au composé spiro de formule générale I ;
A et B représentent chacun indépendamment l'un de l'autre un substituant choisi dans le groupe constitué par H, F, Cl, Br, I, -CF₃, -SF₅, -OH, -O-CH₃, -O-C₂H₅,-O-CF₃, -S-CF₃, méthyle, éthyle, n-propyle, isopropyle, n-butyle, sec-butyle, isobutyle, tert-butyle, cyclopentyle, cyclohexyle, -NH-S(=O)₂-CH₃ et -NH-S(=O)₂-phényle ;
à condition qu'une des positions A et la position B de ce radical phényle ne soient pas substituées avec des substituants qui sont reliés au radical phényle à chaque fois par un atome identique choisi dans le groupe constitué par l'oxygène, le soufre et l'azote ;
C représente à chaque fois H ;
un radical choisi dans le groupe constitué par naphtyle, quinolinyle, (1,4)-benzodioxanyle, (1,3)-benzodioxolyle, pyridinyle, thiazolyle et oxazolyle, le radical pouvant à chaque fois éventuellement être substitué avec 1, 2, 3, 4 ou 5 substituants choisis indépendamment les uns des autres dans le groupe constitué par F, Cl, Br, I, -O-CH₃, -O-C₂H₅, méthyle, éthyle, n-propyle, isopropyle, n-butyle, sec-butyle, isobutyle, tert-butyle, -S(=O)₂-CH₃, -S(=O)₂-C₂H₅, -NH-S(=O)₂-CH₃, -NH-S(=O)₂-C₂H₅, -S(=O)₂-NH-CH₃ et -S(=O)₂-NH-C₂H₅ ;
ou -(CHR¹⁷)-R²⁰, - (CHR¹⁷)-(CHR¹⁸)-R²⁰ ou -(CHR¹⁷)-(CHR¹⁸)-(CHR¹⁹)-R²⁰ ;
R³ représente un radical choisi dans le groupe constitué par méthyle, éthyle, n-propyle, isopropyle, n-butyle, sec-butyle, isobutyle, tert-butyle et n-pentyle ;
R⁴ représente un radical hydrogène ;
R⁵ et R⁷ représentent chacun indépendamment les uns des autres un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle, n-eicosanyle, vinyle, 1-propényle, 2-propényle, 1-butényle, 2-butényle, 3-butényle et 2-méthyl-1-propényle, le radical pouvant à chaque fois éventuellement être substitué avec 1, 2, 3, 4 ou 5 substituants choisis indépendamment les uns des autres dans le groupe constitué par F, Cl et Br ;
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle et adamantyle, le radical pouvant à chaque fois éventuellement être substitué avec 1, 2 ou 3 substituants choisis indépendamment les uns des autres dans le groupe constitué par méthyle, éthyle, n-propyle, isopropyle, n-butyle, sec-butyle, isobutyle et tert-butyle ;
un radical choisi dans le groupe constitué par phényle, naphtyle, (1,4)-benzodioxanyle et pyridinyle, le radical pouvant à chaque fois éventuellement être substitué avec 1, 2, 3, 4 ou 5 substituants choisis indépendamment les uns des autres dans le groupe constitué par -SF₅, F, Cl, Br, I, -CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅,-C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -0-phényle, -0-benzyle et phényle, la partie cyclique des radicaux cyclopentyle, cyclohexyle, -0-phényle, -0-benzyle et phényle pouvant à chaque fois être substituée avec 1, 2, 3, 4 ou 5 substituants choisis indépendamment les uns des autres dans le groupe constitué par F, Cl, Br, méthyle, éthyle, n-propyle, isopropyle, n-butyle, sec-butyle, isobutyle et tert-butyle ;
ou -(CR²¹R²²)-R²⁵, -(CR²¹R²²)-(CHR²³)-R²⁵, -(CR²¹R²²)-(CHR²³)-O-R²⁵, -(CR²¹R²²)-(CHR²³)-(CHR²⁴)-R²⁵, -(CR²¹R²²)-(CHR²³)-(CHR²⁴)-O-R²⁵, -(CR²¹R²²)-(CHR²³)-(CHR²⁴)-N(CH₃)-R²⁵ ou- (CR²¹R²²)-(CHR²³)-(CHR²⁴)-N(C₂H₅)-R²⁵ ;
R⁶ et R⁸ représentent chacun un radical hydrogène ;
ou un radical méthyle ou éthyle ;
R⁹ représente un radical choisi dans le groupe constitué par phényle, pyridinyle et naphtyle, le radical pouvant à chaque fois éventuellement être substitué avec 1, 2, 3, 4 ou 5 substituants choisis indépendamment les uns des autres dans le groupe constitué par F, Cl, Br, I, -CF₃, -O-CF₃, -S-CF₃, -OH,-O-CH₃, -O-C₂H₅, -NH-S (=0) ₂-CH₃, morpholinyle, pipéridinyle, pyrrolidinyle, pipérazinyle, phényle, méthyle, éthyle, n-propyle, isopropyle, n-butyle, sec-butyle, isobutyle, tert-butyle, n-pentyle, n-hexyle, n-heptyle et n-octyle ; -(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰, -CR³¹=CR³²-R³³ ou -C≡C-R³⁴ ;
R¹⁰ représente un radical choisi dans le groupe constitué par phényle et naphtyle, le radical pouvant à chaque fois éventuellement être substitué avec 1, 2, 3, 4 ou 5 substituants choisis indépendamment les uns des autres dans le groupe constitué par F, Cl, Br, I, -CF₃, -OH, -O-CH₃, -O-C₂H₅, méthyle, éthyle, n-propyle, isopropyle, n-butyle, sec-butyle, isobutyle et tert-butyle ;
- (CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰, -CR³¹=CR³²-R³³ ou -C≡C-R³⁴ ;
R¹⁷, R¹⁸, R¹⁹, R²³, R²⁴, R²⁸ et R²⁹ représentent chacun indépendamment les uns des autres
un radical hydrogène ;
un radical choisi dans le groupe constitué par méthyle, éthyle, n-propyle, isopropyle, n-butyle, sec-butyle, isobutyle et tert-butyle ;
ou un radical phényle ;
R²⁰ représente un radical phényle ;
R²¹ et R²² représentent chacun indépendamment l'un de l'autre
un radical hydrogène ;
un radical choisi dans le groupe constitué par méthyle, éthyle, n-propyle, isopropyle, n-butyle, sec-butyle, isobutyle et tert-butyle ;
ou un radical phényle ;
R²⁵ représente un radical choisi dans le groupe constitué par méthyle, éthyle, n-propyle, isopropyle, n-butyle, sec-butyle, isobutyle et tert-butyle ;
un radical choisi dans le groupe constitué par cyclopentyle, cyclohexyle, tétrahydrofuranyle, tétrahydrothiophényle, pyrrolidinyle, morpholinyle, pipéridinyle et pipérazinyle ;
ou un radical choisi dans le groupe constitué par phényle, naphtyle, (1,3)-benzodioxolyle, (1,4)-benzodioxanyle, thiophényle et furanyle, le radical pouvant à chaque fois éventuellement être substitué avec 1, 2, 3, 4 ou 5 substituants choisis indépendamment les uns des autres dans le groupe constitué par F, Cl, Br, -OH, -O-CH₃, -O-C₂H₅, méthyle, éthyle, n-propyle, isopropyle, n-butyle, sec-butyle, isobutyle et tert-butyle ;
R²⁶ et R²⁷ représentent chacun indépendamment l'un de l'autre
un radical hydrogène ;
un radical choisi dans le groupe constitué par méthyle, éthyle, n-propyle, isopropyle, n-butyle, sec-butyle, isobutyle et tert-butyle ;
un radical phényle ou -OH ;
R³⁰ représente un radical phényle, qui peut être substitué avec 1, 2, 3, 4 ou 5 substituants choisis indépendamment les uns des autres dans le groupe constitué par F, Cl, Br, -NH-S(=O)₂-CH₃, méthyle, éthyle, n-propyle, isopropyle, n-butyle, sec-butyle, isobutyle et tert-butyle ;
R³¹ représente un radical hydrogène ;
ou un radical choisi dans le groupe constitué par méthyle, éthyle, n-propyle, isopropyle, n-butyle, sec-butyle, isobutyle et tert-butyle ;
R³² représente un radical hydrogène ;
un radical choisi dans le groupe constitué par méthyle, éthyle, n-propyle, isopropyle, n-butyle, sec-butyle, isobutyle et tert-butyle ;
un radical choisi dans le groupe constitué par Phényle, naphtyle, furanyle et thiophényle, le radical pouvant éventuellement être substitué avec 1, 2, 3, 4 ou 5 substituants choisis indépendamment les uns des autres dans le groupe constitué par -SF₅, F, Cl, Br, I, -CF₃, -O-CF₃, -S-CF₃, -O-CH₃, -O-C₂H₅, phényle, -S-CH₃,-S-C₂H₅, cyclopentyle, cyclohexyle, méthyle, éthyle, n-propyle, isopropyle, n-butyle, sec-butyle, isobutyle et tert-butyle ;
-CH₂-R³⁵ ou -CH=CH-R³⁶ ;
R³³ et R³⁴ représentent chacun indépendamment l'un de l'autre
un radical choisi dans le groupe constitué par méthyle, éthyle, n-propyle, isopropyle, n-butyle, sec-butyle, isobutyle et tert-butyle ;
ou un radical choisi dans le groupe constitué par phényle, pyridinyle et naphtyle, le radical pouvant à chaque fois éventuellement être substitué avec 1, 2, 3, 4 ou 5 substituants choisis indépendamment les uns des autres dans le groupe constitué par -SF₅, F, Cl, Br,-CF₃, -O-CF₃, -S-CF₃, phényle, morpholinyle, pipéridinyle, pyrrolidinyle, pipérazinyle, cyclopentyle, cyclohexyle, -OH, -O-CH₃, -O-C₂H₅, méthyle, éthyle, n-propyle, isopropyle, n-butyle, sec-butyle, isobutyle, tert-butyle, n-pentyle, n-hexyle, n-heptyle et n-octyle ;
et
R³⁵ et R³⁶ représentent chacun un radical phényle ;
à chaque fois éventuellement sous la forme d'un de leurs stéréoisomères purs, notamment des énantiomères ou des diastéréomères, de leurs racémats ou sous la forme d'un mélange de stéréoisomères, notamment des énantiomères ou/ou des diastéréomères, en un rapport de mélange quelconque, ou à chaque fois sous la forme de sels correspondants, ou à chaque fois sous la forme de solvates correspondants.

21. Composés selon une ou plusieurs des revendications 1 à 20, **caractérisés en ce que**
m représente 0, 1 ou 2 ;
n représente 0, 1 ou 2 ;
R¹ représente un radical choisi dans le groupe constitué par benzimidazolyle, benzoxazolyle, benzotriazolyle, benzisoxazolyle et benzothiazolyle, le radical pouvant à chaque fois être substitué avec 1, 2, 3, 4 ou 5 substituants choisis indépendamment les uns des autres dans le groupe constitué par F, Cl, Br, -CF₃, -O-CF₃, -S-CF₃, méthyle, éthyle, n-propyle, isopropyle, n-butyle, sec-butyle, isobutyle et tert-butyle ;
un groupe -C(=O)-NR⁵R⁶ ;
un groupe -C(=S)-NR⁷R⁸ ;
un groupe -C(=O)-R⁹
ou un groupe -S(=O)₂-R¹⁰ ;
R² représente un radical tert-butyle ;
un radical choisi dans le groupe constitué par phényle, 2-méthanesulfonamido-phényle, 2-éthanesulfonamido-phényle, 2-trifluorométhyl-phényle, 2-trifluorométhylsulfanyl-phényle, 2-éthyl-phényle, 2-tert-butyl-phényle, 2-éthylamino-sulfonyl-phényle, 2-méthylamino-sulfonyl-phényle, 2-bromo-phényle, 2-chloro-phényle, 2-fluoro-phényle, 2-méthyl-phényle, 2-trifluorométhoxy-phényle, 2-méthoxy-phényle, 2-éthoxy-phényle, 2-propyl-phényle, 2-iodo-phényle, 3-chloro-phényle, 3-méthyl-phényle, 3-tert-butyl-phényle, 3-trifluorométhylsulfanyl-phényle, 3-trifluorométhyl-phényle, 3-méthanesulfonamido-phényle, 3-éthanesulfonamido-phényle, 3-fluoro-phényle, 3-propyl-phényle, 3-isopropyl-phényle, 3-bromo-phényle, 3-méthoxy-phényle, 3-éthyl-phényle, 3-éthylamino-sulfonyl-phényle, 3-méthylamino-sulfonyl-phényle, 3-éthoxy-phényle, 3-trifluorométhoxy-phényle, 3-iodophényle, 4-méthylamino-sulfonyl-phényle, 4-éthylamino-sulfonyl-phényle, 4-méthanesulfonamido-phényle, 4-éthanesulfonamido-phényle, 4-bromo-phényle, 4-méthoxy-phényle, 4-chloro-phényle, 4-fluoro-phényle, 4-tert-butyl-phényle, 4-trifluorométhylsulfanyl-phényle, 4-méthyl-phényle, 4-isopropyl-phényle, 4-trifluorométhyl-phényle, 4-propyl-phényle, 4-iodo-phényle, 4-trifluorométhoxy-phényle, 4-éthyl-phényle, 4-éthoxy-phényle, 2-fluoro-3-trifluorométhylphényle, (2,3)-difluorophényle, (2,3)-diméthyl-phényle, (2,3)-dichlorophényle, 3-fluoro-2-trifluorométhylphényle, (2,4)-dichloro-phényle, (2,4)-difluorophényle, 4-fluoro-2-trifluorométhyl-phényle, (2,4)-diméthoxyphényle, 2-chloro-4-fluoro-phényle, (2,4)-dibromo-phényle, 2-fluoro-4-trifluorométhyl-phényle, (2,5)-difluoro-phényle, 2-fluoro-5-trifluorométhyl-phényle, 5-fluoro-2-trifluorométhyl-phényle, 5-chloro-2-trifluorométhyl-phényle, 5-bromo-2-trifluorométhyl-phényle, (2,5)-diméthoxy-phényle, (2,5)-bis-trifluorométhyl-phényle, (2,5)-dichloro-phényle, (2,5)-dibromo-phényle, 2-fluoro-6-trifluorométhyl-phényle, (2,6)-diméthoxy-phényle, (2,6)-diméthyl-phényle, (2,6)-dichloro-phényle, 2-chloro-6-fluoro-phényle, 2-bromo-6-chloro-phényle, 2-bromo-6-fluoro-phényle, (2,6)-difluoro-phényle, (2,6)-difluoro-3-méthyl-phényle, (2,6)-dibromo-phényle, (2,6)-dichlorophényle, 3-chloro-2-fluoro-phényle, (3,4)-dichlorophényle, 4-fluoro-3-trifluorométhylphényle, 3-fluoro-4-trifluorométhyl-phényle, (3,4)-difluoro-phényle, 4-chloro-3-trifluorométhyle, 4-bromo-3-méthyl-phényle, 4-bromo-5-méthyl-phényle, 3-chloro-4-fluoro-phényle, (3,4)-dibromo-phényle, 4-chloro-3-méthyl-phényle, 4-bromo-3-méthyl-phényle, 4-fluoro-3-méthyl-phényle, (3,5)-diméthoxy-phényle, (3,5)-bis-trifluorométhyl-phényle, (3,5)-difluoro-phényle, (3,5)-dichloro-phényle, 3-fluoro-5-trifluorométhyl-phényle, 5-fluoro-3-trifluorométhyl-phényle, (3,5)-dibromo-phényle, 5-chloro-4-fluoro-phényle, 5-bromo-4-méthyl-phényle, (2,3,4)-trifluorophényle, (2,3,4)-trichlorophényle, (2,3,6)-trifluoro-phényle, 5-chloro-2-méthoxy-phényle, (2,3)-difluoro-4-méthyl-phényle, (2,4,5)-trifluoro-phényle, (2,4,5)-trichloro-phényle, (2,4)-dichloro-5-fluoro-phényle, (2,4,6)-trichloro-phényle, (2,4,6)-triméthylphényle, (2,4,6)-trifluoro-phényle, (2,4,6)-triméthoxy-phényle, (2,3,4,5)-tétrafluoro-phényle, 4-méthoxy-2,3,6-triméthyl-phényle, 4-méthoxy-2,3,6-triméthyl-phényle, 4-chloro-2,5-diméthyl-phényle, 2-chloro-6-fluoro-3-méthyl-phényle, 6-chloro-2-fluoro-3-méthyle, (2,3,4,5,6)-pentafluoro-phényle, 3-fluoro-4-méthylsulfonamido-phényle, 3-chloro-4-méthylsulfonamido-phényle, 3-bromo-4-méthylsulfonamido-phényle, 3-méthoxy-4-méthylsulfonamido-phényle, 3-hydroxy-4-méthylsulfonamido-phényle, 3-trifluorométhyl-4-méthylsulfonamido-phényle, 3-trifluorométhoxy-4-méthylsulfonamido-phényle, 3-méthyl-4-méthylsulfonamido-phényle, 3-éthyl-4-méthylsulfonamido-phényle, 3-isopropyl-4-méthylsulfonamido-phényle, 3-propyl-4-méthylsulfonamido-phényle, 3-tert-butyl-4-méthylsulfonamido-phényle, 3-fluoro-4-phénylsulfonamido-phényle, 3-chloro-4-phénylsulfonamido-phényle, 3-bromo-4-phénylsulfonamido-phényle, 3-méthoxy-4-phénylsulfonamido-phényle, 3-hydroxy-4-phénylsulfonamido-phényle, 3-trifluorométhyl-4-phénylsulfonamido-phényle, 3-trifluorométhoxy-4-phénylsulfonamido-phényle, 3-méthyl-4-phénylsulfonamido-phényle, 3-éthyl-4-phénylsulfonamido-phényle, 3-isopropyl-4-phénylsulfonamido-phényle, 3-propyl-4-phénylsulfonamido-phényle, 3-tert-butyl-4-phénylsulfonamido-phényle, 4-fluoro-3-méthylsulfonamido-phényle, 4-chloro-3-méthylsulfonamido-phényle, 4-bromo-3-méthylsulfonamido-phényle, 4-méthoxy-3-méthylsulfonamido-phényle, 4-hydroxy-3-méthylsulfonamido-phényle, 4-trifluorométhyl-3-méthylsulfonamido-phényle, 4-trifluorométhoxy-3-méthylsulfonamido-phényle, 4-méthyl-3-méthylsulfonamido-phényle, 4-éthyl-3-méthylsulfonamido-phényle, 4-isopropyl-3-méthylsulfonamido-phényle, 4-propyl-3-méthylsulfonamido-phényle, 4-tert-butyl-3-méthylsulfonamido-phényle, 4-fluoro-3-phénylsulfonamido-phényle, 4-chloro-3-phénylsulfonamido-phényle, 4-bromo-3-phénylsulfonamido-phényle, 4-méthoxy-3-phénylsulfonamido-phényle, 4-hydroxy-3-phénylsulfonamido-phényle, 4-trifluorométhyl-3-phénylsulfonamido-phényle, 4-trifluorométhoxy-3-phénylsulfonamido-phényle, 4-méthyl-3-phénylsulfonamido-phényle, 4-éthyl-3-phénylsulfonamido-phényle, 4-isopropyl-3-phénylsulfonamido-phényle, 4-propyl-3-phénylsulfonamido-phényle, 4-tert-butyl-3-phénylsulfonamido-phényle, 2-cyclohexyl-phényle, 3-cyclohexyl-phényle et 4-cyclohexyl-phényle ;
un radical choisi dans le groupe constitué par quinolinyle, (1,4)-benzodioxanyle, (1,3)-benzodioxolyle, naphtyle et thiazolyle ;
un radical pyridinyle, le radical pouvant à chaque fois être substitué avec 1 ou 2 substituants choisis indépendamment l'un de l'autre dans le groupe constitué par F, Cl et Br ;
-(CHR¹⁷)-R²⁰ ou -(CHR¹⁷)-(CHR¹⁸)-R²⁰ ;
R³ représente un radical méthyle ou éthyle ;
R⁴ représente un radical hydrogène ;
R⁵ et R⁷ représentent chacun indépendamment l'un de l'autre un radical choisi dans le groupe constitué par méthyle, éthyle, n-propyle, isopropyle, n-butyle, sec-butyle, isobutyle, tert-butyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle, n-eicosanyle, vinyle, 1-propényle, 2-propényle, 1-butényle, 2-butényle, 3-butényle et 2-méthyl-1-propényle, le radical pouvant à chaque fois éventuellement être substitué avec 1, 2, 3, 4 ou 5 substituants choisis indépendamment les uns des autres dans le groupe constitué par F, Cl et Br ;
un radical choisi dans le groupe constitué par cyclopropyle, cyclobutyle, cyclopentyle, cyclohexyle, cycloheptyle, cyclooctyle, cyclononyle, cyclodécyle, cycloundécyle, cyclododécyle, cyclopentényle, cyclohexényle, cycloheptényle et adamantyle ; le radical pouvant à chaque fois éventuellement être substitué avec 1, 2 ou 3 substituants choisis indépendamment les uns des autres dans le groupe constitué par méthyle, éthyle, n-propyle, isopropyle, n-butyle, sec-butyle, isobutyle et tert-butyle ;
un radical choisi dans le groupe constitué par phényle, naphtyle, (1,4)-benzodioxanyle et pyridinyle, le radical pouvant à chaque fois éventuellement être substitué avec 1, 2, 3, 4 ou 5 substituants choisis indépendamment les uns des autres dans le groupe constitué par -SF₅, F, Cl, Br, I, -CN, -CF₃, -O-CH₃, -O-C₂H₅, -NO₂, -O-CF₃, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅,-C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -C(=O)-CH₃, -C(=O)-C₂H₅, -C(=O)-C(CH₃)₃, cyclohexyle, cyclopentyle, -0-phényle, -0-benzyle et phényle, la partie cyclique des radicaux cyclopentyle, cyclohexyle, -0-phényle, -0-benzyle et phényle pouvant à chaque fois être substituée avec 1, 2, 3, 4 ou 5 substituants choisis indépendamment les uns des autres dans le groupe constitué par F, Cl, Br, méthyle, éthyle, n-propyle, isopropyle, n-butyle, sec-butyle, isobutyle et tert-butyle ;
ou - (CR²¹R²²)-R²⁵, -(CR²¹R²²)- (CHR²³)-R²⁵, -(CR²¹R²²)-(CHR²³)-O-R²⁵, -(CR²¹R²²)-(CHR²³)-(CHR²⁴)-R²⁵, -(CR²¹R²²)-(CHR²³)-(CHR²⁴)-O-R²⁵, -(CR²¹R²²)-(CHR²³)-(CHR²⁴)-N(CH₃)-R²⁵ ou -(CR²¹R²²)-(CHR²³)-(CHR²⁴)-N(C₂H₅)-R²⁵ ;
R⁶ et R⁸ représentent chacun un radical hydrogène ;
ou un radical méthyle ou éthyle ;
R⁹ représente un radical choisi dans le groupe constitué par phényle, pyridinyle et naphtyle, le radical pouvant à chaque fois éventuellement être substitué avec 1, 2, 3, 4 ou 5 substituants choisis indépendamment les uns des autres dans le groupe constitué par F, Cl, Br, I, -CF₃, -O-CF₃, -S-CF₃, -OH,-O-CH₃, -O-C₂H₅, -NH-S(=O)₂-CH₃, morpholinyle, pipéridinyle, pyrrolidinyle, pipérazinyle, phényle, méthyle, éthyle, n-propyle, isopropyle, n-butyle, sec-butyle, isobutyle, tert-butyle, n-pentyle, n-hexyle, n-heptyle et n-octyle ;
-(CR²⁶R²⁷-R³⁰, -(CR²⁶R²⁷)-(CHR²⁸)-R³⁰, -(CR²⁶R²⁷)-(CHR²⁸)-(CHR²⁹)-R³⁰, -CR³¹=CR³²-R³³ ou -C≡C-R³⁴ ;
R¹⁰ représente un radical choisi dans le groupe constitué par phényle et naphtyle, le radical pouvant à chaque fois éventuellement être substitué avec 1, 2, 3, 4 ou 5 substituants choisis indépendamment les uns des autres dans le groupe constitué par F, Cl, Br, I, -CF₃, -OH, -O-CH₃, -O-C₂H₅, méthyle, éthyle, n-propyle, isopropyle, n-butyle, sec-butyle, isobutyle et tert-butyle ;
-(CR²⁶R²⁷)-R³⁰, -(CR²⁶R²⁷)-(CHR²⁸)-R³⁰, -(CR²⁶R²⁷)-(CHR²⁸)-(CHR²⁹)-R³⁰, -CR³¹=CR³²-R³³ ou -C≡C-R³⁴ ;
R¹⁷, R¹⁸, R¹⁹, R²³, R²⁴, R²⁰ et R²⁹ représentent chacun indépendamment les uns des autres
un radical hydrogène ;
un radical choisi dans le groupe constitué par méthyle, éthyle, n-propyle, isopropyle, n-butyle, sec-butyle, isobutyle et tert-butyle ;
ou un radical phényle ;
R²⁰ représente un radical phényle ;
R²¹ et R²² représentent chacun indépendamment l'un de l'autre
un radical hydrogène ;
un radical choisi dans le groupe constitué par méthyle, éthyle, n-propyle, isopropyle, n-butyle, sec-butyle, isobutyle et tert-butyle ;
ou un radical phényle ;
R²⁵ représente un radical choisi dans le groupe constitué par méthyle, éthyle, n-propyle, isopropyle, n-butyle, sec-butyle, isobutyle et tert-butyle ;
un radical choisi dans le groupe constitué par cyclopentyle, cyclohexyle, tétrahydrofuranyle, tétrahydrothiophényle, pyrrolidinyle, morpholinyle, pipéridinyle et pipérazinyle ;
ou un radical choisi dans le groupe constitué par phényle, naphtyle, (1,3)-benzodioxolyle, (1,4)-benzodioxanyle, thiophényle et furanyle, le radical pouvant à chaque fois éventuellement être substitué avec 1, 2, 3, 4 ou 5 substituants choisis indépendamment les uns des autres dans le groupe constitué par F, Cl, Br, -OH, -O-CH₃, -O-C₂H₅, méthyle, éthyle, n-propyle, isopropyle, n-butyle, sec-butyle, isobutyle et tert-butyle ;
R²⁶ et R²⁷ représentent chacun indépendamment l'un de l'autre
un radical hydrogène ;
un radical choisi dans le groupe constitué par méthyle, éthyle, n-propyle, isopropyle, n-butyle, sec-butyle, isobutyle et tert-butyle ;
un radical phényle ;
ou -OH ;
R³⁰ représente un radical phényle, qui peut être substitué avec 1, 2, 3, 4 ou 5 substituants choisis indépendamment les uns des autres dans le groupe constitué par F, Cl, Br, -NH-S(=O)₂-CH₃, méthyle, éthyle, n-propyle, isopropyle, n-butyle, sec-butyle, isobutyle et tert-butyle ;
R³¹ un radical hydrogène ;
ou un radical choisi dans le groupe constitué par méthyle, éthyle, n-propyle, isopropyle, n-butyle, sec-butyle, isobutyle et tert-butyle ;
R³² représente un radical hydrogène ;
un radical choisi dans le groupe constitué par méthyle, éthyle, n-propyle, isopropyle, n-butyle, sec-butyle, isobutyle et tert-butyle ;
un radical choisi dans le groupe constitué par phényle, naphtyle, furanyle et thiophényle, qui peut éventuellement être substitué avec 1, 2, 3, 4 ou 5 substituants choisis indépendamment les uns des autres dans le groupe constitué par -SF₅, F, Cl, Br, I, -CF₃, -O-CF₃, -S-CF₃, -O-CH₃, -O-C₂H₅, phényle, -S-CH₃,-S-C₂H₅, cyclopentyle, cyclohexyle, méthyle, éthyle, n-propyle, isopropyle, n-butyle, sec-butyle, isobutyle et tert-butyle ;
-CH₂-R³⁵ ou -CH=CH-R³⁶ ;
R³³ et R³⁴ représentent chacun indépendamment l'un de l'autre
un radical choisi dans le groupe constitué par méthyle, éthyle, n-propyle, isopropyle, n-butyle, sec-butyle, isobutyle et tert-butyle ;
ou un radical choisi dans le groupe constitué par phényle, pyridinyle et naphtyle, le radical pouvant à chaque fois éventuellement être substitué avec 1, 2, 3, 4 ou 5 substituants choisis indépendamment les uns des autres dans le groupe constitué par -SF₅, F, Cl, Br,-CF₃, -O-CF₃, -S-CF₃, phényle, morpholinyle, pipéridinyle, pyrrolidinyle, pipérazinyle, cyclopentyle, cyclohexyle, -OH, -O-CH₃, -O-C₂H₅, méthyle, éthyle, n-propyle, isopropyle, n-butyle, sec-butyle, isobutyle, tert-butyle, n-pentyle, n-hexyle, n-heptyle et n-octyle ;
et
R³⁵ et R³⁶ représentent chacun un radical phényle ;
à chaque fois éventuellement sous la forme d'un de leurs stéréoisomères purs, notamment des énantiomères ou des diastéréomères, de leurs racémats ou sous la forme d'un mélange de stéréoisomères, notamment des énantiomères ou/ou des diastéréomères, en un rapport de mélange quelconque, ou à chaque fois sous la forme de sels correspondants, ou à chaque fois sous la forme de solvates correspondants.

22. Composés selon une ou plusieurs des revendications 1 à 21, choisis dans le groupe constitué par
[1] phénylamide de l'acide 3-(3-méthoxy-phényl)-1-oxa-2,8-diaza-spiro[4.5]déc-2-én-8-oïque
[2] phénylamide de l'acide 3-phényl-1-oxa-2,8-diaza-spiro[4.5]déc-2-én-8-oïque
[3] phénylamide de l'acide 3-(4-chloro-phényl)-1-oxa-2,8-diaza-spiro[4.5]déc-2-én-8-oïque
[4] phénylamide de l'acide 3-(4-tert-butyl-phényl)-1-oxa-2,8-diaza-spiro[4.5]déc-2-én-8-oïque
[5] phénylamide de l'acide 3-p-tolyl-1-oxa-2,8-diaza-spiro[4.5]déc-2-én-8-oïque
[6] phénylamide de l'acide 3-(4-trifluorométhyl-phényl)-1-oxa-2,8-diaza-spiro[4.5]déc-2-én-8-oïque
[7] m-tolylamide de l'acide 3-(3-méthoxy-phényl)-1-oxa-2,8-diaza-spiro[4.5]déc-2-én-8-oïque
[8] m-tolylamide de l'acide 3-(3-méthoxy-phényl)-1-oxa-2,8-diaza-spiro[4.5]déc-2-én-8-oïque
[9] m-tolylamide de l'acide 3-phényl-1-oxa-2,8-diaza-spiro[4.5]déc-2-én-8-oïque
[10] m-tolylamide de l'acide 3-(4-chloro-phényl)-1-oxa-2,8-diaza-spiro[4.5]déc-2-én-8-oïque
[11] m-tolylamide de l'acide 3-(4-tert-butyl-phényl)-1-oxa-2,8-diaza-spiro[4.5]déc-2-én-8-oïque
[12] m-tolylamide de l'acide 3-p-tolyl-1-oxa-2,8-diaza-spiro[4.5]déc-2-én-8-oïque
[13] m-tolylamide de l'acide 3-(4-trifluorométhyl-phényl)-1-oxa-2,8-diaza-spiro[4.5]déc-2-én-8-oïque
[14] ((3-éthyl-phényl)-amide de l'acide 3-(3-méthoxy-phényl)-1-oxa-2,8-diaza-spiro[4.5]déc-2-én-8-oïque
[15] ((3-éthyl-phényl)-amide de l'acide 3-(3-méthoxy-phényl)-1-oxa-2,8-diaza-spiro[4.5]déc-2-én-8-oïque
[16] ((3-éthyl-phényl)-amide de l'acide 3-phényl-1-oxa-2,8-diaza-spiro[4.5]déc-2-én-8-oïque
[17] ((3-éthyl-phényl)-amide de l'acide 3-(4-chloro-phényl)-1-oxa-2,8-diaza-spiro[4.5]déc-2-én-8-oïque
[18] ((3-éthyl-phényl)-amide de l'acide 3-(4-tert-butyl-phényl)-1-oxa-2,8-diaza-spiro[4.5]déc-2-én-8-oïque
[19] ((3-éthyl-phényl)-amide de l'acide 3-p-tolyl-1-oxa-2,8-diaza-spiro[4.5]déc-2-én-8-oïque
[20] ((3-éthyl-phényl)-amide de l'acide 3-(4-trifluorométhyl-phényl)-1-oxa-2,8-diaza-spiro[4.5]déc-2-én-8-oïque
[21] ((2-propyl-phényl)-amide de l'acide 3-(3-méthoxy-phényl)-1-oxa-2,8-diaza-spiro[4.5]déc-2-én-8-oïque
[22] ((2-propyl-phényl)-amide de l'acide 3-phényl-1-oxa-2,8-diaza-spiro[4.5]déc-2-én-8-oïque
[23] ((2-propyl-phényl)-amide de l'acide 3-(4-chloro-phényl)-1-oxa-2,8-diaza-spiro[4.5]déc-2-én-8-oïque
[24] ((2-propyl-phényl)-amide de l'acide 3-(4-tert-butyl-phényl)-1-oxa-2,8-diaza-spiro[4.5]déc-2-én-8-oïque
[25] ((2-propyl-phényl)-amide de l'acide 3-p-tolyl-1-oxa-2,8-diaza-spiro[4.5]déc-2-én-8-oïque
[26] ((2-propyl-phényl)-amide de l'acide 3-(4-trifluorométhyl-phényl)-1-oxa-2,8-diaza-spiro[4.5]déc-2-én-8-oïque
[27] ((4-fluoro-phényl)-amide de l'acide 3-(3-méthoxy-phényl)-1-oxa-2,8-diaza-spiro[4.5]déc-2-én-8-oïque
[28] ((4-fluoro-phényl)-amide de l'acide 3-phényl-1-oxa-2,8-diaza-spiro[4.5]déc-2-én-8-oïque
[29] ((4-fluoro-phényl)-amide de l'acide 3-(4-chloro-phényl)-1-oxa-2,8-diaza-spiro[4.5]déc-2-én-8-oïque
[30] ((4-fluoro-phényl)-amide de l'acide 3-(4-tert-butyl-phényl)-1-oxa-2,8-diaza-spiro[4.5]déc-2-én-8-oïque
[31] ((4-fluoro-phényl)-amide de l'acide 3-p-tolyl-1-oxa-2,8-diaza-spiro[4.5]déc-2-én-8-oïque
[32] ((4-fluoro-phényl)-amide de l'acide 3-(4-trifluorométhyl-phényl)-1-oxa-2,8-diaza-spiro[4.5]déc-2-én-8-oïque
[33] ((3-chloro-phényl)-amide de l'acide 3-(3-méthoxy-phényl)-1-oxa-2,8-diaza-spiro[4.5]déc-2-én-8-oïque
[34] ((3-chloro-phényl)-amide de l'acide 3-phényl-1-oxa-2,8-diaza-spiro[4.5]déc-2-én-8-oïque
[35] ((3-chloro-phényl)-amide de l'acide 3-(4-chloro-phényl)-1-oxa-2,8-diaza-spiro[4.5]déc-2-én-8-oïque
[36] ((3-chloro-phényl)-amide de l'acide 3-(4-tert-butyl-phényl)-1-oxa-2,8-diaza-spiro[4.5]déc-2-én-8-oïque
[37] ((3-chloro-phényl)-amide de l'acide 3-p-tolyl-1-oxa-2,8-diaza-spiro[4.5]déc-2-én-8-oïque
[38] ((3-chloro-phényl)-amide de l'acide 3-(4-trifluorométhyl-phényl)-1-oxa-2,8-diaza-spiro[4.5]déc-2-én-8-oïque
[39] ((4-chloro-phényl)-amide de l'acide 3-(3-méthoxy-phényl)-1-oxa-2,8-diaza-spiro[4.5]déc-2-én-8-oïque
[40] ((4-chloro-phényl)-amide de l'acide 3-phényl-1-oxa-2,8-diaza-spiro[4.5]déc-2-én-8-oïque
[41] ((4-chloro-phényl)-amide de l'acide 3-(4-chloro-phényl)-1-oxa-2,8-diaza-spiro[4.5]déc-2-én-8-oïque
[42] ((4-chloro-phényl)-amide de l'acide 3-(4-tert-butyl-phényl)-1-oxa-2,8-diaza-spiro[4.5]déc-2-én-8-oïque
[43] ((4-chloro-phényl)-amide de l'acide 3-p-tolyl-1-oxa-2,8-diaza-spiro[4.5]déc-2-én-8-oïque
[44] ((4-iodo-phényl)-amide de l'acide 3-phényl-1-oxa-2,8-diaza-spiro[4.5]déc-2-én-8-oïque
[45] ((4-iodo-phényl)-amide de l'acide 3-(4-tert-butyl-phényl)-1-oxa-2,8-diaza-spiro[4.5]déc-2-én-8-oïque
[46] ((4-iodo-phényl)-amide de l'acide 3-(4-trifluorométhyl-phényl)-1-oxa-2,8-diaza-spiro[4.5]déc-2-én-8-oïque
[47] ((4-méthoxy-phényl)-amide de l'acide 3-(3-méthoxy-phényl)-1-oxa-2,8-diaza-spiro[4.5]déc-2-én-8-oïque
[48] ((4-méthoxy-phényl)-amide de l'acide 3-phényl-1-oxa-2,8-diaza-spiro[4.5]déc-2-én-8-oïque
[49] ((4-méthoxy-phényl)-amide de l'acide 3-(4-chloro-phényl)-1-oxa-2,8-diaza-spiro[4.5]déc-2-én-8-oïque
[50] ((4-méthoxy-phényl)-amide de l'acide 3-(4-tert-butyl-phényl)-1-oxa-2,8-diaza-spiro[4.5]déc-2-én-8-oïque
[51] ((4-méthoxy-phényl)-amide de l'acide 3-p-tolyl-1-oxa-2,8-diaza-spiro[4.5]déc-2-én-8-oïque
[52] ((4-méthoxy-phényl)-amide de l'acide 3-(4-trifluorométhyl-phényl)-1-oxa-2,8-diaza-spiro[4.5]déc-2-én-8-oïque
[53] ((2-méthylsulfanyl-phényl)-amide de l'acide 3-(3-méthoxy-phényl)-1-oxa-2,8-diaza-spiro[4.5]déc-2-én-8-oïque
[54] ((2-méthylsulfanyl-phényl)-amide de l'acide 3-phényl-1-oxa-2,8-diaza-spiro[4.5]déc-2-én-8-oïque
[55] ((2-méthylsulfanyl-phényl)-amide de l'acide 3-(4-chloro-phényl)-1-oxa-2,8-diaza-spiro[4.5]déc-2-én-8-oïque
[56] ((2-méthylsulfanyl-phényl)-amide de l'acide 3-(4-tert-butyl-phényl)-1-oxa-2,8-diaza-spiro[4.5]déc-2-én-8-oïque
[57] ((2-méthylsulfanyl-phényl)-amide de l'acide 3-p-tolyl-1-oxa-2,8-diaza-spiro[4.5]déc-2-én-8-oïque
[58] ((2-méthylsulfanyl-phényl)-amide de l'acide 3-(4-trifluorométhyl-phényl)-1-oxa-2,8-diaza-spiro[4.5]déc-2-én-8-oïque
[59] ((3-méthylsulfanyl-phényl)-amide de l'acide 3-(3-méthoxy-phényl)-1-oxa-2,8-diaza-spiro[4.5]déc-2-én-8-oïque
[60] ((3-méthylsulfanyl-phényl)-amide de l'acide 3-phényl-1-oxa-2,8-diaza-spiro[4.5]déc-2-én-8-oïque
[61] ((3-méthylsulfanyl-phényl)-amide de l'acide 3-(4-chloro-phényl)-1-oxa-2,8-diaza-spiro[4.5]déc-2-én-8-oïque
[62] ((3-méthylsulfanyl-phényl)-amide de l'acide 3-(4-tert-butyl-phényl)-1-oxa-2,8-diaza-spiro[4.5]déc-2-én-8-oïque
[63] ((3-méthylsulfanyl-phényl)-amide de l'acide 3-p-tolyl-1-oxa-2,8-diaza-spiro[4.5]déc-2-én-8-oïque
[64] ((3-méthylsulfanyl-phényl)-amide de l'acide 3-(4-trifluorométhyl-phényl)-1-oxa-2,8-diaza-spiro[4.5]déc-2-én-8-oïque
[65] ((4-méthylsulfanyl-phényl)-amide de l'acide 3-(3-méthoxy-phényl)-1-oxa-2,8-diaza-spiro[4.5]déc-2-én-8-oïque
[66] ((4-méthylsulfanyl-phényl)-amide de l'acide 3-phényl-1-oxa-2,8-diaza-spiro[4.5]déc-2-én-8-oïque
[67] ((4-méthylsulfanyl-phényl)-amide de l'acide 3-(4-chloro-phényl)-1-oxa-2,8-diaza-spiro[4.5]déc-2-én-8-oïque
[68] ((4-méthylsulfanyl-phényl)-amide de l'acide 3-(4-tert-butyl-phényl)-1-oxa-2,8-diaza-spiro[4.5]déc-2-én-8-oïque
[69] ((4-méthylsulfanyl-phényl)-amide de l'acide 3-p-tolyl-1-oxa-2,8-diaza-spiro[4.5]déc-2-én-8-oïque
[70] ((4-méthylsulfanyl-phényl)-amide de l'acide 3-(4-trifluorométhyl-phényl)-1-oxa-2,8-diaza-spiro[4.5]déc-2-én-8-oïque
[71] ((2-isopropyl-phényl)-amide de l'acide 3-(3-méthoxy-phényl)-1-oxa-2,8-diaza-spiro[4.5]déc-2-én-8-oïque
[72] ((2-isopropyl-phényl)-amide de l'acide 3-phényl-1-oxa-2,8-diaza-spiro[4.5]déc-2-én-8-oïque
[73] ((2-isopropyl-phényl)-amide de l'acide 3-(4-chloro-phényl)-1-oxa-2,8-diaza-spiro[4.5]déc-2-én-8-oïque
[74] ((2-isopropyl-phényl)-amide de l'acide 3-(4-tert-butyl-phényl)-1-oxa-2,8-diaza-spiro[4.5]déc-2-én-8-oïque
[75] ((2-isopropyl-phényl)-amide de l'acide 3-p-tolyl-1-oxa-2,8-diaza-spiro[4.5]déc-2-én-8-oïque
[76] ((2-isopropyl-phényl)-amide de l'acide 3-(4-trifluorométhyl-phényl)-1-oxa-2,8-diaza-spiro[4.5]déc-2-én-8-oïque
[77] ((4-isopropyl-phényl)-amide de l'acide 3-(3-méthoxy-phényl)-1-oxa-2,8-diaza-spiro[4.5]déc-2-én-8-oïque
[78] ((4-isopropyl-phényl)-amide de l'acide 3-phényl-1-oxa-2,8-diaza-spiro[4.5]déc-2-én-8-oïque
[79] ((4-isopropyl-phényl)-amide de l'acide 3-(4-chloro-phényl)-1-oxa-2,8-diaza-spiro[4.5]déc-2-én-8-oïque
[80] ((4-isopropyl-phényl)-amide de l'acide 3-(4-tert-butyl-phényl)-1-oxa-2,8-diaza-spiro[4.5]déc-2-én-8-oïque
[81] ((4-isopropyl-phényl)-amide de l'acide 3-p-tolyl-1-oxa-2,8-diaza-spiro[4.5]déc-2-én-8-oïque
[82] ((4-isopropyl-phényl)-amide de l'acide 3-(4-trifluorométhyl-phényl)-1-oxa-2,8-diaza-spiro[4.5]déc-2-én-8-oïque
[83] ((2-trifluorométhyl-phényl)-amide de l'acide 3-(3-méthoxy-phényl)-1-oxa-2,8-diaza-spiro[4.5]déc-2-én-8-oïque
[84] ((2-trifluorométhyl-phényl)-amide de l'acide 3-phényl-1-oxa-2,8-diaza-spiro[4.5]déc-2-én-8-oïque
[85] ((2-trifluorométhyl-phényl)-amide de l'acide 3-(4-chloro-phényl)-1-oxa-2,8-diaza-spiro[4.5]déc-2-én-8-oïque
[86] ((2-trifluorométhyl-phényl)-amide de l'acide 3-(4-tert-butyl-phényl)-1-oxa-2,8-diaza-spiro[4.5]déc-2-én-8-oïque
[87] ((2-trifluorométhyl-phényl)-amide de l'acide 3-p-tolyl-1-oxa-2,8-diaza-spiro[4.5]déc-2-én-8-oïque
[88] ((2-trifluorométhyl-phényl)-amide de l'acide 3-(4-trifluorométhyl-phényl)-1-oxa-2,8-diaza-spiro[4.5]déc-2-én-8-oïque
[89] ((3-trifluorométhyl-phényl)-amide de l'acide 3-(3-méthoxy-phényl)-1-oxa-2,8-diaza-spiro[4.5]déc-2-én-8-oïque
[90] ((3-trifluorométhyl-phényl)-amide de l'acide 3-phényl-1-oxa-2,8-diaza-spiro[4.5]déc-2-én-8-oïque
[91] ((3-trifluorométhyl-phényl)-amide de l'acide 3-(4-chloro-phényl)-1-oxa-2,8-diaza-spiro[4.5]déc-2-én-8-oïque
[92] ((3-trifluorométhyl-phényl)-amide de l'acide 3-(4-tert-butyl-phényl)-1-oxa-2,8-diaza-spiro[4.5]déc-2-én-8-oïque
[93] ((3-trifluorométhyl-phényl)-amide de l'acide 3-p-tolyl-1-oxa-2,8-diaza-spiro[4.5]déc-2-én-8-oïque
[94] ((3-trifluorométhyl-phényl)-amide de l'acide 3-(4-trifluorométhyl-phényl)-1-oxa-2,8-diaza-spiro[4.5]déc-2-én-8-oïque
[95] biphényl-4-ylamide de l'acide 3-phényl-1-oxa-2,8-diaza-spiro[4.5]déc-2-én-8-oïque
[96] biphényl-4-ylamide de l'acide 3-(4-tert-butyl-phényl)-1-oxa-2,8-diaza-spiro[4.5]déc-2-én-8-oïque
[97] biphényl-4-ylamide de l'acide 3-p-tolyl-1-oxa-2,8-diaza-spiro[4.5]déc-2-én-8-oïque
[98] ((2-phénoxy-phényl)-amide de l'acide 3-(3-méthoxy-phényl)-1-oxa-2,8-diaza-spiro[4.5]déc-2-én-8-oïque
[99] ((2-phénoxy-phényl)-amide de l'acide 3-phényl-1-oxa-2,8-diaza-spiro[4.5]déc-2-én-8-oïque
[100] ((2-phénoxy-phényl)-amide de l'acide 3-(4-chloro-phényl)-1-oxa-2,8-diaza-spiro[4.5]déc-2-én-8-oïque
[101] ((2-phénoxy-phényl)-amide de l'acide 3-(4-tert-butyl-phényl)-1-oxa-2,8-diaza-spiro[4.5]déc-2-én-8-oïque
[102] ((2-phénoxy-phényl)-amide de l'acide 3-p-tolyl-1-oxa-2,8-diaza-spiro[4.5]déc-2-én-8-oïque
[103] ((2-phénoxy-phényl)-amide de l'acide 3-(4-trifluorométhyl-phényl)-1-oxa-2,8-diaza-spiro[4.5]déc-2-én-8-oïque
[104] ((4-benzyloxy-phényl)-amide de l'acide 3-(3-méthoxy-phényl)-1-oxa-2,8-diaza-spiro[4.5]déc-2-én-8-oïque
[105] ((4-benzyloxy-phényl)-amide de l'acide 3-phényl-1-oxa-2,8-diaza-spiro[4.5]déc-2-én-8-oïque
[106] ((4-benzyloxy-phényl)-amide de l'acide 3-(4-chloro-phényl)-1-oxa-2,8-diaza-spiro[4.5]déc-2-én-8-oïque
[107] ((4-benzyloxy-phényl)-amide de l'acide 3-(4-tert-butyl-phényl)-1-oxa-2,8-diaza-spiro[4.5]déc-2-én-8-oïque
[108] ((4-benzyloxy-phényl)-amide de l'acide 3-p-tolyl-1-oxa-2,8-diaza-spiro[4.5]déc-2-én-8-oïque
[109] ((4-benzyloxy-phényl)-amide de l'acide 3-(4-trifluorométhyl-phényl)-1-oxa-2,8-diaza-spiro[4.5]déc-2-én-8-oïque
[110] cyclohexylamide de l'acide 3-(3-méthoxy-phényl)-1-oxa-2,8-diaza-spiro[4.5]déc-2-én-8-oïque
[111] cyclohexylamide de l'acide 3-phényl-1-oxa-2,8-diaza-spiro[4.5]déc-2-én-8-oïque
[112] cyclohexylamide de l'acide 3-(4-chloro-phényl)-1-oxa-2,8-diaza-spiro[4.5]déc-2-én-8-oïque
[113] cyclohexylamide de l'acide 3-(4-tert-butyl-phényl)-1-oxa-2,8-diaza-spiro[4.5]déc-2-én-8-oïque
[114] cyclohexylamide de l'acide 3-p-tolyl-1-oxa-2,8-diaza-spiro[4.5]déc-2-én-8-oïque
[115] cyclohexylamide de l'acide 3-(4-trifluorométhyl-phényl)-1-oxa-2,8-diaza-spiro[4.5]déc-2-én-8-oïque
[116] benzylamide de l'acide 3-(3-méthoxy-phényl)-1-oxa-2,8-diaza-spiro[4.5]déc-2-én-8-oïque
[117] benzylamide de l'acide 3-phényl-1-oxa-2,8-diaza-spiro[4.5]déc-2-én-8-oïque
[118] benzylamide de l'acide 3-(4-chloro-phényl)-1-oxa-2,8-diaza-spiro[4.5]déc-2-én-8-oïque
[119] benzylamide de l'acide 3-(4-tert-butyl-phényl)-1-oxa-2,8-diaza-spiro[4.5]déc-2-én-8-oïque
[120] benzylamide de l'acide 3-p-tolyl-1-oxa-2,8-diaza-spiro[4.5]déc-2-én-8-oïque
[121] benzylamide de l'acide 3-(4-trifluorométhyl-phényl)-1-oxa-2,8-diaza-spiro[4.5]déc-2-én-8-oïque
[122] phénéthyl-amide de l'acide 3-(3-méthoxy-phényl)-1-oxa-2,8-diaza-spiro[4.5]déc-2-én-8-oïque
[123] phénéthyl-amide de l'acide 3-phényl-1-oxa-2,8-diaza-spiro[4.5]déc-2-én-8-oïque
[124] phénéthyl-amide de l'acide 3-(4-chloro-phényl)-1-oxa-2,8-diaza-spiro[4.5]déc-2-én-8-oïque
[125] phénéthyl-amide de l'acide 3-(4-tert-butyl-phényl)-1-oxa-2,8-diaza-spiro[4.5]déc-2-én-8-oïque
[126] phénéthyl-amide de l'acide 3-p-tolyl-1-oxa-2,8-diaza-spiro[4.5]déc-2-én-8-oïque
[127] phénéthyl-amide de l'acide 3-(4-trifluorométhyl-phényl)-1-oxa-2,8-diaza-spiro[4.5]déc-2-én-8-oïque
[128] 4-méthyl-benzylamide de l'acide 3-(3-méthoxy-phényl)-1-oxa-2,8-diaza-spiro[4.5]déc-2-én-8-oïque
[129] 4-méthyl-benzylamide de l'acide 3-phényl-1-oxa-2,8-diaza-spiro[4.5]déc-2-én-8-oïque
[130] 4-méthyl-benzylamide de l'acide 3-(4-chloro-phényl)-1-oxa-2,8-diaza-spiro[4.5]déc-2-én-8-oïque
[131] 4-méthyl-benzylamide de l'acide 3-(4-tert-butyl-phényl)-1-oxa-2,8-diaza-spiro[4.5]déc-2-én-8-oïque
[132] 4-méthyl-benzylamide de l'acide 3-p-tolyl-1-oxa-2,8-diaza-spiro[4.5]déc-2-én-8-oïque
[133] 4-méthyl-benzylamide de l'acide 3-(4-trifluorométhyl-phényl)-1-oxa-2,8-diaza-spiro[4.5]déc-2-én-8-oïque-
[134] 4-méthoxy-benzylamide de l'acide 3-(3-méthoxy-phényl)-1-oxa-2,8-diaza-spiro[4.5]déc-2-én-8-oïque
[135] 4-méthoxy-benzylamide de l'acide 3-phényl-1-oxa-2,8-diaza-spiro[4.5]déc-2-én-8-oïque
[136] 4-méthoxy-benzylamide de l'acide 3-(4-chloro-phényl)-1-oxa-2,8-diaza-spiro[4.5]déc-2-én-8-oïque
[137] 4-méthoxy-benzylamide de l'acide 3-(4-tert-butyl-phényl)-1-oxa-2,8-diaza-spiro[4.5]déc-2-én-8-oïque
[138] 4-méthoxy-benzylamide de l'acide 3-p-tolyl-1-oxa-2,8-diaza-spiro[4.5]déc-2-én-8-oïque
[139] 4-méthoxy-benzylamide de l'acide 3-(4-trifluorométhyl-phényl)-1-oxa-2,8-diaza-spiro[4.5]déc-2-én-8-oïque
[140] ((4-tert-butyl-phényl)-amide de l'acide 3-(3-méthoxy-phényl)-1-oxa-2,8-diaza-spiro[4.5]déc-2-én-8-oïque
[141] ((4-tert-butyl-phényl)-amide de l'acide 3-phényl-1-oxa-2,8-diaza-spiro[4.5]déc-2-én-8-oïque
[142] ((4-tert-butyl-phényl)-amide de l'acide 3-(4-chloro-phényl)-1-oxa-2,8-diaza-spiro[4.5]déc-2-én-8-oïque
[143] ((4-tert-butyl-phényl)-amide de l'acide 3-(4-tert-butyl-phényl)-1-oxa-2,8-diaza-spiro[4.5]déc-2-én-8-oïque
[144] ((4-tert-butyl-phényl)-amide de l'acide 3-p-tolyl-1-oxa-2,8-diaza-spiro[4.5]déc-2-én-8-oïque
[145] ((4-tert-butyl-phényl)-amide de l'acide 3-(4-trifluorométhyl-phényl)-1-oxa-2,8-diaza-spiro[4.5]déc-2-én-8-oïque
[146] p-tolylamide de l'acide 3-(3-méthoxy-phényl)-1-oxa-2,8-diaza-spiro[4.5]déc-2-én-8-oïque
[147] p-tolylamide de l'acide 3-phényl-1-oxa-2,8-diaza-spiro[4.5]déc-2-én-8-oïque
[148] p-tolylamide de l'acide 3-(4-chloro-phényl)-1-oxa-2,8-diaza-spiro[4.5]déc-2-én-8-oïque
[149] p-tolylamide de l'acide 3-(4-tert-butyl-phényl)-1-oxa-2,8-diaza-spiro[4.5]déc-2-én-8-oïque
[150] o-tolylamide de l'acide 3-(3-méthoxy-phényl)-1-oxa-2,8-diaza-spiro[4.5]déc-2-én-8-oïque
[151] o-tolylamide de l'acide 3-phényl-1-oxa-2,8-diaza-spiro[4.5]déc-2-én-8-oïque
[152] o-tolylamide de l'acide 3-(4-chloro-phényl)-1-oxa-2,8-diaza-spiro[4.5]déc-2-én-8-oïque
[153] o-tolylamide de l'acide 3-(4-tert-butyl-phényl)-1-oxa-2,8-diaza-spiro[4.5]déc-2-én-8-oïque
[154] o-tolylamide de l'acide 3-p-tolyl-1-oxa-2,8-diaza-spiro[4.5]déc-2-én-8-oïque
[155] o-tolylamide de l'acide 3-(4-trifluorométhyl-phényl)-1-oxa-2,8-diaza-spiro[4.5]déc-2-én-8-oïque
[156] ((2-éthyl-phényl)-amide de l'acide 3-(3-méthoxy-phényl)-1-oxa-2,8-diaza-spiro[4.5]déc-2-én-8-oïque
[157] ((2-éthyl-phényl)-amide de l'acide 3-phényl-1-oxa-2,8-diaza-spiro[4.5]déc-2-én-8-oïque
[158] ((2-éthyl-phényl)-amide de l'acide 3-(4-chloro-phényl)-1-oxa-2,8-diaza-spiro[4.5]déc-2-én-8-oïque
[159] ((2-éthyl-phényl)-amide de l'acide 3-(4-tert-butyl-phényl)-1-oxa-2,8-diaza-spiro[4.5]déc-2-én-8-oïque
[160] ((2-éthyl-phényl)-amide de l'acide 3-p-tolyl-1-oxa-2,8-diaza-spiro[4.5]déc-2-én-8-oïque
[161] ((2-éthyl-phényl)-amide de l'acide 3-(4-trifluorométhyl-phényl)-1-oxa-2,8-diaza-spiro[4.5]déc-2-én-8-oïque
[162] ((4-éthyl-phényl)-amide de l'acide 3-(3-méthoxy-phényl)-1-oxa-2,8-diaza-spiro[4.5]déc-2-én-8-oïque
[163] ((4-éthyl-phényl)-amide de l'acide 3-phényl-1-oxa-2,8-diaza-spiro[4.5]déc-2-én-8-oïque
[164] ((4-éthyl-phényl)-amide de l'acide 3-(4-chloro-phényl)-1-oxa-2,8-diaza-spiro[4.5]déc-2-én-8-oïque
[165] ((4-éthyl-phényl)-amide de l'acide 3-(4-tert-butyl-phényl)-1-oxa-2,8-diaza-spiro[4.5]déc-2-én-8-oïque
[166] ((4-éthyl-phényl)-amide de l'acide 3-p-tolyl-1-oxa-2,8-diaza-spiro[4.5]déc-2-én-8-oïque
[167] ((4-éthyl-phényl)-amide de l'acide 3-(4-trifluorométhyl-phényl)-1-oxa-2,8-diaza-spiro[4.5]déc-2-én-8-oïque
[168] ((2-fluoro-phényl)-amide de l'acide 3-(3-méthoxy-phényl)-1-oxa-2,8-diaza-spiro[4.5]déc-2-én-8-oïque
[169] ((2-fluoro-phényl)-amide de l'acide 3-phényl-1-oxa-2,8-diaza-spiro[4.5]déc-2-én-8-oïque
[170] ((2-fluoro-phényl)-amide de l'acide 3-(4-chloro-phényl)-1-oxa-2,8-diaza-spiro[4.5]déc-2-én-8-oïque
[171] ((2-fluoro-phényl)-amide de l'acide 3-(4-tert-butyl-phényl)-1-oxa-2,8-diaza-spiro[4.5]déc-2-én-8-oïque
[172] ((2-fluoro-phényl)-amide de l'acide 3-p-tolyl-1-oxa-2,8-diaza-spiro[4.5]déc-2-én-8-oïque
[173] ((2-fluoro-phényl)-amide de l'acide 3-(4-trifluorométhyl-phényl)-1-oxa-2,8-diaza-spiro[4.5]déc-2-én-8-oïque
[174] ((3-fluoro-phényl)-amide de l'acide 3-(3-méthoxy-phényl)-1-oxa-2,8-diaza-spiro[4.5]déc-2-én-8-oïque
[175] ((3-fluoro-phényl)-amide de l'acide 3-phényl-1-oxa-2,8-diaza-spiro[4.5]déc-2-én-8-oïque
[176] ((3-fluoro-phényl)-amide de l'acide 3-(4-chloro-phényl)-1-oxa-2,8-diaza-spiro[4.5]déc-2-én-8-oïque
[177] ((3-fluoro-phényl)-amide de l'acide 3-(4-tert-butyl-phényl)-1-oxa-2,8-diaza-spiro[4.5]déc-2-én-8-oïque
[178] ((3-fluoro-phényl)-amide de l'acide 3-p-tolyl-1-oxa-2,8-diaza-spiro[4.5]déc-2-én-8-oïque
[179] ((3-fluoro-phényl)-amide de l'acide 3-(4-trifluorométhyl-phényl)-1-oxa-2,8-diaza-spiro[4.5]déc-2-én-8-oïque
[180] ((2-chloro-phényl)-amide de l'acide 3-(3-méthoxy-phényl)-1-oxa-2,8-diaza-spiro[4.5]déc-2-én-8-oïque
[181] ((2-chloro-phényl)-amide de l'acide 3-phényl-1-oxa-2,8-diaza-spiro[4.5]déc-2-én-8-oïque
[182] ((2-chloro-phényl)-amide de l'acide 3-(4-chloro-phényl)-1-oxa-2,8-diaza-spiro[4.5]déc-2-én-8-oïque
[183] ((2-chloro-phényl)-amide de l'acide 3-(4-tert-butyl-phényl)-1-oxa-2,8-diaza-spiro[4.5]déc-2-én-8-oïque
[184] ((2-chloro-phényl)-amide de l'acide 3-p-tolyl-1-oxa-2,8-diaza-spiro[4.5]déc-2-én-8-oïque
[185] ((2-chloro-phényl)-amide de l'acide 3-(4-trifluorométhyl-phényl)-1-oxa-2,8-diaza-spiro[4.5]déc-2-én-8-oïque
[186] ((2-bromo-phényl)-amide de l'acide 3-(3-méthoxy-phényl)-1-oxa-2,8-diaza-spiro[4.5]déc-2-én-8-oïque
[187] ((2-bromo-phényl)-amide de l'acide 3-phényl-1-oxa-2,8-diaza-spiro[4.5]déc-2-én-8-oïque
[188] ((2-bromo-phényl)-amide de l'acide 3-(4-chloro-phényl)-1-oxa-2,8-diaza-spiro[4.5]déc-2-én-8-oïque
[189] ((2-bromo-phényl)-amide de l'acide 3-(4-tert-butyl-phényl)-1-oxa-2,8-diaza-spiro[4.5]déc-2-én-8-oïque
[190] ((2-bromo-phényl)-amide de l'acide 3-p-tolyl-1-oxa-2,8-diaza-spiro[4.5]déc-2-én-8-oïque
[191] ((2-bromo-phényl)-amide de l'acide 3-(4-trifluorométhyl-phényl)-1-oxa-2,8-diaza-spiro[4.5]déc-2-én-8-oïque
[192] ((3-bromo-phényl)-amide de l'acide 3-(3-méthoxy-phényl)-1-oxa-2,8-diaza-spiro[4.5]déc-2-én-8-oïque
[193] ((3-bromo-phényl)-amide de l'acide 3-phényl-1-oxa-2,8-diaza-spiro[4.5]déc-2-én-8-oïque
[194] ((3-bromo-phényl)-amide de l'acide 3-(4-tert-butyl-phényl)-1-oxa-2,8-diaza-spiro[4.5]déc-2-én-8-oïque
[195] ((3-bromo-phényl)-amide de l'acide 3-(4-trifluorométhyl-phényl)-1-oxa-2,8-diaza-spiro[4.5]déc-2-én-8-oïque
[196] ((4-bromo-phényl)-amide de l'acide 3-(3-méthoxy-phényl)-1-oxa-2,8-diaza-spiro[4.5]déc-2-én-8-oïque
[197] ((4-bromo-phényl)-amide de l'acide 3-phényl-1-oxa-2,8-diaza-spiro[4.5]déc-2-én-8-oïque
[198] ((2-méthoxy-phényl)-amide de l'acide 3-(3-méthoxy-phényl)-1-oxa-2,8-diaza-spiro[4.5]déc-2-én-8-oïque
[199] ((2-méthoxy-phényl)-amide de l'acide 3-phényl-1-oxa-2,8-diaza-spiro[4.5]déc-2-én-8-oïque
[200] ((2-méthoxy-phényl)-amide de l'acide 3-(4-chloro-phényl)-1-oxa-2,8-diaza-spiro[4.5]déc-2-én-8-oïque
[201] ((2-méthoxy-phényl)-amide de l'acide 3-(4-tert-butyl-phényl)-1-oxa-2,8-diaza-spiro[4.5]déc-2-én-8-oïque
[202] ((2-méthoxy-phényl)-amide de l'acide 3-p-tolyl-1-oxa-2,8-diaza-spiro[4.5]déc-2-én-8-oïque
[203] ((2-méthoxy-phényl)-amide de l'acide 3-(4-trifluorométhyl-phényl)-1-oxa-2,8-diaza-spiro[4.5]déc-2-én-8-oïque
[204] ((3-méthoxy-phényl)-amide de l'acide 3-(3-méthoxy-phényl)-1-oxa-2,8-diaza-spiro[4.5]déc-2-én-8-oïque
[205] ((3-méthoxy-phényl)-amide de l'acide 3-phényl-1-oxa-2,8-diaza-spiro[4.5]déc-2-én-8-oïque
[206] ((3-méthoxy-phényl)-amide de l'acide 3-(4-chloro-phényl)-1-oxa-2,8-diaza-spiro[4.5]déc-2-én-8-oïque
[207] ((3-méthoxy-phényl)-amide de l'acide 3-(4-tert-butyl-phényl)-1-oxa-2,8-diaza-spiro[4.5]déc-2-én-8-oïque
[208] ((3-méthoxy-phényl)-amide de l'acide 3-p-tolyl-1-oxa-2,8-diaza-spiro[4.5]déc-2-én-8-oïque
[209] ((3-méthoxy-phényl)-amide de l'acide 3-(4-trifluorométhyl-phényl)-1-oxa-2,8-diaza-spiro[4.5]déc-2-én-8-oïque
[210] ((4-trifluorométhyl-phényl)-amide de l'acide 3-(3-méthoxy-phényl)-1-oxa-2,8-diaza-spiro[4.5]déc-2-én-8-oïque
[211] ((4-trifluorométhyl-phényl)-amide de l'acide 3-phényl-1-oxa-2,8-diaza-spiro[4.5]déc-2-én-8-oïque
[212] ((4-trifluorométhyl-phényl)-amide de l'acide 3-(4-chloro-phényl)-1-oxa-2,8-diaza-spiro[4.5]déc-2-én-8-oïque
[213] ((4-trifluorométhyl-phényl)-amide de l'acide 3-(4-tert-butyl-phényl)-1-oxa-2,8-diaza-spiro[4.5]déc-2-én-8-oïque
[214] ((4-trifluorométhyl-phényl)-amide de l'acide 3-p-tolyl-1-oxa-2,8-diaza-spiro[4.5]déc-2-én-8-oïque
[215] ((4-trifluorométhyl-phényl)-amide de l'acide 3-(4-trifluorométhyl-phényl)-1-oxa-2,8-diaza-spiro[4.5]déc-2-én-8-oique
[216] ((4-phénoxy-phényl)-amide de l'acide 3-(3-méthoxy-phényl)-1-oxa-2,8-diaza-spiro[4.5]déc-2-én-8-oïque
[217] ((4-phénoxy-phényl)-amide de l'acide 3-(4-chloro-phényl)-1-oxa-2,8-diaza-spiro[4.5]déc-2-én-8-oïque
[218] ((4-phénoxy-phényl)-amide de l'acide 3-(4-tert-butyl-phényl)-1-oxa-2,8-diaza-spiro[4.5]déc-2-én-8-oïque
[219] ((4-phénoxy-phényl)-amide de l'acide 3-p-tolyl-1-oxa-2,8-diaza-spiro[4.5]déc-2-én-8-oïque
[220] ((4-phénoxy-phényl)-amide de l'acide 3-(4-trifluorométhyl-phényl)-1-oxa-2,8-diaza-spiro[4.5]déc-2-én-8-oïque
[221] ((2-chloro-5-trifluorométhyl-phényl)-amide de l'acide 3-(3-méthoxy-phényl)-1-oxa-2,8-diaza-spiro[4.5]déc-2-én-8-oïque
[222] ((2-chloro-5-trifluorométhyl-phényl)-amide de l'acide 3-phényl-1-oxa-2,8-diaza-spiro[4.5]déc-2-én-8-oïque
[223] ((2-chloro-5-trifluorométhyl-phényl)-amide de l'acide 3-(4-chloro-phényl)-1-oxa-2,8-diaza-spiro[4.5]déc-2-én-8-oique
[224] ((2-chloro-5-trifluorométhyl-phényl)-amide de l'acide 3-(4-tert-butyl-phényl)-1-oxa-2,8-diaza-spiro[4.5]déc-2-én-8-oïque
[225] ((2-chloro-5-trifluorométhyl-phényl)-amide de l'acide 3-p-tolyl-1-oxa-2,8-diaza-spiro[4.5]déc-2-én-8-oïque
[226] ((2-chloro-5-trifluorométhyl-phényl)-amide de l'acide 3-(4-trifluorométhyl-phényl)-1-oxa-2,8-diaza-spiro[4.5]déc-2-én-8-oïque
[227] ((4-chloro-2-trifluorométhyl-phényl)-amide de l'acide 3-(3-méthoxy-phényl)-1-oxa-2,8-diaza-spiro[4.5]déc-2-én-8-oïque
[228] ((4-chloro-2-trifluorométhyl-phényl)-amide de l'acide 3-phényl-1-oxa-2,8-diaza-spiro[4.5]déc-2-én-8-oïque
[229] ((4-chloro-2-trifluorométhyl-phényl)-amide de l'acide 3-(4-chloro-phényl)-1-oxa-2,8-diaza-spiro[4.5]déc-2-én-8-oïque
[230] ((4-chloro-2-trifluorométhyl-phényl)-amide de l'acide 3-(4-tert-butyl-phényl)-1-oxa-2,8-diaza-spiro[4.5]déc-2-én-8-oique
[231] ((4-chloro-2-trifluorométhyl-phényl)-amide de l'acide 3-p-tolyl-1-oxa-2,8-diaza-spiro[4.5]déc-2-én-8-oïque
[232] ((4-chloro-3-trifluorométhyl-phényl)-amide de l'acide 3-(3-méthoxy-phényl)-1-oxa-2,8-diaza-spiro[4.5]déc-2-én-8-oïque
[233] ((4-chloro-3-trifluorométhyl-phényl)-amide de l'acide 3-phényl-1-oxa-2,8-diaza-spiro[4.5]déc-2-én-8-oïque
[234] ((4-chloro-3-trifluorométhyl-phényl)-amide de l'acide 3-(4-chloro-phényl)-1-oxa-2,8-diaza-spiro[4.5]déc-2-én-8-oïque
[235] ((4-chloro-3-trifluorométhyl-phényl)-amide de l'acide 3-(4-tert-butyl-phényl)-1-oxa-2,8-diaza-spiro[4.5]déc-2-én-8-oique
[236] ((4-chloro-3-trifluorométhyl-phényl)-amide de l'acide 3-p-tolyl-1-oxa-2,8-diaza-spiro[4.5]déc-2-én-8-oïque
[237] ((4-chloro-3-trifluorométhyl-phényl)-amide de l'acide 3-(4-trifluorométhyl-phényl)-1-oxa-2,8-diaza-spiro[4.5]déc-2-én-8-oique
[238] ((2-tert-butyl-6-méthyl-phényl)-amide de l'acide 3-(3-méthoxy-phényl)-1-oxa-2,8-diaza-spiro[4.5]déc-2-én-8-oïque
[239] ((2-tert-butyl-6-méthyl-phényl)-amide de l'acide 3-phényl-1-oxa-2,8-diaza-spiro[4.5]déc-2-én-8-oïque
[240] ((2-tert-butyl-6-méthyl-phényl)-amide de l'acide 3-(4-chloro-phényl)-1-oxa-2,8-diaza-spiro[4.5]déc-2-én-8-oïque
[241] ((2-tert-butyl-6-méthyl-phényl)-amide de l'acide 3-(4-tert-butyl-phényl)-1-oxa-2,8-diaza-spiro[4.5]déc-2-én-8-oïque
[242] ((2-tert-butyl-6-méthyl-phényl)-amide de l'acide 3-p-tolyl-1-oxa-2,8-diaza-spiro[4.5]déc-2-én-8-oïque
[243] ((2-tert-butyl-6-méthyl-phényl)-amide de l'acide 3-(4-trifluorométhyl-phényl)-1-oxa-2,8-diaza-spiro[4.5]déc-2-én-8-oïque
[244] ((4-trifluorométhoxy-phényl)-amide de l'acide 3-(3-méthoxy-phényl)-1-oxa-2,8-diaza-spiro[4.5]déc-2-én-8-oïque
[245] ((4-trifluorométhoxy-phényl)-amide de l'acide 3-phényl-1-oxa-2,8-diaza-spiro[4.5]déc-2-én-8-oïque
[246] ((4-trifluorométhoxy-phényl)-amide de l'acide 3-(4-chloro-phényl)-1-oxa-2,8-diaza-spiro[4.5]déc-2-én-8-oïque
[247] ((4-trifluorométhoxy-phényl)-amide de l'acide 3-(4-tert-butyl-phényl)-1-oxa-2,8-diaza-spiro[4.5]déc-2-én-8-oïque
[248] ((4-trifluorométhoxy-phényl)-amide de l'acide 3-p-tolyl-1-oxa-2,8-diaza-spiro[4.5]déc-2-én-8-oïque
[249] cyclohexylamide de l'acide 3-(3-méthoxy-phényl)-1-oxa-2,8-diaza-spiro[4.5]déc-2-ène-8-thiocarboxylique
[250] cyclohexylamide de l'acide 3-phényl-1-oxa-2,8-diaza-spiro[4.5]déc-2-ène-8-thiocarboxylique
[251] cyclohexylamide de l'acide 3-(4-chloro-phényl)-1-oxa-2,8-diaza-spiro[4.5]déc-2-ène-8-thiocarboxylique
[252] cyclohexylamide de l'acide 3-(4-tert-butyl-phényl)-1-oxa-2,8-diaza-spiro[4.5]déc-2-ène-8-thiocarboxylique
[253] cyclohexylamide de l'acide 3-p-tolyl-1-oxa-2,8-diaza-spiro[4.5]déc-2-ène-8-thiocarboxylique
[254] cyclohexylamide de l'acide 3-(4-trifluorométhyl-phényl)-1-oxa-2,8-diaza-spiro[4.5]déc-2-ène-8-thiocarboxylique
[255] phénylamide de l'acide 3-(3-méthoxy-phényl)-1-oxa-2,8-diaza-spiro[4.5]déc-2-ène-8-thiocarboxylique
[256] phénylamide de l'acide 3-phényl-1-oxa-2,8-diaza-spiro[4.5]déc-2-ène-8-thiocarboxylique
[257] phénylamide de l'acide 3-(4-chloro-phényl)-1-oxa-2,8-diaza-spiro[4.5]déc-2-ène-8-thiocarboxylique
[258] phénylamide de l'acide 3-(4-tert-butyl-phényl)-1-oxa-2,8-diaza-spiro[4.5]déc-2-ène-8-thiocarboxylique
[259] phénylamide de l'acide 3-p-tolyl-1-oxa-2,8-diaza-spiro[4.5]déc-2-ène-8-thiocarboxylique
[260] phénylamide de l'acide 3-(4-trifluorométhyl-phényl)-1-oxa-2,8-diaza-spiro[4.5]déc-2-ène-8-thiocarboxylique
[261] ((3-trifluorométhyl-phényl)-amide de l'acide 3-(3-méthoxy-phényl)-1-oxa-2,8-diaza-spiro[4.5]déc-2-ène-8-thiocarboxylique
[262] ((3-trifluorométhyl-phényl)-amide de l'acide 3-phényl-1-oxa-2,8-diaza-spiro[4.5]déc-2-ène-8-thiocarboxylique
[263] ((3-trifluorométhyl-phényl)-amide de l'acide 3-(4-chloro-phényl)-1-oxa-2,8-diaza-spiro[4.5]déc-2-ène-8-thiocarboxylique
[264] ((3-trifluorométhyl-phényl)-amide de l'acide 3-(4-tert-butyl-phényl)-1-oxa-2,8-diaza-spiro[4.5]déc-2-ène-8-thiocarboxylique
[265] ((3-trifluorométhyl-phényl)-amide de l'acide 3-p-tolyl-1-oxa-2,8-diaza-spiro[4.5]déc-2-ène-8-thiocarboxylique
[266] ((3-trifluorométhyl-phényl)-amide de l'acide 3-(4-trifluorométhyl-phényl)-1-oxa-2,8-diaza-spiro[4.5]déc-2-ène-8-thiocarboxylique
[267] ((2-méthoxy-phényl)-amide de l'acide 3-(3-méthoxy-phényl)-1-oxa-2,8-diaza-spiro[4.5]déc-2-ène-8-thiocarboxylique
[268] ((2-méthoxy-phényl)-amide de l'acide 3-phényl-1-oxa-2,8-diaza-spiro[4.5]déc-2-ène-8-thiocarboxylique
[269] ((2-méthoxy-phényl)-amide de l'acide 3-(4-chloro-phényl)-1-oxa-2,8-diaza-spiro[4.5]déc-2-ène-8-thiocarboxylique
[270] ((2-méthoxy-phényl)-amide de l'acide 3-(4-tert-butyl-phényl)-1-oxa-2,8-diaza-spiro[4.5]déc-2-ène-8-thiocarboxylique
[271] ((2-méthoxy-phényl)-amide de l'acide 3-p-tolyl-1-oxa-2,8-diaza-spiro[4.5]déc-2-ène-8-thiocarboxylique
[272] ((2-méthoxy-phényl)-amide de l'acide 3-(4-trifluorométhyl-phényl)-1-oxa-2,8-diaza-spiro[4.5]déc-2-ène-8-thiocarboxylique
[273] ((3-méthoxy-phényl)-amide de l'acide 3-(3-méthoxy-phényl)-1-oxa-2,8-diaza-spiro[4.5]déc-2-ène-8-thiocarboxylique
[274] ((3-méthoxy-phényl)-amide de l'acide 3-phényl-1-oxa-2,8-diaza-spiro[4.5]déc-2-ène-8-thiocarboxylique
[275] ((3-méthoxy-phényl)-amide de l'acide 3-(4-chloro-phényl)-1-oxa-2,8-diaza-spiro[4.5]déc-2-ène-8-thiocarboxylique
[276] ((3-méthoxy-phényl)-amide de l'acide 3-p-tolyl-1-oxa-2,8-diaza-spiro[4.5]déc-2-ène-8-thiocarboxylique
[277] ((3-méthoxy-phényl)-amide de l'acide 3-(4-trifluorométhyl-phényl)-1-oxa-2,8-diaza-spiro[4.5]déc-2-ène-8-thiocarboxylique
[278] ((4-tert-butyl-phényl)-amide de l'acide 3-(3-méthoxy-phényl)-1-oxa-2,8-diaza-spiro[4.5]déc-2-ène-8-thiocarboxylique
[279] ((4-tert-butyl-phényl)-amide de l'acide 3-phényl-1-oxa-2,8-diaza-spiro[4.5]déc-2-ène-8-thiocarboxylique
[280] ((4-tert-butyl-phényl)-amide de l'acide 3-(4-chloro-phényl)-1-oxa-2,8-diaza-spiro[4.5]déc-2-ène-8-thiocarboxylique
[281] ((4-tert-butyl-phényl)-amide de l'acide 3-(4-tert-butyl-phényl)-1-oxa-2,8-diaza-spiro[4.5]déc-2-ène-8-thiocarboxylique
[282] ((4-tert-butyl-phényl)-amide de l'acide 3-p-tolyl-1-oxa-2,8-diaza-spiro[4.5]déc-2-ène-8-thiocarboxylique
[283] ((4-tert-butyl-phényl)-amide de l'acide 3-(4-trifluorométhyl-phényl)-1-oxa-2,8-diaza-spiro[4.5]déc-2-ène-8-thiocarboxylique
[284] 2-méthyl-benzylamide de l'acide 3-(3-méthoxy-phényl)-1-oxa-2,8-diaza-spiro[4.5]déc-2-én-8-oïque
[285] 2-méthyl-benzylamide de l'acide 3-phényl-1-oxa-2,8-diaza-spiro[4.5]déc-2-én-8-oïque
[286] 2-méthyl-benzylamide de l'acide 3-(4-chloro-phényl)-1-oxa-2,8-diaza-spiro[4.5]déc-2-én-8-oïque
[287] 2-méthyl-benzylamide de l'acide 3-(4-tert-butyl-phényl)-1-oxa-2,8-diaza-spiro[4.5]déc-2-én-8-oïque
[288] cyclopentylamide de l'acide 3-(4-chloro-phényl)-1-oxa-2,8-diaza-spiro[4.5]déc-2-ène-8-thiocarboxylique
[289] cyclopentylamide de l'acide 3-(4-tert-butyl-phényl)-1-oxa-2,8-diaza-spiro[4.5]déc-2-ène-8-thiocarboxylique
[290] cyclopentylamide de l'acide 3-p-tolyl-1-oxa-2,8-diaza-spiro[4.5]déc-2-ène-8-thiocarboxylique
[291] cyclopentylamide de l'acide 3-(4-trifluorométhyl-phényl)-1-oxa-2,8-diaza-spiro[4.5]déc-2-ène-8-thiocarboxylique
[292] cyclohexylméthyl-amide de l'acide 3-phényl-1-oxa-2,8-diaza-spiro[4.5]déc-2-ène-8-thiocarboxylique
[293] cyclohexylméthyl-amide de l'acide 3-(4-chloro-phényl)-1-oxa-2,8-diaza-spiro[4.5]déc-2-ène-8-thiocarboxylique
[294] cyclohexylméthyl-amide de l'acide 3-(4-tert-butyl-phényl)-1-oxa-2,8-diaza-spiro[4.5]déc-2-ène-8-thiocarboxylique
[295] cyclohexylméthyl-amide de l'acide 3-p-tolyl-1-oxa-2,8-diaza-spiro[4.5]déc-2-ène-8-thiocarboxylique
[296] cyclohexylméthyl-amide de l'acide 3-(4-trifluorométhyl-phényl)-1-oxa-2,8-diaza-spiro[4.5]déc-2-ène-8-thiocarboxylique
[297] cyclooctylamide de l'acide 3-(3-méthoxy-phényl)-1-oxa-2,8-diaza-spiro[4.5]déc-2-ène-8-thiocarboxylique
[298] cyclooctylamide de l'acide 3-phényl-1-oxa-2,8-diaza-spiro[4.5]déc-2-ène-8-thiocarboxylique
[299] cyclooctylamide de l'acide 3-(4-chloro-phényl)-1-oxa-2,8-diaza-spiro[4.5]déc-2-ène-8-thiocarboxylique
[300] cyclooctylamide de l'acide 3-p-tolyl-1-oxa-2,8-diaza-spiro[4.5]déc-2-ène-8-thiocarboxylique
[301] cyclooctylamide de l'acide 3-(4-trifluorométhyl-phényl)-1-oxa-2,8-diaza-spiro[4.5]déc-2-ène-8-thiocarboxylique
[302] ((2-morpholin-4-yl-éthyl)-amide de l'acide 3-(3-méthoxy-phényl)-1-oxa-2,8-diaza-spiro[4.5]déc-2-ène-8-thiocarboxylique
[303] ((4-tert-butyl-phényl)-amide de l'acide 3-tert-butyl-1-oxa-2,8-diaza-spiro[4.5]déc-2-én-8-oïque
[304] 3-phényl-1-(3-phényl-1-oxa-2,8-diaza-spiro[4.5]déc-2-én-8-yl)-propynone
[305] 1-(3-tert-butyl-1-oxa-2,8-diaza-spiro[4.5]déc-2-én-8-yl)-3-phényl-propynone
[306] ((4-tert-butyl-phényl)-méthyl-amide de l'acide 3-phényl-1-oxa-2,8-diaza-spiro[4.5]déc-2-én-8-oïque
[307] ((4-tert-butyl-phényl)-(3-phényl-1-oxa-2,8-diaza-spiro[4.5]déc-2-én-8-yl)-méthanone
[308] ((4-hydroxy-3-méthoxy-phényl)-(3-phényl-1-oxa-2,8-diaza-spiro[4.5]déc-2-én--yl)-méthanone
[309] ((4-iodo-phényl)-(3-phényl-1-oxa-2,8-diaza-spiro[4.5]déc-2-én-8-yl)-méthanone
[310] p-tolylamide de l'acide 3-tert-butyl-1-oxa-2,8-diaza-spiro[4.5]déc-2-én-8-oïque
[311] p-tolylamide de l'acide 3-(4-trifluorométhoxy-phényl)-1-oxa-2,8-diaza-spiro[4.5]déc-2-én-8-oïque
[312] ((4-tert-butyl-phényl)-amide de l'acide 3-(3-méthoxy-phényl)-1-oxa-2,8-diaza-spiro[4.5]déc-2-én-8-oïque
[313] 2-(4-tert-butyl-phényl)-1-(3-phényl-1-oxa-2,8-diaza-spiro[4.5]déc-2-én-8-yl)-éthanone
[314] 1-(3-phényl-1-oxa-2,8-diaza-spiro[4.5]déc-2-én-8-yl)-3-(4-trifluorométhyl-phényl)-propénone
[315] 3-(4-hydroxy-3-méthoxy-phényl)-1-(3-phényl-1-oxa-2,8-diaza-spiro[4.5]déc-2-én-8-yl)-propénone
[316] ((4-tert-butyl-phényl)-[3-(4-tert-butyl-phényl)-1-oxa-2,8-diaza-spiro[4.5]déc-2-én-8-yl]-méthanone
[317] 3-(4-tert-butyl-phényl)-1-[3-(4-tert-butyl-phényl)-1-oxa-2,8-diaza-spiro[4.5]déc-2-én-8-yl]-propénone
[318] 3-(4-tert-butyl-phényl)-1-[3-(4-trifluorométhoxy-phényl)-1-oxa-2,8-diaza-spiro[4.5]déc-2-én-8-yl]-propénone
[319] ((4-éthyl-phényl)-amide de l'acide 3-naphtalén-2-yl-1-oxa-2,8-diaza-spiro[4.5]déc-2-én-8-oïque
[320] 1-(3-phényl-1-oxa-2,8-diaza-spiro[4.5]déc-2-én-8-yl)-3,3-di-p-tolyl-propénone
[321] 3-(4-tert-butyl-phényl)-2-méthyl-1-(3-phényl-1-oxa-2,8-diaza-spiro[4.5]déc-2-én-8-yl)-propénone
[322] benzhydryl-amide de l'acide 3-phényl-1-oxa-2,8-diaza-spiro[4.5]déc-2-én-8-oique
[323] 3-(4-tert-butyl-phényl)-1-(3-phényl-1-oxa-2,8-diaza-spiro[4.5]déc-2-én-8-yl)-propénone
[324] 2-(4-tert-butyl-benzylidéne)-1-(3-phényl-1-oxa-2,8-diaza-spiro[4.5]déc-2-én-8-yl)-butén-1-one
[325] 3-(4-isopropyl-phényl)-1-(3-phényl-1-oxa-2,8-diaza-spiro[4.5]déc-2-én-8-yl)-propénone
[326] 3-(4-octyl-phényl)-1-(3-phényl-1-oxa-2,8-diaza-spiro[4.5]déc-2-én-8-yl)-propénone
[327] 3-(4-butyl-phényl)-1-(3-phényl-1-oxa-2,8-diaza-spiro[4.5]déc-2-én-8-yl)-propénone
[328] 3-(4-péntyl-phényl)-1-(3-phényl-1-oxa-2,8-diaza-spiro[4.5]déc-2-én-8-yl)-propénone
[329] ((4-tert-butyl-phényl)-amide de l'acide 3-(3-chloro-pyridin-2-yl)-1-oxa-2,8-diaza-spiro[4.5]déc-2-én-8-oïque
[330] ((4-pentafluorosulfanyl-phényl)-amide de l'acide 3-phényl-1-oxa-2,8-diaza-spiro[4.5]déc-2-ène-8-thiocarboxylique
[333] N-(4-tert-butylphényl)-3-(3-chlorophényl)-1-oxa-2,8-diazaspiro[4.5]déc-2-én-8-carboxamide
[334] ((E)-3-(4-cyclohexylphényl)-1-(3-(3-méthoxyphényl)-1-oxa-2,8-diazaspiro[4.5]déc-2-én-8-yl)prop-2-én-1-one
[335] N-(4-tert-butylphényl)-3-(4-cyclohexylphényl)-1-oxa-2,8-diazaspiro[4.5]déc-2-ène-8-carboxamide
[336] ((E)-3-(4-tert-butylphényl)-3-phényl-1-(3-phényl-1-oxa-2,8-diazaspiro[4.5]déc-2-én-8-yl)prop-2-én-1-one
[337] ((2E,4E)-3-(4-tert-butylphényl)-5-phényl-1-(3-phényl-1-oxa-2,8-diazaspiro[4.5]déc-2-én-8-yl)penta-2,4-dién-1-one
[338] ((Z)-3-(4-tert-butylphényl)-1-(3-phényl-1-oxa-2,8-diazaspiro[4.5]déc-2-én-8-yl)-3-(thiophén-3-yl)prop-2-én-1-one
[339] ((E)-3-(4-tert-butylphényl)-1-(3-(3-méthoxyphényl)-1-oxa-2,8-diazaspiro[4.5]déc-2-én-8-yl)-3-(4-(trifluorométhyl)phényl)prop-2-én-1-one
[340] ((E)-N-(5-(8-(3-(4-tert-butylphényl)acryloyl)-1-oxa-2,8-diazaspiro[4.5]déc-2-én-3-yl)-2-fluorophényl)méthanesulfonamide
[341] ((E)-3-(4-pentafluorosulfanyl)-1-(3-(3-méthoxyphényl)-1-oxa-2,8-diazaspiro[4.5]déc-2-én-8-yl)prop-2-én-1-one
[342] ((E)-1-(3-(3-méthoxyphényl)-1-oxa-2,8-diazaspiro[4.5]déc-2-én-8-yl)-3-(2-morpholino-6-(trifluorométhyl)pyridin-3-yl)prop-2-én-1-one
[343] N-(2-fluoro-4-(1-oxo-1-(3-phényl-1-oxa-2,8-diazaspiro[4.5]déc-2-én-8-yl)propan-2-yl)phényl)méthanesulfonamide
[344] N-(2-fluoro-4-(1-(3-(3-méthoxyphényl)-1-oxa-2,8-diazaspiro[4.5]déc-2-én-8-yl)-1-oxopropan-2-yl)phényl)méthanesulfonamide
[345] ((Z)-3-(4-tert-butylphényl)-1-(3-(3-méthoxyphényl)-1-oxa-2,8-diazaspiro[4.5]déc-2-én-8-yl)-3-(thiophén-3-yl)prop-2-én-1-one
[346] ((E)-3-(3-bromophényl)-1-(3-phényl-1-oxa-2,8-diazaspiro[4.5]déc-2-én-8-yl)prop-2-én-1-one
[347] ((E)-3-(2-bromophényl)-1-(3-phényl-1-oxa-2,8-diazaspiro[4.5]déc-2-én-8-yl)prop-2-én-1-one
[348] 3-(3-fluorophényl)-N-(4-(trifluorométhyl)phényl)-1-oxa-2,8-diazaspiro[4.5]déc-2-ène-8-carboxamide
[349] N-(4-tert-butylphényl)-3-(4-fluorophényl)-1-oxa-2,8-diazaspiro[4.5]déc-2-ène-8-carboxamide
[350] 3-(4-tert-butylphényl)-1-(3-phényl-1-oxa-2,8-diazaspiro[4.5]déc-2-én-8-yl)propan-1-one
[351] N-(4-tert-butylphényl)-3-(3-fluorophényl)-1-oxa-2,8-diazaspiro[4.5]déc-2-ène-8-carboxamide
[352] N-(4-tert-butylphényl)-3-(2-fluorophényl)-1-oxa-2,8-diazaspiro[4.5]déc-2-ène-8-carboxamide
[353] ((E)-3-(4-tert-butylphényl)-1-(3-(3-fluorophényl)-1-oxa-2,8-diazaspiro[4.5]déc-2-én-8-yl)prop-2-én-1-one
[354] ((Z)-1-(3-(3-méthoxyphényl)-1-oxa-2,8-diazaspiro[4.5]déc-2-én-8-yl)-4,4-diméthyl-3-phénylpent-2-én-1-one
[355] ((E)-1-(3-phényl-1-oxa-2,8-diazaspiro[4.5]déc-2-én-8-yl)-3-(2-(pipéridin-1-yl)-6-(trifluorométhyl)pyridin-3-yl)prop-2-én-1-one [356] (E)-1-(3-(3-chlorophényl)-1-oxa-2,8-diazaspiro[4.5]déc-2-én-8-yl)-3-(2-(pipéridin-1-yl)-6-(trifluorométhyl)pyridin-3-yl)prop-2-én-1-one [357] (2E,4E)-3-tert-butyl-1-(3-(3-méthoxyphényl)-1-oxa-2,8-diazaspiro[4.5]déc-2-én-8-yl)-5-phénylpénta-2,4-dién-1-one
[358] ((2E,4E)-3-(4-tert-butylphényl)-1-(3-(3-fluorophényl)-1-oxa-2,8-diazaspiro[4.5]déc-2-én-8-yl)-5-phénylpenta-2,4-dién-1-one
[359] ((Z)-3-(4-tert-butylphényl)-3-(3,4-dichlorophényl)-1-(3-(2-fluorophényl)-1-oxa-2,8-diazaspiro[4.5]déc-2-én-8-yl)prop-2-én-1-one
[360] ((Z)-3-(4-tert-butylphényl)-3-(3,4-dichlorophényl)-1-(3-(3-fluorophényl)-1-oxa-2,8-diazaspiro[4.5]déc-2-én-8-yl)prop-2-én-1-one
[361] 1-(3-(3-méthoxyphényl)-1-oxa-2,8-diazaspiro[4.5]déc-2-én-8-yl)-3-(4-(trifluorométhyl)phényl)prop-2-yn-1-one
[362] ((E)-1-(3-(3-méthoxyphényl)-1-oxa-2,8-diazaspiro[4.5]déc-2-én-8-yl)-3-(6-(trifluorométhyl)pyridin-3-yl)prop-2-én-1-one
[363] 1-(3-phényl-1-oxa-2,8-diazaspiro[4.5]déc-2-én-8-yl)-3-(4-(trifluorométhyl)phényl)prop-2-yn-1-one
[364] N-(4-tert-butylphényl)-3-(2-fluorophényl)-6-méthyl-1-oxa-2,8-diazaspiro[4.5]déc-2-én-8-carboxamide
[365] ((E)-1-(3-(3-fluorophényl)-1-oxa-2,8-diazaspiro[4.5]déc-2-én-8-yl)-3-(2-(pipéridin-1-yl)-6-(trifluorométhyl)pyridin-3-yl)prop-2-én-1-one
[366] N-(2-fluoro-4-(1-(3-(3-fluorophényl)-1-oxa-2,8-diazaspiro[4.5]déc-2-én-8-yl)-1-oxopropan-2-yl)phényl)méthanesulfonamide
[367] ((E)-1-(6-méthyl-3-phényl-1-oxa-2,8-diazaspiro[4.5]déc-2-én-8-yl)-3-(2-(pipéridin-1-yl)-6-(trifluorométhyl)pyridin-3-yl)prop-2-én-1-one
[368] ((E)-3-(2-bromo-4-tert-butylphényl)-1-(3-phényl-1-oxa-2,8-diazaspiro[4.5]déc-2-én-8-yl)prop-2-én-1-one
[369] N-(4-tert-butylphényl)-6-méthyl-3-phényl-1-oxa-2,8-diazaspiro[4.5]déc-2-én-8-carboxamide
[370] 2-(4-tert-butylphényl)-1-(3-phényl-1-oxa-2,8-diazaspiro[4.5]déc-2-én-8-yl)propan-1-one
[371] N-(4-tert-butylphényl)-3-phényl-1-oxa-2,7-diazaspiro[4.4]none-2-ène-7-carboxamide
[372] N-(4-tert-butylbenzyl)-3-phényl-1-oxa-2,8-diazaspiro[4.5]déc-2-ène-8-carboxamide
[373] N-(4-tert-butylphényl)-3-phényl-1-oxa-2,8-diazaspiro[4.6]undéc-2-ène-8-carboxamide
[374] N-(4-tert-butylbenzyl)-3-(3-méthoxyphényl)-1-oxa-2,8-diazaspiro[4.5]déc-2-ène-8-carboxamide
[375] N-(1-(4-tert-butylphényl)éthyl)-3-(3-méthoxyphényl)-1-oxa-2,8-diazaspiro[4.5]déc-2-ène-8-carboxamide
[376] N-(4-tert-butylcyclohexyl)-3-phényl-1-oxa-2,8-diazaspiro[4.5]déc-2-ène-8-carboxamide
[377] N-(4-tert-butylphénéthyl)-3-phényl-1-oxa-2,8-diazaspiro[4.5]déc-2-ène-8-carboxamide
[378] N-(4-tert-butylphényl)-3-(4-chloro-3-méthoxyphényl)-1-oxa-2,8-diazaspiro[4.5]déc-2-ène-8-carboxamide
[379] 8-(5-tert-butyl-1H-benzo[d]imidazol-2-yl)-3-phényl-1-oxa-2,8-diazaspiro[4.5]déc-2-ène
[380] 3-phényl-8-(5-(trifluorométhyl)-1H-benzo[d]imidazol-2-yl)-1-oxa-2,8-diazaspiro[4.5]déc-2-ène
[381] ((E)-N-(2-fluoro-4-(3-oxo-3-(3-phényl-1-oxa-2,8-diazaspiro[4.5]déc-2-én-8-yl)prop-1-ényl)phényl)méthanesulfonamide
[382] ((E)-N-(2-fluoro-4-(3-(3-(3-méthoxyphényl)-1-oxa-2,8-diazaspiro[4.5]déc-2-én-8-yl)-3-oxoprop-1-ényl)phényl)méthanesulfonamide
[383] 5-tert-butyl-2-(3-phényl-1-oxa-2,8-diazaspiro[4.5]déc-2-én-8-yl)benzo[d]oxazole
[384] 5-tert-butyl-2-(3-(3-méthoxyphényl)-1-oxa-2,8-diazaspiro[4.5]déc-2-én-8-yl)benzo[d]oxazole
[385] 6-méthyl-3-phényl-N-(4-(trifluorométhyl)phényl)-1-oxa-2,8-diazaspiro[4.5]déc-2-ène-8-carboxamide
[386] 3-benzyl-N-(4-tert-butylphényl)-1-oxa-2,8-diazaspiro[4.5]déc-2-ène-8-carboxamide
[387] N-(4-tert-butylphényl)-3-phénéthyl-1-oxa-2,8-diazaspiro[4.5]déc-2-ène-8-carboxamide
[388] 3-(4-hydroxy-3-méthoxy-phényl)-1-(3-phényl-1-oxa-2,8-diaza-spiro[4.5]décén-8-yl)-propénone
[389] 3-(4-tert-butyl-phényl)-1-(3-phényl-1-oxa-2,8-diaza-spiro[4.5]décén-8-yl)-propénone
[390] ((4-tert-butyl-phényl)-amide de l'acide 3-(3-chloro-pyridin-2-yl)-1-oxa-2,8-diaza-spiro[4.5]déc-2-én-8-oïque
[391] 3,3-di-p-tolyl-1-(3-p-tolyl-1-oxa-2,8-diaza-spiro[4.5]déc-2-én-8-yl)-propénone
[392] 3-(4-tert-butyl-phényl)-1-[3-(4-chloro-phényl)1-oxa-2,8-diaza-spiro[4.5]déc-2-én-8-yl]-propénone
[393] 3-(4-tert-butyl-phényl)-2-éthyl-1-(3-p-tolyl-1-oxa-2,8-diaza-spiro[4.5]déc-2-én-8-yl)-propénone
[394] ((4-tert-butyl-phényl)-(3-p-tolyl-1-oxa-2,8-diaza-spiro[4.5]déc-2-én-8-yl)-méthanone
[395] 3-(4-tert-butyl-phényl)-1-(3-p-tolyl-1-oxa-2,8-diaza-spiro[4.5]déc-2-én-8-yl)-propénone
[396] 3-(4-tert-butyl-phényl)-2-méthyl-1-(3-p-tolyl-1-oxa-2,8-diaza-spiro[4.5]déc-2-én-8-yl)-propénone
[397] 2-(4-tert-butyl-phényl)-1-[3-(3-méthoxy-phényl)-1-oxa-2,8-diaza-spiro[4.5]déc-2-én-8-yl]-but-2-én-1-one
[398] 3-(4-tert-butyl-phényl)-1-[3-(3-méthoxy-phényl)-1-oxa-2,8-diaza-spiro[4.5]déc-2-én-8-yl]-propynone
[399] 3-(4-tert-butyl-phényl)-1-[3-(3-méthoxy-phényl)-1-oxa-2,8-diaza-spiro[4.5]déc-2-én-8-yl]-propénone
[400] 1-[3-(3-méthoxy-phényl)-1-oxa-2,8-diaza-spiro[4.5]déc-2-én-8-yl]-2,2-diphényl-propan-1-one
[401] 1-[3-(3-méthoxy-phényl)-1-oxa-2,8-diaza-spiro[4.5]déc-2-én-8-yl]-2,2-diphényl-éthanone
[402] ((2,3)-dihydrobenzo[1.4]dioxin-6-yl)-amide de l'acide 3-(3-méthoxy-phényl)-1-oxa-2,8-diaza-spiro[4.5]déc-2-én-8-oïque
[403] 3-(6-tert-butyl-pyridin-3-yl)-1-[3-(3-méthoxy-phényl)-1-oxa-2,8-diaza-spiro[4.5]déc-2-én-8-yl]-propénone
[404] 3-(6-tert-butyl-pyridin-3-yl)-1-[3-(4-chloro-3-méthoxy-phényl)-1-oxa-2,8-diaza-spiro[4.5]déc-2-én-8-yl]-propénone
[405] 2,2-diphényl-1-(3-phényl-1-oxa-2,8-diaza-spiro[4.5]déc-2-én-8-yl)-propan-1-one
[406] 3-(6-tert-butyl-pyridin-3-yl)-1-(3-phényl-1-oxa-2,8-diaza-spiro[4.5]déc-2-én-8-yl)-propénone
[407] ((4-tert-butyl-phényl)-amide de l'acide 3-(4-isopropyl-phényl)-1-oxa-2,8-diaza-spiro[4.5]déc-2-én-8-oïque
[408] ((4-tert-butyl-phényl)amide de l'acide 3-(2,3-dihydro-benzo[1,4]dioxin-6-yl)-1-oxa-2,8-diaza-spiro[4.5]déc-2-én-8-oïque
[409] N-[2-fluoro-4-[3-(3-méthoxy-phényl)-1-oxa-2,8-diaza-spiro[4.5][déc-2-én-8-carbonyl]-phényl]-méthanesulfonamide
[410] N-[2-fluoro-4-(3-phényl-1-oxa-2,8-diaza-spiro[4.5][déc-2-én-8-carbonyl]-phényl]-méthanesulfonamide
[411] 3-(4-cyclohexyl-phényl)-1-(3-phényl-1-oxa-2,8-diaza-spiro[4.5]déc-2-én-8-yl)-propénone
[412] 1-[3-(3-méthoxy-phényl)-1-oxa-2,8-diaza-spiro[4.5]déc-2-én-8-yl]-2,2,2-triphényl-éthanone [415] [3-(4-chloro-3-méthoxy-phényl)-1-oxa-2,8-diaza-spiro[4.5]déc-2-én-8-yl]-(2-chloro-6-trifluorométhyl-pyridin-3-yl)-méthanone
[416] [3-(3-méthoxy-phényl)-1-oxa-2,8-diaza-spiro[4.5]déc-2-én-8-yl]-(2-morpholin-4-yl-6-trifluorométhyl-pyridin-3-yl)-méthanone
[417] 3-(4-tert-butyl-phényl)-1-[3-(3-chloro-phényl)-1-oxa-2,8-diaza-spiro[4.5]déc-2-én-8-yl]-propénone
[418] 3-(4-tert-butyl-phényl)-1-[3-(2,3-dihydro-benzo[1.4]dioxin-6-yl)-1-oxa-2,8-diaza-spiro[4.5]déc-2-én-8-yl-propénone
[419] 3-(4-tert-butyl-phényl)-1-[3-(4-cyclohexyl-phényl)-1-oxa-2,8-diaza-spiro[4.5]déc-2-én-8-yl]-propénone
[420] N-[4-[3-(4-cyclohexyl-phényl)-1-oxa-2,8-diaza-spiro[4.5]déc-2-én-8-carbonyl]-2-fluoro-phényl]-méthanesulfonamide
[421] 2-hydroxy-2,2-diphényl-1-(3-phényl-1-oxa-2,8-diaza-spiro[4.5]déc-2-8-yl]-éthanone
[423] 3-(4-tert-butyl-phényl)-1-(3-quinolin-3-yl-1-oxa-2,8-diaza-spiro[4.5]déc-2-én-8-yl)-propénone
[424] ((4-trifluorométhyl-phényl)-amide de l'acide 3-(2,3-dihydro-benzo[1.4]dioxin-6-yl)-1-oxa-2,8-diaza-spiro[4.5]déc-2-én-8-oïque
[425] ((4-trifluorométhyl-phényl)-amide de l'acide 3-(3-chloro-phényl)-1-oxa-2,8-diaza-spiro[4.5]déc-2-én-8-oïque
[426] ((4-tert-butyl-phényl)-[3-(3-méthoxy-phényl)-1-oxa-2,8-diaza-spiro[4.5]déc-2-én-8-yl]-méthanone
[427] 3-(4-cyclohexyl-phényl)-1-oxa-2,8-diaza-sprio[4.5]déc-2-én-8-oïque(4-trifluorométhyl-phényl)amide
[428] 3-(4-tert-butyl-phényl)-3-(4-chloro-phényl)-1-[3-(3-chloro-phényl)-1-oxa-2,8-diaza-spiro[4.5]déc-2-én-8-yl]-propénone
[429] 3-(4-tert-butyl-phényl)-1-[3-(3-chloro-phényl)-1-oxa-2,8-diaza-spiro[4.5]déc-2-én-8-yl]-3-(3-méthoxy-phényl)-propénone
[430] 3-(4-tert-butyl-phényl)-1-[3-(4-chloro-3-méthoxy-phényl)-1-oxa-2,8-diaza-spiro[4.5]déc-2-én-8-yl]-3-(4-trifluorométhyl-phényl)-propénone
[431] 3-(4-pentafluorosulfanyl-phényl)-1-[3-(3-méthoxy-phényl)-1-oxa-2,8-diaza-spiro[4.5]déc-2-én-8-yl]-propénone
[432] 3-(4-tert-butyl-phényl)-1-[3-(4-chloro-3-méthoxy-phényl)-1-oxa-2,8-diaza-spiro[4.5]déc-2-én-8-yl]-propénone
[433] 3-(4-tert-butyl-phényl)-1-[3-(4-chloro-3-méthoxy-phényl)-1-oxa-2,8-diaza-spiro[4.5]déc-2-én-8-yl]-propynone
[434] 3-(4-tert-butyl-phényl)-1-[3-(3-méthoxy-phényl)-1-oxa-2,8-diaza-spiro[4.5]déc-2-én-8-yl]-3-(2-trifluorométhyl-phényl)-propénone
[435] ((4-isopropyl-phényl)-amide de l'acide 3-thiazol-2-yl-1-oxa-2,8-diaza-spiro[4.5]déc-2-én-8-oïque
[436] ((4-trifluorométhoxy-phényl)-amide de l'acide 3-thiazol-2-yl-1-oxa-2,8-diaza-spiro[4.5]déc-2-én-8-oïque
[437] 3,3-bis-(4-tert-butyl-phényl)-1-(3-phényl-1-oxa-2,8-diaza-spiro[4.5]déc-2-én-8-yl)-propénone et
[438] ((4-tert-butyl-phényl)-amide de l'acide 3-thiazol-2-yl-1-oxa-2,8-diaza-spiro[4.5]déc-2-én-8-oïque ;
à chaque fois éventuellement sous la forme d'un de leurs stéréoisomères purs, notamment des énantiomères ou des diastéréomères, de leurs racémats ou sous la forme d'un mélange de stéréoisomères, notamment des énantiomères ou/ou des diastéréomères, en un rapport de mélange quelconque, ou à chaque fois sous la forme de sels correspondants, ou à chaque fois sous la forme de solvates correspondants.

23. Composés selon une ou plusieurs des revendications 1 à 22, **caractérisés en ce qu'**ils présentent lors de l'essai FLIPR en une concentration de 10 µM une inhibition de l'afflux d'ions Ca²⁺ dans les ganglions rachidiens de rats d'au moins 30 %, de préférence d'au moins 40 %, de manière particulièrement préférée d'au moins 50 %, de manière tout particulièrement préférée d'au moins 70 %, de manière encore davantage préférée d'au moins 90 %, en comparaison de l'inhibition maximale atteignable de l'afflux d'ions Ca²⁺ avec la capsaïcine en une concentration de 10 µM.

24. Procédé de fabrication de composés spiro substitués de formule générale I selon une ou plusieurs des revendications 1 à 23, **caractérisé en ce qu'**au moins un composé de formule générale II 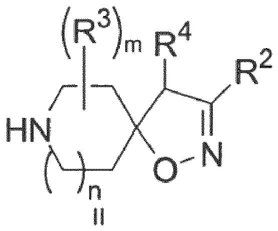 dans laquelle R², R³, R⁴, m et n ont la signification selon une ou plusieurs des revendications 1 à 23, est mis en réaction dans un milieu réactionnel avec au moins un isocyanate de formule générale R⁵-N=C=O, R⁵ ayant la signification selon une ou plusieurs des revendications 1 à 23, éventuellement en présence d'au moins une base, de préférence en présence d'au moins une base choisie dans le groupe constitué par la triéthylamine, la 4,4-diméthylaminopyridine, la diisopropyléthylamine, la pyridine et la N-méthylmorpholine, pour former au moins un composé de formule générale I, dans laquelle R², R³, R⁴, m et n ont la signification indiquée précédemment et R¹ représente -C(=O)-NR⁵R⁶, R⁵ ayant la signification indiquée précédemment et R⁶ représentant un radical hydrogène, et celui-ci est éventuellement purifié et/ou isolé
ou
au moins un composé de formule générale II est mis en réaction dans un milieu réactionnel avec au moins un isothiocyanate de formule générale S=C=N-R⁷, R⁷ ayant la signification selon une ou plusieurs des revendications 1 à 23, éventuellement en présence d'au moins une base, de préférence en présence d'au moins une base choisie dans le groupe constitué par la triéthylamine, la 4,4-diméthylaminopyridine, la diisopropyléthylamine, la pyridine et la N-méthylmorpholine, pour former au moins un composé de formule générale I, dans laquelle R², R³, R⁴, m et n ont la signification indiquée précédemment et R¹ représente -C(=S)-NR⁷R⁸, R⁷ ayant la signification indiquée précédemment et R⁸ représentant un radical hydrogène, et celui-ci est éventuellement purifié et/ou isolé
et au moins un composé de formule générale I, dans laquelle R², R³, R⁴, m et n ont la signification indiquée précédemment et R¹ représente -C(=O)-NR⁵R⁶ ou-C(=S)-NR⁷R⁸, R⁶ et R⁸ représentant chacun un radical hydrogène, est éventuellement mis en réaction dans un milieu réactionnel, en présence d'au moins une base, de préférence en présence d'au moins un sel d'hydrure métallique ou d'un sel d'alcoolate métallique, de manière particulièrement préférée en présence d'un sel d'hydrure métallique ou d'un sel d'alcoolate métallique choisi dans le groupe constitué par l'hydrure de sodium, l'hydrure de potassium, le tert-butanolate de potassium, le tert-butanolate de sodium, le méthanolate de potassium, le méthanolate de sodium, l'éthanolate de sodium et l'éthanolate de potassium, avec au moins un composé de formule générale LG-R⁶ ou de formule générale LG-R⁸, LG représentant un groupe partant, de préférence un atome d'halogène, de manière particulièrement préférée un atome de chlore, et R⁶ et R⁸ ayant la signification indiquée précédemment à l'exception de l'hydrogène, pour former au moins un composé de formule générale I, dans laquelle R² à R⁴, m et n ont la signification indiquée précédemment et R¹ représente - C(=O)-NR⁵R⁶ ou -C(=S)-NR⁷R⁸, et celui-ci est éventuellement purifié et/ou isolé,
ou
au moins un composé de formule générale II est mis en réaction dans un milieu réactionnel, en présence d'au moins une base, de préférence en présence d'au moins un sel d'hydrure métallique, de manière particulièrement préférée en présence d'un hydrure de sodium et/ou de potassium, avec au moins un composé de formule générale LG-R¹, R¹ ayant la signification selon une ou plusieurs des revendications 1 à 23 à l'exception de -C(=O)-NR⁵R⁶, -C(=S)-NR⁷R⁸, -C(=O)-R⁹ et -S((=O)-R¹⁰, et LG représentant un groupe partant, de préférence un atome d'halogène, de manière particulièrement préférée un atome de chlore, pour former au moins un composé de formule générale I, dans laquelle R¹ à R⁴, m et n ont la signification indiquée précédemment, et celui-ci est éventuellement purifié et/ou isolé
ou
au moins un composé de formule générale II est mis en réaction dans un milieu réactionnel en présence d'au moins un réducteur, avec au moins un composé de formule générale R¹-C(=O)-H, R¹ ayant la signification selon une ou plusieurs des revendications 1 à 23 à l'exception de -C(=O)-NR⁵R⁶, -C(=S)-NR⁷R⁸, -C(=O)-R⁹ et -S(=O)-R¹⁰, pour former au moins un composé de formule générale I, dans laquelle R¹ à R⁴, m et n ont la signification indiquée précédemment, et celui-ci est éventuellement purifié et/ou isolé
ou
au moins un composé de formule générale II est mis en réaction dans un milieu réactionnel, éventuellement en présence d'au moins une base, avec au moins un composé de formule générale R⁹-C(=O)-LG, R⁹ ayant la signification selon une ou plusieurs des revendications 1 à 23 et LG représentant un groupe partant, de préférence un radical halogène, ou dans un milieu réactionnel en présence d'au moins un réactif de couplage, éventuellement en présence d'au moins une base, avec un composé de formule générale R⁹-C(=O)-OH, R⁹ ayant la signification selon une ou plusieurs des revendications 1 à 23, pour former au moins un composé de formule générale I, dans laquelle R² à R⁴, m et n ont la signification indiquée précédemment et R¹ représente -C((=O)-R⁹, et celui-ci est éventuellement purifié et/ou isolé
ou
au moins un composé de formule générale II est mis en réaction dans un milieu réactionnel, éventuellement en présence d'au moins une base, avec au moins un composé de formule générale R¹⁰-S(=O)₂-LG, R¹⁰ ayant la signification selon une ou plusieurs des revendications 1 à 23 et LG représentant un groupe partant, de préférence un radical halogène, pour former au moins un composé de formule générale I, dans laquelle R² à R⁴, m et n ont la signification indiquée précédemment et R¹ représente -S(=O)₂-R¹⁰, et celui-ci est éventuellement purifié et/ou isolé.

25. Médicament contenant au moins un composé selon une ou plusieurs des revendications 1 à 23 et éventuellement un ou plusieurs adjuvants physiologiquement compatibles.

26. Médicament selon la revendication 25, pour la prophylaxie et/ou le traitement de la douleur, de préférence de la douleur choisie dans le groupe constitué par la douleur aiguë, la douleur chronique et la douleur neuropathique.

27. Médicament selon la revendication 25, pour le traitement et/ou la prophylaxie d'une ou de plusieurs maladies choisies dans le groupe constitué par la douleur articulaire ; les migraines ; les dépressions ; les neuropathies ; les lésions nerveuses ; les maladies neurodégénératives, de préférence choisies dans le groupe constitué par la sclérose en plaques, la maladie d'Alzheimer, la maladie de Parkinson et la maladie d'Huntington ; les dysfonctionnements cognitifs, de préférence les déficiences cognitives, de manière particulièrement préférée les troubles de la mémoire ; l'épilepsie ; les maladies des voies respiratoires, de préférence choisies dans le groupe constitué par l'asthme et l'inflammation pulmonaire ; la toux ; l'incontinence urinaire ; une vessie hyperactive (overactive bladder, OAB) ; les ulcères gastriques ; le syndrome du côlon irritable ; les accidents vasculaires cérébraux ; les irritations oculaires ; les irritations cutanées ; les maladies cutanées neurotiques ; les maladies inflammatoires, de préférence les inflammations de l'intestin ; la diarrhée ; le prurit ; les troubles de l'alimentation, de préférence choisis dans le groupe constitué par la boulimie, la cachexie, l'anorexie et l'obésité ; la dépendance à des médicaments ; l'abus de médicaments ; les phénomènes de sevrage en cas de dépendance à des médicaments ; le développement de tolérance à des médicaments, de préférence aux opioïdes naturels ou synthétiques ; la dépendance à des drogues ; l'abus de drogues ; les phénomènes de sevrage en cas de dépendance à des drogues ; la dépendance à l'alcool ; l'abus d'alcool et les phénomènes de sevrage en cas de dépendance à l'alcool ; pour la diurèse ; pour l'antinatriurèse ; pour la régulation du système cardiovasculaire ; pour l'augmentation de la vigilance ; pour l'augmentation de la libido ; pour la modulation de l'activité physique ; pour l'anxiolyse ; pour l'anesthésie locale et/ou pour l'inhibition des effets secondaires indésirables, de préférence choisis dans le groupe constitué par l'hyperthermie, l'hypertension et le rétrécissement des bronches déclenché par l'administration d'agonistes des récepteurs vanilloïdes 1 (récepteurs de VR1/TRPV1), de préférence choisis dans le groupe constitué par la capsaïcine, la résinifératoxine, l'olvanil, l'arvanil, SDZ-249665, SDZ-249482, le nuvanil et le capsavanil.

28. Utilisation d'au moins un composé selon une ou plusieurs des revendications 1 à 23 pour la fabrication d'un médicament pour la prophylaxie et/ou le traitement de la douleur, de préférence de la douleur choisie dans le groupe constitué par la douleur aiguë, la douleur chronique et la douleur neuropathique.

29. Utilisation d'au moins un composé selon une ou plusieurs des revendications 1 à 23 pour la fabrication d'un médicament pour la prophylaxie et/ou le traitement d'une ou de plusieurs maladies choisies dans le groupe constitué par la douleur articulaire ; les migraines ; les dépressions ; les neuropathies ; les lésions nerveuses ; les maladies neurodégénératives, de préférence choisies dans le groupe constitué par la sclérose en plaques, la maladie d'Alzheimer, la maladie de Parkinson et la maladie d'Huntington ; les dysfonctionnements cognitifs, de préférence les déficiences cognitives, de manière particulièrement préférée les troubles de la mémoire ; l'épilepsie ; l'incontinence urinaire ; une vessie hyperactive (overactive bladder, OAB) ; les ulcères gastriques ; le syndrome du côlon irritable ; les accidents vasculaires cérébraux ; la diarrhée ; le prurit ; les troubles de l'alimentation, de préférence choisis dans le groupe constitué par la boulimie, la cachexie, l'anorexie et l'obésité ; la dépendance à des médicaments ; l'abus de médicaments ; les phénomènes de sevrage en cas de dépendance à des médicaments ; le développement de tolérance à des médicaments, de préférence aux opioïdes naturels ou synthétiques ; la dépendance à des drogues ; l'abus de drogues ; les phénomènes de sevrage en cas de dépendance à des drogues ; la dépendance à l'alcool ; l'abus d'alcool et les phénomènes de sevrage en cas de dépendance à l'alcool ; pour la diurèse ; pour l'antinatriurèse ; pour la régulation du système cardiovasculaire ; pour l'augmentation de la vigilance ; pour l'augmentation de la libido ; pour la modulation de l'activité physique ; pour l'anxiolyse ; pour l'anesthésie locale et/ou pour l'inhibition des effets secondaires indésirables, de préférence choisis dans le groupe constitué par l'hyperthermie, l'hypertension et le rétrécissement des bronches déclenché par l'administration d'agonistes du récepteur vanilloïdes 1 (récepteurs de VR1/TRPV1), de préférence choisis dans le groupe constitué par la capsaïcine, la résinifératoxine, l'olvanil, l'arvanil, SDZ-249665, SDZ-249482, le nuvanil et le capsavanil.

30. Utilisation d'au moins un composé selon une ou plusieurs des revendications 1 à 23 pour la fabrication d'un médicament pour la prophylaxie et/ou le traitement d'une ou de plusieurs maladies choisies dans le groupe constitué par les maladies des voies respiratoires, de préférence choisies dans le groupe constitué par l'asthme et l'inflammation pulmonaire ; la toux ; les irritations oculaires ; les irritations cutanées ; les maladies cutanées neurotiques et les maladies inflammatoires, de préférence les inflammations de l'intestin.
